# EUROPEAN PATENT APPLICATION

(11) **EP 3 425 395 A1**
(43) Date of publication of application: **09.01.2019**
(21) Application number: 17760186.1
(22) Date of filing: 03.03.2017
(51) Int. Cl.: G01N 33/53, A61K 39/35, A61K 49/00, A61P 37/08, C07K 4/10

(54) **SOYBEAN ALLERGY ANTIGEN**

(30) Priority: 03.03.2016 JP 2016041547
(71) Applicant: Hoyu Co., Ltd., Nagoya-shi, Aichi 461-8650 (JP)
(72) Inventor: MATSUNAGA, Kayoko, Toyoake-shi Aichi 470-1192 (JP); YAGAMI, Akiko, Toyoake-shi Aichi 470-1192 (JP); SHIMOJO, Naoshi, Nagakute-shi Aichi 480-1136 (JP); NAKAMURA, Masashi, Nagakute-shi Aichi 480-1136 (JP); SATO, Nayu, Nagakute-shi Aichi 480-1136 (JP)
(74) Representative: Keirstead, Tanis Evelyne
(86) International application number: PCT/JP2017/008593
(87) International publication number: WO 2017/150724

(57) **Abstract**

The present invention provides novel antigens of an allergy to soybean, methods and kits for diagnosing an allergy to soybean, pharmaceutical compositions comprising such an antigen, soybeans or processed products of soybean in which such an antigen is eliminated, and a tester for determining the presence or absence of a soybean antigen in an object of interest.

## Description

### TECHNICAL FIELD

The present invention relates to a novel antigen of an allergy to soybean. The present invention also relates to a kit, a composition, and a method for diagnosing allergy to soybean. The present invention also relates to a pharmaceutical composition comprising such an antigen and soybean or processed products of soybean in which such an antigen is eliminated or reduced. The present invention further relates to a tester for determining the presence or absence of a soybean antigen in an object of interest.

### BACKGROUND ART

In serum and tissues of allergic patients, IgE antibodies specific to particular antigens are produced. Physiological consequences caused by interaction between such IgE antibodies and such particular antigens elicit allergic reactions.

In the process of production of conventional allergy testing agents, antigen reagents are commonly prepared simply by grinding a candidate allergenic food, material or the like (Patent Literature 1). As seen above, conventional antigen reagents do not necessarily contain only a particular antigenic protein inducing an allergic reaction (allergen component), and rather contain different types of protein components. Thus, conventional antigen reagents contain varied amounts of allergen components. For this reason, the only case where conventional allergy tests have permitted detection of a positive allergic reaction is when in a conventional antigen reagent containing many types of proteins, a particular protein acting as an allergen component is present in an amount exceeding a threshold that allows determination of a positive reaction for binding to an IgE antibody. However, no determination of a positive reaction was possible and diagnosis efficiency was not sufficiently high when using a conventional allergy testing agent in patients possessing an IgE antibody binding to an allergen component present in small amounts in an allergen such as food.

The severity and symptoms of an allergic reaction do not necessarily correlate with the content of an allergen component. Even when a patient's IgE antibody reacts with an allergen component present in trace amounts in a candidate allergic food, material or the like, the allergic reaction may develop allergic symptoms or may affect the severity of those symptoms.

An attempt to increase the diagnostic efficiency is being made by examining IgE antibodies to components composing a crude antigen to distinguish sensitization that directly contributes to a diagnosis from sensitization based on cross-antigenicity by a pan-allergen or the like. 8 soybean allergens are currently registered to the World Health Organization/Intemational Union of Immunological Societies (WHO/IUIS).

However, while it is necessary to exhaustively identify allergen components in candidate allergic foods and materials in order to enhance the reliability of allergy tests, the patient detection rate by the measurement of such allergenic components is far insufficient. Identification of novel allergens in soybean is very important not only for increasing the precision of diagnosis, but also for determining targets of therapeutic agents and low allergenic foods.

Meanwhile, in the field of protein separation and purification, various efforts have conventionally been made to develop methods for separating and purifying a protein or nucleic acid of interest from cell extracts or the like. Such methods may well be exemplified by dialysis based on salt concentration, and centrifugal separation.

Other efforts have been made to develop many purification methods based on electric charges of protein or nucleic acid residues or on the difference in molecular weight. Electric charge-based purification methods can be exemplified by column chromatography using ion exchange resins, and isoelectric focusing. Purifications based on molecular weight difference can be exemplified by centrifugal separation, molecular-sieve column chromatography, and SDS-PAGE (sodium dodecyl sulfate-polyacrylamide gel electrophoresis).

In recent years, a method for separating and purifying many different proteins from a small amount of sample has been used, which is more specifically a two-dimensional electrophoresis consisting of isoelectric focusing in the first dimension, followed by SDS-PAGE in the second dimension. The present applicant has conventionally developed some 2D electrophoresis methods with high separation ability (Patent Literature 2-5).

### CITATION LIST

### PATENT LITERATURE

PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548

### SUMMARY OF INVENTION

### TECHNICAL PROBLEM

The present invention provides novel antigens of an allergy to soybean. The present invention also provides methods and kits for diagnosing allergy to soybean. The present invention also provides pharmaceutical compositions comprising such an antigen and soybean or processed products of soybean in which such an antigen is eliminated or reduced. The present invention further provides testers for determining the presence or absence of a soybean antigen in an object of interest.

### SOLUTION TO PROBLEM

In order to solve the aforementioned problems, the present inventors had made intensive studies to identify causative antigens of an allergy to soybean. As a result, the inventors succeeded in identifying novel antigens to which an IgE antibody in the serum of a soybean-allergic patient specifically binds. The present invention has been completed based on this finding.

Thus, in one embodiment, the present invention can be as defined below.
[1] A kit for diagnosing an allergy to a soybean, the kit comprising, as an antigen, at least one of proteins defined below in any of the following (1) to (8):
   (1)
      (1A) Bowman-Birk type proteinase inhibitor D-II or a variant thereof, which is defined below in any of (1A-a) to (1A-e):
         (1A-a) a protein comprising an amino acid sequence with deletion, substitution, insertion or addition of one or several amino acids in SEQ ID NO: 2;
         (1A-b) a protein comprising an amino acid sequence having at least 70% identity to the amino acid sequence of SEQ ID NO: 2;
         (1A-c) a protein comprising an amino acid sequence encoded by a nucleotide sequence with deletion, substitution, insertion or addition of one or several nucleotides in SEQ ID NO: 1;
         (1A-d) a protein comprising an amino acid sequence encoded by a nucleotide sequence having at least 70% identity to the nucleotide sequence of SEQ ID NO: 1; or
         (1A-e) a protein comprising an amino acid sequence encoded by a nucleic acid that hybridizes under stringent conditions with a nucleic acid having a nucleotide sequence complementary to the nucleotide sequence of SEQ ID NO: 1; or
      (1B) a protein comprising at least one amino acid sequence selected from the group consisting of SEQ ID NOs: 17-20;
   (2)
      (2A) Ribulose bisphosphate carboxylase large chain or a variant thereof, which is defined below in any of (2A-a) to (2A-e):
         (2A-a) a protein comprising an amino acid sequence with deletion, substitution, insertion or addition of one or several amino acids in SEQ ID NO: 4;
         (2A-b) a protein comprising an amino acid sequence having at least 70% identity to the amino acid sequence of SEQ ID NO: 4;
         (2A-c) a protein comprising an amino acid sequence encoded by a nucleotide sequence with deletion, substitution, insertion or addition of one or several nucleotides in SEQ ID NO: 3;
         (2A-d) a protein comprising an amino acid sequence encoded by a nucleotide sequence having at least 70% identity to the nucleotide sequence of SEQ ID NO: 3; or
         (2A-e) a protein comprising an amino acid sequence encoded by a nucleic acid that hybridizes under stringent conditions with a nucleic acid having a nucleotide sequence complementary to the nucleotide sequence of SEQ ID NO: 3; or
      (2B) a protein comprising at least one amino acid sequence selected from the group consisting of SEQ ID NOs: 21-28;
   (3)
      (3A) Gamma-glutamyl hydrolase or a variant thereof, which is defined below in any of (3A-a) to (3A-e):
         (3A-a) a protein comprising an amino acid sequence with deletion, substitution, insertion or addition of one or several amino acids in SEQ ID NO: 6;
         (3A-b) a protein comprising an amino acid sequence having at least 70% identity to the amino acid sequence of SEQ ID NO: 6;
         (3A-c) a protein comprising an amino acid sequence encoded by a nucleotide sequence with deletion, substitution, insertion or addition of one or several nucleotides in SEQ ID NO: 5;
         (3A-d) a protein comprising an amino acid sequence encoded by a nucleotide sequence having at least 70% identity to the nucleotide sequence of SEQ ID NO: 5; or
         (3A-e) a protein comprising an amino acid sequence encoded by a nucleic acid that hybridizes under stringent conditions with a nucleic acid having a nucleotide sequence complementary to the nucleotide sequence of SEQ ID NO: 5; or
      (3B) a protein comprising an amino acid sequence of SEQ ID NO: 29 or 30;
   (4)
      (4A) Guanine nucleotide-binding protein subunit beta-like protein or a variant thereof, which is defined below in any of (4A-a) to (4A-e):
         (4A-a) a protein comprising an amino acid sequence with deletion, substitution, insertion or addition of one or several amino acids in SEQ ID NO: 8;
         (4A-b) a protein comprising an amino acid sequence having at least 70% identity to the amino acid sequence of SEQ ID NO: 8;
         (4A-c) a protein comprising an amino acid sequence encoded by a nucleotide sequence with deletion, substitution, insertion or addition of one or several nucleotides in SEQ ID NO: 7;
         (4A-d) a protein comprising an amino acid sequence encoded by a nucleotide sequence having at least 70% identity to the nucleotide sequence of SEQ ID NO: 7; or
         (4A-e) a protein comprising an amino acid sequence encoded by a nucleic acid that hybridizes under stringent conditions with a nucleic acid having a nucleotide sequence complementary to the nucleotide sequence of SEQ ID NO: 7; or
      (4B) a protein comprising an amino acid sequence of SEQ ID NO: 31 or 32;
   (5)
      (5A) Protein SLE3 or a variant thereof, which is defined below in any of (5A-a) to (5A-e):
         (5A-a) a protein comprising an amino acid sequence with deletion, substitution, insertion or addition of one or several amino acids in SEQ ID NO: 10;
         (5A-b) a protein comprising an amino acid sequence having at least 70% identity to the amino acid sequence of SEQ ID NO: 10;
         (5A-c) a protein comprising an amino acid sequence encoded by a nucleotide sequence with deletion, substitution, insertion or addition of one or several nucleotides in SEQ ID NO: 9;
         (5A-d) a protein comprising an amino acid sequence encoded by a nucleotide sequence having at least 70% identity to the nucleotide sequence of SEQ ID NO: 9; or
         (5A-e) a protein comprising an amino acid sequence encoded by a nucleic acid that hybridizes under stringent conditions with a nucleic acid having a nucleotide sequence complementary to the nucleotide sequence of SEQ ID NO: 9; or
      (5B) a protein comprising an amino acid sequence of SEQ ID NO: 33 or 34;
   (6)
      (6A) Seed biotin-containing protein SBP65 or a variant thereof, which is defined below in any of (6A-a) to (6A-e):
         (6A-a) a protein comprising an amino acid sequence with deletion, substitution, insertion or addition of one or several amino acids in SEQ ID NO: 12;
         (6A-b) a protein comprising an amino acid sequence having at least 70% identity to the amino acid sequence of SEQ ID NO: 12;
         (6A-c) a protein comprising an amino acid sequence encoded by a nucleotide sequence with deletion, substitution, insertion or addition of one or several nucleotides in SEQ ID NO: 11;
         (6A-d) a protein comprising an amino acid sequence encoded by a nucleotide sequence having at least 70% identity to the nucleotide sequence of SEQ ID NO: 11; or
         (6A-e) a protein comprising an amino acid sequence encoded by a nucleic acid that hybridizes under stringent conditions with a nucleic acid having a nucleotide sequence complementary to the nucleotide sequence of SEQ ID NO: 11; or
      (6B) a protein comprising at least one amino acid sequence selected from the group consisting of SEQ ID NOs: 35-53;
   (7)
      (7A) Ferritin-2 or a variant thereof, which is defined below in any of (7A-a) to (7A-e):
         (7A-a) a protein comprising an amino acid sequence with deletion, substitution, insertion or addition of one or several amino acids in SEQ ID NO: 14;
         (7A-b) a protein comprising an amino acid sequence having at least 70% identity to the amino acid sequence of SEQ ID NO: 14;
         (7A-c) a protein comprising an amino acid sequence encoded by a nucleotide sequence with deletion, substitution, insertion or addition of one or several nucleotides in SEQ ID NO: 13;
         (7A-d) a protein comprising an amino acid sequence encoded by a nucleotide sequence having at least 70% identity to the nucleotide sequence of SEQ ID NO: 13; or
         (7A-e) a protein comprising an amino acid sequence encoded by a nucleic acid that hybridizes under stringent conditions with a nucleic acid having a nucleotide sequence complementary to the nucleotide sequence of SEQ ID NO: 13; or
      (7B) a protein comprising at least one amino acid sequence selected from the group consisting of SEQ ID NOs: 54-61;
   (8)
      (8A) Cell division cycle protein 48 homolog or a variant thereof, which is defined below in any of (8A-a) to (8A-e):
         (8A-a) a protein comprising an amino acid sequence with deletion, substitution, insertion or addition of one or several amino acids in SEQ ID NO: 16;
         (8A-b) a protein comprising an amino acid sequence having at least 70% identity to the amino acid sequence of SEQ ID NO: 16;
         (8A-c) a protein comprising an amino acid sequence encoded by a nucleotide sequence with deletion, substitution, insertion or addition of one or several nucleotides in SEQ ID NO: 15;
         (8A-d) a protein comprising an amino acid sequence encoded by a nucleotide sequence having at least 70% identity to the nucleotide sequence of SEQ ID NO: 15; or
         (8A-e) a protein comprising an amino acid sequence encoded by a nucleic acid that hybridizes under stringent conditions with a nucleic acid having a nucleotide sequence complementary to the nucleotide sequence of SEQ ID NO: 15; or
      (8B) a protein comprising at least one amino acid sequence selected from the group consisting of SEQ ID NOs: 62-68.
[2] A composition for diagnosing an allergy to a soybean, comprising, as an antigen, at least one of proteins as defined above in any of (1) to (8) of [1].
[3] A method for providing an indicator for diagnosing an allergy to a soybean in a subject, the method comprising the steps of:
   (i) contacting a sample obtained from the subject with an antigen, wherein the sample is a solution comprising an Ig antibody;
   (ii) detecting binding between the IgE antibody present in the sample from the subject and the antigen; and
   (iii) when the binding between the IgE antibody in the subject and the antigen is detected, an indicator of the fact that the subject is allergic to a soybean is provided;
   wherein the antigen is at least one of proteins as defined above in any of (1) to (8) of [1].
[4] A pharmaceutical composition comprising at least one of proteins as defined above in any of (1) to (8) of [1].
[5] The pharmaceutical composition as set forth in [4], wherein the pharmaceutical composition is intended for the treatment of an allergy to a soybean.
[6] A soybean or processed product of soybean, in which an antigen is eliminated or reduced, wherein antigen is at least one of proteins as defined above in any of (1) to (8) of [1].
[7] A tester for determining the presence or absence of a soybean antigen in an object of interest, comprising an antibody that binds to at least one of proteins as defined above in any of (1) to (8) of [1].
[8] A soybean-derived antigen which is at least one of proteins as defined above in any of (1) to (8) of [1] and is causative of an allergy to soybean.
[9] A kit for diagnosing an allergy to a soybean, the kit comprising, as an antigen, at least one of proteins defined below in any one of (1α) to (55α):
   (1α)
      (1αA1) endoplasmin homolog isoform X2 or a variant thereof, which is defined below in any of (1αA1-a) to (1αA1-e):
         (1αA1-a) a protein comprising an amino acid sequence with deletion, substitution, insertion or addition of one or several amino acids in SEQ ID NO: 70;
         (1αA1-b) a protein comprising an amino acid sequence having at least 70% identity to the amino acid sequence of SEQ ID NO: 70;
         (1oA1-c) a protein comprising an amino acid sequence encoded by a nucleotide sequence with deletion, substitution, insertion or addition of one or several nucleotides in SEQ ID NO: 69;
         (1αA1-d) a protein comprising an amino acid sequence encoded by a nucleotide sequence having at least 70% identity to the nucleotide sequence of SEQ ID NO: 69; or
         (1αA1-e) a protein comprising an amino acid sequence encoded by a nucleic acid that hybridizes under stringent conditions with a nucleic acid having a nucleotide sequence complementary to the nucleotide sequence of SEQ ID NO: 69;
      (1αB1) a protein comprising at least one amino acid sequence selected from the group consisting of SEQ ID NOs: 71-87,
      (1αA2) endoplasmin homolog isoform X2 or a variant thereof, which is defined below in any of (1αA2-a) to (1αA2-e):
         (1αA2-a) a protein comprising an amino acid sequence with deletion, substitution, insertion or addition of one or several amino acids in SEQ ID NO: 89;
         (1αA2-b) a protein comprising an amino acid sequence having at least 70% identity to the amino acid sequence of SEQ ID NO: 89;
         (1αA2-c) a protein comprising an amino acid sequence encoded by a nucleotide sequence with deletion, substitution, insertion or addition of one or several nucleotides in SEQ ID NO: 88;
         (1αA2-d) a protein comprising an amino acid sequence encoded by a nucleotide sequence having at least 70% identity to the nucleotide sequence of SEQ ID NO: 88; or
         (1αA2-e) a protein comprising an amino acid sequence encoded by a nucleic acid that hybridizes under stringent conditions with a nucleic acid having a nucleotide sequence complementary to the nucleotide sequence of SEQ ID NO: 88; or
      (1αB2) a protein comprising at least one amino acid sequence selected from the group consisting of SEQ ID NOs: 90-104;
   (2α)
      (2αA1) heat shock cognate protein 80 or a variant thereof, which is defined below in any of (2αA1-a) to (2αA1-e):
         (2αA1-a) a protein comprising an amino acid sequence with deletion, substitution, insertion or addition of one or several amino acids in SEQ ID NO: 106;
         (2αA1-b) a protein comprising an amino acid sequence having at least 70% identity to the amino acid sequence of SEQ ID NO: 106;
         (2αA1-c) a protein comprising an amino acid sequence encoded by a nucleotide sequence with deletion, substitution, insertion or addition of one or several nucleotides in SEQ ID NO: 105;
         (2αA1-d) a protein comprising an amino acid sequence encoded by a nucleotide sequence having at least 70% identity to the nucleotide sequence of SEQ ID NO: 105; or
         (2αA1-e) a protein comprising an amino acid sequence encoded by a nucleic acid that hybridizes under stringent conditions with a nucleic acid having a nucleotide sequence complementary to the nucleotide sequence of SEQ ID NO: 105; or
      (2αB1) a protein comprising at least one amino acid sequence selected from the group consisting of SEQ ID NOs: 107-120;
      (2αA2) heat shock protein 90-1 or a variant thereof, which is defined below in any of (2αA2-a) to (2αA2-e):
         (2αA2-a) a protein comprising an amino acid sequence with deletion, substitution, insertion or addition of one or several amino acids in SEQ ID NO: 122;
         (2αA2-b) a protein comprising an amino acid sequence having at least 70% identity to the amino acid sequence of SEQ ID NO: 122;
         (2αA2-c) a protein comprising an amino acid sequence encoded by a nucleotide sequence with deletion, substitution, insertion or addition of one or several nucleotides in SEQ ID NO: 121;
         (2αA2-d) a protein comprising an amino acid sequence encoded by a nucleotide sequence having at least 70% identity to the nucleotide sequence of SEQ ID NO: 121; or
         (2αA2-e) a protein comprising an amino acid sequence encoded by a nucleic acid that hybridizes under stringent conditions with a nucleic acid having a nucleotide sequence complementary to the nucleotide sequence of SEQ ID NO: 121; or
      (2αB2) a protein comprising at least one amino acid sequence selected from the group consisting of SEQ ID NOs: 123-137;
      (2αA3) hypothetical protein GLYMA_02G302500 or a variant thereof, which is defined below in any of (2αA3-a) to (2αA3-e):
         (2αA3-a) a protein comprising an amino acid sequence with deletion, substitution, insertion or addition of one or several amino acids in SEQ ID NO: 139;
         (2αA3-b) a protein comprising an amino acid sequence having at least 70% identity to the amino acid sequence of SEQ ID NO: 139;
         (2αA3-c) a protein comprising an amino acid sequence encoded by a nucleotide sequence with deletion, substitution, insertion or addition of one or several nucleotides in SEQ ID NO: 138;
         (2αA3-d) a protein comprising an amino acid sequence encoded by a nucleotide sequence having at least 70% identity to the nucleotide sequence of SEQ ID NO: 138; or
         (2αA3-e) a protein comprising an amino acid sequence encoded by a nucleic acid that hybridizes under stringent conditions with a nucleic acid having a nucleotide sequence complementary to the nucleotide sequence of SEQ ID NO: 138; or
      (2αB3) a protein comprising at least one amino acid sequence selected from the group consisting of SEQ ID NOs: 140-153;
   (4α)
      (4αA) embryo-specific urease isoform X1 or a variant thereof, which is defined below in any of (4αA-a) to (4αA-e):
         (4αA-a) a protein comprising an amino acid sequence with deletion, substitution, insertion or addition of one or several amino acids in SEQ ID NO: 155;
         (4αA-b) a protein comprising an amino acid sequence having at least 70% identity to the amino acid sequence of SEQ ID NO: 155;
         (4αA-c) a protein comprising an amino acid sequence encoded by a nucleotide sequence with deletion, substitution, insertion or addition of one or several nucleotides in SEQ ID NO: 154;
         (4αA-d) a protein comprising an amino acid sequence encoded by a nucleotide sequence having at least 70% identity to the nucleotide sequence of SEQ ID NO: 154; or
         (4αA-e) a protein comprising an amino acid sequence encoded by a nucleic acid that hybridizes under stringent conditions with a nucleic acid having a nucleotide sequence complementary to the nucleotide sequence of SEQ ID NO: 154; or
      (4αB) a protein comprising at least one amino acid sequence selected from the group consisting of SEQ ID NOs: 156 and 157;
   (5α)
      (5αA) alpha-1,4 glucan phosphorylase L isozyme, chloroplastic/amyloplastic or a variant thereof, which is defined below in any of (5αA-a) to (5αA-e):
         (5αA-a) a protein comprising an amino acid sequence with deletion, substitution, insertion or addition of one or several amino acids in SEQ ID NO: 159;
         (5αA-b) a protein comprising an amino acid sequence having at least 70% identity to the amino acid sequence of SEQ ID NO: 159;
         (5αA-c) a protein comprising an amino acid sequence encoded by a nucleotide sequence with deletion, substitution, insertion or addition of one or several nucleotides in SEQ ID NO: 158;
         (5αA-d) a protein comprising an amino acid sequence encoded by a nucleotide sequence having at least 70% identity to the nucleotide sequence of SEQ ID NO: 158; or
         (5αA-e) a protein comprising an amino acid sequence encoded by a nucleic acid that hybridizes under stringent conditions with a nucleic acid having a nucleotide sequence complementary to the nucleotide sequence of SEQ ID NO: 158; or
      (5αB) a protein comprising at least one amino acid sequence selected from the group consisting of SEQ ID NOs: 160 and 161;
   (6α)
      (6αA1) aconitate hydratase 1 or a variant thereof, which is defined below in any of (6αA1-a) to (6αA1-e):
         (6αA1-a) a protein comprising an amino acid sequence with deletion, substitution, insertion or addition of one or several amino acids in SEQ ID NO: 163;
         (6αA1-b) a protein comprising an amino acid sequence having at least 70% identity to the amino acid sequence of SEQ ID NO: 163;
         (6αA1-c) a protein comprising an amino acid sequence encoded by a nucleotide sequence with deletion, substitution, insertion or addition of one or several nucleotides in SEQ ID NO: 162;
         (6αA1-d) a protein comprising an amino acid sequence encoded by a nucleotide sequence having at least 70% identity to the nucleotide sequence of SEQ ID NO: 162; or
         (6αA1-e) a protein comprising an amino acid sequence encoded by a nucleic acid that hybridizes under stringent conditions with a nucleic acid having a nucleotide sequence complementary to the nucleotide sequence of SEQ ID NO: 162;
      (6αB1) a protein comprising at least one amino acid sequence selected from the group consisting of SEQ ID NOs: 164-167; or
      (6αA2) aconitate hydratase 1 or a variant thereof, which is defined below in any of (6αA2-a) to (6αA2-e):
         (6αA2-a) a protein comprising an amino acid sequence with deletion, substitution, insertion or addition of one or several amino acids in SEQ ID NO: 169;
         (6αA2-b) a protein comprising an amino acid sequence having at least 70% identity to the amino acid sequence of SEQ ID NO: 169;
         (6αA2-c) a protein comprising an amino acid sequence encoded by a nucleotide sequence with deletion, substitution, insertion or addition of one or several nucleotides in SEQ ID NO: 168;
         (6αA2-d) a protein comprising an amino acid sequence encoded by a nucleotide sequence having at least 70% identity to the nucleotide sequence of SEQ ID NO: 168; or
         (6αA2-e) a protein comprising an amino acid sequence encoded by a nucleic acid that hybridizes under stringent conditions with a nucleic acid having a nucleotide sequence complementary to the nucleotide sequence of SEQ ID NO: 168; or
      (6αB2) a protein comprising at least one amino acid sequence selected from the group consisting of SEQ ID NOs: 170-172;
   (7α)
      (7αA) methionine synthase or a variant thereof, which is defined below in any of (7αA-a) to (7αA-e):
         (7αA-a) a protein comprising an amino acid sequence with deletion, substitution, insertion or addition of one or several amino acids in SEQ ID NO: 174;
         (7αA-b) a protein comprising an amino acid sequence having at least 70% identity to the amino acid sequence of SEQ ID NO: 174;
         (7αA-c) a protein comprising an amino acid sequence encoded by a nucleotide sequence with deletion, substitution, insertion or addition of one or several nucleotides in SEQ ID NO: 173;
         (7αA-d) a protein comprising an amino acid sequence encoded by a nucleotide sequence having at least 70% identity to the nucleotide sequence of SEQ ID NO: 173; or
         (7αA-e) a protein comprising an amino acid sequence encoded by a nucleic acid that hybridizes under stringent conditions with a nucleic acid having a nucleotide sequence complementary to the nucleotide sequence of SEQ ID NO: 173; or
      (7αB) a protein comprising at least one amino acid sequence selected from the group consisting of SEQ ID NOs: 175-187;
   (8α)
      (8αA) transketolase, chloroplastic or a variant thereof, which is defined below in any of (8αA-a) to (8αA-e):
         (8αA-a) a protein comprising an amino acid sequence with deletion, substitution, insertion or addition of one or several amino acids in SEQ ID NO: 189;
         (8αA-b) a protein comprising an amino acid sequence having at least 70% identity to the amino acid sequence of SEQ ID NO: 189;
         (8αA-c) a protein comprising an amino acid sequence encoded by a nucleotide sequence with deletion, substitution, insertion or addition of one or several nucleotides in SEQ ID NO: 188;
         (8αA-d) a protein comprising an amino acid sequence encoded by a nucleotide sequence having at least 70% identity to the nucleotide sequence of SEQ ID NO: 188; or
         (8αA-e) a protein comprising an amino acid sequence encoded by a nucleic acid that hybridizes under stringent conditions with a nucleic acid having a nucleotide sequence complementary to the nucleotide sequence of SEQ ID NO: 188; or
      (8αB) a protein comprising at least one amino acid sequence selected from the group consisting of SEQ ID NOs: 190-199;
   (9α)
      (9αA) lysine-tRNA ligase, cytoplasmic-like isoform X2 or a variant thereof, which is defined below in any of (9αA-a) to (9αA-e):
         (9αA-a) a protein comprising an amino acid sequence with deletion, substitution, insertion or addition of one or several amino acids in SEQ ID NO: 201;
         (9αA-b) a protein comprising an amino acid sequence having at least 70% identity to the amino acid sequence of SEQ ID NO: 201;
         (9αA-c) a protein comprising an amino acid sequence encoded by a nucleotide sequence with deletion, substitution, insertion or addition of one or several nucleotides in SEQ ID NO: 200;
         (9αA-d) a protein comprising an amino acid sequence encoded by a nucleotide sequence having at least 70% identity to the nucleotide sequence of SEQ ID NO: 200; or
         (9αA-e) a protein comprising an amino acid sequence encoded by a nucleic acid that hybridizes under stringent conditions with a nucleic acid having a nucleotide sequence complementary to the nucleotide sequence of SEQ ID NO: 200; or
      (9αB) a protein comprising an amino acid sequence of SEQ ID NO: 202;
   (10α)
      (10αA1) chaperonin CPN60-2, mitochondrial or a variant thereof, which is defined below in any of (10αA1-a) to (10αA1-e):
         (10αA1-a) a protein comprising an amino acid sequence with deletion, substitution, insertion or addition of one or several amino acids in SEQ ID NO: 204;
         (10αA1-b) a protein comprising an amino acid sequence having at least 70% identity to the amino acid sequence of SEQ ID NO: 204;
         (10αA1-c) a protein comprising an amino acid sequence encoded by a nucleotide sequence with deletion, substitution, insertion or addition of one or several nucleotides in SEQ ID NO: 203;
         (10αA1-d) a protein comprising an amino acid sequence encoded by a nucleotide sequence having at least 70% identity to the nucleotide sequence of SEQ ID NO: 203; or
         (10αA1-e) a protein comprising an amino acid sequence encoded by a nucleic acid that hybridizes under stringent conditions with a nucleic acid having a nucleotide sequence complementary to the nucleotide sequence of SEQ ID NO: 203;
      (10αB1) a protein comprising at least one amino acid sequence selected from the group consisting of SEQ ID NOs: 205-218;
      (10αA2) chaperonin CPN60-2, mitochondrial-like isoform X1 or a variant thereof, which is defined below in any of (10αA2-a) to (10αA2-e):
         (10αA2-a) a protein comprising an amino acid sequence with deletion, substitution, insertion or addition of one or several amino acids in SEQ ID NO: 220;
         (10αA2-b) a protein comprising an amino acid sequence having at least 70% identity to the amino acid sequence of SEQ ID NO: 220;
         (10αA2-c) a protein comprising an amino acid sequence encoded by a nucleotide sequence with deletion, substitution, insertion or addition of one or several nucleotides in SEQ ID NO: 219;
         (10αA2-d) a protein comprising an amino acid sequence encoded by a nucleotide sequence having at least 70% identity to the nucleotide sequence of SEQ ID NO: 219; or
         (10αA2-e) a protein comprising an amino acid sequence encoded by a nucleic acid that hybridizes under stringent conditions with a nucleic acid having a nucleotide sequence complementary to the nucleotide sequence of SEQ ID NO: 219; or
      (10αB2) a protein comprising at least one amino acid sequence selected from the group consisting of SEQ ID NOs: 221-231;
   (11α)
      (11αA) polyadenylate-binding protein 8 isoform X3 or a variant thereof, which is defined below in any of (11αA-a) to (11αA-e):
         (11αA-a) a protein comprising an amino acid sequence with deletion, substitution, insertion or addition of one or several amino acids in SEQ ID NO: 233;
         (11αA-b) a protein comprising an amino acid sequence having at least 70% identity to the amino acid sequence of SEQ ID NO: 233;
         (11αA-c) a protein comprising an amino acid sequence encoded by a nucleotide sequence with deletion, substitution, insertion or addition of one or several nucleotides in SEQ ID NO: 232;
         (11αA-d) a protein comprising an amino acid sequence encoded by a nucleotide sequence having at least 70% identity to the nucleotide sequence of SEQ ID NO: 232; or
         (11αA-e) a protein comprising an amino acid sequence encoded by a nucleic acid that hybridizes under stringent conditions with a nucleic acid having a nucleotide sequence complementary to the nucleotide sequence of SEQ ID NO: 232; or
      (11αB) a protein comprising at least one amino acid sequence selected from the group consisting of SEQ ID NOs: 234-237;
   (12α)
      (12αA) pyrophosphate-fructose 6-phosphate 1-phosphotransferase subunit alpha-like or a variant thereof, which is defined below in any of (12αA-a) to (12αA-e):
         (12αA-a) a protein comprising an amino acid sequence with deletion, substitution, insertion or addition of one or several amino acids in SEQ ID NO: 239;
         (12αA-b) a protein comprising an amino acid sequence having at least 70% identity to the amino acid sequence of SEQ ID NO: 239;
         (12αA-c) a protein comprising an amino acid sequence encoded by a nucleotide sequence with deletion, substitution, insertion or addition of one or several nucleotides in SEQ ID NO: 238;
         (12αA-d) a protein comprising an amino acid sequence encoded by a nucleotide sequence having at least 70% identity to the nucleotide sequence of SEQ ID NO: 238; or
         (12αA-e) a protein comprising an amino acid sequence encoded by a nucleic acid that hybridizes under stringent conditions with a nucleic acid having a nucleotide sequence complementary to the nucleotide sequence of SEQ ID NO: 238; or
      (12αB) a protein comprising an amino acid sequence of SEQ ID NO: 240;
   (13α)
      (13αA1) protein disulfide isomerase-like protein precursor or a variant thereof, which is defined below in any of (13αA1-a) to (13αA1-e):
         (13αA1-a) a protein comprising an amino acid sequence with deletion, substitution, insertion or addition of one or several amino acids in SEQ ID NO: 242;
         (13αA1-b) a protein comprising an amino acid sequence having at least 70% identity to the amino acid sequence of SEQ ID NO: 242;
         (13αA1-c) a protein comprising an amino acid sequence encoded by a nucleotide sequence with deletion, substitution, insertion or addition of one or several nucleotides in SEQ ID NO: 241;
         (13αA1-d) a protein comprising an amino acid sequence encoded by a nucleotide sequence having at least 70% identity to the nucleotide sequence of SEQ ID NO: 241; or
         (13αA1-e) a protein comprising an amino acid sequence encoded by a nucleic acid that hybridizes under stringent conditions with a nucleic acid having a nucleotide sequence complementary to the nucleotide sequence of SEQ ID NO: 241;
      (13αB1) a protein comprising at least one amino acid sequence selected from the group consisting of SEQ ID NOs: 243-252;
      (13αA2) protein disulfide-isomerase or a variant thereof, which is defined below in any of (13αA2-a) to (13αA2-e):
         (13αA2-a) a protein comprising an amino acid sequence with deletion, substitution, insertion or addition of one or several amino acids in SEQ ID NO: 254;
         (13αA2-b) a protein comprising an amino acid sequence having at least 70% identity to the amino acid sequence of SEQ ID NO: 254;
         (13αA2-c) a protein comprising an amino acid sequence encoded by a nucleotide sequence with deletion, substitution, insertion or addition of one or several nucleotides in SEQ ID NO: 253;
         (13αA2-d) a protein comprising an amino acid sequence encoded by a nucleotide sequence having at least 70% identity to the nucleotide sequence of SEQ ID NO: 253; or
         (13αA2-e) a protein comprising an amino acid sequence encoded by a nucleic acid that hybridizes under stringent conditions with a nucleic acid having a nucleotide sequence complementary to the nucleotide sequence of SEQ ID NO: 253; or
      (13αB2) a protein comprising at least one amino acid sequence selected from the group consisting of SEQ ID NOs: 255-278;
   (14α)
      (14αA1) V-type proton ATPase subunit B 2 or a variant thereof, which is defined below in any of (14αA1-a) to (14αA1-e):
         (14αA1-a) a protein comprising an amino acid sequence with deletion, substitution, insertion or addition of one or several amino acids in SEQ ID NO: 280;
         (14αA1-b) a protein comprising an amino acid sequence having at least 70% identity to the amino acid sequence of SEQ ID NO: 280;
         (14αA1-c) a protein comprising an amino acid sequence encoded by a nucleotide sequence with deletion, substitution, insertion or addition of one or several nucleotides in SEQ ID NO: 279;
         (14αA1-d) a protein comprising an amino acid sequence encoded by a nucleotide sequence having at least 70% identity to the nucleotide sequence of SEQ ID NO: 279; or
         (14αA1-e) a protein comprising an amino acid sequence encoded by a nucleic acid that hybridizes under stringent conditions with a nucleic acid having a nucleotide sequence complementary to the nucleotide sequence of SEQ ID NO: 279;
      (14αB1) a protein comprising at least one amino acid sequence selected from the group consisting of SEQ ID NOs: 281-284;
      (14αA2) V-type proton ATPase subunit B 2 or a variant thereof, which is defined below in any of (14αA2-a) to (14αA2-e):
         (14αA2-a) a protein comprising an amino acid sequence with deletion, substitution, insertion or addition of one or several amino acids in SEQ ID NO: 286;
         (14αA2-b) a protein comprising an amino acid sequence having at least 70% identity to the amino acid sequence of SEQ ID NO: 286;
         (14αA2-c) a protein comprising an amino acid sequence encoded by a nucleotide sequence with deletion, substitution, insertion or addition of one or several nucleotides in SEQ ID NO: 285;
         (14αA2-d) a protein comprising an amino acid sequence encoded by a nucleotide sequence having at least 70% identity to the nucleotide sequence of SEQ ID NO: 285; or
         (14αA2-e) a protein comprising an amino acid sequence encoded by a nucleic acid that hybridizes under stringent conditions with a nucleic acid having a nucleotide sequence complementary to the nucleotide sequence of SEQ ID NO: 285; or
      (14αB2) a protein comprising at least one amino acid sequence selected from the group consisting of SEQ ID NOs: 287-289;
   (15, 16α)
      (15, 16αA1) UTP-glucose-1-phosphate uridylyltransferase or a variant thereof, which is defined below in any of (15, 16αA1-a) to (15, 16αA1-e):
         (15, 16αA1-a) a protein comprising an amino acid sequence with deletion, substitution, insertion or addition of one or several amino acids in SEQ ID NO: 291;
         (15, 16αA1-b) a protein comprising an amino acid sequence having at least 70% identity to the amino acid sequence of SEQ ID NO: 291;
         (15, 16αA1-c) a protein comprising an amino acid sequence encoded by a nucleotide sequence with deletion, substitution, insertion or addition of one or several nucleotides in SEQ ID NO: 290;
         (15, 16αA1-d) a protein comprising an amino acid sequence encoded by a nucleotide sequence having at least 70% identity to the nucleotide sequence of SEQ ID NO: 290; or
         (15, 16αA1-e) a protein comprising an amino acid sequence encoded by a nucleic acid that hybridizes under stringent conditions with a nucleic acid having a nucleotide sequence complementary to the nucleotide sequence of SEQ ID NO: 290;
      (15, 16αB1) a protein comprising at least one amino acid sequence selected from the group consisting of SEQ ID NOs: 292-304;
      (15, 16αA2) hypothetical protein GLYMA_02G241100 or a variant thereof, which is defined below in any of (15, 16αA2-a) to (15, 16αA2-e):
         (15, 16αA2-a) a protein comprising an amino acid sequence with deletion, substitution, insertion or addition of one or several amino acids in SEQ ID NO: 306;
         (15, 16αA2-b) a protein comprising an amino acid sequence having at least 70% identity to the amino acid sequence of SEQ ID NO: 306;
         (15, 16αA2-c) a protein comprising an amino acid sequence encoded by a nucleotide sequence with deletion, substitution, insertion or addition of one or several nucleotides in SEQ ID NO: 305;
         (15, 16αA2-d) a protein comprising an amino acid sequence encoded by a nucleotide sequence having at least 70% identity to the nucleotide sequence of SEQ ID NO: 305; or
         (15, 16αA2-e) a protein comprising an amino acid sequence encoded by a nucleic acid that hybridizes under stringent conditions with a nucleic acid having a nucleotide sequence complementary to the nucleotide sequence of SEQ ID NO: 305; or
      (15, 16αB2) a protein comprising at least one amino acid sequence selected from the group consisting of SEQ ID NOs: 307-318;
   (17α)
      (17αA1) guanosine nucleotide diphosphate dissociation inhibitor 2 or a variant thereof, which is defined below in any of (17αA1-a) to (17αA1-e):
         (17αA1-a) a protein comprising an amino acid sequence with deletion, substitution, insertion or addition of one or several amino acids in SEQ ID NO: 320;
         (17αA1-b) a protein comprising an amino acid sequence having at least 70% identity to the amino acid sequence of SEQ ID NO: 320;
         (17αA1-c) a protein comprising an amino acid sequence encoded by a nucleotide sequence with deletion, substitution, insertion or addition of one or several nucleotides in SEQ ID NO: 319;
         (17αA1-d) a protein comprising an amino acid sequence encoded by a nucleotide sequence having at least 70% identity to the nucleotide sequence of SEQ ID NO: 319; or
         (17αA1-e) a protein comprising an amino acid sequence encoded by a nucleic acid that hybridizes under stringent conditions with a nucleic acid having a nucleotide sequence complementary to the nucleotide sequence of SEQ ID NO: 319;
      (17αB1) a protein comprising at least one amino acid sequence selected from the group consisting of SEQ ID NOs: 321-325;
      (17αA2) rab GDP dissociation inhibitor alpha-like or a variant thereof, which is defined below in any of (17αA2-a) to (17αA2-e):
         (17αA2-a) a protein comprising an amino acid sequence with deletion, substitution, insertion or addition of one or several amino acids in SEQ ID NO: 327;
         (17αA2-b) a protein comprising an amino acid sequence having at least 70% identity to the amino acid sequence of SEQ ID NO: 327;
         (17αA2-c) a protein comprising an amino acid sequence encoded by a nucleotide sequence with deletion, substitution, insertion or addition of one or several nucleotides in SEQ ID NO: 326;
         (17αA2-d) a protein comprising an amino acid sequence encoded by a nucleotide sequence having at least 70% identity to the nucleotide sequence of SEQ ID NO: 326; or
         (17αA2-e) a protein comprising an amino acid sequence encoded by a nucleic acid that hybridizes under stringent conditions with a nucleic acid having a nucleotide sequence complementary to the nucleotide sequence of SEQ ID NO: 326; or
      (17αB2) a protein comprising at least one amino acid sequence selected from the group consisting of SEQ ID NOs: 328-332;
   (18α)
      (18αA) hypothetical protein GLYMA_10G028300 or a variant thereof, which is defined below in any of (18αA-a) to (18αA-e):
         (18αA-a) a protein comprising an amino acid sequence with deletion, substitution, insertion or addition of one or several amino acids in SEQ ID NO: 334;
         (18αA-b) a protein comprising an amino acid sequence having at least 70% identity to the amino acid sequence of SEQ ID NO: 334;
         (18αA-e) a protein comprising an amino acid sequence encoded by a nucleotide sequence with deletion, substitution, insertion or addition of one or several nucleotides in SEQ ID NO: 333;
         (18αA-d) a protein comprising an amino acid sequence encoded by a nucleotide sequence having at least 70% identity to the nucleotide sequence of SEQ ID NO: 333; or
         (18αA-e) a protein comprising an amino acid sequence encoded by a nucleic acid that hybridizes under stringent conditions with a nucleic acid having a nucleotide sequence complementary to the nucleotide sequence of SEQ ID NO: 333; or
      (18αB) a protein comprising at least one amino acid sequence selected from the group consisting of SEQ ID NOs: 335-339;
   (19α)
      (19αA) sucrose binding protein homolog S-64 or a variant thereof, which is defined below in any of (19αA-a) to (19αA-e):
         (19αA-a) a protein comprising an amino acid sequence with deletion, substitution, insertion or addition of one or several amino acids in SEQ ID NO: 341;
         (19αA-b) a protein comprising an amino acid sequence having at least 70% identity to the amino acid sequence of SEQ ID NO: 341;
         (19αA-c) a protein comprising an amino acid sequence encoded by a nucleotide sequence with deletion, substitution, insertion or addition of one or several nucleotides in SEQ ID NO: 340;
         (19αA-d) a protein comprising an amino acid sequence encoded by a nucleotide sequence having at least 70% identity to the nucleotide sequence of SEQ ID NO: 340; or
         (19αA-e) a protein comprising an amino acid sequence encoded by a nucleic acid that hybridizes under stringent conditions with a nucleic acid having a nucleotide sequence complementary to the nucleotide sequence of SEQ ID NO: 340; or
      (19αB) a protein comprising at least one amino acid sequence selected from the group consisting of SEQ ID NOs: 342-346;
   (25α)
      (25αA1) succinyl-CoA ligase [ADP-forming] subunit beta, mitochondrial or a variant thereof, which is defined below in any of (25αA1-a) to (25αA1-e):
         (25αA1-a) a protein comprising an amino acid sequence with deletion, substitution, insertion or addition of one or several amino acids in SEQ ID NO: 348;
         (25αA1-b) a protein comprising an amino acid sequence having at least 70% identity to the amino acid sequence of SEQ ID NO: 348;
         (25αA1-c) a protein comprising an amino acid sequence encoded by a nucleotide sequence with deletion, substitution, insertion or addition of one or several nucleotides in SEQ ID NO: 347;
         (25αA1-d) a protein comprising an amino acid sequence encoded by a nucleotide sequence having at least 70% identity to the nucleotide sequence of SEQ ID NO: 347; or
         (25αA1-e) a protein comprising an amino acid sequence encoded by a nucleic acid that hybridizes under stringent conditions with a nucleic acid having a nucleotide sequence complementary to the nucleotide sequence of SEQ ID NO: 347;
      (25αB1) a protein comprising at least one amino acid sequence selected from the group consisting of SEQ ID NOs: 349-356;
      (25αA2) succinyl-CoA ligase [ADP-forming] subunit beta, mitochondrial or a variant thereof, which is defined below in any of (25αA2-a) to (25αA2-e):
         (25αA2-a) a protein comprising an amino acid sequence with deletion, substitution, insertion or addition of one or several amino acids in SEQ ID NO: 358;
         (25αA2-b) a protein comprising an amino acid sequence having at least 70% identity to the amino acid sequence of SEQ ID NO: 358;
         (25αA2-c) a protein comprising an amino acid sequence encoded by a nucleotide sequence with deletion, substitution, insertion or addition of one or several nucleotides in SEQ ID NO: 357;
         (25αA2-d) a protein comprising an amino acid sequence encoded by a nucleotide sequence having at least 70% identity to the nucleotide sequence of SEQ ID NO: 357; or
         (25αA2-e) a protein comprising an amino acid sequence encoded by a nucleic acid that hybridizes under stringent conditions with a nucleic acid having a nucleotide sequence complementary to the nucleotide sequence of SEQ ID NO: 357; or
      (25αB2) a protein comprising at least one amino acid sequence selected from the group consisting of SEQ ID NOs: 359-365;
   (26α)
      (26αA1) phosphoglycerate kinase, cytosolic or a variant thereof, which is defined below in any of (26αA1-a) to (26αA1-e):
         (26αA1-a) a protein comprising an amino acid sequence with deletion, substitution, insertion or addition of one or several amino acids in SEQ ID NO: 367;
         (26αA1-b) a protein comprising an amino acid sequence having at least 70% identity to the amino acid sequence of SEQ ID NO: 367;
         (26αA1-c) a protein comprising an amino acid sequence encoded by a nucleotide sequence with deletion, substitution, insertion or addition of one or several nucleotides in SEQ ID NO: 366;
         (26αA1-d) a protein comprising an amino acid sequence encoded by a nucleotide sequence having at least 70% identity to the nucleotide sequence of SEQ ID NO: 366; or
         (26αA1-e) a protein comprising an amino acid sequence encoded by a nucleic acid that hybridizes under stringent conditions with a nucleic acid having a nucleotide sequence complementary to the nucleotide sequence of SEQ ID NO: 366;
      (26αB1) a protein comprising at least one amino acid sequence selected from the group consisting of SEQ ID NOs: 368-382;
      (26αA2) phosphoglycerate kinase, cytosolic or a variant thereof, which is defined below in any of (26αA2-a) to (26αA2-e):
         (26αA2-a) a protein comprising an amino acid sequence with deletion, substitution, insertion or addition of one or several amino acids in SEQ ID NO: 384;
         (26αA2-b) a protein comprising an amino acid sequence having at least 70% identity to the amino acid sequence of SEQ ID NO: 384;
         (26αA2-c) a protein comprising an amino acid sequence encoded by a nucleotide sequence with deletion, substitution, insertion or addition of one or several nucleotides in SEQ ID NO: 383;
         (26αA2-d) a protein comprising an amino acid sequence encoded by a nucleotide sequence having at least 70% identity to the nucleotide sequence of SEQ ID NO: 383; or
         (26αA2-e) a protein comprising an amino acid sequence encoded by a nucleic acid that hybridizes under stringent conditions with a nucleic acid having a nucleotide sequence complementary to the nucleotide sequence of SEQ ID NO: 383;
      (26αB2) a protein comprising at least one amino acid sequence selected from the group consisting of SEQ ID NOs: 385-392;
      (26αA3) phosphoglycerate kinase, cytosolic or a variant thereof, which is defined below in any of (26αA3-a) to (26αA3-e):
         (26αA3-a) a protein comprising an amino acid sequence with deletion, substitution, insertion or addition of one or several amino acids in SEQ ID NO: 394;
         (26αA3-b) a protein comprising an amino acid sequence having at least 70% identity to the amino acid sequence of SEQ ID NO: 394;
         (26αA3-c) a protein comprising an amino acid sequence encoded by a nucleotide sequence with deletion, substitution, insertion or addition of one or several nucleotides in SEQ ID NO: 393;
         (26αA3-d) a protein comprising an amino acid sequence encoded by a nucleotide sequence having at least 70% identity to the nucleotide sequence of SEQ ID NO: 393; or
         (26αA3-e) a protein comprising an amino acid sequence encoded by a nucleic acid that hybridizes under stringent conditions with a nucleic acid having a nucleotide sequence complementary to the nucleotide sequence of SEQ ID NO: 393; or
      (26αB3) a protein comprising at least one amino acid sequence selected from the group consisting of SEQ ID NOs: 395-409;
   (27α)
      (27αA1) alcohol dehydrogenase 1 or a variant thereof, which is defined below in any of (27αA1-a) to (27αA1-e):
         (27αA1-a) a protein comprising an amino acid sequence with deletion, substitution, insertion or addition of one or several amino acids in SEQ ID NO: 411;
         (27αA1-b) a protein comprising an amino acid sequence having at least 70% identity to the amino acid sequence of SEQ ID NO: 411;
         (27αA1-c) a protein comprising an amino acid sequence encoded by a nucleotide sequence with deletion, substitution, insertion or addition of one or several nucleotides in SEQ ID NO: 410;
         (27αA1-d) a protein comprising an amino acid sequence encoded by a nucleotide sequence having at least 70% identity to the nucleotide sequence of SEQ ID NO: 410; or
         (27αA1-e) a protein comprising an amino acid sequence encoded by a nucleic acid that hybridizes under stringent conditions with a nucleic acid having a nucleotide sequence complementary to the nucleotide sequence of SEQ ID NO: 410;
      (27αB1) a protein comprising at least one amino acid sequence selected from the group consisting of SEQ ID NOs: 412-416;
      (27αA2) alcohol dehydrogenase 1 or a variant thereof, which is defined below in any of (27αA2-a) to (27αA2-e):
         (27αA2-a) a protein comprising an amino acid sequence with deletion, substitution, insertion or addition of one or several amino acids in SEQ ID NO: 418;
         (27αA2-b) a protein comprising an amino acid sequence having at least 70% identity to the amino acid sequence of SEQ ID NO: 418;
         (27αA2-c) a protein comprising an amino acid sequence encoded by a nucleotide sequence with deletion, substitution, insertion or addition of one or several nucleotides in SEQ ID NO: 417;
         (27αA2-d) a protein comprising an amino acid sequence encoded by a nucleotide sequence having at least 70% identity to the nucleotide sequence of SEQ ID NO: 417; or
         (27αA2-e) a protein comprising an amino acid sequence encoded by a nucleic acid that hybridizes under stringent conditions with a nucleic acid having a nucleotide sequence complementary to the nucleotide sequence of SEQ ID NO: 417;
      (27αB2) a protein comprising at least one amino acid sequence selected from the group consisting of SEQ ID NOs: 419 and 420;
      (27αA3) alcohol dehydrogenase 1-like or a variant thereof, which is defined below in any of (27αA3-a) to (27αA3-e):
         (27αA3-a) a protein comprising an amino acid sequence with deletion, substitution, insertion or addition of one or several amino acids in SEQ ID NO: 422;
         (27αA3-b) a protein comprising an amino acid sequence having at least 70% identity to the amino acid sequence of SEQ ID NO: 422;
         (27αA3-c) a protein comprising an amino acid sequence encoded by a nucleotide sequence with deletion, substitution, insertion or addition of one or several nucleotides in SEQ ID NO: 421;
         (27αA3-d) a protein comprising an amino acid sequence encoded by a nucleotide sequence having at least 70% identity to the nucleotide sequence of SEQ ID NO: 421; or
         (27αA3-e) a protein comprising an amino acid sequence encoded by a nucleic acid that hybridizes under stringent conditions with a nucleic acid having a nucleotide sequence complementary to the nucleotide sequence of SEQ ID NO: 421; or
      (27αB3) a protein comprising at least one amino acid sequence selected from the group consisting of SEQ ID NOs: 423-427;
   (28α)
      (28αA) 35 kDa seed maturation protein isoform X1 or a variant thereof, which is defined below in any of (28αA-a) to (28αA-e):
         (28αA-a) a protein comprising an amino acid sequence with deletion, substitution, insertion or addition of one or several amino acids in SEQ ID NO: 429;
         (28αA-b) a protein comprising an amino acid sequence having at least 70% identity to the amino acid sequence of SEQ ID NO: 429;
         (28αA-c) a protein comprising an amino acid sequence encoded by a nucleotide sequence with deletion, substitution, insertion or addition of one or several nucleotides in SEQ ID NO: 428;
         (28αA-d) a protein comprising an amino acid sequence encoded by a nucleotide sequence having at least 70% identity to the nucleotide sequence of SEQ ID NO: 428; or
         (28αA-e) a protein comprising an amino acid sequence encoded by a nucleic acid that hybridizes under stringent conditions with a nucleic acid having a nucleotide sequence complementary to the nucleotide sequence of SEQ ID NO: 428; or
         (28αB) a protein comprising at least one amino acid sequence selected from the group consisting of SEQ ID NOs: 430-433;
   (29-33α)
      (29-33αA1) glyceraldehyde-3-phosphate dehydrogenase isoform X1 or a variant thereof, which is defined below in any of (29-33αA1-a) to (29-33αA1-e):
         (29-33αA1-a) a protein comprising an amino acid sequence with deletion, substitution, insertion or addition of one or several amino acids in SEQ ID NO: 435;
         (29-33αA1-b) a protein comprising an amino acid sequence having at least 70% identity to the amino acid sequence of SEQ ID NO: 435;
         (29-33αA1-c) a protein comprising an amino acid sequence encoded by a nucleotide sequence with deletion, substitution, insertion or addition of one or several nucleotides in SEQ ID NO: 434;
         {29-33αA1-d) a protein comprising an amino acid sequence encoded by a nucleotide sequence having at least 70% identity to the nucleotide sequence of SEQ ID NO: 434; or
         (29-33αA1-e) a protein comprising an amino acid sequence encoded by a nucleic acid that hybridizes under stringent conditions with a nucleic acid having a nucleotide sequence complementary to the nucleotide sequence of SEQ ID NO: 434;
      (29-33αB1) a protein comprising at least one amino acid sequence selected from the group consisting of SEQ ID NOs: 436-439;
      (29-33αA2) glyceraldehyde-3-phosphate dehydrogenase, cytosolic or a variant thereof, which is defined below in any of (29-33αA2-a) to (29-33αA2-e):
         (29-33αA2-a) a protein comprising an amino acid sequence with deletion, substitution, insertion or addition of one or several amino acids in SEQ ID NO: 441;
         (29-33αA2-b) a protein comprising an amino acid sequence having at least 70% identity to the amino acid sequence of SEQ ID NO: 441;
         (29-33αA2-c) a protein comprising an amino acid sequence encoded by a nucleotide sequence with deletion, substitution, insertion or addition of one or several nucleotides in SEQ ID NO: 440;
         (29-33αA2-d) a protein comprising an amino acid sequence encoded by a nucleotide sequence having at least 70% identity to the nucleotide sequence of SEQ ID NO: 440; or
         (29-33αA2-e) a protein comprising an amino acid sequence encoded by a nucleic acid that hybridizes under stringent conditions with a nucleic acid having a nucleotide sequence complementary to the nucleotide sequence of SEQ ID NO: 440;
      (29-33αB2) a protein comprising at least one amino acid sequence selected from the group consisting of SEQ ID NOs: 442-445;
      (29-33αA3) uncharacterized protein LOG100782924 or a variant thereof, which is defined below in any of (29-33αA3-a) to (29-33αA3-e):
         (29-33αA3-a) a protein comprising an amino acid sequence with deletion, substitution, insertion or addition of one or several amino acids in SEQ ID NO: 447;
         (29-33αA3-b) a protein comprising an amino acid sequence having at least 70% identity to the amino acid sequence of SEQ ID NO: 447;
         (29-33αA3-c) a protein comprising an amino acid sequence encoded by a nucleotide sequence with deletion, substitution, insertion or addition of one or several nucleotides in SEQ ID NO: 446;
         (29-33αA3-d) a protein comprising an amino acid sequence encoded by a nucleotide sequence having at least 70% identity to the nucleotide sequence of SEQ ID NO: 446; or
         (29-33αA3-e) a protein comprising an amino acid sequence encoded by a nucleic acid that hybridizes under stringent conditions with a nucleic acid having a nucleotide sequence complementary to the nucleotide sequence of SEQ ID NO: 446; or
         (29-33αB3) a protein comprising at least one amino acid sequence selected from the group consisting of SEQ ID NOs: 448-451,
   (34α)
      (34αA1) bifunctional UDP-glucose 4-epimerase and UDP-xylose 4-epimerase 1-like or a variant thereof, which is defined below in any of (34αA1-a) to (34αA1-e):
         (34αA1-a) a protein comprising an amino acid sequence with deletion, substitution, insertion or addition of one or several amino acids in SEQ ID NO: 453;
         (34αA1-b) a protein comprising an amino acid sequence having at least 70% identity to the amino acid sequence of SEQ ID NO: 453;
         (34αA1-c) a protein comprising an amino acid sequence encoded by a nucleotide sequence with deletion, substitution, insertion or addition of one or several nucleotides in SEQ ID NO: 452;
         (34αA1-d) a protein comprising an amino acid sequence encoded by a nucleotide sequence having at least 70% identity to the nucleotide sequence of SEQ ID NO: 452; or
         (34αA1-e) a protein comprising an amino acid sequence encoded by a nucleic acid that hybridizes under stringent conditions with a nucleic acid having a nucleotide sequence complementary to the nucleotide sequence of SEQ ID NO: 452;
      (34αB1) a protein comprising an amino acid sequence of SEQ ID NO: 454;
      (34αA2) uncharacterized protein LOC100803483 or a variant thereof, which is defined below in any of (34αA2-a) to (34αA2-e):
         (34αA2-a) a protein comprising an amino acid sequence with deletion, substitution, insertion or addition of one or several amino acids in SEQ ID NO: 456;
         (34αA2-b) a protein comprising an amino acid sequence having at least 70% identity to the amino acid sequence of SEQ ID NO: 456;
         (34αA2-c) a protein comprising an amino acid sequence encoded by a nucleotide sequence with deletion, substitution, insertion or addition of one or several nucleotides in SEQ ID NO: 455;
         (34αA2-d) a protein comprising an amino acid sequence encoded by a nucleotide sequence having at least 70% identity to the nucleotide sequence of SEQ ID NO: 455; or
         (34αA2-e) a protein comprising an amino acid sequence encoded by a nucleic acid that hybridizes under stringent conditions with a nucleic acid having a nucleotide sequence complementary to the nucleotide sequence of SEQ ID NO: 455; or
      (34αB2) a protein comprising at least one amino acid sequence selected from the group consisting of SEQ ID NOs: 457 and 458;
   (35α)
      (35αA1) elongation factor 1-alpha or a variant thereof, which is defined below in any of (35αA1-a) to (35αA1-e):
         (35αA1-a) a protein comprising an amino acid sequence with deletion, substitution, insertion or addition of one or several amino acids in SEQ ID NO: 460;
         (35αA1-b) a protein comprising an amino acid sequence having at least 70% identity to the amino acid sequence of SEQ ID NO: 460;
         {35αA1-c) a protein comprising an amino acid sequence encoded by a nucleotide sequence with deletion, substitution, insertion or addition of one or several nucleotides in SEQ ID NO: 459;
         (35αA1-d) a protein comprising an amino acid sequence encoded by a nucleotide sequence having at least 70% identity to the nucleotide sequence of SEQ ID NO: 459; or
         (35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
      (35αB1) a protein comprising at least one amino acid sequence selected from the group consisting of SEQ ID NOs: 461-466;
      (35αA2) elongation factor 1-alpha or a variant thereof, which is defined below in any of (35αA2-a) to (35αA2-e):
         (35αA2-a) a protein comprising an amino acid sequence with deletion, substitution, insertion or addition of one or several amino acids in SEQ ID NO: 468;
         (35αA2-b) a protein comprising an amino acid sequence having at least 70% identity to the amino acid sequence of SEQ ID NO: 468;
         (35αA2-c) a protein comprising an amino acid sequence encoded by a nucleotide sequence with deletion, substitution, insertion or addition of one or several nucleotides in SEQ ID NO: 467;
         (35αA2-d) a protein comprising an amino acid sequence encoded by a nucleotide sequence having at least 70% identity to the nucleotide sequence of SEQ ID NO: 467; or
         (35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
      (35αB2) a protein comprising at least one amino acid sequence selected from the group consisting of SEQ ID NOs: 469-471;
      (35αA3) elongation factor-1A isoform X1 or a variant thereof, which is defined below in any of (35αA3-a) to (35αA3-e):
         (35αA3-a) a protein comprising an amino acid sequence with deletion, substitution, insertion or addition of one or several amino acids in SEQ ID NO: 473;
         (35αA3-b) a protein comprising an amino acid sequence having at least 70% identity to the amino acid sequence of SEQ ID NO: 473;
         (35αA3-c) a protein comprising an amino acid sequence encoded by a nucleotide sequence with deletion, substitution, insertion or addition of one or several nucleotides in SEQ ID NO: 472;
         (35αA3-d) a protein comprising an amino acid sequence encoded by a nucleotide sequence having at least 70% identity to the nucleotide sequence of SEQ ID NO: 472; or
         (35αA3-e) a protein comprising an amino acid sequence encoded by a nucleic acid that hybridizes under stringent conditions with a nucleic acid having a nucleotide sequence complementary to the nucleotide sequence of SEQ ID NO: 472; or
      (35αB3) a protein comprising at least one amino acid sequence selected from the group consisting of SEQ ID NOs: 474-477;
   (36, 37α)
      (36, 37αA1) seed maturation protein PM34 or a variant thereof, which is defined below in any of (36, 37αA1-a) to (36, 37αA1-e):
         (36, 37αA1-a) a protein comprising an amino acid sequence with deletion, substitution, insertion or addition of one or several amino acids in SEQ ID NO: 479;
         (36, 37αA1-b) a protein comprising an amino acid sequence having at least 70% identity to the amino acid sequence of SEQ ID NO: 479;
         (36, 37αA1-c) a protein comprising an amino acid sequence encoded by a nucleotide sequence with deletion, substitution, insertion or addition of one or several nucleotides in SEQ ID NO: 478;
         (36, 37αA1-d) a protein comprising an amino acid sequence encoded by a nucleotide sequence having at least 70% identity to the nucleotide sequence of SEQ ID NO: 478; or
         (36, 37αA1-e) a protein comprising an amino acid sequence encoded by a nucleic acid that hybridizes under stringent conditions with a nucleic acid having a nucleotide sequence complementary to the nucleotide sequence of SEQ ID NO: 478;
      (36, 37αB1) a protein comprising at least one amino acid sequence selected from the group consisting of SEQ ID NOs: 480-483;
      (36, 37αA2) uncharacterized protein LOC100809384 or a variant thereof, which is defined below in any of (36, 37αA2-a) to (36, 37αA2-e):
         (36, 37αA2-a) a protein comprising an amino acid sequence with deletion, substitution, insertion or addition of one or several amino acids in SEQ ID NO: 485;
         (36, 37αA2-b) a protein comprising an amino acid sequence having at least 70% identity to the amino acid sequence of SEQ ID NO: 485;
         (36, 37αA2-c) a protein comprising an amino acid sequence encoded by a nucleotide sequence with deletion, substitution, insertion or addition of one or several nucleotides in SEQ ID NO: 484;
         (36, 37αA2-d) a protein comprising an amino acid sequence encoded by a nucleotide sequence having at least 70% identity to the nucleotide sequence of SEQ ID NO: 484; or
         (36, 37αA2-e) a protein comprising an amino acid sequence encoded by a nucleic acid that hybridizes under stringent conditions with a nucleic acid having a nucleotide sequence complementary to the nucleotide sequence of SEQ ID NO: 484;
      (36, 37αB2) a protein comprising at least one amino acid sequence selected from the group consisting of SEQ ID NOs: 486 and 487;
      (36, 37αA3) hypothetical protein GLYMA_10G155300 or a variant thereof, which is defined below in any of (36, 37αA3-a) to (36, 37αA3-e):
         (36, 37αA3-a) a protein comprising an amino acid sequence with deletion, substitution, insertion or addition of one or several amino acids in SEQ ID NO: 489;
         (36, 37αA3-b) a protein comprising an amino acid sequence having at least 70% identity to the amino acid sequence of SEQ ID NO: 489;
         (36, 37αA3-c) a protein comprising an amino acid sequence encoded by a nucleotide sequence with deletion, substitution, insertion or addition of one or several nucleotides in SEQ ID NO: 488;
         (36, 37αA3-d) a protein comprising an amino acid sequence encoded by a nucleotide sequence having at least 70% identity to the nucleotide sequence of SEQ ID NO: 488; or
         (36, 37αA3-e) a protein comprising an amino acid sequence encoded by a nucleic acid that hybridizes under stringent conditions with a nucleic acid having a nucleotide sequence complementary to the nucleotide sequence of SEQ ID NO: 488; or
      (36, 37αB3) a protein comprising at least one amino acid sequence selected from the group consisting of SEQ ID NOs: 490-493;
   (38α)
      (38αA) seed biotin-containing protein SBP65-like isoform X1 or a variant thereof, which is defined below in any of (38αA-a) to (38αA-e):
         (38αA-a) a protein comprising an amino acid sequence with deletion, substitution, insertion or addition of one or several amino acids in SEQ ID NO: 495;
         (38αA-b) a protein comprising an amino acid sequence having at least 70% identity to the amino acid sequence of SEQ ID NO: 495;
         (38αA-c) a protein comprising an amino acid sequence encoded by a nucleotide sequence with deletion, substitution, insertion or addition of one or several nucleotides in SEQ ID NO: 494;
         (38αA-d) a protein comprising an amino acid sequence encoded by a nucleotide sequence having at least 70% identity to the nucleotide sequence of SEQ ID NO: 494; or
         (38αA-e) a protein comprising an amino acid sequence encoded by a nucleic acid that hybridizes under stringent conditions with a nucleic acid having a nucleotide sequence complementary to the nucleotide sequence of SEQ ID NO: 494; or
      (38αB) a protein comprising at least one amino acid sequence selected from the group consisting of SEQ ID NOs: 496-513;
   (39α)
      (39αA) ATP synthase subunit O, mitochondrial-like or a variant thereof, which is defined below in any of (39αA-a) to (39αA-e):
         (39αA-a) a protein comprising an amino acid sequence with deletion, substitution, insertion or addition of one or several amino acids in SEQ ID NO: 515;
         (39αA-b) a protein comprising an amino acid sequence having at least 70% identity to the amino acid sequence of SEQ ID NO: 515;
         (39αA-c) a protein comprising an amino acid sequence encoded by a nucleotide sequence with deletion, substitution, insertion or addition of one or several nucleotides in SEQ ID NO: 514;
         (39αA-d) a protein comprising an amino acid sequence encoded by a nucleotide sequence having at least 70% identity to the nucleotide sequence of SEQ ID NO: 514; or
         (39αA-e) a protein comprising an amino acid sequence encoded by a nucleic acid that hybridizes under stringent conditions with a nucleic acid having a nucleotide sequence complementary to the nucleotide sequence of SEQ ID NO: 514; or
      (39αB) a protein comprising an amino acid sequence of SEQ ID NO: 516;
   (40, 56α)
      (40, 56αA) P24 oleosin isoform A or a variant thereof, which is defined below in any of (40, 56αA-a) to (40, 56αA-e):
         (40, 56αA-a) a protein comprising an amino acid sequence with deletion, substitution, insertion or addition of one or several amino acids in SEQ ID NO: 518;
         (40, 56αA-b) a protein comprising an amino acid sequence having at least 70% identity to the amino acid sequence of SEQ ID NO: 518;
         (40, 56αA-c) a protein comprising an amino acid sequence encoded by a nucleotide sequence with deletion, substitution, insertion or addition of one or several nucleotides in SEQ ID NO: 517;
         (40, 56αA-d) a protein comprising an amino acid sequence encoded by a nucleotide sequence having at least 70% identity to the nucleotide sequence of SEQ ID NO: 517; or
         (40, 56αA-e) a protein comprising an amino acid sequence encoded by a nucleic acid that hybridizes under stringent conditions with a nucleic acid having a nucleotide sequence complementary to the nucleotide sequence of SEQ ID NO: 517; or
      (40, 56αB) a protein comprising at least one amino acid sequence selected from the group consisting of SEQ ID NOs: 519 and 520;
   (41α)
      (41αA) uncharacterized protein At3g49720-like isoform X2 or a variant thereof, which is defined below in any of (41αA-a) to (41αA-e):
         (41αA-a) a protein comprising an amino acid sequence with deletion, substitution, insertion or addition of one or several amino acids in SEQ ID NO: 522;
         (41αA-b) a protein comprising an amino acid sequence having at least 70% identity to the amino acid sequence of SEQ ID NO: 522;
         (41αA-c) a protein comprising an amino acid sequence encoded by a nucleotide sequence with deletion, substitution, insertion or addition of one or several nucleotides in SEQ ID NO: 521;
         (41αA-d) a protein comprising an amino acid sequence encoded by a nucleotide sequence having at least 70% identity to the nucleotide sequence of SEQ ID NO: 521; or
         (41αA-e) a protein comprising an amino acid sequence encoded by a nucleic acid that hybridizes under stringent conditions with a nucleic acid having a nucleotide sequence complementary to the nucleotide sequence of SEQ ID NO: 521; or
      (41αB) a protein comprising an amino acid sequence of SEQ ID NO: 523;
   (42, 43α)
      (42, 43αA1) superoxide dismutase [Mn], mitochondrial or a variant thereof, which is defined below in any of (42, 43αA1-a) to (42, 43αA1-e):
         (42, 43αA1-a) a protein comprising an amino acid sequence with deletion, substitution, insertion or addition of one or several amino acids in SEQ ID NO: 525;
         (42, 43αA1-b) a protein comprising an amino acid sequence having at least 70% identity to the amino acid sequence of SEQ ID NO: 525;
         (42, 43αA1-c) a protein comprising an amino acid sequence encoded by a nucleotide sequence with deletion, substitution, insertion or addition of one or several nucleotides in SEQ ID NO: 524;
         (42, 43αA1-d) a protein comprising an amino acid sequence encoded by a nucleotide sequence having at least 70% identity to the nucleotide sequence of SEQ ID NO: 524; or
         (42, 43αA1-e) a protein comprising an amino acid sequence encoded by a nucleic acid that hybridizes under stringent conditions with a nucleic acid having a nucleotide sequence complementary to the nucleotide sequence of SEQ ID NO: 524;
      (42, 43αB1) a protein comprising at least one amino acid sequence selected from the group consisting of SEQ ID NOs: 526-531;
      (42, 43αA2) hypothetical protein GLYMA_04G221300 or a variant thereof, which is defined below in any of (42, 43αA2-a) to (42, 43αA2-e):
         (42, 43αA2-a) a protein comprising an amino acid sequence with deletion, substitution, insertion or addition of one or several amino acids in SEQ ID NO: 533;
         (42, 43αA2-b) a protein comprising an amino acid sequence having at least 70% identity to the amino acid sequence of SEQ ID NO: 533;
         (42, 43αA2-c) a protein comprising an amino acid sequence encoded by a nucleotide sequence with deletion, substitution, insertion or addition of one or several nucleotides in SEQ ID NO: 532;
         (42, 43αA2-d) a protein comprising an amino acid sequence encoded by a nucleotide sequence having at least 70% identity to the nucleotide sequence of SEQ ID NO: 532; or
         (42, 43αA2-e) a protein comprising an amino acid sequence encoded by a nucleic acid that hybridizes under stringent conditions with a nucleic acid having a nucleotide sequence complementary to the nucleotide sequence of SEQ ID NO: 532; or
      (42, 43αB2) a protein comprising at least one amino acid sequence selected from the group consisting of SEQ ID NOs: 534-539;
   (45α)
      (45αA1) uncharacterized protein LOC100499771 or a variant thereof, which is defined below in any of (45αA1-a) to (45αA1-e):
         (45αA1-a) a protein comprising an amino acid sequence with deletion, substitution, insertion or addition of one or several amino acids in SEQ ID NO: 541;
         (45αA1-b) a protein comprising an amino acid sequence having at least 70% identity to the amino acid sequence of SEQ ID NO: 541;
         (45αA1-c) a protein comprising an amino acid sequence encoded by a nucleotide sequence with deletion, substitution, insertion or addition of one or several nucleotides in SEQ ID NO: 540;
         (45αA1-d) a protein comprising an amino acid sequence encoded by a nucleotide sequence having at least 70% identity to the nucleotide sequence of SEQ ID NO: 540; or
         (45αA1-e) a protein comprising an amino acid sequence encoded by a nucleic acid that hybridizes under stringent conditions with a nucleic acid having a nucleotide sequence complementary to the nucleotide sequence of SEQ ID NO: 540;
      (45αB1) a protein comprising at least one amino acid sequence selected from the group consisting of SEQ ID NOs: 542-545;
      (45αA2) hypothetical protein GLYMA_09G192800 or a variant thereof, which is defined below in any of (45αA2-a) to (45αA2-e):
         (45αA2-a) a protein comprising an amino acid sequence with deletion, substitution, insertion or addition of one or several amino acids in SEQ ID NO: 547;
         (45αA2-b) a protein comprising an amino acid sequence having at least 70% identity to the amino acid sequence of SEQ ID NO: 547;
         (45αA2-c) a protein comprising an amino acid sequence encoded by a nucleotide sequence with deletion, substitution, insertion or addition of one or several nucleotides in SEQ ID NO: 546;
         (45αA2-d) a protein comprising an amino acid sequence encoded by a nucleotide sequence having at least 70% identity to the nucleotide sequence of SEQ ID NO: 546; or
         (45αA2-e) a protein comprising an amino acid sequence encoded by a nucleic acid that hybridizes under stringent conditions with a nucleic acid having a nucleotide sequence complementary to the nucleotide sequence of SEQ ID NO: 546; or
      (45αB2) a protein comprising at least one amino acid sequence selected from the group consisting of SEQ ID NOs: 548-551;
   (46α)
      (46αA) seed maturation protein PM22 or a variant thereof, which is defined below in any of (46αA-a) to (46αA-e):
         (46αA-a) a protein comprising an amino acid sequence with deletion, substitution, insertion or addition of one or several amino acids in SEQ ID NO: 553;
         (46αA-b) a protein comprising an amino acid sequence having at least 70% identity to the amino acid sequence of SEQ ID NO: 553;
         (46αA-c) a protein comprising an amino acid sequence encoded by a nucleotide sequence with deletion, substitution, insertion or addition of one or several nucleotides in SEQ ID NO: 552;
         (46αA-d) a protein comprising an amino acid sequence encoded by a nucleotide sequence having at least 70% identity to the nucleotide sequence of SEQ ID NO: 552; or
         (46αA-e) a protein comprising an amino acid sequence encoded by a nucleic acid that hybridizes under stringent conditions with a nucleic acid having a nucleotide sequence complementary to the nucleotide sequence of SEQ ID NO: 552; or
      (46αB) a protein comprising an amino acid sequence of SEQ ID NO: 554;
   (47α)
      (47αA1) eukaryotic translation initiation factor 5A-2 or a variant thereof, which is defined below in any of (47αA1-a) to (47αA1-e):
         (47αA1-a) a protein comprising an amino acid sequence with deletion, substitution, insertion or addition of one or several amino acids in SEQ ID NO: 556;
         (47αA1-b) a protein comprising an amino acid sequence having at least 70% identity to the amino acid sequence of SEQ ID NO: 556;
         (47αA1-c) a protein comprising an amino acid sequence encoded by a nucleotide sequence with deletion, substitution, insertion or addition of one or several nucleotides in SEQ ID NO: 555;
         (47αA1-d) a protein comprising an amino acid sequence encoded by a nucleotide sequence having at least 70% identity to the nucleotide sequence of SEQ ID NO: 555; or
         (47αA1-e) a protein comprising an amino acid sequence encoded by a nucleic acid that hybridizes under stringent conditions with a nucleic acid having a nucleotide sequence complementary to the nucleotide sequence of SEQ ID NO: 555;
      (47αB1) a protein comprising at least one amino acid sequence selected from the group consisting of SEQ ID NOs: 557 and 558;
      (47αA2) uncharacterized protein LOC100305510 or a variant thereof, which is defined below in any of (47αA2-a) to (47αA2-e):
         (47αA2-a) a protein comprising an amino acid sequence with deletion, substitution, insertion or addition of one or several amino acids in SEQ ID NO: 560;
         (47αA2-b) a protein comprising an amino acid sequence having at least 70% identity to the amino acid sequence of SEQ ID NO: 560;
         (47αA2-c) a protein comprising an amino acid sequence encoded by a nucleotide sequence with deletion, substitution, insertion or addition of one or several nucleotides in SEQ ID NO: 559;
         (47αA2-d) a protein comprising an amino acid sequence encoded by a nucleotide sequence having at least 70% identity to the nucleotide sequence of SEQ ID NO: 559; or
         (47αA2-e) a protein comprising an amino acid sequence encoded by a nucleic acid that hybridizes under stringent conditions with a nucleic acid having a nucleotide sequence complementary to the nucleotide sequence of SEQ ID NO: 559; or
      (47αB2) a protein comprising at least one amino acid sequence selected from the group consisting of SEQ ID NOs: 561-563;
   (48α)
      (48αA) uncharacterized protein LOCI00306570 or a variant thereof, which is defined below in any of (48αA-a) to (48αA-e):
         (48αA-a) a protein comprising an amino acid sequence with deletion, substitution, insertion or addition of one or several amino acids in SEQ ID NO: 565;
         (48αA-b) a protein comprising an amino acid sequence having at least 70% identity to the amino acid sequence of SEQ ID NO: 565;
         (48αA-c) a protein comprising an amino acid sequence encoded by a nucleotide sequence with deletion, substitution, insertion or addition of one or several nucleotides in SEQ ID NO: 564;
         (48αA-d) a protein comprising an amino acid sequence encoded by a nucleotide sequence having at least 70% identity to the nucleotide sequence of SEQ ID NO: 564; or
         (48αA-e) a protein comprising an amino acid sequence encoded by a nucleic acid that hybridizes under stringent conditions with a nucleic acid having a nucleotide sequence complementary to the nucleotide sequence of SEQ ID NO: 564; or
      (48αB) a protein comprising at least one amino acid sequence selected from the group consisting of SEQ ID NOs: 566-570;
   (50α)
      (50αA) uncharacterized protein LOC100813859 or a variant thereof, which is defined below in any of (50αA-a) to (50αA-e):
         (50αA-a) a protein comprising an amino acid sequence with deletion, substitution, insertion or addition of one or several amino acids in SEQ ID NO: 572;
         (50αA-b) a protein comprising an amino acid sequence having at least 70% identity to the amino acid sequence of SEQ ID NO: 572;
         (50αA-c) a protein comprising an amino acid sequence encoded by a nucleotide sequence with deletion, substitution, insertion or addition of one or several nucleotides in SEQ ID NO: 571;
         (50αA-d) a protein comprising an amino acid sequence encoded by a nucleotide sequence having at least 70% identity to the nucleotide sequence of SEQ ID NO: 571; or
         (50αA-e) a protein comprising an amino acid sequence encoded by a nucleic acid that hybridizes under stringent conditions with a nucleic acid having a nucleotide sequence complementary to the nucleotide sequence of SEQ ID NO: 571; or
      (50αB) a protein comprising at least one amino acid sequence selected from the group consisting of SEQ ID NOs: 573-576;
   (51α)
      (51αA) 14-3-3-like protein or a variant thereof, which is defined below in any of (51αA-a) to (51αA-e):
         (51αA-a) a protein comprising an amino acid sequence with deletion, substitution, insertion or addition of one or several amino acids in SEQ ID NO: 578;
         (51αA-b) a protein comprising an amino acid sequence having at least 70% identity to the amino acid sequence of SEQ ID NO: 578;
         (51αA-c) a protein comprising an amino acid sequence encoded by a nucleotide sequence with deletion, substitution, insertion or addition of one or several nucleotides in SEQ ID NO: 577;
         (51αA-d) a protein comprising an amino acid sequence encoded by a nucleotide sequence having at least 70% identity to the nucleotide sequence of SEQ ID NO: 577; or
         (51αA-e) a protein comprising an amino acid sequence encoded by a nucleic acid that hybridizes under stringent conditions with a nucleic acid having a nucleotide sequence complementary to the nucleotide sequence of SEQ ID NO: 577; or
      (51αB) a protein comprising at least one amino acid sequence selected from the group consisting of SEQ ID NOs: 579-582;
   (52α)
      (52αA) probable fructokinase-4 or a variant thereof, which is defined below in any of (52αA-a) to (52αA-e):
         (52αA-a) a protein comprising an amino acid sequence with deletion, substitution, insertion or addition of one or several amino acids in SEQ ID NO: 584;
         (52αA-b) a protein comprising an amino acid sequence having at least 70% identity to the amino acid sequence of SEQ ID NO: 584;
         (52αA-c) a protein comprising an amino acid sequence encoded by a nucleotide sequence with deletion, substitution, insertion or addition of one or several nucleotides in SEQ ID NO: 583;
         (52αA-d) a protein comprising an amino acid sequence encoded by a nucleotide sequence having at least 70% identity to the nucleotide sequence of SEQ ID NO: 583; or
         (52αA-e) a protein comprising an amino acid sequence encoded by a nucleic acid that hybridizes under stringent conditions with a nucleic acid having a nucleotide sequence complementary to the nucleotide sequence of SEQ ID NO: 583; or
      (52αB) a protein comprising at least one amino acid sequence selected from the group consisting of SEQ ID NOs: 585-588;
   (53α)
      (53αA1) triosephosphate isomerase isoform X1 or a variant thereof, which is defined below in any of (53αA1-a) to (53αA1-e):
         (53αA1-a) a protein comprising an amino acid sequence with deletion, substitution, insertion or addition of one or several amino acids in SEQ ID NO: 590;
         (53αA1-b) a protein comprising an amino acid sequence having at least 70% identity to the amino acid sequence of SEQ ID NO: 590;
         (53αA1-c) a protein comprising an amino acid sequence encoded by a nucleotide sequence with deletion, substitution, insertion or addition of one or several nucleotides in SEQ ID NO: 589;
         (53αA1-d) a protein comprising an amino acid sequence encoded by a nucleotide sequence having at least 70% identity to the nucleotide sequence of SEQ ID NO: 589; or
         (53αA1-e) a protein comprising an amino acid sequence encoded by a nucleic acid that hybridizes under stringent conditions with a nucleic acid having a nucleotide sequence complementary to the nucleotide sequence of SEQ ID NO: 589;
      (53αB1) a protein comprising at least one amino acid sequence selected from the group consisting of SEQ ID NOs: 591-597;
      (53αA2) triosephosphate isomerase, cytosolic or a variant thereof, which is defined below in any of (53αA2-a) to (53αA2-e):
         (53αA2-a) a protein comprising an amino acid sequence with deletion, substitution, insertion or addition of one or several amino acids in SEQ ID NO: 599;
         (53αA2-b) a protein comprising an amino acid sequence having at least 70% identity to the amino acid sequence of SEQ ID NO: 599;
         (53αA2-c) a protein comprising an amino acid sequence encoded by a nucleotide sequence with deletion, substitution, insertion or addition of one or several nucleotides in SEQ ID NO: 598;
         (53αA2-d) a protein comprising an amino acid sequence encoded by a nucleotide sequence having at least 70% identity to the nucleotide sequence of SEQ ID NO: 598; or
         (53αA2-e) a protein comprising an amino acid sequence encoded by a nucleic acid that hybridizes under stringent conditions with a nucleic acid having a nucleotide sequence complementary to the nucleotide sequence of SEQ ID NO: 598; or
      (53αB2) a protein comprising at least one amino acid sequence selected from the group consisting of SEQ ID NOs: 600-604;
   (54α)
      (54αA1) superoxide dismutase [Fe], chloroplastic isoform X1 or a variant thereof, which is defined below in any of (54αA1-a) to (54αA1-e):
         (54αA1-a) a protein comprising an amino acid sequence with deletion, substitution, insertion or addition of one or several amino acids in SEQ ID NO: 606;
         (54αA1-b) a protein comprising an amino acid sequence having at least 70% identity to the amino acid sequence of SEQ ID NO: 606;
         (53αA1-c) a protein comprising an amino acid sequence encoded by a nucleotide sequence with deletion, substitution, insertion or addition of one or several nucleotides in SEQ ID NO: 605;
         (54αA1-d) a protein comprising an amino acid sequence encoded by a nucleotide sequence having at least 70% identity to the nucleotide sequence of SEQ ID NO: 605; or
         (54αA1-e) a protein comprising an amino acid sequence encoded by a nucleic acid that hybridizes under stringent conditions with a nucleic acid having a nucleotide sequence complementary to the nucleotide sequence of SEQ ID NO: 605;
      (54αB1) a protein comprising at least one amino acid sequence selected from the group consisting of SEQ ID NOs: 607-611;
      (54αA2) iron-superoxide dismutase or a variant thereof, which is defined below in any of (54αA2-a) to (54αA2-e):
         (54αA2-a) a protein comprising an amino acid sequence with deletion, substitution, insertion or addition of one or several amino acids in SEQ ID NO: 613;
         (54αA2-b) a protein comprising an amino acid sequence having at least 70% identity to the amino acid sequence of SEQ ID NO: 613;
         (54αA2-c) a protein comprising an amino acid sequence encoded by a nucleotide sequence with deletion, substitution, insertion or addition of one or several nucleotides in SEQ ID NO: 612;
         (54αA2-d) a protein comprising an amino acid sequence encoded by a nucleotide sequence having at least 70% identity to the nucleotide sequence of SEQ ID NO: 612; or
         (54αA2-e) a protein comprising an amino acid sequence encoded by a nucleic acid that hybridizes under stringent conditions with a nucleic acid having a nucleotide sequence complementary to the nucleotide sequence of SEQ ID NO: 612; or
      (54αB2) a protein comprising at least one amino acid sequence selected from the group consisting of SEQ ID NOs: 614-616; or
   (55α)
      (55αA1) 51 kDa seed maturation protein precursor or a variant thereof, which is defined below in any of (55αA1-a) to (55αA1-e):
         (55αA1-a) a protein comprising an amino acid sequence with deletion, substitution, insertion or addition of one or several amino acids in SEQ ID NO: 618;
         (55αA1-b) a protein comprising an amino acid sequence having at least 70% identity to the amino acid sequence of SEQ ID NO: 618;
         (55αA1-c) a protein comprising an amino acid sequence encoded by a nucleotide sequence with deletion, substitution, insertion or addition of one or several nucleotides in SEQ ID NO: 617;
         (55αA1-d) a protein comprising an amino acid sequence encoded by a nucleotide sequence having at least 70% identity to the nucleotide sequence of SEQ ID NO: 617; or
         (55αA1-e) a protein comprising an amino acid sequence encoded by a nucleic acid that hybridizes under stringent conditions with a nucleic acid having a nucleotide sequence complementary to the nucleotide sequence of SEQ ID NO: 617;
      (55αB1) a protein comprising at least one amino acid sequence selected from the group consisting of SEQ ID NOs: 619-635;
      (55αA2) Lea protein precursor or a variant thereof, which is defined below in any of (55αA2-a) to (55αA2-e):
         (55αA2-a) a protein comprising an amino acid sequence with deletion, substitution, insertion or addition of one or several amino acids in SEQ ID NO: 637;
         (55αA2-b) a protein comprising an amino acid sequence having at least 70% identity to the amino acid sequence of SEQ ID NO: 637;
         (55αA2-c) a protein comprising an amino acid sequence encoded by a nucleotide sequence with deletion, substitution, insertion or addition of one or several nucleotides in SEQ ID NO: 636;
         (55αA2-d) a protein comprising an amino acid sequence encoded by a nucleotide sequence having at least 70% identity to the nucleotide sequence of SEQ ID NO: 636; or
         (55αA2-e) a protein comprising an amino acid sequence encoded by a nucleic acid that hybridizes under stringent conditions with a nucleic acid having a nucleotide sequence complementary to the nucleotide sequence of SEQ ID NO: 636; or
      (55αB2) a protein comprising at least one amino acid sequence selected from the group consisting of SEQ ID NOs: 638-649.
[10] A composition for diagnosing an allergy to a soybean, comprising, as an antigen, at least one of proteins as defined above in any of (1α) to (55α) of [9].
[11] A method for providing an indicator for diagnosing an allergy to a soybean in a subject, the method comprising the steps of:
   (i) contacting a sample obtained from the subject with an antigen, wherein the sample is a solution comprising an Ig antibody;
   (ii) detecting binding between the IgE antibody present in the sample from the subject and the antigen; and
   (iii) when the binding between the IgE antibody in the subject and the antigen is detected, an indicator of the fact that the subject is allergic to a soybean is provided;
   wherein the antigen is at least one of proteins as defined above in any of (1α) to (55α) of [9].
[12] A pharmaceutical composition comprising at least one of proteins as defined above in any of (1α) to (55α) of [9].
[13] The pharmaceutical composition as set forth in [12], wherein the pharmaceutical composition is intended for the treatment of an allergy to a soybean.
[14] A soybean or processed products of soybean in which an antigen is eliminated or reduced, wherein the antigen is at least one of proteins as defined above in any of (1α) to (55α) of [9].
[15] A tester for determining the presence or absence of a soybean antigen in an object of interest, comprising an antibody that binds to at least one of proteins as defined above in any of (1α) to (55α) of [9].
[16] A soybean-derived antigen which is at least one of proteins as defined above in any of (1α) to (55α) of [9] and is causative of an allergy to soybean.

### ADVANTAGEOUS EFFECTS OF INVENTION

The present invention can provide novel antigens of an allergy to soybean. Since the novel antigens (allergen components) that trigger a soybean allergy were identified according to this invention, this invention can provide highly sensitive methods and kits for diagnosing an allergy to soybean, pharmaceutical compositions comprising such an antigen, soybean or processed products of soybean in which such an antigen is eliminated or reduced.

### BRIEF DESCRIPTION OF DRAWINGS

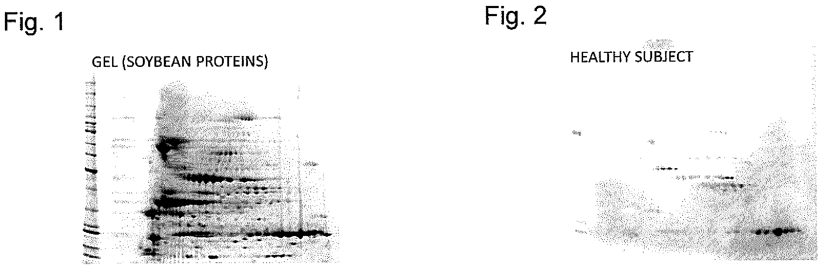

Fig. 1 is a photograph of a gel showing a protein electrophoretic pattern in two-dimensional electrophoresis of proteins contained in soybean. The bands at the left of the photograph are bands of molecular weight markers.
Fig. 2 is a photograph of an immunoblot of a two-dimensional electrophoretic pattern of proteins contained in soybean stained with serum of a healthy subject.

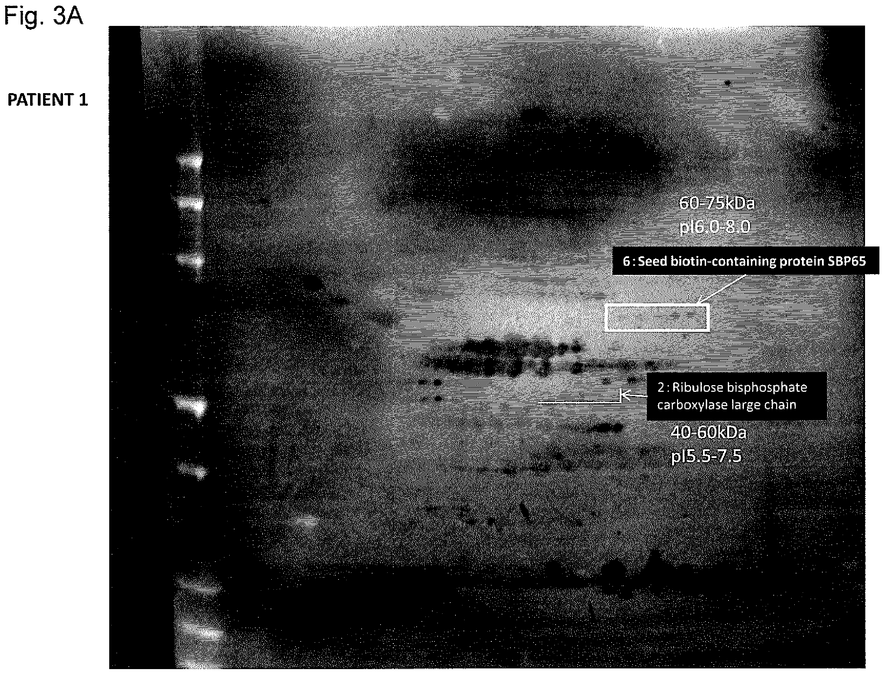

Fig. 3A is a photograph of an immunoblot of a two-dimensional electrophoretic pattern of proteins contained in soybean stained with serum of Soybean-allergic patient 1. The arrows indicate spots where the serum of the soybean-allergic patient specifically reacted.

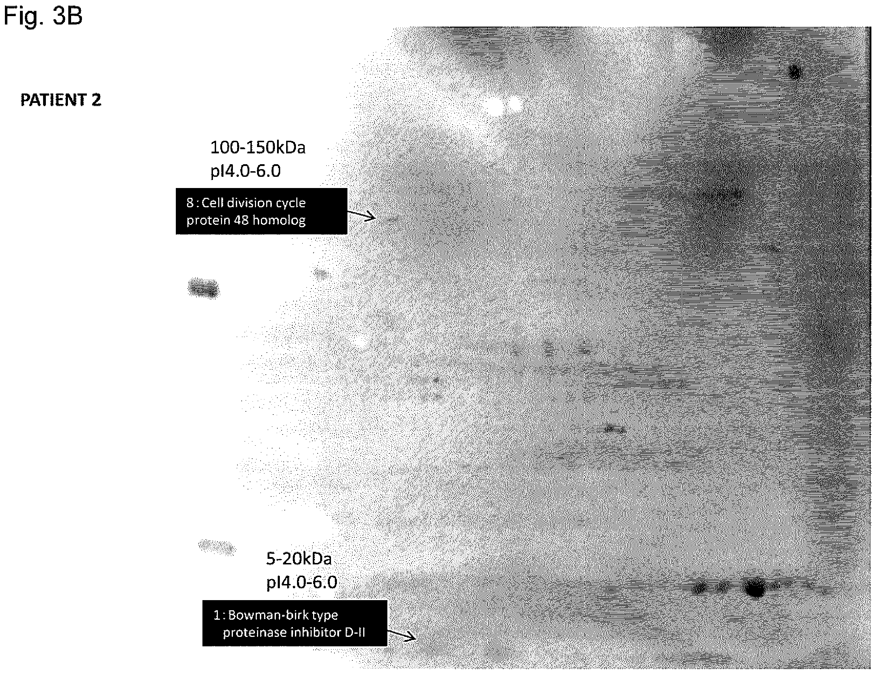

Fig. 3B is a photograph of an immunoblot of a two-dimensional electrophoretic pattern of proteins contained in soybean stained with serum of Soybean-allergic patient 2. The arrows indicate spots where the serum of the soybean-allergic patient specifically reacted.

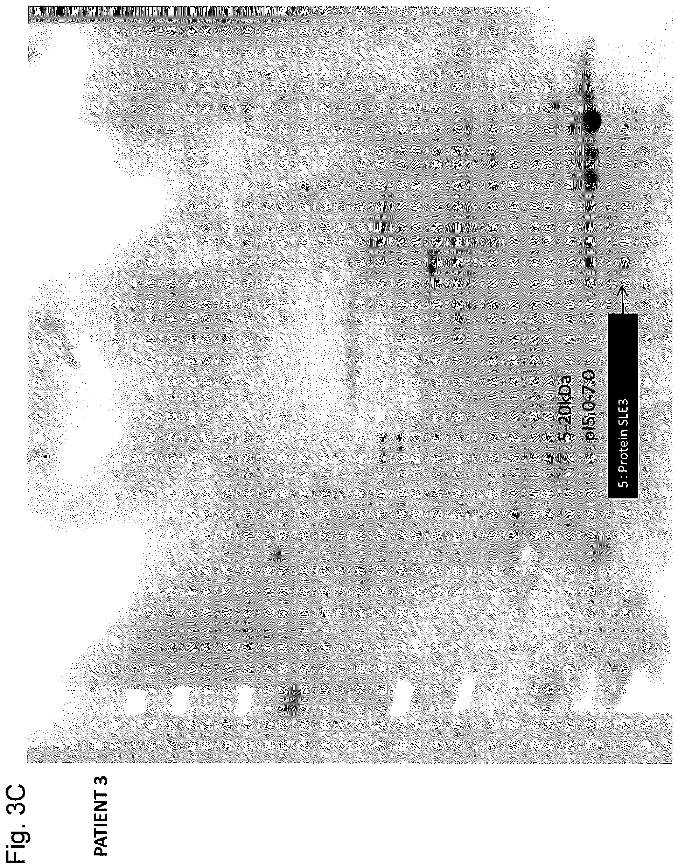

Fig. 3C is a photograph of an immunoblot of a two-dimensional electrophoretic pattern of proteins contained in soybean stained with serum of Soybean-allergic patient 3. The arrows indicate spots where the serum of the soybean-allergic patient specifically reacted.

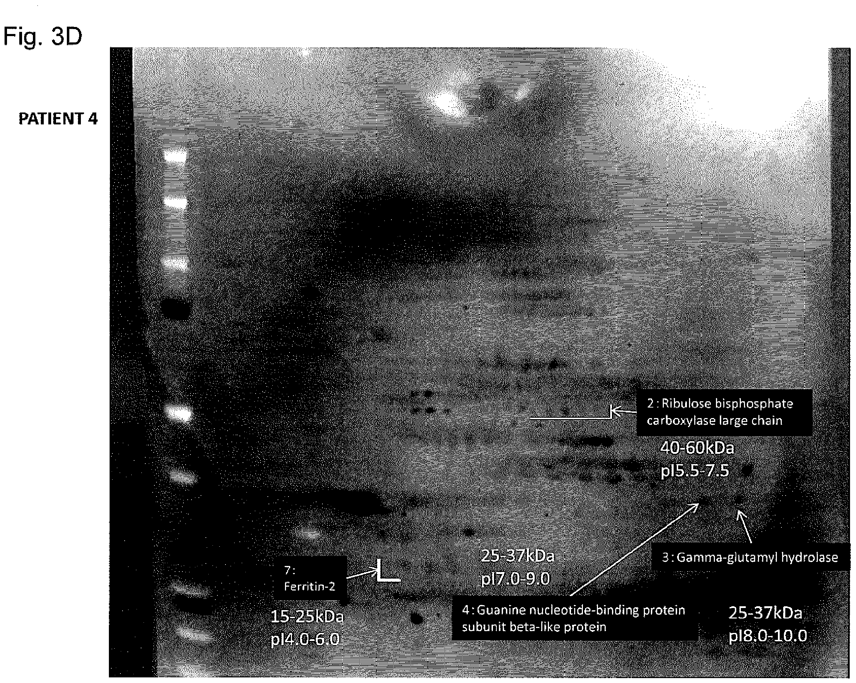

Fig. 3D is a photograph of an immunoblot of a two-dimensional electrophoretic pattern of proteins contained in soybean stained with serum of Soybean-allergic patient 4. The arrows indicate spots where the serum of the soybean-allergic patient specifically reacted.

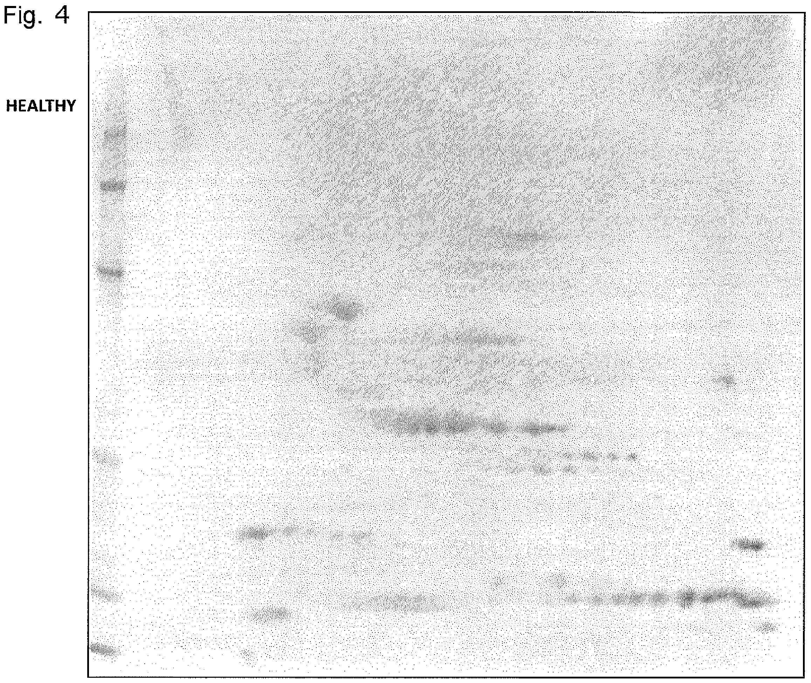

Fig. 4 is a photograph of an immunoblot of a two-dimensional electrophoretic pattern of proteins contained in soybean stained with serum of a healthy subject.

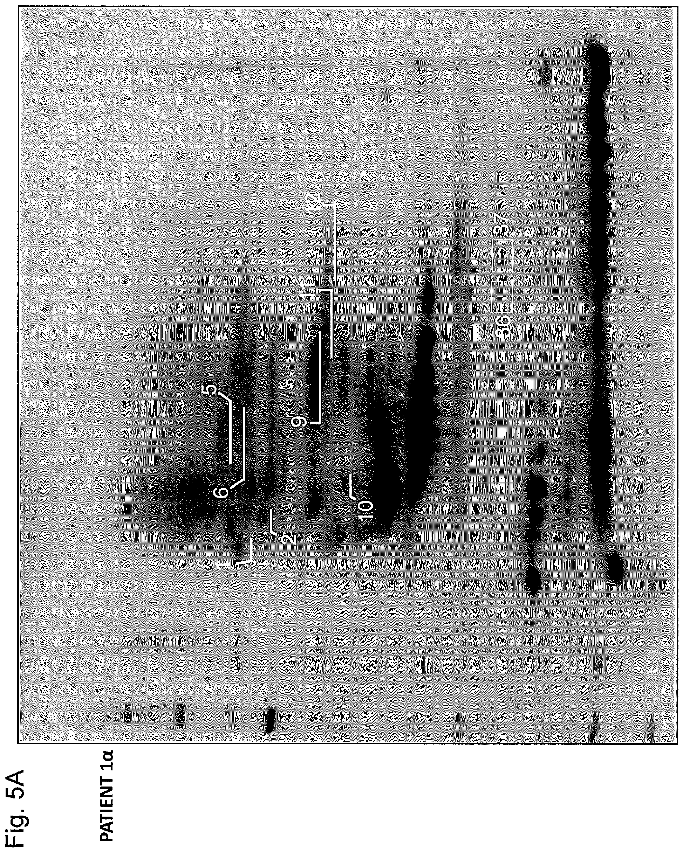

Fig. 5A is a photograph of an immunoblot of a two-dimensional electrophoretic pattern of proteins contained in soybean stained with serum of Soybean-allergic patient 1α. The spots indicated with a white line or enclosed in a square are spot where the serum of the soybean-allergic patient specifically reacted. The numbers (n) indicated are the numbers of the spots and correspond to the spots indicated with "nα" herein.

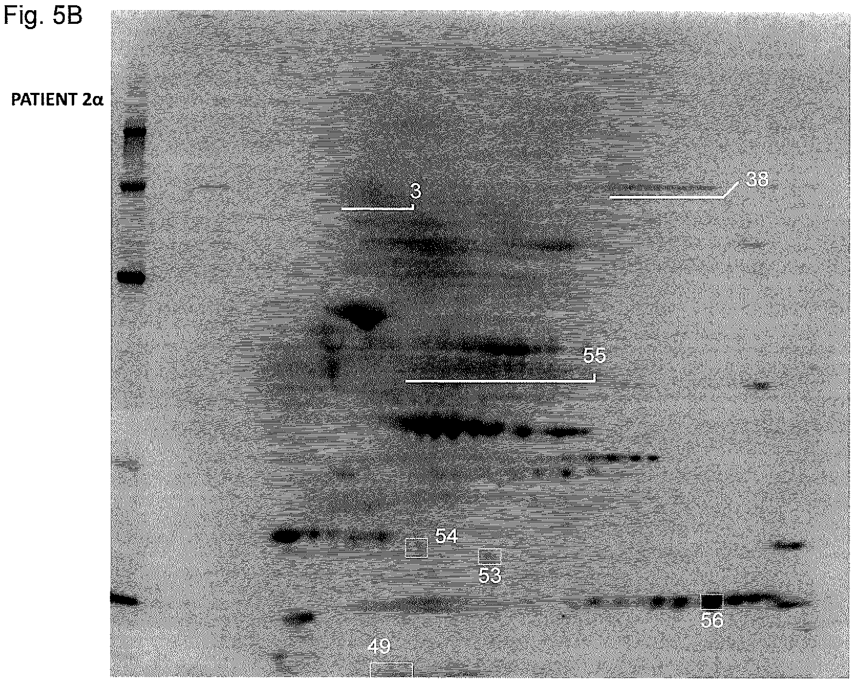

Fig. 5B is a photograph of an immunoblot of a two-dimensional electrophoretic pattern of proteins contained in soybean stained with serum of Soybean-allergic patient 2α. The spots indicated with a white line or enclosed in a square are spot where the serum of the soybean-allergic patient specifically reacted. The numbers (n) indicated are the numbers of the spots and correspond to the spots indicated with "nα" herein.

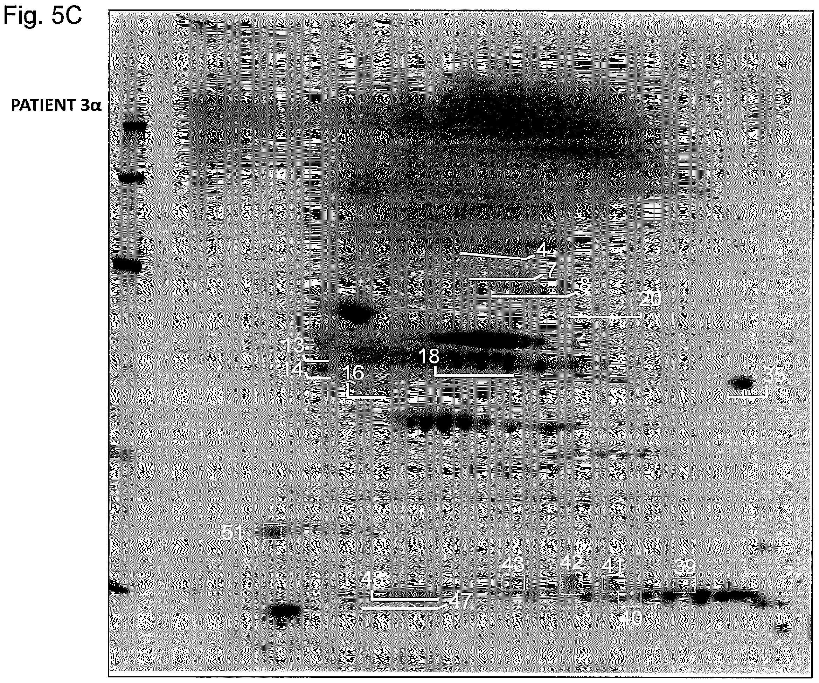

Fig. 5C is a photograph of an immunoblot of a two-dimensional electrophoretic pattern of proteins contained in soybean stained with serum of Soybean-allergic patient 3α. The spots indicated with a white line or enclosed in a square are spot where the serum of the soybean-allergic patient specifically reacted. The numbers (n) indicated are the numbers of the spots and correspond to the spots indicated with "nα" herein.

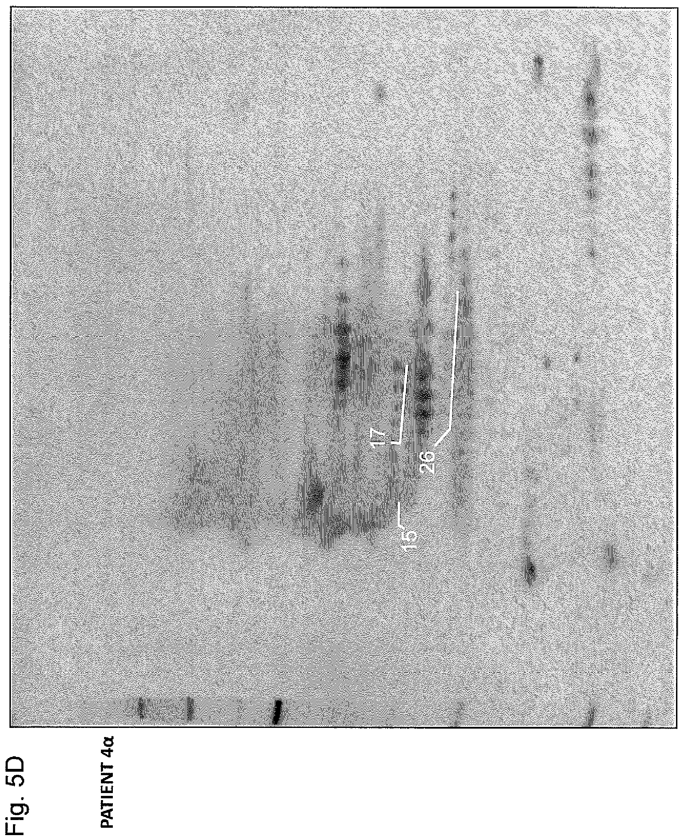

Fig. 5D is a photograph of an immunoblot of a two-dimensional electrophoretic pattern of proteins contained in soybean stained with serum of Soybean-allergic patient 4α. The spots indicated with a while line are spots where the serum of the soybean-allergic patient specifically reacted. The numbers (n) indicated are the numbers of the spots and correspond to the spots indicated with "nα" herein.

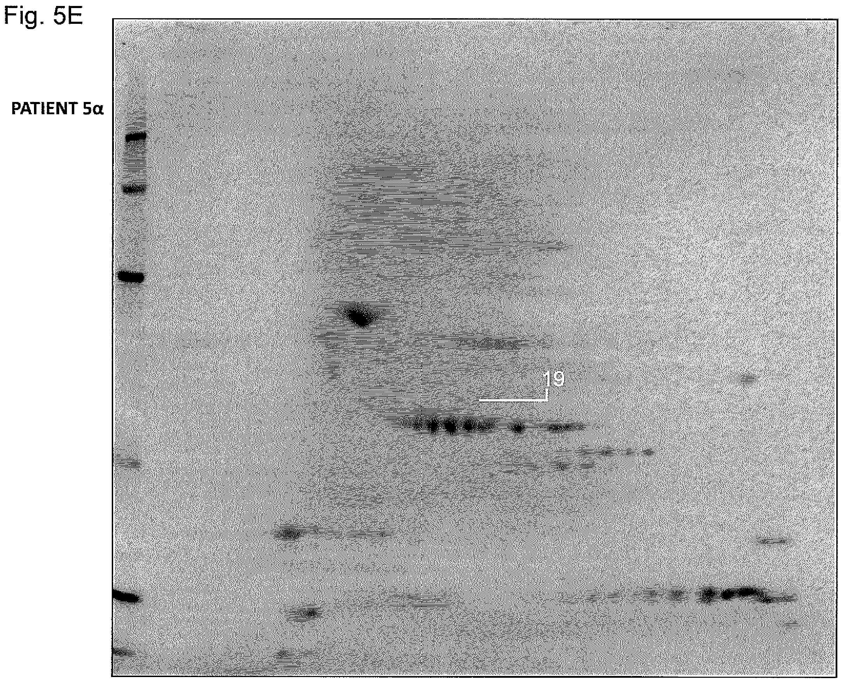

Fig. 5E is a photograph of an immunoblot of a two-dimensional electrophoretic pattern of proteins contained in soybean stained with serum of Soybean-allergic patient 5α. The spots indicated with a while line are spots where the serum of the soybean-allergic patient specifically reacted. The numbers (n) indicated are the numbers of the spots and correspond to the spots indicated with "nα" herein.

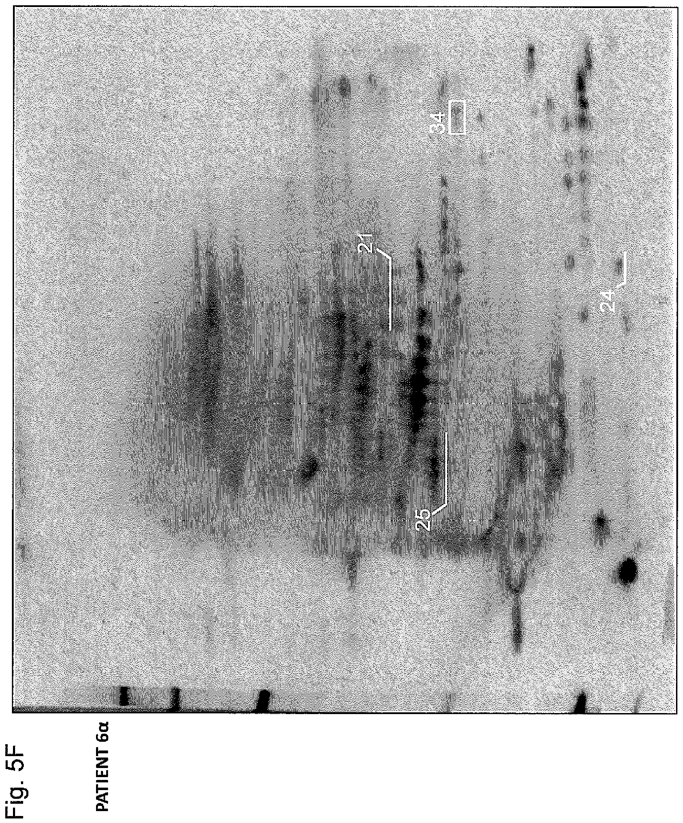

Fig. 5F is a photograph of an immunoblot of a two-dimensional electrophoretic pattern of proteins contained in soybean stained with serum of Soybean-allergic patient 6α. The spots indicated with a white line or enclosed in a square are spot where the serum of the soybean-allergic patient specifically reacted. The numbers (n) indicated are the numbers of the spots and correspond to the spots indicated with "nα" herein.

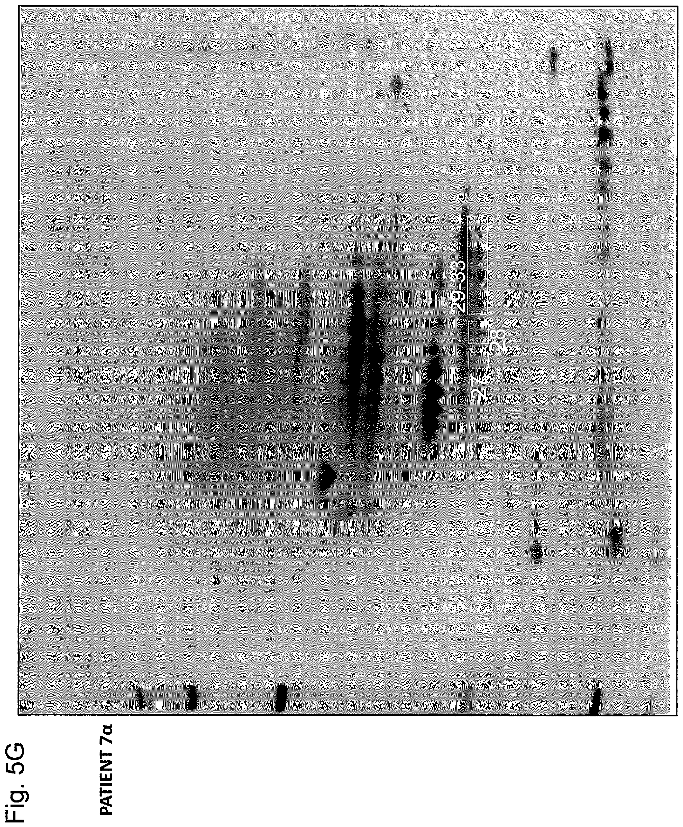

Fig. 5G is a photograph of an immunoblot of a two-dimensional electrophoretic pattern of proteins contained in soybean stained with serum of Soybean-allergic patient 7α. The spots enclosed in a square are spots where the serum of the soybean-allergic patient specifically reacted. The numbers (n) indicated are the numbers of the spots and correspond to the spots indicated with "nα" herein.

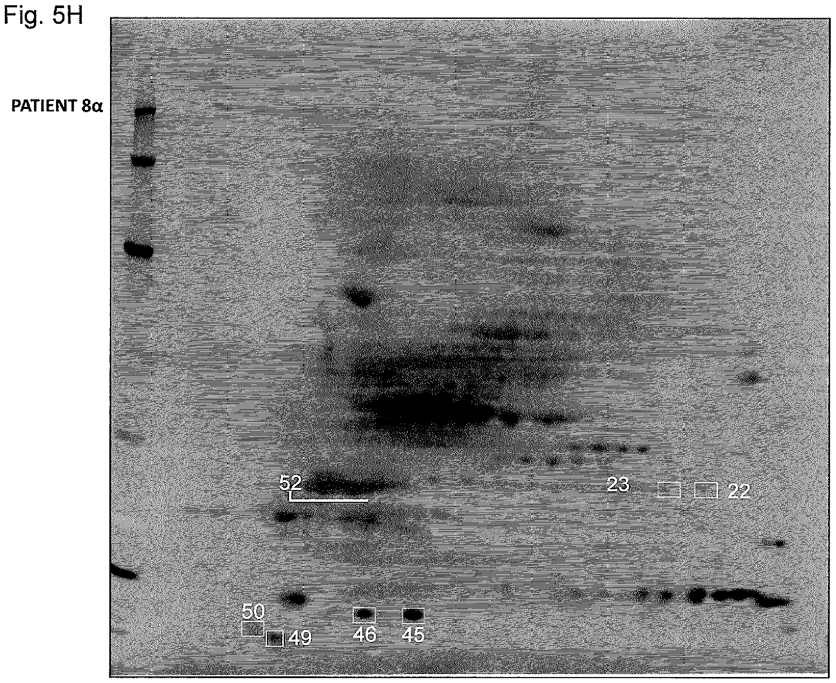

Fig. 5H is a photograph of an immunoblot of a two-dimensional electrophoretic pattern of proteins contained in soybean stained with serum of Soybean-allergic patient 8α. The spots indicated with a white line or enclosed in a square are spot where the serum of the soybean-allergic patient specifically reacted. The numbers (n) indicated are the numbers of the spots and correspond to the spots indicated with "nα" herein.

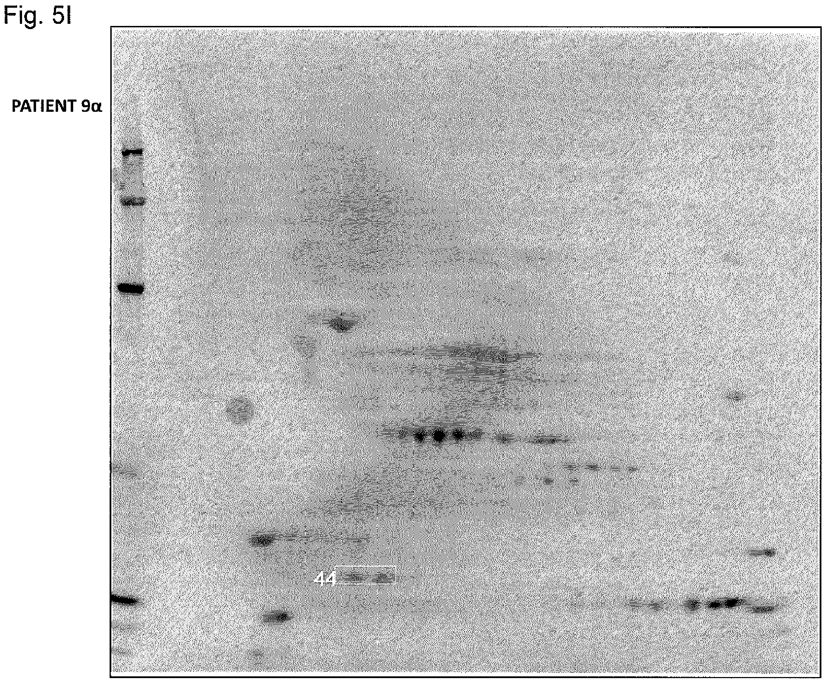

Fig. 5I is a photograph of an immunoblot of a two-dimensional electrophoretic pattern of proteins contained in soybean stained with serum of Soybean-allergic patient 9α. The spots indicated with a white line or enclosed in a square are spot where the serum of the soybean-allergic patient specifically reacted. The numbers (n) indicated are the numbers of the spots and correspond to the spots indicated with "nα" herein.

### DESCRIPTION OF EMBODIMENTS

The present invention will be described in detail below, but the present invention is not limited to them.

Unless otherwise defined herein, all scientific and technical terms used in relation to the present invention shall have meanings commonly understood by those skilled in the art.

Amino acid sequences herein are represented by one-letter amino acid symbols well known to those skilled in the art and the left end corresponds to the amino terminal and the right end corresponds to the carboxy terminal.

As referred to herein, the "allergy" refers to the state in which, when a certain antigen enters the body of a living individual sensitized to said antigen, the living individual shows a hypersensitive reaction detrimental to him/her. In blood and tissues of individuals with many food-allergic diseases, IgE antibodies specific to antigens are produced. IgE antibodies bind to mast cells or basophils. When an antigen specific to such an IgE antibody enters again the body of a patient with an allergic disease, said antigen combines with the IgE antibody bound to mast cells or basophils, and the IgE antibody crosslinks said antigen on the cell surface, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Such allergic reactions caused by particular antigens occur through the aforementioned pathway.

As referred to herein, the "allergy to soybean" refers to the state in which an individual has an allergic reaction caused by proteins, etc. present in soybean which act as an antigen. The allergy to soybean can produce an allergic reaction upon contact with, or consumption of, an antigen present in soybean. In general, allergic reactions caused by consumption of foods are particularly referred to as "food allergies". The allergy to soybean may be a food allergy.

As referred to herein, the "antigen" refers to a substance that provokes an allergic reaction, and is also referred to as an "allergen component". The antigen is preferably a protein.

As referred to herein, the "protein" refers to a molecule having a structure in which naturally occurring amino acids are joined together by peptide bond. The number of amino acids present in a protein is not particularly limited, but proteins having about 2 to 50 amino acids joined together by peptide bond are in some cases called "peptides". In the case where amino acids can form different enantiomers, the amino acids are understood to form an L-enantiomer, unless otherwise indicated. The amino acid sequences of proteins or peptides as used herein are represented by one-letter symbols of amino acids in accordance with standard usage and the notational convention commonly used in the art. The leftward direction represents the amino-terminal direction, and the rightward direction represents the carboxy-terminal direction.

### Identification of antigens

Proteins contained in soybean were analyzed by the aforementioned technique to identify causative antigens of an allergy to soybean. To be specific, soybean proteins were subjected to two-dimensional electrophoresis under the conditions described below.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: with the total gel-strip length being taken as 1, the gel-strip length up to pH 5 was "a = 0.15 to 0.3", the gel-strip length from pH 5 to 7 was "b = 0.4 to 0.7", and the gel-strip length above pH 7 was "c = 0.15 to 0.3". More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bris-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and 1mM EDTA.

As a result, the following spots in a two-dimensional electrophoresis gel run under the conditions described above for proteins in soybean have been revealed to exhibit specific binding to IgE antibodies from soybean-allergic patients.
Spot 1: Molecular weight 1 to 30 kDa, pI 3.0 to 7.0
Spot 2: Molecular weight 30 to 75 kDa, pI 4.0 to 8.5
Spot 3: Molecular weight 20 to 50 kDa, pI 7.0 to 12.0
Spot 4: Molecular weight 20 to 50 kDa, pI 6.0 to 10.0
Spot 5: Molecular weight 2 to 30 kDa, pI 4.0 to 9.0
Spot 6: Molecular weight 50 to 100 kDa, pI 5.0 to 10.0
Spot 7: Molecular weight 5 to 37 kDa, pI 3.0 to 7.0
Spot 8: Molecular weight 75 to 200 kDa, pI 4.0 to 6.0
Spot 1α: Molecular weight 70 to 120 kDa, pI 2.5 to 6.5
Spot 2α: Molecular weight 50 to 120 kDa, pI 2.5 to 6.5
Spot 4α: Molecular weight 70 to 120 kDa, pI 3.5 to 7.5
Spot 5α: Molecular weight 70 to 170 kDa, pI 3.5 to 7.5
Spot 6α: Molecular weight 70 to 120 kDa, pI 3.5 to 7.5
Spot 7α: Molecular weight 50 to 120 kDa, pI 3.5 to 7.5
Spot 8α: Molecular weight 50 to 120 kDa, pI 3.5 to 8.5
Spot 9α: Molecular weight 50 to 120 kDa, pI 3.5 to 8.5
Spot 10α: Molecular weight 30 to 90 kDa, pI 3.5 to 7.5
Spot 11α: Molecular weight 30 to 90 kDa, pI 3.5 to 8.5
Spot 12α: Molecular weight 30 to 90 kDa, pI 3.5 to 8.5
Spot 13α: Molecular weight 30 to 90 kDa, pI 3.5 to 7.5
Spot 14α: Molecular weight 30 to 90 kDa, pI 2.5 to 6.5
Spot 15α, 16α: Molecular weight 20 to 90 kDa, pI 3.5 to 7.5
Spot 17α: Molecular weight 20 to 90 kDa, pI 3.5 to 7.5
Spot 18α: Molecular weight 30 to 90 kDa, pI 3.5 to 8.5
Spot 19α: Molecular weight 30 to 90 kDa, pI 3.5 to 8.5
Spot 25α: Molecular weight 20 to 90 kDa, pI 3.5 to 7.5
Spot 26α: Molecular weight 20 to 90 kDa, pI 3.5 to 7.5
Spot 27α: Molecular weight 20 to 90 kDa, pI 3.5 to 7.5
Spot 28α: Molecular weight 10 to 70 kDa, pI 3.5 to 7.5
Spot 29α, 30α, 31α, 32α, 33α: Molecular weight 10 to 70 kDa, pI 3.5 to 8.5
Spot 34α: Molecular weight 10 to 70 kDa, pI 5.5 to 10.5
Spot 35α: Molecular weight 20 to 90 kDa, pI 5.5 to 10.5
Spot 36α, 37α: Molecular weight 10 to 70 kDa, pI 3.5 to 8.5
Spot 38a: Molecular weight 70 to 170 kDa, pI 5.5 to 10.5
Spot 39α: Molecular weight 5 to 60 kDa, pI 5.5 to 10.5
Spot 40α, 56α: Molecular weight 5 to 60 kDa, pI 5.5 to 10.5
Spot 41α: Molecular weight 5 to 60 kDa, pI 5.5 to 10.5
Spot 42α, 43α: Molecular weight 5 to 60 kDa, pI 5.5 to 10.5
Spot 45α: Molecular weight 2 to 50 kDa, pI 3.5 to 7.5
Spot 46α: Molecular weight 2 to 50 kDa, pI 3.5 to 7.5
Spot 47α: Molecular weight 5 to 50 kDa, pI 3.5 to 7.5
Spot 48α: Molecular weight 2 to 50 kDa, pI 3.5 to 8.5
Spot 50α: Molecular weight 2 to 50 kDa, pI 2.5 to 6.5
Spot 51α: Molecular weight 5 to 60 kDa, pI 2.5 to 6.5
Spot 52α: Molecular weight 10 to 70 kDa, pI 3.5 to 7.5
Spot 53α: Molecular weight 5 to 60 kDa, pI 3.5 to 8.5
Spot 54α: Molecular weight 5 to 60 kDa, pI 3.5 to 8.5
Spot 55α: Molecular weight 30 to 90 kDa, pI 5.5 to 10.5

### Antigen

### (1) Antigen in spot 1

As the result of sequence identification of the antigen in spot 1 by mass spectroscopy, the following amino acid sequences were detected.
DQSSSYDDDEYSK (SEQ ID NO: 17)
KSDQSSSYDDDEYSK (SEQ ID NO: 18)
SDQSSSYDDDEYSK (SEQ ID NO: 19)
SYDDDEYSK (SEQ ID NO: 20)

Also, the mass spectroscopic data obtained for spot 1 on a mass spectrometer was analyzed by comparing the data against the Uniprot protein data, and as a result, the antigen in question was identified as Bowman-Birk type proteinase inhibitor D-II (amino acid sequence: SEQ ID NO: 2, encoding nucleotide sequence: SEQ ID NO: 1).

Accordingly, in the present invention, the antigen in spot 1 can be any of (1A-a) to (1A-e) and (1B) as defined below:
(1A-a) a protein comprising an amino acid sequence with deletion, substitution, insertion or addition of one or several amino acids in SEQ ID NO: 2;
(1A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 2;
(1A-c) a protein comprising an amino acid sequence encoded by a nucleotide sequence with deletion, substitution, insertion or addition of one or several nucleotides in SEQ ID NO: 1;
(1A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 1;
(1A-e) a protein comprising an amino acid sequence encoded by a nucleic acid that hybridizes under stringent conditions with a nucleic acid having a nucleotide sequence complementary to the nucleotide sequence of SEQ ID NO: 1;
(1B) a protein comprising at least one amino acid sequence selected from the group consisting of SEQ ID NOs: 17-20, preferably a protein comprising at least 2 or 3 or all sequences of the amino acid sequences; more preferably a protein comprising at least one amino acid sequence selected from the group consisting of SEQ ID NOs: 17-19, preferably a protein comprising at least 2 or all sequences of the amino acid sequences. As referred to above, the amino acid sequence of any of SEQ ID NOs: 17-20 may be an amino acid sequence derived therefrom by deletion, substitution, insertion or addition of one or several amino acids.

The proteins of (1A-a) to (1A-e) and (1B) as defined above also include those proteins whose amino acid residues are modified by phosphorylation, sugar chain modification, aminoacylation, ring-opening, deamination or the like.

The proteins of (1A-a) to (1A-e) and (1B) as defined above can be proteins that are found in a protein spot with a molecular weight of around 1 to 30 kDa, preferably around 2 to 25 kDa, more preferably around 5 to 20 kDa and an isoelectric point of 3.0 to 7.0, preferably 4.0 to 6.0 on gels used in the two-dimensional electrophoresis performed under the conditions described above in the subsection titled "Identification of antigens".

Preferably, a protein that is an antigen in spot 1 has activity equivalent to that of the protein having the amino acid sequence of SEQ ID NO: 2 (Bowman-Birk type proteinase inhibitor D-II) or causative of an allergy to soybean.

### (2) Antigen in spot 2

As the result of sequence identification of the antigen in spot 2 by mass spectroscopy, the following amino acid sequences were detected.
ALRALRLEDLRIPTAYIK (SEQ ID NO: 21)
ALRLSGGDHVHAGTVVGK (SEQ ID NO: 22)
LEGEREITLGFVDLLRDDFVEK (SEQ ID NO: 23)
LRLEDLRIPTAYIK (SEQ ID NO: 24)
LTYYTPDYETK (SEQ ID NO: 25)
TDGLTSLDRYK (SEQ ID NO: 26)
TFQGPPHGIQVERD (SEQ ID NO: 27)
TFQGPPHGIQVERDK (SEQ ID NO: 28)

Also, the mass spectroscopic data obtained for spot 2 on a mass spectrometer was analyzed by comparing the data against the Uniprot protein data, and as a result, the antigen in question was identified as Ribulose bisphosphate carboxylase large chain (amino acid sequence: SEQ ID NO: 4, encoding nucleotide sequence: SEQ ID NO: 3).

Accordingly, in the present invention, the antigen in spot 2 can be any of (2A-a) to (2A-e) and (2B) as defined below:
(2A-a) a protein comprising an amino acid sequence with deletion, substitution, insertion or addition of one or several amino acids in SEQ ID NO: 4;
(2A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 4;
(2A-c) a protein comprising an amino acid sequence encoded by a nucleotide sequence with deletion, substitution, insertion or addition of one or several nucleotides in SEQ ID NO: 3;
(2A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 3;
(2A-e) a protein comprising an amino acid sequence encoded by a nucleic acid that hybridizes under stringent conditions with a nucleic acid having a nucleotide sequence complementary to the nucleotide sequence of SEQ ID NO: 3;
(2B) a protein comprising at least one amino acid sequence selected from the group consisting of SEQ ID NOs: 21-28, preferably a protein comprising at least 2, 3, 4, 5, 6 or 7 or all sequences of the amino acid sequences. As referred to above, the amino acid sequence of any of SEQ ID NOs: 21-28 may be an amino acid sequence derived therefrom by deletion, substitution, insertion or addition of one or several amino acids.

The proteins of (2A-a) to (2A-e) and (2B) as defined above also include those proteins whose amino acid residues are modified by phosphorylation, sugar chain modification, aminoacylation, ring-opening, deamination or the like.

The proteins of (2A-a) to (2A-e) and (2B) as defined above can be proteins that are found in a protein spot with a molecular weight of around 30 to 75 kDa, preferably around 37 to 70 kDa, more preferably around 40 to 60 kDa and an isoelectric point of 4.0 to 8.5, preferably 5.5 to 7.5 on gels used in the two-dimensional electrophoresis performed under the conditions described above in the subsection titled "Identification of antigens".

Preferably, a protein that is an antigen in spot 2 has activity equivalent to that of the protein having the amino acid sequence of SEQ ID NO: 4 (Ribulose bisphosphate carboxylase large chain) or causative of an allergy to soybean.

### (3) Antigen in spot 3

As the result of sequence identification of the antigen in spot 3 by mass spectroscopy, the following amino acid sequences were detected.
APHTEDAIRVTQSTANFFISEARK (SEQ ID NO: 29)
FVESGGARVIPLIYNESPENLNK (SEQ ID NO: 30)

Also, the mass spectroscopic data obtained for spot 3 on a mass spectrometer was analyzed by comparing the data against the Uniprot protein data, and as a result, the antigen in question was identified as Gamma-glutamyl hydrolase (amino acid sequence: SEQ ID NO: 6, encoding nucleotide sequence: SEQ ID NO: 5).

Accordingly, in the present invention, the antigen in spot 3 can be any of (3A-a) to (3A-e) and (3B) as defined below:
(3A-a) a protein comprising an amino acid sequence with deletion, substitution, insertion or addition of one or several amino acids in SEQ ID NO: 6;
(3A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 6;
(3A-c) a protein comprising an amino acid sequence encoded by a nucleotide sequence with deletion, substitution, insertion or addition of one or several nucleotides in SEQ ID NO: 5;
(3A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 5.
(3A-e) a protein comprising an amino acid sequence encoded by a nucleic acid that hybridizes under stringent conditions with a nucleic acid having a nucleotide sequence complementary to the nucleotide sequence of SEQ ID NO: 5;
(3B) a protein comprising an amino acid sequence of SEQ ID NO: 29 or 30, preferably a protein comprising the amino acid sequences of SEQ ID NO: 29 and 30. As referred to above, the amino acid sequence(s) of SEQ ID NOs: 29 and/or 30 may be an amino acid sequence(s) derived therefrom by deletion, substitution, insertion or addition of one or several amino acids.

The proteins of (3A-a) to (3A-e) and (3B) as defined above also include those proteins whose amino acid residues are modified by phosphorylation, sugar chain modification, aminoacylation, ring-opening, deamination or the like.

The proteins of (3A-a) to (3A-e) and (3B) as defined above can be proteins that are found in a protein spot with a molecular weight of around 20 to 50 kDa, preferably around 25 to 40 kDa, more preferably around 25 to 37 kDa and an isoelectric point of 7.0 to 12.0, preferably 8.0 to 10.0 on gels used in the two-dimensional electrophoresis performed under the conditions described above in the subsection titled "Identification of antigens".

Preferably, a protein that is an antigen in spot 3 has activity equivalent to that of the protein having the amino acid sequence of SEQ ID NO: 6 (Gamma-glutamyl hydrolase) or causative of an allergy to soybean.

### (4) Antigen in spot 4

As the result of sequence identification of the antigen in spot 4 by mass spectroscopy, the following amino acid sequences were detected.
IWDLESK (SEQ ID NO: 31)
SIVEDLK (SEQ ID NO: 32)

Also, the mass spectroscopic data obtained for spot 4 on a mass spectrometer was analyzed by comparing the data against the Uniprot protein data, and as a result, the antigen in question was identified as Guanine nucleotide-binding protein subunit beta-like protein (amino acid sequence: SEQ ID NO: 8, encoding nucleotide sequence: SEQ ID NO: 7).

Accordingly, in the present invention, the antigen in spot 4 can be any of (4-a) to (4-e) and (4B) as defined below:
(4-a) a protein comprising an amino acid sequence with deletion, substitution, insertion or addition of one or several amino acids in SEQ ID NO: 8;
(4-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 8;
(4-c) a protein comprising an amino acid sequence encoded by a nucleotide sequence with deletion, substitution, insertion or addition of one or several nucleotides in SEQ ID NO: 7;
(4A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 7;
(4A-e) a protein comprising an amino acid sequence encoded by a nucleic acid that hybridizes under stringent conditions with a nucleic acid having a nucleotide sequence complementary to the nucleotide sequence of SEQ ID NO: 7;
(4B) a protein comprising an amino acid sequence of SEQ ID NO: 31 or 32, preferably a protein comprising the amino acid sequences of SEQ ID NO: 31 and 32. As referred to above, the amino acid sequence(s) of SEQ ID NOs: 31 and/or 32 may be an amino acid sequence(s) derived therefrom by deletion, substitution, insertion or addition of one or several amino acids.

The proteins of (4A-a) to (4A-e) and (4B) as defined above also include those proteins whose amino acid residues are modified by phosphorylation, sugar chain modification, aminoacylation, ring-opening, deamination or the like.

The proteins of (4A-a) to (4A-e) and (4B) as defined above can be proteins that are found in a protein spot with a molecular weight of around 20 to 50 kDa, preferably around 25 to 40 kDa, more preferably around 25 to 37 kDa and an isoelectric point of 6.0 to 10.0, preferably 7.0 to 9.0 on gels used in the two-dimensional electrophoresis performed under the conditions described above in the subsection titled "Identification of antigens".

Preferably, a protein that is an antigen in spot 4 has activity equivalent to that of the protein having the amino acid sequence of SEQ ID NO: 8 (Guanine nucleotide-binding protein subunit beta-like protein) or causative of an allergy to soybean.

### (5) Antigen in spot 5

As the result of sequence identification of the antigen in spot 5 by mass spectroscopy, the following amino acid sequences were detected.
QELDERARQGETVVPGGTGGK (SEQ ID NO: 33)
SGEERAQEEAIDIDESK (SEQ ID NO: 34)

Also, the mass spectroscopic data obtained for spot 5 on a mass spectrometer was analyzed by comparing the data against the Uniprot protein data, and as a result, the antigen in question was identified as Protein SLE3 (amino acid sequence: SEQ ID NO: 10, encoding nucleotide sequence: SEQ ID NO: 9).

Accordingly, in the present invention, the antigen in spot 5 can be any of (5A-a) to (5A-e) and (5B) as defined below:
(5A-a) a protein comprising an amino acid sequence with deletion, substitution, insertion or addition of one or several amino acids in SEQ ID NO: 10;
(5A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 10;
(5A-c) a protein comprising an amino acid sequence encoded by a nucleotide sequence with deletion, substitution, insertion or addition of one or several nucleotides in SEQ ID NO: 9;
(5A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 9;
(5A-e) a protein comprising an amino acid sequence encoded by a nucleic acid that hybridizes under stringent conditions with a nucleic acid having a nucleotide sequence complementary to the nucleotide sequence of SEQ ID NO: 9;
(5B) a protein comprising an amino acid sequence of SEQ ID NO: 33 or 34, preferably a protein comprising the amino acid sequences of SEQ ID NO: 33 and 34. As referred to above, the amino acid sequence(s) of SEQ ID NOs: 33 and/or 34 may be an amino acid sequence(s) derived therefrom by deletion, substitution, insertion or addition of one or several amino acids.

The proteins of (5A-a) to (5A-e) and (5B) as defined above also include those proteins whose amino acid residues are modified by phosphorylation, sugar chain modification, aminoacylation, ring-opening, deamination or the like.

The proteins of (5A-a) to (5A-e) and (5B) as defined above can be proteins that are found in a protein spot with a molecular weight of around 2 to 30 kDa, preferably around 3 to 25 kDa, more preferably around 5 to 20 kDa and an isoelectric point of 4.0 to 9.0, preferably 5.0 to 7.0 on gels used in the two-dimensional electrophoresis performed under the conditions described above in the subsection titled "Identification of antigens".

Preferably, a protein that is an antigen in spot 5 has activity equivalent to that of the protein having the amino acid sequence of SEQ ID NO: 10 (Protein SLE3) or causative of an allergy to soybean.

### (6) Antigen in spot 6

As the result of sequence identification of the antigen in spot 6 by mass spectroscopy, the following amino acid sequences were detected.
AAAHVVEGAAGYAGHK (SEQ ID NO: 35)
AKDYTLQAAEK (SEQ ID NO: 36)
ARETHELGAHFESLADK (SEQ ID NO: 37)
ATAVGWAAAHFSAEK (SEQ ID NO: 38)
DTPQGSIEALQAGERVK (SEQ ID NO: 39)
DYTLQAAEK (SEQ ID NO: 40)
EEAQRELEAK (SEQ ID NO: 41)
EGTGSIVFTAIGETVSSAGEK (SEQ ID NO: 42)
ERGRESGGQVVAEK (SEQ ID NO: 43)
GLAASAGETAK (SEQ ID NO: 44)
GQQGYAVTK (SEQ ID NO: 45)
KPSQPQEAEERPSEGIGETVRQYAQK (SEQ ID NO: 46)
PAEIVQER (SEQ ID NO: 47)
PQGSIEALQAGERVK (SEQ ID NO: 48)
QAASETLNTTTQTAQEK (SEQ ID NO: 49)
SAGGTTASYVGEK (SEQ ID NO: 50)
SLTSIGEK (SEQ ID NO: 51)
TPQGSIEALQAGERVK (SEQ ID NO: 52)
VTDHAAANVVGNK (SEQ ID NO: 53)

Also, the mass spectroscopic data obtained for spot 6 on a mass spectrometer was analyzed by comparing the data against the Uniprot protein data, and as a result, the antigen in question was identified as Seed biotin-containing protein SBP65 (amino acid sequence: SEQ ID NO: 12, encoding nucleotide sequence: SEQ ID NO: 11).

Accordingly, in the present invention, the antigen in spot 6 can be any of (6A-a) to (6A-e) and (6B) as defined below:
(6A-a) a protein comprising an amino acid sequence with deletion, substitution, insertion or addition of one or several amino acids in SEQ ID NO: 12;
(6A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 12;
(6A-c) a protein comprising an amino acid sequence encoded by a nucleotide sequence with deletion, substitution, insertion or addition of one or several nucleotides in SEQ ID NO: 11;
(6A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 11;
(6A-e) a protein comprising an amino acid sequence encoded by a nucleic acid that hybridizes under stringent conditions with a nucleic acid having a nucleotide sequence complementary to the nucleotide sequence of SEQ ID NO: 11;
(6B) a protein comprising at least one amino acid sequence selected from the group consisting of SEQ ID NOs: 35-53, preferably a protein comprising at least 2, 3, 4, 5, 6, 7, 8, 9, 10, 11, 12, 13, 14, 15, 16, 17 or 18 or all sequences of the amino acid sequences; more preferably a protein comprising at least one amino acid sequence selected from the group consisting of SEQ ID NOs: 35-39, 41-44, 46, 48-50, 52 and 53, preferably a protein comprising at least 2, 3, 4, 5, 6, 7, 8, 9, 10, 11, 12, 13 or 14 or all sequences of the amino acid sequences. As referred to above, the amino acid sequence of any of SEQ ID NOs: 35-53 may be an amino acid sequence derived therefrom by deletion, substitution, insertion or addition of one or several amino acids.

The proteins of (6A-a) to (6A-e) and (6B) as defined above also include those proteins whose amino acid residues are modified by phosphorylation, sugar chain modification, aminoacylation, ring-opening, deamination or the like.

The proteins of (6A-a) to (6A-e) and (6B) as defined above can be proteins that are found in a protein spot with a molecular weight of around 50 to 100 kDa, preferably around 55 to 90 kDa, more preferably around 60 to 75 kDa and an isoelectric point of 5.0 to 10.0, preferably 6.0 to 8.0 on gels used in the two-dimensional electrophoresis performed under the conditions described above in the subsection titled "Identification of antigens".

Preferably, a protein that is an antigen in spot 6 has activity equivalent to that of the protein having the amino acid sequence of SEQ ID NO: 12 (Seed biotin-containing protein SBP65) or causative of an allergy to soybean.

### (7) Antigen in spot 7

As the result of sequence identification of the antigen in spot 7 by mass spectroscopy, the following amino acid sequences were detected.
AASNAPAPLAGVIFEPFQELKK (SEQ ID NO: 54)
ASNAPAPLAGVIFEPFQELKK (SEQ ID NO: 55)
FFKESSEEEREHAEQLIK (SEQ ID NO: 56)
GHGVWHFDQK (SEQ ID NO: 57)
IAEYVAQLRLVGK (SEQ ID NO: 58)
KIAEYVAQLRLVGK (SEQ ID NO: 59)
LLHDEDHV (SEQ ID NO: 60)
SNAPAPLAGVIFEPFQELKK (SEQ ID NO: 61)

Also, the mass spectroscopic data obtained for spot 7 on a mass spectrometer was analyzed by comparing the data against the Uniprot protein data, and as a result, the antigen in question was identified as Ferritin-2 (amino acid sequence: SEQ ID NO: 14, encoding nucleotide sequence: SEQ ID NO: 13).

Accordingly, in the present invention, the antigen in spot 7 can be any of (7A-a) to (7A-e) and (7B) as defined below:
(7A-a) a protein comprising an amino acid sequence with deletion, substitution, insertion or addition of one or several amino acids in SEQ ID NO: 14;
(7A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 14;
(7A-c) a protein comprising an amino acid sequence encoded by a nucleotide sequence with deletion, substitution, insertion or addition of one or several nucleotides in SEQ ID NO: 13;
(7A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 13;
(7A-e) a protein comprising an amino acid sequence encoded by a nucleic acid that hybridizes under stringent conditions with a nucleic acid having a nucleotide sequence complementary to the nucleotide sequence of SEQ ID NO: 13;
(7B) a protein comprising at least one amino acid sequence selected from the group consisting of SEQ ID NOs: 54-61, preferably a protein comprising at least 2, 3, 4, 5, 6 or 7 or all sequences of the amino acid sequences; more preferably a protein comprising at least one amino acid sequence selected from the group consisting of SEQ ID NOs: 54-59 and 61, preferably a protein comprising at least 2, 3, 4, 5 or 6 or all sequences of the amino acid sequences. As referred to above, the amino acid sequence of any of SEQ ID NOs: 54-61 may be an amino acid sequence derived therefrom by deletion, substitution, insertion or addition of one or several amino acids.

The proteins of (7A-a) to (7A-e) and (7B) as defined above also include those proteins whose amino acid residues are modified by phosphorylation, sugar chain modification, aminoacylation, ring-opening, deamination or the like.

The proteins of (7A-a) to (7A-e) and (7B) as defined above can be proteins that are found in a protein spot with a molecular weight of around 5 to 37 kDa, preferably around 10 to 30 kDa, more preferably around 15 to 25 kDa and an isoelectric point of 3.0 to 7.0, preferably 4.0 to 6.0 on gels used in the two-dimensional electrophoresis performed under the conditions described above in the subsection titled "Identification of antigens".

Preferably, a protein that is an antigen in spot 7 has activity equivalent to that of the protein having the amino acid sequence of SEQ ID NO: 14 (Ferritin-2) or causative of an allergy to soybean.

### (8) Antigen in spot 8

As the result of sequence identification of the antigen in spot 8 by mass spectroscopy, the following amino acid sequences were detected.
AGESESNLRK (SEQ ID NO: 62)
GESEANVREIFDK (SEQ ID NO: 63)
GILLYGPPGSGK (SEQ ID NO: 64)
LAGESESNLRK (SEQ ID NO: 65)
LSDDVDLERIAK (SEQ ID NO: 66)
RELQETVQYPVEHPEK (SEQ ID NO: 67)
TVFIIGATNRPDIID (SEQ ID NO: 68)

Also, the mass spectroscopic data obtained for spot 8 on a mass spectrometer was analyzed by comparing the data against the Uniprot protein data, and as a result, the antigen in question was identified as Cell division cycle protein 48 homolog (amino acid sequence: SEQ ID NO: 16, encoding nucleotide sequence: SEQ ID NO: 15).

Accordingly, in the present invention, the antigen in spot 8 can be any of (8A-a) to (8A-e) and (8B) as defined below:
(8A-a) a protein comprising an amino acid sequence with deletion, substitution, insertion or addition of one or several amino acids in SEQ ID NO: 16;
(8A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 16;
(8A-c) a protein comprising an amino acid sequence encoded by a nucleotide sequence with deletion, substitution, insertion or addition of one or several nucleotides in SEQ ID NO: 15;
(8A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 15;
(8A-e) a protein comprising an amino acid sequence encoded by a nucleic acid that hybridizes under stringent conditions with a nucleic acid having a nucleotide sequence complementary to the nucleotide sequence of SEQ ID NO: 15;
(8B) a protein comprising at least one amino acid sequence selected from the group consisting of SEQ ID NOs: 62-68, preferably a protein comprising at least 2, 3, 4, 5 or 6 or all sequences of the amino acid sequences. As referred to above, the amino acid sequence of any of SEQ ID NOs: 62-68 may be an amino acid sequence derived therefrom by deletion, substitution, insertion or addition of one or several amino acids.

The proteins of (8A-a) to (8A-e) and (8B) as defined above also include those proteins whose amino acid residues are modified by phosphorylation, sugar chain modification, aminoacylation, ring-opening, deamination or the like.

The proteins of (8A-a) to (8A-e) and (8B) as defined above can be proteins that are found in a protein spot with a molecular weight of around 75 to 200 kDa, preferably around 100 to 170 kDa, more preferably around 100 to 150 kDa and an isoelectric point of 3.0 to 7.0, preferably 4.0 to 6.0 on gels used in the two-dimensional electrophoresis performed under the conditions described above in the subsection titled "Identification of antigens".

Preferably, a protein that is an antigen in spot 8 has activity equivalent to that of the protein having the amino acid sequence of SEQ ID NO: 16 (Cell division cycle protein 48 homolog) or causative of an allergy to soybean.

### (1α) Antigen in spot 1α

As the result of sequence identification of the antigen in spot 1α by mass spectroscopy, the following amino acid sequences were detected.
APQDLYESYYNANK (SEQ ID NO: 71)
AVLFVPPK (SEQ ID NO: 72)
DFASRIYDSVK (SEQ ID NO: 73)
DIFLRELISNASDALDK (SEQ ID NO: 74)
DIFYITGTSK (SEQ ID NO: 75)
DNVDDVK (SEQ ID NO: 76)
EDSKPETDAVNDDNDVK (SEQ ID NO: 77)
EEAGEYLEESK (SEQ ID NO: 78)
EQLENSPFLERLK (SEQ ID NO: 79)
EVLGEGDNTK (SEQ ID NO: 80)
EVTEEEYTK (SEQ ID NO: 81)
FYHSLAK (SEQ ID NO: 82)
IGAVPHGLSTDSDVVK (SEQ ID NO: 83)
LGIIEDATNRNRLAK (SEQ ID NO: 84)
NPEDEGVK (SEQ ID NO: 85)
SGTSAFVEK (SEQ ID NO: 86)
TSLDISPEATVEEEDDTEVEAESDAK (SEQ ID NO: 87)
APQDLYESYYNANK (SEQ ID NO: 90)
AVLFVPPK (SEQ ID NO: 91)
DFASRIYDSVK (SEQ ID NO: 92)
DIFLRELISNASDALDK (SEQ ID NO: 93)
DIFYITGTSK (SEQ ID NO: 94)
DNVDDVK (SEQ ID NO: 95)
DVLGEGDNTK (SEQ ID NO: 96)
EEAGEYLQESK (SEQ ID NO: 97)
EQLENSPFLERLK (SEQ ID NO: 98)
EVTEEEYTK (SEQ ID NO: 99)
FYHSLAK (SEQ ID NO: 100)
IGAVPHGLSTDSDVVK (SEQ ID NO: 101)
LGIIEDATNRNRLAK (SEQ ID NO: 102)
NPEDEGVK (SEQ ID NO: 103)
SGTSAFVEK (SEQ ID NO: 104)

Also, the mass spectroscopic data obtained for spot 1α on a mass spectrometer was analyzed by comparing the data against the NCBI protein data, and as a result, endoplasmin homolog isoform X2 (amino acid sequence: SEQ ID NO: 70, encoding nucleotide sequence: SEQ ID NO: 69) was identified as a protein comprising any of SEQ ID NOs: 71 to 87 and endoplasmin homolog isoform X2 (amino acid sequence: SEQ ID NO: 89, encoding nucleotide sequence: SEQ ID NO: 88) was identified as a protein comprising any of SEQ ID NOs: 90 to 104.

Accordingly, the antigen in spot 1α in the present invention can be any of proteins selected from the group consisting of the proteins as defined below in (1αA1-a) to (1αA1-e), (1αB1), (1αA2-a) to (1αA2-e), and (1αB2):
(1αA1-a) a protein comprising an amino acid sequence with deletion, substitution, insertion or addition of one or several amino acids in SEQ ID NO: 70;
(1αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 70;
(1αA1-c) a protein comprising an amino acid sequence encoded by a nucleotide sequence with deletion, substitution, insertion or addition of one or several nucleotides in SEQ ID NO: 69;
(1αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 69;
(1αA1-e) a protein comprising an amino acid sequence encoded by a nucleic acid that hybridizes under stringent conditions with a nucleic acid having a nucleotide sequence complementary to the nucleotide sequence of SEQ ID NO: 69;
   (1αB1) a protein comprising at least one amino acid sequence selected from the group consisting of SEQ ID NOs: 71-87, preferably a protein comprising at least 2, 3, 4, 5, 6, 7, 8, 9, 10, 11, 12, 13, 14, 15, or 16 or all sequences of the amino acid sequences. As referred to above, the amino acid sequence of any of SEQ ID NOs: 71 to 87 may be an amino acid sequence derived therefrom by deletion, substitution, insertion or addition of one or several amino acids;
(1αA2-a) a protein comprising an amino acid sequence with deletion, substitution, insertion or addition of one or several amino acids in SEQ ID NO: 89;
(1αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 89;
(1αA2-c) a protein comprising an amino acid sequence encoded by a nucleotide sequence with deletion, substitution, insertion or addition of one or several nucleotides in SEQ ID NO: 88;
(1αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 88;
(1αA2-e) a protein comprising an amino acid sequence encoded by a nucleic acid that hybridizes under stringent conditions with a nucleic acid having a nucleotide sequence complementary to the nucleotide sequence of SEQ ID NO: 88;
   (1αB2) a protein comprising at least one amino acid sequence selected from the group consisting of SEQ ID NOs: 90-104, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 71 to 87 may be an amino acid sequence derived therefrom by deletion, substitution, insertion or addition of one or several amino acids.

The protein selected from the group consisting of the proteins as defined above in (1αA1-a) to (1αA1-e), (1αB1), (1αA2-a) to (1αA2-e), and (1αB2) also include those proteins whose amino acid residues are modified by phosphorylation, sugar chain modification, aminoacylation, ring-opening, deamination or the like.

The protein selected from the group consisting of the proteins as defined above in (1αA1-a) to (1αA1-e), (1αB1), (1αA2-a) to (1αA2-e), and (1αB2) can be proteins that are found in a protein spot with a molecular weight of around 70 to 120 kDa, preferably around 80 to 110 kDa, and an isoelectric point of 2.5 to 6.5, preferably 3.0 to 6.0 on gels used in the two-dimensional electrophoresis performed under the conditions described above in the subsection titled "Identification of antigens".

Preferably, a protein that is an antigen in spot 1α has activity equivalent to that of the protein having the amino acid sequence of SEQ ID NO: 70 or 89 (endoplasmin homolog isoform X2) or causative of an allergy to soybean.

### (2α) Antigen in spot 2α

As the result of sequence identification of the antigen in spot 2α by mass spectroscopy, the following amino acid sequences were detected.
ADLVNNLGTIARSGTK (SEQ ID NO: 107)
AVENSPFLEK (SEQ ID NO: 108)
EDQLEYLEERRLK (SEQ ID NO: 109)
EEYAAFYK (SEQ ID NO: 110)
EGQNDIYYITGESK (SEQ ID NO: 111)
EIFLRELISNASDALDK (SEQ ID NO: 112)
FYEAFSK (SEQ ID NO: 113)
HFSVEGQLEFK (SEQ ID NO: 114)
IRFESLTDK (SEQ ID NO: 115)
LAELLRYHSTK (SEQ ID NO: 116)
LDESEDEK (SEQ ID NO: 117)
LGIHEDSQNK (SEQ ID NO: 118)
RAPFDLFDTRK (SEQ ID NO: 119)
VEDVDEDK (SEQ ID NO: 120)
ADLVNNLGTIARSGTK (SEQ ID NO: 123)
AVENSPFLEK (SEQ ID NO: 124)
EDQLEYLEERRLK (SEQ ID NO: 125)
EEYSAFYK (SEQ ID NO: 126)
EGQSDIYYITGESK (SEQ ID NO: 127)
EIFLRELISNASDALDK (SEQ ID NO: 128)
FYEAFSK (SEQ ID NO: 129)
HFSVEGQLEFK (SEQ ID NO: 130)
IAELLRYHSTK (SEQ ID NO: 131)
IRFESLTDK (SEQ ID NO: 132)
LDESEDEK (SEQ ID NO: 133)
LGIHEDSQNK (SEQ ID NO: 134)
RAPFDLFDTRK (SEQ ID NO: 135)
SGDELTSLK (SEQ ID NO: 136)
VEDVDEEK (SEQ ID NO: 137)
ADLVNNLGTIARSGTK (SEQ ID NO: 140)
AVENSPFLEK (SEQ ID NO: 141)
EDQLEYLEERRLK (SEQ ID NO: 142)
EEYAAFYK (SEQ ID NO: 143)
EGQNDIYYITGESK (SEQ ID NO: 144)
EIFLRELISNASDALDK (SEQ ID NO: 145)
FYEAFSK (SEQ ID NO: 146)
HFSVEGQLEFK (SEQ ID NO: 147)
IRFESLTDK (SEQ ID NO: 148)
LAELLRYHSTK (SEQ ID NO: 149)
LDESEDEK (SEQ ID NO: 150)
LGIHEDSQNK (SEQ ID NO: 151)
RAPFDLFDTK (SEQ ID NO: 152)
VEDVDEDK (SEQ ID NO: 153)

Also, the mass spectroscopic data obtained for spot 2α on a mass spectrometer was analyzed by comparing the data against the NCBI protein data, and as a result, heat shock cognate protein 80 (amino acid sequence: SEQ ID NO: 106, encoding nucleotide sequence: SEQ ID NO: 105) was identified as a protein comprising any of SEQ ID NOs: 107 to 120, heat shock protein 90-1 (amino acid sequence: SEQ ID NO: 122, encoding nucleotide sequence: SEQ ID NO: 121) was identified as a protein comprising any of SEQ ID NOs: 123 to 137, and hypothetical protein GLYMA_02G302500 was identified as a protein comprising any of SEQ ID NOs: 140 to 153.

Accordingly, the antigen in spot 1α in the present invention can be any of proteins selected from the group consisting of the proteins as defined below in (2αA1-a) to (2αA1-e), (2αB1), (2αA2-a) to (2αA2-e), (2αB2), (2αA3-a) to (2αA3-e), and (2αB3):
(2αA1-a) a protein comprising an amino acid sequence with deletion, substitution, insertion or addition of one or several amino acids in SEQ ID NO: 106;
(2αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 106;
(2αA1-c) a protein comprising an amino acid sequence encoded by a nucleotide sequence with deletion, substitution, insertion or addition of one or several nucleotides in SEQ ID NO: 105;
(2αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 105;
(2αA1-e) a protein comprising an amino acid sequence encoded by a nucleic acid that hybridizes under stringent conditions with a nucleic acid having a nucleotide sequence complementary to the nucleotide sequence of SEQ ID NO: 105;
(2αB1) a protein comprising at least one amino acid sequence selected from the group consisting of SEQ ID NOs: 107-120, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 107 to 120 may be an amino acid sequence derived therefrom by deletion, substitution, insertion or addition of one or several amino acids;
(2αA2-a) a protein comprising an amino acid sequence with deletion, substitution, insertion or addition of one or several amino acids in SEQ ID NO: 122;
(2αA2-b) a protein comprising an amino acid sequence having at least 70%, preferably at least 75%, at least 80%, at least 85%, at least 90%, at least 95%, at least 97%, at least 98%,
or at least 99% identity to the amino acid sequence of SEQ ID NO: 122;
(2αA2-c) a protein comprising an amino acid sequence encoded by a nucleotide sequence with deletion, substitution, insertion or addition of one or several nucleotides in SEQ ID NO: 121;
(2αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 121;
(2αA2-e) a protein comprising an amino acid sequence encoded by a nucleic acid that hybridizes under stringent conditions with a nucleic acid having a nucleotide sequence complementary to the nucleotide sequence of SEQ ID NO: 121;
(2αB2) a protein comprising at least one amino acid sequence selected from the group consisting of SEQ ID NOs: 123-137, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 123 to 137 may be an amino acid sequence derived therefrom by deletion, substitution, insertion or addition of one or several amino acids.
(2αA3-a) a protein comprising an amino acid sequence with deletion, substitution, insertion or addition of one or several amino acids in SEQ ID NO: 139;
(2αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 139;
(2αA3-c) a protein comprising an amino acid sequence encoded by a nucleotide sequence with deletion, substitution, insertion or addition of one or several nucleotides in SEQ ID NO: 138;
(2αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 138;
(2αA3-e) a protein comprising an amino acid sequence encoded by a nucleic acid that hybridizes under stringent conditions with a nucleic acid having a nucleotide sequence complementary to the nucleotide sequence of SEQ ID NO: 138;
(2αB3) a protein comprising at least one amino acid sequence selected from the group consisting of SEQ ID NOs: 140-153, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 140 to 153 may be an amino acid sequence derived therefrom by deletion, substitution, insertion or addition of one or several amino acids.

The protein selected from the group consisting of the proteins as defined above in (2αA1-a) to (2αA1-e), (2αB1), (2αA2-a) to (2αA2-e), (2αB2), (2αA3-a) to (2αA3-e), and (2αB3) also include those proteins whose amino acid residues are modified by phosphorylation, sugar chain modification, aminoacylation, ring-opening, deamination or the like.

The protein selected from the group consisting of the proteins as defined above in (2αA1-a) to (2αA1-e), (2αB1), (2αA2-a) to (2αA2-e), (2αB2), (2αA3-a) to (2αA3-e), and (2αB3) can be proteins that are found in a protein spot with a molecular weight of around 50 to 120 kDa, preferably around 60 to 110 kDa, and an isoelectric point of 2.5 to 6.5, preferably 3.0 to 6.0 on gels used in the two-dimensional electrophoresis performed under the conditions described above in the subsection titled "Identification of antigens".

Preferably, a protein that is an antigen in spot 2α has activity equivalent to that of the protein having the amino acid sequence of SEQ ID NO: 106 (heat shock cognate protein 80), the protein having the amino acid sequence of SEQ ID NO: 122 (heat shock protein 90-1), or the protein having the amino acid sequence of SEQ ID NO: 139 (hypothetical protein GLYMA_02G302500), or causative of an allergy to soybean.

### (4α) Antigen in spot 4α

As the result of sequence identification of the antigen in spot 4α by mass spectroscopy, the following amino acid sequences were detected.
AGGALSIAFVSK (SEQ ID NO: 156)
DGLIVSIGK (SEQ ID NO: 157)

Also, the mass spectroscopic data obtained for spot 4α on a mass spectrometer was analyzed by comparing the data against the NCBI protein data, and as a result, embryo-specific urease isoform X1 (amino acid sequence: SEQ ID NO: 155, encoding nucleotide sequence: SEQ ID NO: 154) was identified as a protein comprising SEQ ID NO: 156 or 157.

Accordingly, the antigens in spot 4α in the present invention can be any protein selected from the group consisting of the proteins as defined below in (4αA-a) to (4αA-e) and (4αB):
(4αA-a) a protein comprising an amino acid sequence with deletion, substitution, insertion or addition of one or several amino acids in SEQ ID NO: 155;
(4αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 155;
(4αA-c) a protein comprising an amino acid sequence encoded by a nucleotide sequence with deletion, substitution, insertion or addition of one or several nucleotides in SEQ ID NO: 154;
(4αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 154;
(4αA-e) a protein comprising an amino acid sequence encoded by a nucleic acid that hybridizes under stringent conditions with a nucleic acid having a nucleotide sequence complementary to the nucleotide sequence of SEQ ID NO: 154;
(4αB) a protein comprising at least one amino acid sequence selected from the group consisting of SEQ ID NOs: 156 and 157, preferably a protein comprising both the amino acid sequences. As referred to above, the amino acid sequence of any of SEQ ID NOs: 156 and 157 may be an amino acid sequence derived therefrom by deletion, substitution, insertion or addition of one or several amino acids.

The proteins selected from the group consisting of (4αA-a) to (4αA-e) and (4αB) as defined above also include those proteins whose amino acid residues are modified by phosphorylation, sugar chain modification, aminoacylation, ring-opening, deamination or the like.

The proteins selected from the group consisting of (4αA-a) to (4αA-e) and (4αB) as defined above can be proteins that are found in a protein spot with a molecular weight of around 70 to 120 kDa, preferably around 80 to 110 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 4α has activity equivalent to that of the protein having the amino acid sequence of SEQ ID NO: 155 (embryo-specific urease isoform X1) or causative of an allergy to soybean.

### (5α) Antigen in spot 5α

As the result of sequence identification of the antigen in spot 5α by mass spectroscopy, the following amino acid sequences were detected.
AFATYVQAK (SEQ ID NO: 160)
QQEATTSLSSFTPDASSIASSIK (SEQ ID NO: 161)

Also, the mass spectroscopic data obtained for spot 5α on a mass spectrometer was analyzed by comparing the data against the NCBI protein data, and as a result, alpha-1,4 glucan phosphorylase L isozyme, chloroplastic/amyloplastic (amino acid sequence: SEQ ID NO: 159, encoding nucleotide sequence: SEQ ID NO: 158) was identified as a protein comprising SEQ ID NO: 160 or 161.

Accordingly, the antigens in spot 5α in the present invention can be any protein selected from the group consisting of the proteins as defined below in (5αA-a) to (5αA-e) and (5αB):
(5αA-a) a protein comprising an amino acid sequence with deletion, substitution, insertion or addition of one or several amino acids in SEQ ID NO: 159;
(5αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 159;
(5αA-c) a protein comprising an amino acid sequence encoded by a nucleotide sequence with deletion, substitution, insertion or addition of one or several nucleotides in SEQ ID NO: 158;
(5αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 158;
(5αA-e) a protein comprising an amino acid sequence encoded by a nucleic acid that hybridizes under stringent conditions with a nucleic acid having a nucleotide sequence complementary to the nucleotide sequence of SEQ ID NO: 158;
(5αB) a protein comprising at least one amino acid sequence selected from the group consisting of SEQ ID NOs: 160 and 161, preferably a protein comprising both the amino acid sequences. As referred to above, the amino acid sequence of any of SEQ ID NOs: 160 and 161 may be an amino acid sequence derived therefrom by deletion, substitution, insertion or addition of one or several amino acids.

The proteins selected from the group consisting of (5αA-a) to (5αA-e) and (5αB) as defined above also include those proteins whose amino acid residues are modified by phosphorylation, sugar chain modification, aminoacylation, ring-opening, deamination or the like.

The proteins selected from the group consisting of (5αA-a) to (5αA-e) and (5αB) as defined above can be proteins that are found in a protein spot with a molecular weight of around 70 to 170 kDa, preferably around 80 to 160 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 5α has activity equivalent to that of the protein having the amino acid sequence of SEQ ID NO: 159 (alpha-1,4 glucan phosphorylase L isozyme, chloroplastic/amyloplastic) or causative of an allergy to soybean.

### (6α) Antigen in spot 6α

As the result of sequence identification of the antigen in spot 6α by mass spectroscopy, the following amino acid sequences were detected.
FLNGEVGPK (SEQ ID NO: 164)
LSVFDAAEK (SEQ ID NO: 165)
LSVFDVAEK (SEQ ID NO: 166)
TSLAPGSGVVTK (SEQ ID NO: 167)
FLNGEVGPK (SEQ ID NO: 170)
LSVFDVAEK (SEQ ID NO: 171)
TSLAPGSGVVTK (SEQ ID NO: 172)

Also, the mass spectroscopic data obtained for spot 6α on a mass spectrometer was analyzed by comparing the data against the NCBI protein data, and as a result, aconitate hydratase 1 (amino acid sequence: SEQ ID NO: 163, encoding nucleotide sequence: SEQ ID NO: 162) was identified as a protein comprising any of SEQ ID NOs: 164 to 167 and aconitate hydratase 1 (amino acid sequence: SEQ ID NO: 169, encoding nucleotide sequence: SEQ ID NO: 168) was identified as a protein comprising any of SEQ ID NOs: 170 to 172.

Accordingly, the antigen in spot 6α in the present invention can be any of proteins selected from the group consisting of the proteins as defined below in (6αA1-a) to (6αA1-e), (6αB1), (6αA2-a) to (6αA2-e), and (6αB2):
(6αA1-a) a protein comprising an amino acid sequence with deletion, substitution, insertion or addition of one or several amino acids in SEQ ID NO: 163;
(6αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 163;
(6αA1-c) a protein comprising an amino acid sequence encoded by a nucleotide sequence with deletion, substitution, insertion or addition of one or several nucleotides in SEQ ID NO: 162;
(6αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 162;
(6αA1-e) a protein comprising an amino acid sequence encoded by a nucleic acid that hybridizes under stringent conditions with a nucleic acid having a nucleotide sequence complementary to the nucleotide sequence of SEQ ID NO: 162;
(6αB1) a protein comprising at least one amino acid sequence selected from the group consisting of SEQ ID NOs: 164-167, preferably a protein comprising at least 2, 3, or all sequences of the amino acid sequences. As referred to above, the amino acid sequence of any of SEQ ID NOs: 164 to 167 may be an amino acid sequence derived therefrom by deletion, substitution, insertion or addition of one or several amino acids;
(6αA2-a) a protein comprising an amino acid sequence with deletion, substitution, insertion or addition of one or several amino acids in SEQ ID NO: 169;
(6αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 169;
(6αA2-c) a protein comprising an amino acid sequence encoded by a nucleotide sequence with deletion, substitution, insertion or addition of one or several nucleotides in SEQ ID NO: 168;
(6αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 168;
(6αA2-e) a protein comprising an amino acid sequence encoded by a nucleic acid that hybridizes under stringent conditions with a nucleic acid having a nucleotide sequence complementary to the nucleotide sequence of SEQ ID NO: 168;
(6αB2) a protein comprising at least one amino acid sequence selected from the group consisting of SEQ ID NOs: 170-172, preferably a protein comprising at least 2, or all sequences of the amino acid sequences. As referred to above, the amino acid sequence of any of SEQ ID NOs: 170 to 172 may be an amino acid sequence derived therefrom by deletion, substitution, insertion or addition of one or several amino acids.

The protein selected from the group consisting of the proteins as defined above in (6αA1-a) to (6αA1-e), (6αB1), (6αA2-a) to (6αA2-e), and (6αB2) also include those proteins whose amino acid residues are modified by phosphorylation, sugar chain modification, aminoacylation, ring-opening, deamination or the like.

The protein selected from the group consisting of the proteins as defined above in (6αA1-a) to (6αA1-e), (6αB1), (6αA2-a) to (6αA2-e), and (6αB2) can be proteins that are found in a protein spot with a molecular weight of around 70 to 120 kDa, preferably around 80 to 110 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 6α has activity equivalent to that of the protein having the amino acid sequence of SEQ ID NO: 163 or 169 (aconitate hydratase 1) or causative of an allergy to soybean.

### (7α) Antigen in spot 7α

As the result of sequence identification of the antigen in spot 7α by mass spectroscopy, the following amino acid sequences were detected.
AGITVIQIDEAALREGLPLRK (SEQ ID NO: 175)
ALGVDTVPVLVGPVTYLLLSKPAK (SEQ ID NO: 176)
DEAFFSGNAAALASRK (SEQ ID NO: 177)
DEVEDLEK (SEQ ID NO: 178)
ISEEEYVK (SEQ ID NO: 179)
IVEVNALAK (SEQ ID NO: 180)
LIRNELAK (SEQ ID NO: 181)
LLSVFREGVK (SEQ ID NO: 182)
LVVSTSSSLLHTAVDLVNETK (SEQ ID NO: 183)
SFSLLSLLPK (SEQ ID NO: 184)
SSPRVTNEAVQK (SEQ ID NO: 185)
TLTSLNGVTAYGFDLVRGTNTLDLIK (SEQ ID NO: 186)
YGAGIGPGVYDIHSPRIPPTEEIADRINK (SEQ ID NO: 187)

Also, the mass spectroscopic data obtained for spot 7α on a mass spectrometer was analyzed by comparing the data against the NCBI protein data, and as a result, methionine synthase (amino acid sequence: SEQ ID NO: 174, encoding nucleotide sequence: SEQ ID NO: 173) was identified as a protein comprising any of SEQ ID NOs: 175 to 187.

Accordingly, the antigens in spot 7α in the present invention can be any protein selected from the group consisting of the proteins as defined below in (7αA-a) to (7αA-e) and (7αB):
(7αA-a) a protein comprising an amino acid sequence with deletion, substitution, insertion or addition of one or several amino acids in SEQ ID NO: 174;
(7αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 174;
(7αA-c) a protein comprising an amino acid sequence encoded by a nucleotide sequence with deletion, substitution, insertion or addition of one or several nucleotides in SEQ ID NO: 173;
(7αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 173;
(7αA-e) a protein comprising an amino acid sequence encoded by a nucleic acid that hybridizes under stringent conditions with a nucleic acid having a nucleotide sequence complementary to the nucleotide sequence of SEQ ID NO: 173;
(7αB) a protein comprising at least one amino acid sequence selected from the group consisting of SEQ ID NOs: 175-187, preferably a protein comprising at least 2, 3, 4, 5, 6, 7, 8, 9, 10, 11 or 12 or all sequences of the amino acid sequences. As referred to above, the amino acid sequence of any of SEQ ID NOs: 175-187 may be an amino acid sequence derived therefrom by deletion, substitution, insertion or addition of one or several amino acids.

The proteins selected from the group consisting of (7αA-a) to (7αA-e) and (7αB) as defined above also include those proteins whose amino acid residues are modified by phosphorylation, sugar chain modification, aminoacylation, ring-opening, deamination or the like.

The proteins selected from the group consisting of (7αA-a) to (7αA-e) and (7αB) as defined above can be proteins that are found in a protein spot with a molecular weight of around 50 to 120 kDa, preferably around 60 to 110 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 7α has activity equivalent to that of the protein having the amino acid sequence of SEQ ID NO: 174 (methionine synthase) or causative of an allergy to soybean.

### (8α) Antigen in spot 8α

As the result of sequence identification of the antigen in spot 8α by mass spectroscopy, the following amino acid sequences were detected.
AIGIDRFGASAPAGRIYK (SEQ ID NO: 190)
ALPTYTPESPADATRNLSQTNLNALAK (SEQ ID NO: 191)
ANSYSVHGSALGAK (SEQ ID NO: 192)
ATADAALVEK (SEQ ID NO: 193)
DKPTLIK (SEQ ID NO: 194)
GGYTISDNSTGNKPDVILIGTGSELEIAAK (SEQ ID NO: 195)
LPQLPGTSIEGVEK (SEQ ID NO: 196)
NGNNGYDDIRAAIK (SEQ ID NO: 197)
SVNTIRFLAIDAVEK (SEQ ID NO: 198)
VTTTIGYGSPNK (SEQ ID NO: 199)

Also, the mass spectroscopic data obtained for spot 8α on a mass spectrometer was analyzed by comparing the data against the NCBI protein data, and as a result, transketolase, chloroplastic (amino acid sequence: SEQ ID NO: 189, encoding nucleotide sequence: SEQ ID NO: 188) was identified as a protein comprising any of SEQ ID NOs: 190 to 199.

Accordingly, the antigens in spot 8α in the present invention can be any protein selected from the group consisting of the proteins as defined below in (8αA-a) to (8αA-e) and (8αB):
(8αA-a) a protein comprising an amino acid sequence with deletion, substitution, insertion or addition of one or several amino acids in SEQ ID NO: 189;
(8αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 189;
(8αA-c) a protein comprising an amino acid sequence encoded by a nucleotide sequence with deletion, substitution, insertion or addition of one or several nucleotides in SEQ ID NO: 188;
(8αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 188;
(8αA-e) a protein comprising an amino acid sequence encoded by a nucleic acid that hybridizes under stringent conditions with a nucleic acid having a nucleotide sequence complementary to the nucleotide sequence of SEQ ID NO: 188;
(8αB) a protein comprising at least one amino acid sequence selected from the group consisting of SEQ ID NOs: 190-199, preferably a protein comprising at least 2, 3, 4, 5, 6, 7, 8 or 9 or all sequences of the amino acid sequences. As referred to above, the amino acid sequence of any of SEQ ID NOs: 190-199 may be an amino acid sequence derived therefrom by deletion, substitution, insertion or addition of one or several amino acids.

The proteins selected from the group consisting of (8αA-a) to (8αA-e) and (8αB) as defined above also include those proteins whose amino acid residues are modified by phosphorylation, sugar chain modification, aminoacylation, ring-opening, deamination or the like.

The proteins selected from the group consisting of (8αA-a) to (8αA-e) and (8αB) as defined above can be proteins that are found in a protein spot with a molecular weight of around 50 to 120 kDa, preferably around 60 to 110 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 8α has activity equivalent to that of the protein having the amino acid sequence of SEQ ID NO: 189 (transketolase, chloroplastic) or causative of an allergy to soybean.

### (9α) Antigen in spot 9α

As the result of sequence identification of the antigen in spot 9α by mass spectroscopy, the following amino acid sequences were detected.
DLSSEEANQYLK (SEQ ID NO: 202)

Also, the mass spectroscopic data obtained for spot 9α on a mass spectrometer was analyzed by comparing the data against the NCBI protein data, and as a result, lysine-tRNA ligase, cytoplasmic-like isoform X2 (amino acid sequence: SEQ ID NO: 201, encoding nucleotide sequence: SEQ ID NO: 200) was identified as a protein comprising SEQ ID NO: 202.

Accordingly, the antigens in spot 9α in the present invention can be any protein selected from the group consisting of the proteins as defined below in (9αA-a) to (9αA-e) and (9αB):
(9αA-a) a protein comprising an amino acid sequence with deletion, substitution, insertion or addition of one or several amino acids in SEQ ID NO: 201;
(9αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 201;
(9αA-c) a protein comprising an amino acid sequence encoded by a nucleotide sequence with deletion, substitution, insertion or addition of one or several nucleotides in SEQ ID NO: 200;
(9αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 200;
(9αA-e) a protein comprising an amino acid sequence encoded by a nucleic acid that hybridizes under stringent conditions with a nucleic acid having a nucleotide sequence complementary to the nucleotide sequence of SEQ ID NO: 200;
(9αB) a protein comprising an amino acid sequence of SEQ ID NO: 202. As referred to above, the amino acid sequence(s) of SEQ ID NO: 202 may be an amino acid sequence(s) derived therefrom by deletion, substitution, insertion or addition of one or several amino acids.

The proteins selected from the group consisting of (9αA-a) to (9αA-e) and (9αB) as defined above also include those proteins whose amino acid residues are modified by phosphorylation, sugar chain modification, aminoacylation, ring-opening, deamination or the like.

The proteins selected from the group consisting of (9αA-a) to (9αA-e) and (9αB) as defined above can be proteins that are found in a protein spot with a molecular weight of around 50 to 120 kDa, preferably around 60 to 110 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 9α has activity equivalent to that of the protein having the amino acid sequence of SEQ ID NO: 201 (lysine-tRNA ligase, cytoplasmic-like isoform X2) or causative of an allergy to soybean.

### (10α) Antigen in spot 10α

As the result of sequence identification of the antigen in spot 10α by mass spectroscopy, the following amino acid sequences were detected.
AAVEEGIVPGGGVALLYASSELDK (SEQ ID NO: 205)
DRVTDALNATK (SEQ ID NO: 206)
EGVITISDGK (SEQ ID NO: 207)
GRNVVIEQSFGAPK (SEQ ID NO: 208)
GVEELADAVK (SEQ ID NO: 209)
IGGASEAEVGEK (SEQ ID NO: 210)
IGVQIIQNALK (SEQ ID NO: 211)
ISSINAIVK (SEQ ID NO: 212)
LLEQNDPDLGYDAAK (SEQ ID NO: 213)
LQTANFDQK (SEQ ID NO: 214)
LSGGVAVLK (SEQ ID NO: 215)
NIGASLVK (SEQ ID NO: 216)
TGIIDPLK (SEQ ID NO: 217)
TPVHTIASNAGVEGAVVVGK (SEQ ID NO: 218)
AGIIDPLK (SEQ ID NO: 221)
DRVTDALNATK (SEQ ID NO: 222)
EGVITISDGK (SEQ ID NO: 223)
GRNVVIEQSFGAPK (SEQ ID NO: 224)
GVEELADAVK (SEQ ID NO: 225)
IGGASEAEVGEK (SEQ ID NO: 226)
IGVQIIQNALK (SEQ ID NO: 227)
ISSINAIVK (SEQ ID NO: 228)
LLEQENHDLGYDAAK (SEQ ID NO: 229)
LQTANFDQK (SEQ ID NO: 230)
LSGGVAVLK (SEQ ID NO: 231)

Also, the mass spectroscopic data obtained for spot 10α on a mass spectrometer was analyzed by comparing the data against the NCBI protein data, and as a result, chaperonin CPN60-2, mitochondrial (amino acid sequence: SEQ ID NO: 204, encoding nucleotide sequence: SEQ ID NO: 203) was identified as a protein comprising any of SEQ ID NOs: 205 to 218 and chaperonin CPN60-2, mitochondrial-like isoform X1 (amino acid sequence: SEQ ID NO: 220, encoding nucleotide sequence: SEQ ID NO: 219) was identified as a protein comprising any of SEQ ID NOs: 221 to 231.

Accordingly, the antigen in spot 10α in the present invention can be any of proteins selected from the group consisting of the proteins as defined below in (10αA1-a) to (10αA1-e), (10αB1), (10αA2-a) to (10αA2-e), and (10αB2):
(10αA1-a) a protein comprising an amino acid sequence with deletion, substitution, insertion or addition of one or several amino acids in SEQ ID NO: 204;
(10αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 204;
(10αA1-c) a protein comprising an amino acid sequence encoded by a nucleotide sequence with deletion, substitution, insertion or addition of one or several nucleotides in SEQ ID NO: 203;
(10αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 203;
(10αA1-e) a protein comprising an amino acid sequence encoded by a nucleic acid that hybridizes under stringent conditions with a nucleic acid having a nucleotide sequence complementary to the nucleotide sequence of SEQ ID NO: 203;
(110αB1 a protein comprising at least one amino acid sequence selected from the group consisting of SEQ ID NOs: 205-218, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 205 to 218 may be an amino acid sequence derived therefrom by deletion, substitution, insertion or addition of one or several amino acids;
(10αA2-a) a protein comprising an amino acid sequence with deletion, substitution, insertion or addition of one or several amino acids in SEQ ID NO: 220;
(10αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 220;
(10αA2-c) a protein comprising an amino acid sequence encoded by a nucleotide sequence with deletion, substitution, insertion or addition of one or several nucleotides in SEQ ID NO: 219;
(10αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 219;
(10αA2-e) a protein comprising an amino acid sequence encoded by a nucleic acid that hybridizes under stringent conditions with a nucleic acid having a nucleotide sequence complementary to the nucleotide sequence of SEQ ID NO: 219;
(10αB2) a protein comprising at least one amino acid sequence selected from the group consisting of SEQ ID NOs: 221-231, preferably a protein comprising at least 2, 3, 4, 5, 6, 7, 8, 9, or 10, or all sequences of the amino acid sequences. As referred to above, the amino acid sequence of any of SEQ ID NOs: 221 to 231 may be an amino acid sequence derived therefrom by deletion, substitution, insertion or addition of one or several amino acids.

The protein selected from the group consisting of the proteins as defined above in (10αA1-a) to (10αA1-e), (10αB1), (10αA2-a) to (10αA2-e), and (10αB2) also include those proteins whose amino acid residues are modified by phosphorylation, sugar chain modification, aminoacylation, ring-opening, deamination or the like.

The protein selected from the group consisting of the proteins as defined above in (10αA1-a) to (10αA1-e), (10αB1), (10αA2-a) to (10αA2-e), and (10αB2) can be proteins that are found in a protein spot with a molecular weight of around 30 to 90 kDa, preferably around 40 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 10α has activity equivalent to that of the protein having the amino acid sequence of SEQ ID NO: 204 (haperonin CPN60-2, mitochondrial) or the protein having the amino acid sequence of SEQ ID NO: 220 (chaperonin CPN60-2, mitochondrial-like isoform X1) or causative of an allergy to soybean.

### (11α) Antigen in spot 11α

As the result of sequence identification of the antigen in spot 11α by mass spectroscopy, the following amino acid sequences were detected.
AIDVLNFTPLNGK (SEQ ID NO: 234)
FNNVFVK (SEQ ID NO: 235)
GFGFVNFANVDDAAK (SEQ ID NO: 236)
SGAANVFIK (SEQ ID NO: 237)

Also, the mass spectroscopic data obtained for spot 11α on a mass spectrometer was analyzed by comparing the data against the NCBI protein data, and as a result, polyadenylate-binding protein 8 isoform X3 (amino acid sequence: SEQ ID NO: 233, encoding nucleotide sequence: SEQ ID NO: 232) was identified as a protein comprising any of SEQ ID NOs: 234 to 237.

Accordingly, the antigens in spot 11α in the present invention can be any protein selected from the group consisting of the proteins as defined below in (11αA-a) to (11αA-e) and (11αB):
(11αA-a) a protein comprising an amino acid sequence with deletion, substitution, insertion or addition of one or several amino acids in SEQ ID NO: 233;
(11αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 233;
(11αA-c) a protein comprising an amino acid sequence encoded by a nucleotide sequence with deletion, substitution, insertion or addition of one or several nucleotides in SEQ ID NO: 232;
(11αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 232;
(11αA-e) a protein comprising an amino acid sequence encoded by a nucleic acid that hybridizes under stringent conditions with a nucleic acid having a nucleotide sequence complementary to the nucleotide sequence of SEQ ID NO: 232;
(11αB) a protein comprising at least one amino acid sequence selected from the group consisting of SEQ ID NOs: 234-237, preferably a protein comprising at least 2 or 3 or all sequences of the amino acid sequences. As referred to above, the amino acid sequence of any of SEQ ID NOs: 234-237 may be an amino acid sequence derived therefrom by deletion, substitution, insertion or addition of one or several amino acids.

The proteins selected from the group consisting of (11αA-a) to (11αA-e) and (11αB) as defined above also include those proteins whose amino acid residues are modified by phosphorylation, sugar chain modification, aminoacylation, ring-opening, deamination or the like.

The proteins selected from the group consisting of (11αA-a) to (11αA-e) and (11αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 11α has activity equivalent to that of the protein having the amino acid sequence of SEQ ID NO: 233 (polyadenylate-binding protein 8 isoform X3) or causative of an allergy to soybean.

### (12α) Antigen in spot 12α

As the result of sequence identification of the antigen in spot 12α by mass spectroscopy, the following amino acid sequences were detected.
VVGVPVTLNGDLK (SEQ ID NO: 240)

Also, the mass spectroscopic data obtained for spot 12α on a mass spectrometer was analyzed by comparing the data against the NCBI protein data, and as a result, pyrophosphate-fructose 6-phosphate 1-phosphotransferase subunit alpha-like (amino acid sequence: SEQ ID NO: 239, encoding nucleotide sequence: SEQ ID NO: 238) was identified as a protein comprising SEQ ID NO: 240.

Accordingly, the antigens in spot 12α in the present invention can be any protein selected from the group consisting of the proteins as defined below in (12αA-a) to (12αA-e) and (12αB):
(12αA-a) a protein comprising an amino acid sequence with deletion, substitution, insertion or addition of one or several amino acids in SEQ ID NO: 239;
(12αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 239;
(12αA-c) a protein comprising an amino acid sequence encoded by a nucleotide sequence with deletion, substitution, insertion or addition of one or several nucleotides in SEQ ID NO: 238;
(12αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 238;
(12αA-e) a protein comprising an amino acid sequence encoded by a nucleic acid that hybridizes under stringent conditions with a nucleic acid having a nucleotide sequence complementary to the nucleotide sequence of SEQ ID NO: 238;
(12αB) a protein comprising an amino acid sequence of SEQ ID NO: 240. As referred to above, the amino acid sequence(s) of SEQ ID NO: 240 may be an amino acid sequence(s) derived therefrom by deletion, substitution, insertion or addition of one or several amino acids.

The proteins selected from the group consisting of (12αA-a) to (12αA-e) and (12αB) as defined above also include those proteins whose amino acid residues are modified by phosphorylation, sugar chain modification, aminoacylation, ring-opening, deamination or the like.

The proteins selected from the group consisting of (12αA-a) to (12αA-e) and (12αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 12α has activity equivalent to that of the protein having the amino acid sequence of SEQ ID NO: 239 (pyrophosphate-fructose 6-phosphate 1-phosphotransferase subunit alpha-like) or causative of an allergy to soybean.

### (13α) Antigen in spot 13α

As the result of sequence identification of the antigen in spot 13a by mass spectroscopy, the following amino acid sequences were detected.
AASILSSHDPPVVLAK (SEQ ID NO: 243)
DGNVAPFVK (SEQ ID NO: 244)
DLASQYDVRGYPTIK (SEQ ID NO: 245)
EDIIEFIEK (SEQ ID NO: 246)
EEQVPLIIIQHNDGK (SEQ ID NO: 247)
EFVLTLDHSNFHDTVSK (SEQ ID NO: 248)
FFKPNLEADHIPTWLK (SEQ ID NO: 249)
FFNSPNAK (SEQ ID NO: 250)
FSGEEFDNFSALAEK (SEQ ID NO: 251)
FVEESSTPVVTVFNNDPSNHPFVAK (SEQ ID NO: 252)
AASILSSHDPPIVLAK (SEQ ID NO: 255)
DGHVAPFVK (SEQ ID NO: 256)
DLASQYDVK (SEQ ID NO: 257)
EDIIEFIEK (SEQ ID NO: 258)
EEQVPLIIIQHNDGK (SEQ ID NO: 259)
EFVLTLDHSNFHDTVSK (SEQ ID NO: 260)
FFKPNLEADHIPTWLK (SEQ ID NO: 261)
FFNSPNAK (SEQ ID NO: 262)
FSGEEFDNFSALAEK (SEQ ID NO: 263)
FVEESSTPVVTVFNNEPSNHPFVVK (SEQ ID NO: 264)
GFPTINILRNGGK (SEQ ID NO: 265)
GPREADGIVDYLK (SEQ ID NO: 266)
LAPEYEK (SEQ ID NO: 267)
LDATANDIPSETFDVQGYPTVYFRSASGK (SEQ ID NO: 268)
LRSDYDFGHTLNAK (SEQ ID NO: 269)
LSQYDGGRTK (SEQ ID NO: 270)
QLAPILDEVAISYQNEADVVIAK (SEQ ID NO: 271)
QQGVSFLVGDVESSQGAFQYFGLK (SEQ ID NO: 272)
QSGPASTEIK (SEQ ID NO: 273)
SADEATAFIGENK (SEQ ID NO: 274)
SEPIPETNDEPVK (SEQ ID NO: 275)
VAIVGVFPK (SEQ ID NO: 276)
VVVGASLEDIVFK (SEQ ID NO: 277)
YREAAEQYK (SEQ ID NO: 278)

Also, the mass spectroscopic data obtained for spot 13α on a mass spectrometer was analyzed by comparing the data against the NCBI protein data, and as a result, protein disulfide isomerase-like protein precursor (amino acid sequence: SEQ ID NO: 242, encoding nucleotide sequence: SEQ ID NO: 241) was identified as a protein comprising any of SEQ ID NOs: 243 to 252 and protein disulfide-isomerase (amino acid sequence: SEQ ID NO: 254, encoding nucleotide sequence: SEQ ID NO: 253) was identified as a protein comprising any of SEQ ID NOs: 255 to 278.

Accordingly, the antigen in spot 13α in the present invention can be any of proteins selected from the group consisting of the proteins as defined below in (13αA1-a) to (13αA1-e), (13αB1), (13αA2-a) to (13αA2-e), and (13αB2):
(13αA1-a) a protein comprising an amino acid sequence with deletion, substitution, insertion or addition of one or several amino acids in SEQ ID NO: 242;
(13αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 242;
(13αA1-c) a protein comprising an amino acid sequence encoded by a nucleotide sequence with deletion, substitution, insertion or addition of one or several nucleotides in SEQ ID NO: 241;
(13αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 241;
(13αA1-e) a protein comprising an amino acid sequence encoded by a nucleic acid that hybridizes under stringent conditions with a nucleic acid having a nucleotide sequence complementary to the nucleotide sequence of SEQ ID NO: 241;
(13αB1) a protein comprising at least one amino acid sequence selected from the group consisting of SEQ ID NOs: 243-252, preferably a protein comprising at least 2, 3, 4, 5, 6, 7, 8,
or 9, or all sequences of the amino acid sequences. As referred to above, the amino acid sequence of any of SEQ ID NOs: 205 to 218 may be an amino acid sequence derived therefrom by deletion, substitution, insertion or addition of one or several amino acids;
(13αA2-a) a protein comprising an amino acid sequence with deletion, substitution, insertion or addition of one or several amino acids in SEQ ID NO: 254;
(13αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 254;
(13αA2-c) a protein comprising an amino acid sequence encoded by a nucleotide sequence with deletion, substitution, insertion or addition of one or several nucleotides in SEQ ID NO: 253;
(13αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 253;
(13αA2-e) a protein comprising an amino acid sequence encoded by a nucleic acid that hybridizes under stringent conditions with a nucleic acid having a nucleotide sequence complementary to the nucleotide sequence of SEQ ID NO: 253;
(13αB2) a protein comprising at least one amino acid sequence selected from the group consisting of SEQ ID NOs: 255-278, preferably a protein comprising at least 2, 3, 4, 5, 6, 7, 8, 9, 10, 11, 12, 13, 14, 15, 16, 17, 18, 19, 20, 21, 22, or 23, or all sequences of the amino acid sequences. As referred to above, the amino acid sequence of any of SEQ ID NOs: 221 to 231 may be an amino acid sequence derived therefrom by deletion, substitution, insertion or addition of one or several amino acids.

The protein selected from the group consisting of the proteins as defined above in (13αA1-a) to (13αA1-e), (13αB1), (13αA2-a) to (13αA2-e), and (13αB2) also include those proteins whose amino acid residues are modified by phosphorylation, sugar chain modification, aminoacylation, ring-opening, deamination or the like.

The protein selected from the group consisting of the proteins as defined above in (13αA1-a) to (13αA1-e), (13αB1), (13αA2-a) to (13αA2-e), and (13αB2) can be proteins that are found in a protein spot with a molecular weight of around 30 to 90 kDa, preferably around 40 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 13α has activity equivalent to that of the protein having the amino acid sequence of SEQ ID NO: 242 (protein disulfide isomerase-like protein precursor) or the protein having the amino acid sequence of SEQ ID NO: 254 (protein disulfide-isomerase) or causative of an allergy to soybean.

### (14α) Antigen in spot 14α

As the result of sequence identification of the antigen in spot 14α by mass spectroscopy, the following amino acid sequences were detected.
DHSDVSNQLYANYAIGK (SEQ ID NO: 281)
FQEIVNIRLGDGTTRRGQVLEVDGEK (SEQ ID NO: 282)
FTTVQFTGEVLK (SEQ ID NO: 283)
TLDQFYSRDASN (SEQ ID NO: 284)
FQEIVNIRLGDGTTRRGQVLEVDGEK (SEQ ID NO: 287)
FTTVQFTGEVLK (SEQ ID NO: 288)
TLDQFYSRDAGN (SEQ ID NO: 289)

Also, the mass spectroscopic data obtained for spot 14α on a mass spectrometer was analyzed by comparing the data against the NCBI protein data, and as a result, V-type proton ATPase subunit B2 (amino acid sequence: SEQ ID NO: 280, encoding nucleotide sequence: SEQ ID NO: 279) was identified as a protein comprising any of SEQ ID NOs: 281 to 284 and V-type proton ATPase subunit B2 (amino acid sequence: SEQ ID NO: 286, encoding nucleotide sequence: SEQ ID NO: 285) was identified as a protein comprising any of SEQ ID NOs: 287 to 289.

Accordingly, the antigen in spot 14α in the present invention can be any of proteins selected from the group consisting of the proteins as defined below in (14αA1-a) to (14αA1-e), (14αB1), (14αA2-a) to (14αA2-e), and (14αB2):
(14αA1-a) a protein comprising an amino acid sequence with deletion, substitution, insertion or addition of one or several amino acids in SEQ ID NO: 280;
(14αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 280;
(14αA1-c) a protein comprising an amino acid sequence encoded by a nucleotide sequence with deletion, substitution, insertion or addition of one or several nucleotides in SEQ ID NO: 279;
(14αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 279;
(14αA1-e) a protein comprising an amino acid sequence encoded by a nucleic acid that hybridizes under stringent conditions with a nucleic acid having a nucleotide sequence complementary to the nucleotide sequence of SEQ ID NO: 279;
(14αB1) a protein comprising at least one amino acid sequence selected from the group consisting of SEQ ID NOs: 281-284, preferably a protein comprising at least 2, 3, or all sequences of the amino acid sequences. As referred to above, the amino acid sequence of any of SEQ ID NOs: 281 to 284 may be an amino acid sequence derived therefrom by deletion, substitution, insertion or addition of one or several amino acids;
(14αA2-a) a protein comprising an amino acid sequence with deletion, substitution, insertion or addition of one or several amino acids in SEQ ID NO: 286;
(14αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 286;
(14αA2-c) a protein comprising an amino acid sequence encoded by a nucleotide sequence with deletion, substitution, insertion or addition of one or several nucleotides in SEQ ID NO: 285;
(14αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 285;
(14αA2-e) a protein comprising an amino acid sequence encoded by a nucleic acid that hybridizes under stringent conditions with a nucleic acid having a nucleotide sequence complementary to the nucleotide sequence of SEQ ID NO: 285;
(14αB2) a protein comprising at least one amino acid sequence selected from the group consisting of SEQ ID NOs: 287-289, preferably a protein comprising at least 2, or all sequences of the amino acid sequences. As referred to above, the amino acid sequence of any of SEQ ID NOs: 287 to 289 may be an amino acid sequence derived therefrom by deletion, substitution, insertion or addition of one or several amino acids.

The protein selected from the group consisting of the proteins as defined above in (14αA1-a) to (14αA1-e), (14αB1), (14αA2-a) to (14αA2-e), and (14αB2) also include those proteins whose amino acid residues are modified by phosphorylation, sugar chain modification, aminoacylation, ring-opening, deamination or the like.

The protein selected from the group consisting of the proteins as defined above in (14αA1-a) to (14αA1-e), (14αB1), (14αA2-a) to (14αA2-e), and (14αB2) can be proteins that are found in a protein spot with a molecular weight of around 30 to 90 kDa, preferably around 40 to 80 kDa, and an isoelectric point of 2.5 to 6.5, preferably 3.0 to 6.0 on gels used in the two-dimensional electrophoresis performed under the conditions described above in the subsection titled "Identification of antigens".

Preferably, a protein that is an antigen in spot 14α has activity equivalent to that of the protein having the amino acid sequence of SEQ ID NO: 280 or 286 (V-type proton ATPase subunit B2) or causative of an allergy to soybean.

### (15α, 16α) Antigens in spots 15α, 16α

As the result of sequence identification of the antigens in spots 15α and 16α by mass spectroscopy, the following amino acid sequences were detected.
AIGINVPRSRFLPVK (SEQ ID NO: 292)
ARENPENPSIELGPEFK (SEQ ID NO: 293)
ATSDLLLVQSDLYTLEDGFVIRNK (SEQ ID NO: 294)
EINGPEDL (SEQ ID NO: 295)
EYVFVANSDNLGAIVDLK (SEQ ID NO: 296)
ILNHLIQNK (SEQ ID NO: 297)
IQTPTDEVVVPYDTLAPTPEGSSEVK (SEQ ID NO: 298)
LDALLSQGK (SEQ ID NO: 299)
RLVEADALK (SEQ ID NO: 300)
SAVAGLNEISENEK (SEQ ID NO: 301)
SIPSIVELDSLK (SEQ ID NO: 302)
VLQLETAAGAAIRFFDK (SEQ ID NO: 303)
VSNFLGRFK (SEQ ID NO: 304)
AIGINVPRSRFLPVK (SEQ ID NO: 307)
ATSDLLLVQSDLYTLEDGFVIRNK (SEQ ID NO: 308)
EYVFVANSDNLGAIVDLK (SEQ ID NO: 309)
ILNHLIQNK (SEQ ID NO: 310)
IQTPTDEVVVPYDTLAPTPDGSSDVK (SEQ ID NO: 311)
LDALLSQGK (SEQ ID NO: 312)
LEVPDGAVISDK (SEQ ID NO: 313)
RLVEADALK (SEQ ID NO: 314)
SAVAGLNEISESEK (SEQ ID NO: 315)
SIPSIVELDSLK (SEQ ID NO: 316)
VLQLETAAGAAIRFFDK (SEQ ID NO: 317)
VSNFLGRFK (SEQ ID NO: 318)

Also, the mass spectroscopic data obtained for spots 15α, 16α on a mass spectrometer was analyzed by comparing the data against the NCBI protein data, and as a result, UTP-glucose-1-phosphate uridylyltransferase (amino acid sequence: SEQ ID NO: 291, encoding nucleotide sequence: SEQ ID NO: 290) was identified as a protein comprising any of SEQ ID NOs: 292 to 304 and the hypothetical protein GLYMA_02G241100 (amino acid sequence: SEQ ID NO: 306, encoding nucleotide sequence: SEQ ID NO: 305) was identified as a protein comprising any of SEQ ID NOs: 307 to 318.

Accordingly, the antigen in spot 15α, 16α in the present invention can be any of proteins selected from the group consisting of the proteins as defined below in (15, 16αA1-a) to (15, 16αA1-e), (15, 16αB1), (15, 16αA2-a) to (15, 16αA2-e), and (15, 16αB2):
(15, 16αA1-a) a protein comprising an amino acid sequence with deletion, substitution, insertion or addition of one or several amino acids in SEQ ID NO: 291;
(15, 16αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 291;
(15, 16αA1-c) a protein comprising an amino acid sequence encoded by a nucleotide sequence with deletion, substitution, insertion or addition of one or several nucleotides in SEQ ID NO: 290;
(15, 16αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 290;
(15, 16αA1-e) a protein comprising an amino acid sequence encoded by a nucleic acid that hybridizes under stringent conditions with a nucleic acid having a nucleotide sequence complementary to the nucleotide sequence of SEQ ID NO: 290;
(15, 16αB1) a protein comprising at least one amino acid sequence selected from the group consisting of SEQ ID NOs: 292-304, preferably a protein comprising at least 2, 3, 4, 5, 6, 7, 8, 9, 10, 11, or 12 or all sequences of the amino acid sequences. As referred to above, the amino acid sequence of any of SEQ ID NOs: 292 to 304 may be an amino acid sequence derived therefrom by deletion, substitution, insertion or addition of one or several amino acids;
(15, 16αA2-a) a protein comprising an amino acid sequence with deletion, substitution, insertion or addition of one or several amino acids in SEQ ID NO: 306;
(15, 16αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 306;
(15, 16αA2-c) a protein comprising an amino acid sequence encoded by a nucleotide sequence with deletion, substitution, insertion or addition of one or several nucleotides in SEQ ID NO: 305;
(15, 16αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 305;
(15, 16αA2-e) a protein comprising an amino acid sequence encoded by a nucleic acid that hybridizes under stringent conditions with a nucleic acid having a nucleotide sequence complementary to the nucleotide sequence of SEQ ID NO: 305;
(15, 16αB2) a protein comprising at least one amino acid sequence selected from the group consisting of SEQ ID NOs: 307-318, preferably a protein comprising at least 2, 3, 4, 5, 6, 7, 8, 9, 10, or 11 or all sequences of the amino acid sequences. As referred to above, the amino acid sequence of any of SEQ ID NOs: 307 to 318 may be an amino acid sequence derived therefrom by deletion, substitution, insertion or addition of one or several amino acids.

The protein selected from the group consisting of the proteins as defined above in (15, 16αA1-a) to (15, 16αA1-e), (15, 16αB1), (15, 16αA2-a) to (15, 16αA2-e), and (15, 16αB2) also include those proteins whose amino acid residues are modified by phosphorylation, sugar chain modification, aminoacylation, ring-opening, deamination or the like.

The protein selected from the group consisting of the proteins as defined above in (15, 16αA1-a) to (15, 16αA1-e), (15, 16αB1), (15, 16αA2-a) to (15, 16αA2-e), and (15, 16αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 15α or 16α has activity equivalent to that of the protein having the amino acid sequence of SEQ ID NO: 291 (UTP-glucose-1-phosphate uridylyltransferase) or the protein having the amino acid sequence of SEQ ID NO: 306 (hypothetical protein GLYMA_02G241100) or causative of an allergy to soybean.

### (17α) Antigen in spot 17α

As the result of sequence identification of the antigen in spot 17α by mass spectroscopy, the following amino acid sequences were detected.
AVDGSFVFNK (SEQ ID NO: 321)
FFIYVQNYNESDPK (SEQ ID NO: 322)
VEFDEEGK (SEQ ID NO: 323)
VIDLSVDLSAASAAEE (SEQ ID NO: 324)
VVGVTSEGETAK (SEQ ID NO: 325)
AVDGSYVFNK (SEQ ID NO: 328)
FFIYVQNYNESDPK (SEQ ID NO: 329)
VEFDEEGK (SEQ ID NO: 330)
VIDLSVDLSAASAAEE (SEQ ID NO: 331)
VVGVTSEGETAK (SEQ ID NO: 332)

Also, the mass spectroscopic data obtained for spot 17α on a mass spectrometer was analyzed by comparing the data against the NCBI protein data, and as a result, guanosine nucleotide diphosphate dissociation inhibitor 2 (amino acid sequence: SEQ ID NO: 320, encoding nucleotide sequence: SEQ ID NO: 319) was identified as a protein comprising any of SEQ ID NOs: 321 to 325 and rab GDP dissociation inhibitor alpha-like (amino acid sequence: SEQ ID NO: 327, encoding nucleotide sequence: SEQ ID NO: 326) was identified as a protein comprising any of SEQ ID NOs: 328 to 332.

Accordingly, the antigen in spot 17α in the present invention can be any of proteins selected from the group consisting of the proteins as defined below in (17αA1-a) to (17αA1-e), (17αB1), (17αA2-a) to (17αA2-e), and (17αB2):
(17αA1-a) a protein comprising an amino acid sequence with deletion, substitution, insertion or addition of one or several amino acids in SEQ ID NO: 320;
(17αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 320;
(17αA1-c) a protein comprising an amino acid sequence encoded by a nucleotide sequence with deletion, substitution, insertion or addition of one or several nucleotides in SEQ ID NO: 319;
(17αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 319;
(17αA1-e) a protein comprising an amino acid sequence encoded by a nucleic acid that hybridizes under stringent conditions with a nucleic acid having a nucleotide sequence complementary to the nucleotide sequence of SEQ ID NO: 319;
(17αB1) a protein comprising at least one amino acid sequence selected from the group consisting of SEQ ID NOs: 321-325, preferably a protein comprising at least 2, 3, or 4 or all sequences of the amino acid sequences. As referred to above, the amino acid sequence of any of SEQ ID NOs: 321 to 325 maybe an amino acid sequence derived therefrom by deletion, substitution, insertion or addition of one or several amino acids.
(17αA2-a) a protein comprising an amino acid sequence with deletion, substitution, insertion or addition of one or several amino acids in SEQ ID NO: 327;
(17αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 327;
(17αA2-c) a protein comprising an amino acid sequence encoded by a nucleotide sequence with deletion, substitution, insertion or addition of one or several nucleotides in SEQ ID NO: 326;
(17αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 326;
(17αA2-e) a protein comprising an amino acid sequence encoded by a nucleic acid that hybridizes under stringent conditions with a nucleic acid having a nucleotide sequence complementary to the nucleotide sequence of SEQ ID NO: 326;
(17αB2) a protein comprising at least one amino acid sequence selected from the group consisting of SEQ ID NOs: 328-332, preferably a protein comprising at least 2, 3, or 4 or all sequences of the amino acid sequences. As referred to above, the amino acid sequence of any of SEQ ID NOs: 328 to 332 may be an amino acid sequence derived therefrom by deletion, substitution, insertion or addition of one or several amino acids;

The protein selected from the group consisting of the proteins as defined above in (17αA1-a) to (17αA1-e), (17αB1), (17αA2-a) to (17αA2-e), and (17αB2) also include those proteins whose amino acid residues are modified by phosphorylation, sugar chain modification, aminoacylation, ring-opening, deamination or the like.

The protein selected from the group consisting of the proteins as defined above in (17αA1-a) to (17αA1-e), (17αB1), (17αA2-a) to (17αA2-e), and (17αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 170α has activity equivalent to that of the protein having the amino acid sequence of SEQ ID NO: 320 (guanosine nucleotide diphosphate dissociation inhibitor 2) or the protein having the amino acid sequence of SEQ ID NO: 327 (rab GDP dissociation inhibitor alpha-like) or causative of an allergy to soybean.

### (18α) Antigen in spot 18α

As the result of sequence identification of the antigen in spot 18α by mass spectroscopy, the following amino acid sequences were detected.
DFETRVETEGGRIRVLK (SEQ ID NO: 335)
DNIVSSLDNVAK (SEQ ID NO: 336)
GRAVLGLVSESETEK (SEQ ID NO: 337)
LFDQQNEGSIFAISREQVRALAPTK (SEQ ID NO: 338)
RPTISNGYGRLTEVGPDDDEK (SEQ ID NO: 339)

Also, the mass spectroscopic data obtained for spot 18α on a mass spectrometer was analyzed by comparing the data against the NCBI protein data, and as a result, hypothetical protein GLYMA_10G028300 (amino acid sequence: SEQ ID NO: 334, encoding nucleotide sequence: SEQ ID NO: 333) was identified as a protein comprising any of SEQ ID NOs: 335 to 339.

Accordingly, the antigens in spot 18α in the present invention can be any protein selected from the group consisting of the proteins as defined below in (18αA-a) to (18αA-e) and (18αB):
(18αA-a) a protein comprising an amino acid sequence with deletion, substitution, insertion or addition of one or several amino acids in SEQ ID NO: 334;
(18αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 334;
(18αA-c) a protein comprising an amino acid sequence encoded by a nucleotide sequence with deletion, substitution, insertion or addition of one or several nucleotides in SEQ ID NO: 333;
(18αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 333;
(18αA-e) a protein comprising an amino acid sequence encoded by a nucleic acid that hybridizes under stringent conditions with a nucleic acid having a nucleotide sequence complementary to the nucleotide sequence of SEQ ID NO: 333;
(18αB) a protein comprising at least one amino acid sequence selected from the group consisting of SEQ ID NOs: 335-339, preferably a protein comprising at least 2, 3 or 4 or all sequences of the amino acid sequences. As referred to above, the amino acid sequence of any of SEQ ID NOs: 335-339 may be an amino acid sequence derived therefrom by deletion, substitution, insertion or addition of one or several amino acids.

The proteins selected from the group consisting of (18αA-a) to (18αA-e) and (18αB) as defined above also include those proteins whose amino acid residues are modified by phosphorylation, sugar chain modification, aminoacylation, ring-opening, deamination or the like.

The proteins selected from the group consisting of (18αA-a) to (18αA-e) and (18αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 18α has activity equivalent to that of the protein having the amino acid sequence of SEQ ID NO: 334 (hypothetical protein GLYMA_10G028300) or causative of an allergy to soybean.

### (19α) Antigen in spot 19α

As the result of sequence identification of the antigen in spot 19α by mass spectroscopy, the following amino acid sequences were detected.
AQFNIFSK (SEQ ID NO: 342)
DFSTRVETEGGSIRVLK (SEQ ID NO: 343)
DNIVSSLDNVAK (SEQ ID NO: 344)
GRAVLGLVRESETEK (SEQ ID NO: 345)
RPTFSNGYGRLTEVGPDDEK (SEQ ID NO: 346)

Also, the mass spectroscopic data obtained for spot 19α on a mass spectrometer was analyzed by comparing the data against the NCBI protein data, and as a result, sucrose binding protein homolog S-64 (amino acid sequence: SEQ ID NO: 341, encoding nucleotide sequence: SEQ ID NO: 340) was identified as a protein comprising any of SEQ ID NOs: 342 to 346.

Accordingly, the antigens in spot 19α in the present invention can be any protein selected from the group consisting of the proteins as defined below in (19αA-a) to (19αA-e) and (19αB):
(19αA-a) a protein comprising an amino acid sequence with deletion, substitution, insertion or addition of one or several amino acids in SEQ ID NO: 341;
(19αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 341;
(19αA-c) a protein comprising an amino acid sequence encoded by a nucleotide sequence with deletion, substitution, insertion or addition of one or several nucleotides in SEQ ID NO: 340;
(19αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 340;
(19αA-e) a protein comprising an amino acid sequence encoded by a nucleic acid that hybridizes under stringent conditions with a nucleic acid having a nucleotide sequence complementary to the nucleotide sequence of SEQ ID NO: 340;
(19αB) a protein comprising at least one amino acid sequence selected from the group consisting of SEQ ID NOs: 342-346, preferably a protein comprising at least 2, 3 or 4 or all sequences of the amino acid sequences. As referred to above, the amino acid sequence of any of SEQ ID NOs: 342-346 may be an amino acid sequence derived therefrom by deletion, substitution, insertion or addition of one or several amino acids.

The proteins selected from the group consisting of (19αA-a) to (19αA-e) and (19αB) as defined above also include those proteins whose amino acid residues are modified by phosphorylation, sugar chain modification, aminoacylation, ring-opening, deamination or the like.

The proteins selected from the group consisting of (19αA-a) to (19αA-e) and (19αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 19α has activity equivalent to that of the protein having the amino acid sequence of SEQ ID NO: 341 (sucrose binding protein homolog S-64) or causative of an allergy to soybean.

### (25α) Antigen in spot 25α

As the result of sequence identification of the antigen in spot 25α by mass spectroscopy, the following amino acid sequences were detected.
DLFPNENELVVK (SEQ ID NO: 349)
EIFSLRDTTQEDPREVTAAK (SEQ ID NO: 350)
LHGGTPANFLDVGGNASENQVVEAFK (SEQ ID NO: 351)
LNFDDNAAYRQK (SEQ ID NO: 352)
SQILAGGRGLGTFK (SEQ ID NO: 353)
TDQVEDIAGK (SEQ ID NO: 354)
VPVVVRLEGTNVDQGK (SEQ ID NO: 355)
VVDGLALK (SEQ ID NO: 356)
DLFPNENELVVK (SEQ ID NO: 359)
EIFALRDTTQEDPREVTAAK (SEQ ID NO: 360)
LHGGTPANFLDVGGNASEGQVVEAFK (SEQ ID NO: 361)
LNFDDNAAYRQK (SEQ ID NO: 362)
SQILAGGRGLGTFK (SEQ ID NO: 363)
TDQVEDIAGK (SEQ ID NO: 364)
VVDGLALK (SEQ ID NO: 365)

Also, the mass spectroscopic data obtained for spot 25α on a mass spectrometer was analyzed by comparing the data against the NCBI protein data, and as a result, succinyl-CoA ligase [ADP-forming] subunit beta, mitochondrial (amino acid sequence: SEQ ID NO: 348, encoding nucleotide sequence: SEQ ID NO: 347) was identified as a protein comprising any of SEQ ID NOs: 349 to 356 and succinyl-CoA ligase [ADP-forming] subunit beta, mitochondrial (amino acid sequence: SEQ ID NO: 358, encoding nucleotide sequence: SEQ ID NO: 357) was identified as a protein comprising any of SEQ ID NOs: 359 to 365.

Accordingly, the antigen in spot 25α in the present invention can be any of proteins selected from the group consisting of the proteins as defined below in (25αA1-a) to (25αA1-e), (25αB1), (25αA2-a) to (25αA2-e), and (25αB2):
(25αA1-a) a protein comprising an amino acid sequence with deletion, substitution, insertion or addition of one or several amino acids in SEQ ID NO: 348;
(25αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 348;
(25αA1-c) a protein comprising an amino acid sequence encoded by a nucleotide sequence with deletion, substitution, insertion or addition of one or several nucleotides in SEQ ID NO: 347;
(25αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 347;
(25αA1-e) a protein comprising an amino acid sequence encoded by a nucleic acid that hybridizes under stringent conditions with a nucleic acid having a nucleotide sequence complementary to the nucleotide sequence of SEQ ID NO: 347;
(25αB1) a protein comprising at least one amino acid sequence selected from the group consisting of SEQ ID NOs: 349-356, preferably a protein comprising at least 2, 3, 4, 5, 6, or 7 or all sequences of the amino acid sequences. As referred to above, the amino acid sequence of any of SEQ ID NOs: 349 to 356 may be an amino acid sequence derived therefrom by deletion, substitution, insertion or addition of one or several amino acids;
(25αA2-a) a protein comprising an amino acid sequence with deletion, substitution, insertion or addition of one or several amino acids in SEQ ID NO: 358;
(25αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 358;
(25αA2-c) a protein comprising an amino acid sequence encoded by a nucleotide sequence with deletion, substitution, insertion or addition of one or several nucleotides in SEQ ID NO: 357;
(25αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 357;
(25αA2-e) a protein comprising an amino acid sequence encoded by a nucleic acid that hybridizes under stringent conditions with a nucleic acid having a nucleotide sequence complementary to the nucleotide sequence of SEQ ID NO: 357;
(25αB2) a protein comprising at least one amino acid sequence selected from the group consisting of SEQ ID NOs: 359-365, preferably a protein comprising at least 2, 3, 4, 5, or 6 or all sequences of the amino acid sequences. As referred to above, the amino acid sequence of any of SEQ ID NOs: 359 to 365 may be an amino acid sequence derived therefrom by deletion, substitution, insertion or addition of one or several amino acids.

The protein selected from the group consisting of the proteins as defined above in (25αA1-a) to (25αA1-e), (25αB1), (25αA2-a) to (25αA2-e), and (25αB2) also include those proteins whose amino acid residues are modified by phosphorylation, sugar chain modification, aminoacylation, ring-opening, deamination or the like.

The protein selected from the group consisting of the proteins as defined above in (25αA1-a) to (25αA1-e), (25αB1), (25αA2-a) to (25αA2-e), and (25αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 25α has activity equivalent to that of the protein having the amino acid sequence of SEQ ID NO: 348 or 358 (succinyl-CoA ligase [ADP-forming] subunit beta, mitochondrial) or causative of an allergy to soybean.

### (26α) Antigen in spot 26α

As the result of sequence identification of the antigen in spot 26α by mass spectroscopy, the following amino acid sequences were detected.
AQGYSVGSSLVEEDK (SEQ ID NO: 368)
ELDYLVGAVSNPK (SEQ ID NO: 369)
FAADANSK (SEQ ID NO: 370)
FATGTEAIAK (SEQ ID NO: 371)
GVSLLLPTDVVIADK (SEQ ID NO: 372)
GVTTIIGGGDSVAAVEK (SEQ ID NO: 373)
IGVIESLLEK (SEQ ID NO: 374)
LASLADLYVNDAFGTAHRAHASTEGVAK (SEQ ID NO: 375)
LSLATTLLEK (SEQ ID NO: 376)
LVTQLPEGGVLLLENVRFYK (SEQ ID NO: 377)
NDPEFAK (SEQ ID NO: 378)
QLPGVLALDDA (SEQ ID NO: 379)
RPFAAIVGGSK (SEQ ID NO: 380)
VILSSHLGRPK (SEQ ID NO: 381)
YLTGYGAK (SEQ ID NO: 382)
ELDYLVGAVSNPK (SEQ ID NO: 385)
ELPGVLALDEAVPVAV (SEQ ID NO: 386)
FAVGTEAIAK (SEQ ID NO: 387)
GVSLLLPSDVVIADK (SEQ ID NO: 388)
GVTTIIGGGDSVAAVEK (SEQ ID NO: 389)
IGVIESLLEK (SEQ ID NO: 390)
RPFAAIVGGSK (SEQ ID NO: 391)
VILSSHLGRPK (SEQ ID NO: 392)
AQGYSVGSSLVEEDK (SEQ ID NO: 395)
ELDYLVGAVSNPK (SEQ ID NO: 396)
FAADANSK (SEQ ID NO: 397)
FATGTEAIAK (SEQ ID NO: 398)
GVSLLLPTDVVIADK (SEQ ID NO: 399)
GVTTIIGGGDSVAAVEK (SEQ ID NO: 400)
IGVIESLLEK (SEQ ID NO: 401)
LASLADLYVNDAFGTAHRAHASTEGVAK (SEQ ID NO: 402)
LSLATTLLEK (SEQ ID NO: 403)
LVAQLPEGGVLLLENVRFYK (SEQ ID NO: 404)
NDPEFAK (SEQ ID NO: 405)
QLPGVLALDDA (SEQ ID NO: 406)
RPFAAIVGGSK (SEQ ID NO: 407)
TFGEALDK (SEQ ID NO: 408)
VILSSHLGRPK (SEQ ID NO: 409)

Also, the mass spectroscopic data obtained for spot 26α on a mass spectrometer was analyzed by comparing the data against the NCBI protein data, and as a result, phosphoglycerate kinase, cytosolic (amino acid sequence: SEQ ID NO: 367, encoding nucleotide sequence: SEQ ID NO: 366) was identified as a protein comprising any of SEQ ID NOs: 368 to 382, phosphoglycerate kinase, cytosolic (amino acid sequence: SEQ ID NO: 384, encoding nucleotide sequence: SEQ ID NO: 383) was identified as a protein comprising any of SEQ ID NOs: 385 to 392, and phosphoglycerate kinase, cytosolic (amino acid sequence: SEQ ID NO: 394, encoding nucleotide sequence: SEQ ID NO: 393) was identified as a protein comprising any of SEQ ID NOs: 395 to 409.

Accordingly, the antigen in spot 26α in the present invention can be any of proteins selected from the group consisting of the proteins as defined below in (26αA1-a) to (26αA1-e), (26αB1), (26αA2-a) to (26αA2-e), (26αB2), (26αA3-a) to (26αA3-e), and (26αB3):
(26αA1-a) a protein comprising an amino acid sequence with deletion, substitution, insertion or addition of one or several amino acids in SEQ ID NO: 367;
(26αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 367;
(26αA1-c) a protein comprising an amino acid sequence encoded by a nucleotide sequence with deletion, substitution, insertion or addition of one or several nucleotides in SEQ ID NO: 366;
(26αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 366;
(26αA1-e) a protein comprising an amino acid sequence encoded by a nucleic acid that hybridizes under stringent conditions with a nucleic acid having a nucleotide sequence complementary to the nucleotide sequence of SEQ ID NO: 366;
(26αB1) a protein comprising at least one amino acid sequence selected from the group consisting of SEQ ID NOs: 368-382, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 368 to 382 may be an amino acid sequence derived therefrom by deletion, substitution, insertion or addition of one or several amino acids;
(26αA2-a) a protein comprising an amino acid sequence with deletion, substitution, insertion or addition of one or several amino acids in SEQ ID NO: 384;
(26αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 384;
(26αA2-c) a protein comprising an amino acid sequence encoded by a nucleotide sequence with deletion, substitution, insertion or addition of one or several nucleotides in SEQ ID NO: 383;
(26αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 383;
(26αA2-e) a protein comprising an amino acid sequence encoded by a nucleic acid that hybridizes under stringent conditions with a nucleic acid having a nucleotide sequence complementary to the nucleotide sequence of SEQ ID NO: 383;
(26αB2) a protein comprising at least one amino acid sequence selected from the group consisting of SEQ ID NOs: 385-392, preferably a protein comprising at least 2, 3, 4, 5, 6, or 7 or all sequences of the amino acid sequences. As referred to above, the amino acid sequence of any of SEQ ID NOs: 385 to 392 may be an amino acid sequence derived therefrom by deletion, substitution, insertion or addition of one or several amino acids.
(26αA3-a) a protein comprising an amino acid sequence with deletion, substitution, insertion or addition of one or several amino acids in SEQ ID NO: 394;
(26αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 394;
(26αA3-c) a protein comprising an amino acid sequence encoded by a nucleotide sequence with deletion, substitution, insertion or addition of one or several nucleotides in SEQ ID NO: 393;
(26αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 393;
(26αA3-e) a protein comprising an amino acid sequence encoded by a nucleic acid that hybridizes under stringent conditions with a nucleic acid having a nucleotide sequence complementary to the nucleotide sequence of SEQ ID NO: 393;
(26αB3) a protein comprising at least one amino acid sequence selected from the group consisting of SEQ ID NOs: 395-409, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 395 to 409 may be an amino acid sequence derived therefrom by deletion, substitution, insertion or addition of one or several amino acids.

The protein selected from the group consisting of the proteins as defined above in (26αA1-a) to (26αA1-e), (26αB1), (26αA2-a) to (26αA2-e), (26αB2), (26αA3-a) to (26αA3-e), and (26αB3) also include those proteins whose amino acid residues are modified by phosphorylation, sugar chain modification, aminoacylation, ring-opening, deamination or the like.

The protein selected from the group consisting of the proteins as defined above in (26αA1-a) to (26αA1-e), (26αB1), (26αA2-a) to (26αA2-e), (26αB2), (26αA3-a) to (26αA3-e), and (26αB3) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 25α has activity equivalent to that of the protein having the amino acid sequence of SEQ ID NO: 367, 384, or 394 (phosphoglycerate kinase, cytosolic) or causative of an allergy to soybean.

### (27α) Antigen in spot 27α

As the result of sequence identification of the antigen in spot 27α by mass spectroscopy, the following amino acid sequences were detected.
FGVNEFVNPK (SEQ ID NO: 412)
FITHTVPFSEINK (SEQ ID NO: 413)
GTFYGNYKPRTDLPSVVEK (SEQ ID NO: 414)
THPVNFLNERTLK (SEQ ID NO: 415)
VNPAAPLDK (SEQ ID NO: 416)
GTFYGHYRPRTDIPGVVEK (SEQ ID NO: 419)
INPNAPLDK (SEQ ID NO: 420)
FGVNEFVNPK (SEQ ID NO: 423)
FITHTVPFSEINK (SEQ ID NO: 424)
GTFYGNYKPRTDLPSVVEK (SEQ ID NO: 425)
INPAAPLDK (SEQ ID NO: 426)
TAPINFLNERTLK (SEQ ID NO: 427)

Also, the mass spectroscopic data obtained for spot 27α on a mass spectrometer was analyzed by comparing the data against the NCBI protein data, and as a result, alcohol dehydrogenase 1 (amino acid sequence: SEQ ID NO: 411, encoding nucleotide sequence: SEQ ID NO: 410) was identified as a protein comprising any of SEQ ID NOs: 412 to 416, alcohol dehydrogenase 1 (amino acid sequence: SEQ ID NO: 418, encoding nucleotide sequence: SEQ ID NO: 417) was identified as a protein comprising any of SEQ ID NOs: 419 and 420, and alcohol dehydrogenase 1-like (amino acid sequence: SEQ ID NO: 422, encoding nucleotide sequence: SEQ ID NO: 421) was identified as a protein comprising any of SEQ ID NOs: 423 to 427.

Accordingly, the antigen in spot 27α in the present invention can be any of proteins selected from the group consisting of the proteins as defined below in (27αA1-a) to (27αA1-e), (27αB1), (27αA2-a) to (27αA2-e), (27αB2), (27αA3-a) to (27αA3-e), and (27αB3):
(27αA1-a) a protein comprising an amino acid sequence with deletion, substitution, insertion or addition of one or several amino acids in SEQ ID NO: 411;
(27αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 411;
(27αA1-c) a protein comprising an amino acid sequence encoded by a nucleotide sequence with deletion, substitution, insertion or addition of one or several nucleotides in SEQ ID NO: 410;
(27αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 410;
(27αA1-e) a protein comprising an amino acid sequence encoded by a nucleic acid that hybridizes under stringent conditions with a nucleic acid having a nucleotide sequence complementary to the nucleotide sequence of SEQ ID NO: 410;
(27αB1) a protein comprising at least one amino acid sequence selected from the group consisting of SEQ ID NOs: 412-416, preferably a protein comprising at least 2, 3, or 4 or all sequences of the amino acid sequences. As referred to above, the amino acid sequence of any of SEQ ID NOs: 412 to 416 may be an amino acid sequence derived therefrom by deletion, substitution, insertion or addition of one or several amino acids;
(27αA2-a) a protein comprising an amino acid sequence with deletion, substitution, insertion or addition of one or several amino acids in SEQ ID NO: 418;
(27αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 418;
(27αA2-c) a protein comprising an amino acid sequence encoded by a nucleotide sequence with deletion, substitution, insertion or addition of one or several nucleotides in SEQ ID NO: 417;
(27αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 417;
(27αA2-e) a protein comprising an amino acid sequence encoded by a nucleic acid that hybridizes under stringent conditions with a nucleic acid having a nucleotide sequence complementary to the nucleotide sequence of SEQ ID NO: 417;
(27αB2) a protein comprising at least one amino acid sequence selected from the group consisting of SEQ ID NOs: 419 and 420, preferably a protein comprising both the amino acid sequences. As referred to above, the amino acid sequence of any of SEQ ID NOs: 419, 420 may be an amino acid sequence derived therefrom by deletion, substitution, insertion or addition of one or several amino acids.
(27αA3-a) a protein comprising an amino acid sequence with deletion, substitution, insertion or addition of one or several amino acids in SEQ ID NO: 422;
(27αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 422;
(27αA3-c) a protein comprising an amino acid sequence encoded by a nucleotide sequence with deletion, substitution, insertion or addition of one or several nucleotides in SEQ ID NO: 421;
(27αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 421;
(27αA3-e) a protein comprising an amino acid sequence encoded by a nucleic acid that hybridizes under stringent conditions with a nucleic acid having a nucleotide sequence complementary to the nucleotide sequence of SEQ ID NO: 421;
(27αB3) a protein comprising at least one amino acid sequence selected from the group consisting of SEQ ID NOs: 423-427, preferably a protein comprising at least 2, 3, or 4 or all sequences of the amino acid sequences. As referred to above, the amino acid sequence of any of SEQ ID NOs: 423 to 427 may be an amino acid sequence derived therefrom by deletion, substitution, insertion or addition of one or several amino acids.

The protein selected from the group consisting of the proteins as defined above in (27αA1-a) to (27αA1-e), (27αB1), (27αA2-a) to (27αA2-e), (27αB2), (27αA3-a) to (27αA3-e), and (27αB3) also include those proteins whose amino acid residues are modified by phosphorylation, sugar chain modification, aminoacylation, ring-opening, deamination or the like.

The protein selected from the group consisting of the proteins as defined above in (27αA1-a) to (27αA1-e), (27αB1), (27αA2-a) to (27αA2-e), (27αB2), (27αA3-a) to (27αA3-e), and (27αB3) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 27α has activity equivalent to that of the protein having the amino acid sequence of SEQ ID NO: 411 or 418 (alcohol dehydrogenase 1) or the protein having the amino acid sequence of SEQ ID NO: 422 (alcohol dehydrogenase 1-like) or causative of an allergy to soybean.

### (28α) Antigen in spot 28α

As the result of sequence identification of the antigen in spot 28α by mass spectroscopy, the following amino acid sequences were detected.
AGEYTDYAAQK (SEQ ID NO: 430)
DIGDINRTNERDEGYDLHDEANFK (SEQ ID NO: 431)
DLEDANRLRDNTFNTWK (SEQ ID NO: 432)
EAVVGKPHHEEVYAK (SEQ ID NO: 433)

Also, the mass spectroscopic data obtained for spot 28α on a mass spectrometer was analyzed by comparing the data against the NCBI protein data, and as a result, 35 kDa seed maturation protein isoform X1 (amino acid sequence: SEQ ID NO: 429, encoding nucleotide sequence: SEQ ID NO: 428) was identified as a protein comprising any of SEQ ID NO: 430 to 433.

Accordingly, the antigens in spot 28α in the present invention can be any protein selected from the group consisting of the proteins as defined below in (28αA-a) to (28αA-e) and (28αB):
(28αA-a) a protein comprising an amino acid sequence with deletion, substitution, insertion or addition of one or several amino acids in SEQ ID NO: 429;
(28αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 429;
(28αA-c) a protein comprising an amino acid sequence encoded by a nucleotide sequence with deletion, substitution, insertion or addition of one or several nucleotides in SEQ ID NO: 428;
(28αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 428;
(28αA-e) a protein comprising an amino acid sequence encoded by a nucleic acid that hybridizes under stringent conditions with a nucleic acid having a nucleotide sequence complementary to the nucleotide sequence of SEQ ID NO: 428;
(28αB) a protein comprising at least one amino acid sequence selected from the group consisting of SEQ ID NOs: 430-433, preferably a protein comprising at least 2 or 3 or all sequences of the amino acid sequences. As referred to above, the amino acid sequence of any of SEQ ID NOs: 430-433 may be an amino acid sequence derived therefrom by deletion, substitution, insertion or addition of one or several amino acids.

The proteins selected from the group consisting of (28αA-a) to (28αA-e) and (28αB) as defined above also include those proteins whose amino acid residues are modified by phosphorylation, sugar chain modification, aminoacylation, ring-opening, deamination or the like.

The proteins selected from the group consisting of (28αA-a) to (28αA-e) and (28αB) as defined above can be proteins that are found in a protein spot with a molecular weight of around 10 to 70 kDa, preferably around 20 to 60 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 28α has activity equivalent to that of the protein having the amino acid sequence of SEQ ID NO: 422 (35 kDa seed maturation protein isoform X1) or causative of an allergy to soybean.

### (29α, 30α, 31α, 32α, 33α) Antigens in spots 29α to 33α

As the result of sequence identification of the antigens in spots 29α to 33α by mass spectroscopy, the following amino acid sequences were detected.
EASYDEIK (SEQ ID NO: 436)
GILGYTEDDVVSTDFIGDSRSSIFDAK (SEQ ID NO: 437)
VIISAPSK (SEQ ID NO: 438)
VLPALNGK (SEQ ID NO: 439)
EASYDEIK (SEQ ID NO: 442)
GILGYTEDDVVSTDFVGDSRSSIFDAK (SEQ ID NO: 443)
VIISAPSK (SEQ ID NO: 444)
VLPALNGK (SEQ ID NO: 445)
AGISLNDNFVK (SEQ ID NO: 448)
GILGYTEDDVVSTDFVGDNRSSIFDAK (SEQ ID NO: 449)
VLPALNGK (SEQ ID NO: 450)
VVISAPSK (SEQ ID NO: 451)

Also, the mass spectroscopic data obtained for spots 29α to 33α on a mass spectrometer was analyzed by comparing the data against the NCBI protein data, and as a result, glyceraldehyde-3-phosphate dehydrogenase isoform X1 (amino acid sequence: SEQ ID NO: 435, encoding nucleotide sequence: SEQ ID NO: 434) was identified as a protein comprising any of SEQ ID NO: 436 to 439, glyceraldehyde-3-phosphate dehydrogenase, cytosolic (amino acid sequence: SEQ ID NO: 441, encoding nucleotide sequence: SEQ ID NO: 440) was identified as a protein comprising any of SEQ ID NO: 442 to 445, and uncharacterized protein LOC100782924 (amino acid sequence: SEQ ID NO: 447, encoding nucleotide sequence: SEQ ID NO: 446) was identified as a protein comprising any of SEQ ID NO: 448 to 451.

Accordingly, the antigen in spot 29α to 33α in the present invention can be any of proteins selected from the group consisting of the proteins as defined below in (29-33αA1-a) to (29-33αA1-e), (29-33αB1), (29-33αA2-a) to (29-33αA2-e), (29-33αB2), (29-33αA3-a) to (29-33αA3-e), and (29-33αB3):
(29-33αA1-a) a protein comprising an amino acid sequence with deletion, substitution, insertion or addition of one or several amino acids in SEQ ID NO: 435;
(29-33αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 435;
(29-33αA1-c) a protein comprising an amino acid sequence encoded by a nucleotide sequence with deletion, substitution, insertion or addition of one or several nucleotides in SEQ ID NO: 434;
(29-33αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 434;
(29-33αA1-e) a protein comprising an amino acid sequence encoded by a nucleic acid that hybridizes under stringent conditions with a nucleic acid having a nucleotide sequence complementary to the nucleotide sequence of SEQ ID NO: 434;
(29-33αB1) a protein comprising at least one amino acid sequence selected from the group consisting of SEQ ID NOs: 436-439, preferably a protein comprising at least 2, 3, or all sequences of the amino acid sequences. As referred to above, the amino acid sequence of any of SEQ ID NOs: 436 to 439 may be an amino acid sequence derived therefrom by deletion, substitution, insertion or addition of one or several amino acids;
(29-33αA2-a) a protein comprising an amino acid sequence with deletion, substitution, insertion or addition of one or several amino acids in SEQ ID NO: 441;
(29-33αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 441;
(29-33αA2-c) a protein comprising an amino acid sequence encoded by a nucleotide sequence with deletion, substitution, insertion or addition of one or several nucleotides in SEQ ID NO: 440;
(29-33αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 440;
(29-33αA2-e) a protein comprising an amino acid sequence encoded by a nucleic acid that hybridizes under stringent conditions with a nucleic acid having a nucleotide sequence complementary to the nucleotide sequence of SEQ ID NO: 440;
(29-33αB2) a protein comprising at least one amino acid sequence selected from the group consisting of SEQ ID NOs: 442-445, preferably a protein comprising at least 2, 3, or all sequences of the amino acid sequences. As referred to above, the amino acid sequence of any of SEQ ID NOs: 442 to 445 may be an amino acid sequence derived therefrom by deletion, substitution, insertion or addition of one or several amino acids.
(29-33αA3-a) a protein comprising an amino acid sequence with deletion, substitution, insertion or addition of one or several amino acids in SEQ ID NO: 447;
(29-33αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 447;
(29-33αA3-c) a protein comprising an amino acid sequence encoded by a nucleotide sequence with deletion, substitution, insertion or addition of one or several nucleotides in SEQ ID NO: 446;
(29-33αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 446;
(29-33αA3-e) a protein comprising an amino acid sequence encoded by a nucleic acid that hybridizes under stringent conditions with a nucleic acid having a nucleotide sequence complementary to the nucleotide sequence of SEQ ID NO: 446;
(29-33αB3) a protein comprising at least one amino acid sequence selected from the group consisting of SEQ ID NOs: 448-451, preferably a protein comprising at least 2, 3, or all sequences of the amino acid sequences. As referred to above, the amino acid sequence of any of SEQ ID NOs: 448 to 451 may be an amino acid sequence derived therefrom by deletion, substitution, insertion or addition of one or several amino acids.

The protein selected from the group consisting of the proteins as defined above in (29-33αA1-a) to (29-33αA1-e), (29-33αB1), (29-33αA2-a) to (29-33αA2-c), (29-33αB2), (29-33αA3-a) to (29-33αA3-e), and (29-33αB3) also include those proteins whose amino acid residues are modified by phosphorylation, sugar chain modification, aminoacylation, ring-opening, deamination or the like.

The protein selected from the group consisting of the proteins as defined above in (29-33αA1-a) to (29-33αA1-e), (29-33αB1), (29-33αA2-a) to (29-33αA2-e), (29-33αB2), (29-33αA3-a) to (29-33αA3-e), and (29-33αB3) can be proteins that are found in a protein spot with a molecular weight of around 10 to 70 kDa, preferably around 20 to 60 kDa, and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in any of spots 29α to 33α has activity equivalent to that of the protein having the amino acid sequence of SEQ ID NO: 435 (glyceraldehyde-3-phosphate dehydrogenase isoform XI), the protein having the amino acid sequence of SEQ ID NO: 441 (glyceraldehyde-3-phosphate dehydrogenase, cytosolic), or the protein having the amino acid sequence of SEQ ID NO: 447 (uncharacterized protein LOC100782924) or causative of an allergy to soybean.

### (34α) Antigen in spot 34α

As the result of sequence identification of the antigen in spot 34α by mass spectroscopy, the following amino acid sequences were detected.
LFLEEIARDIQK (SEQ ID NO: 454)
LFLEEIARDIQK (SEQ ID NO: 457)
TTFDAVIHFAGLK (SEQ ID NO: 458)

Also, the mass spectroscopic data obtained for spot 34α on a mass spectrometer was analyzed by comparing the data against the NCBI protein data, and as a result, bifunctional UDP-glucose 4-epimerase and UDP-xylose 4-epimerase 1-like (amino acid sequence: SEQ ID NO: 453, encoding nucleotide sequence: SEQ ID NO: 452) was identified as a protein comprising SEQ ID NO: 454 and uncharacterized protein LOC100803483 (amino acid sequence: SEQ ID NO: 456, encoding nucleotide sequence: SEQ ID NO: 455) was identified as a protein comprising SEQ ID NO: 457 or 458.

Accordingly, the antigen in spot 34α in the present invention can be any of proteins selected from the group consisting of the proteins as defined below in (34αA1-a) to (34αA1-e), (34αB1), (34αA2-a) to (34αA2-e), and (34αB2):
(34αA1-a) a protein comprising an amino acid sequence with deletion, substitution, insertion or addition of one or several amino acids in SEQ ID NO: 453;
(34αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 453;
(34αA1-c) a protein comprising an amino acid sequence encoded by a nucleotide sequence with deletion, substitution, insertion or addition of one or several nucleotides in SEQ ID NO: 452;
(34αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 452;
(34αA1-e) a protein comprising an amino acid sequence encoded by a nucleic acid that hybridizes under stringent conditions with a nucleic acid having a nucleotide sequence complementary to the nucleotide sequence of SEQ ID NO: 452;
(34αB1) a protein comprising an amino acid sequence of SEQ ID NOs: 454. As referred to above, the amino acid sequence of any of SEQ ID NOs: 464 may be an amino acid sequence derived therefrom by deletion, substitution, insertion or addition of one or several amino acids;
(34αA2-a) a protein comprising an amino acid sequence with deletion, substitution, insertion or addition of one or several amino acids in SEQ ID NO: 456;
(34αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 456;
(34αA2-c) a protein comprising an amino acid sequence encoded by a nucleotide sequence with deletion, substitution, insertion or addition of one or several nucleotides in SEQ ID NO: 455;
(34αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 455;
(34αA2-e) a protein comprising an amino acid sequence encoded by a nucleic acid that hybridizes under stringent conditions with a nucleic acid having a nucleotide sequence complementary to the nucleotide sequence of SEQ ID NO: 455;
(34αB2) a protein comprising at least one amino acid sequence selected from the group consisting of SEQ ID NOs: 457 and 458, preferably a protein comprising both the amino acid sequences. As referred to above, the amino acid sequence of any of SEQ ID NOs: 457, 458 may be an amino acid sequence derived therefrom by deletion, substitution, insertion or addition of one or several amino acids.

The protein selected from the group consisting of the proteins as defined above in (34αA1-a) to (34αA1-e), (34αB1), (34αA2-a) to (34αA2-e), and (34αB2) also include those proteins whose amino acid residues are modified by phosphorylation, sugar chain modification, aminoacylation, ring-opening, deamination or the like.

The protein selected from the group consisting of the proteins as defined above in (34αA1-a) to (34αA1-e), (34αB1), (34αA2-a) to (34αA2-e), and (34αB2) can be proteins that are found in a protein spot with a molecular weight of around 10 to 70 kDa, preferably around 20 to 60 kDa, and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 34α has activity equivalent to that of the protein having the amino acid sequence of SEQ ID NO: 453 (bifunctional UDP-glucose 4-epimerase and UDP-xylose 4-epimerase 1-like) or the protein having the amino acid sequence of SEQ ID NO: 456 (uncharacterized protein LOC100803483) or causative of an allergy to soybean.

### (35α) Antigen in spot 35α

As the result of sequence identification of the antigen in spot 35α by mass spectroscopy, the following amino acid sequences were detected.
ARYDEIVK (SEQ ID NO: 461)
DGQTREHALLAFTLGVK (SEQ ID NO: 462)
EVSSYLK (SEQ ID NO: 463)
FSEILTK (SEQ ID NO: 464)
STTTGHLIYK (SEQ ID NO: 465)
THINIVVIGHVDSGK (SEQ ID NO: 466)
DGQTREHALLAFTLGVK (SEQ ID NO: 469)
EVSSYLK (SEQ ID NO: 470)
STTTGHLIYK (SEQ ID NO: 471)
ARYDEIVK (SEQ ID NO: 474)
DGQTREHALLSFTLGVK (SEQ ID NO: 475)
EVSSYLK (SEQ ID NO: 476)
STTTGHLIYK (SEQ ID NO: 477)

Also, the mass spectroscopic data obtained for spot 35α on a mass spectrometer was analyzed by comparing the data against the NCBI protein data, and as a result, elongation factor 1-alpha (amino acid sequence: SEQ ID NO: 460, encoding nucleotide sequence: SEQ ID NO: 459) was identified as a protein comprising any of SEQ ID NOs: 461 to 466, elongation factor 1-alpha (amino acid sequence: SEQ ID NO: 468, encoding nucleotide sequence: SEQ ID NO: 467) was identified as a protein comprising any of SEQ ID NOs: 469 to 471, and elongation factor-lA isoform XI (amino acid sequence: SEQ ID NO: 473, encoding nucleotide sequence: SEQ ID NO: 472) was identified as a protein comprising any of SEQ ID NOs: 474 to 477.

Accordingly, the antigen in spot 35α in the present invention can be any of proteins selected from the group consisting of the proteins as defined below in (35αA1-a) to (35αA1-e), (35αB1), (35αA2-a) to (35αA2-e), (35αB2), (35αA3-a) to (35αA3-e), and (35αB3):
(35αA1-a) a protein comprising an amino acid sequence with deletion, substitution, insertion or addition of one or several amino acids in SEQ ID NO: 460;
(35αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 460;
(35αA1-c) a protein comprising an amino acid sequence encoded by a nucleotide sequence with deletion, substitution, insertion or addition of one or several nucleotides in SEQ ID NO: 459;
(35αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 459;
(35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
(35αB1) a protein comprising at least one amino acid sequence selected from the group consisting of SEQ ID NOs: 461-466, preferably a protein comprising at least 2, 3, 4, or 5 or all sequences of the amino acid sequences. As referred to above, the amino acid sequence of any of SEQ ID NOs: 412 to 416 may be an amino acid sequence derived therefrom by deletion, substitution, insertion or addition of one or several amino acids;
(35αA2-a) a protein comprising an amino acid sequence with deletion, substitution, insertion or addition of one or several amino acids in SEQ ID NO: 468;
(35αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 468;
(35αA2-c) a protein comprising an amino acid sequence encoded by a nucleotide sequence with deletion, substitution, insertion or addition of one or several nucleotides in SEQ ID NO: 467;
(35αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 467;
(35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
(35αB2) a protein comprising at least one amino acid sequence selected from the group consisting of SEQ ID NOs: 469-471, preferably a protein comprising at least 2, or all sequences of the amino acid sequences. As referred to above, the amino acid sequence of any of SEQ ID NOs: 469 to 471 may be an amino acid sequence derived therefrom by deletion, substitution, insertion or addition of one or several amino acids.
(35αA3-a) a protein comprising an amino acid sequence with deletion, substitution, insertion or addition of one or several amino acids in SEQ ID NO: 473;
(35αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 473;
(35αA3-c) a protein comprising an amino acid sequence encoded by a nucleotide sequence with deletion, substitution, insertion or addition of one or several nucleotides in SEQ ID NO: 472;
(35αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 472;
(35αA3-e) a protein comprising an amino acid sequence encoded by a nucleic acid that hybridizes under stringent conditions with a nucleic acid having a nucleotide sequence complementary to the nucleotide sequence of SEQ ID NO: 472;
(35αB3) a protein comprising at least one amino acid sequence selected from the group consisting of SEQ ID NOs: 474-477, preferably a protein comprising at least 2, 3, or all sequences of the amino acid sequences. As referred to above, the amino acid sequence of any of SEQ ID NOs: 474 to 477 may be an amino acid sequence derived therefrom by deletion, substitution, insertion or addition of one or several amino acids.

The protein selected from the group consisting of the proteins as defined above in (35αA1-a) to (35αA1-e), (35αB1), (35αA2-a) to (35αA2-e), (35αB2), (35αA3-a) to (35αA3-e), and (35αB3) also include those proteins whose amino acid residues are modified by phosphorylation, sugar chain modification, aminoacylation, ring-opening, deamination or the like.

The protein selected from the group consisting of the proteins as defined above in (35αA1-a) to (35αA1-e), (35αB1), (35αA2-a) to (35αA2-e), (35αB2), (35αA3-a) to (35αA3-e), and (35αB3) can be proteins that are found in a protein spot with a molecular weight of around 20 to 90 kDa, preferably around 30 to 80 kDa, and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 35α has activity equivalent to that of the protein having the amino acid sequence of SEQ ID NO: 460 or 468 (elongation factor 1-alpha) or the protein having the amino acid sequence of SEQ ID NO: 473 (elongation factor-1A isoform X1) or causative of an allergy to soybean.

### (36α, 37α) Antigens in spots 36α, 37α

As the result of sequence identification of the antigens in spots 36α and 37α by mass spectroscopy, the following amino acid sequences were detected.
EGSSIINTTSVNAYK (SEQ ID NO: 480)
FPPQQQQTQPGK (SEQ ID NO: 481)
GAIVAYTRGLALQLVSK (SEQ ID NO: 482)
LLDYTSTK (SEQ ID NO: 483)
EGATVAFTYVK (SEQ ID NO: 486)
QVIDLVVK (SEQ ID NO: 487)
EGSSIINTTSVNAYK (SEQ ID NO: 490)
FPPQQQQTQPGK (SEQ ID NO: 491).
GAIVAYTRGLALQLVSK (SEQ ID NO: 492)
LLDYTSTK (SEQ ID NO: 493)

Also, the mass spectroscopic data obtained for spots 36α and 37α on a mass spectrometer was analyzed by comparing the data against the NCBI protein data, and as a result, seed maturation protein PM34 (amino acid sequence: SEQ ID NO: 479, encoding nucleotide sequence: SEQ ID NO: 478) was identified as a protein comprising any of SEQ ID NOs: 480 to 483, uncharacterized protein LOC100809384 (amino acid sequence: SEQ ID NO: 485, encoding nucleotide sequence: SEQ ID NO: 484) was identified as a protein comprising SEQ ID NO: 486 or 487, and hypothetical protein GLYMA_10G155300 (amino acid sequence: SEQ ID NO: 489, encoding nucleotide sequence: SEQ ID NO: 488) was identified as a protein comprising any of SEQ ID NOs: 4490 to 493.

Accordingly, the antigen in spot 36α, 37α in the present invention can be any of proteins selected from the group consisting of the proteins as defined below in (36, 37αA1-a) to (36, 37αA1-e), (36, 37αB1), (36, 37αA2-a) to (36, 37αA2-e), (36, 37αB2), (36, 37αA3-a) to (36, 37αA3-e), and (36, 37αB3):
(36, 37αA1-a) a protein comprising an amino acid sequence with deletion, substitution, insertion or addition of one or several amino acids in SEQ ID NO: 479;
(36, 37αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 479;
(36, 37αA1-c) a protein comprising an amino acid sequence encoded by a nucleotide sequence with deletion, substitution, insertion or addition of one or several nucleotides in SEQ ID NO: 478;
(36, 37αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 478;
(36, 37αA1-e) a protein comprising an amino acid sequence encoded by a nucleic acid that hybridizes under stringent conditions with a nucleic acid having a nucleotide sequence complementary to the nucleotide sequence of SEQ ID NO: 478;
(36, 37αB1) a protein comprising at least one amino acid sequence selected from the group consisting of SEQ ID NOs: 480-483, preferably a protein comprising at least 2, 3, or all sequences of the amino acid sequences. As referred to above, the amino acid sequence of any of SEQ ID NOs: 480 to 483 may be an amino acid sequence derived therefrom by deletion, substitution, insertion or addition of one or several amino acids;
(36, 37αA2-a) a protein comprising an amino acid sequence with deletion, substitution, insertion or addition of one or several amino acids in SEQ ID NO: 485;
(36, 37αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 485;
(36, 37αA2-c) a protein comprising an amino acid sequence encoded by a nucleotide sequence with deletion, substitution, insertion or addition of one or several nucleotides in SEQ ID NO: 484;
(36, 37αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 484;
(36, 37αA2-e) a protein comprising an amino acid sequence encoded by a nucleic acid that hybridizes under stringent conditions with a nucleic acid having a nucleotide sequence complementary to the nucleotide sequence of SEQ ID NO: 484;
(36, 37αB2) a protein comprising at least one amino acid sequence selected from the group consisting of SEQ ID NOs: 486 and 487, preferably a protein comprising both the amino acid sequences. As referred to above, the amino acid sequence of any of SEQ ID NOs: 486, 487 may be an amino acid sequence derived therefrom by deletion, substitution, insertion or addition of one or several amino acids.
(36, 37αA3-a) a protein comprising an amino acid sequence with deletion, substitution, insertion or addition of one or several amino acids in SEQ ID NO: 489;
(36, 37αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 489;
(36, 37αA3-c) a protein comprising an amino acid sequence encoded by a nucleotide sequence with deletion, substitution, insertion or addition of one or several nucleotides in SEQ ID NO: 488;
(36, 37αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 488;
(36, 37αA3-e) a protein comprising an amino acid sequence encoded by a nucleic acid that hybridizes under stringent conditions with a nucleic acid having a nucleotide sequence complementary to the nucleotide sequence of SEQ ID NO: 488;
(36, 37αB3) a protein comprising at least one amino acid sequence selected from the group consisting of SEQ ID NOs: 490-493, preferably a protein comprising at least 2, 3, or all sequences of the amino acid sequences. As referred to above, the amino acid sequence of any of SEQ ID NOs: 490 to 493 may be an amino acid sequence derived therefrom by deletion, substitution, insertion or addition of one or several amino acids.

The protein selected from the group consisting of the proteins as defined above in (36, 37αA1-a) to (36, 37αA1-e), (36, 37αB1), (36, 37αA2-a) to (36, 37αA2-e), (36, 37αB2), (36, 37αA3-a) to (36, 37αA3-e), and (36, 37αB3) also include those proteins whose amino acid residues are modified by phosphorylation, sugar chain modification, aminoacylation, ring-opening, deamination or the like.

The protein selected from the group consisting of the proteins as defined above in (36, 37αA1-a) to (36, 37αA1-e), (36, 37αB1), (36, 37αA2-a) to (36, 37αA2-e), (36, 37αB2), (36, 37αA3-a) to (36, 37αA3-e), and (36, 37αB3) can be proteins that are found in a protein spot with a molecular weight of around 10 to 70 kDa, preferably around 20 to 60 kDa, and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 36α or 37α has activity equivalent to that of the protein having the amino acid sequence of SEQ ID NO: 479 (seed maturation protein PM34), the protein having the amino acid sequence of SEQ ID NO: 485 (uncharacterized protein LOC100809384), or the protein having the amino acid sequence of SEQ ID NO: 489 (hypothetical protein GLYMA_10G155300) or causative of an allergy to soybean.

### (38α) Antigen in spot 38α

As the result of sequence identification of the antigen in spot 38α by mass spectroscopy, the following amino acid sequences were detected.
AAGSTTAQYVGEK (SEQ ID NO: 496)
ALATGWSAAHYSTEITVEGTK (SEQ ID NO: 497)
AQVAGSTTVQYAGEK (SEQ ID NO: 498)
DTIGRVDNITTPLAK (SEQ ID NO: 499)
DTIGSVDHITTPLAEK (SEQ ID NO: 500)
ERELASDRARSGNIVK (SEQ ID NO: 501)
ERYERANQATSEAAK (SEQ ID NO: 502)
GGGEEQGRRREEIGEENK (SEQ ID NO: 503)
GLAAAAAGENAK (SEQ ID NO: 504)
GRVVIESEK (SEQ ID NO: 505)
GVAEYAGQK (SEQ ID NO: 506)
LGSAAHTLVK (SEQ ID NO: 507)
QSSLEEIFK (SEQ ID NO: 508)
QSSLEEISK (SEQ ID NO: 509)
REQKPLGSIIGESFQGVQEK (SEQ ID NO: 510)
TGNETQDIGQK (SEQ ID NO: 511)
VIVAGEGEK (SEQ ID NO: 512)
VQQHQGEGGGGVLNAIGETIAEIAETTK (SEQ ID NO: 513)

Also, the mass spectroscopic data obtained for spot 38α on a mass spectrometer was analyzed by comparing the data against the NCBI protein data, and as a result, seed biotin-containing protein SBP65-like isoform X1 (amino acid sequence: SEQ ID NO: 495, encoding nucleotide sequence: SEQ ID NO: 494) was identified as a protein comprising any of SEQ ID NOs: 496 to 513.

Accordingly, the antigens in spot 38α in the present invention can be any protein selected from the group consisting of the proteins as defined below in (38αA-a) to (38αA-e) and (38αB):
(38αA-a) a protein comprising an amino acid sequence with deletion, substitution, insertion or addition of one or several amino acids in SEQ ID NO: 495;
(38αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 495;
(38αA-c) a protein comprising an amino acid sequence encoded by a nucleotide sequence with deletion, substitution, insertion or addition of one or several nucleotides in SEQ ID NO: 494;
(38αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 494;
(38αA-e) a protein comprising an amino acid sequence encoded by a nucleic acid that hybridizes under stringent conditions with a nucleic acid having a nucleotide sequence complementary to the nucleotide sequence of SEQ ID NO: 494;
(38αB) a protein comprising at least one amino acid sequence selected from the group consisting of SEQ ID NOs: 496-513, preferably a protein comprising at least 2, 3, 4, 5, 6, 7, 8, 9, 10, 11, 12, 13, 14, 15, 16 or 17 or all sequences of the amino acid sequences. As referred to above, the amino acid sequence of any of SEQ ID NOs: 496-513 may be an amino acid sequence derived therefrom by deletion, substitution, insertion or addition of one or several amino acids.

The proteins selected from the group consisting of (38αA-a) to (38αA-e) and (38αB) as defined above also include those proteins whose amino acid residues are modified by phosphorylation, sugar chain modification, aminoacylation, ring-opening, deamination or the like.

The proteins selected from the group consisting of (38αA-a) to (38αA-e) and (38αB) as defined above can be proteins that are found in a protein spot with a molecular weight of around 70 to 170 kDa, preferably around 80 to 160 kDa and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 38α has activity equivalent to that of the protein having the amino acid sequence of SEQ ID NO: 495 (seed biotin-containing protein SBP65-like isoform XI) or causative of an allergy to soybean.

### (39α) Antigen in spot 39α

As the result of sequence identification of the antigen in spot 39α by mass spectroscopy, the following amino acid sequences were detected.
ETLQEIIGSGAK (SEQ ID NO: 516)

Also, the mass spectroscopic data obtained for spot 39α on a mass spectrometer was analyzed by comparing the data against the NCBI protein data, and as a result, ATP synthase subunit O, mitochondrial-like (amino acid sequence: SEQ ID NO: 515, encoding nucleotide sequence: SEQ ID NO: 514) was identified as a protein comprising SEQ ID NO: 516.

Accordingly, the antigens in spot 39α in the present invention can be any protein selected from the group consisting of the proteins as defined below in (39αA-a) to (39αA-e) and (39αB):
(39αA-a) a protein comprising an amino acid sequence with deletion, substitution, insertion or addition of one or several amino acids in SEQ ID NO: 515;
(39αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 515;
(39αA-c) a protein comprising an amino acid sequence encoded by a nucleotide sequence with deletion, substitution, insertion or addition of one or several nucleotides in SEQ ID NO: 514;
(39αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 514;
(39αA-e) a protein comprising an amino acid sequence encoded by a nucleic acid that hybridizes under stringent conditions with a nucleic acid having a nucleotide sequence complementary to the nucleotide sequence of SEQ ID NO: 514;
(39αB) a protein comprising an amino acid sequence of SEQ ID NO: 516. As referred to above, the amino acid sequence(s) of SEQ ID NO: 516 may be an amino acid sequence(s) derived therefrom by deletion, substitution, insertion or addition of one or several amino acids.

The proteins selected from the group consisting of (39αA-a) to (39αA-e) and (39αB) as defined above also include those proteins whose amino acid residues are modified by phosphorylation, sugar chain modification, aminoacylation, ring-opening, deamination or the like.

The proteins selected from the group consisting of (39αA-a) to (39αA-e) and (39αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 39α has activity equivalent to that of the protein having the amino acid sequence of SEQ ID NO: 515 (ATP synthase subunit O, mitochondrial-like) or causative of an allergy to soybean.

### (40α, 56α) Antigens in spots 40α, 56α

As the result of sequence identification of the antigens in spots 40α and 56α by mass spectroscopy, the following amino acid sequences were detected.
EVGQDIQSK (SEQ ID NO: 519)
RTTVTATTATA (SEQ ID NO: 520)

Also, the mass spectroscopic data obtained for spots 40α and 56α on a mass spectrometer was analyzed by comparing the data against the NCBI protein data, and as a result, P24 oleosin isoform A (amino acid sequence: SEQ ID NO: 518, encoding nucleotide sequence: SEQ ID NO: 517) was identified as a protein comprising SEQ ID NO: 519 or 520.

Accordingly, the antigens in spot 40α, 56α in the present invention can be any protein selected from the group consisting of the proteins as defined below in (40, 56αA-a) to (40, 56αA-e) and (40, 56αB):
(40, 56αA-a) a protein comprising an amino acid sequence with deletion, substitution, insertion or addition of one or several amino acids in SEQ ID NO: 518;
(40, 56αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 518;
(40, 56αA-c) a protein comprising an amino acid sequence encoded by a nucleotide sequence with deletion, substitution, insertion or addition of one or several nucleotides in SEQ ID NO: 517;
(40, 56αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 517;
(40, 56αA-e) a protein comprising an amino acid sequence encoded by a nucleic acid that hybridizes under stringent conditions with a nucleic acid having a nucleotide sequence complementary to the nucleotide sequence of SEQ ID NO: 517;
(40, 56αB) a protein comprising at least one amino acid sequence selected from the group consisting of SEQ ID NOs: 519 and 520, preferably a protein comprising both the amino acid sequences. As referred to above, the amino acid sequence of any of SEQ ID NOs: 519 and 520 may be an amino acid sequence derived therefrom by deletion, substitution, insertion or addition of one or several amino acids.

The proteins selected from the group consisting of (40, 56αA-a) to (40, 56αA-e) and (40, 56αB) as defined above also include those proteins whose amino acid residues are modified by phosphorylation, sugar chain modification, aminoacylation, ring-opening, deamination or the like.

The proteins selected from the group consisting of (40, 56αA-a) to (40, 56αA-e) and (40, 56αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 40α or 56α has activity equivalent to that of the protein having the amino acid sequence of SEQ ID NO: 518 (P24 oleosin isoform A) or causative of an allergy to soybean.

### (41α) Antigen in spot 41α

As the result of sequence identification of the antigen in spot 41α by mass spectroscopy, the following amino acid sequences were detected.
NAYGGSMK (SEQ ID NO: 523)

Also, the mass spectroscopic data obtained for spot 41α on a mass spectrometer was analyzed by comparing the data against the NCBI protein data, and as a result, uncharacterized protein At3g49720-like isoform X2 (amino acid sequence: SEQ ID NO: 522, encoding nucleotide sequence: SEQ ID NO: 521) was identified as a protein comprising SEQ ID NO: 523.

Accordingly, the antigens in spot 41α in the present invention can be any protein selected from the group consisting of the proteins as defined below in (41αA-a) to (41αA-e) and (41αB):
(41αA-a) a protein comprising an amino acid sequence with deletion, substitution, insertion or addition of one or several amino acids in SEQ ID NO: 522;
(41αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 522;
(41αA-c) a protein comprising an amino acid sequence encoded by a nucleotide sequence with deletion, substitution, insertion or addition of one or several nucleotides in SEQ ID NO: 521;
(41αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 521;
(41αA-e) a protein comprising an amino acid sequence encoded by a nucleic acid that hybridizes under stringent conditions with a nucleic acid having a nucleotide sequence complementary to the nucleotide sequence of SEQ ID NO: 521;
(41αB) a protein comprising an amino acid sequence of SEQ ID NO: 523. As referred to above, the amino acid sequence of SEQ ID NO: 523 may be an amino acid sequence derived therefrom by deletion, substitution, insertion or addition of one or several amino acids.

The proteins selected from the group consisting of (41αA-a) to (41αA-e) and (41αB) as defined above also include those proteins whose amino acid residues are modified by phosphorylation, sugar chain modification, aminoacylation, ring-opening, deamination or the like.

The proteins selected from the group consisting of (41αA-a) to (41αA-e) and (41αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 41α has activity equivalent to that of the protein having the amino acid sequence of SEQ ID NO: 515 (ATP synthase subunit O, mitochondrial-like) or causative of an allergy to soybean.

### (42α, 43α) Antigen in spots 42α, 43α

As the result of sequence identification of the antigens in spots 42α and 43α by mass spectroscopy, the following amino acid sequences were detected.
ALEQLQDAIAK (SEQ ID NO: 526)
HHQTYITNYNK (SEQ ID NO: 527)
NLAPVREGGGEPPK (SEQ ID NO: 528)
NVRPDYLK (SEQ ID NO: 529)
RLVVETTANQDPLVTK (SEQ ID NO: 530)
YASEVYEK (SEQ ID NO: 531)
ALEQLQDAVAK (SEQ ID NO: 534)
HHQTYITNFNK (SEQ ID NO: 535)
NLAPVREGGGEPPK (SEQ ID NO: 536)
NVRPDYLK (SEQ ID NO: 537)
RLVVETTANQDPLVTK (SEQ ID NO: 538)
YASEVYEK (SEQ ID NO: 539)

Also, the mass spectroscopic data obtained for spots 42α and 43α on a mass spectrometer was analyzed by comparing the data against the NCBI protein data, and as a result, superoxide dismutase [Mn], mitochondrial (amino acid sequence: SEQ ID NO: 525, encoding nucleotide sequence: SEQ ID NO: 524) was identified as a protein comprising any of SEQ ID NOs: 526 to 531 and hypothetical protein GLYMA_04G221300 (amino acid sequence: SEQ ID NO: 533, encoding nucleotide sequence: SEQ ID NO: 532) was identified as a protein comprising any of SEQ ID NOs: 534 to 539.

Accordingly, the antigen in spot 42α, 43α in the present invention can be any of proteins selected from the group consisting of the proteins as defined below in (42, 43αA1-a) to (42, 43αA1-e), (42, 43αB1), (42, 43αA2-a) to (42, 43αA2-e), and (42, 43αB2):
(42, 43αA1-a) a protein comprising an amino acid sequence with deletion, substitution, insertion or addition of one or several amino acids in SEQ ID NO: 525;
(42, 43αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 525;
(42, 43αA2-c) a protein comprising an amino acid sequence encoded by a nucleotide sequence with deletion, substitution, insertion or addition of one or several nucleotides in SEQ ID NO: 524;
(42, 43αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 524;
(42, 43αA1-e) a protein comprising an amino acid sequence encoded by a nucleic acid that hybridizes under stringent conditions with a nucleic acid having a nucleotide sequence complementary to the nucleotide sequence of SEQ ID NO: 524;
(42, 43αB1) a protein comprising at least one amino acid sequence selected from the group consisting of SEQ ID NOs: 526-531, preferably a protein comprising at least 2, 3, 4, or 5 or all sequences of the amino acid sequences. As referred to above, the amino acid sequence of any of SEQ ID NOs: 526 to 531 may be an amino acid sequence derived therefrom by deletion, substitution, insertion or addition of one or several amino acids;
(42, 43αA2-a) a protein comprising an amino acid sequence with deletion, substitution, insertion or addition of one or several amino acids in SEQ ID NO: 533;
(42, 43αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 533;
(42, 43αA2-c) a protein comprising an amino acid sequence encoded by a nucleotide sequence with deletion, substitution, insertion or addition of one or several nucleotides in SEQ ID NO: 532;
(42, 43αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 532;
(42, 43αA2-e) a protein comprising an amino acid sequence encoded by a nucleic acid that hybridizes under stringent conditions with a nucleic acid having a nucleotide sequence complementary to the nucleotide sequence of SEQ ID NO: 532;
(42, 43αB2) a protein comprising at least one amino acid sequence selected from the group consisting of SEQ ID NOs: 534-539, preferably a protein comprising at least 2, 3, 4, or 5 or all sequences of the amino acid sequences. As referred to above, the amino acid sequence of any of SEQ ID NOs: 534 to 539 may be an amino acid sequence derived therefrom by deletion, substitution, insertion or addition of one or several amino acids.

The protein selected from the group consisting of the proteins as defined above in (42, 43αA1-a) to (42, 43αA1-e), (42, 43αB1), (42, 43αA2-a) to (42, 43αA2-e), and (42, 43αB2) also include those proteins whose amino acid residues are modified by phosphorylation, sugar chain modification, aminoacylation, ring-opening, deamination or the like.

The protein selected from the group consisting of the proteins as defined above in (42, 43αA1-a) to (42, 43αA1-e), (42, 43αB1), (42, 43αA2-a) to (42, 43αA2-e), and (42, 43αB2) can be proteins that are found in a protein spot with a molecular weight of around 5 to 60 kDa, preferably around 10 to 50 kDa, and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 42α or 43α has activity equivalent to that of the protein having the amino acid sequence of SEQ ID NO: 525 (superoxide dismutase [Mn], mitochondrial) or the protein having the amino acid sequence of SEQ ID NO: 533 (hypothetical protein GLYMA_04G221300) or causative of an allergy to soybean.

### (45α) Antigen in spot 45α

As the result of sequence identification of the antigen in spot 45α by mass spectroscopy, the following amino acid sequences were detected.
HVPGFIERAEELK (SEQ ID NO: 542)
RFALLVEDLK (SEQ ID NO: 543)
VANVESGGEFTISSAEEIIK (SEQ ID NO: 544)
YTNALGLELDLTDK (SEQ ID NO: 545)
HVPGFIERAEELK (SEQ ID NO: 548)
RFALLVEDLK (SEQ ID NO: 549)
VANVESGGEFTISSAEEIIK (SEQ ID NO: 550)
YTNALGLELDLTDK (SEQ ID NO: 551)

Also, the mass spectroscopic data obtained for spot 45α on a mass spectrometer was analyzed by comparing the data against the NCBI protein data, and as a result, uncharacterized protein LOC100499771 (amino acid sequence: SEQ ID NO: 541, encoding nucleotide sequence: SEQ ID NO: 540) was identified as a protein comprising any of SEQ ID NOs: 542 to 545 and hypothetical protein GLYMA_09G192800 (amino acid sequence: SEQ ID NO: 547, encoding nucleotide sequence: SEQ ID NO: 546) was identified as a protein comprising any of SEQ ID NOs: 548 to 551.

Accordingly, the antigen in spot 45α in the present invention can be any of proteins selected from the group consisting of the proteins as defined below in (45αA1-a) to (45αA1-e), (45αB1), (45αA2-a) to (45αA2e), and (45αB2):
(45αA1-a) a protein comprising an amino acid sequence with deletion, substitution, insertion or addition of one or several amino acids in SEQ ID NO: 541;
(45αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 541;
(45αA1-c) a protein comprising an amino acid sequence encoded by a nucleotide sequence with deletion, substitution, insertion or addition of one or several nucleotides in SEQ ID NO: 540;
(45αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 540;
(45αA1-e) a protein comprising an amino acid sequence encoded by a nucleic acid that hybridizes under stringent conditions with a nucleic acid having a nucleotide sequence complementary to the nucleotide sequence of SEQ ID NO: 540;
(45αB1) a protein comprising at least one amino acid sequence selected from the group consisting of SEQ ID NOs: 542-545, preferably a protein comprising at least 2, 3, or all sequences of the amino acid sequences. As referred to above, the amino acid sequence of any of SEQ ID NOs: 542 to 545 may be an amino acid sequence derived therefrom by deletion, substitution, insertion or addition of one or several amino acids;
(45αA2-a) a protein comprising an amino acid sequence with deletion, substitution, insertion or addition of one or several amino acids in SEQ ID NO: 547;
(45αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 547;
(45αA2-c) a protein comprising an amino acid sequence encoded by a nucleotide sequence with deletion, substitution, insertion or addition of one or several nucleotides in SEQ ID NO: 546;
(45αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 546;
(45αA2-e) a protein comprising an amino acid sequence encoded by a nucleic acid that hybridizes under stringent conditions with a nucleic acid having a nucleotide sequence complementary to the nucleotide sequence of SEQ ID NO: 546;
(45αB2) a protein comprising at least one amino acid sequence selected from the group consisting of SEQ ID NOs: 548-551, preferably a protein comprising at least 2, 3, or all sequences of the amino acid sequences. As referred to above, the amino acid sequence of any of SEQ ID NOs: 548 to 551 may be an amino acid sequence derived therefrom by deletion, substitution, insertion or addition of one or several amino acids.

The protein selected from the group consisting of the proteins as defined above in (45αA1-a) to (45αA1-e), (45αB1), (45αA2-a) to (45αA2-e), and (45αB2) also include those proteins whose amino acid residues are modified by phosphorylation, sugar chain modification, aminoacylation, ring-opening, deamination or the like.

The protein selected from the group consisting of the proteins as defined above in (45αA1-a) to (45αA1-e), (45αB1), (45αA2-a) to (45αA2-e), and (45αB2) can be proteins that are found in a protein spot with a molecular weight of around 2 to 50 kDa, preferably around 5 to 40 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 45α has activity equivalent to that of the protein having the amino acid sequence of SEQ ID NO: 541 (uncharacterized protein LOC100499771) or the protein having the amino acid sequence of SEQ ID NO: 547 (hypothetical protein GLYMA_09G192800) or causative of an allergy to soybean.

### (46α) Antigen in spot 46α

As the result of sequence identification of the antigen in spot 46α by mass spectroscopy, the following amino acid sequences were detected.
DNVEYLAK (SEQ ID NO: 554)

Also, the mass spectroscopic data obtained for spot 46α on a mass spectrometer was analyzed by comparing the data against the NCBI protein data, and as a result, seed maturation protein PM22 (amino acid sequence: SEQ ID NO: 553, encoding nucleotide sequence: SEQ ID NO: 552) was identified as a protein comprising SEQ ID NO: 554.

Accordingly, the antigens in spot 46α in the present invention can be any protein selected from the group consisting of the proteins as defined below in (46αA-a) to (46αA-e) and (46αB):
(46αA-a) a protein comprising an amino acid sequence with deletion, substitution, insertion or addition of one or several amino acids in SEQ ID NO: 553;
(46αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 553;
(46αA-c) a protein comprising an amino acid sequence encoded by a nucleotide sequence with deletion, substitution, insertion or addition of one or several nucleotides in SEQ ID NO: 552;
(46αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 552;
(46αA-e) a protein comprising an amino acid sequence encoded by a nucleic acid that hybridizes under stringent conditions with a nucleic acid having a nucleotide sequence complementary to the nucleotide sequence of SEQ ID NO: 552;
(46αB) a protein comprising an amino acid sequence of SEQ ID NO: 554. As referred to above, the amino acid sequence(s) of SEQ ID NO: 554 may be an amino acid sequence(s) derived therefrom by deletion, substitution, insertion or addition of one or several amino acids.

The proteins selected from the group consisting of (46αA-a) to (46αA-e) and (46αB) as defined above also include those proteins whose amino acid residues are modified by phosphorylation, sugar chain modification, aminoacylation, ring-opening, deamination or the like.

The proteins selected from the group consisting of (46αA-a) to (46αA-e) and (46αB) as defined above can be proteins that are found in a protein spot with a molecular weight of around 2 to 50 kDa, preferably around 5 to 40 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 46α has activity equivalent to that of the protein having the amino acid sequence of SEQ ID NO: 553 (seed maturation protein PM22) or causative of an allergy to soybean.

### (47α) Antigen in spot 47α

As the result of sequence identification of the antigen in spot 47α by mass spectroscopy, the following amino acid sequences were detected.
LPTDESLLTQIK (SEQ ID NO: 557)
NGYIVIK (SEQ ID NO: 558)
NGYIVIK (SEQ ID NO: 561)
TYPQQAGTIRK (SEQ ID NO: 562)
VVEVSTSK (SEQ ID NO: 563)

Also, the mass spectroscopic data obtained for spot 47α on a mass spectrometer was analyzed by comparing the data against the NCBI protein data, and as a result, eukaryotic translation initiation factor 5A-2 (amino acid sequence: SEQ ID NO: 556, encoding nucleotide sequence: SEQ ID NO: 555) was identified as a protein comprising SEQ ID NO: 557 or 558 and uncharacterized protein LOC100305510 (amino acid sequence: SEQ ID NO: 560, encoding nucleotide sequence: SEQ ID NO: 559) was identified as a protein comprising any of SEQ ID NOs: 561 to 563.

Accordingly, the antigen in spot 47α in the present invention can be any of proteins selected from the group consisting of the proteins as defined below in (47αA1-a) to (47αA1-e), (47αB1), (47αA2-a) to (47αA2-e), and (47αB2):
(47αA1-a) a protein comprising an amino acid sequence with deletion, substitution, insertion or addition of one or several amino acids in SEQ ID NO: 556;
(47αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 556;
(47αA1-c) a protein comprising an amino acid sequence encoded by a nucleotide sequence with deletion, substitution, insertion or addition of one or several nucleotides in SEQ ID NO: 555;
(47αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 555;
(47αA1-e) a protein comprising an amino acid sequence encoded by a nucleic acid that hybridizes under stringent conditions with a nucleic acid having a nucleotide sequence complementary to the nucleotide sequence of SEQ ID NO: 555;
(47αB1) a protein comprising at least one amino acid sequence selected from the group consisting of SEQ ID NOs: 557 and 558, preferably a protein comprising both the amino acid sequences. As referred to above, the amino acid sequence of any of SEQ ID NOs: 557, 558 may be an amino acid sequence derived therefrom by deletion, substitution, insertion or addition of one or several amino acids;
(47αA2-a) a protein comprising an amino acid sequence with deletion, substitution, insertion or addition of one or several amino acids in SEQ ID NO: 560;
(47αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 560;
(47αA2-c) a protein comprising an amino acid sequence encoded by a nucleotide sequence with deletion, substitution, insertion or addition of one or several nucleotides in SEQ ID NO: 559;
(47αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 559;
(47αA2-e) a protein comprising an amino acid sequence encoded by a nucleic acid that hybridizes under stringent conditions with a nucleic acid having a nucleotide sequence complementary to the nucleotide sequence of SEQ ID NO: 559;
(47αB2) a protein comprising at least one amino acid sequence selected from the group consisting of SEQ ID NOs: 561-563, preferably a protein comprising at least 2, or all sequences of the amino acid sequences. As referred to above, the amino acid sequence of any of SEQ ID NOs: 548 to 551 may be an amino acid sequence derived therefrom by deletion, substitution, insertion or addition of one or several amino acids.

The protein selected from the group consisting of the proteins as defined above in (47αA1-a) to (47αA1-e), (47αB1), (47αA2-a) to (47αA2-e), and (47αB2) also include those proteins whose amino acid residues are modified by phosphorylation, sugar chain modification, aminoacylation, ring-opening, deamination or the like.

The protein selected from the group consisting of the proteins as defined above in (47αA1-a) to (47αA1-e), (47αB1), (47αA2-a) to (47αA2-e), and (47αB2) can be proteins that are found in a protein spot with a molecular weight of around 2 to 50 kDa, preferably around 5 to 40 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 47α has activity equivalent to that of the protein having the amino acid sequence of SEQ ID NO: 556 (eukaryotic translation initiation factor 5A-2) or the protein having the amino acid sequence of SEQ ID NO: 560 (uncharacterized protein LOC100305510) or causative of an allergy to soybean.

### (48α) Antigen in spot 48α

As the result of sequence identification of the antigen in spot 48α by mass spectroscopy, the following amino acid sequences were detected.
AEFPIFDK (SEQ ID NO: 566)
DARGNNVNLADYK (SEQ ID NO: 567)
DGNVVDRYAPTTSPLSIEK (SEQ ID NO: 568)
GGLFGDSIK (SEQ ID NO: 569)
VDVNGDNAAPLYK (SEQ ID NO: 570)

Also, the mass spectroscopic data obtained for spot 48α on a mass spectrometer was analyzed by comparing the data against the NCBI protein data, and as a result, uncharacterized protein LOC100306570 (amino acid sequence: SEQ ID NO: 565, encoding nucleotide sequence: SEQ ID NO: 564) was identified as a protein comprising any of SEQ ID NOs: 566 to 570.

Accordingly, the antigens in spot 48α in the present invention can be any protein selected from the group consisting of the proteins as defined below in (48αA-a) to (48αA-e) and (48αB):
(48αA-a) a protein comprising an amino acid sequence with deletion, substitution, insertion or addition of one or several amino acids in SEQ ID NO: 565;
(48αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 565;
(48αA-c) a protein comprising an amino acid sequence encoded by a nucleotide sequence with deletion, substitution, insertion or addition of one or several nucleotides in SEQ ID NO: 564;
(48αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 564;
(48αA-e) a protein comprising an amino acid sequence encoded by a nucleic acid that hybridizes under stringent conditions with a nucleic acid having a nucleotide sequence complementary to the nucleotide sequence of SEQ ID NO: 564;
(48αB) a protein comprising at least one amino acid sequence selected from the group consisting of SEQ ID NOs: 566-570, preferably a protein comprising at least 2, 3 or 4 or all sequences of the amino acid sequences. As referred to above, the amino acid sequence of any of SEQ ID NOs: 566-570 may be an amino acid sequence derived therefrom by deletion, substitution, insertion or addition of one or several amino acids.

The proteins selected from the group consisting of (48αA-a) to (48αA-e) and (48αB) as defined above also include those proteins whose amino acid residues are modified by phosphorylation, sugar chain modification, aminoacylation, ring-opening, deamination or the like.

The proteins selected from the group consisting of (48αA-a) to (48αA-e) and (48αB) as defined above can be proteins that are found in a protein spot with a molecular weight of around 2 to 50 kDa, preferably around 5 to 40 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 48α has activity equivalent to that of the protein having the amino acid sequence of SEQ ID NO: 565 (uncharacterized protein LOC100306570) or causative of an allergy to soybean.

### (50α) Antigen in spot 50α

As the result of sequence identification of the antigen in spot 50α by mass spectroscopy, the following amino acid sequences were detected.
AADYLHNYQGDNTTAPSQPAESK (SEQ ID NO: 573)
LVAEAAQSALK (SEQ ID NO: 574)
QGIGQYVDK (SEQ ID NO: 575)
VADAAGDLLDAAGK (SEQ ID NO: 576)

Also, the mass spectroscopic data obtained for spot 50α on a mass spectrometer was analyzed by comparing the data against the NCBI protein data, and as a result, uncharacterized protein LOC100813859 (amino acid sequence: SEQ ID NO: 572, encoding nucleotide sequence: SEQ ID NO: 571) was identified as a protein comprising any of SEQ ID NOs: 573 to 576.

Accordingly, the antigens in spot 50α in the present invention can be any protein selected from the group consisting of the proteins as defined below in (50αA-a) to (50αA-e) and (50αB):
(50αA-a) a protein comprising an amino acid sequence with deletion, substitution, insertion or addition of one or several amino acids in SEQ ID NO: 572;
(50αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 572;
(50αA-c) a protein comprising an amino acid sequence encoded by a nucleotide sequence with deletion, substitution, insertion or addition of one or several nucleotides in SEQ ID NO: 571;
(50αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 571;
(50αA-e) a protein comprising an amino acid sequence encoded by a nucleic acid that hybridizes under stringent conditions with a nucleic acid having a nucleotide sequence complementary to the nucleotide sequence of SEQ ID NO: 571;
(50αB) a protein comprising at least one amino acid sequence selected from the group consisting of SEQ ID NOs: 573-576, preferably a protein comprising at least 2 or 3 or all sequences of the amino acid sequences. As referred to above, the amino acid sequence of any of SEQ ID NOs: 573-576 may be an amino acid sequence derived therefrom by deletion, substitution, insertion or addition of one or several amino acids.

The proteins selected from the group consisting of (50αA-a) to (50αA-e) and (50αB) as defined above also include those proteins whose amino acid residues are modified by phosphorylation, sugar chain modification, aminoacylation, ring-opening, deamination or the like.

The proteins selected from the group consisting of (50αA-a) to (50αA-e) and (50αB) as defined above can be proteins that are found in a protein spot with a molecular weight of around 2 to 50 kDa, preferably around 5 to 40 kDa and an isoelectric point of 2.5 to 6.5, preferably 3.0 to 6.0 on gels used in the two-dimensional electrophoresis performed under the conditions described above in the subsection titled "Identification of antigens".

Preferably, a protein that is an antigen in spot 50α has activity equivalent to that of the protein having the amino acid sequence of SEQ ID NO: 572 (uncharacterized protein LOCI 00813859) or causative of an allergy to soybean.

### (51α) Antigen in spot 51α

As the result of sequence identification of the antigen in spot 51α by mass spectroscopy, the following amino acid sequences were detected.
EAAESTLSAYK (SEQ ID NO: 579)
LLDSRLIPSASSGDSK (SEQ ID NO: 580)
QAFDEAIAELDTLGEESYK (SEQ ID NO: 581)
VSAAADNEELTVEERNLLSVAYK (SEQ ID NO: 582)

Also, the mass spectroscopic data obtained for spot 51α on a mass spectrometer was analyzed by comparing the data against the NCBI protein data, and as a result, 14-3-3-like protein (amino acid sequence: SEQ ID NO: 578, encoding nucleotide sequence: SEQ ID NO: 577) was identified as a protein comprising any of SEQ ID NOs: 579 to 582.

Accordingly, the antigens in spot 51α in the present invention can be any protein selected from the group consisting of the proteins as defined below in (51αA-a) to (51αA-e) and (51αB):
(51αA-a) a protein comprising an amino acid sequence with deletion, substitution, insertion or addition of one or several amino acids in SEQ ID NO: 578;
(51αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 578;
(51αA-c) a protein comprising an amino acid sequence encoded by a nucleotide sequence with deletion, substitution, insertion or addition of one or several nucleotides in SEQ ID NO: 577;
(51αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 577;
(51αA-e) a protein comprising an amino acid sequence encoded by a nucleic acid that hybridizes under stringent conditions with a nucleic acid having a nucleotide sequence complementary to the nucleotide sequence of SEQ ID NO: 577;
(51αB) a protein comprising at least one amino acid sequence selected from the group consisting of SEQ ID NOs: 579-582, preferably a protein comprising at least 2 or 3 or all sequences of the amino acid sequences. As referred to above, the amino acid sequence of any of SEQ ID NOs: 579-582 may be an amino acid sequence derived therefrom by deletion, substitution, insertion or addition of one or several amino acids.

The proteins selected from the group consisting of (51αA-a) to (51αA-e) and (51α8) as defined above also include those proteins whose amino acid residues are modified by phosphorylation, sugar chain modification, aminoacylation, ring-opening, deamination or the like.

The proteins selected from the group consisting of (51αA-a) to (51αA-e) and (51αB) as defined above can be proteins that are found in a protein spot with a molecular weight of around 5 to 60 kDa, preferably around 10 to 50 kDa and an isoelectric point of 2.5 to 6.5, preferably 3.0 to 6.0 on gels used in the two-dimensional electrophoresis performed under the conditions described above in the subsection titled "Identification of antigens".

Preferably, a protein that is an antigen in spot 51α has activity equivalent to that of the protein having the amino acid sequence of SEQ ID NO: 578 (14-3-3-like protein) or causative of an allergy to soybean.

### (52α) Antigen in spot 52α

As the result of sequence identification of the antigen in spot 52α by mass spectroscopy, the following amino acid sequences were detected.
APGGAPANVAIAVSRLGGK (SEQ ID NO: 585)
GSVDAFHVNTVDTTGAGDSFVGALLAK (SEQ ID NO: 586)
IVDDQSILEDEPRLREVLK (SEQ ID NO: 587)
VSDAELEFLTGSDK (SEQ ID NO: 588)

Also, the mass spectroscopic data obtained for spot 52α on a mass spectrometer was analyzed by comparing the data against the NCBI protein data, and as a result, probable fructokinase-4 (amino acid sequence: SEQ ID NO: 584, encoding nucleotide sequence: SEQ ID NO: 583) was identified as a protein comprising any of SEQ ID NOs: 585 to 588.

Accordingly, the antigens in spot 52α in the present invention can be any protein selected from the group consisting of the proteins as defined below in (52αA-a) to (52αA-e) and (52αB):
(52αA-a) a protein comprising an amino acid sequence with deletion, substitution, insertion or addition of one or several amino acids in SEQ ID NO: 584;
(52αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 584;
(52αA-c) a protein comprising an amino acid sequence encoded by a nucleotide sequence with deletion, substitution, insertion or addition of one or several nucleotides in SEQ ID NO: 583;
(52αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 583;
(52αA-e) a protein comprising an amino acid sequence encoded by a nucleic acid that hybridizes under stringent conditions with a nucleic acid having a nucleotide sequence complementary to the nucleotide sequence of SEQ ID NO: 583;
(52αB) a protein comprising at least one amino acid sequence selected from the group consisting of SEQ ID NOs: 585-588, preferably a protein comprising at least 2 or 3 or all sequences of the amino acid sequences. As referred to above, the amino acid sequence of any of SEQ ID NOs: 585-588 may be an amino acid sequence derived therefrom by deletion, substitution, insertion or addition of one or several amino acids.

The proteins selected from the group consisting of (52αA-a) to (52αA-e) and (52αB) as defined above also include those proteins whose amino acid residues are modified by phosphorylation, sugar chain modification, aminoacylation, ring-opening, deamination or the like.

The proteins selected from the group consisting of (52αA-a) to (52αA-e) and (52αB) as defined above can be proteins that are found in a protein spot with a molecular weight of around 5 to 60 kDa, preferably around 10 to 50 kDa and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 52α has activity equivalent to that of the protein having the amino acid sequence of SEQ ID NO: 584 (probable fructokinase-4) or causative of an allergy to soybean.

### (53α) Antigen in spot 53α

As the result of sequence identification of the antigen in spot 53α by mass spectroscopy, the following amino acid sequences were detected.
AEFVDIINAATVK (SEQ ID NO: 591)
ELAAQPDVDGFLVGGASLK (SEQ ID NO: 592)
FFVGGNWK (SEQ ID NO: 593)
IVTTLNEAK (SEQ ID NO: 594)
VATPAQAQEVHADLRK (SEQ ID NO: 595)
VAYALQQGLK (SEQ ID NO: 596)
VPGEDVVEVVVSPPFVFLPFVK (SEQ ID NO: 597)
ELAAQPDVDGFLVGGASLKPEFVDIINAATVK (SEQ ID NO: 600)
FFVGGNWK (SEQ ID NO: 601)
IVTTLNEAK (SEQ ID NO: 602)
VATPAQAQEVHADLRK (SEQ ID NO: 603)
VAYALQQGLK (SEQ ID NO: 604)

Also, the mass spectroscopic data obtained for spot 53α on a mass spectrometer was analyzed by comparing the data against the NCBI protein data, and as a result, triosephosphate isomerase isoform X1 (amino acid sequence: SEQ ID NO: 590, encoding nucleotide sequence: SEQ ID NO: 589) was identified as a protein comprising any of SEQ ID NOs: 591 to 597 and triosephosphate isomerase, cytosolic (amino acid sequence: SEQ ID NO: 599, encoding nucleotide sequence: SEQ ID NO: 598) was identified as a protein comprising any of SEQ ID NOs: 600 to 604.

Accordingly, the antigen in spot 53α in the present invention can be any of proteins selected from the group consisting of the proteins as defined below in (53αA1-a) to (53αA1-e), (53αB1), (53αA2-a) to (53αA2-e), and (53αB2):
(53αA1-a) a protein comprising an amino acid sequence with deletion, substitution, insertion or addition of one or several amino acids in SEQ ID NO: 590;
(53αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 590;
{53αA1-c) a protein comprising an amino acid sequence encoded by a nucleotide sequence with deletion, substitution, insertion or addition of one or several nucleotides in SEQ ID NO: 589;
(53αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 589;
(53αA1-c) a protein comprising an amino acid sequence encoded by a nucleic acid that hybridizes under stringent conditions with a nucleic acid having a nucleotide sequence complementary to the nucleotide sequence of SEQ ID NO: 589;
(53αB1) a protein comprising at least one amino acid sequence selected from the group consisting of SEQ ID NOs: 591-597, preferably a protein comprising at least 2, 3, 4, 5, or 6 or all sequences of the amino acid sequences. As referred to above, the amino acid sequence of any of SEQ ID NOs: 591 to 597 may be an amino acid sequence derived therefrom by deletion, substitution, insertion or addition of one or several amino acids;
(53αA2-a) a protein comprising an amino acid sequence with deletion, substitution, insertion or addition of one or several amino acids in SEQ ID NO: 599;
(53αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 599;
(53αA2-c) a protein comprising an amino acid sequence encoded by a nucleotide sequence with deletion, substitution, insertion or addition of one or several nucleotides in SEQ ID NO: 598;
(53αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 598;
(53αA2-e) a protein comprising an amino acid sequence encoded by a nucleic acid that hybridizes under stringent conditions with a nucleic acid having a nucleotide sequence complementary to the nucleotide sequence of SEQ ID NO: 598;
(53αB2) a protein comprising at least one amino acid sequence selected from the group consisting of SEQ ID NOs: 600-604, preferably a protein comprising at least 2, 3, or 4 or all sequences of the amino acid sequences. As referred to above, the amino acid sequence of any of SEQ ID NOs: 600 to 604 may be an amino acid sequence derived therefrom by deletion, substitution, insertion or addition of one or several amino acids.

The protein selected from the group consisting of the proteins as defined above in (53αA1-a) to (53αA1-e), (53αB1), (53αA2-a) to (53αA2-e), and (53αB2) also include those proteins whose amino acid residues are modified by phosphorylation, sugar chain modification, aminoacylation, ring-opening, deamination or the like.

The protein selected from the group consisting of the proteins as defined above in (53αA1-a) to (53αA1-e), (53αB1), (53αA2-a) to (53αA2-e), and (53αB2) can be proteins that are found in a protein spot with a molecular weight of around 5 to 60 kDa, preferably around 10 to 50 kDa, and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 53α has activity equivalent to that of the protein having the amino acid sequence of SEQ ID NO: 590 (triosephosphate isomerase isoform X1) or the protein having the amino acid sequence of SEQ ID NO: 599 (triosephosphate isomerase, cytosolic) or causative of an allergy to soybean.

### (54α) Antigen in spot 54α

As the result of sequence identification of the antigen in spot 54α by mass spectroscopy, the following amino acid sequences were detected.
FDGENVANPPSPDEDNK (SEQ ID NO: 607)
LVSWDAVSSRLEQAK (SEQ ID NO: 608)
QVVGTELDGK (SEQ ID NO: 609)
SLEEIIVTSYNK (SEQ ID NO: 610)
TYVENLK (SEQ ID NO: 611)
QIVGTELDGK (SEQ ID NO: 614)
SLEEIIVTSYNK (SEQ ID NO: 615)
TYVENLK (SEQ ID NO: 616)

Also, the mass spectroscopic data obtained for spot 54α on a mass spectrometer was analyzed by comparing the data against the NCBI protein data, and as a result, superoxide dismutase [Fe], chloroplastic isoform X1 (amino acid sequence: SEQ ID NO: 606, encoding nucleotide sequence: SEQ ID NO: 605) as a protein comprising any of SEQ ID NOs: 607 to 611 and iron-superoxide dismutase (amino acid sequence: SEQ ID NO: 613, encoding nucleotide sequence: SEQ ID NO: 612) was identified as a protein comprising any of SEQ ID NOs: 614 to 616.

Accordingly, the antigen in spot 54α in the present invention can be any of proteins selected from the group consisting of the proteins as defined below in (54αA1-a) to (54αA1-e), (54αB1), (54αA2-a) to (54αA2-e), and (54αB2):
(54αA1-a) a protein comprising an amino acid sequence with deletion, substitution, insertion or addition of one or several amino acids in SEQ ID NO: 606;
(54αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 606;
(54αA1-c) a protein comprising an amino acid sequence encoded by a nucleotide sequence with deletion, substitution, insertion or addition of one or several nucleotides in SEQ ID NO: 605;
(54αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 605;
(54αA1-e) a protein comprising an amino acid sequence encoded by a nucleic acid that hybridizes under stringent conditions with a nucleic acid having a nucleotide sequence complementary to the nucleotide sequence of SEQ ID NO: 605;
(54αB1) a protein comprising at least one amino acid sequence selected from the group consisting of SEQ ID NOs: 607-611, preferably a protein comprising at least 2, 3, or 4 or all sequences of the amino acid sequences. As referred to above, the amino acid sequence of any of SEQ ID NOs: 591 to 597 may be an amino acid sequence derived therefrom by deletion, substitution, insertion or addition of one or several amino acids;
(54αA2-a) a protein comprising an amino acid sequence with deletion, substitution, insertion or addition of one or several amino acids in SEQ ID NO: 613;
(54αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 613;
(54αA2-c) a protein comprising an amino acid sequence encoded by a nucleotide sequence with deletion, substitution, insertion or addition of one or several nucleotides in SEQ ID NO: 612;
(54αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 612;
(54αA2-e) a protein comprising an amino acid sequence encoded by a nucleic acid that hybridizes under stringent conditions with a nucleic acid having a nucleotide sequence complementary to the nucleotide sequence of SEQ ID NO: 612;
(54αB2) a protein comprising at least one amino acid sequence selected from the group consisting of SEQ ID NOs: 614-616, preferably a protein comprising at least 2, or all sequences of the amino acid sequences. As referred to above, the amino acid sequence of any of SEQ ID NOs: 614 to 616 may be an amino acid sequence derived therefrom by deletion, substitution, insertion or addition of one or several amino acids.

The protein selected from the group consisting of the proteins as defined above in (54αA1-a) to (54αA1-e), (54αB1), (54αA2-a) to (54αA2-e), and (54αB2) also include those proteins whose amino acid residues are modified by phosphorylation, sugar chain modification, aminoacylation, ring-opening, deamination or the like.

The protein selected from the group consisting of the proteins as defined above in (54αA1-a) to (54αA1-e), (54αB1), (54αA2-a) to (54αA2-e), and (54αB2) can be proteins that are found in a protein spot with a molecular weight of around 5 to 60 kDa, preferably around 10 to 50 kDa, and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 54α has activity equivalent to that of the protein having the amino acid sequence of SEQ ID NO: 606 (superoxide dismutase [Fe], chloroplastic isoform X1) or the protein having the amino acid sequence of SEQ ID NO: 613 (iron-superoxide dismutase) or causative of an allergy to soybean.

### (55α) Antigen in spot 55α

As the result of sequence identification of the antigen in spot 55α by mass spectroscopy, the following amino acid sequences were detected.
DYAGDAAQNVK (SEQ ID NO: 619)
DYASDAAQK (SEQ ID NO: 620)
DYASDATDAAK (SEQ ID NO: 621)
DYASDAVQK (SEQ ID NO: 622)
DYASDTAQRTK (SEQ ID NO: 623)
DYASDTAQTSK (SEQ ID NO: 624)
DYASEASDVAQNTK (SEQ ID NO: 625)
DYATDAAQK (SEQ ID NO: 626)
DYVGDAAQRSK (SEQ ID NO: 627)
EASDYASETAK (SEQ ID NO: 628)
EYAGDVALNAK (SEQ ID NO: 629)
EYVGDAAQK (SEQ ID NO: 630)
GAAEYASDAAQRTK (SEQ ID NO: 631)
GYVGDAAQK (SEQ ID NO: 632)
LQDIASEAGQYSAEK (SEQ ID NO: 633)
LSEGLGFK (SEQ ID NO: 634)
VSDYATDTAQK (SEQ ID NO: 635)
DYAGDAVQNIK (SEQ ID NO: 638)
DYASDAAQRTK (SEQ ID NO: 639)
DYASDATDAAK (SEQ ID NO: 640)
DYASDTAQRTK (SEQ ID NO: 641)
DYVGDAAQK (SEQ ID NO: 642)
EASDYASETAQK (SEQ ID NO: 643)
EASEYASDAAQRTK (SEQ ID NO: 644)
EYAGDVAQNAK (SEQ ID NO: 645)
GYVGDAAQK (SEQ ID NO: 646)
LQDIASEAGQYSTEK (SEQ ID NO: 647)
LTEGLGFK (SEQ ID NO: 648)
VSDYAGDAAQK (SEQ ID NO: 649)

Also, the mass spectroscopic data obtained for spot 55α on a mass spectrometer was analyzed by comparing the data against the NCBI protein data, and as a result, 51 kDa seed maturation protein precursor (amino acid sequence: SEQ ID NO: 618, encoding nucleotide sequence: SEQ ID NO: 617) was identified as a protein comprising any of SEQ ID NOs: 619 to 635 and Lea protein precursor (amino acid sequence: SEQ ID NO: 637, encoding nucleotide sequence: SEQ ID NO: 636) was identified as a protein comprising any of SEQ ID NOs: 638 to 649.

Accordingly, the antigen in spot 55α in the present invention can be any of proteins selected from the group consisting of the proteins as defined below in (55αA1-a) to (55αA1-e), (55αB1), (55αA2-a) to (55αA2-e), and (55αB2):
(55αA1-a) a protein comprising an amino acid sequence with deletion, substitution, insertion or addition of one or several amino acids in SEQ ID NO: 618;
(55αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 618;
(55αA1-c) a protein comprising an amino acid sequence encoded by a nucleotide sequence with deletion, substitution, insertion or addition of one or several nucleotides in SEQ ID NO: 617;
(55αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 617;
(55αA1-e) a protein comprising an amino acid sequence encoded by a nucleic acid that hybridizes under stringent conditions with a nucleic acid having a nucleotide sequence complementary to the nucleotide sequence of SEQ ID NO: 617;
(55αB1) a protein comprising at least one amino acid sequence selected from the group consisting of SEQ ID NOs: 619-635, preferably a protein comprising at least 2, 3, 4, 5, 6, 7, 8, 9, 10, 11, 12, 13, 14, 15, or 16 or all sequences of the amino acid sequences. As referred to above, the amino acid sequence of any of SEQ ID NOs: 619 to 635 may be an amino acid sequence derived therefrom by deletion, substitution, insertion or addition of one or several amino acids;
(55αA2-a) a protein comprising an amino acid sequence with deletion, substitution, insertion or addition of one or several amino acids in SEQ ID NO: 637;
(55αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 637;
(55αA2-c) a protein comprising an amino acid sequence encoded by a nucleotide sequence with deletion, substitution, insertion or addition of one or several nucleotides in SEQ ID NO: 636;
(55αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 636;
(55αA2-e) a protein comprising an amino acid sequence encoded by a nucleic acid that hybridizes under stringent conditions with a nucleic acid having a nucleotide sequence complementary to the nucleotide sequence of SEQ ID NO: 636;
(55αB2) a protein comprising at least one amino acid sequence selected from the group consisting of SEQ ID NOs: 638-649, preferably a protein comprising at least 2, 3, 4, 5, 6, 7, 8, 9, 10, or 11 or all sequences of the amino acid sequences. As referred to above, the amino acid sequence of any of SEQ ID NOs: 638 to 649 may be an amino acid sequence derived therefrom by deletion, substitution, insertion or addition of one or several amino acids.

The protein selected from the group consisting of the proteins as defined above in (55αA1-a) to (55αA1-e), (55αB1), (55αA2-a) to (55αA2- e), and (55αB2) also include those proteins whose amino acid residues are modified by phosphorylation, sugar chain modification, aminoacylation, ring-opening, deamination or the like.

The protein selected from the group consisting of the proteins as defined above in (55αA1-a) to (55αA1-e), (55αB1), (55αA2-a) to (55αA2-e), and (55αB2) can be proteins that are found in a protein spot with a molecular weight of around 30 to 90 kDa, preferably around 40 to 80 kDa, and an isoelectric point of 5.5 to 10.5, preferably 6.0 to 10.0 on gels used in the two-dimensional electrophoresis performed under the conditions described above in the subsection titled "Identification of antigens".

Preferably, a protein that is an antigen in spot 55α has activity equivalent to that of the protein having the amino acid sequence of SEQ ID NO: 618 (51 kDa seed maturation protein precursor) or the protein having the amino acid sequence of SEQ ID NO: 637 (Lea protein precursor) or causative of an allergy to soybean.

By stating herein "deletion, substitution, insertion or addition of one or several amino acids" in relation to amino acid sequence, it is meant that in an amino acid sequence of interest, one or several amino acids (e.g., 30%, preferably 25%, 20%, 15%, 10%, 5%, 3%, 2%, or 1% of amino acids with respect to the total length of the amino acid sequence) are deleted, one or several amino acids are substituted by any other amino acids, any other amino acids are inserted, and/or any other amino acids are added.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine
Group B: aspartic acid, glutamic acid
Group C: asparagine, glutamine
Group D: lysine, arginine
Group E: serine, threonine
Group F: phenylalanine, tyrosine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol.157, p.105-132, 1982).

As referred to herein, the percent identity between two amino acid sequences can be determined by visual inspection and mathematical calculation. Alternatively, the percent identity can be determined using a computer program. Examples of such computer programs include BLAST FASTA (Altschul, et al., J. Mol. Biol., 215: 403-410 (1990)), and ClustalW. In particular, various conditions (parameters) for identity searches with the BLAST program are described in Altschul, et al. (Nucl. Acids. Res., 25, p.3389-3402, 1997), and are publicly available on the websites of the National Center for Biotechnology Information (NCBI) and DNA Data Bank of Japan (DDBJ) (Altschul, et al., BLAST Handbook, Altschul, et al., NCB/NLM/NIH Bethesda, MD 20894). Also, the percent identity can be determined using a genetic information processing software program, such as GENETYX Ver.7 (Genetyx Corporation), DINASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides (e.g., 30%, preferably 25%, 20%, 15%, 10%, 5%, 3%, 2% or 1%, of amino acids with respect to the total length of the nucleotide sequence) are deleted, one or several nucleotides are substituted by any other nucleotides, any other nucleotides are inserted, and/or any other nucleotides are added. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. Examples of such sequence comparison computer programs include the BLASTN program, version 2.2.7, available on the website of the National Library of Medicine (http://www.ncbi.nlm.nih.gov/blast/bl2seq/bls.html) (Altschul, et al. (1990) J. Mol. Biol., 215: 403-10), or the WU-BLAST 2.0 algorithm. Standard default parameter settings for WU-BLAST 2.0 are found and available on the following website: http://blast.wustl.edu.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6xSSC at 42°C to 55°C, more preferably 1×SSC to 3xSSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5×SSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of 0.1×SSC to 2×SSC at 55°C to 65°C, more preferably 0.1×SSC to 1×SSC at 60°C to 65°C, most preferably 0.2×SSC at 63°C. Washing is carried out under the conditions of 0.2×SSC to 2×SSC at 50°C to 68°C, more preferably 0.2×SSC at 60 to 65°C.

Antigens may be obtained by separating and purifying them from soybean using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced therethrough. Exemplary vectors include plasmid vectors and viral vectors. Those skilled in the art can select an appropriate expression vector for the expression of a recombinant protein depending on the type of host cells to be used.

The "host cells" refers to cells that undergo gene transfer or genetic transformation by a vector. The host cells can be appropriately selected by those skilled in the art depending on the type of the vector to be used. The host cells can be derived from prokaryotes such as E. coli. When prokaryotic cells like E coli. are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above.

### Diagnosis kit and method

The present invention provides a method for providing an indicator for diagnosing an allergy to a soybean in a subject, the method comprising the steps of:
(i) contacting a sample obtained from the subject with an antigen, wherein the sample is a solution comprising an Ig antibody;
(ii) detecting binding between an IgE antibody present in the sample from the subject and the antigen; and
(iii) when the binding between the IgE antibody in the subject and the antigen is detected, an indicator of the fact that the subject is allergic to a soybean is provided;
wherein the antigen is at least one of proteins as defined above in any of (1) to (8) and (1α) to (55α).

The sample obtained from a subject is a solution containing an Ig antibody, preferably an IgE antibody, as collected from the subject. Examples of such solutions include blood, saliva, sputum, snivel, urine, sweat, and tear. The sample obtained from the subject may be subjected to pretreatment for increasing the concentration of an Ig antibody in the sample before being contacted with an antigen. The pretreatment of a sample may involve, for example, collection of the serum from the blood. In a particularly preferred mode, the step (i) mentioned above is carried out by contacting an IgE antibody present in the serum obtained from a subject with an antigen.

Detection of contact and binding between the sample obtained from a subject and an antigen can be carried out by using a known method. Examples of such methods that can be used include detection by ELISA (Enzyme-Linked Immunosorbent Assay), sandwich immunoassay, immunoblotting, or immunoprecipitation. These are all techniques for detecting binding between an antigen and an IgE antibody from a subject by contacting and binding the IgE antibody from a subject with the antigen, allowing an enzymatically labelled secondary antibody to act on the IgE antibody specifically bound to the antigen, and adding an enzyme substrate (generally, chromogenic or luminescent reagent) to detect an enzymatic reaction product. Also, a method for detecting a fluorescently labeled secondary antibody can be used. Furthermore, a method for detecting antigen-antibody binding as a refraction angle using surface plasmon resonance can be used.

The antigen may be provided as an isolated antigen in a state immobilized on a carrier. In this case, the steps (i) and (ii) mentioned above can be carried out using ELISA, sandwich immunoassay, or the like. Also, the step (i) mentioned above can be carried out by contacting the sample obtained from a subject with an antigen-immobilized surface. The isolated antigen may be obtained by separating and purifying it from soybean using a combination of protein purification methods well known to those skilled in the art, or by preparing it using a genetic recombination technique.

The antigen may be detected by immunoblotting in a state separated by two-dimensional electrophoresis. The two-dimensional electrophoresis is a technique for separating a protein sample by performing isoelectric focusing in the first dimension and performing SDS-PAGE (SDS-polyacrylamide gel electrophoresis) in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be defined by reference to the descriptions in Patent Literatures 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (1) to (8) and (1α) to (55α) are antigens that specifically bind to IgE antibodies from patients with allergy to soybean. Therefore, when binding between an IgE antibody from a subject and the antigen is detected, an indicator of the fact that the subject is allergic to soybean is provided.

The present invention further provides a kit for diagnosing an allergy to a soybean, comprising at least one of the aforementioned antigens (1) to (8) and (1α) to (55α). The diagnosis kit of this invention may be used in the aforementioned method for providing an indicator for diagnosing an allergy to a soybean or in a diagnosis method as described later. The diagnosis kit of this invention may comprise not only the at least one of the aforementioned antigens (1) to (8) and (1α) to (55α), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

The present invention further provides a composition for diagnosing an allergy to a soybean, comprising at least one of the aforementioned antigens (1) to (8) and (1α) to (55α). The diagnosis composition of this invention can be used in a diagnosis method as described below. The diagnosis composition of this invention may further comprise a pharmaceutically acceptable carrier and/or additives commonly used with the antigen of this invention depending on the need.

In one mode, the present invention provides a method for diagnosing an allergy to a soybean in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic to a soybean;
wherein the antigen is at least one of proteins as defined above in any of (1) to (8) and (1α) to (55α). In this method, the steps (i) and (ii) are performed as described above regarding the corresponding steps of the method for providing an indicator for diagnosing an allergy to a soybean.

In another mode, the present invention provides a method for diagnosing an allergy to a soybean in a subject, the method comprising administering to the subject at least one of the aforementioned antigens (1) to (8) and (1α) to (55α). This method may be performed in the form of a skin test characterized by applying the antigen onto the skin. Examples of the skin test include various forms of tests, such as: a prick test in which a diagnosis composition is applied onto the skin and then a tiny prick to such an extent as not to provoke bleeding is made in the skin to allow an antigen to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which an antigen is administered intracutaneously to observe a reaction. If a skin reaction such as swelling occurs in a skin portion to which the antigen has been applied, the subject of interest is diagnosed as having an allergy to a soybean. The amount of the antigen to be applied to the skin in such tests can be, for example, not more than 100 µg per dose.

In yet another mode, the present invention provides at least one of the aforementioned antigens (1) to (8) and (1α) to (55α), intended for use in the diagnosis of an allergy to a soybean.

In still another mode, the present invention provides use of at least one of the aforementioned antigens (1) to (8) and (1α) to (55α) for the production of a diagnostic agent for an allergy to a soybean.

### Pharmaceutical composition and treatment method

The present invention provides a pharmaceutical composition comprising at least one of the aforementioned antigens (1) to (8) and (1α) to (55α). In one mode, the aforementioned pharmaceutical composition is used for the treatment and diagnosis of an allergy to a soybean.

The present invention also provides a method for treating an allergy to a soybean, the method comprising administering at least one of the aforementioned antigens (1) to (8) and (1α) to (55α) to a patient in need of a treatment for an allergy to a soybean.

In another mode, the present invention provides at least one of the aforementioned antigens (1) to (8) and (1α) to (55α), intended for use in the treatment for an allergy to a soybean. In yet another mode, the present invention provides use of at least one of the aforementioned antigens (1) to (8) and (1α) to (55α) for the production of a therapeutic agent for an allergy to a soybean.

In the process of allergy treatment, a hyposensitization therapy aiming to induce immunological tolerance by administering an antigen to a patient is often adopted. The at least one of the aforementioned antigens (1) to (8) and (1α) to (55α) can be used as an active ingredient for a hyposensitization therapy or a load test for an allergy to a soybean. Here, the antigen protein to be used in the hyposensitization therapy and the load test may be a protein that has been expressed and purified and may be a protein that has been expressed in food such as rice, such as rice-based vaccine expressing pollen allergens.

The pharmaceutical composition of the present invention can be administered by common administration routes. Examples of common administration routes include oral, sublingual, percutaneous, intracutaneous, subcutaneous, intravascular, intranasal, intramuscular, and intraperitoneal administrations.

The pharmaceutical composition of the present invention can be used as a pharmaceutical composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the pharmaceutical composition can be selected by those skilled in the art as appropriate depending on the administration route. The pharmaceutical composition can be in the form of, for example, tablet, capsule, troche, sublingual tablet, parenteral injection, intranasal spray, poultice, solution, cream, or lotion. The administration dose, frequency and/or period of the pharmaceutical composition of this invention can be selected by a physician as appropriate depending on the administration route and the patient's condition and characteristics such as age and body weight. For example, the pharmaceutical composition may be administered to an adult patient at a dose of not more than 100 µg per dose. The administration interval can be, for example, once daily, once weekly, twice weekly, once per three months or so. The administration period can be, for example, several weeks to several years. The pharmaceutical composition may be administered in such a manner that the dose is increased in incremental steps over the administration period.

### Tester

The present invention provides a tester comprising an antibody for at least one of the aforementioned antigens (1) to (8) and (1α) to (55α).

The antibody can be prepared by a conventional method. For example, the antibody may be prepared by immunizing a mammal such as rabbit with one of the aforementioned antigens (1) to (8) and (1α) to (55α). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and an antigen. Any given carrier known to those skilled in the art can be used.

In one mode, the aforementioned tester is used to determine the presence or absence of an antigen in foods or in products of interest in a food production line. The tester may also be used for quality inspection of production lines and pre-shipment products by manufacturers, or may be used for self-checking of the presence or absence of an antigen in a food of interest by consumers.

Examples of a method for determining the presence or absence of an antigen include a method in which a prepared tester comprising an Ig antibody is contacted with a sample obtained from a food, etc., ELISA or the like method is used to detect whether there is a binding between the Ig antibody and an antigen in the sample, and if the binding between the Ig antibody and the antigen is detected, it is determined that the antibody remains in the food, etc. of interest.

### Allergen-free food and the like

The present invention provides a soybean or processed product of soybean in which at least one of the aforementioned antigens (1) to (8) and (1α) to (55α) is eliminated or reduced.

The method for eliminating or reducing the antigen of the present invention in a soybean or processed products of soybean is not limited. The elimination or reduction of the antigen may be conducted by any method, as long as the method permits the elimination or reduction of the antigen.

For example, the soybean of the present invention whose antigen is eliminated or reduced may be obtained by preparing a soybean in which the expression of the antigen of the present invention is knocked out, using a gene knock-out technique.

An antigen of the present invention may be the artefact that an antigen of the present invention assumed the removal or a reduced soybean raw material as for the removal or the reduced processed products of soybean. In the case of using an ordinary soybean as a source ingredient, a treatment for removing or reducing the antigen of this invention is performed before or after preparation of a processed product of soybean. The methods for removing or reducing the antigen of the present invention include a method to remove protein component in a high pressure treatment and food such as elution with the neutral salt solution or the high temperature steam with an antigen of the present invention in the processed products of soybean which assumed a normal soybean raw material.

### EXAMPLES

The following describes examples of the present invention. The technical scope of this invention is not limited by these examples.

### Example 1: Confirmation of a protein pattern

Proteins contained in soybean were investigated using a two-dimensional electrophoresis method described below.

### Protein extraction

Commercially available dry soybeans (several beans) were powdered. 500 µl of water was added to 50 mg of the powder and shaking extraction was conducted using a vortex mixer at 25°C for 10 minutes. After the shaking extraction, a liquid protein extract was collected.

Thereafter, the precipitation procedure was repeated twice using a 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (50 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS (3-[(3-cholamidopropyl)dimethylammonio]propanesulfonate)
0.5% (v/v) IPG buffer; produced by GE
Moderate amount of BPB (bromophenol blue)

### Penetration of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

The second-dimensional electrophoresis gels obtained through the foregoing series of treatments were subjected to fluorescent image scanning on Typhoon 9400 (produced by GE). The results of the two-dimensional electrophoresis are shown in Fig. 1. Molecular weight marker bands are found at the left of the panels. The positions of the bands denote particular molecular weights (in KDa).

### Example 2: Identification of antigens by immunoblotting (1)

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 20-fold with milliQ water.

To be specific, proteins in the two-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, the gels treated by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from a patient with a soybean allergy or a serum sample from a healthy subject.

Immunoblotting of the membrane was carried out according to the following procedure.
(1) The transferred membrane was shaken in a 5% skim milk/PBST solution (a PBS buffer containing 0.3% Tween 20 nonionic surfactant) at room temperature for one hour.
(2) The membrane was left to stand in a solution of 3% primary antibody serum in 5% skim milk/PBST at room temperature for one hour.
(3) The membrane was washed with a PBST solution (5 min. × 3 times).
(4) The membrane was left to stand in a 1:2500 dilution of the secondary antibody, anti-human IgE-HRP (horseradish peroxidase), with a 3% skim milk/PBST solution at room temperature for one hour.
(5) The membrane was washed with a PBST solution (5 min. × 3 times).
(6) The membrane was left to stand in Pierce Western Blotting Substrate Plus (produced by Thermo Fisher Scientific) for 5 minutes.

### Analysis

The membrane obtained through the foregoing series of treatments was subjected to fluorescent image scanning on Typhoon 9400 (produced by GE).

The immunoblots obtained with the serum from the soybean-allergic patient were compared with those obtained with the control serum from the healthy subject. By immunoblotting using the serum from soybean-allergic Patient 1, Patient 2, Patient 3, and Patient 4, 8 spots were detected (Figs. 3A to D), which are different from the spots detected when the serum of the healthy subject was used (Fig. 2) and different from those of the known soybean allergen proteins.

The molecular weights and isoelectric points of the 8 spots are as follows.
Spot 1: Molecular weight 5 to 20 kDa, pI 4.0 to 6.0
Spot 2: Molecular weight 40 to 60 kDa, pI 5.5 to 7.5
Spot 3: Molecular weight 25 to 37 kDa, pI 8.0 to 10.0
Spot 4: Molecular weight 25 to 37 kDa, pI 7.0 to 9.0
Spot 5: Molecular weight 5 to 20 kDa, pI 5.0 to 7.0
Spot 6: Molecular weight 60 to 75 kDa, pI 6.0 to 8.0
Spot 7: Molecular weight 15 to 25 kDa, pI 4.0 to 6.0
Spot 8: Molecular weight 100 to 150 kDa, pI 4.0 to 6.0

### Example 3: Mass spectrometry and identification of antigens (1)

The amino acid sequences of the antigens that form the above eight protein spots were identified by mass spectroscopy.

To be specific, protein extraction and mass spectroscopy were done by the following procedure.
(1) Soybeans were subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 pmol/µL lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF5600, produced by AB Sciex).
(6) Identification of proteins based on the MS data obtained with the mass spectrometer was done by searching the NCBI database.

### Results

The mass spectrometry of the spots resulted in the detection of the following amino acid sequences.
Spot 1: the amino acid sequences set forth in SEQ ID NO: 17 to 20
Spot 2: the amino acid sequences set forth in SEQ ID NO: 21 to 28
Spot 3: the amino acid sequences set forth in SEQ ID NO: 29, 30
Spot 4: the amino acid sequences set forth in SEQ ID NO: 31, 32
Spot 5: the amino acid sequences set forth in SEQ ID NO: 33, 34
Spot 6: the amino acid sequences set forth in SEQ ID NO: 35 to 53
Spot 7: the amino acid sequences set forth in SEQ ID NO: 54 to 61
Spot 8: the amino acid sequences set forth in SEQ ID NO: 62 to 68

Furthermore, the spots were identified as the following proteins by the analysis of the mass data obtained from the mass spectrometer for the spots at NCBI.
Spot 1: Bowman-Birk type proteinase inhibitor D-II (UniProt accession number P01064, amino acid sequence: SEQ ID NO: 2, encoding nucleotide sequence: SEQ ID NO: 1)
Spot 2: Ribulose bisphosphate carboxylase large chain (UniProt accession number P27066, amino acid sequence: SEQ ID NO: 4, encoding nucleotide sequence: SEQ ID NO: 3)
Spot 3: Gamma-glutamyl hydrolase (UniProt accession number P93164, amino acid sequence: SEQ ID NO: 6, encoding nucleotide sequence: SEQ ID NO: 5)
Spot 4: Guanine nucleotide-binding protein subunit beta-like protein (UniProt accession number Q39836, amino acid sequence: SEQ ID NO: 8, encoding nucleotide sequence: SEQ ID NO: 7)
Spot 5: Protein SLE3 (UniProt accession number C6T0L2, amino acid sequence: SEQ ID NO: 10, encoding nucleotide sequence: SEQ ID NO: 9)
Spot 6: Seed biotin-containing protein SBP65 (UniProt accession number Q39846, amino acid sequence: SEQ ID NO: 12, encoding nucleotide sequence: SEQ ID NO: 11)
Spot 7: Ferritin-2 (UniProt accession number Q94IC4, amino acid sequence: SEQ ID NO: 14, encoding nucleotide sequence: SEQ ID NO: 13)
Spot 8: Cell division cycle protein 48 homolog (UniProt accession number P54774, amino acid sequence: SEQ ID NO: 16, encoding nucleotide sequence: SEQ ID NO: 15)

### Example 4: Identification of antigens by immunoblotting (2)

The immunoblots obtained with the serum from the soybean-allergic patient were compared with those obtained with the control serum from the healthy subject, in the same manner as in Example 2. By immunoblotting using the serum from soybean-allergic Patient 1α, Patient 2α, Patient 3α, Patient 4α, Patient 5α, Patient 6α, Patient 7α, Patient 8α, and Patient 9α, 56 spots were detected (Figs. 5A to I), which are different from the spots detected when the serum of the healthy subject was used (Fig. 4) and different from those of the known soybean allergen proteins.

The molecular weights and isoelectric points of the 56 spots are as follows.
Spot 1α: Molecular weight 80 to 110 kDa, pI 3.0 to 6.0
Spot 2α: Molecular weight 60 to 110 kDa, pI 3.0 to 6.0
Spot 3α: Molecular weight 100 to 150 kDa, pI 4.0 to 6.0
Spot 4α: Molecular weight 80 to 110 kDa, pI 4.0 to 7.0
Spot 5α: Molecular weight 80 to 160 kDa, pI 4.0 to 7.0
Spot 6α: Molecular weight 80 to 110 kDa, pI 4.0 to 7.0
Spot 7α: Molecular weight 60 to 110 kDa, pI 4.0 to 7.0
Spot 8α: Molecular weight 60 to 110 kDa, pI 4.0 to 8.0
Spot 9α: Molecular weight 60 to 110 kDa, pI 4.0 to 8.0
Spot 10α: Molecular weight 40 to 80 kDa, pI 4.0 to 7.0
Spot 11α: Molecular weight 40 to 80 kDa, pI 4.0 to 8.0
Spot 12α: Molecular weight 40 to 80 kDa, pI 4.0 to 8.0
Spot 13α: Molecular weight 40 to 80 kDa, pI 4.0 to 7.0
Spot 14α: Molecular weight 40 to 80 kDa, pI 3.0 to 6.0
Spot 15α, 16α: Molecular weight 30 to 80 kDa, pI 4.0 to 7.0
Spot 17α: Molecular weight 30 to 80 kDa, pI 4.0 to 7.0
Spot 18α: Molecular weight 40 to 80 kDa, pI 4.0 to 8.0
Spot 19α: Molecular weight 40 to 80 kDa, pI 4.0 to 8.0
Spot 20α: Molecular weight 60 to 75 kDa, pI 6.0 to 8.0
Spot 21α: Molecular weight 40 to 60 kDa, pI 5.0 to 8.0
Spot 22α: Molecular weight 25 to 37 kDa, pI 8.0 to 10.0
Spot 23α: Molecular weight 25 to 37 kDa, pI 7.0 to 9.0
Spot 24α: Molecular weight 5 to 20 kDa, pI 5.0 to 7.0
Spot 25α: Molecular weight 30 to 80 kDa, pI 4.0 to 7.0
Spot 26α: Molecular weight 30 to 80 kDa, pI 4.0 to 7.0
Spot 27α: Molecular weight 30 to 80 kDa, pI 4.0 to 7.0
Spot 28α: Molecular weight 20 to 60 kDa, pI 4.0 to 7.0
Spot 29α, 30α, 31α, 32α, 33α: Molecular weight 20 to 60 kDa, pI 4.0 to 8.0
Spot 34α: Molecular weight 20 to 60 kDa, pI 6.0 to 10.0
Spot 35α: Molecular weight 30 to 80 kDa, pI 6.0 to 10.0
Spot 36α, 37α: Molecular weight 20 to 60 kDa, pI 4.0 to 8.0
Spot 38α: Molecular weight 80 to 160 kDa, pI 6.0 to 10.0
Spot 39α: Molecular weight 10 to 50 kDa, pI 6.0 to 10.0
Spot 40α, 56α: Molecular weight 10 to 50 kDa, pI 6.0 to 10.0
Spot 41α: Molecular weight 10 to 50 kDa, pI 6.0 to 10.0
Spot 42α, 43α: Molecular weight 10 to 50 kDa, pI 6.0 to 10.0
Spot 44α: Molecular weight 10 to 50 kDa, pI 4.0 to 7.0
Spot 45α: Molecular weight 5 to 40 kDa, pI 4.0 to 7.0
Spot 46α: Molecular weight 5 to 40 kDa, pI 4.0 to 7.0
Spot 47α: Molecular weight 5 to 40 kDa, pI 4.0 to 7.0
Spot 48α: Molecular weight 5 to 40 kDa, pI 4.0 to 8.0
Spot 49α: Molecular weight 5 to 20 kDa, pI 4.0 to 7.0
Spot 50α: Molecular weight 5 to 40 kDa, pI 3.0 to 6.0
Spot 51α: Molecular weight 10 to 50 kDa, pI 3.0 to 6.0
Spot 52α: Molecular weight 20 to 60 kDa, pI 4.0 to 7.0
Spot 53α: Molecular weight 10 to 50 kDa, pI 4.0 to 8.0
Spot 54α: Molecular weight 10 to 50 kDa, pI 4.0 to 8.0
Spot 55α: Molecular weight 40 to 80 kDa, pI 6.0 to 10.0

### Example 5: Mass spectrometry and identification of antigens (2)

The amino acid sequences of the antigens that form the 56 protein spots were identified by mass spectroscopy, in the same manner as in Example 3.

The mass spectrometry of the spots resulted in the detection of the following amino acid sequences. Furthermore, the spots were identified as the following proteins by the analysis of the mass data obtained from the mass spectrometer for the spots at NCBI.

### Spot 1α:

The amino acid sequences set forth in SEQ ID NOs: 71 to 87 were detected by the mass spectrometry. The protein identified from this mass data was endoplasmin homolog isoform X2 (NCBI accession number XP_006596543.1, amino acid sequence: SEQ ID NO: 70, encoding nucleotide sequence: SEQ ID NO: 69).

The amino acid sequences set forth in SEQ ID NOs: 90 to 104 were detected by the mass spectrometry. The protein identified from this mass data was endoplasmin homolog isoform X2 (NCBI accession number XP_006601356.1, amino acid sequence: SEQ ID NO: 89, encoding nucleotide sequence: SEQ ID NO: 88).

### Spot 2α

The amino acid sequences set forth in SEQ ID NOs: 107 to 120 were detected by the mass spectrometry. The protein identified from this mass data was heat shock cognate protein 80 (NCBI accession number XP_003544594.1, amino acid sequence: SEQ ID NO: 106, encoding nucleotide sequence: SEQ ID NO: 105).

The amino acid sequences set forth in SEQ ID NOs: 123 to 137 were detected by the mass spectrometry. The protein identified from this mass data was heat shock protein 90-1 (NCBI accession number NP_001236612.1, amino acid sequence: SEQ ID NO: 122, encoding nucleotide sequence: SEQ ID NO: 121).

The amino acid sequences set forth in SEQ ID NOs: 140 to 153 were detected by the mass spectrometry. The protein identified from this mass data was hypothetical protein GLYMA_02G302500 (NCBI accession number KRH73936.1, amino acid sequence: SEQ ID NO: 139, encoding nucleotide sequence: SEQ ID NO: 138).

### Spot 3α:

The amino acid sequences set forth in SEQ ID NOs: 62 to 68 were detected by the mass spectrometry, as in Spot 8 of Example 3. The protein identified from this mass data was Cell division cycle protein 48 homolog (UniProt accession number P54774, amino acid sequence: SEQ ID NO: 16, encoding nucleotide sequence: SEQ ID NO: 15).

### Spot 4α:

The amino acid sequences set forth in SEQ ID NOs: 156, 157 were detected by the mass spectrometry. The protein identified from this mass data was embryo-specific urease isoform X1 (NCBI accession number XP_014630847.1, amino acid sequence: SEQ ID NO: 155, encoding nucleotide sequence: SEQ ID NO: 154).

### Spot 5α:

The amino acid sequences set forth in SEQ ID NOs: 160, 161 were detected by the mass spectrometry. The protein identified from this mass data was alpha-1,4 glucan phosphorylase L isozyme, chloroplastic/amyloplastic (NCBI accession number XP_006603904.1, amino acid sequence: SEQ ID NO: 159, encoding nucleotide sequence: SEQ ID NO: 158).

### Spot 6α:

The amino acid sequences set forth in SEQ ID NOs: 164 to 167 were detected by the mass spectrometry. The protein identified from this mass data was aconitate hydratase 1 (NCBI accession number XP_003537655.1, amino acid sequence: SEQ ID NO: 163, encoding nucleotide sequence: SEQ ID NO: 162).

The amino acid sequences set forth in SEQ ID NOs: 170 to 172 were detected by the mass spectrometry. The protein identified from this mass data was aconitate hydratase 1 (NCBI accession number XP_003517155.1, amino acid sequence: SEQ ID NO: 169, encoding nucleotide sequence: SEQ ID NO: 168).

### Spot 7α:

The amino acid sequences set forth in SEQ ID NOs: 175 to 187 were detected by the mass spectrometry. The protein identified from this mass data was methionine synthase (NCBI accession number NP_001235794.1, amino acid sequence: SEQ ID NO: 174, encoding nucleotide sequence: SEQ ID NO: 173).

### Spot 8α:

The amino acid sequences set forth in SEQ ID NOs: 190 to 199 were detected by the mass spectrometry. The protein identified from this mass data was transketolase, chloroplastic (NCBI accession number XP_003521870.1, amino acid sequence: SEQ ID NO: 189, encoding nucleotide sequence: SEQ ID NO: 188).

### Spot 9α:

The amino acid sequence set forth in SEQ ID NO: 202 was detected by the mass spectrometry. The protein identified from this mass data was lysine-tRNA ligase, cytoplasmic-like isoform X2 (NCBI accession number XP_014625509.1, amino acid sequence: SEQ ID NO: 201, encoding nucleotide sequence: SEQ ID NO: 200).

### Spot 10α

The amino acid sequences set forth in SEQ ID NOs: 205 to 218 were detected by the mass spectrometry. The protein identified from this mass data was chaperonin CPN60-2, mitochondrial (NCBI accession number XP_003556325.1, amino acid sequence: SEQ ID NO: 204, encoding nucleotide sequence: SEQ ID NO: 203).

The amino acid sequences set forth in SEQ ID NOs: 221 to 231 were detected by the mass spectrometry. The protein identified from this mass data was chaperonin CPN60-2, mitochondrial-like isoform X1 (NCBI accession number XP_003535967.1, amino acid sequence: SEQ ID NO: 220, encoding nucleotide sequence: SEQ ID NO: 219).

### Spot 11α:

The amino acid sequences set forth in SEQ ID NOs: 234 to 237 were detected by the mass spectrometry. The protein identified from this mass data was polyadenylate-binding protein 8 isoform X3 (NCBI accession number XP_006578034.1, amino acid sequence: SEQ ID NO: 233, encoding nucleotide sequence: SEQ ID NO: 232).

### Spot 12α:

The amino acid sequence set forth in SEQ ID NO: 240 was detected by the mass spectrometry. The protein identified from this mass data was pyrophosphate-fructose 6-phosphate 1-phosphotransferase subunit alpha-like (NCBI accession number XP_003555655.1, amino acid sequence: SEQ ID NO: 239, encoding nucleotide sequence: SEQ ID NO: 238).

### Spot 13α:

The amino acid sequences set forth in SEQ ID NOs: 243 to 252 were detected by the mass spectrometry. The protein identified from this mass data was protein disulfide isomerase-like protein precursor (NCBI accession number NP_001238009.1, amino acid sequence: SEQ ID NO: 242, encoding nucleotide sequence: SEQ ID NO: 241).

The amino acid sequences set forth in SEQ ID NOs: 255 to 278 were detected by the mass spectrometry. The protein identified from this mass data was protein disulfide-isomerase (NCBI accession number XP_006581588.1, amino acid sequence: SEQ ID NO: 254, encoding nucleotide sequence: SEQ ID NO: 253).

### Spot 14α:

The amino acid sequences set forth in SEQ ID NOs: 281 to 284 were detected by the mass spectrometry. The protein identified from this mass data was V-type proton ATPase subunit B 2 (NCBI accession number XP_003552473.1, amino acid sequence: SEQ ID NO: 280, encoding nucleotide sequence: SEQ ID NO: 279).

The amino acid sequences set forth in SEQ ID NOs: 287 to 289 were detected by the mass spectrometry. The protein identified from this mass data was V-type proton ATPase subunit B 2 (NCBI accession number XP_006605857.1, amino acid sequence: SEQ ID NO: 286, encoding nucleotide sequence: SEQ ID NO: 275).

### Spots 15α, 16α:

The amino acid sequences set forth in SEQ ID NOs: 292 to 304 were detected by the mass spectrometry. The protein identified from this mass data was UTP-glucose-1-phosphate uridylyltransferase (NCBI accession number XP_003544964.1, amino acid sequence: SEQ ID NO: 291, encoding nucleotide sequence: SEQ ID NO: 290).

The amino acid sequences set forth in SEQ ID NOs: 307 to 318 were detected by the mass spectrometry. The protein identified from this mass data was hypothetical protein GLYMA_02G241100 (NCBI accession number KRH72929.1, amino acid sequence: SEQ ID NO: 306, encoding nucleotide sequence: SEQ ID NO: 305).

### Spot 17α:

The amino acid sequences set forth in SEQ ID NOs: 321 to 325 were detected by the mass spectrometry. The protein identified from this mass data was guanosine nucleotide diphosphate dissociation inhibitor 2 (NCBI accession number XP_003531293.1, amino acid sequence: SEQ ID NO: 320, encoding nucleotide sequence: SEQ ID NO: 319).

The amino acid sequences set forth in SEQ ID NOs: 328 to 332 were detected by the mass spectrometry. The protein identified from this mass data was rab GDP dissociation inhibitor alpha-like (NCBI accession number NP_001276273.1, amino acid sequence: SEQ ID NO: 327, encoding nucleotide sequence: SEQ ID NO: 326).

### Spot 18α:

The amino acid sequences set forth in SEQ ID NOs: 335 to 339 were detected by the mass spectrometry. The protein identified from this mass data was hypothetical protein GLYMA_10G028300 (NCBI accession number KRH32039.1, amino acid sequence: SEQ ID NO: 334, encoding nucleotide sequence: SEQ ID NO: 333).

### Spot 19α:

The amino acid sequences set forth in SEQ ID NOs: 342 to 346 were detected by the mass spectrometry. The protein identified from this mass data was sucrose binding protein homolog S-64 (NCBI accession number AAF05723.1, amino acid sequence: SEQ ID NO: 341, encoding nucleotide sequence: SEQ ID NO: 340).

### Spot 20α:

The amino acid sequences set forth in SEQ ID NOs: 35 to 53 were detected by the mass spectrometry, as in Spot 6 of Example 3. The protein identified from this mass data was Seed biotin-containing protein SBP65 (UniProt accession number Q39846, amino acid sequence: SEQ ID NO: 12, encoding nucleotide sequence: SEQ ID NO: 11).

### Spot 21α:

The amino acid sequences set forth in SEQ ID NOs: 21 to 28 were detected by the mass spectrometry, as in Spot 2 of Example 3. The protein identified from this mass data was Ribulose bisphosphate carboxylase large chain (UniProt accession number P27066, amino acid sequence: SEQ ID NO: 4, encoding nucleotide sequence: SEQ ID NO: 3).

### Spot 22α:

The amino acid sequences set forth in SEQ ID NOs: 29, 30 were detected by the mass spectrometry, as in Spot 3 of Example 3. The protein identified from this mass data was Gamma-glutamyl hydrolase (UniProt accession number P93164, amino acid sequence: SEQ ID NO: 6, encoding nucleotide sequence: SEQ ID NO: 5).

### Spot 23α:

The amino acid sequences set forth in SEQ ID NOs: 31, 32 were detected by the mass spectrometry, as in Spot 4 of Example 3. The protein identified from this mass data was Guanine nucleotide-binding protein subunit beta-like protein (UniProt accession number Q39836, amino acid sequence: SEQ ID NO: 8, encoding nucleotide sequence: SEQ ID NO: 7).

### Spot 24α:

The amino acid sequences set forth in SEQ ID NOs: 33, 34 were detected by the mass spectrometry, as in Spot 5 of Example 3. The protein identified from this mass data was Protein SLE3 (UniProt accession number C6T0L2, amino acid sequence: SEQ ID NO: 10, encoding nucleotide sequence: SEQ ID NO: 9).

### Spot 25α:

The amino acid sequences set forth in SEQ ID NOs: 349 to 356 were detected by the mass spectrometry. The protein identified from this mass data was succinyl-CoA ligase [ADP-forming] subunit beta, mitochondrial (NCBI accession number XP_003537078.1, amino acid sequence: SEQ ID NO: 348, encoding nucleotide sequence: SEQ ID NO: 347).

The amino acid sequences set forth in SEQ ID NOs: 359 to 365 were detected by the mass spectrometry. The protein identified from this mass data was succinyl-CoA ligase [ADP-forming] subunit beta, mitochondrial (NCBI accession number XP_003542431.1, amino acid sequence: SEQ ID NO: 358, encoding nucleotide sequence: SEQ ID NO: 357).

### Spot 26α:

The amino acid sequences set forth in SEQ ID NOs: 368 to 382 were detected by the mass spectrometry. The protein identified from this mass data was phosphoglycerate kinase, cytosolic (NCBI accession number XP_003546821.1, amino acid sequence: SEQ ID NO: 367, encoding nucleotide sequence: SEQ ID NO: 366).

The amino acid sequences set forth in SEQ ID NOs: 385 to 392 were detected by the mass spectrometry. The protein identified from this mass data was phosphoglycerate kinase, cytosolic (NCBI accession number XP_003531482.1, amino acid sequence: SEQ ID NO: 384, encoding nucleotide sequence: SEQ ID NO: 383).

The amino acid sequences set forth in SEQ ID NOs: 395 to 409 were detected by the mass spectrometry. The protein identified from this mass data was phosphoglycerate kinase, cytosolic (NCBI accession number XP_003531483.1, amino acid sequence: SEQ ID NO: 394, encoding nucleotide sequence: SEQ ID NO: 393).

### Spot 27α:

The amino acid sequences set forth in SEQ ID NOs: 412 to 416 were detected by the mass spectrometry. The protein identified from this mass data was alcohol dehydrogenase 1 (NCBI accession number XP_003526673.1, amino acid sequence: SEQ ID NO: 411, encoding nucleotide sequence: SEQ ID NO: 410).

The amino acid sequences set forth in SEQ ID NOs: 419, 420 were detected by the mass spectrometry. The protein identified from this mass data was alcohol dehydrogenase 1 (NCBI accession number XP_003545664.3, amino acid sequence: SEQ ID NO: 418, encoding nucleotide sequence: SEQ ID NO: 417).

The amino acid sequences set forth in SEQ ID NOs: 423 to 427 were detected by the mass spectrometry. The protein identified from this mass data was alcohol dehydrogenase 1-like (NCBI accession number XP_003544738.1, amino acid sequence: SEQ ID NO: 422, encoding nucleotide sequence: SEQ ID NO: 421).

### Spot 28α:

The amino acid sequences set forth in SEQ ID NOs: 430 to 433 were detected by the mass spectrometry. The protein identified from this mass data was 35 kDa seed maturation protein isoform X1 (NCBI accession number XP_006576267.1, amino acid sequence: SEQ ID NO: 429, encoding nucleotide sequence: SEQ ID NO: 428).

### Spots 29α, 30α, 31α, 32α, 33α:

The amino acid sequences set forth in SEQ ID NOs: 436 to 439 were detected by the mass spectrometry. The protein identified from this mass data was glyceraldehyde-3-phosphate dehydrogenase isoform X1 (NCBI accession number XP_014631598.1, amino acid sequence: SEQ ID NO: 435, encoding nucleotide sequence: SEQ ID NO: 434).

The amino acid sequences set forth in SEQ ID NOs: 442 to 445 were detected by the mass spectrometry. The protein identified from this mass data was glyceraldehyde-3-phosphate dehydrogenase, cytosolic (NCBI accession number XP_006578692.1, amino acid sequence: SEQ ID NO: 441, encoding nucleotide sequence: SEQ ID NO: 440).

The amino acid sequences set forth in SEQ ID NOs: 448 to 451 were detected by the mass spectrometry. The protein identified from this mass data was uncharacterized protein LOC100782924 (NCBI accession number NP_001240046.1, amino acid sequence: SEQ ID NO: 447, encoding nucleotide sequence: SEQ ID NO: 446).

### Spot 34α:

The amino acid sequence set forth in SEQ ID NO: 454 was detected by the mass spectrometry. The protein identified from this mass data was bifunctional UDP-glucose 4-epimerase and UDP-xylose 4-epimerase 1-like (NCBI accession number XP_003532591.1, amino acid sequence: SEQ ID NO: 453, encoding nucleotide sequence: SEQ ID NO: 452).

The amino acid sequences set forth in SEQ ID NOs: 457, 458 were detected by the mass spectrometry. The protein identified from this mass data was uncharacterized protein LOC100803483 (NCBI accession number NP_001241075.1, amino acid sequence: SEQ ID NO: 456, encoding nucleotide sequence: SEQ ID NO: 455).

### Spot 35α:

The amino acid sequences set forth in SEQ ID NOs: 461 to 466 were detected by the mass spectrometry. The protein identified from this mass data was elongation factor 1-alpha (NCBI accession number XP_003553292.1, amino acid sequence: SEQ ID NO: 460, encoding nucleotide sequence: SEQ ID NO: 459).

The amino acid sequences set forth in SEQ ID NOs: 469 to 471 were detected by the mass spectrometry. The protein identified from this mass data was elongation factor 1-alpha (NCBI accession number XP_003547695.1, amino acid sequence: SEQ ID NO: 468, encoding nucleotide sequence: SEQ ID NO: 467).

The amino acid sequences set forth in SEQ ID NOs: 474 to 477 were detected by the mass spectrometry. The protein identified from this mass data was elongation factor-1A isoform X1 (NCBI accession number XP_006600131.1, amino acid sequence: SEQ ID NO: 473, encoding nucleotide sequence: SEQ ID NO: 472).

### Spots 36α, 37α:

The amino acid sequences set forth in SEQ ID NOs: 480 to 483 were detected by the mass spectrometry. The protein identified from this mass data was seed maturation protein PM34 (NCBI accession number NP_001238285.1, amino acid sequence: SEQ ID NO: 479, encoding nucleotide sequence: SEQ ID NO: 478).

The amino acid sequences set forth in SEQ ID NOs: 486, 487 were detected by the mass spectrometry. The protein identified from this mass data was uncharacterized protein LOC100809384 (NCBI accession number NP_001241158.1, amino acid sequence: SEQ ID NO: 485, encoding nucleotide sequence: SEQ ID NO: 484).

The amino acid sequences set forth in SEQ ID NOs: 490 to 493 were detected by the mass spectrometry. The protein identified from this mass data was hypothetical protein GLYMA_10G155300 (NCBI accession number KRH33956.1, amino acid sequence: SEQ ID NO: 489, encoding nucleotide sequence: SEQ ID NO: 488).

### Spot 38α

The amino acid sequences set forth in SEQ ID NOs: 496 to 513 were detected by the mass spectrometry. The protein identified from this mass data was seed biotin-containing protein SBP65-like isoform X1 (NCBI accession number XP_003526237.1, amino acid sequence: SEQ ID NO: 495, encoding nucleotide sequence: SEQ ID NO: 494).

### Spot 39α:

The amino acid sequence set forth in SEQ ID NO: 516 was detected by the mass spectrometry. The protein identified from this mass data was ATP synthase subunit O, mitochondrial-like (NCBI accession number XP_003546884.1, amino acid sequence: SEQ ID NO: 515, encoding nucleotide sequence: SEQ ID NO: 514).

### Spots 40α, 56α:

The amino acid sequences set forth in SEQ ID NOs: 519, 520 were detected by the mass spectrometry. The protein identified from this mass data was P24 oleosin isoform A (NCBI accession number XP_003553822.1, amino acid sequence: SEQ ID NO: 518, encoding nucleotide sequence: SEQ ID NO: 517).

### Spot 41α:

The amino acid sequence set forth in SEQ ID NO: 523 was detected by the mass spectrometry. The protein identified from this mass data was uncharacterized protein At3g49720-like isoform X2 (NCBI accession number XP_006600908.1, amino acid sequence: SEQ ID NO: 522, encoding nucleotide sequence: SEQ ID NO: 521).

### Spots 42α, 43α

The amino acid sequences set forth in SEQ ID NOs: 526 to 531 were detected by the mass spectrometry. The protein identified from this mass data was superoxide dismutase [Mn], mitochondrial (NCBI accession number XP_003526813.1, amino acid sequence: SEQ ID NO: 525, encoding nucleotide sequence: SEQ ID NO: 524).

The amino acid sequences set forth in SEQ ID NOs: 534 to 539 were detected by the mass spectrometry. The protein identified from this mass data was hypothetical protein GLYMA_04G221300 (NCBI accession number KRH64185.1, amino acid sequence: SEQ ID NO: 533, encoding nucleotide sequence: SEQ ID NO: 532).

### Spot 44α:

The amino acid sequences set forth in SEQ ID NOs: 54 to 61 were detected by the mass spectrometry, as in Spot 7 of Example 3. The protein identified from this mass data was Ferritin-2 (UniProt accession number Q94IC4, amino acid sequence: SEQ ID NO: 14, encoding nucleotide sequence: SEQ ID NO: 13).

### Spot 45α:

The amino acid sequences set forth in SEQ ID NOs: 542 to 545 were detected by the mass spectrometry. The protein identified from this mass data was uncharacterized protein LOC100499771 (NCBI accession number NP_001235797.1, amino acid sequence: SEQ ID NO: 541, encoding nucleotide sequence: SEQ ID NO: 540).

The amino acid sequences set forth in SEQ ID NOs: 548 to 551 were detected by the mass spectrometry. The protein identified from this mass data was hypothetical protein GLYMA_09G192800 (NCBI accession number KRH39322.1, amino acid sequence: SEQ ID NO: 547, encoding nucleotide sequence: SEQ ID NO: 546).

### Spot 46α:

The amino acid sequence set forth in SEQ ID NO: 554 was detected by the mass spectrometry. The protein identified from this mass data was seed maturation protein PM22 (NCBI accession number NP_001237935.1, amino acid sequence: SEQ ID NO: 553, encoding nucleotide sequence: SEQ ID NO: 552).

### Spot 47α:

The amino acid sequences set forth in SEQ ID NOs: 557, 558 were detected by the mass spectrometry. The protein identified from this mass data was eukaryotic translation initiation factor 5A-2 (NCBI accession number XP_003549690.1, amino acid sequence: SEQ ID NO: 556, encoding nucleotide sequence: SEQ ID NO: 555).

The amino acid sequences set forth in SEQ ID NOs: 561 to 563 were detected by the mass spectrometry. The protein identified from this mass data was uncharacterized protein LOC100305510 (NCBI accession number NP_001236395.1, amino acid sequence: SEQ ID NO: 560, encoding nucleotide sequence: SEQ ID NO: 559).

### Spot 48α:

The amino acid sequences set forth in SEQ ID NOs: 566 to 570 were detected by the mass spectrometry. The protein identified from this mass data was uncharacterized protein LOC100306570 (NCBI accession number NP_001236895.1, amino acid sequence: SEQ ID NO: 565, encoding nucleotide sequence: SEQ ID NO: 564).

### Spot 49α:

The amino acid sequences set forth in SEQ ID NOs: 17 to 20 were detected by the mass spectrometry, as in Spot 1 of Example 3. The protein identified from this mass data was Bowman-Birk type proteinase inhibitor D-II (UniProt accession number P01064, amino acid sequence: SEQ ID NO: 2, encoding nucleotide sequence: SEQ ID NO: 1).

### Spot 50α:

The amino acid sequences set forth in SEQ ID NOs: 573 to 576 were detected by the mass spectrometry. The protein identified from this mass data was uncharacterized protein LOC100813859 (NCBI accession number NP_001239816.1, amino acid sequence: SEQ ID NO: 572, encoding nucleotide sequence: SEQ ID NO: 571).

### Spot 51α:

The amino acid sequences set forth in SEQ ID NOs: 579 to 582 were detected by the mass spectrometry. The protein identified from this mass data was 14-3-3-like protein (NCBI accession number XP_003526571.1, amino acid sequence: SEQ ID NO: 578, encoding nucleotide sequence: SEQ ID NO: 577).

### Spot 52α:

The amino acid sequences set forth in SEQ ID NOs: 585 to 588 were detected by the mass spectrometry. The protein identified from this mass data was probable fructokinase-4 (NCBI accession number XP_003543564.1, amino acid sequence: SEQ ID NO: 584, encoding nucleotide sequence: SEQ ID NO: 583).

### Spot 53α:

The amino acid sequences set forth in SEQ ID NOs: 591 to 597 were detected by the mass spectrometry. The protein identified from this mass data was triosephosphate isomerase isoform X1 (NCBI accession number XP_006593480.2, amino acid sequence: SEQ ID NO: 590, encoding nucleotide sequence: SEQ ID NO: 589).

The amino acid sequences set forth in SEQ ID NOs: 600 to 604 were detected by the mass spectrometry. The protein identified from this mass data was triosephosphate isomerase, cytosolic (NCBI accession number XP_003547334.1, amino acid sequence: SEQ ID NO: 599, encoding nucleotide sequence: SEQ ID NO: 598).

### Spot 54α:

The amino acid sequences set forth in SEQ ID NOs: 607 to 611 were detected by the mass spectrometry. The protein identified from this mass data was superoxide dismutase [Fe], chloroplastic isoform X1 (NCBI accession number XP_014627751.1, amino acid sequence: SEQ ID NO: 606, encoding nucleotide sequence: SEQ ID NO: 605).

The amino acid sequences set forth in SEQ ID NOs: 614 to 616 were detected by the mass spectrometry. The protein identified from this mass data was iron-superoxide dismutase (NCBI accession number NP_001237901.1, amino acid sequence: SEQ ID NO: 613, encoding nucleotide sequence: SEQ ID NO: 612).

### Spot 55α:

The amino acid sequences set forth in SEQ ID NOs: 619 to 635 were detected by the mass spectrometry. The protein identified from this mass data was 51 kDa seed maturation protein precursor (NCBI accession number NP_001238138.1, amino acid sequence: SEQ ID NO: 618, encoding nucleotide sequence: SEQ ID NO: 617).

The amino acid sequences set forth in SEQ ID NOs: 638 to 649 were detected by the mass spectrometry. The protein identified from this mass data was Lea protein precursor (NCBI accession number NP_001238201.1, amino acid sequence: SEQ ID NO: 637, encoding nucleotide sequence: SEQ ID NO: 636).

### INDUSTRIAL APPLICABILITY

The present invention can provide novel antigens of an allergy to soybean, methods and kits for diagnosing an allergy to soybean, pharmaceutical compositions comprising such an antigen, and soybeans or processed products of soybean in which such an antigen is eliminated or reduced.

## Claims

1. A kit for diagnosing an allergy to a soybean, the kit comprising, as an antigen, at least one of proteins defined below in any of the following (1) to (8):
(1)
(1A) Bowman-Birk type proteinase inhibitor D-II or a variant thereof, which is defined below in any of (1A-a) to (1A-e):
(1A-a) a protein comprising an amino acid sequence with deletion, substitution, insertion or addition of one or several amino acids in SEQ ID NO: 2;
(1A-b) a protein comprising an amino acid sequence having at least 70% identity to the amino acid sequence of SEQ ID NO: 2;
(1A-c) a protein comprising an amino acid sequence encoded by a nucleotide sequence with deletion, substitution, insertion or addition of one or several nucleotides in SEQ ID NO: 1;
(1A-d) a protein comprising an amino acid sequence encoded by a nucleotide sequence having at least 70% identity to the nucleotide sequence of SEQ ID NO: 1; or
(1A-e) a protein comprising an amino acid sequence encoded by a nucleic acid that hybridizes under stringent conditions with a nucleic acid having a nucleotide sequence complementary to the nucleotide sequence of SEQ ID NO: 1; or
(1B) a protein comprising at least one amino acid sequence selected from the group consisting of SEQ ID NOs: 17-20;
(2)
(2A) Ribulose bisphosphate carboxylase large chain or a variant thereof, which is defined below in any of (2A-a) to (2A-e):
(2A-a) a protein comprising an amino acid sequence with deletion, substitution, insertion or addition of one or several amino acids in SEQ ID NO: 4;
(2A-b) a protein comprising an amino acid sequence having at least 70% identity to the amino acid sequence of SEQ ID NO: 4;
(2A-c) a protein comprising an amino acid sequence encoded by a nucleotide sequence with deletion, substitution, insertion or addition of one or several nucleotides in SEQ ID NO: 3;
(2A-d) a protein comprising an amino acid sequence encoded by a nucleotide sequence having at least 70% identity to the nucleotide sequence of SEQ ID NO: 3; or
(2A-e) a protein comprising an amino acid sequence encoded by a nucleic acid that hybridizes under stringent conditions with a nucleic acid having a nucleotide sequence complementary to the nucleotide sequence of SEQ ID NO: 3; or
(2B) a protein comprising at least one amino acid sequence selected from the group consisting of SEQ ID NOs: 21-28;
(3)
(3A) Gamma-glutamyl hydrolase or a variant thereof, which is defined below in any of (3A-a) to (3A-e):
(3A-a) a protein comprising an amino acid sequence with deletion, substitution, insertion or addition of one or several amino acids in SEQ ID NO: 6;
(3A-b) a protein comprising an amino acid sequence having at least 70% identity to the amino acid sequence of SEQ ID NO: 6;
(3A-c) a protein comprising an amino acid sequence encoded by a nucleotide sequence with deletion, substitution, insertion or addition of one or several nucleotides in SEQ ID NO: 5;
(3A-d) a protein comprising an amino acid sequence encoded by a nucleotide sequence having at least 70% identity to the nucleotide sequence of SEQ ID NO: 5; or
(3A-e) a protein comprising an amino acid sequence encoded by a nucleic acid that hybridizes under stringent conditions with a nucleic acid having a nucleotide sequence complementary to the nucleotide sequence of SEQ ID NO: 5; or
(3B) a protein comprising an amino acid sequence of SEQ ID NO: 29 or 30;
(4)
(4A) Guanine nucleotide-binding protein subunit beta-like protein or a variant thereof, which is defined below in any of (4A-a) to (4A-e):
(4A-a) a protein comprising an amino acid sequence with deletion, substitution, insertion or addition of one or several amino acids in SEQ ID NO: 8;
(4A-b) a protein comprising an amino acid sequence having at least 70% identity to the amino acid sequence of SEQ ID NO: 8;
(4A-c) a protein comprising an amino acid sequence encoded by a nucleotide sequence with deletion, substitution, insertion or addition of one or several nucleotides in SEQ ID NO: 7;
(4A-d) a protein comprising an amino acid sequence encoded by a nucleotide sequence having at least 70% identity to the nucleotide sequence of SEQ ID NO: 7; or
(4A-e) a protein comprising an amino acid sequence encoded by a nucleic acid that hybridizes under stringent conditions with a nucleic acid having a nucleotide sequence complementary to the nucleotide sequence of SEQ ID NO: 7; or
(4B) a protein comprising an amino acid sequence of SEQ ID NO: 31 or 32;
(5)
(5A) Protein SLE3 or a variant thereof, which is defined below in any of (5A-a) to (5A-e):
(5A-a) a protein comprising an amino acid sequence with deletion, substitution, insertion or addition of one or several amino acids in SEQ ID NO: 10;
(5A-b) a protein comprising an amino acid sequence having at least 70% identity to the amino acid sequence of SEQ ID NO: 10;
(5A-c) a protein comprising an amino acid sequence encoded by a nucleotide sequence with deletion, substitution, insertion or addition of one or several nucleotides in SEQ ID NO: 9;
(5A-d) a protein comprising an amino acid sequence encoded by a nucleotide sequence having at least 70% identity to the nucleotide sequence of SEQ ID NO: 9; or
(5A-e) a protein comprising an amino acid sequence encoded by a nucleic acid that hybridizes under stringent conditions with a nucleic acid having a nucleotide sequence complementary to the nucleotide sequence of SEQ ID NO: 9; or
(5B) a protein comprising an amino acid sequence of SEQ ID NO: 33 or 34;
(6)
(6A) Seed biotin-containing protein SBP65 or a variant thereof, which is defined below in any of (6A-a) to (6A-e):
(6A-a) a protein comprising an amino acid sequence with deletion, substitution, insertion or addition of one or several amino acids in SEQ ID NO: 12;
(6A-b) a protein comprising an amino acid sequence having at least 70% identity to the amino acid sequence of SEQ ID NO: 12;
(6A-c) a protein comprising an amino acid sequence encoded by a nucleotide sequence with deletion, substitution, insertion or addition of one or several nucleotides in SEQ ID NO: 11;
(6A-d) a protein comprising an amino acid sequence encoded by a nucleotide sequence having at least 70% identity to the nucleotide sequence of SEQ ID NO: 11; or
(6A-e) a protein comprising an amino acid sequence encoded by a nucleic acid that hybridizes under stringent conditions with a nucleic acid having a nucleotide sequence complementary to the nucleotide sequence of SEQ ID NO: 11; or
(6B) a protein comprising at least one amino acid sequence selected from the group consisting of SEQ ID NOs: 35-53;
(7)
(7A) Ferritin-2 or a variant thereof, which is defined below in any of (7A-a) to (7A-e):
(7A-a) a protein comprising an amino acid sequence with deletion, substitution, insertion or addition of one or several amino acids in SEQ ID NO: 14;
(7A-b) a protein comprising an amino acid sequence having at least 70% identity to the amino acid sequence of SEQ ID NO: 14;
(7A-c) a protein comprising an amino acid sequence encoded by a nucleotide sequence with deletion, substitution, insertion or addition of one or several nucleotides in SEQ ID NO: 13;
(7A-d) a protein comprising an amino acid sequence encoded by a nucleotide sequence having at least 70% identity to the nucleotide sequence of SEQ ID NO: 13; or
(7A-e) a protein comprising an amino acid sequence encoded by a nucleic acid that hybridizes under stringent conditions with a nucleic acid having a nucleotide sequence complementary to the nucleotide sequence of SEQ ID NO: 13; or
(7B) a protein comprising at least one amino acid sequence selected from the group consisting of SEQ ID NOs: 54-61;
(8)
(8A) Cell division cycle protein 48 homolog or a variant thereof, which is defined below in any of (8A-a) to (8A-e):
(8A-a) a protein comprising an amino acid sequence with deletion, substitution, insertion or addition of one or several amino acids in SEQ ID NO: 16;
(8A-b) a protein comprising an amino acid sequence having at least 70% identity to the amino acid sequence of SEQ ID NO: 16;
(8A-c) a protein comprising an amino acid sequence encoded by a nucleotide sequence with deletion, substitution, insertion or addition of one or several nucleotides in SEQ ID NO: 15;
(8A-d) a protein comprising an amino acid sequence encoded by a nucleotide sequence having at least 70% identity to the nucleotide sequence of SEQ ID NO: 15; or
(8A-e) a protein comprising an amino acid sequence encoded by a nucleic acid that hybridizes under stringent conditions with a nucleic acid having a nucleotide sequence complementary to the nucleotide sequence of SEQ ID NO: 15; or
(8B) a protein comprising at least one amino acid sequence selected from the group consisting of SEQ ID NOs: 62-68.

2. A kit for diagnosing an allergy to a soybean, the kit comprising, as an antigen, at least one of proteins defined below in any one of (1α) to (55α):
(1α)
(1αA1) endoplasmin homolog isoform X2 or a variant thereof, which is defined below in any of (1αA1-a) to (1αA1-e):
(1αA1-a) a protein comprising an amino acid sequence with deletion, substitution, insertion or addition of one or several amino acids in SEQ ID NO: 70;
(1αA1-b) a protein comprising an amino acid sequence having at least 70% identity to the amino acid sequence of SEQ ID NO: 70;
(1αA1-c) a protein comprising an amino acid sequence encoded by a nucleotide sequence with deletion, substitution, insertion or addition of one or several nucleotides in SEQ ID NO: 69;
(1αA1-d) a protein comprising an amino acid sequence encoded by a nucleotide sequence having at least 70% identity to the nucleotide sequence of SEQ ID NO: 69; or
(1αA1-e) a protein comprising an amino acid sequence encoded by a nucleic acid that hybridizes under stringent conditions with a nucleic acid having a nucleotide sequence complementary to the nucleotide sequence of SEQ ID NO: 69;
(1αB1) a protein comprising at least one amino acid sequence selected from the group consisting of SEQ ID NOs: 71-87,
(1αA2) endoplasmin homolog isoform X2 or a variant thereof, which is defined below in any of (1αA2-a) to (1αA2-e):
(1αA2-a) a protein comprising an amino acid sequence with deletion, substitution, insertion or addition of one or several amino acids in SEQ ID NO: 89;
(1αA2-b) a protein comprising an amino acid sequence having at least 70% identity to the amino acid sequence of SEQ ID NO: 89;
(1αA2-c) a protein comprising an amino acid sequence encoded by a nucleotide sequence with deletion, substitution, insertion or addition of one or several nucleotides in SEQ ID NO: 88;
(1αA2-d) a protein comprising an amino acid sequence encoded by a nucleotide sequence having at least 70% identity to the nucleotide sequence of SEQ ID NO: 88; or
(1αA2-e) a protein comprising an amino acid sequence encoded by a nucleic acid that hybridizes under stringent conditions with a nucleic acid having a nucleotide sequence complementary to the nucleotide sequence of SEQ ID NO: 88; or
(1αB2) a protein comprising at least one amino acid sequence selected from the group consisting of SEQ ID NOs: 90-104;
(2α)
(2αA1) heat shock cognate protein 80 or a variant thereof, which is defined below in any of (2αA1-a) to (2αA1-e):
(2αA1-a) a protein comprising an amino acid sequence with deletion, substitution, insertion or addition of one or several amino acids in SEQ ID NO: 106;
(2αA1-b) a protein comprising an amino acid sequence having at least 70% identity to the amino acid sequence of SEQ ID NO: 106;
(2αA1-c) a protein comprising an amino acid sequence encoded by a nucleotide sequence with deletion, substitution, insertion or addition of one or several nucleotides in SEQ ID NO: 105;
(2αA1-d) a protein comprising an amino acid sequence encoded by a nucleotide sequence having at least 70% identity to the nucleotide sequence of SEQ ID NO: 105; or
(2αA1-e) a protein comprising an amino acid sequence encoded by a nucleic acid that hybridizes under stringent conditions with a nucleic acid having a nucleotide sequence complementary to the nucleotide sequence of SEQ ID NO: 105; or
(2αB1) a protein comprising at least one amino acid sequence selected from the group consisting of SEQ ID NOs: 107-120;
(2αA2) heat shock protein 90-1 or a variant thereof, which is defined below in any of (2αA2-a) to (2αA2-e):
(2αA2-a) a protein comprising an amino acid sequence with deletion, substitution, insertion or addition of one or several amino acids in SEQ ID NO: 122;
(2αA2-b) a protein comprising an amino acid sequence having at least 70% identity to the amino acid sequence of SEQ ID NO: 122;
(2αA2-c) a protein comprising an amino acid sequence encoded by a nucleotide sequence with deletion, substitution, insertion or addition of one or several nucleotides in SEQ ID NO: 121;
(2αA2-d) a protein comprising an amino acid sequence encoded by a nucleotide sequence having at least 70% identity to the nucleotide sequence of SEQ ID NO: 121; or
(2αA2-e) a protein comprising an amino acid sequence encoded by a nucleic acid that hybridizes under stringent conditions with a nucleic acid having a nucleotide sequence complementary to the nucleotide sequence of SEQ ID NO: 121;
(2αB2) a protein comprising at least one amino acid sequence selected from the group consisting of SEQ ID NOs: 123-137;
(2αA3) hypothetical protein GLYMA_02G302500 or a variant thereof, which is defined below in any of (2αA3-a) to (2αA3-e):
(2αA3-a) a protein comprising an amino acid sequence with deletion, substitution, insertion or addition of one or several amino acids in SEQ ID NO: 139;
(2αA3-b) a protein comprising an amino acid sequence having at least 70% identity to the amino acid sequence of SEQ ID NO: 139;
(2αA3-c) a protein comprising an amino acid sequence encoded by a nucleotide sequence with deletion, substitution, insertion or addition of one or several nucleotides in SEQ ID NO: 138;
(2αA3-d) a protein comprising an amino acid sequence encoded by a nucleotide sequence having at least 70% identity to the nucleotide sequence of SEQ ID NO: 138; or
(2αA3-e) a protein comprising an amino acid sequence encoded by a nucleic acid that hybridizes under stringent conditions with a nucleic acid having a nucleotide sequence complementary to the nucleotide sequence of SEQ ID NO: 138; or
(2αB3) a protein comprising at least one amino acid sequence selected from the group consisting of SEQ ID NOs: 140-153;
(4α)
(4αA) embryo-specific urease isoform X1 or a variant thereof, which is defined below in any of (4αA-a) to (4αA-e):
(4αA-a) a protein comprising an amino acid sequence with deletion, substitution, insertion or addition of one or several amino acids in SEQ ID NO: 155;
(4αA-b) a protein comprising an amino acid sequence having at least 70% identity to the amino acid sequence of SEQ ID NO: 155;
(4αA-c) a protein comprising an amino acid sequence encoded by a nucleotide sequence with deletion, substitution, insertion or addition of one or several nucleotides in SEQ ID NO: 154;
(4αA-d) a protein comprising an amino acid sequence encoded by a nucleotide sequence having at least 70% identity to the nucleotide sequence of SEQ ID NO: 154; or
(4αA-e) a protein comprising an amino acid sequence encoded by a nucleic acid that hybridizes under stringent conditions with a nucleic acid having a nucleotide sequence complementary to the nucleotide sequence of SEQ ID NO: 154; or
(4αB) a protein comprising at least one amino acid sequence selected from the group consisting of SEQ ID NOs: 156-157;
(5α)
(5αA) alpha-1,4 glucan phosphorylase L isozyme, chloroplastic/amyloplastic or a variant thereof, which is defined below in any of (5αA-a) to (5αA-e):
(5αA-a) a protein comprising an amino acid sequence with deletion, substitution, insertion or addition of one or several amino acids in SEQ ID NO: 159;
(5αA-b) a protein comprising an amino acid sequence having at least 70% identity to the amino acid sequence of SEQ ID NO: 159;
(5αA-c) a protein comprising an amino acid sequence encoded by a nucleotide sequence with deletion, substitution, insertion or addition of one or several nucleotides in SEQ ID NO: 158;
(5αA-d) a protein comprising an amino acid sequence encoded by a nucleotide sequence having at least 70% identity to the nucleotide sequence of SEQ ID NO: 158; or
(5αA-e) a protein comprising an amino acid sequence encoded by a nucleic acid that hybridizes under stringent conditions with a nucleic acid having a nucleotide sequence complementary to the nucleotide sequence of SEQ ID NO: 158; or
(5αB) a protein comprising at least one amino acid sequence selected from the group consisting of SEQ ID NOs: 160-161;
(6α)
(6αA1) aconitate hydratase 1 or a variant thereof, which is defined below in any of (6αA1-a) to (6αA1-e):
(6αA1-a) a protein comprising an amino acid sequence with deletion, substitution, insertion or addition of one or several amino acids in SEQ ID NO: 163;
(6αA1-b) a protein comprising an amino acid sequence having at least 70% identity to the amino acid sequence of SEQ ID NO: 163;
(6αA1-c) a protein comprising an amino acid sequence encoded by a nucleotide sequence with deletion, substitution, insertion or addition of one or several nucleotides in SEQ ID NO: 162;
+(6αA1-d) a protein comprising an amino acid sequence encoded by a nucleotide sequence having at least 70% identity to the nucleotide sequence of SEQ ID NO: 162; or
(6αA1-e) a protein comprising an amino acid sequence encoded by a nucleic acid that hybridizes under stringent conditions with a nucleic acid having a nucleotide sequence complementary to the nucleotide sequence of SEQ ID NO: 162;
(6αB1) a protein comprising at least one amino acid sequence selected from the group consisting of SEQ ID NOs: 164-167; or
(6αA2) aconitate hydratase 1 or a variant thereof, which is defined below in any of (6αA2-a) to (6αA2-e):
(6αA2-a) a protein comprising an amino acid sequence with deletion, substitution, insertion or addition of one or several amino acids in SEQ ID NO: 169;
(6αA2-b) a protein comprising an amino acid sequence having at least 70% identity to the amino acid sequence of SEQ ID NO: 169;
(6αA2-c) a protein comprising an amino acid sequence encoded by a nucleotide sequence with deletion, substitution, insertion or addition of one or several nucleotides in SEQ ID NO: 168;
(6αA2-d) a protein comprising an amino acid sequence encoded by a nucleotide sequence having at least 70% identity to the nucleotide sequence of SEQ ID NO: 168; or
(6αA2-e) a protein comprising an amino acid sequence encoded by a nucleic acid that hybridizes under stringent conditions with a nucleic acid having a nucleotide sequence complementary to the nucleotide sequence of SEQ ID NO: 168; or
(6αB2) a protein comprising at least one amino acid sequence selected from the group consisting of SEQ ID NOs: 170-172;
(7α)
(7αA) methionine synthase or a variant thereof, which is defined below in any of (7αA-a) to (7αA-e):
(7αA-a) a protein comprising an amino acid sequence with deletion, substitution, insertion or addition of one or several amino acids in SEQ ID NO: 174;
(7αA-b) a protein comprising an amino acid sequence having at least 70% identity to the amino acid sequence of SEQ ID NO: 174;
(7αA-c) a protein comprising an amino acid sequence encoded by a nucleotide sequence with deletion, substitution, insertion or addition of one or several nucleotides in SEQ ID NO: 173;
(7αA-d) a protein comprising an amino acid sequence encoded by a nucleotide sequence having at least 70% identity to the nucleotide sequence of SEQ ID NO: 173; or
(7αA-e) a protein comprising an amino acid sequence encoded by a nucleic acid that hybridizes under stringent conditions with a nucleic acid having a nucleotide sequence complementary to the nucleotide sequence of SEQ ID NO: 173; or
(7αB) a protein comprising at least one amino acid sequence selected from the group consisting of SEQ ID NOs: 175-187;
(8α)
(8αA) transketolase, chloroplastic or a variant thereof, which is defined below in any of (8αA-a) to (8αA-e):
(8αA-a) a protein comprising an amino acid sequence with deletion, substitution, insertion or addition of one or several amino acids in SEQ ID NO: 189;
(8αA-b) a protein comprising an amino acid sequence having at least 70% identity to the amino acid sequence of SEQ ID NO: 189;
(8αA-c) a protein comprising an amino acid sequence encoded by a nucleotide sequence with deletion, substitution, insertion or addition of one or several nucleotides in SEQ ID NO: 188;
(8αA-d) a protein comprising an amino acid sequence encoded by a nucleotide sequence having at least 70% identity to the nucleotide sequence of SEQ ID NO: 188; or
(8αA-e) a protein comprising an amino acid sequence encoded by a nucleic acid that hybridizes under stringent conditions with a nucleic acid having a nucleotide sequence complementary to the nucleotide sequence of SEQ ID NO: 188; or
(8αB) a protein comprising at least one amino acid sequence selected from the group consisting of SEQ ID NOs: 190-199;
(9α)
(9αA) lysine-tRNA ligase, cytoplasmic-like isoform X2 or a variant thereof, which is defined below in any of (9αA-a) to (9αA-e):
(9αA-a) a protein comprising an amino acid sequence with deletion, substitution, insertion or addition of one or several amino acids in SEQ ID NO: 201;
(9αA-b) a protein comprising an amino acid sequence having at least 70% identity to the amino acid sequence of SEQ ID NO: 201;
(9αA-c) a protein comprising an amino acid sequence encoded by a nucleotide sequence with deletion, substitution, insertion or addition of one or several nucleotides in SEQ ID NO: 201;
(9αA-d) a protein comprising an amino acid sequence encoded by a nucleotide sequence having at least 70% identity to the nucleotide sequence of SEQ ID NO: 200; or
(9αA-e) a protein comprising an amino acid sequence encoded by a nucleic acid that hybridizes under stringent conditions with a nucleic acid having a nucleotide sequence complementary to the nucleotide sequence of SEQ ID NO: 200; or
(9αB) a protein comprising an amino acid sequence of SEQ ID NO: 202;
(10α)
(10αA1) chaperonin CPN60-2, mitochondrial or a variant thereof, which is defined below in any of (10αA1-a) to (10αA1-e):
(10αA1-a) a protein comprising an amino acid sequence with deletion, substitution, insertion or addition of one or several amino acids in SEQ ID NO: 204;
(10αA1-b) a protein comprising an amino acid sequence having at least 70% identity to the amino acid sequence of SEQ ID NO: 204;
(10αA1-c) a protein comprising an amino acid sequence encoded by a nucleotide sequence with deletion, substitution, insertion or addition of one or several nucleotides in SEQ ID NO: 203;
(10αA1-d) a protein comprising an amino acid sequence encoded by a nucleotide sequence having at least 70% identity to the nucleotide sequence of SEQ ID NO: 203; or
(10αA1-e) a protein comprising an amino acid sequence encoded by a nucleic acid that hybridizes under stringent conditions with a nucleic acid having a nucleotide sequence complementary to the nucleotide sequence of SEQ ID NO: 203;
(10αB1) a protein comprising at least one amino acid sequence selected from the group consisting of SEQ ID NOs: 205-218;
(10αA2) chaperonin CPN60-2, mitochondrial-Iike isoform X1 or a variant thereof, which is defined below in any of (10αA2-a) to (10αA2-e):
(10αA2-a) a protein comprising an amino acid sequence with deletion, substitution, insertion or addition of one or several amino acids in SEQ ID NO: 220;
(10αA2-b) a protein comprising an amino acid sequence having at least 70% identity to the amino acid sequence of SEQ ID NO: 220;
(10αA2-c) a protein comprising an amino acid sequence encoded by a nucleotide sequence with deletion, substitution, insertion or addition of one or several nucleotides in SEQ ID NO: 219;
(10αA2-d) a protein comprising an amino acid sequence encoded by a nucleotide sequence having at least 70% identity to the nucleotide sequence of SEQ ID NO: 219; or
(10αA2-e) a protein comprising an amino acid sequence encoded by a nucleic acid that hybridizes under stringent conditions with a nucleic acid having a nucleotide sequence complementary to the nucleotide sequence of SEQ ID NO: 219; or
(10αB2) a protein comprising at least one amino acid sequence selected from the group consisting of SEQ ID NOs: 221-231;
(11α)
(11αA) polyadenylate-binding protein 8 isoform X3 or a variant thereof, which is defined below in any of (11αA-a) to (11αA-e):
(11αA-a) a protein comprising an amino acid sequence with deletion, substitution, insertion or addition of one or several amino acids in SEQ ID NO: 233;
(11αA-b) a protein comprising an amino acid sequence having at least 70% identity to the amino acid sequence of SEQ ID NO: 233;
(11αA-c) a protein comprising an amino acid sequence encoded by a nucleotide sequence with deletion, substitution, insertion or addition of one or several nucleotides in SEQ ID NO: 232;
(11αA-d) a protein comprising an amino acid sequence encoded by a nucleotide sequence having at least 70% identity to the nucleotide sequence of SEQ ID NO: 232; or
(11αA-e) a protein comprising an amino acid sequence encoded by a nucleic acid that hybridizes under stringent conditions with a nucleic acid having a nucleotide sequence complementary to the nucleotide sequence of SEQ ID NO: 232; or
(11αB) a protein comprising at least one amino acid sequence selected from the group consisting of SEQ ID NOs: 234-237;
(12α)
(12αA) pyrophosphate-fructose 6-phosphate 1-phosphotransferase subunit alpha-like or a variant thereof, which is defined below in any of (12αA-a) to (12αA-e):
(12αA-a) a protein comprising an amino acid sequence with deletion, substitution, insertion or addition of one or several amino acids in SEQ ID NO: 239;
(12αA-b) a protein comprising an amino acid sequence having at least 70% identity to the amino acid sequence of SEQ ID NO: 239;
(12αA-c) a protein comprising an amino acid sequence encoded by a nucleotide sequence with deletion, substitution, insertion or addition of one or several nucleotides in SEQ ID NO: 238;
(12αA-d) a protein comprising an amino acid sequence encoded by a nucleotide sequence having at least 70% identity to the nucleotide sequence of SEQ ID NO: 238; or
(12αA-e) a protein comprising an amino acid sequence encoded by a nucleic acid that hybridizes under stringent conditions with a nucleic acid having a nucleotide sequence complementary to the nucleotide sequence of SEQ ID NO: 238; or
(12αB) a protein comprising an amino acid sequence of SEQ ID NO: 240;
(13α)
(13αA1) protein disulfide isomerase-like protein precursor or a variant thereof, which is defined below in any of (13αA1-a) to (13αA1-e):
(13αA1-a) a protein comprising an amino acid sequence with deletion, substitution, insertion or addition of one or several amino acids in SEQ ID NO: 242;
(13αA1-b) a protein comprising an amino acid sequence having at least 70% identity to the amino acid sequence of SEQ ID NO: 242;
(13αA1-c) a protein comprising an amino acid sequence encoded by a nucleotide sequence with deletion, substitution, insertion or addition of one or several nucleotides in SEQ ID NO: 241;
(13αA1-d) a protein comprising an amino acid sequence encoded by a nucleotide sequence having at least 70% identity to the nucleotide sequence of SEQ ID NO: 241; or
(13αA1-e) a protein comprising an amino acid sequence encoded by a nucleic acid that hybridizes under stringent conditions with a nucleic acid having a nucleotide sequence complementary to the nucleotide sequence of SEQ ID NO: 241;
(13αB1) a protein comprising at least one amino acid sequence selected from the group consisting of SEQ ID NOs: 243-252;
(13αA2) protein disulfide-isomerase or a variant thereof, which is defined below in any of (13αA2-a) to (13αA2-e):
(13αA2-a) a protein comprising an amino acid sequence with deletion, substitution, insertion or addition of one or several amino acids in SEQ ID NO: 254;
(13αA2-b) a protein comprising an amino acid sequence having at least 70% identity to the amino acid sequence of SEQ ID NO: 254;
(13αA2-c) a protein comprising an amino acid sequence encoded by a nucleotide sequence with deletion, substitution, insertion or addition of one or several nucleotides in SEQ ID NO: 253;
(13αA2-d) a protein comprising an amino acid sequence encoded by a nucleotide sequence having at least 70% identity to the nucleotide sequence of SEQ ID NO: 253; or
(13αA2-e) a protein comprising an amino acid sequence encoded by a nucleic acid that hybridizes under stringent conditions with a nucleic acid having a nucleotide sequence complementary to the nucleotide sequence of SEQ ID NO: 253; or
(13αB2) a protein comprising at least one amino acid sequence selected from the group consisting of SEQ ID NOs: 255-278;
(14α)
(14αA1) V-type proton ATPase subunit B 2 or a variant thereof, which is defined below in any of (14αA1-a) to (14αA1-e):
(14αA1-a) a protein comprising an amino acid sequence with deletion, substitution, insertion or addition of one or several amino acids in SEQ ID NO: 280;
(14αA1-b) a protein comprising an amino acid sequence having at least 70% identity to the amino acid sequence of SEQ ID NO: 280;
(14αA1-c) a protein comprising an amino acid sequence encoded by a nucleotide sequence with deletion, substitution, insertion or addition of one or several nucleotides in SEQ ID NO: 279;
(14αA1-d) a protein comprising an amino acid sequence encoded by a nucleotide sequence having at least 70% identity to the nucleotide sequence of SEQ ID NO: 279; or
(14αA1-e) a protein comprising an amino acid sequence encoded by a nucleic acid that hybridizes under stringent conditions with a nucleic acid having a nucleotide sequence complementary to the nucleotide sequence of SEQ ID NO: 279;
(14αB1) a protein comprising at least one amino acid sequence selected from the group consisting of SEQ ID NOs: 281-284;
(14αA2) V-type proton ATPase subunit B 2 or a variant thereof, which is defined below in any of (14αA2-a) to (14αA2-e):
(14αA2-a) a protein comprising an amino acid sequence with deletion, substitution, insertion or addition of one or several amino acids in SEQ ID NO: 286;
(14αA2-b) a protein comprising an amino acid sequence having at least 70% identity to the amino acid sequence of SEQ ID NO: 286;
(14αA2-c) a protein comprising an amino acid sequence encoded by a nucleotide sequence with deletion, substitution, insertion or addition of one or several nucleotides in SEQ ID NO: 285;
(14αA2-d) a protein comprising an amino acid sequence encoded by a nucleotide sequence having at least 70% identity to the nucleotide sequence of SEQ ID NO: 285; or
(14αA2-e) a protein comprising an amino acid sequence encoded by a nucleic acid that hybridizes under stringent conditions with a nucleic acid having a nucleotide sequence complementary to the nucleotide sequence of SEQ ID NO: 285; or
(14αB2) a protein comprising at least one amino acid sequence selected from the group consisting of SEQ ID NOs: 287-289;
(15, 16α)
(15, 16αA1) UTP-glucose-1-phosphate uridylyltransferase or a variant thereof, which is defined below in any of (15, 16αA1-a) to (15, 16αA1-e):
(15, 16αA1-a) a protein comprising an amino acid sequence with deletion, substitution, insertion or addition of one or several amino acids in SEQ ID NO: 291;
(15, 16αA1-b) a protein comprising an amino acid sequence having at least 70% identity to the amino acid sequence of SEQ ID NO: 291;
(15, 16αA1-c) a protein comprising an amino acid sequence encoded by a nucleotide sequence with deletion, substitution, insertion or addition of one or several nucleotides in SEQ ID NO: 290;
(15, 16αA1-d) a protein comprising an amino acid sequence encoded by a nucleotide sequence having at least 70% identity to the nucleotide sequence of SEQ ID NO: 290; or
(15, 16αA1-e) a protein comprising an amino acid sequence encoded by a nucleic acid that hybridizes under stringent conditions with a nucleic acid having a nucleotide sequence complementary to the nucleotide sequence of SEQ ID NO: 290;
(15, 16αB1) a protein comprising at least one amino acid sequence selected from the group consisting of SEQ ID NOs: 292-304;
(15, 16αA2) hypothetical protein GLYMA_02G241100 or a variant thereof, which is defined below in any of (15, 16αA2-a) to (15, 16αA2-e):
(15, 16αA2-a) a protein comprising an amino acid sequence with deletion, substitution, insertion or addition of one or several amino acids in SEQ ID NO: 306;
(15, 16αA2-b) a protein comprising an amino acid sequence having at least 70% identity to the amino acid sequence of SEQ ID NO: 306;
(15, 16αA2-c) a protein comprising an amino acid sequence encoded by a nucleotide sequence with deletion, substitution, insertion or addition of one or several nucleotides in SEQ ID NO: 305;
(15, 16αA2-d) a protein comprising an amino acid sequence encoded by a nucleotide sequence having at least 70% identity to the nucleotide sequence of SEQ ID NO: 305; or
(15, 16αA2-e) a protein comprising an amino acid sequence encoded by a nucleic acid that hybridizes under stringent conditions with a nucleic acid having a nucleotide sequence complementary to the nucleotide sequence of SEQ ID NO: 305; or
(15, 16αB2) a protein comprising at least one amino acid sequence selected from the group consisting of SEQ ID NOs: 307-318;
(17α)
(17αA1) guanosine nucleotide diphosphate dissociation inhibitor 2 or a variant thereof, which is defined below in any of (17αA1-a) to (17αA1-e):
(17αA1-a) a protein comprising an amino acid sequence with deletion, substitution, insertion or addition of one or several amino acids in SEQ ID NO: 320;
(17αA1-b) a protein comprising an amino acid sequence having at least 70% identity to the amino acid sequence of SEQ ID NO: 320;
(17αA1-c) a protein comprising an amino acid sequence encoded by a nucleotide sequence with deletion, substitution, insertion or addition of one or several nucleotides in SEQ ID NO: 319;
(17αA1-d) a protein comprising an amino acid sequence encoded by a nucleotide sequence having at least 70% identity to the nucleotide sequence of SEQ ID NO: 319; or
(17αA1-e) a protein comprising an amino acid sequence encoded by a nucleic acid that hybridizes under stringent conditions with a nucleic acid having a nucleotide sequence complementary to the nucleotide sequence of SEQ ID NO: 319;
(17αB1) a protein comprising at least one amino acid sequence selected from the group consisting of SEQ ID NOs: 321-325;
(17αA2) rab GDP dissociation inhibitor alpha-like or a variant thereof, which is defined below in any of (17αA2-a) to (17αA2-e):
(17αA2-a) a protein comprising an amino acid sequence with deletion, substitution, insertion or addition of one or several amino acids in SEQ ID NO: 327;
(17αA2-b) a protein comprising an amino acid sequence having at least 70% identity to the amino acid sequence of SEQ ID NO: 327;
(17αA2-c) a protein comprising an amino acid sequence encoded by a nucleotide sequence with deletion, substitution, insertion or addition of one or several nucleotides in SEQ ID NO: 326;
(17αA2-d) a protein comprising an amino acid sequence encoded by a nucleotide sequence having at least 70% identity to the nucleotide sequence of SEQ ID NO: 326; or
(17αA2-e) a protein comprising an amino acid sequence encoded by a nucleic acid that hybridizes under stringent conditions with a nucleic acid having a nucleotide sequence complementary to the nucleotide sequence of SEQ ID NO: 326; or
(17αB2) a protein comprising at least one amino acid sequence selected from the group consisting of SEQ ID NOs: 328-332;
(18α)
(18αA) hypothetical protein GLYMA_10G028300 or a variant thereof, which is defined below in any of (18αA-a) to (18αA-e):
(18αA-a) a protein comprising an amino acid sequence with deletion, substitution, insertion or addition of one or several amino acids in SEQ ID NO: 334;
(18αA-b) a protein comprising an amino acid sequence having at least 70% identity to the amino acid sequence of SEQ ID NO: 334;
(18αA-c) a protein comprising an amino acid sequence encoded by a nucleotide sequence with deletion, substitution, insertion or addition of one or several nucleotides in SEQ ID NO: 333;
(18αA-d) a protein comprising an amino acid sequence encoded by a nucleotide sequence having at least 70% identity to the nucleotide sequence of SEQ ID NO: 333; or
(18αA-e) a protein comprising an amino acid sequence encoded by a nucleic acid that hybridizes under stringent conditions with a nucleic acid having a nucleotide sequence complementary to the nucleotide sequence of SEQ ID NO: 333; or
(18αB) a protein comprising at least one amino acid sequence selected from the group consisting of SEQ ID NOs: 335-339;
(19α)
(19αA) sucrose binding protein homolog S-64 or a variant thereof, which is defined below in any of (19αA-a) to (19αA-e):
(19αA-a) a protein comprising an amino acid sequence with deletion, substitution, insertion or addition of one or several amino acids in SEQ ID NO: 341;
(19αA-b) a protein comprising an amino acid sequence having at least 70% identity to the amino acid sequence of SEQ ID NO: 341;
(19αA-c) a protein comprising an amino acid sequence encoded by a nucleotide sequence with deletion, substitution, insertion or addition of one or several nucleotides in SEQ ID NO: 340;
(19αA-d) a protein comprising an amino acid sequence encoded by a nucleotide sequence having at least 70% identity to the nucleotide sequence of SEQ ID NO: 340; or
(19αA-e) a protein comprising an amino acid sequence encoded by a nucleic acid that hybridizes under stringent conditions with a nucleic acid having a nucleotide sequence complementary to the nucleotide sequence of SEQ ID NO: 340; or
(19αB) a protein comprising at least one amino acid sequence selected from the group consisting of SEQ ID NOs: 342-346;
(25α)
(25αA1) succinyl-CoA ligase [ADP-forming] subunit beta, mitochondrial or a variant thereof, which is defined below in any of (25αA1-a) to (25αA1-e):
(25αA1-a) a protein comprising an amino acid sequence with deletion, substitution, insertion or addition of one or several amino acids in SEQ ID NO: 348;
(25αA1-b) a protein comprising an amino acid sequence having at least 70% identity to the amino acid sequence of SEQ ID NO: 348;
(25αA1-c) a protein comprising an amino acid sequence encoded by a nucleotide sequence with deletion, substitution, insertion or addition of one or several nucleotides in SEQ ID NO: 347;
(25αA1-d) a protein comprising an amino acid sequence encoded by a nucleotide sequence having at least 70% identity to the nucleotide sequence of SEQ ID NO: 347; or
(25αA1-e) a protein comprising an amino acid sequence encoded by a nucleic acid that hybridizes under stringent conditions with a nucleic acid having a nucleotide sequence complementary to the nucleotide sequence of SEQ ID NO: 347;
(25αB1) a protein comprising at least one amino acid sequence selected from the group consisting of SEQ ID NOs: 349-356;
(25αA2) succinyl-CoA ligase [ADP-forming] subunit beta, mitochondrial or a variant thereof, which is defined below in any of (25αA2-a) to (25αA2-e):
(25αA2-a) a protein comprising an amino acid sequence with deletion, substitution, insertion or addition of one or several amino acids in SEQ ID NO: 358;
(25αA2-b) a protein comprising an amino acid sequence having at least 70% identity to the amino acid sequence of SEQ ID NO: 358;
(25αA2-c) a protein comprising an amino acid sequence encoded by a nucleotide sequence with deletion, substitution, insertion or addition of one or several nucleotides in SEQ ID NO: 357;
(25αA2-d) a protein comprising an amino acid sequence encoded by a nucleotide sequence having at least 70% identity to the nucleotide sequence of SEQ ID NO: 357; or
(25αA2-e) a protein comprising an amino acid sequence encoded by a nucleic acid that hybridizes under stringent conditions with a nucleic acid having a nucleotide sequence complementary to the nucleotide sequence of SEQ ID NO: 357; or
(25αB2) a protein comprising at least one amino acid sequence selected from the group consisting of SEQ ID NOs: 359-365;
(26α)
(26αA1) phosphoglycerate kinase, cytosolic or a variant thereof, which is defined below in any of (26αA1-a) to (26αA1-e):
(26αA1-a) a protein comprising an amino acid sequence with deletion, substitution, insertion or addition of one or several amino acids in SEQ ID NO: 367;
(26αA1-b) a protein comprising an amino acid sequence having at least 70% identity to the amino acid sequence of SEQ ID NO: 367;
(26αA1-c) a protein comprising an amino acid sequence encoded by a nucleotide sequence with deletion, substitution, insertion or addition of one or several nucleotides in SEQ ID NO: 366;
(26αA1-d) a protein comprising an amino acid sequence encoded by a nucleotide sequence having at least 70% identity to the nucleotide sequence of SEQ ID NO: 366; or
(26αA1-e) a protein comprising an amino acid sequence encoded by a nucleic acid that hybridizes under stringent conditions with a nucleic acid having a nucleotide sequence complementary to the nucleotide sequence of SEQ ID NO: 366;
(26αB1) a protein comprising at least one amino acid sequence selected from the group consisting of SEQ ID NOs: 368-382;
(26αA2) phosphoglycerate kinase, cytosolic or a variant thereof, which is defined below in any of (26αA2-a) to (26αA2-e):
(26αA2-a) a protein comprising an amino acid sequence with deletion, substitution, insertion or addition of one or several amino acids in SEQ ID NO: 384;
(26αA2-b) a protein comprising an amino acid sequence having at least 70% identity to the amino acid sequence of SEQ ID NO: 384;
(26αA2-c) a protein comprising an amino acid sequence encoded by a nucleotide sequence with deletion, substitution, insertion or addition of one or several nucleotides in SEQ ID NO: 383;
(26αA2-d) a protein comprising an amino acid sequence encoded by a nucleotide sequence having at least 70% identity to the nucleotide sequence of SEQ ID NO: 383; or
(26αA2-e) a protein comprising an amino acid sequence encoded by a nucleic acid that hybridizes under stringent conditions with a nucleic acid having a nucleotide sequence complementary to the nucleotide sequence of SEQ ID NO: 383;
(26αB2) a protein comprising at least one amino acid sequence selected from the group consisting of SEQ ID NOs: 385-392;
(26αA3) phosphoglycerate kinase, cytosolic or a variant thereof, which is defined below in any of (26αA3-a) to (26αA3-e):
(26αA3-a) a protein comprising an amino acid sequence with deletion, substitution, insertion or addition of one or several amino acids in SEQ ID NO: 394;
(26αA3-b) a protein comprising an amino acid sequence having at least 70% identity to the amino acid sequence of SEQ ID NO: 394;
(26αA3-c) a protein comprising an amino acid sequence encoded by a nucleotide sequence with deletion, substitution, insertion or addition of one or several nucleotides in SEQ ID NO: 393;
(26αA3-d) a protein comprising an amino acid sequence encoded by a nucleotide sequence having at least 70% identity to the nucleotide sequence of SEQ ID NO: 393; or
(26αA3-e) a protein comprising an amino acid sequence encoded by a nucleic acid that hybridizes under stringent conditions with a nucleic acid having a nucleotide sequence complementary to the nucleotide sequence of SEQ ID NO: 393; or
(26αB3) a protein comprising at least one amino acid sequence selected from the group consisting of SEQ ID NOs: 395-409;
(27α)
(27αA1) alcohol dehydrogenase 1 or a variant thereof, which is defined below in any of (27αA1-a) to (27αA1-e):
(27αA1-a) a protein comprising an amino acid sequence with deletion, substitution, insertion or addition of one or several amino acids in SEQ ID NO: 411;
(27αA1-b) a protein comprising an amino acid sequence having at least 70% identity to the amino acid sequence of SEQ ID NO: 411;
(27αA1-c) a protein comprising an amino acid sequence encoded by a nucleotide sequence with deletion, substitution, insertion or addition of one or several nucleotides in SEQ ID NO: 410;
(27αA1-d) a protein comprising an amino acid sequence encoded by a nucleotide sequence having at least 70% identity to the nucleotide sequence of SEQ ID NO: 410; or
(27αA1-e) a protein comprising an amino acid sequence encoded by a nucleic acid that hybridizes under stringent conditions with a nucleic acid having a nucleotide sequence complementary to the nucleotide sequence of SEQ ID NO: 410;
(27αB1) a protein comprising at least one amino acid sequence selected from the group consisting of SEQ ID NOs: 412-416;
(27αA2) alcohol dehydrogenase 1 or a variant thereof, which is defined below in any of (27αA2-a) to (27αA2-e):
(27αA2-a) a protein comprising an amino acid sequence with deletion, substitution, insertion or addition of one or several amino acids in SEQ ID NO: 418;
(27αA2-b) a protein comprising an amino acid sequence having at least 70% identity to the amino acid sequence of SEQ ID NO: 418;
(27αA2-c) a protein comprising an amino acid sequence encoded by a nucleotide sequence with deletion, substitution, insertion or addition of one or several nucleotides in SEQ ID NO: 417;
(27αA2-d) a protein comprising an amino acid sequence encoded by a nucleotide sequence having at least 70% identity to the nucleotide sequence of SEQ ID NO: 417; or
(27αA2-e) a protein comprising an amino acid sequence encoded by a nucleic acid that hybridizes under stringent conditions with a nucleic acid having a nucleotide sequence complementary to the nucleotide sequence of SEQ ID NO: 417;
(27αB2) a protein comprising at least one amino acid sequence selected from the group consisting of SEQ ID NOs: 419-420;
(27αA3) alcohol dehydrogenase 1-like or a variant thereof, which is defined below in any of (27αA3-a) to (27αA3-e):
(27αA3-a) a protein comprising an amino acid sequence with deletion, substitution, insertion or addition of one or several amino acids in SEQ ID NO: 422;
(27αA3-b) a protein comprising an amino acid sequence having at least 70% identity to the amino acid sequence of SEQ ID NO: 422;
(27αA3-c) a protein comprising an amino acid sequence encoded by a nucleotide sequence with deletion, substitution, insertion or addition of one or several nucleotides in SEQ ID NO: 421;
(27αA3-d) a protein comprising an amino acid sequence encoded by a nucleotide sequence having at least 70% identity to the nucleotide sequence of SEQ ID NO: 421; or
(27αA3-e) a protein comprising an amino acid sequence encoded by a nucleic acid that hybridizes under stringent conditions with a nucleic acid having a nucleotide sequence complementary to the nucleotide sequence of SEQ ID NO: 421; or
(27αB3) a protein comprising at least one amino acid sequence selected from the group consisting of SEQ ID NOs: 423-427;
(28α)
(28αA) 35 kDa seed maturation protein isoform X1 or a variant thereof, which is defined below in any of (28αA-a) to (28αA-e):
(28αA-a) a protein comprising an amino acid sequence with deletion, substitution, insertion or addition of one or several amino acids in SEQ ID NO: 429;
(28αA-b) a protein comprising an amino acid sequence having at least 70% identity to the amino acid sequence of SEQ ID NO: 429;
(28αA-c) a protein comprising an amino acid sequence encoded by a nucleotide sequence with deletion, substitution, insertion or addition of one or several nucleotides in SEQ ID NO: 428;
(28αA-d) a protein comprising an amino acid sequence encoded by a nucleotide sequence having at least 70% identity to the nucleotide sequence of SEQ ID NO: 428; or
(28αA-e) a protein comprising an amino acid sequence encoded by a nucleic acid that hybridizes under stringent conditions with a nucleic acid having a nucleotide sequence complementary to the nucleotide sequence of SEQ ID NO: 428; or
(28αB) a protein comprising at least one amino acid sequence selected from the group consisting of SEQ ID NOs: 430-433;
(29-33α)
(29-33αA1) glyceraldehyde-3-phosphate dehydrogenase isoform X1 or a variant thereof, which is defined below in any of (29-33αA1-a) to (29-33αA1-e):
(29-33αA1-a) a protein comprising an amino acid sequence with deletion, substitution, insertion or addition of one or several amino acids in SEQ ID NO: 435;
(29-33αA1-b) a protein comprising an amino acid sequence having at least 70% identity to the amino acid sequence of SEQ ID NO: 435;
(29-33αA1-c) a protein comprising an amino acid sequence encoded by a nucleotide sequence with deletion, substitution, insertion or addition of one or several nucleotides in SEQ ID NO: 434;
(29-33αA1-d) a protein comprising an amino acid sequence encoded by a nucleotide sequence having at least 70% identity to the nucleotide sequence of SEQ ID NO: 434; or
(29-33αA1-e) a protein comprising an amino acid sequence encoded by a nucleic acid that hybridizes under stringent conditions with a nucleic acid having a nucleotide sequence complementary to the nucleotide sequence of SEQ ID NO: 434;
(29-33αB1) a protein comprising at least one amino acid sequence selected from the group consisting of SEQ ID NOs: 436-439;
(29-33αA2) glyceraldehyde-3-phosphate dehydrogenase, cytosolic or a variant thereof, which is defined below in any of (29-33αA2-a) to (29-33αA2-e):
(29-33αA2-a) a protein comprising an amino acid sequence with deletion, substitution, insertion or addition of one or several amino acids in SEQ ID NO: 441;
(29-33αA2-b) a protein comprising an amino acid sequence having at least 70% identity to the amino acid sequence of SEQ ID NO: 441;
(29-33αA2-c) a protein comprising an amino acid sequence encoded by a nucleotide sequence with deletion, substitution, insertion or addition of one or several nucleotides in SEQ ID NO: 440;
(29-33αA2-d) a protein comprising an amino acid sequence encoded by a nucleotide sequence having at least 70% identity to the nucleotide sequence of SEQ ID NO: 440; or
(29-33αA2-e) a protein comprising an amino acid sequence encoded by a nucleic acid that hybridizes under stringent conditions with a nucleic acid having a nucleotide sequence complementary to the nucleotide sequence of SEQ ID NO: 440;
(29-33αB2) a protein comprising at least one amino acid sequence selected from the group consisting of SEQ ID NOs: 442-445;
(29-33αA3) uncharacterized protein LOC100782924 or a variant thereof, which is defined below in any of (29-33αA3-a) to (29-33αA3-e):
(29-33αA3-a) a protein comprising an amino acid sequence with deletion, substitution, insertion or addition of one or several amino acids in SEQ ID NO: 447;
(29-33αA3-b) a protein comprising an amino acid sequence having at least 70% identity to the amino acid sequence of SEQ ID NO: 447;
(29-33αA3-c) a protein comprising an amino acid sequence encoded by a nucleotide sequence with deletion, substitution, insertion or addition of one or several nucleotides in SEQ ID NO: 446;
(29-33αA3-d) a protein comprising an amino acid sequence encoded by a nucleotide sequence having at least 70% identity to the nucleotide sequence of SEQ ID NO: 446; or
(29-33αA3-e) a protein comprising an amino acid sequence encoded by a nucleic acid that hybridizes under stringent conditions with a nucleic acid having a nucleotide sequence complementary to the nucleotide sequence of SEQ ID NO: 446; or
(29-33αB3) a protein comprising at least one amino acid sequence selected from the group consisting of SEQ ID NOs: 448-451,
(34α)
(34αA1) bifunctional UDP-glucose 4-epimerase and UDP-xylose 4-epimerase 1-like or a variant thereof, which is defined below in any of (34αA1-a) to (34αA1-e):
(34αA1-a) a protein comprising an amino acid sequence with deletion, substitution, insertion or addition of one or several amino acids in SEQ ID NO: 453;
(34αA1-b) a protein comprising an amino acid sequence having at least 70% identity to the amino acid sequence of SEQ ID NO: 453;
(34αA1-c) a protein comprising an amino acid sequence encoded by a nucleotide sequence with deletion, substitution, insertion or addition of one or several nucleotides in SEQ ID NO: 452;
(34αA1-d) a protein comprising an amino acid sequence encoded by a nucleotide sequence having at least 70% identity to the nucleotide sequence of SEQ ID NO: 452; or
(34αA1-e) a protein comprising an amino acid sequence encoded by a nucleic acid that hybridizes under stringent conditions with a nucleic acid having a nucleotide sequence complementary to the nucleotide sequence of SEQ ID NO: 452;
(34αB1) a protein comprising an amino acid sequence of SEQ ID NO: 454;
(34αA2) uncharacterized protein LOCI00803483 or a variant thereof, which is defined below in any of (34αA2-a) to (34αA2-e):
(34αA2-a) a protein comprising an amino acid sequence with deletion, substitution, insertion or addition of one or several amino acids in SEQ ID NO: 456;
(34αA2-b) a protein comprising an amino acid sequence having at least 70% identity to the amino acid sequence of SEQ ID NO: 456;
(34αA2-c) a protein comprising an amino acid sequence encoded by a nucleotide sequence with deletion, substitution, insertion or addition of one or several nucleotides in SEQ ID NO: 455;
(34αA2-d) a protein comprising an amino acid sequence encoded by a nucleotide sequence having at least 70% identity to the nucleotide sequence of SEQ ID NO: 455; or
(34αA2-e) a protein comprising an amino acid sequence encoded by a nucleic acid that hybridizes under stringent conditions with a nucleic acid having a nucleotide sequence complementary to the nucleotide sequence of SEQ ID NO: 455; or
(34αB2) a protein comprising at least one amino acid sequence selected from the group consisting of SEQ ID NOs: 457 and 458;
(35α)
(35αA1) elongation factor 1-alpha or a variant thereof, which is defined below in any of (35αA1-a) to (35αA1-e):
(35αA1-a) a protein comprising an amino acid sequence with deletion, substitution, insertion or addition of one or several amino acids in SEQ ID NO: 460;
(35αA1-b) a protein comprising an amino acid sequence having at least 70% identity to the amino acid sequence of SEQ ID NO: 460;
(35αA1-c) a protein comprising an amino acid sequence encoded by a nucleotide sequence with deletion, substitution, insertion or addition of one or several nucleotides in SEQ ID NO: 459;
(35αA1-d) a protein comprising an amino acid sequence encoded by a nucleotide sequence having at least 70% identity to the nucleotide sequence of SEQ ID NO: 459; or
(35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
(35αB1) a protein comprising at least one amino acid sequence selected from the group consisting of SEQ ID NOs: 461-466;
(35αA2) elongation factor 1-alpha or a variant thereof, which is defined below in any of (35αA2-a) to (35αA2-e):
(35αA2-a) a protein comprising an amino acid sequence with deletion, substitution, insertion or addition of one or several amino acids in SEQ ID NO: 468;
(35αA2-b) a protein comprising an amino acid sequence having at least 70% identity to the amino acid sequence of SEQ ID NO: 468;
(35αA2-c) a protein comprising an amino acid sequence encoded by a nucleotide sequence with deletion, substitution, insertion or addition of one or several nucleotides in SEQ ID NO: 467;
(35αA2-d) a protein comprising an amino acid sequence encoded by a nucleotide sequence having at least 70% identity to the nucleotide sequence of SEQ ID NO: 467; or
(35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
(35αB2) a protein comprising at least one amino acid sequence selected from the group consisting of SEQ ID NOs: 469-471;
(35αA3) elongation factor-1A isoform X1 or a variant thereof, which is defined below in any of (35αA3-a) to (35αA3-e):
(35αA3-a) a protein comprising an amino acid sequence with deletion, substitution, insertion or addition of one or several amino acids in SEQ ID NO: 473;
(35αA3-b) a protein comprising an amino acid sequence having at least 70% identity to the amino acid sequence of SEQ ID NO: 473;
(35αA3-c) a protein comprising an amino acid sequence encoded by a nucleotide sequence with deletion, substitution, insertion or addition of one or several nucleotides in SEQ ID NO: 472;
(35αA3-d) a protein comprising an amino acid sequence encoded by a nucleotide sequence having at least 70% identity to the nucleotide sequence of SEQ ID NO: 472; or
(35αA3-e) a protein comprising an amino acid sequence encoded by a nucleic acid that hybridizes under stringent conditions with a nucleic acid having a nucleotide sequence complementary to the nucleotide sequence of SEQ ID NO: 472; or
(35αB3) a protein comprising at least one amino acid sequence selected from the group consisting of SEQ ID NOs: 474-477;
(36, 37α)
(36, 37αA1) seed maturation protein PM34 or a variant thereof, which is defined below in any of (36, 37αA1-a) to (36, 37αA1-e):
(36, 37αA1-a) a protein comprising an amino acid sequence with deletion, substitution, insertion or addition of one or several amino acids in SEQ ID NO: 479;
(36, 37αA1-b) a protein comprising an amino acid sequence having at least 70% identity to the amino acid sequence of SEQ ID NO: 479;
(36, 37αA1-c) a protein comprising an amino acid sequence encoded by a nucleotide sequence with deletion, substitution, insertion or addition of one or several nucleotides in SEQ ID NO: 478;
(36, 37αA1-d) a protein comprising an amino acid sequence encoded by a nucleotide sequence having at least 70% identity to the nucleotide sequence of SEQ ID NO: 478; or
(36, 37αA1-e) a protein comprising an amino acid sequence encoded by a nucleic acid that hybridizes under stringent conditions with a nucleic acid having a nucleotide sequence complementary to the nucleotide sequence of SEQ ID NO: 478;
(36, 37αB1) a protein comprising at least one amino acid sequence selected from the group consisting of SEQ ID NOs: 480-483;
(36, 37αA2) uncharacterized protein LOC100809384 or a variant thereof, which is defined below in any of (36, 37αA2-a) to (36, 37αA2-e):
(36, 37αA2-a) a protein comprising an amino acid sequence with deletion, substitution, insertion or addition of one or several amino acids in SEQ ID NO: 485;
(36, 37αA2-b) a protein comprising an amino acid sequence having at least 70% identity to the amino acid sequence of SEQ ID NO: 485;
(36, 37αA2-c) a protein comprising an amino acid sequence encoded by a nucleotide sequence with deletion, substitution, insertion or addition of one or several nucleotides in SEQ ID NO: 484;
(36, 37αA2-d) a protein comprising an amino acid sequence encoded by a nucleotide sequence having at least 70% identity to the nucleotide sequence of SEQ ID NO: 484; or
(36, 37αA2-e) a protein comprising an amino acid sequence encoded by a nucleic acid that hybridizes under stringent conditions with a nucleic acid having a nucleotide sequence complementary to the nucleotide sequence of SEQ ID NO: 484;
(36, 37αB2) a protein comprising at least one amino acid sequence selected from the group consisting of SEQ ID NOs: 486 and 487;
(36, 37αA3) hypothetical protein GLYMA_10G155300 or a variant thereof, which is defined below in any of (36, 37αA3-a) to (36, 37αA3-e):
(36, 37αA3-a) a protein comprising an amino acid sequence with deletion, substitution, insertion or addition of one or several amino acids in SEQ ID NO: 489;
(36, 37αA3-b) a protein comprising an amino acid sequence having at least 70% identity to the amino acid sequence of SEQ ID NO: 489;
(36, 37αA3-c) a protein comprising an amino acid sequence encoded by a nucleotide sequence with deletion, substitution, insertion or addition of one or several nucleotides in SEQ ID NO: 488;
(36, 37αA3-d) a protein comprising an amino acid sequence encoded by a nucleotide sequence having at least 70% identity to the nucleotide sequence of SEQ ID NO: 488; or
(36, 37αA3-e) a protein comprising an amino acid sequence encoded by a nucleic acid that hybridizes under stringent conditions with a nucleic acid having a nucleotide sequence complementary to the nucleotide sequence of SEQ ID NO: 488; or
(36, 37αB3) a protein comprising at least one amino acid sequence selected from the group consisting of SEQ ID NOs: 490-493;
(38α)
(38αA) seed biotin-containing protein SBP65-like isoform XI or a variant thereof, which is defined below in any of (38αA-a) to (38αA-e):
(38αA-a) a protein comprising an amino acid sequence with deletion, substitution, insertion or addition of one or several amino acids in SEQ ID NO: 495;
(38αA-b) a protein comprising an amino acid sequence having at least 70% identity to the amino acid sequence of SEQ ID NO: 495;
(38αA-c) a protein comprising an amino acid sequence encoded by a nucleotide sequence with deletion, substitution, insertion or addition of one or several nucleotides in SEQ ID NO: 494;
(38αA-d) a protein comprising an amino acid sequence encoded by a nucleotide sequence having at least 70% identity to the nucleotide sequence of SEQ ID NO: 494; or
(38αA-e) a protein comprising an amino acid sequence encoded by a nucleic acid that hybridizes under stringent conditions with a nucleic acid having a nucleotide sequence complementary to the nucleotide sequence of SEQ ID NO: 494; or
(38αB) a protein comprising at least one amino acid sequence selected from the group consisting of SEQ ID NOs: 496-513;
(39α)
(39αA) ATP synthase subunit O, mitochondrial-like or a variant thereof, which is defined below in any of (39αA-a) to (39αA-e):
(39αA-a) a protein comprising an amino acid sequence with deletion, substitution, insertion or addition of one or several amino acids in SEQ ID NO: 515;
(39αA-b) a protein comprising an amino acid sequence having at least 70% identity to the amino acid sequence of SEQ ID NO: 515;
(39αA-c) a protein comprising an amino acid sequence encoded by a nucleotide sequence with deletion, substitution, insertion or addition of one or several nucleotides in SEQ ID NO: 514;
(39αA-d) a protein comprising an amino acid sequence encoded by a nucleotide sequence having at least 70% identity to the nucleotide sequence of SEQ ID NO: 514; or
(39αA-e) a protein comprising an amino acid sequence encoded by a nucleic acid that hybridizes under stringent conditions with a nucleic acid having a nucleotide sequence complementary to the nucleotide sequence of SEQ ID NO: 514; or
(39αB) a protein comprising an amino acid sequence of SEQ ID NO: 516;
(40, 56α)
(40, 56αA) P24 oleosin isoform A or a variant thereof, which is defined below in any of (40, 56αA-a) to (40, 56αA-e):
(40, 56αA-a) a protein comprising an amino acid sequence with deletion, substitution, insertion or addition of one or several amino acids in SEQ ID NO: 518;
(40, 56αA-b) a protein comprising an amino acid sequence having at least 70% identity to the amino acid sequence of SEQ ID NO: 518;
(40, 56αA-c) a protein comprising an amino acid sequence encoded by a nucleotide sequence with deletion, substitution, insertion or addition of one or several nucleotides in SEQ ID NO: 517;
(40, 56αA-d) a protein comprising an amino acid sequence encoded by a nucleotide sequence having at least 70% identity to the nucleotide sequence of SEQ ID NO: 517; or
(40, 56αA-e) a protein comprising an amino acid sequence encoded by a nucleic acid that hybridizes under stringent conditions with a nucleic acid having a nucleotide sequence complementary to the nucleotide sequence of SEQ ID NO: 517; or
(40, 56αB) a protein comprising at least one amino acid sequence selected from the group consisting of SEQ ID NOs: 519 and 520;
(41α)
(41αA) uncharacterized protein At3g49720-like isoform X2 or a variant thereof, which is defined below in any of (41αA-a) to (41αA-e):
(41αA-a) a protein comprising an amino acid sequence with deletion, substitution, insertion or addition of one or several amino acids in SEQ ID NO: 522;
(41αA-b) a protein comprising an amino acid sequence having at least 70% identity to the amino acid sequence of SEQ ID NO: 522;
(41αA-c) a protein comprising an amino acid sequence encoded by a nucleotide sequence with deletion, substitution, insertion or addition of one or several nucleotides in SEQ ID NO: 521;
(41αA-d) a protein comprising an amino acid sequence encoded by a nucleotide sequence having at least 70% identity to the nucleotide sequence of SEQ ID NO: 521; or
(41αA-e) a protein comprising an amino acid sequence encoded by a nucleic acid that hybridizes under stringent conditions with a nucleic acid having a nucleotide sequence complementary to the nucleotide sequence of SEQ ID NO: 521; or
(41αB) a protein comprising an amino acid sequence of SEQ ID NO: 523;
(42, 43α)
(42, 43αA1) superoxide dismutase [Mn], mitochondrial or a variant thereof, which is defined below in any of (42, 43αA1-a) to (42, 43αA1-e):
(42, 43αA1-a) a protein comprising an amino acid sequence with deletion, substitution, insertion or addition of one or several amino acids in SEQ ID NO: 525;
(42, 43αA1-b) a protein comprising an amino acid sequence having at least 70% identity to the amino acid sequence of SEQ ID NO: 525;
(42, 43αA1-c) a protein comprising an amino acid sequence encoded by a nucleotide sequence with deletion, substitution, insertion or addition of one or several nucleotides in SEQ ID NO: 524;
(42, 43αA1-d) a protein comprising an amino acid sequence encoded by a nucleotide sequence having at least 70% identity to the nucleotide sequence of SEQ ID NO: 524; or
(42, 43αA1-e) a protein comprising an amino acid sequence encoded by a nucleic acid that hybridizes under stringent conditions with a nucleic acid having a nucleotide sequence complementary to the nucleotide sequence of SEQ ID NO: 524;
(42, 43αB1) a protein comprising at least one amino acid sequence selected from the group consisting of SEQ ID NOs: 526-531;
(42, 43αA2) hypothetical protein GLYMA_04G221300 or a variant thereof, which is defined below in any of (42, 43αA2-a) to (42, 43αA2-e):
(42, 43αA2-a) a protein comprising an amino acid sequence with deletion, substitution, insertion or addition of one or several amino acids in SEQ ID NO: 533;
(42, 43αA2-b) a protein comprising an amino acid sequence having at least 70% identity to the amino acid sequence of SEQ ID NO: 533;
(42, 43αA2-c) a protein comprising an amino acid sequence encoded by a nucleotide sequence with deletion, substitution, insertion or addition of one or several nucleotides in SEQ ID NO: 532;
(42, 43αA2-d) a protein comprising an amino acid sequence encoded by a nucleotide sequence having at least 70% identity to the nucleotide sequence of SEQ ID NO: 532; or
(42, 43αA2-e) a protein comprising an amino acid sequence encoded by a nucleic acid that hybridizes under stringent conditions with a nucleic acid having a nucleotide sequence complementary to the nucleotide sequence of SEQ ID NO: 532; or
(42, 43αB2) a protein comprising at least one amino acid sequence selected from the group consisting of SEQ ID NOs: 534-539;
(45α)
(45αA1) uncharacterized protein LOC100499771 or a variant thereof, which is defined below in any of (45αA1-a) to (45αA1-e):
(45αA1-a) a protein comprising an amino acid sequence with deletion, substitution, insertion or addition of one or several amino acids in SEQ ID NO: 541;
(45αA1-b) a protein comprising an amino acid sequence having at least 70% identity to the amino acid sequence of SEQ ID NO: 541;
(45αA1-c) a protein comprising an amino acid sequence encoded by a nucleotide sequence with deletion, substitution, insertion or addition of one or several nucleotides in SEQ ID NO: 540;
(45αA1-d) a protein comprising an amino acid sequence encoded by a nucleotide sequence having at least 70% identity to the nucleotide sequence of SEQ ID NO: 540; or
(45αA1-e) a protein comprising an amino acid sequence encoded by a nucleic acid that hybridizes under stringent conditions with a nucleic acid having a nucleotide sequence complementary to the nucleotide sequence of SEQ ID NO: 540;
(45αB1) a protein comprising at least one amino acid sequence selected from the group consisting of SEQ ID NOs: 542-545;
(45αA2) hypothetical protein GLYMA_09G192800 or a variant thereof, which is defined below in any of (45αA2-a) to (45αA2-e):
(45αA2-a) a protein comprising an amino acid sequence with deletion, substitution, insertion or addition of one or several amino acids in SEQ ID NO: 547;
(45αA2-b) a protein comprising an amino acid sequence having at least 70% identity to the amino acid sequence of SEQ ID NO: 547;
(45αA2-c) a protein comprising an amino acid sequence encoded by a nucleotide sequence with deletion, substitution, insertion or addition of one or several nucleotides in SEQ ID NO: 546;
(45αA2-d) a protein comprising an amino acid sequence encoded by a nucleotide sequence having at least 70% identity to the nucleotide sequence of SEQ ID NO: 546; or
(45αA2-e) a protein comprising an amino acid sequence encoded by a nucleic acid that hybridizes under stringent conditions with a nucleic acid having a nucleotide sequence complementary to the nucleotide sequence of SEQ ID NO: 546; or
(45αB2) a protein comprising at least one amino acid sequence selected from the group consisting of SEQ ID NOs: 548-551;
(46α)
(46αA) seed maturation protein PM22 or a variant thereof, which is defined below in any of (46αA-a) to (46αA-e):
(46αA-a) a protein comprising an amino acid sequence with deletion, substitution, insertion or addition of one or several amino acids in SEQ ID NO: 553;
(46αA-b) a protein comprising an amino acid sequence having at least 70% identity to the amino acid sequence of SEQ ID NO: 553;
(46αA-c) a protein comprising an amino acid sequence encoded by a nucleotide sequence with deletion, substitution, insertion or addition of one or several nucleotides in SEQ ID NO: 552;
(46αA-d) a protein comprising an amino acid sequence encoded by a nucleotide sequence having at least 70% identity to the nucleotide sequence of SEQ ID NO: 552; or
(46αA-e) a protein comprising an amino acid sequence encoded by a nucleic acid that hybridizes under stringent conditions with a nucleic acid having a nucleotide sequence complementary to the nucleotide sequence of SEQ ID NO: 552; or
(46αB) a protein comprising an amino acid sequence of SEQ ID NO: 554;
(47α)
(47αA1) eukaryotic translation initiation factor 5A-2 or a variant thereof, which is defined below in any of (47αA1-a) to (47αA1-e):
(47αA1-a) a protein comprising an amino acid sequence with deletion, substitution, insertion or addition of one or several amino acids in SEQ ID NO: 556;
(47αA1-b) a protein comprising an amino acid sequence having at least 70% identity to the amino acid sequence of SEQ ID NO: 556;
(47αA1-c) a protein comprising an amino acid sequence encoded by a nucleotide sequence with deletion, substitution, insertion or addition of one or several nucleotides in SEQ ID NO: 555;
(47αA1-d) a protein comprising an amino acid sequence encoded by a nucleotide sequence having at least 70% identity to the nucleotide sequence of SEQ ID NO: 555; or
(47αA1-e) a protein comprising an amino acid sequence encoded by a nucleic acid that hybridizes under stringent conditions with a nucleic acid having a nucleotide sequence complementary to the nucleotide sequence of SEQ ID NO: 555;
(47αB1) a protein comprising at least one amino acid sequence selected from the group consisting of SEQ ID NOs: 557-558;
(47αA2) uncharacterized protein LOC100305510 or a variant thereof, which is defined below in any of (47αA2-a) to (47αA2-e):
(47αA2-a) a protein comprising an amino acid sequence with deletion, substitution, insertion or addition of one or several amino acids in SEQ ID NO: 560;
(47αA2-b) a protein comprising an amino acid sequence having at least 70% identity to the amino acid sequence of SEQ ID NO: 560;
(47αA2-c) a protein comprising an amino acid sequence encoded by a nucleotide sequence with deletion, substitution, insertion or addition of one or several nucleotides in SEQ ID NO: 559;
(47αA2-d) a protein comprising an amino acid sequence encoded by a nucleotide sequence having at least 70% identity to the nucleotide sequence of SEQ ID NO: 559; or
(47αA2-e) a protein comprising an amino acid sequence encoded by a nucleic acid that hybridizes under stringent conditions with a nucleic acid having a nucleotide sequence complementary to the nucleotide sequence of SEQ ID NO: 559; or
(47αB2) a protein comprising at least one amino acid sequence selected from the group consisting of SEQ ID NOs: 561-563;
(48α)
(48αA) uncharacterized protein LOC100306570 or a variant thereof, which is defined below in any of (48αA-a) to (48αA-e):
(48αA-a) a protein comprising an amino acid sequence with deletion, substitution, insertion or addition of one or several amino acids in SEQ ID NO: 565;
(48αA-b) a protein comprising an amino acid sequence having at least 70% identity to the amino acid sequence of SEQ ID NO: 565;
(48αA-c) a protein comprising an amino acid sequence encoded by a nucleotide sequence with deletion, substitution, insertion or addition of one or several nucleotides in SEQ ID NO: 564;
(48αA-d) a protein comprising an amino acid sequence encoded by a nucleotide sequence having at least 70% identity to the nucleotide sequence of SEQ ID NO: 564; or
(48αA-e) a protein comprising an amino acid sequence encoded by a nucleic acid that hybridizes under stringent conditions with a nucleic acid having a nucleotide sequence complementary to the nucleotide sequence of SEQ ID NO: 564; or
(48αB) a protein comprising at least one amino acid sequence selected from the group consisting of SEQ ID NOs: 566-570;
(50α)
(50αA) uncharacterized protein LOC100813859 or a variant thereof, which is defined below in any of (50αA-a) to (50αA-e):
(50αA-a) a protein comprising an amino acid sequence with deletion, substitution, insertion or addition of one or several amino acids in SEQ ID NO: 572;
(50αA-b) a protein comprising an amino acid sequence having at least 70% identity to the amino acid sequence of SEQ ID NO: 572;
(50αA-c) a protein comprising an amino acid sequence encoded by a nucleotide sequence with deletion, substitution, insertion or addition of one or several nucleotides in SEQ ID NO: 571;
(50αA-d) a protein comprising an amino acid sequence encoded by a nucleotide sequence having at least 70% identity to the nucleotide sequence of SEQ ID NO: 571; or
(50αA-e) a protein comprising an amino acid sequence encoded by a nucleic acid that hybridizes under stringent conditions with a nucleic acid having a nucleotide sequence complementary to the nucleotide sequence of SEQ ID NO: 571; or
(50αB) a protein comprising at least one amino acid sequence selected from the group consisting of SEQ ID NOs: 573-576;
(51α)
(51αA) 14-3-3-like protein or a variant thereof, which is defined below in any of (51αA-a) to (51αA-e):
(51αA-a) a protein comprising an amino acid sequence with deletion, substitution, insertion or addition of one or several amino acids in SEQ ID NO: 578;
(51αA-b) a protein comprising an amino acid sequence having at least 70% identity to the amino acid sequence of SEQ ID NO: 578;
(51αA-c) a protein comprising an amino acid sequence encoded by a nucleotide sequence with deletion, substitution, insertion or addition of one or several nucleotides in SEQ ID NO: 577;
(51αA-d) a protein comprising an amino acid sequence encoded by a nucleotide sequence having at least 70% identity to the nucleotide sequence of SEQ ID NO: 577; or
(51αA-e) a protein comprising an amino acid sequence encoded by a nucleic acid that hybridizes under stringent conditions with a nucleic acid having a nucleotide sequence complementary to the nucleotide sequence of SEQ ID NO: 577; or
(51αB) a protein comprising at least one amino acid sequence selected from the group consisting of SEQ ID NOs: 579-582;
(52α)
(52αA) probable fructokinase-4 or a variant thereof, which is defined below in any of (52αA-a) to (52αA-e):
(52αA-a) a protein comprising an amino acid sequence with deletion, substitution, insertion or addition of one or several amino acids in SEQ ID NO: 584;
(52αA-b) a protein comprising an amino acid sequence having at least 70% identity to the amino acid sequence of SEQ ID NO: 584;
(52αA-c) a protein comprising an amino acid sequence encoded by a nucleotide sequence with deletion, substitution, insertion or addition of one or several nucleotides in SEQ ID NO: 583;
(52αA-d) a protein comprising an amino acid sequence encoded by a nucleotide sequence having at least 70% identity to the nucleotide sequence of SEQ ID NO: 583; or
(52αA-e) a protein comprising an amino acid sequence encoded by a nucleic acid that hybridizes under stringent conditions with a nucleic acid having a nucleotide sequence complementary to the nucleotide sequence of SEQ ID NO: 583; or
(52αB) a protein comprising at least one amino acid sequence selected from the group consisting of SEQ ID NOs: 585-588;
(53α)
(53αA1) triosephosphate isomerase isoform X1 or a variant thereof, which is defined below in any of (53αA1-a) to (53αA1-e):
(53αA1-a) a protein comprising an amino acid sequence with deletion, substitution, insertion or addition of one or several amino acids in SEQ ID NO: 590;
(53αA1-b) a protein comprising an amino acid sequence having at least 70% identity to the amino acid sequence of SEQ ID NO: 590;
(53αA1-c) a protein comprising an amino acid sequence encoded by a nucleotide sequence with deletion, substitution, insertion or addition of one or several nucleotides in SEQ ID NO: 589;
(53αA1-d) a protein comprising an amino acid sequence encoded by a nucleotide sequence having at least 70% identity to the nucleotide sequence of SEQ ID NO: 589; or
(53αA1-e) a protein comprising an amino acid sequence encoded by a nucleic acid that hybridizes under stringent conditions with a nucleic acid having a nucleotide sequence complementary to the nucleotide sequence of SEQ ID NO: 589;
(53αB1) a protein comprising at least one amino acid sequence selected from the group consisting of SEQ ID NOs: 591-597;
(53αA2) triosephosphate isomerase, cytosolic or a variant thereof, which is defined below in any of (53αA2-a) to (53αA2-e):
(53αA2-a) a protein comprising an amino acid sequence with deletion, substitution, insertion or addition of one or several amino acids in SEQ ID NO: 599;
(53αA2-b) a protein comprising an amino acid sequence having at least 70% identity to the amino acid sequence of SEQ ID NO: 599;
(53αA2-c) a protein comprising an amino acid sequence encoded by a nucleotide sequence with deletion, substitution, insertion or addition of one or several nucleotides in SEQ ID NO: 598;
(53αA2-d) a protein comprising an amino acid sequence encoded by a nucleotide sequence having at least 70% identity to the nucleotide sequence of SEQ ID NO: 598; or
(53αA2-e) a protein comprising an amino acid sequence encoded by a nucleic acid that hybridizes under stringent conditions with a nucleic acid having a nucleotide sequence complementary to the nucleotide sequence of SEQ ID NO: 598; or
(53αB2) a protein comprising at least one amino acid sequence selected from the group consisting of SEQ ID NOs: 600-604;
(54α)
(54αA1) superoxide dismutase [Fe], chloroplastic isoform X1 or a variant thereof, which is defined below in any of (54αA1-a) to (54αA1-e):
(54αA1-a) a protein comprising an amino acid sequence with deletion, substitution, insertion or addition of one or several amino acids in SEQ ID NO: 606;
(54αA1-b) a protein comprising an amino acid sequence having at least 70% identity to the amino acid sequence of SEQ ID NO: 606;
(53αA1-c) a protein comprising an amino acid sequence encoded by a nucleotide sequence with deletion, substitution, insertion or addition of one or several nucleotides in SEQ ID NO: 605;
(54αA1-d) a protein comprising an amino acid sequence encoded by a nucleotide sequence having at least 70% identity to the nucleotide sequence of SEQ ID NO: 605; or
(54αA1-e) a protein comprising an amino acid sequence encoded by a nucleic acid that hybridizes under stringent conditions with a nucleic acid having a nucleotide sequence complementary to the nucleotide sequence of SEQ ID NO: 605;
(54αB1) a protein comprising at least one amino acid sequence selected from the group consisting of SEQ ID NOs: 607-611;
(54αA2) iron-superoxide dismutase or a variant thereof, which is defined below in any of (54αA2-a) to (54αA2-e):
(54αA2-a) a protein comprising an amino acid sequence with deletion, substitution, insertion or addition of one or several amino acids in SEQ ID NO: 613;
(54αA2-b) a protein comprising an amino acid sequence having at least 70% identity to the amino acid sequence of SEQ ID NO: 613;
(54αA2-c) a protein comprising an amino acid sequence encoded by a nucleotide sequence with deletion, substitution, insertion or addition of one or several nucleotides in SEQ ID NO: 612;
(54αA2-d) a protein comprising an amino acid sequence encoded by a nucleotide sequence having at least 70% identity to the nucleotide sequence of SEQ ID NO: 612; or
(54αA2-e) a protein comprising an amino acid sequence encoded by a nucleic acid that hybridizes under stringent conditions with a nucleic acid having a nucleotide sequence complementary to the nucleotide sequence of SEQ ID NO: 612; or
(54αB2) a protein comprising at least one amino acid sequence selected from the group consisting of SEQ ID NOs: 614-616; or
(55α)
(55αA1) 51 kDa seed maturation protein precursor or a variant thereof, which is defined below in any of (55αA1-a) to (55αA1-e):
(55αA1-a) a protein comprising an amino acid sequence with deletion, substitution, insertion or addition of one or several amino acids in SEQ ID NO: 618;
(55αA1-b) a protein comprising an amino acid sequence having at least 70% identity to the amino acid sequence of SEQ ID NO: 618;
(55αA1-c) a protein comprising an amino acid sequence encoded by a nucleotide sequence with deletion, substitution, insertion or addition of one or several nucleotides in SEQ ID NO: 617;
(55αA1-d) a protein comprising an amino acid sequence encoded by a nucleotide sequence having at least 70% identity to the nucleotide sequence of SEQ ID NO: 617; or
(55αA1-e) a protein comprising an amino acid sequence encoded by a nucleic acid that hybridizes under stringent conditions with a nucleic acid having a nucleotide sequence complementary to the nucleotide sequence of SEQ ID NO: 617;
(55αB1) a protein comprising at least one amino acid sequence selected from the group consisting of SEQ ID NOs: 619-635;
(55αA2) Lea protein precursor or a variant thereof, which is defined below in any of (55αA2-a) to (55αA2-e):
(55αA2-a) a protein comprising an amino acid sequence with deletion, substitution, insertion or addition of one or several amino acids in SEQ ID NO: 637;
(55αA2-b) a protein comprising an amino acid sequence having at least 70% identity to the amino acid sequence of SEQ ID NO: 637;
(55αA2-c) a protein comprising an amino acid sequence encoded by a nucleotide sequence with deletion, substitution, insertion or addition of one or several nucleotides in SEQ ID NO: 636;
(55αA2-d) a protein comprising an amino acid sequence encoded by a nucleotide sequence having at least 70% identity to the nucleotide sequence of SEQ ID NO: 636; or
(55αA2-e) a protein comprising an amino acid sequence encoded by a nucleic acid that hybridizes under stringent conditions with a nucleic acid having a nucleotide sequence complementary to the nucleotide sequence of SEQ ID NO: 636; or
(55αB2) a protein comprising at least one amino acid sequence selected from the group consisting of SEQ ID NOs: 638-649.

3. A composition for diagnosing an allergy to a soybean, the composition comprising, as an antigen, at least one of proteins as defined in any of (1) to (8) of claim 1 or at least one of proteins as defined in any of (1α) to (55α) of claim 2.

4. A method for providing an indicator for diagnosing an allergy to a soybean in a subject, the method comprising the steps of:
(i) contacting a sample obtained from the subject with an antigen, wherein the sample is a solution comprising an Ig antibody;
(ii) detecting binding between the IgE antibody present in the sample obtained from the subject and the antigen; and
(iii) when the binding between the IgE antibody in the subject and the antigen is detected, an indicator of the fact that the subject is allergic to a soybean is provided;
wherein the antigen is at least one of proteins as defined in any of (1) to (8) of claim 1 or at least one of proteins as defined in any of (1α) to (55α) of claim 2.

5. A pharmaceutical composition comprising at least one of proteins as defined in any of (1) to (8) of claim 1 or at least one of proteins as defined in any of (1α) to (55α) of claim 2.

6. The pharmaceutical composition according to claim 5, wherein the pharmaceutical composition is intended for the treatment of an allergy to a soybean.

7. A soybean or a processed product of soybean in which an antigen is eliminated or reduced, wherein the antigen is at least one of proteins as defined in any of (1) to (8) of claim 1 or at least one of proteins as defined in any of (1α) to (55α) of claim 2.

8. A tester for determining the presence or absence of a soybean antigen in an object of interest, the tester comprising an antibody that binds to at least one of proteins as defined in any of (1) to (8) of claim 1 or at least one of proteins as defined in any of (1α) to (55α) of claim 2.

9. A soybean-derived antigen, which is at least one of proteins as defined in any of (1) to (8) of claim 1 or at least one of proteins as defined in any of (1α) to (55α) of claim 2 and is causative of an allergy to a soybean.


SEQUENCE LISTING

<110>  FUJITA ACADEMY
       HOYU CO., LTD.

<120>  Antigen of Soy Allergy

<130>  FA1621-17011

<150>  JP2016-041547
<151>  2016-03-03

<160>  649

<170>  PatentIn version 3.5

<210>  1
<211>  249
<212>  DNA
<213>  Glycine max

<220>
<221>  CDS
<222>  (1)..(249)

<400>  1
atg tgt att ctg agc ttc ctc aaa agt gat cag tca tca agt tat gat      48
Met Cys Ile Leu Ser Phe Leu Lys Ser Asp Gln Ser Ser Ser Tyr Asp
1               5                   10                  15

gat gat gag tat tca aaa cca tgc tgt gat ctc tgc atg tgc aca cgc      96
Asp Asp Glu Tyr Ser Lys Pro Cys Cys Asp Leu Cys Met Cys Thr Arg
            20                  25                  30

tca atg cct cct caa tgc agc tgt gaa gat att agg ctg aat tca tgc      144
Ser Met Pro Pro Gln Cys Ser Cys Glu Asp Ile Arg Leu Asn Ser Cys
        35                  40                  45

cac tca gat tgt aag agc tgt atg tgc aca cgc tca cag cca gga cag      192
His Ser Asp Cys Lys Ser Cys Met Cys Thr Arg Ser Gln Pro Gly Gln
    50                  55                  60

tgt cgt tgt ctt gac acc aac gac ttc tgc tac aaa cct tgc aag tcc      240
Cys Arg Cys Leu Asp Thr Asn Asp Phe Cys Tyr Lys Pro Cys Lys Ser
65                  70                  75                  80

aga gat gac                                                          249
Arg Asp Asp

<210>  2
<211>  83
<212>  PRT
<213>  Glycine max

<400>  2

Met Cys Ile Leu Ser Phe Leu Lys Ser Asp Gln Ser Ser Ser Tyr Asp
1               5                   10                  15

Asp Asp Glu Tyr Ser Lys Pro Cys Cys Asp Leu Cys Met Cys Thr Arg
            20                  25                  30

Ser Met Pro Pro Gln Cys Ser Cys Glu Asp Ile Arg Leu Asn Ser Cys
        35                  40                  45

His Ser Asp Cys Lys Ser Cys Met Cys Thr Arg Ser Gln Pro Gly Gln
    50                  55                  60

Cys Arg Cys Leu Asp Thr Asn Asp Phe Cys Tyr Lys Pro Cys Lys Ser
65                  70                  75                  80

Arg Asp Asp

<210> 3
<211> 1425
<212> DNA
<213> Glycine max

<220>
<221> CDS
<222> (1)..(1425)

<400> 3
atg tca cca caa aca gag act aaa gca agt gtt ggg ttc aaa gct ggt 48
Met Ser Pro Gln Thr Glu Thr Lys Ala Ser Val Gly Phe Lys Ala Gly
1               5                   10                  15

gtt aaa gat tat aaa ttg act tat tat act cct gac tat gaa acc aaa 96
Val Lys Asp Tyr Lys Leu Thr Tyr Tyr Thr Pro Asp Tyr Glu Thr Lys
            20                  25                  30

gat act gat atc ttg gca gca ttc cga gta act cct caa cca gga gtt 144
Asp Thr Asp Ile Leu Ala Ala Phe Arg Val Thr Pro Gln Pro Gly Val
        35                  40                  45

ccg cct gaa gaa gca ggt gcc gcg gta gcc gcc gaa tct tct act ggt 192
Pro Pro Glu Glu Ala Gly Ala Ala Val Ala Ala Glu Ser Ser Thr Gly
    50                  55                  60

aca tgg aca act gtg tgg acc gat ggg ctt acc agt ctt gat cgt tac 240
Thr Trp Thr Thr Val Trp Thr Asp Gly Leu Thr Ser Leu Asp Arg Tyr
65                  70                  75                  80

aaa ggg cga tgc tac ggc ctt gaa cct gtt gct ggg gaa gaa aat caa 288
Lys Gly Arg Cys Tyr Gly Leu Glu Pro Val Ala Gly Glu Glu Asn Gln
                85                  90                  95

tat att gct tat gta gct tat ccc tta gac ctt ttt gaa gaa ggt tct 336
Tyr Ile Ala Tyr Val Ala Tyr Pro Leu Asp Leu Phe Glu Glu Gly Ser
            100                 105                 110

gtt act aac atg ttt act tcc att gtc ggt aat gta ttt ggg ttc aag 384
Val Thr Asn Met Phe Thr Ser Ile Val Gly Asn Val Phe Gly Phe Lys
        115                 120                 125

gcc ctg cgt gct cta cgt ctg gag gat ttg cga atc cct act gct tat 432
Ala Leu Arg Ala Leu Arg Leu Glu Asp Leu Arg Ile Pro Thr Ala Tyr
    130                 135                 140

att aaa act ttc caa ggt ccg cct cat ggc atc caa gtt gag aga gat 480
Ile Lys Thr Phe Gln Gly Pro Pro His Gly Ile Gln Val Glu Arg Asp
145                 150                 155                 160

aaa ttg aac aag tat ggt cgt ccc cta tta gga tgt act att aaa cct 528
Lys Leu Asn Lys Tyr Gly Arg Pro Leu Leu Gly Cys Thr Ile Lys Pro
                165                 170                 175

aaa ttg ggg tta tcc gct aag aat tat ggt aga gct gtt tat gaa tgt 576
Lys Leu Gly Leu Ser Ala Lys Asn Tyr Gly Arg Ala Val Tyr Glu Cys
            180                 185                 190

ctt cgt ggg gga ctt gat ttt acc aaa gat gat gaa aat gtg aat tcc 624
Leu Arg Gly Gly Leu Asp Phe Thr Lys Asp Asp Glu Asn Val Asn Ser
        195                 200                 205

caa cca ttt atg cgt tgg aga gac cgt ttc tta ttt tgt gcc gaa gcc 672
Gln Pro Phe Met Arg Trp Arg Asp Arg Phe Leu Phe Cys Ala Glu Ala
    210                 215                 220

att ttt aaa tca cag gct gaa aca ggt gaa atc aaa ggg cat tac ttg 720
Ile Phe Lys Ser Gln Ala Glu Thr Gly Glu Ile Lys Gly His Tyr Leu
225                 230                 235                 240

aat gca act gcg ggt aca tgc gaa gaa atg atg aaa aga gct gta ttt 768
Asn Ala Thr Ala Gly Thr Cys Glu Glu Met Met Lys Arg Ala Val Phe
                245                 250                 255

gcc aga gaa tta ggc gtt cct atc gta atg cat gat tat tta acg ggg 816
Ala Arg Glu Leu Gly Val Pro Ile Val Met His Asp Tyr Leu Thr Gly
            260                 265                 270

gga ttc act gca aat act agc ttg gct cat tat tgc cga gat aat ggt 864
Gly Phe Thr Ala Asn Thr Ser Leu Ala His Tyr Cys Arg Asp Asn Gly
        275                 280                 285

cta ctt ctt cat ata cac cgt gca atg cat gca gtt atc gac aga caa 912
Leu Leu Leu His Ile His Arg Ala Met His Ala Val Ile Asp Arg Gln
    290                 295                 300

aag aat cat ggt atg cac ttt cgt gta cta gct aaa gca tta cgt ttg 960
Lys Asn His Gly Met His Phe Arg Val Leu Ala Lys Ala Leu Arg Leu
305                 310                 315                 320

tct ggt gga gat cat gtt cac gcc ggt acc gta gta ggt aaa ctt gaa 1008
Ser Gly Gly Asp His Val His Ala Gly Thr Val Val Gly Lys Leu Glu
                325                 330                 335

ggg gaa aga gaa atc act tta ggt ttt gtt gat tta cta cgt gat gat 1056
Gly Glu Arg Glu Ile Thr Leu Gly Phe Val Asp Leu Leu Arg Asp Asp
            340                 345                 350

ttt gtt gaa aaa gat cga agt cgc ggt att tat ttc act cag gat tgg 1104
Phe Val Glu Lys Asp Arg Ser Arg Gly Ile Tyr Phe Thr Gln Asp Trp
        355                 360                 365

gtt tct cta cca ggt gtt ttg cct gta gct tcg gga ggt att cac gtt 1152
Val Ser Leu Pro Gly Val Leu Pro Val Ala Ser Gly Gly Ile His Val
    370                 375                 380

tgg cat atg cct gct ctg acc gag atc ttt ggg gat gat tct gta ctc     1200
Trp His Met Pro Ala Leu Thr Glu Ile Phe Gly Asp Asp Ser Val Leu
385                 390                 395                 400

caa ttt ggc gga gga act tta gga cac cct tgg gga aat gca cca ggt     1248
Gln Phe Gly Gly Gly Thr Leu Gly His Pro Trp Gly Asn Ala Pro Gly
                405                 410                 415

gct gta gct aat cga gta gct ctt gaa gca tgt gtg cag gct cga aat     1296
Ala Val Ala Asn Arg Val Ala Leu Glu Ala Cys Val Gln Ala Arg Asn
            420                 425                 430

gaa gga cgc gat ctt gct cgt gaa ggt aat gaa att atc cgt gag gct     1344
Glu Gly Arg Asp Leu Ala Arg Glu Gly Asn Glu Ile Ile Arg Glu Ala
        435                 440                 445

agc aaa tgg agt cct gaa tta gct gct gct tgt gaa gta tgg aag gag     1392
Ser Lys Trp Ser Pro Glu Leu Ala Ala Ala Cys Glu Val Trp Lys Glu
    450                 455                 460

atc aaa ttt gaa ttc gaa gca atg gat act ttg                         1425
Ile Lys Phe Glu Phe Glu Ala Met Asp Thr Leu
465                 470                 475

<210>  4
<211>  475
<212>  PRT
<213>  Glycine max

<400>  4

Met Ser Pro Gln Thr Glu Thr Lys Ala Ser Val Gly Phe Lys Ala Gly
1               5                   10                  15

Val Lys Asp Tyr Lys Leu Thr Tyr Tyr Thr Pro Asp Tyr Glu Thr Lys
            20                  25                  30

Asp Thr Asp Ile Leu Ala Ala Phe Arg Val Thr Pro Gln Pro Gly Val
        35                  40                  45

Pro Pro Glu Glu Ala Gly Ala Ala Val Ala Ala Glu Ser Ser Thr Gly
    50                  55                  60

Thr Trp Thr Thr Val Trp Thr Asp Gly Leu Thr Ser Leu Asp Arg Tyr
65                  70                  75                  80

Lys Gly Arg Cys Tyr Gly Leu Glu Pro Val Ala Gly Glu Glu Asn Gln
                85                  90                  95

Tyr Ile Ala Tyr Val Ala Tyr Pro Leu Asp Leu Phe Glu Glu Gly Ser
            100                 105                 110

Val Thr Asn Met Phe Thr Ser Ile Val Gly Asn Val Phe Gly Phe Lys
        115                 120                 125

Ala Leu Arg Ala Leu Arg Leu Glu Asp Leu Arg Ile Pro Thr Ala Tyr
    130                 135                 140

Ile Lys Thr Phe Gln Gly Pro Pro His Gly Ile Gln Val Glu Arg Asp
145                 150                 155                 160

Lys Leu Asn Lys Tyr Gly Arg Pro Leu Leu Gly Cys Thr Ile Lys Pro
                165                 170                 175

Lys Leu Gly Leu Ser Ala Lys Asn Tyr Gly Arg Ala Val Tyr Glu Cys
            180                 185                 190

Leu Arg Gly Gly Leu Asp Phe Thr Lys Asp Asp Glu Asn Val Asn Ser
        195                 200                 205

Gln Pro Phe Met Arg Trp Arg Asp Arg Phe Leu Phe Cys Ala Glu Ala
    210                 215                 220

Ile Phe Lys Ser Gln Ala Glu Thr Gly Glu Ile Lys Gly His Tyr Leu
225                 230                 235                 240

Asn Ala Thr Ala Gly Thr Cys Glu Glu Met Met Lys Arg Ala Val Phe
                245                 250                 255

Ala Arg Glu Leu Gly Val Pro Ile Val Met His Asp Tyr Leu Thr Gly
            260                 265                 270

Gly Phe Thr Ala Asn Thr Ser Leu Ala His Tyr Cys Arg Asp Asn Gly
        275                 280                 285

Leu Leu Leu His Ile His Arg Ala Met His Ala Val Ile Asp Arg Gln
    290                 295                 300

Lys Asn His Gly Met His Phe Arg Val Leu Ala Lys Ala Leu Arg Leu
305                 310                 315                 320

Ser Gly Gly Asp His Val His Ala Gly Thr Val Val Gly Lys Leu Glu
                325                 330                 335

Gly Glu Arg Glu Ile Thr Leu Gly Phe Val Asp Leu Leu Arg Asp Asp
            340                 345                 350

Phe Val Glu Lys Asp Arg Ser Arg Gly Ile Tyr Phe Thr Gln Asp Trp
        355                 360                 365

Val Ser Leu Pro Gly Val Leu Pro Val Ala Ser Gly Gly Ile His Val

370                 375                 380

Trp His Met Pro Ala Leu Thr Glu Ile Phe Gly Asp Asp Ser Val Leu
385                 390                 395                 400

Gln Phe Gly Gly Gly Thr Leu Gly His Pro Trp Gly Asn Ala Pro Gly
                405                 410                 415

Ala Val Ala Asn Arg Val Ala Leu Glu Ala Cys Val Gln Ala Arg Asn
            420                 425                 430

Glu Gly Arg Asp Leu Ala Arg Glu Gly Asn Glu Ile Ile Arg Glu Ala
        435                 440                 445

Ser Lys Trp Ser Pro Glu Leu Ala Ala Ala Cys Glu Val Trp Lys Glu
    450                 455                 460

Ile Lys Phe Glu Phe Glu Ala Met Asp Thr Leu
465                 470                 475

<210>  5
<211>  1026
<212>  DNA
<213>  Glycine max

<220>
<221>  CDS
<222>  (1)..(1026)

<400>  5
atg cca aac gac agc gtt ctg agt ctt ttc ttc ttc gtt acc ctc ttc      48
Met Pro Asn Asp Ser Val Leu Ser Leu Phe Phe Phe Val Thr Leu Phe
1               5                   10                  15

aca tgc ctt ctc tct gct act tcc cac gat gac cat att ttc cta ccg      96
Thr Cys Leu Leu Ser Ala Thr Ser His Asp Asp His Ile Phe Leu Pro
            20                  25                  30

agt caa ctc cat gac gat gac tca gtg agt tgc act gca acc gac cca      144
Ser Gln Leu His Asp Asp Asp Ser Val Ser Cys Thr Ala Thr Asp Pro
        35                  40                  45

agc ctt aac tac aag ccg gtg atc gga atc ctg acc cac ccc ggc gac      192
Ser Leu Asn Tyr Lys Pro Val Ile Gly Ile Leu Thr His Pro Gly Asp
    50                  55                  60

ggt gcc tct ggc cgg ctc agc aac gcc acc ggg gtt tct tac atc gcc      240
Gly Ala Ser Gly Arg Leu Ser Asn Ala Thr Gly Val Ser Tyr Ile Ala
65                  70                  75                  80

gcc tcc tac gtt aaa ttc gtg gag tcc ggt ggt gct aga gtt att ccc      288
Ala Ser Tyr Val Lys Phe Val Glu Ser Gly Gly Ala Arg Val Ile Pro
                85                  90                  95

ctt atc tac aat gag tca cct gag aac ctt aac aag aag ctc gat ttg      336

Leu Ile Tyr Asn Glu Ser Pro Glu Asn Leu Asn Lys Lys Leu Asp Leu
            100                 105                 110

gtc aat gga gtg ctc ttc act ggt ggg tgg gct gtt tct ggt cca tat     384
Val Asn Gly Val Leu Phe Thr Gly Gly Trp Ala Val Ser Gly Pro Tyr
        115                 120                 125

ttg gat act ctt gga aac att ttt aag aaa gct tta gag aga aat gat     432
Leu Asp Thr Leu Gly Asn Ile Phe Lys Lys Ala Leu Glu Arg Asn Asp
    130                 135                 140

gct gga gac cat ttc cca gta att gcc ttc aac tta ggt gga aac ctc     480
Ala Gly Asp His Phe Pro Val Ile Ala Phe Asn Leu Gly Gly Asn Leu
145                 150                 155                 160

gta ata agg atc gta agc gag caa act gat att ctt gaa cca ttt act     528
Val Ile Arg Ile Val Ser Glu Gln Thr Asp Ile Leu Glu Pro Phe Thr
                165                 170                 175

gcg tcg agt ttg cca agt tct ctt gta ttg tgg aac gaa gca aat gct     576
Ala Ser Ser Leu Pro Ser Ser Leu Val Leu Trp Asn Glu Ala Asn Ala
            180                 185                 190

aaa gga agt cta ttt caa aga ttt cct tca gat ttg ttg aca cag ctg     624
Lys Gly Ser Leu Phe Gln Arg Phe Pro Ser Asp Leu Leu Thr Gln Leu
        195                 200                 205

aag aca gat tgt ctc gtc ctg cac aat cat cgt tat gcc ata tcc cca     672
Lys Thr Asp Cys Leu Val Leu His Asn His Arg Tyr Ala Ile Ser Pro
    210                 215                 220

cgc aag ctt caa tat aat acc aag tta tct gac ttt ttt gag att ctg     720
Arg Lys Leu Gln Tyr Asn Thr Lys Leu Ser Asp Phe Phe Glu Ile Leu
225                 230                 235                 240

gca aca tct ggg gat aga gat gga aag act ttc gtt tcc aca gca cgt     768
Ala Thr Ser Gly Asp Arg Asp Gly Lys Thr Phe Val Ser Thr Ala Arg
                245                 250                 255

ggt agg aaa tat ccc gtg act gtc aac cta tgg caa cca gag aaa aat     816
Gly Arg Lys Tyr Pro Val Thr Val Asn Leu Trp Gln Pro Glu Lys Asn
            260                 265                 270

gcg ttt gag tgg gct aca tct ttg aaa gcc cct cac aca gag gat gca     864
Ala Phe Glu Trp Ala Thr Ser Leu Lys Ala Pro His Thr Glu Asp Ala
        275                 280                 285

att cgg gtt act cag tcc aca gca aac ttt ttc ata agt gaa gct agg     912
Ile Arg Val Thr Gln Ser Thr Ala Asn Phe Phe Ile Ser Glu Ala Arg
    290                 295                 300

aaa tcc aca aac aca cct gat gct caa aaa gtg cga gat agt ctc atc     960
Lys Ser Thr Asn Thr Pro Asp Ala Gln Lys Val Arg Asp Ser Leu Ile
305                 310                 315                 320

tac aat tac aaa cct act ttt ggt gga acg gct ggg aaa gga tat gat     1008
Tyr Asn Tyr Lys Pro Thr Phe Gly Gly Thr Ala Gly Lys Gly Tyr Asp
                325                 330                 335

caa gtg tat ctc ttt gaa                                             1026
Gln Val Tyr Leu Phe Glu
            340

<210>  6
<211>  342
<212>  PRT
<213>  Glycine max

<400>  6

Met Pro Asn Asp Ser Val Leu Ser Leu Phe Phe Phe Val Thr Leu Phe
1               5                   10                  15

Thr Cys Leu Leu Ser Ala Thr Ser His Asp Asp His Ile Phe Leu Pro
            20                  25                  30

Ser Gln Leu His Asp Asp Asp Ser Val Ser Cys Thr Ala Thr Asp Pro
        35                  40                  45

Ser Leu Asn Tyr Lys Pro Val Ile Gly Ile Leu Thr His Pro Gly Asp
    50                  55                  60

Gly Ala Ser Gly Arg Leu Ser Asn Ala Thr Gly Val Ser Tyr Ile Ala
65                  70                  75                  80

Ala Ser Tyr Val Lys Phe Val Glu Ser Gly Gly Ala Arg Val Ile Pro
                85                  90                  95

Leu Ile Tyr Asn Glu Ser Pro Glu Asn Leu Asn Lys Lys Leu Asp Leu
            100                 105                 110

Val Asn Gly Val Leu Phe Thr Gly Gly Trp Ala Val Ser Gly Pro Tyr
        115                 120                 125

Leu Asp Thr Leu Gly Asn Ile Phe Lys Lys Ala Leu Glu Arg Asn Asp
    130                 135                 140

Ala Gly Asp His Phe Pro Val Ile Ala Phe Asn Leu Gly Gly Asn Leu
145                 150                 155                 160

Val Ile Arg Ile Val Ser Glu Gln Thr Asp Ile Leu Glu Pro Phe Thr
                165                 170                 175

Ala Ser Ser Leu Pro Ser Ser Leu Val Leu Trp Asn Glu Ala Asn Ala
            180                 185                 190

Lys Gly Ser Leu Phe Gln Arg Phe Pro Ser Asp Leu Leu Thr Gln Leu
        195                 200                 205

Lys Thr Asp Cys Leu Val Leu His Asn His Arg Tyr Ala Ile Ser Pro
    210                 215                 220

Arg Lys Leu Gln Tyr Asn Thr Lys Leu Ser Asp Phe Phe Glu Ile Leu
225                 230                 235                 240

Ala Thr Ser Gly Asp Arg Asp Gly Lys Thr Phe Val Ser Thr Ala Arg
                245                 250                 255

Gly Arg Lys Tyr Pro Val Thr Val Asn Leu Trp Gln Pro Glu Lys Asn
            260                 265                 270

Ala Phe Glu Trp Ala Thr Ser Leu Lys Ala Pro His Thr Glu Asp Ala
        275                 280                 285

Ile Arg Val Thr Gln Ser Thr Ala Asn Phe Phe Ile Ser Glu Ala Arg
    290                 295                 300

Lys Ser Thr Asn Thr Pro Asp Ala Gln Lys Val Arg Asp Ser Leu Ile
305                 310                 315                 320

Tyr Asn Tyr Lys Pro Thr Phe Gly Gly Thr Ala Gly Lys Gly Tyr Asp
                325                 330                 335

Gln Val Tyr Leu Phe Glu
            340

<210>  7
<211>  975
<212>  DNA
<213>  Glycine max

<220>
<221>  CDS
<222>  (1)..(975)

<400>  7
atg gcg gaa gga ctc gtc ctg aag gga acc atg cgc gcc cac acc gac      48
Met Ala Glu Gly Leu Val Leu Lys Gly Thr Met Arg Ala His Thr Asp
1               5                   10                  15

gtc gtg acg gcg att gcc act ccc atc gac aac tcc gac atg atc gtg      96
Val Val Thr Ala Ile Ala Thr Pro Ile Asp Asn Ser Asp Met Ile Val
            20                  25                  30

acg gcg tcg cgc gac aga tcc atc att ctc tgg cac ctc acc aag gag      144
Thr Ala Ser Arg Asp Arg Ser Ile Ile Leu Trp His Leu Thr Lys Glu
        35                  40                  45

gac aag acc tac ggt gtc ccc cgc cgc cgc ctc acc gga cat tct cac      192
Asp Lys Thr Tyr Gly Val Pro Arg Arg Arg Leu Thr Gly His Ser His
    50                  55                  60

ttc gtc cag gac gtc gtc ctc tca tcc gac ggt cag ttc gcc ctc tcc      240
Phe Val Gln Asp Val Val Leu Ser Ser Asp Gly Gln Phe Ala Leu Ser
65                  70                  75                  80

ggc tcc tgg gac ggc gag ctc cgc ctc tgg gac ctc gcg gct ggc acc 288
Gly Ser Trp Asp Gly Glu Leu Arg Leu Trp Asp Leu Ala Ala Gly Thr
                85                  90                  95

tct gcc cgc cgc ttc gtt ggc cac acc aag gac gtg ctc tcc gtg gcg 336
Ser Ala Arg Arg Phe Val Gly His Thr Lys Asp Val Leu Ser Val Ala
            100                 105                 110

ttc tcc atc gac aac cgt cag atc gtg tcg gcc tct cgt gac cgc acg 384
Phe Ser Ile Asp Asn Arg Gln Ile Val Ser Ala Ser Arg Asp Arg Thr
        115                 120                 125

atc aag ctg tgg aac acc ctg ggt gag tgc aag tac aca atc caa gat 432
Ile Lys Leu Trp Asn Thr Leu Gly Glu Cys Lys Tyr Thr Ile Gln Asp
    130                 135                 140

ggc gat gcg cat tcg gat tgg gta agt tgc gtc cgt ttc agc cct agc 480
Gly Asp Ala His Ser Asp Trp Val Ser Cys Val Arg Phe Ser Pro Ser
145                 150                 155                 160

act ctt cag cca acc att gtt tct gct tca tgg gac agg acc gtt aag 528
Thr Leu Gln Pro Thr Ile Val Ser Ala Ser Trp Asp Arg Thr Val Lys
                165                 170                 175

gtt tgg aac ctg acc aac tgc aag ctg agg aac acc ctt gct gga cac 576
Val Trp Asn Leu Thr Asn Cys Lys Leu Arg Asn Thr Leu Ala Gly His
            180                 185                 190

aat ggg tat gtg aat act gtt gct gtt tcc cct gat ggc tct ctc tgt 624
Asn Gly Tyr Val Asn Thr Val Ala Val Ser Pro Asp Gly Ser Leu Cys
        195                 200                 205

gcc agt ggt ggc aaa gat ggg gtt att ctt ctg tgg gat ttg gct gag 672
Ala Ser Gly Gly Lys Asp Gly Val Ile Leu Leu Trp Asp Leu Ala Glu
    210                 215                 220

ggt aag cgt ctt tac tct ctc gat gct ggc tca atc atc cat gca ctc 720
Gly Lys Arg Leu Tyr Ser Leu Asp Ala Gly Ser Ile Ile His Ala Leu
225                 230                 235                 240

tgc ttc agc ccc agc agg tac tgg ctc tgc gcc gcc acc gag cag agc 768
Cys Phe Ser Pro Ser Arg Tyr Trp Leu Cys Ala Ala Thr Glu Gln Ser
                245                 250                 255

atc aag atc tgg gat ttg gag agc aag agt atc gtt gag gat ttg aag 816
Ile Lys Ile Trp Asp Leu Glu Ser Lys Ser Ile Val Glu Asp Leu Lys
            260                 265                 270

gtt gac ctc aag act gag gct gat gcc acc tcc ggt ggt ggt aac gcc 864
Val Asp Leu Lys Thr Glu Ala Asp Ala Thr Ser Gly Gly Gly Asn Ala
        275                 280                 285

aac aag aag aag gtt att tat tgc aca agt ttg aac tgg agt gcg gat 912
Asn Lys Lys Lys Val Ile Tyr Cys Thr Ser Leu Asn Trp Ser Ala Asp
    290                 295                 300

gga agc act ttg ttt agt ggc tat acc gat ggt gtg gcc aga gtt tgg 960
Gly Ser Thr Leu Phe Ser Gly Tyr Thr Asp Gly Val Ala Arg Val Trp
305                 310                 315                 320

gct att gga cgt tat 975
Ala Ile Gly Arg Tyr

                325

<210>  8
<211>  325
<212>  PRT
<213>  Glycine max

<400>  8

Met Ala Glu Gly Leu Val Leu Lys Gly Thr Met Arg Ala His Thr Asp
1               5                   10                  15

Val Val Thr Ala Ile Ala Thr Pro Ile Asp Asn Ser Asp Met Ile Val
            20                  25                  30

Thr Ala Ser Arg Asp Arg Ser Ile Ile Leu Trp His Leu Thr Lys Glu
        35                  40                  45

Asp Lys Thr Tyr Gly Val Pro Arg Arg Arg Leu Thr Gly His Ser His
    50                  55                  60

Phe Val Gln Asp Val Val Leu Ser Ser Asp Gly Gln Phe Ala Leu Ser
65                  70                  75                  80

Gly Ser Trp Asp Gly Glu Leu Arg Leu Trp Asp Leu Ala Ala Gly Thr
                85                  90                  95

Ser Ala Arg Arg Phe Val Gly His Thr Lys Asp Val Leu Ser Val Ala
            100                 105                 110

Phe Ser Ile Asp Asn Arg Gln Ile Val Ser Ala Ser Arg Asp Arg Thr
        115                 120                 125

Ile Lys Leu Trp Asn Thr Leu Gly Glu Cys Lys Tyr Thr Ile Gln Asp
    130                 135                 140

Gly Asp Ala His Ser Asp Trp Val Ser Cys Val Arg Phe Ser Pro Ser
145                 150                 155                 160

Thr Leu Gln Pro Thr Ile Val Ser Ala Ser Trp Asp Arg Thr Val Lys
                165                 170                 175

Val Trp Asn Leu Thr Asn Cys Lys Leu Arg Asn Thr Leu Ala Gly His
            180                 185                 190

Asn Gly Tyr Val Asn Thr Val Ala Val Ser Pro Asp Gly Ser Leu Cys
        195                 200                 205

Ala Ser Gly Gly Lys Asp Gly Val Ile Leu Leu Trp Asp Leu Ala Glu

210                 215                 220

Gly Lys Arg Leu Tyr Ser Leu Asp Ala Gly Ser Ile Ile His Ala Leu
225                 230                 235                 240

Cys Phe Ser Pro Ser Arg Tyr Trp Leu Cys Ala Ala Thr Glu Gln Ser
                245                 250                 255

Ile Lys Ile Trp Asp Leu Glu Ser Lys Ser Ile Val Glu Asp Leu Lys
            260                 265                 270

Val Asp Leu Lys Thr Glu Ala Asp Ala Thr Ser Gly Gly Gly Asn Ala
        275                 280                 285

Asn Lys Lys Lys Val Ile Tyr Cys Thr Ser Leu Asn Trp Ser Ala Asp
    290                 295                 300

Gly Ser Thr Leu Phe Ser Gly Tyr Thr Asp Gly Val Ala Arg Val Trp
305                 310                 315                 320

Ala Ile Gly Arg Tyr
                325

<210>  9
<211>  303
<212>  DNA
<213>  Glycine max

<220>
<221>  CDS
<222>  (1)..(303)

<400>  9
atg gca tct cat caa caa aac aag caa gag ctt gat gaa agg gca agg      48
Met Ala Ser His Gln Gln Asn Lys Gln Glu Leu Asp Glu Arg Ala Arg
1               5                   10                  15

caa gga gag act gtt gtt ccg ggt ggc act ggt ggc aag agc ctt gag      96
Gln Gly Glu Thr Val Val Pro Gly Gly Thr Gly Gly Lys Ser Leu Glu
            20                  25                  30

gct cag caa cat ctt gct gaa gga agg agc agg gga ggg aaa aca agg      144
Ala Gln Gln His Leu Ala Glu Gly Arg Ser Arg Gly Gly Lys Thr Arg
        35                  40                  45

aag gag cag ctg ggg aca gaa ggg tac cat gaa atg gga cgc aaa ggt      192
Lys Glu Gln Leu Gly Thr Glu Gly Tyr His Glu Met Gly Arg Lys Gly
    50                  55                  60

ggg ttg agc act atg gac aag tca gga gaa gaa cgt gct caa gag gaa      240
Gly Leu Ser Thr Met Asp Lys Ser Gly Glu Glu Arg Ala Gln Glu Glu
65                  70                  75                  80

gcc att gac atc gat gag tcc aag ttc agg act ggt aat aac caa gac      288

Ala Ile Asp Ile Asp Glu Ser Lys Phe Arg Thr Gly Asn Asn Gln Asp
                85                  90                  95

aag aac cag aac aag                                                303
Lys Asn Gln Asn Lys
            100

<210>  10
<211>  101
<212>  PRT
<213>  Glycine max

<400>  10

Met Ala Ser His Gln Gln Asn Lys Gln Glu Leu Asp Glu Arg Ala Arg
1               5                   10                  15

Gln Gly Glu Thr Val Val Pro Gly Gly Thr Gly Gly Lys Ser Leu Glu
            20                  25                  30

Ala Gln Gln His Leu Ala Glu Gly Arg Ser Arg Gly Gly Lys Thr Arg
        35                  40                  45

Lys Glu Gln Leu Gly Thr Glu Gly Tyr His Glu Met Gly Arg Lys Gly
    50                  55                  60

Gly Leu Ser Thr Met Asp Lys Ser Gly Glu Glu Arg Ala Gln Glu Glu
65                  70                  75                  80

Ala Ile Asp Ile Asp Glu Ser Lys Phe Arg Thr Gly Asn Asn Gln Asp
                85                  90                  95

Lys Asn Gln Asn Lys
            100

<210>  11
<211>  1929
<212>  DNA
<213>  Glycine max

<220>
<221>  CDS
<222>  (1)..(1929)

<400>  11
atg gcg tct gaa caa ttg gct cgc aga gag aac acc acc acc gag aag      48
Met Ala Ser Glu Gln Leu Ala Arg Arg Glu Asn Thr Thr Thr Glu Lys
1               5                   10                  15

gag att cac gtt gag aaa cac agg gtt ccc aag atg gcc acc cac ttc      96
Glu Ile His Val Glu Lys His Arg Val Pro Lys Met Ala Thr His Phe
            20                  25                  30

gag cac ctt gcg gag caa gct aaa gaa tct gac atc acc gct ggg aag     144

Glu His Leu Ala Glu Gln Ala Lys Glu Ser Asp Ile Thr Ala Gly Lys
        35                  40                  45

gat act ccg cag ggt agc ata gaa gcc ttg caa gct gga gag agg gtc      192
Asp Thr Pro Gln Gly Ser Ile Glu Ala Leu Gln Ala Gly Glu Arg Val
    50                  55                  60

aaa gat cat gca gga aaa gcc atg gga gat att ggt gga cgt ggg aaa      240
Lys Asp His Ala Gly Lys Ala Met Gly Asp Ile Gly Gly Arg Gly Lys
65                  70                  75                  80

gcc aga gag act cac gag ctg ggt gca cac ttt gaa tcc ctg gct gac      288
Ala Arg Glu Thr His Glu Leu Gly Ala His Phe Glu Ser Leu Ala Asp
                85                  90                  95

aaa gta act gac cat gca gca gcc aat gta gtt gga aac aaa gag agt      336
Lys Val Thr Asp His Ala Ala Ala Asn Val Val Gly Asn Lys Glu Ser
            100                 105                 110

caa aga gaa gct cgt ggt ggt gtt cgt gat gtt ggg aag ttc gaa atg      384
Gln Arg Glu Ala Arg Gly Gly Val Arg Asp Val Gly Lys Phe Glu Met
        115                 120                 125

aga act gaa ggg gga gaa aaa gga aac aag gat aga cca gaa ctg aaa      432
Arg Thr Glu Gly Gly Glu Lys Gly Asn Lys Asp Arg Pro Glu Leu Lys
    130                 135                 140

aca cga acc aga gag gta ata gga aga acg gag aaa gaa aga gga aga      480
Thr Arg Thr Arg Glu Val Ile Gly Arg Thr Glu Lys Glu Arg Gly Arg
145                 150                 155                 160

gaa agt gga gga caa gta gtg gca gag aaa gga aga gaa aca gaa act      528
Glu Ser Gly Gly Gln Val Val Ala Glu Lys Gly Arg Glu Thr Glu Thr
                165                 170                 175

gca agg gga agg gtt ggt gct gaa aat gaa ggt gca aga aca acc gct      576
Ala Arg Gly Arg Val Gly Ala Glu Asn Glu Gly Ala Arg Thr Thr Ala
            180                 185                 190

gtt ata act tgc acg ttg gaa aaa ggt ggt ggt act cag aaa cca ata      624
Val Ile Thr Cys Thr Leu Glu Lys Gly Gly Gly Thr Gln Lys Pro Ile
        195                 200                 205

aga gaa gaa gag aga gag agt gaa agt gaa aga tca gcg tgg gaa cag      672
Arg Glu Glu Glu Arg Glu Ser Glu Ser Glu Arg Ser Ala Trp Glu Gln
    210                 215                 220

att tcc aac tac agt gat caa gcc aca caa ggt gtg aag gaa aag tac      720
Ile Ser Asn Tyr Ser Asp Gln Ala Thr Gln Gly Val Lys Glu Lys Tyr
225                 230                 235                 240

gag aga gca aag caa gca gca agc gag aca ctg aac acc aca aca caa      768
Glu Arg Ala Lys Gln Ala Ala Ser Glu Thr Leu Asn Thr Thr Thr Gln
                245                 250                 255

acc gca caa gag aaa agc gca cag gca aag aat ctt gca gca caa gca      816
Thr Ala Gln Glu Lys Ser Ala Gln Ala Lys Asn Leu Ala Ala Gln Ala
            260                 265                 270

aag gac gca acg tta gag aag ggt cag caa ggc tat gct gtg aca aag      864
Lys Asp Ala Thr Leu Glu Lys Gly Gln Gln Gly Tyr Ala Val Thr Lys
        275                 280                 285

gac acc att tca agt gca gcg aaa acc gct tcg gag aaa act gcg cca 912
Asp Thr Ile Ser Ser Ala Ala Lys Thr Ala Ser Glu Lys Thr Ala Pro
    290                 295                 300

gtg gca gag aag gcc aaa gac tac act ctt cag gct gcg gag aag gcc 960
Val Ala Glu Lys Ala Lys Asp Tyr Thr Leu Gln Ala Ala Glu Lys Ala
305                 310                 315                 320

aag agt gcg ggt ggc aca acg gcg agt tac gtc gga gag aaa gcg gtg 1008
Lys Ser Ala Gly Gly Thr Thr Ala Ser Tyr Val Gly Glu Lys Ala Val
                325                 330                 335

cag gcc aag gat gtc gcc gtg gag agt ggg aag agt gcg gct ggg tat 1056
Gln Ala Lys Asp Val Ala Val Glu Ser Gly Lys Ser Ala Ala Gly Tyr
            340                 345                 350

gct gcc aag gtg gct gct gat ttg agg gac aag gcc acc gcg gtg ggg 1104
Ala Ala Lys Val Ala Ala Asp Leu Arg Asp Lys Ala Thr Ala Val Gly
        355                 360                 365

tgg gcc gcg gcc cat ttt tcg gcg gaa aag acg gtg gaa ggg acc aag 1152
Trp Ala Ala Ala His Phe Ser Ala Glu Lys Thr Val Glu Gly Thr Lys
    370                 375                 380

gcc gcc gcc cat gtt gtc gag gga gcg gcc ggg tat gcc gga cat aag 1200
Ala Ala Ala His Val Val Glu Gly Ala Ala Gly Tyr Ala Gly His Lys
385                 390                 395                 400

gcc gcg gag ctt gct tcg atg tcg gcc ggt gct gtt aaa ggg ctg gct 1248
Ala Ala Glu Leu Ala Ser Met Ser Ala Gly Ala Val Lys Gly Leu Ala
                405                 410                 415

gct tct gct ggt gag act gct aag gaa tac acc gca aag aag aaa gag 1296
Ala Ser Ala Gly Glu Thr Ala Lys Glu Tyr Thr Ala Lys Lys Lys Glu
            420                 425                 430

gag gca cag cgg gaa ttg gag gcc aaa aaa cca tct caa cct cag gaa 1344
Glu Ala Gln Arg Glu Leu Glu Ala Lys Lys Pro Ser Gln Pro Gln Glu
        435                 440                 445

gct gaa gag agg cca tca gaa gga att ggc gaa act gtg agg cag tat 1392
Ala Glu Glu Arg Pro Ser Glu Gly Ile Gly Glu Thr Val Arg Gln Tyr
    450                 455                 460

gca caa aaa cca aag cca agt gaa gga aac cct cag aag gag gga acc 1440
Ala Gln Lys Pro Lys Pro Ser Glu Gly Asn Pro Gln Lys Glu Gly Thr
465                 470                 475                 480

gga agc atc gtg ttt aca gca ata ggc gaa aca gta agc agt gcc ggt 1488
Gly Ser Ile Val Phe Thr Ala Ile Gly Glu Thr Val Ser Ser Ala Gly
                485                 490                 495

gag aag gta aag aaa ccg ttt aaa aac act atg gga gga gaa agt gaa 1536
Glu Lys Val Lys Lys Pro Phe Lys Asn Thr Met Gly Gly Glu Ser Glu
            500                 505                 510

gga gga gga ggg aaa gaa gag gga aag agt gtc att ggt aaa agt ttg 1584
Gly Gly Gly Gly Lys Glu Glu Gly Lys Ser Val Ile Gly Lys Ser Leu
        515                 520                 525

acc agc att ggt gag aag ctg ggt gat gca aaa cag aga gag gag ctg 1632
Thr Ser Ile Gly Glu Lys Leu Gly Asp Ala Lys Gln Arg Glu Glu Leu
    530                 535                 540

tta gac aac gtc act ggc aac atc acc gag gga gga ggc gag gtg ttg 1680
Leu Asp Asn Val Thr Gly Asn Ile Thr Glu Gly Gly Gly Glu Val Leu
545                 550                 555                 560

gga gca gta ggt gaa acc gtt gct gag att gga cag aac atg atg aag 1728
Gly Ala Val Gly Glu Thr Val Ala Glu Ile Gly Gln Asn Met Met Lys
                565                 570                 575

cca gcg gag ata gtt cag gaa cgc gct cac gtt cga caa gcg ggt ggg 1776
Pro Ala Glu Ile Val Gln Glu Arg Ala His Val Arg Gln Ala Gly Gly
            580                 585                 590

gtc ttg gat gcc att ggt gaa acc att gct gag att gct gaa acg act 1824
Val Leu Asp Ala Ile Gly Glu Thr Ile Ala Glu Ile Ala Glu Thr Thr
        595                 600                 605

cga gtc atg gtt tct gga gag gac gag aga gtg ctg agg cag agc gtg 1872
Arg Val Met Val Ser Gly Glu Asp Glu Arg Val Leu Arg Gln Ser Val
    610                 615                 620

gtg ttg gag act cgc gta act ggt cgt gcc aag cat gaa gaa ggg tcg 1920
Val Leu Glu Thr Arg Val Thr Gly Arg Ala Lys His Glu Glu Gly Ser
625                 630                 635                 640

cac ggt gct 1929
His Gly Ala

<210> 12
<211> 643
<212> PRT
<213> Glycine max

<400> 12

Met Ala Ser Glu Gln Leu Ala Arg Arg Glu Asn Thr Thr Thr Glu Lys
1               5                   10                  15

Glu Ile His Val Glu Lys His Arg Val Pro Lys Met Ala Thr His Phe
            20                  25                  30

Glu His Leu Ala Glu Gln Ala Lys Glu Ser Asp Ile Thr Ala Gly Lys
        35                  40                  45

Asp Thr Pro Gln Gly Ser Ile Glu Ala Leu Gln Ala Gly Glu Arg Val
    50                  55                  60

Lys Asp His Ala Gly Lys Ala Met Gly Asp Ile Gly Gly Arg Gly Lys
65                  70                  75                  80

Ala Arg Glu Thr His Glu Leu Gly Ala His Phe Glu Ser Leu Ala Asp
                85                  90                  95

Lys Val Thr Asp His Ala Ala Ala Asn Val Val Gly Asn Lys Glu Ser
            100                 105                 110

Gln Arg Glu Ala Arg Gly Gly Val Arg Asp Val Gly Lys Phe Glu Met
        115                 120                 125

Arg Thr Glu Gly Gly Glu Lys Gly Asn Lys Asp Arg Pro Glu Leu Lys
    130                 135                 140

Thr Arg Thr Arg Glu Val Ile Gly Arg Thr Glu Lys Glu Arg Gly Arg
145                 150                 155                 160

Glu Ser Gly Gly Gln Val Val Ala Glu Lys Gly Arg Glu Thr Glu Thr
                165                 170                 175

Ala Arg Gly Arg Val Gly Ala Glu Asn Glu Gly Ala Arg Thr Thr Ala
            180                 185                 190

Val Ile Thr Cys Thr Leu Glu Lys Gly Gly Gly Thr Gln Lys Pro Ile
        195                 200                 205

Arg Glu Glu Glu Arg Glu Ser Glu Ser Glu Arg Ser Ala Trp Glu Gln
    210                 215                 220

Ile Ser Asn Tyr Ser Asp Gln Ala Thr Gln Gly Val Lys Glu Lys Tyr
225                 230                 235                 240

Glu Arg Ala Lys Gln Ala Ala Ser Glu Thr Leu Asn Thr Thr Thr Gln
                245                 250                 255

Thr Ala Gln Glu Lys Ser Ala Gln Ala Lys Asn Leu Ala Ala Gln Ala
            260                 265                 270

Lys Asp Ala Thr Leu Glu Lys Gly Gln Gln Gly Tyr Ala Val Thr Lys
        275                 280                 285

Asp Thr Ile Ser Ser Ala Ala Lys Thr Ala Ser Glu Lys Thr Ala Pro
    290                 295                 300

Val Ala Glu Lys Ala Lys Asp Tyr Thr Leu Gln Ala Ala Glu Lys Ala
305                 310                 315                 320

Lys Ser Ala Gly Gly Thr Thr Ala Ser Tyr Val Gly Glu Lys Ala Val
                325                 330                 335

Gln Ala Lys Asp Val Ala Val Glu Ser Gly Lys Ser Ala Ala Gly Tyr
            340                 345                 350

Ala Ala Lys Val Ala Ala Asp Leu Arg Asp Lys Ala Thr Ala Val Gly

355                 360                 365

Trp Ala Ala Ala His Phe Ser Ala Glu Lys Thr Val Glu Gly Thr Lys
    370                 375                 380

Ala Ala Ala His Val Val Glu Gly Ala Ala Gly Tyr Ala Gly His Lys
385                 390                 395                 400

Ala Ala Glu Leu Ala Ser Met Ser Ala Gly Ala Val Lys Gly Leu Ala
                405                 410                 415

Ala Ser Ala Gly Glu Thr Ala Lys Glu Tyr Thr Ala Lys Lys Lys Glu
            420                 425                 430

Glu Ala Gln Arg Glu Leu Glu Ala Lys Lys Pro Ser Gln Pro Gln Glu
        435                 440                 445

Ala Glu Glu Arg Pro Ser Glu Gly Ile Gly Glu Thr Val Arg Gln Tyr
    450                 455                 460

Ala Gln Lys Pro Lys Pro Ser Glu Gly Asn Pro Gln Lys Glu Gly Thr
465                 470                 475                 480

Gly Ser Ile Val Phe Thr Ala Ile Gly Glu Thr Val Ser Ser Ala Gly
                485                 490                 495

Glu Lys Val Lys Lys Pro Phe Lys Asn Thr Met Gly Gly Glu Ser Glu
            500                 505                 510

Gly Gly Gly Gly Lys Glu Glu Gly Lys Ser Val Ile Gly Lys Ser Leu
        515                 520                 525

Thr Ser Ile Gly Glu Lys Leu Gly Asp Ala Lys Gln Arg Glu Glu Leu
    530                 535                 540

Leu Asp Asn Val Thr Gly Asn Ile Thr Glu Gly Gly Gly Glu Val Leu
545                 550                 555                 560

Gly Ala Val Gly Glu Thr Val Ala Glu Ile Gly Gln Asn Met Met Lys
                565                 570                 575

Pro Ala Glu Ile Val Gln Glu Arg Ala His Val Arg Gln Ala Gly Gly
            580                 585                 590

Val Leu Asp Ala Ile Gly Glu Thr Ile Ala Glu Ile Ala Glu Thr Thr
        595                 600                 605

Arg Val Met Val Ser Gly Glu Asp Glu Arg Val Leu Arg Gln Ser Val
    610                 615                 620

Val Leu Glu Thr Arg Val Thr Gly Arg Ala Lys His Glu Glu Gly Ser
625                 630                 635                 640

His Gly Ala

<210>  13
<211>  771
<212>  DNA
<213>  Glycine max

<220>
<221>  CDS
<222>  (1)..(771)

<220>
<221>  misc_feature
<222>  (242)..(242)
<223>  n is a, c, g, or t

<400>  13
atg gcc ctt tct tgc tcc aaa gtt tta agc ttt tat ctc tcg ccc gtt      48
Met Ala Leu Ser Cys Ser Lys Val Leu Ser Phe Tyr Leu Ser Pro Val
1               5                   10                  15

gtg ggt ggt ggt gat gtt ccg aaa aag ctc act ttt tct tct ttt ttg      96
Val Gly Gly Gly Asp Val Pro Lys Lys Leu Thr Phe Ser Ser Phe Leu
            20                  25                  30

ggt ctt tct aag ggt gtt ggt gga agc agg agc tcg agg gtg tgt gct      144
Gly Leu Ser Lys Gly Val Gly Gly Ser Arg Ser Ser Arg Val Cys Ala
        35                  40                  45

gct tca aat gca ccc gca cca ctc gct ggg gtc ata ttt gaa cca ttt      192
Ala Ser Asn Ala Pro Ala Pro Leu Ala Gly Val Ile Phe Glu Pro Phe
    50                  55                  60

caa gag ctc aag aag gat tat ctt gct gtt ccg att gca cac aat gtt      240
Gln Glu Leu Lys Lys Asp Tyr Leu Ala Val Pro Ile Ala His Asn Val
65                  70                  75                  80

tnc ttg gct cgt cag aac tat gca gat gac tct gaa tct gca atc aac      288
Xaa Leu Ala Arg Gln Asn Tyr Ala Asp Asp Ser Glu Ser Ala Ile Asn
                85                  90                  95

gag cag atc aat gtg gaa tac aac gtt tcc tat gtg tac cac gcc ttg      336
Glu Gln Ile Asn Val Glu Tyr Asn Val Ser Tyr Val Tyr His Ala Leu
            100                 105                 110

ttt gcg tac ttc gac aga gac aac ata gct ctc aaa gga ctt gca aag      384
Phe Ala Tyr Phe Asp Arg Asp Asn Ile Ala Leu Lys Gly Leu Ala Lys
        115                 120                 125

ttc ttc aag gaa tca agt gaa gaa gaa aga gag cat gcc gag cag ctt      432
Phe Phe Lys Glu Ser Ser Glu Glu Glu Arg Glu His Ala Glu Gln Leu
    130                 135                 140

ata aag tat cag aac att cgt ggt gga cga gtg gtg ctg cac cca atc 480
Ile Lys Tyr Gln Asn Ile Arg Gly Gly Arg Val Val Leu His Pro Ile
145                 150                 155                 160

aca agt cct ccc tcg gaa ttt gag cat tcg gaa aaa ggg gat gcc ttg 528
Thr Ser Pro Pro Ser Glu Phe Glu His Ser Glu Lys Gly Asp Ala Leu
                165                 170                 175

tat gcc atg gaa cta gct ttg tct ttg gag aag ttg acg aat gag aaa 576
Tyr Ala Met Glu Leu Ala Leu Ser Leu Glu Lys Leu Thr Asn Glu Lys
            180                 185                 190

ctt ctg cac gtt cac agt gtg gca gaa cgt aac aat gat cct caa ytd 624
Leu Leu His Val His Ser Val Ala Glu Arg Asn Asn Asp Pro Gln Xaa
        195                 200                 205

gca gac ttc att gag agc gag ttc ttg tat gag cag gtt aaa tcg att 672
Ala Asp Phe Ile Glu Ser Glu Phe Leu Tyr Glu Gln Val Lys Ser Ile
    210                 215                 220

aaa aag att gca gag tat gtg gct caa ctg aga tta gtt gga aag ggt 720
Lys Lys Ile Ala Glu Tyr Val Ala Gln Leu Arg Leu Val Gly Lys Gly
225                 230                 235                 240

cat ggt gtg tgg cac ttt gat caa aag ctt ctt cac gat gaa gat cat 768
His Gly Val Trp His Phe Asp Gln Lys Leu Leu His Asp Glu Asp His
                245                 250                 255

gta 771
Val

<210> 14
<211> 257
<212> PRT
<213> Glycine max

<220>
<221> misc_feature
<222> (81)..(81)
<223> The 'Xaa' at location 81 stands for Tyr, Cys, Ser, or Phe.

<220>
<221> misc_feature
<222> (208)..(208)
<223> The 'Xaa' at location 208 stands for Leu, or Phe.

<400> 14

Met Ala Leu Ser Cys Ser Lys Val Leu Ser Phe Tyr Leu Ser Pro Val
1               5                   10                  15

Val Gly Gly Gly Asp Val Pro Lys Lys Leu Thr Phe Ser Ser Phe Leu
            20                  25                  30

Gly Leu Ser Lys Gly Val Gly Gly Ser Arg Ser Ser Arg Val Cys Ala
        35                  40                  45

Ala Ser Asn Ala Pro Ala Pro Leu Ala Gly Val Ile Phe Glu Pro Phe
    50                  55                  60

Gln Glu Leu Lys Lys Asp Tyr Leu Ala Val Pro Ile Ala His Asn Val
65                  70                  75                  80

Xaa Leu Ala Arg Gln Asn Tyr Ala Asp Asp Ser Glu Ser Ala Ile Asn
                85                  90                  95

Glu Gln Ile Asn Val Glu Tyr Asn Val Ser Tyr Val Tyr His Ala Leu
            100                 105                 110

Phe Ala Tyr Phe Asp Arg Asp Asn Ile Ala Leu Lys Gly Leu Ala Lys
        115                 120                 125

Phe Phe Lys Glu Ser Ser Glu Glu Glu Arg Glu His Ala Glu Gln Leu
    130                 135                 140

Ile Lys Tyr Gln Asn Ile Arg Gly Gly Arg Val Val Leu His Pro Ile
145                 150                 155                 160

Thr Ser Pro Pro Ser Glu Phe Glu His Ser Glu Lys Gly Asp Ala Leu
                165                 170                 175

Tyr Ala Met Glu Leu Ala Leu Ser Leu Glu Lys Leu Thr Asn Glu Lys
            180                 185                 190

Leu Leu His Val His Ser Val Ala Glu Arg Asn Asn Asp Pro Gln Xaa
        195                 200                 205

Ala Asp Phe Ile Glu Ser Glu Phe Leu Tyr Glu Gln Val Lys Ser Ile
    210                 215                 220

Lys Lys Ile Ala Glu Tyr Val Ala Gln Leu Arg Leu Val Gly Lys Gly
225                 230                 235                 240

His Gly Val Trp His Phe Asp Gln Lys Leu Leu His Asp Glu Asp His
                245                 250                 255

Val

<210>  15
<211>  2421
<212>  DNA
<213>  Glycine max

<220>

<221>  CDS
<222>  (1)..(2421)

<400>  15
atg tct cag caa ggc gaa tca tcg gac ccg aaa tcg ggg aag aag gac      48
Met Ser Gln Gln Gly Glu Ser Ser Asp Pro Lys Ser Gly Lys Lys Asp
1               5                   10                  15

ttc tca acc gcg att ctg gaa cgc aaa aag tcg ccg aac cgg ctt gtc      96
Phe Ser Thr Ala Ile Leu Glu Arg Lys Lys Ser Pro Asn Arg Leu Val
            20                  25                  30

gtg gat gag gct gtc aac gac gac aac tcc gtg gtc acc atg cat ccc      144
Val Asp Glu Ala Val Asn Asp Asp Asn Ser Val Val Thr Met His Pro
        35                  40                  45

caa acc atg gaa aag ctt cag ctt ttc cga ggg gac act atc ctc atc      192
Gln Thr Met Glu Lys Leu Gln Leu Phe Arg Gly Asp Thr Ile Leu Ile
    50                  55                  60

aag ggt aag aaa cgg aag gat acc att tgc ata gct ctt gct gat gag      240
Lys Gly Lys Lys Arg Lys Asp Thr Ile Cys Ile Ala Leu Ala Asp Glu
65                  70                  75                  80

aac tgt gag gag ccg aag atc agg atg aat aaa gtt gtg agg tca aat      288
Asn Cys Glu Glu Pro Lys Ile Arg Met Asn Lys Val Val Arg Ser Asn
                85                  90                  95

ttg cgg gtt cgg ctt gga gat gtt gtg tct gtg cac cag tgc ccg gat      336
Leu Arg Val Arg Leu Gly Asp Val Val Ser Val His Gln Cys Pro Asp
            100                 105                 110

gtt aaa tat ggg aaa cgt gtg cac att ctg cct att gat gat acc att      384
Val Lys Tyr Gly Lys Arg Val His Ile Leu Pro Ile Asp Asp Thr Ile
        115                 120                 125

gaa ggt gtc act ggc aat ctg ttt gat gct ttc ttg aaa ccc tat ttc      432
Glu Gly Val Thr Gly Asn Leu Phe Asp Ala Phe Leu Lys Pro Tyr Phe
    130                 135                 140

ttg gaa gct tac cgt cct gtc aga aaa gga gat cta ttt ctt gtc cgg      480
Leu Glu Ala Tyr Arg Pro Val Arg Lys Gly Asp Leu Phe Leu Val Arg
145                 150                 155                 160

gga ggg atg aga agt gta gag ttc aag gtg gtt gaa acc gat cct gga      528
Gly Gly Met Arg Ser Val Glu Phe Lys Val Val Glu Thr Asp Pro Gly
                165                 170                 175

gaa tat tgt gtt gtt gct cca gac act gaa att ttc tgt gag ggg gag      576
Glu Tyr Cys Val Val Ala Pro Asp Thr Glu Ile Phe Cys Glu Gly Glu
            180                 185                 190

cct ttg aaa aga gaa gat gaa gag aga ctg gat gaa gtc ggg tat gat      624
Pro Leu Lys Arg Glu Asp Glu Glu Arg Leu Asp Glu Val Gly Tyr Asp
        195                 200                 205

gat gtg ggt ggt gtc agg aaa caa atg gct cag att cgt gag ttg gtt      672
Asp Val Gly Gly Val Arg Lys Gln Met Ala Gln Ile Arg Glu Leu Val
    210                 215                 220

gag ctt cca ttg agg cat cca cag ctt ttt aaa tca att ggt gtg aaa      720
Glu Leu Pro Leu Arg His Pro Gln Leu Phe Lys Ser Ile Gly Val Lys
225                 230                 235                 240

cca ccc aaa gga att tta ctt tat gga ccc cca ggt tct ggg aag aca 768
Pro Pro Lys Gly Ile Leu Leu Tyr Gly Pro Pro Gly Ser Gly Lys Thr
                245                 250                 255

tta ata gca aga gct gta gct aat gaa act gga gct ttt ttc ttt tgt 816
Leu Ile Ala Arg Ala Val Ala Asn Glu Thr Gly Ala Phe Phe Phe Cys
            260                 265                 270

att aac gga cca gag att atg tcc aaa ctt gct gga gag agt gaa agc 864
Ile Asn Gly Pro Glu Ile Met Ser Lys Leu Ala Gly Glu Ser Glu Ser
        275                 280                 285

aat ctc agg aaa gcg ttt gaa gaa gca gaa aag aat gca cca tcc att 912
Asn Leu Arg Lys Ala Phe Glu Glu Ala Glu Lys Asn Ala Pro Ser Ile
    290                 295                 300

atc ttc att gat gaa att gat tcc att gcc ccc aag cgg gag aag aca 960
Ile Phe Ile Asp Glu Ile Asp Ser Ile Ala Pro Lys Arg Glu Lys Thr
305                 310                 315                 320

cat ggt gaa gtt gaa agg agg att gtt tca caa ctt ttg act ctt atg 1008
His Gly Glu Val Glu Arg Arg Ile Val Ser Gln Leu Leu Thr Leu Met
                325                 330                 335

gat gga tta aaa tct cgt gct cat gtt att gtt att gga gct act aat 1056
Asp Gly Leu Lys Ser Arg Ala His Val Ile Val Ile Gly Ala Thr Asn
            340                 345                 350

cgt cca aat agc att gac cca gca ttg aga agg ttt ggt aga ttt gat 1104
Arg Pro Asn Ser Ile Asp Pro Ala Leu Arg Arg Phe Gly Arg Phe Asp
        355                 360                 365

agg gaa att gat att ggt gtt ccc gat gaa gtt ggg aga ctt gaa gtc 1152
Arg Glu Ile Asp Ile Gly Val Pro Asp Glu Val Gly Arg Leu Glu Val
    370                 375                 380

ctt cgc ata cat act aaa aac atg aag cta tct gat gat gtt gat tta 1200
Leu Arg Ile His Thr Lys Asn Met Lys Leu Ser Asp Asp Val Asp Leu
385                 390                 395                 400

gag aga att gca aaa gat act cat ggg tat gtt ggt gct gac ctt gct 1248
Glu Arg Ile Ala Lys Asp Thr His Gly Tyr Val Gly Ala Asp Leu Ala
                405                 410                 415

gcc ctt tgc act gaa gct gct ttg caa tgc att agg gag aag atg gat 1296
Ala Leu Cys Thr Glu Ala Ala Leu Gln Cys Ile Arg Glu Lys Met Asp
            420                 425                 430

gta att gac ttg gag gat gag act att gat gca gaa gta ctg aat tcc 1344
Val Ile Asp Leu Glu Asp Glu Thr Ile Asp Ala Glu Val Leu Asn Ser
        435                 440                 445

atg gca gtt aca aat gag cat ttc cag act gcc ctt gga aca agc aac 1392
Met Ala Val Thr Asn Glu His Phe Gln Thr Ala Leu Gly Thr Ser Asn
    450                 455                 460

cca tca gct tta cgt gaa act gtt gtt gaa gtg cct aat gtc agc tgg 1440
Pro Ser Ala Leu Arg Glu Thr Val Val Glu Val Pro Asn Val Ser Trp
465                 470                 475                 480

gaa gac att gga ggc ctt gaa aat gtc aag cgt gaa ctg caa gag act 1488
Glu Asp Ile Gly Gly Leu Glu Asn Val Lys Arg Glu Leu Gln Glu Thr

485                 490                 495

gta caa tat cca gtt gag cac cca gaa aag ttt gag aag ttt ggt atg       1536
Val Gln Tyr Pro Val Glu His Pro Glu Lys Phe Glu Lys Phe Gly Met
            500                 505                 510

tct cca tct aaa gga gta ctt ttc tat ggt cct cca gga tgt ggg aaa       1584
Ser Pro Ser Lys Gly Val Leu Phe Tyr Gly Pro Pro Gly Cys Gly Lys
        515                 520                 525

act ttg tta gcc aaa gca att gct aat gaa tgt caa gct aac ttc atc       1632
Thr Leu Leu Ala Lys Ala Ile Ala Asn Glu Cys Gln Ala Asn Phe Ile
    530                 535                 540

agt gtg aaa ggt cca gaa ttg ctt aca atg tgg ttt ggt gaa agt gaa       1680
Ser Val Lys Gly Pro Glu Leu Leu Thr Met Trp Phe Gly Glu Ser Glu
545                 550                 555                 560

gct aat gtt aga gaa att ttt gac aag gcc aga caa tcg gct cca tgt       1728
Ala Asn Val Arg Glu Ile Phe Asp Lys Ala Arg Gln Ser Ala Pro Cys
                565                 570                 575

gtc ctc ttc ttt gat gag ctt gac tct att gcc act cag aga gga agc       1776
Val Leu Phe Phe Asp Glu Leu Asp Ser Ile Ala Thr Gln Arg Gly Ser
            580                 585                 590

agt gtt ggg gat gct ggt ggt gcg gct gat aga gtt cta aat caa ctt       1824
Ser Val Gly Asp Ala Gly Gly Ala Ala Asp Arg Val Leu Asn Gln Leu
        595                 600                 605

ttg act gaa atg gat ggc atg tct gcc aaa aag act gtg ttc ata att       1872
Leu Thr Glu Met Asp Gly Met Ser Ala Lys Lys Thr Val Phe Ile Ile
    610                 615                 620

ggg gca act aac aga cct gac atc att gac cca gca ctt ctt cga cca       1920
Gly Ala Thr Asn Arg Pro Asp Ile Ile Asp Pro Ala Leu Leu Arg Pro
625                 630                 635                 640

ggc cgt ctg gat caa ttg atc tat att cct ctc cct gat gag gat tcc       1968
Gly Arg Leu Asp Gln Leu Ile Tyr Ile Pro Leu Pro Asp Glu Asp Ser
                645                 650                 655

cgg cat caa atc ttt aaa gcc tgc ttg agg aag tca cct atc gca aaa       2016
Arg His Gln Ile Phe Lys Ala Cys Leu Arg Lys Ser Pro Ile Ala Lys
            660                 665                 670

aat gtt gat ttg aga gca ttg gct agg cac act caa ggg ttc agt ggt       2064
Asn Val Asp Leu Arg Ala Leu Ala Arg His Thr Gln Gly Phe Ser Gly
        675                 680                 685

gct gat att aca gaa ata tgc cag cga gca tgc aaa tat gcc ata aga       2112
Ala Asp Ile Thr Glu Ile Cys Gln Arg Ala Cys Lys Tyr Ala Ile Arg
    690                 695                 700

gaa aac ata gag aag gac att gag cga gag agg aag agt agg gaa aac       2160
Glu Asn Ile Glu Lys Asp Ile Glu Arg Glu Arg Lys Ser Arg Glu Asn
705                 710                 715                 720

cct gag gcc atg gat gaa gat acc gtc gac gac gaa gtt gcg gag atc       2208
Pro Glu Ala Met Asp Glu Asp Thr Val Asp Asp Glu Val Ala Glu Ile
                725                 730                 735

aag gct gct cat ttt gag gag tca atg aag ttt gca cgg agg agt gtt       2256

Lys Ala Ala His Phe Glu Glu Ser Met Lys Phe Ala Arg Arg Ser Val
            740                 745                 750

agt gat gct gat att cgc aag tat cag gca ttt gct cag acc tta cag      2304
Ser Asp Ala Asp Ile Arg Lys Tyr Gln Ala Phe Ala Gln Thr Leu Gln
        755                 760                 765

caa tca aga ggg ttt gga agt gaa ttt agg ttt cca gag tct gga gat      2352
Gln Ser Arg Gly Phe Gly Ser Glu Phe Arg Phe Pro Glu Ser Gly Asp
    770                 775                 780

agg act act act gga tct gac ccc ttt gca gct tct gct ggt ggg gct      2400
Arg Thr Thr Thr Gly Ser Asp Pro Phe Ala Ala Ser Ala Gly Gly Ala
785                 790                 795                 800

gac gaa gat gat cta tat agt                                          2421
Asp Glu Asp Asp Leu Tyr Ser
                805

<210>  16
<211>  807
<212>  PRT
<213>  Glycine max

<400>  16

Met Ser Gln Gln Gly Glu Ser Ser Asp Pro Lys Ser Gly Lys Lys Asp
1               5                   10                  15

Phe Ser Thr Ala Ile Leu Glu Arg Lys Lys Ser Pro Asn Arg Leu Val
            20                  25                  30

Val Asp Glu Ala Val Asn Asp Asp Asn Ser Val Val Thr Met His Pro
        35                  40                  45

Gln Thr Met Glu Lys Leu Gln Leu Phe Arg Gly Asp Thr Ile Leu Ile
    50                  55                  60

Lys Gly Lys Lys Arg Lys Asp Thr Ile Cys Ile Ala Leu Ala Asp Glu
65                  70                  75                  80

Asn Cys Glu Glu Pro Lys Ile Arg Met Asn Lys Val Val Arg Ser Asn
                85                  90                  95

Leu Arg Val Arg Leu Gly Asp Val Val Ser Val His Gln Cys Pro Asp
            100                 105                 110

Val Lys Tyr Gly Lys Arg Val His Ile Leu Pro Ile Asp Asp Thr Ile
        115                 120                 125

Glu Gly Val Thr Gly Asn Leu Phe Asp Ala Phe Leu Lys Pro Tyr Phe
    130                 135                 140

Leu Glu Ala Tyr Arg Pro Val Arg Lys Gly Asp Leu Phe Leu Val Arg
145                 150                 155                 160

Gly Gly Met Arg Ser Val Glu Phe Lys Val Val Glu Thr Asp Pro Gly
                165                 170                 175

Glu Tyr Cys Val Val Ala Pro Asp Thr Glu Ile Phe Cys Glu Gly Glu
            180                 185                 190

Pro Leu Lys Arg Glu Asp Glu Glu Arg Leu Asp Glu Val Gly Tyr Asp
        195                 200                 205

Asp Val Gly Gly Val Arg Lys Gln Met Ala Gln Ile Arg Glu Leu Val
    210                 215                 220

Glu Leu Pro Leu Arg His Pro Gln Leu Phe Lys Ser Ile Gly Val Lys
225                 230                 235                 240

Pro Pro Lys Gly Ile Leu Leu Tyr Gly Pro Pro Gly Ser Gly Lys Thr
                245                 250                 255

Leu Ile Ala Arg Ala Val Ala Asn Glu Thr Gly Ala Phe Phe Phe Cys
            260                 265                 270

Ile Asn Gly Pro Glu Ile Met Ser Lys Leu Ala Gly Glu Ser Glu Ser
        275                 280                 285

Asn Leu Arg Lys Ala Phe Glu Glu Ala Glu Lys Asn Ala Pro Ser Ile
    290                 295                 300

Ile Phe Ile Asp Glu Ile Asp Ser Ile Ala Pro Lys Arg Glu Lys Thr
305                 310                 315                 320

His Gly Glu Val Glu Arg Arg Ile Val Ser Gln Leu Leu Thr Leu Met
                325                 330                 335

Asp Gly Leu Lys Ser Arg Ala His Val Ile Val Ile Gly Ala Thr Asn
            340                 345                 350

Arg Pro Asn Ser Ile Asp Pro Ala Leu Arg Arg Phe Gly Arg Phe Asp
        355                 360                 365

Arg Glu Ile Asp Ile Gly Val Pro Asp Glu Val Gly Arg Leu Glu Val
    370                 375                 380

Leu Arg Ile His Thr Lys Asn Met Lys Leu Ser Asp Asp Val Asp Leu
385                 390                 395                 400

Glu Arg Ile Ala Lys Asp Thr His Gly Tyr Val Gly Ala Asp Leu Ala
                405                 410                 415

Ala Leu Cys Thr Glu Ala Ala Leu Gln Cys Ile Arg Glu Lys Met Asp
            420                 425                 430

Val Ile Asp Leu Glu Asp Glu Thr Ile Asp Ala Glu Val Leu Asn Ser
        435                 440                 445

Met Ala Val Thr Asn Glu His Phe Gln Thr Ala Leu Gly Thr Ser Asn
    450                 455                 460

Pro Ser Ala Leu Arg Glu Thr Val Val Glu Val Pro Asn Val Ser Trp
465                 470                 475                 480

Glu Asp Ile Gly Gly Leu Glu Asn Val Lys Arg Glu Leu Gln Glu Thr
                485                 490                 495

Val Gln Tyr Pro Val Glu His Pro Glu Lys Phe Glu Lys Phe Gly Met
            500                 505                 510

Ser Pro Ser Lys Gly Val Leu Phe Tyr Gly Pro Pro Gly Cys Gly Lys
        515                 520                 525

Thr Leu Leu Ala Lys Ala Ile Ala Asn Glu Cys Gln Ala Asn Phe Ile
    530                 535                 540

Ser Val Lys Gly Pro Glu Leu Leu Thr Met Trp Phe Gly Glu Ser Glu
545                 550                 555                 560

Ala Asn Val Arg Glu Ile Phe Asp Lys Ala Arg Gln Ser Ala Pro Cys
                565                 570                 575

Val Leu Phe Phe Asp Glu Leu Asp Ser Ile Ala Thr Gln Arg Gly Ser
            580                 585                 590

Ser Val Gly Asp Ala Gly Gly Ala Ala Asp Arg Val Leu Asn Gln Leu
        595                 600                 605

Leu Thr Glu Met Asp Gly Met Ser Ala Lys Lys Thr Val Phe Ile Ile
    610                 615                 620

Gly Ala Thr Asn Arg Pro Asp Ile Ile Asp Pro Ala Leu Leu Arg Pro
625                 630                 635                 640

Gly Arg Leu Asp Gln Leu Ile Tyr Ile Pro Leu Pro Asp Glu Asp Ser
                645                 650                 655

Arg His Gln Ile Phe Lys Ala Cys Leu Arg Lys Ser Pro Ile Ala Lys
            660                 665                 670

Asn Val Asp Leu Arg Ala Leu Ala Arg His Thr Gln Gly Phe Ser Gly
        675                 680                 685

Ala Asp Ile Thr Glu Ile Cys Gln Arg Ala Cys Lys Tyr Ala Ile Arg
    690                 695                 700

Glu Asn Ile Glu Lys Asp Ile Glu Arg Glu Arg Lys Ser Arg Glu Asn
705                 710                 715                 720

Pro Glu Ala Met Asp Glu Asp Thr Val Asp Asp Glu Val Ala Glu Ile
                725                 730                 735

Lys Ala Ala His Phe Glu Glu Ser Met Lys Phe Ala Arg Arg Ser Val
            740                 745                 750

Ser Asp Ala Asp Ile Arg Lys Tyr Gln Ala Phe Ala Gln Thr Leu Gln
        755                 760                 765

Gln Ser Arg Gly Phe Gly Ser Glu Phe Arg Phe Pro Glu Ser Gly Asp
    770                 775                 780

Arg Thr Thr Thr Gly Ser Asp Pro Phe Ala Ala Ser Ala Gly Gly Ala
785                 790                 795                 800

Asp Glu Asp Asp Leu Tyr Ser
                805

<210> 17
<211> 13
<212> PRT
<213> Glycine max

<400> 17

Asp Gln Ser Ser Ser Tyr Asp Asp Asp Glu Tyr Ser Lys
1               5                   10

<210> 18
<211> 15
<212> PRT
<213> Glycine max

<400> 18

Lys Ser Asp Gln Ser Ser Ser Tyr Asp Asp Asp Glu Tyr Ser Lys
1               5                   10                  15

<210>  19
<211>  14
<212>  PRT
<213>  Glycine max

<400>  19

Ser Asp Gln Ser Ser Ser Tyr Asp Asp Asp Glu Tyr Ser Lys
1               5                   10

<210>  20
<211>  9
<212>  PRT
<213>  Glycine max

<400>  20

Ser Tyr Asp Asp Asp Glu Tyr Ser Lys
1               5

<210>  21
<211>  18
<212>  PRT
<213>  Glycine max

<400>  21

Ala Leu Arg Ala Leu Arg Leu Glu Asp Leu Arg Ile Pro Thr Ala Tyr
1               5                   10                  15

Ile Lys

<210>  22
<211>  18
<212>  PRT
<213>  Glycine max

<400>  22

Ala Leu Arg Leu Ser Gly Gly Asp His Val His Ala Gly Thr Val Val
1               5                   10                  15

Gly Lys

<210>  23
<211>  22
<212>  PRT
<213>  Glycine max

<400>  23

Leu Glu Gly Glu Arg Glu Ile Thr Leu Gly Phe Val Asp Leu Leu Arg
1               5                   10                  15

Asp Asp Phe Val Glu Lys
            20

<210>  24
<211>  14
<212>  PRT
<213>  Glycine max

<400>  24

Leu Arg Leu Glu Asp Leu Arg Ile Pro Thr Ala Tyr Ile Lys
1               5                   10

<210>  25
<211>  11
<212>  PRT
<213>  Glycine max

<400>  25

Leu Thr Tyr Tyr Thr Pro Asp Tyr Glu Thr Lys
1               5                   10

<210>  26
<211>  11
<212>  PRT
<213>  Glycine max

<400>  26

Thr Asp Gly Leu Thr Ser Leu Asp Arg Tyr Lys
1               5                   10

<210>  27
<211>  14
<212>  PRT
<213>  Glycine max

<400>  27

Thr Phe Gln Gly Pro Pro His Gly Ile Gln Val Glu Arg Asp
1               5                   10

<210>  28
<211>  15
<212>  PRT
<213>  Glycine max

<400>  28

Thr Phe Gln Gly Pro Pro His Gly Ile Gln Val Glu Arg Asp Lys
1               5                   10                  15

<210>  29
<211>  24
<212>  PRT

<213>  Glycine max

<400>  29

Ala Pro His Thr Glu Asp Ala Ile Arg Val Thr Gln Ser Thr Ala Asn
1               5                   10                  15

Phe Phe Ile Ser Glu Ala Arg Lys
            20

<210>  30
<211>  23
<212>  PRT
<213>  Glycine max

<400>  30

Phe Val Glu Ser Gly Gly Ala Arg Val Ile Pro Leu Ile Tyr Asn Glu
1               5                   10                  15

Ser Pro Glu Asn Leu Asn Lys
            20

<210>  31
<211>  7
<212>  PRT
<213>  Glycine max

<400>  31

Ile Trp Asp Leu Glu Ser Lys
1               5

<210>  32
<211>  7
<212>  PRT
<213>  Glycine max

<400>  32

Ser Ile Val Glu Asp Leu Lys
1               5

<210>  33
<211>  21
<212>  PRT
<213>  Glycine max

<400>  33

Gln Glu Leu Asp Glu Arg Ala Arg Gln Gly Glu Thr Val Val Pro Gly
1               5                   10                  15

Gly Thr Gly Gly Lys
            20

<210>  34
<211>  17
<212>  PRT
<213>  Glycine max

<400>  34

Ser Gly Glu Glu Arg Ala Gln Glu Glu Ala Ile Asp Ile Asp Glu Ser
1               5                   10                  15

Lys

<210>  35
<211>  16
<212>  PRT
<213>  Glycine max

<400>  35

Ala Ala Ala His Val Val Glu Gly Ala Ala Gly Tyr Ala Gly His Lys
1               5                   10                  15

<210>  36
<211>  11
<212>  PRT
<213>  Glycine max

<400>  36

Ala Lys Asp Tyr Thr Leu Gln Ala Ala Glu Lys
1               5                   10

<210>  37
<211>  17
<212>  PRT
<213>  Glycine max

<400>  37

Ala Arg Glu Thr His Glu Leu Gly Ala His Phe Glu Ser Leu Ala Asp
1               5                   10                  15

Lys

<210>  38
<211>  15
<212>  PRT
<213>  Glycine max

<400>  38

Ala Thr Ala Val Gly Trp Ala Ala Ala His Phe Ser Ala Glu Lys
1               5                   10                  15

<210>  39
<211>  17
<212>  PRT
<213>  Glycine max

<400>  39

Asp Thr Pro Gln Gly Ser Ile Glu Ala Leu Gln Ala Gly Glu Arg Val
1               5                   10                  15

Lys

<210>  40
<211>  9
<212>  PRT
<213>  Glycine max

<400>  40

Asp Tyr Thr Leu Gln Ala Ala Glu Lys
1               5

<210>  41
<211>  10
<212>  PRT
<213>  Glycine max

<400>  41

Glu Glu Ala Gln Arg Glu Leu Glu Ala Lys
1               5                   10

<210>  42
<211>  21
<212>  PRT
<213>  Glycine max

<400>  42

Glu Gly Thr Gly Ser Ile Val Phe Thr Ala Ile Gly Glu Thr Val Ser
1               5                   10                  15

Ser Ala Gly Glu Lys
            20

<210>  43
<211>  14
<212>  PRT
<213>  Glycine max

<400>  43

Glu Arg Gly Arg Glu Ser Gly Gly Gln Val Val Ala Glu Lys
1               5                   10

<210>  44
<211>  11
<212>  PRT
<213>  Glycine max

<400>  44

Gly Leu Ala Ala Ser Ala Gly Glu Thr Ala Lys
1               5                   10


<210>  45
<211>  9
<212>  PRT
<213>  Glycine max

<400>  45

Gly Gln Gln Gly Tyr Ala Val Thr Lys
1               5


<210>  46
<211>  26
<212>  PRT
<213>  Glycine max

<400>  46

Lys Pro Ser Gln Pro Gln Glu Ala Glu Glu Arg Pro Ser Glu Gly Ile
1               5                   10                  15


Gly Glu Thr Val Arg Gln Tyr Ala Gln Lys
            20                  25


<210>  47
<211>  8
<212>  PRT
<213>  Glycine max

<400>  47

Pro Ala Glu Ile Val Gln Glu Arg
1               5


<210>  48
<211>  15
<212>  PRT
<213>  Glycine max

<400>  48

Pro Gln Gly Ser Ile Glu Ala Leu Gln Ala Gly Glu Arg Val Lys
1               5                   10                  15


<210>  49
<211>  17
<212>  PRT

<213>  Glycine max

<400>  49

Gln Ala Ala Ser Glu Thr Leu Asn Thr Thr Thr Gln Thr Ala Gln Glu
1               5                   10                  15

Lys

<210>  50
<211>  13
<212>  PRT
<213>  Glycine max

<400>  50

Ser Ala Gly Gly Thr Thr Ala Ser Tyr Val Gly Glu Lys
1               5                   10

<210>  51
<211>  8
<212>  PRT
<213>  Glycine max

<400>  51

Ser Leu Thr Ser Ile Gly Glu Lys
1               5

<210>  52
<211>  16
<212>  PRT
<213>  Glycine max

<400>  52

Thr Pro Gln Gly Ser Ile Glu Ala Leu Gln Ala Gly Glu Arg Val Lys
1               5                   10                  15

<210>  53
<211>  13
<212>  PRT
<213>  Glycine max

<400>  53

Val Thr Asp His Ala Ala Ala Asn Val Val Gly Asn Lys
1               5                   10

<210>  54
<211>  22
<212>  PRT
<213>  Glycine max

<400>  54

Ala Ala Ser Asn Ala Pro Ala Pro Leu Ala Gly Val Ile Phe Glu Pro
1               5                   10                  15

Phe Gln Glu Leu Lys Lys
            20

<210>  55
<211>  21
<212>  PRT
<213>  Glycine max

<400>  55

Ala Ser Asn Ala Pro Ala Pro Leu Ala Gly Val Ile Phe Glu Pro Phe
1               5                   10                  15

Gln Glu Leu Lys Lys
            20

<210>  56
<211>  18
<212>  PRT
<213>  Glycine max

<400>  56

Phe Phe Lys Glu Ser Ser Glu Glu Glu Arg Glu His Ala Glu Gln Leu
1               5                   10                  15

Ile Lys

<210>  57
<211>  10
<212>  PRT
<213>  Glycine max

<400>  57

Gly His Gly Val Trp His Phe Asp Gln Lys
1               5                   10

<210>  58
<211>  13
<212>  PRT
<213>  Glycine max

<400>  58

Ile Ala Glu Tyr Val Ala Gln Leu Arg Leu Val Gly Lys
1               5                   10

<210>  59
<211>  14
<212>  PRT

<213>  Glycine max

<400>  59

Lys Ile Ala Glu Tyr Val Ala Gln Leu Arg Leu Val Gly Lys
1               5                   10


<210>  60
<211>  8
<212>  PRT
<213>  Glycine max

<400>  60

Leu Leu His Asp Glu Asp His Val
1               5


<210>  61
<211>  20
<212>  PRT
<213>  Glycine max

<400>  61

Ser Asn Ala Pro Ala Pro Leu Ala Gly Val Ile Phe Glu Pro Phe Gln
1               5                   10                  15


Glu Leu Lys Lys
            20


<210>  62
<211>  10
<212>  PRT
<213>  Glycine max

<400>  62

Ala Gly Glu Ser Glu Ser Asn Leu Arg Lys
1               5                   10


<210>  63
<211>  13
<212>  PRT
<213>  Glycine max

<400>  63

Gly Glu Ser Glu Ala Asn Val Arg Glu Ile Phe Asp Lys
1               5                   10


<210>  64
<211>  12
<212>  PRT
<213>  Glycine max

<400>  64

Gly Ile Leu Leu Tyr Gly Pro Pro Gly Ser Gly Lys
1               5                   10

<210>  65
<211>  11
<212>  PRT
<213>  Glycine max

<400>  65

Leu Ala Gly Glu Ser Glu Ser Asn Leu Arg Lys
1               5                   10

<210>  66
<211>  12
<212>  PRT
<213>  Glycine max

<400>  66

Leu Ser Asp Asp Val Asp Leu Glu Arg Ile Ala Lys
1               5                   10

<210>  67
<211>  16
<212>  PRT
<213>  Glycine max

<400>  67

Arg Glu Leu Gln Glu Thr Val Gln Tyr Pro Val Glu His Pro Glu Lys
1               5                   10                  15

<210>  68
<211>  15
<212>  PRT
<213>  Glycine max

<400>  68

Thr Val Phe Ile Ile Gly Ala Thr Asn Arg Pro Asp Ile Ile Asp
1               5                   10                  15

<210>  69
<211>  2448
<212>  DNA
<213>  Glycine max

<220>
<221>  CDS
<222>  (1)..(2448)

<400>  69
atg agg aag tgg acg gtt gct tcc gct ctc ctt ctt ctc tct ctc ctc          48
Met Arg Lys Trp Thr Val Ala Ser Ala Leu Leu Leu Leu Ser Leu Leu
1               5                   10                  15

ttt ctc ttt gca gat caa ggt cga aag ttt cag gcg aat gcc gag gga 96
Phe Leu Phe Ala Asp Gln Gly Arg Lys Phe Gln Ala Asn Ala Glu Gly
            20                  25                  30

gat tcc gac gag ctt gta gat cca ccc aag gtg gag gac aag atc ggt 144
Asp Ser Asp Glu Leu Val Asp Pro Pro Lys Val Glu Asp Lys Ile Gly
        35                  40                  45

gct gtg cct cat ggc ctt tca acc gat tca gat gtc gtc aag agg gag 192
Ala Val Pro His Gly Leu Ser Thr Asp Ser Asp Val Val Lys Arg Glu
    50                  55                  60

gcg gag tcg atc tcg aag aga tct ctc cgg agc aac gcg gag aag ttt 240
Ala Glu Ser Ile Ser Lys Arg Ser Leu Arg Ser Asn Ala Glu Lys Phe
65                  70                  75                  80

gag ttc caa gcg gaa gtg tcg cgg ctt atg gat atc atc atc aat tct 288
Glu Phe Gln Ala Glu Val Ser Arg Leu Met Asp Ile Ile Ile Asn Ser
                85                  90                  95

ctc tac agt aac aag gat atc ttc ctc aga gag ttg att tcc aat gct 336
Leu Tyr Ser Asn Lys Asp Ile Phe Leu Arg Glu Leu Ile Ser Asn Ala
            100                 105                 110

tct gat gct ttg gac aag att agg ttc ctc tcc ctc acg gat aag gag 384
Ser Asp Ala Leu Asp Lys Ile Arg Phe Leu Ser Leu Thr Asp Lys Glu
        115                 120                 125

gtg ttg gga gaa ggt gat aac acc aag ctt gat att cag att aag ttg 432
Val Leu Gly Glu Gly Asp Asn Thr Lys Leu Asp Ile Gln Ile Lys Leu
    130                 135                 140

gac aag gaa aag aaa att ctc tca att cga gac aga ggt atc ggt atg 480
Asp Lys Glu Lys Lys Ile Leu Ser Ile Arg Asp Arg Gly Ile Gly Met
145                 150                 155                 160

acg aag gag gat ttg ata aag aat ttg ggg aca ata gcg aaa tct gga 528
Thr Lys Glu Asp Leu Ile Lys Asn Leu Gly Thr Ile Ala Lys Ser Gly
                165                 170                 175

act tct gca ttt gtt gag aag atg caa aca agt gga gat ctc aat ctg 576
Thr Ser Ala Phe Val Glu Lys Met Gln Thr Ser Gly Asp Leu Asn Leu
            180                 185                 190

att ggg cag ttt gga gtc ggc ttc tac tct gtt tat ctt gtg gcc gac 624
Ile Gly Gln Phe Gly Val Gly Phe Tyr Ser Val Tyr Leu Val Ala Asp
        195                 200                 205

tat gtc gaa gtt att agc aag aat aac gat gat aag cag tat gta tgg 672
Tyr Val Glu Val Ile Ser Lys Asn Asn Asp Asp Lys Gln Tyr Val Trp
    210                 215                 220

gaa tca aaa gct gat gga gca ttc gct att tca gaa gat aca tgg aat 720
Glu Ser Lys Ala Asp Gly Ala Phe Ala Ile Ser Glu Asp Thr Trp Asn
225                 230                 235                 240

gaa cca ctt gga cga gga act gag att aga tta cac ctc aag gaa gag 768
Glu Pro Leu Gly Arg Gly Thr Glu Ile Arg Leu His Leu Lys Glu Glu
                245                 250                 255

gct ggg gaa tac ttg gag gag tct aaa ctg aaa gaa ttg gtg aag aga 816
Ala Gly Glu Tyr Leu Glu Glu Ser Lys Leu Lys Glu Leu Val Lys Arg
            260                 265                 270

tac tct gaa ttt att aac ttc cct atc tat att tgg gca agc aaa gaa 864
Tyr Ser Glu Phe Ile Asn Phe Pro Ile Tyr Ile Trp Ala Ser Lys Glu
        275                 280                 285

gtt gat gtg gag gtt ccc gct gat gaa gat gat tcc agt gat gaa gag 912
Val Asp Val Glu Val Pro Ala Asp Glu Asp Asp Ser Ser Asp Glu Glu
    290                 295                 300

gat tca tct gaa agc agc tcc aag gag gaa agc gaa gat gaa gat gct 960
Asp Ser Ser Glu Ser Ser Ser Lys Glu Glu Ser Glu Asp Glu Asp Ala
305                 310                 315                 320

gat aaa agt gaa gat gaa gaa aaa aag cct aag aca aag aca gtg aag 1008
Asp Lys Ser Glu Asp Glu Glu Lys Lys Pro Lys Thr Lys Thr Val Lys
                325                 330                 335

gaa aca act tat gag tgg gaa ctt tta aat gat gtg aaa gct ata tgg 1056
Glu Thr Thr Tyr Glu Trp Glu Leu Leu Asn Asp Val Lys Ala Ile Trp
            340                 345                 350

ctg agg aat cca aaa gag gtt aca gaa gaa gaa tac acc aaa ttc tac 1104
Leu Arg Asn Pro Lys Glu Val Thr Glu Glu Glu Tyr Thr Lys Phe Tyr
        355                 360                 365

cac tct ctt gcc aag gat ttc agt gat gag aaa cct tta gca tgg agt 1152
His Ser Leu Ala Lys Asp Phe Ser Asp Glu Lys Pro Leu Ala Trp Ser
    370                 375                 380

cat ttc act gct gag ggt gat gtt gag ttc aag gca gtc ctg ttt gta 1200
His Phe Thr Ala Glu Gly Asp Val Glu Phe Lys Ala Val Leu Phe Val
385                 390                 395                 400

cca cct aag gca cct caa gat tta tat gag agc tat tat aac gca aac 1248
Pro Pro Lys Ala Pro Gln Asp Leu Tyr Glu Ser Tyr Tyr Asn Ala Asn
                405                 410                 415

aaa tcg aac ctg aag ttg tat gtt aga cgt gtg ttc att tca gat gaa 1296
Lys Ser Asn Leu Lys Leu Tyr Val Arg Arg Val Phe Ile Ser Asp Glu
            420                 425                 430

ttt aat gaa ctg ttg ccc aag tac cta aac ttt ttg ctg ggt ctt gtt 1344
Phe Asn Glu Leu Leu Pro Lys Tyr Leu Asn Phe Leu Leu Gly Leu Val
        435                 440                 445

gat tct gac act ttg cca ctt aat gta tca cga gaa atg ctt caa caa 1392
Asp Ser Asp Thr Leu Pro Leu Asn Val Ser Arg Glu Met Leu Gln Gln
    450                 455                 460

cat agt agt ctg aag aca atc aag aag aaa ctt att agg aaa gcc ctt 1440
His Ser Ser Leu Lys Thr Ile Lys Lys Lys Leu Ile Arg Lys Ala Leu
465                 470                 475                 480

gat atg atc cgc agg att gct gat gag gat cct gac gag tca act gac 1488
Asp Met Ile Arg Arg Ile Ala Asp Glu Asp Pro Asp Glu Ser Thr Asp
                485                 490                 495

aaa gaa aag aaa gat gca tcc tct gac aat gat gag aag aaa ggt caa 1536
Lys Glu Lys Lys Asp Ala Ser Ser Asp Asn Asp Glu Lys Lys Gly Gln
            500                 505                 510

tat tct aag ttc tgg aat gag ttt ggt aaa tcc att aaa ctt ggt atc 1584
Tyr Ser Lys Phe Trp Asn Glu Phe Gly Lys Ser Ile Lys Leu Gly Ile

515                 520                 525

atc gag gat gcc acc aac aga aat cgt ttg gca aaa ctg ctc aga ttt      1632
Ile Glu Asp Ala Thr Asn Arg Asn Arg Leu Ala Lys Leu Leu Arg Phe
    530                 535                 540

gag agc acc aag tct gag ggt aaa ttg aca tct ctg gat cag tac ata      1680
Glu Ser Thr Lys Ser Glu Gly Lys Leu Thr Ser Leu Asp Gln Tyr Ile
545                 550                 555                 560

tct aga atg aaa act ggg cag aag gat atc ttt tac ata act gga acc      1728
Ser Arg Met Lys Thr Gly Gln Lys Asp Ile Phe Tyr Ile Thr Gly Thr
                565                 570                 575

agc aaa gaa caa ctg gaa aac tct ccc ttc ctt gag cgg ctt aag aag      1776
Ser Lys Glu Gln Leu Glu Asn Ser Pro Phe Leu Glu Arg Leu Lys Lys
            580                 585                 590

aaa aat ttt gag gtt att ttc ttc acg gat cca gtt gat gag tac ttg      1824
Lys Asn Phe Glu Val Ile Phe Phe Thr Asp Pro Val Asp Glu Tyr Leu
        595                 600                 605

atg caa tac ctg atg gat tat gaa gac aag aag ttc caa aat gtg tcc      1872
Met Gln Tyr Leu Met Asp Tyr Glu Asp Lys Lys Phe Gln Asn Val Ser
    610                 615                 620

aag gag ggt ttg aaa ctt ggg aaa gat tca aaa gac aag gaa ctg aag      1920
Lys Glu Gly Leu Lys Leu Gly Lys Asp Ser Lys Asp Lys Glu Leu Lys
625                 630                 635                 640

gaa tct ttt aag gat ctt acc aag tgg tgg aaa act gct ctt tcc aaa      1968
Glu Ser Phe Lys Asp Leu Thr Lys Trp Trp Lys Thr Ala Leu Ser Lys
                645                 650                 655

gac aat gtt gat gat gtg aag att tcc aac cga tta gat aac act cct      2016
Asp Asn Val Asp Asp Val Lys Ile Ser Asn Arg Leu Asp Asn Thr Pro
            660                 665                 670

tgt gta gtg gtg aca tca aaa ttt ggt tgg agt gct aac atg gag aga      2064
Cys Val Val Val Thr Ser Lys Phe Gly Trp Ser Ala Asn Met Glu Arg
        675                 680                 685

atc atg cag tct caa act ttg tca gat gct agc aag caa gca tac atg      2112
Ile Met Gln Ser Gln Thr Leu Ser Asp Ala Ser Lys Gln Ala Tyr Met
    690                 695                 700

cgc ggc aag agg gtt ctt gag atc aat cct agg cac cct att atc aag      2160
Arg Gly Lys Arg Val Leu Glu Ile Asn Pro Arg His Pro Ile Ile Lys
705                 710                 715                 720

gag ctc cga gag aga gta gta aag aac cct gag gat gag ggt gtg aag      2208
Glu Leu Arg Glu Arg Val Val Lys Asn Pro Glu Asp Glu Gly Val Lys
                725                 730                 735

cat aca gca cag ctg atg tac cag act gca ctc ttt gaa agc ggc ttc      2256
His Thr Ala Gln Leu Met Tyr Gln Thr Ala Leu Phe Glu Ser Gly Phe
            740                 745                 750

ctg ctt gat gat ccc aag gat ttt gct tcc cgg att tat gat tca gtg      2304
Leu Leu Asp Asp Pro Lys Asp Phe Ala Ser Arg Ile Tyr Asp Ser Val
        755                 760                 765

aag act agc ctg gac atc agt cct gaa gca aca gtt gag gag gaa gat      2352

Lys Thr Ser Leu Asp Ile Ser Pro Glu Ala Thr Val Glu Glu Glu Asp
    770                 775                 780

gac act gaa gta gag gct gaa agt gac gcg aaa gaa gat tca aag cct      2400
Asp Thr Glu Val Glu Ala Glu Ser Asp Ala Lys Glu Asp Ser Lys Pro
785                 790                 795                 800

gaa act gat gct gtt aac gat gat aat gat gtc aag gac gag ttg tag      2448
Glu Thr Asp Ala Val Asn Asp Asp Asn Asp Val Lys Asp Glu Leu
                805                 810                 815

<210>  70
<211>  815
<212>  PRT
<213>  Glycine max

<400>  70

Met Arg Lys Trp Thr Val Ala Ser Ala Leu Leu Leu Leu Ser Leu Leu
1               5                   10                  15

Phe Leu Phe Ala Asp Gln Gly Arg Lys Phe Gln Ala Asn Ala Glu Gly
            20                  25                  30

Asp Ser Asp Glu Leu Val Asp Pro Pro Lys Val Glu Asp Lys Ile Gly
        35                  40                  45

Ala Val Pro His Gly Leu Ser Thr Asp Ser Asp Val Val Lys Arg Glu
    50                  55                  60

Ala Glu Ser Ile Ser Lys Arg Ser Leu Arg Ser Asn Ala Glu Lys Phe
65                  70                  75                  80

Glu Phe Gln Ala Glu Val Ser Arg Leu Met Asp Ile Ile Ile Asn Ser
                85                  90                  95

Leu Tyr Ser Asn Lys Asp Ile Phe Leu Arg Glu Leu Ile Ser Asn Ala
            100                 105                 110

Ser Asp Ala Leu Asp Lys Ile Arg Phe Leu Ser Leu Thr Asp Lys Glu
        115                 120                 125

Val Leu Gly Glu Gly Asp Asn Thr Lys Leu Asp Ile Gln Ile Lys Leu
    130                 135                 140

Asp Lys Glu Lys Lys Ile Leu Ser Ile Arg Asp Arg Gly Ile Gly Met
145                 150                 155                 160

Thr Lys Glu Asp Leu Ile Lys Asn Leu Gly Thr Ile Ala Lys Ser Gly
                165                 170                 175

Thr Ser Ala Phe Val Glu Lys Met Gln Thr Ser Gly Asp Leu Asn Leu
            180                 185                 190

Ile Gly Gln Phe Gly Val Gly Phe Tyr Ser Val Tyr Leu Val Ala Asp
        195                 200                 205

Tyr Val Glu Val Ile Ser Lys Asn Asn Asp Asp Lys Gln Tyr Val Trp
    210                 215                 220

Glu Ser Lys Ala Asp Gly Ala Phe Ala Ile Ser Glu Asp Thr Trp Asn
225                 230                 235                 240

Glu Pro Leu Gly Arg Gly Thr Glu Ile Arg Leu His Leu Lys Glu Glu
                245                 250                 255

Ala Gly Glu Tyr Leu Glu Glu Ser Lys Leu Lys Glu Leu Val Lys Arg
            260                 265                 270

Tyr Ser Glu Phe Ile Asn Phe Pro Ile Tyr Ile Trp Ala Ser Lys Glu
        275                 280                 285

Val Asp Val Glu Val Pro Ala Asp Glu Asp Asp Ser Ser Asp Glu Glu
    290                 295                 300

Asp Ser Ser Glu Ser Ser Ser Lys Glu Glu Ser Glu Asp Glu Asp Ala
305                 310                 315                 320

Asp Lys Ser Glu Asp Glu Glu Lys Lys Pro Lys Thr Lys Thr Val Lys
                325                 330                 335

Glu Thr Thr Tyr Glu Trp Glu Leu Leu Asn Asp Val Lys Ala Ile Trp
            340                 345                 350

Leu Arg Asn Pro Lys Glu Val Thr Glu Glu Glu Tyr Thr Lys Phe Tyr
        355                 360                 365

His Ser Leu Ala Lys Asp Phe Ser Asp Glu Lys Pro Leu Ala Trp Ser
    370                 375                 380

His Phe Thr Ala Glu Gly Asp Val Glu Phe Lys Ala Val Leu Phe Val
385                 390                 395                 400

Pro Pro Lys Ala Pro Gln Asp Leu Tyr Glu Ser Tyr Tyr Asn Ala Asn
                405                 410                 415

Lys Ser Asn Leu Lys Leu Tyr Val Arg Arg Val Phe Ile Ser Asp Glu
            420                 425                 430

Phe Asn Glu Leu Leu Pro Lys Tyr Leu Asn Phe Leu Leu Gly Leu Val
        435                 440                 445

Asp Ser Asp Thr Leu Pro Leu Asn Val Ser Arg Glu Met Leu Gln Gln
    450                 455                 460

His Ser Ser Leu Lys Thr Ile Lys Lys Lys Leu Ile Arg Lys Ala Leu
465                 470                 475                 480

Asp Met Ile Arg Arg Ile Ala Asp Glu Asp Pro Asp Glu Ser Thr Asp
                485                 490                 495

Lys Glu Lys Lys Asp Ala Ser Ser Asp Asn Asp Glu Lys Lys Gly Gln
            500                 505                 510

Tyr Ser Lys Phe Trp Asn Glu Phe Gly Lys Ser Ile Lys Leu Gly Ile
        515                 520                 525

Ile Glu Asp Ala Thr Asn Arg Asn Arg Leu Ala Lys Leu Leu Arg Phe
    530                 535                 540

Glu Ser Thr Lys Ser Glu Gly Lys Leu Thr Ser Leu Asp Gln Tyr Ile
545                 550                 555                 560

Ser Arg Met Lys Thr Gly Gln Lys Asp Ile Phe Tyr Ile Thr Gly Thr
                565                 570                 575

Ser Lys Glu Gln Leu Glu Asn Ser Pro Phe Leu Glu Arg Leu Lys Lys
            580                 585                 590

Lys Asn Phe Glu Val Ile Phe Phe Thr Asp Pro Val Asp Glu Tyr Leu
        595                 600                 605

Met Gln Tyr Leu Met Asp Tyr Glu Asp Lys Lys Phe Gln Asn Val Ser
    610                 615                 620

Lys Glu Gly Leu Lys Leu Gly Lys Asp Ser Lys Asp Lys Glu Leu Lys
625                 630                 635                 640

Glu Ser Phe Lys Asp Leu Thr Lys Trp Trp Lys Thr Ala Leu Ser Lys
                645                 650                 655

Asp Asn Val Asp Asp Val Lys Ile Ser Asn Arg Leu Asp Asn Thr Pro
            660                 665                 670

Cys Val Val Val Thr Ser Lys Phe Gly Trp Ser Ala Asn Met Glu Arg
        675                 680                 685

Ile Met Gln Ser Gln Thr Leu Ser Asp Ala Ser Lys Gln Ala Tyr Met
    690                 695                 700

Arg Gly Lys Arg Val Leu Glu Ile Asn Pro Arg His Pro Ile Ile Lys
705                 710                 715                 720

Glu Leu Arg Glu Arg Val Val Lys Asn Pro Glu Asp Glu Gly Val Lys
                725                 730                 735

His Thr Ala Gln Leu Met Tyr Gln Thr Ala Leu Phe Glu Ser Gly Phe
            740                 745                 750

Leu Leu Asp Asp Pro Lys Asp Phe Ala Ser Arg Ile Tyr Asp Ser Val
        755                 760                 765

Lys Thr Ser Leu Asp Ile Ser Pro Glu Ala Thr Val Glu Glu Glu Asp
    770                 775                 780

Asp Thr Glu Val Glu Ala Glu Ser Asp Ala Lys Glu Asp Ser Lys Pro
785                 790                 795                 800

Glu Thr Asp Ala Val Asn Asp Asp Asn Asp Val Lys Asp Glu Leu
                805                 810                 815

<210>  71
<211>  14
<212>  PRT
<213>  Glycine max

<400>  71

Ala Pro Gln Asp Leu Tyr Glu Ser Tyr Tyr Asn Ala Asn Lys
1               5                   10

<210>  72
<211>  8
<212>  PRT
<213>  Glycine max

<400>  72

Ala Val Leu Phe Val Pro Pro Lys
1               5

<210>  73
<211>  11
<212>  PRT
<213>  Glycine max

<400>  73

Asp Phe Ala Ser Arg Ile Tyr Asp Ser Val Lys
1               5                   10

<210>  74
<211>  17
<212>  PRT
<213>  Glycine max

<400>  74

Asp Ile Phe Leu Arg Glu Leu Ile Ser Asn Ala Ser Asp Ala Leu Asp
1               5                   10                  15

Lys

<210>  75
<211>  10
<212>  PRT
<213>  Glycine max

<400>  75

Asp Ile Phe Tyr Ile Thr Gly Thr Ser Lys
1               5                   10

<210>  76
<211>  7
<212>  PRT
<213>  Glycine max

<400>  76

Asp Asn Val Asp Asp Val Lys
1               5

<210>  77
<211>  17
<212>  PRT
<213>  Glycine max

<400>  77

Glu Asp Ser Lys Pro Glu Thr Asp Ala Val Asn Asp Asp Asn Asp Val
1               5                   10                  15

Lys

<210>  78
<211>  11
<212>  PRT
<213>  Glycine max

<400>  78

Glu Glu Ala Gly Glu Tyr Leu Glu Glu Ser Lys
1               5                   10

<210>  79
<211>  13
<212>  PRT
<213>  Glycine max

<400>  79

Glu Gln Leu Glu Asn Ser Pro Phe Leu Glu Arg Leu Lys
1               5                   10

<210>  80
<211>  10
<212>  PRT
<213>  Glycine max

<400>  80

Glu Val Leu Gly Glu Gly Asp Asn Thr Lys
1               5                   10

<210>  81
<211>  9
<212>  PRT
<213>  Glycine max

<400>  81

Glu Val Thr Glu Glu Glu Tyr Thr Lys
1               5

<210>  82
<211>  7
<212>  PRT
<213>  Glycine max

<400>  82

Phe Tyr His Ser Leu Ala Lys
1               5

<210>  83
<211>  16
<212>  PRT
<213>  Glycine max

<400>  83

Ile Gly Ala Val Pro His Gly Leu Ser Thr Asp Ser Asp Val Val Lys
1               5                   10                  15

<210>  84
<211>  15
<212>  PRT
<213>  Glycine max

<400>  84

Leu Gly Ile Ile Glu Asp Ala Thr Asn Arg Asn Arg Leu Ala Lys
1               5                   10                  15


<210>  85
<211>  8
<212>  PRT
<213>  Glycine max

<400>  85

Asn Pro Glu Asp Glu Gly Val Lys
1               5


<210>  86
<211>  9
<212>  PRT
<213>  Glycine max

<400>  86

Ser Gly Thr Ser Ala Phe Val Glu Lys
1               5


<210>  87
<211>  26
<212>  PRT
<213>  Glycine max

<400>  87

Thr Ser Leu Asp Ile Ser Pro Glu Ala Thr Val Glu Glu Glu Asp Asp
1               5                   10                  15


Thr Glu Val Glu Ala Glu Ser Asp Ala Lys
            20                  25


<210>  88
<211>  2442
<212>  DNA
<213>  Glycine max


<220>
<221>  CDS
<222>  (1)..(2442)

<400>  88
atg agg aag tgg acg gtt gct tcc gct ctc ctt ctt ctc tct ctc ctc       48
Met Arg Lys Trp Thr Val Ala Ser Ala Leu Leu Leu Leu Ser Leu Leu
1               5                   10                  15

ttt ctc ttt gca gat caa ggt cga aag ttt cag gcg aat gcc gag gga       96
Phe Leu Phe Ala Asp Gln Gly Arg Lys Phe Gln Ala Asn Ala Glu Gly
            20                  25                  30

gat tcc gac gag ctt gta gat cct ccg aag gtg gag gac aag atc ggt 144
Asp Ser Asp Glu Leu Val Asp Pro Pro Lys Val Glu Asp Lys Ile Gly
        35                  40                  45

gct gtg cct cat ggc ctt tcc acc gat tca gat gtc gtc aag agg gag 192
Ala Val Pro His Gly Leu Ser Thr Asp Ser Asp Val Val Lys Arg Glu
    50                  55                  60

tcg gag tcg atc tcg aag aga tct ctc cgg agc aat gcg gag aag ttt 240
Ser Glu Ser Ile Ser Lys Arg Ser Leu Arg Ser Asn Ala Glu Lys Phe
65                  70                  75                  80

gag ttc caa gcg gaa gtg tca cgg ctt atg gat att atc atc aat tct 288
Glu Phe Gln Ala Glu Val Ser Arg Leu Met Asp Ile Ile Ile Asn Ser
                85                  90                  95

ctc tac agt aac aag gat atc ttc ctc aga gag ttg att tcc aat gct 336
Leu Tyr Ser Asn Lys Asp Ile Phe Leu Arg Glu Leu Ile Ser Asn Ala
            100                 105                 110

tct gat gct ttg gac aag att agg ttc ctt tcc ctc acg gat aag gac 384
Ser Asp Ala Leu Asp Lys Ile Arg Phe Leu Ser Leu Thr Asp Lys Asp
        115                 120                 125

gtt ttg gga gaa ggt gat aac acc aag ctt gat att cag att aag ttg 432
Val Leu Gly Glu Gly Asp Asn Thr Lys Leu Asp Ile Gln Ile Lys Leu
    130                 135                 140

gac aag gaa aag aaa att ctc tca att cga gac aga ggt att ggt atg 480
Asp Lys Glu Lys Lys Ile Leu Ser Ile Arg Asp Arg Gly Ile Gly Met
145                 150                 155                 160

acg aag gag gat ttg ata aag aat ttg ggg aca ata gca aaa tct gga 528
Thr Lys Glu Asp Leu Ile Lys Asn Leu Gly Thr Ile Ala Lys Ser Gly
                165                 170                 175

act tct gca ttt gtt gag aag atg caa aca agt gga gat ctc aat ctg 576
Thr Ser Ala Phe Val Glu Lys Met Gln Thr Ser Gly Asp Leu Asn Leu
            180                 185                 190

att ggg cag ttt gga gtc ggc ttc tac tct gtt tat ctc gtg gct gac 624
Ile Gly Gln Phe Gly Val Gly Phe Tyr Ser Val Tyr Leu Val Ala Asp
        195                 200                 205

tat gtc gaa gtc att agc aag aat aat gat gat aaa cag tat gta tgg 672
Tyr Val Glu Val Ile Ser Lys Asn Asn Asp Asp Lys Gln Tyr Val Trp
    210                 215                 220

gaa tca aaa gct gat gga gca ttt gca att tca gaa gat aca tgg aat 720
Glu Ser Lys Ala Asp Gly Ala Phe Ala Ile Ser Glu Asp Thr Trp Asn
225                 230                 235                 240

gaa cct cta ggg cgt gga act gag att aga tta cac ctc aag gaa gag 768
Glu Pro Leu Gly Arg Gly Thr Glu Ile Arg Leu His Leu Lys Glu Glu
                245                 250                 255

gct ggg gag tac ttg cag gag tct aaa ctg aaa gaa ttg gtg aag aga 816
Ala Gly Glu Tyr Leu Gln Glu Ser Lys Leu Lys Glu Leu Val Lys Arg
            260                 265                 270

tac tct gaa ttt att aac ttc cct atc tat att tgg gca agc aaa gag 864
Tyr Ser Glu Phe Ile Asn Phe Pro Ile Tyr Ile Trp Ala Ser Lys Glu
        275                 280                 285

gtt gat gtg gag gtt ccc gct gat gaa gat gat tcc agt gat gaa gag 912
Val Asp Val Glu Val Pro Ala Asp Glu Asp Asp Ser Ser Asp Glu Glu
    290                 295                 300

gat tca tct gaa agc agc tcc aag gag gaa agc gaa gat gaa gat gct 960
Asp Ser Ser Glu Ser Ser Ser Lys Glu Glu Ser Glu Asp Glu Asp Ala
305                 310                 315                 320

gat aaa agt gaa gac gaa gaa aaa aag ccc aag aca aag acg gtg aag 1008
Asp Lys Ser Glu Asp Glu Glu Lys Lys Pro Lys Thr Lys Thr Val Lys
                325                 330                 335

gaa aca act tat gaa tgg gaa ctt tta aat gat gtg aaa gct ata tgg 1056
Glu Thr Thr Tyr Glu Trp Glu Leu Leu Asn Asp Val Lys Ala Ile Trp
            340                 345                 350

ctg agg aat cca aag gag gtt aca gaa gaa gaa tac acc aaa ttc tac 1104
Leu Arg Asn Pro Lys Glu Val Thr Glu Glu Glu Tyr Thr Lys Phe Tyr
        355                 360                 365

cac tct ctt gcc aag gat ttc agt gat gag aaa cct tta gca tgg agt 1152
His Ser Leu Ala Lys Asp Phe Ser Asp Glu Lys Pro Leu Ala Trp Ser
    370                 375                 380

cat ttc act gct gag ggc gat gtc gag ttc aag gca gtc ctg ttt gta 1200
His Phe Thr Ala Glu Gly Asp Val Glu Phe Lys Ala Val Leu Phe Val
385                 390                 395                 400

cca cct aag gca cct caa gat tta tac gag agc tat tat aac gca aac 1248
Pro Pro Lys Ala Pro Gln Asp Leu Tyr Glu Ser Tyr Tyr Asn Ala Asn
                405                 410                 415

aaa tcc aac ctg aag ttg tat gtt aga cgt gtg ttc att tca gat gaa 1296
Lys Ser Asn Leu Lys Leu Tyr Val Arg Arg Val Phe Ile Ser Asp Glu
            420                 425                 430

ttt aat gaa ctg ttg cct aag tac cta aac ttt ttg ctg ggt ctt gtt 1344
Phe Asn Glu Leu Leu Pro Lys Tyr Leu Asn Phe Leu Leu Gly Leu Val
        435                 440                 445

gat tct gac aca ttg cca ctt aat gta tcg cga gaa atg ctt cag caa 1392
Asp Ser Asp Thr Leu Pro Leu Asn Val Ser Arg Glu Met Leu Gln Gln
    450                 455                 460

cat agt agt cta aag aca atc aag aag aaa ctt att agg aaa gcc ctt 1440
His Ser Ser Leu Lys Thr Ile Lys Lys Lys Leu Ile Arg Lys Ala Leu
465                 470                 475                 480

gat atg atc cgt agg att gct gat gag gat cct gat gag tcc act gac 1488
Asp Met Ile Arg Arg Ile Ala Asp Glu Asp Pro Asp Glu Ser Thr Asp
                485                 490                 495

aaa gaa aag aaa gat acg tca tct gac aat aat gag aag aaa ggt caa 1536
Lys Glu Lys Lys Asp Thr Ser Ser Asp Asn Asn Glu Lys Lys Gly Gln
            500                 505                 510

tat tcc aag ttc tgg aat gag ttt ggt aaa tcc att aaa ctt ggt atc 1584
Tyr Ser Lys Phe Trp Asn Glu Phe Gly Lys Ser Ile Lys Leu Gly Ile
        515                 520                 525

atc gag gat gcc acc aac aga aat cgt ttg gca aaa ctg ctc aga ttt 1632
Ile Glu Asp Ala Thr Asn Arg Asn Arg Leu Ala Lys Leu Leu Arg Phe

530                 535                 540

gag agc acc aag tct gag ggt aaa ttg aca tct ctg gat cag tac ata         1680
Glu Ser Thr Lys Ser Glu Gly Lys Leu Thr Ser Leu Asp Gln Tyr Ile
545                 550                 555                 560

tct aga atg aaa gct ggg cag aag gat atc ttt tac ata act gga acc         1728
Ser Arg Met Lys Ala Gly Gln Lys Asp Ile Phe Tyr Ile Thr Gly Thr
                565                 570                 575

agc aaa gaa caa ctg gaa aac tct cca ttc ctt gag cgg ctt aag aag         1776
Ser Lys Glu Gln Leu Glu Asn Ser Pro Phe Leu Glu Arg Leu Lys Lys
            580                 585                 590

aaa aat ttt gag gtt att ttc ttc acg gat cca gtt gat gaa tac ttg         1824
Lys Asn Phe Glu Val Ile Phe Phe Thr Asp Pro Val Asp Glu Tyr Leu
        595                 600                 605

atg caa tac ctg atg gat tat gaa gac aag aag ttc caa aat gtg tcc         1872
Met Gln Tyr Leu Met Asp Tyr Glu Asp Lys Lys Phe Gln Asn Val Ser
    610                 615                 620

aag gag ggt ttg aaa ctc ggg aaa gat tca aaa gac aag gaa ctg aag         1920
Lys Glu Gly Leu Lys Leu Gly Lys Asp Ser Lys Asp Lys Glu Leu Lys
625                 630                 635                 640

gaa tcc ttc aag gac ctt acc aag tgg tgg aaa act gct ctt tcc aaa         1968
Glu Ser Phe Lys Asp Leu Thr Lys Trp Trp Lys Thr Ala Leu Ser Lys
                645                 650                 655

gac aat gtt gat gat gtg aag att tcc aac cga ttg gac aac act cct         2016
Asp Asn Val Asp Asp Val Lys Ile Ser Asn Arg Leu Asp Asn Thr Pro
            660                 665                 670

tgt gta gtg gta aca tca aaa ttt ggt tgg agt gct aac atg gag aga         2064
Cys Val Val Val Thr Ser Lys Phe Gly Trp Ser Ala Asn Met Glu Arg
        675                 680                 685

atc atg cag tct caa act ttg tct gat gct agc aag caa gca tac atg         2112
Ile Met Gln Ser Gln Thr Leu Ser Asp Ala Ser Lys Gln Ala Tyr Met
    690                 695                 700

cgc ggc aag agg gtt ctt gag gtc aat cct agg cac cct att atc aag         2160
Arg Gly Lys Arg Val Leu Glu Val Asn Pro Arg His Pro Ile Ile Lys
705                 710                 715                 720

gag ctc cga gag aga gta gta aag aac cct gag gat gag ggt gtg aaa         2208
Glu Leu Arg Glu Arg Val Val Lys Asn Pro Glu Asp Glu Gly Val Lys
                725                 730                 735

cat aca gca cag ttg atg tac cag acc gca ctc ttc gaa agt ggc ttc         2256
His Thr Ala Gln Leu Met Tyr Gln Thr Ala Leu Phe Glu Ser Gly Phe
            740                 745                 750

ctg ctt gat gat ccc aag gat ttt gct tcc cgt att tat gat tca gtt         2304
Leu Leu Asp Asp Pro Lys Asp Phe Ala Ser Arg Ile Tyr Asp Ser Val
        755                 760                 765

aag acc agc ttg gac atc agt cct gaa gca aca gtt gag gag gaa gat         2352
Lys Thr Ser Leu Asp Ile Ser Pro Glu Ala Thr Val Glu Glu Glu Asp
    770                 775                 780

gac acc gaa gta gag gct gaa agt gat tca aag cct gaa gct gat gcg         2400

Asp Thr Glu Val Glu Ala Glu Ser Asp Ser Lys Pro Glu Ala Asp Ala
785                 790                 795                 800

gtt cat gat ggt gat gtt gat gat gcc aag gac gag ttg tag                    2442
Val His Asp Gly Asp Val Asp Asp Ala Lys Asp Glu Leu
                805                 810

<210>  89
<211>  813
<212>  PRT
<213>  Glycine max

<400>  89

Met Arg Lys Trp Thr Val Ala Ser Ala Leu Leu Leu Leu Ser Leu Leu
1               5                   10                  15

Phe Leu Phe Ala Asp Gln Gly Arg Lys Phe Gln Ala Asn Ala Glu Gly
            20                  25                  30

Asp Ser Asp Glu Leu Val Asp Pro Pro Lys Val Glu Asp Lys Ile Gly
        35                  40                  45

Ala Val Pro His Gly Leu Ser Thr Asp Ser Asp Val Val Lys Arg Glu
    50                  55                  60

Ser Glu Ser Ile Ser Lys Arg Ser Leu Arg Ser Asn Ala Glu Lys Phe
65                  70                  75                  80

Glu Phe Gln Ala Glu Val Ser Arg Leu Met Asp Ile Ile Ile Asn Ser
                85                  90                  95

Leu Tyr Ser Asn Lys Asp Ile Phe Leu Arg Glu Leu Ile Ser Asn Ala
            100                 105                 110

Ser Asp Ala Leu Asp Lys Ile Arg Phe Leu Ser Leu Thr Asp Lys Asp
        115                 120                 125

Val Leu Gly Glu Gly Asp Asn Thr Lys Leu Asp Ile Gln Ile Lys Leu
    130                 135                 140

Asp Lys Glu Lys Lys Ile Leu Ser Ile Arg Asp Arg Gly Ile Gly Met
145                 150                 155                 160

Thr Lys Glu Asp Leu Ile Lys Asn Leu Gly Thr Ile Ala Lys Ser Gly
                165                 170                 175

Thr Ser Ala Phe Val Glu Lys Met Gln Thr Ser Gly Asp Leu Asn Leu
            180                 185                 190

Ile Gly Gln Phe Gly Val Gly Phe Tyr Ser Val Tyr Leu Val Ala Asp
        195                 200                 205

Tyr Val Glu Val Ile Ser Lys Asn Asn Asp Asp Lys Gln Tyr Val Trp
    210                 215                 220

Glu Ser Lys Ala Asp Gly Ala Phe Ala Ile Ser Glu Asp Thr Trp Asn
225                 230                 235                 240

Glu Pro Leu Gly Arg Gly Thr Glu Ile Arg Leu His Leu Lys Glu Glu
                245                 250                 255

Ala Gly Glu Tyr Leu Gln Glu Ser Lys Leu Lys Glu Leu Val Lys Arg
            260                 265                 270

Tyr Ser Glu Phe Ile Asn Phe Pro Ile Tyr Ile Trp Ala Ser Lys Glu
        275                 280                 285

Val Asp Val Glu Val Pro Ala Asp Glu Asp Asp Ser Ser Asp Glu Glu
    290                 295                 300

Asp Ser Ser Glu Ser Ser Ser Lys Glu Glu Ser Glu Asp Glu Asp Ala
305                 310                 315                 320

Asp Lys Ser Glu Asp Glu Glu Lys Lys Pro Lys Thr Lys Thr Val Lys
                325                 330                 335

Glu Thr Thr Tyr Glu Trp Glu Leu Leu Asn Asp Val Lys Ala Ile Trp
            340                 345                 350

Leu Arg Asn Pro Lys Glu Val Thr Glu Glu Glu Tyr Thr Lys Phe Tyr
        355                 360                 365

His Ser Leu Ala Lys Asp Phe Ser Asp Glu Lys Pro Leu Ala Trp Ser
    370                 375                 380

His Phe Thr Ala Glu Gly Asp Val Glu Phe Lys Ala Val Leu Phe Val
385                 390                 395                 400

Pro Pro Lys Ala Pro Gln Asp Leu Tyr Glu Ser Tyr Tyr Asn Ala Asn
                405                 410                 415

Lys Ser Asn Leu Lys Leu Tyr Val Arg Arg Val Phe Ile Ser Asp Glu
            420                 425                 430

Phe Asn Glu Leu Leu Pro Lys Tyr Leu Asn Phe Leu Leu Gly Leu Val
        435                 440                 445

Asp Ser Asp Thr Leu Pro Leu Asn Val Ser Arg Glu Met Leu Gln Gln
    450                 455                 460

His Ser Ser Leu Lys Thr Ile Lys Lys Lys Leu Ile Arg Lys Ala Leu
465                 470                 475                 480

Asp Met Ile Arg Arg Ile Ala Asp Glu Asp Pro Asp Glu Ser Thr Asp
                485                 490                 495

Lys Glu Lys Lys Asp Thr Ser Ser Asp Asn Asn Glu Lys Lys Gly Gln
            500                 505                 510

Tyr Ser Lys Phe Trp Asn Glu Phe Gly Lys Ser Ile Lys Leu Gly Ile
        515                 520                 525

Ile Glu Asp Ala Thr Asn Arg Asn Arg Leu Ala Lys Leu Leu Arg Phe
    530                 535                 540

Glu Ser Thr Lys Ser Glu Gly Lys Leu Thr Ser Leu Asp Gln Tyr Ile
545                 550                 555                 560

Ser Arg Met Lys Ala Gly Gln Lys Asp Ile Phe Tyr Ile Thr Gly Thr
                565                 570                 575

Ser Lys Glu Gln Leu Glu Asn Ser Pro Phe Leu Glu Arg Leu Lys Lys
            580                 585                 590

Lys Asn Phe Glu Val Ile Phe Phe Thr Asp Pro Val Asp Glu Tyr Leu
        595                 600                 605

Met Gln Tyr Leu Met Asp Tyr Glu Asp Lys Lys Phe Gln Asn Val Ser
    610                 615                 620

Lys Glu Gly Leu Lys Leu Gly Lys Asp Ser Lys Asp Lys Glu Leu Lys
625                 630                 635                 640

Glu Ser Phe Lys Asp Leu Thr Lys Trp Trp Lys Thr Ala Leu Ser Lys
                645                 650                 655

Asp Asn Val Asp Asp Val Lys Ile Ser Asn Arg Leu Asp Asn Thr Pro
            660                 665                 670

Cys Val Val Val Thr Ser Lys Phe Gly Trp Ser Ala Asn Met Glu Arg
        675                 680                 685

Ile Met Gln Ser Gln Thr Leu Ser Asp Ala Ser Lys Gln Ala Tyr Met
    690                 695                 700

Arg Gly Lys Arg Val Leu Glu Val Asn Pro Arg His Pro Ile Ile Lys
705                 710                 715                 720

Glu Leu Arg Glu Arg Val Val Lys Asn Pro Glu Asp Glu Gly Val Lys
                725                 730                 735

His Thr Ala Gln Leu Met Tyr Gln Thr Ala Leu Phe Glu Ser Gly Phe
            740                 745                 750

Leu Leu Asp Asp Pro Lys Asp Phe Ala Ser Arg Ile Tyr Asp Ser Val
        755                 760                 765

Lys Thr Ser Leu Asp Ile Ser Pro Glu Ala Thr Val Glu Glu Glu Asp
    770                 775                 780

Asp Thr Glu Val Glu Ala Glu Ser Asp Ser Lys Pro Glu Ala Asp Ala
785                 790                 795                 800

Val His Asp Gly Asp Val Asp Asp Ala Lys Asp Glu Leu
                805                 810


<210>  90
<211>  14
<212>  PRT
<213>  Glycine max

<400>  90

Ala Pro Gln Asp Leu Tyr Glu Ser Tyr Tyr Asn Ala Asn Lys
1               5                   10


<210>  91
<211>  8
<212>  PRT
<213>  Glycine max

<400>  91

Ala Val Leu Phe Val Pro Pro Lys
1               5


<210>  92
<211>  11
<212>  PRT
<213>  Glycine max

<400>  92

Asp Phe Ala Ser Arg Ile Tyr Asp Ser Val Lys
1               5                   10

<210>  93
<211>  17
<212>  PRT
<213>  Glycine max

<400>  93

Asp Ile Phe Leu Arg Glu Leu Ile Ser Asn Ala Ser Asp Ala Leu Asp
1               5                   10                  15

Lys

<210>  94
<211>  10
<212>  PRT
<213>  Glycine max

<400>  94

Asp Ile Phe Tyr Ile Thr Gly Thr Ser Lys
1               5                   10

<210>  95
<211>  7
<212>  PRT
<213>  Glycine max

<400>  95

Asp Asn Val Asp Asp Val Lys
1               5

<210>  96
<211>  10
<212>  PRT
<213>  Glycine max

<400>  96

Asp Val Leu Gly Glu Gly Asp Asn Thr Lys
1               5                   10

<210>  97
<211>  11
<212>  PRT
<213>  Glycine max

<400>  97

Glu Glu Ala Gly Glu Tyr Leu Gln Glu Ser Lys
1               5                   10

<210>  98
<211>  13
<212>  PRT
<213>  Glycine max

<400>  98

Glu Gln Leu Glu Asn Ser Pro Phe Leu Glu Arg Leu Lys
1               5                   10


<210>  99
<211>  9
<212>  PRT
<213>  Glycine max

<400>  99

Glu Val Thr Glu Glu Glu Tyr Thr Lys
1               5


<210>  100
<211>  7
<212>  PRT
<213>  Glycine max

<400>  100

Phe Tyr His Ser Leu Ala Lys
1               5


<210>  101
<211>  16
<212>  PRT
<213>  Glycine max

<400>  101

Ile Gly Ala Val Pro His Gly Leu Ser Thr Asp Ser Asp Val Val Lys
1               5                   10                  15


<210>  102
<211>  15
<212>  PRT
<213>  Glycine max

<400>  102

Leu Gly Ile Ile Glu Asp Ala Thr Asn Arg Asn Arg Leu Ala Lys
1               5                   10                  15


<210>  103
<211>  8
<212>  PRT
<213>  Glycine max

<400>  103

Asn Pro Glu Asp Glu Gly Val Lys
1               5


<210>  104

<211>  9
<212>  PRT
<213>  Glycine max

<400>  104

Ser Gly Thr Ser Ala Phe Val Glu Lys
1               5


<210>  105
<211>  2103
<212>  DNA
<213>  Glycine max


<220>
<221>  CDS
<222>  (1)..(2103)

<400>  105
atg gct tcg gag act gag acg ttc gct ttt cag gcc gag atc aac cag      48
Met Ala Ser Glu Thr Glu Thr Phe Ala Phe Gln Ala Glu Ile Asn Gln
1               5                   10                  15

ctc ctc agt cta ata atc aac acc ttc tac agc aac aag gag atc ttt      96
Leu Leu Ser Leu Ile Ile Asn Thr Phe Tyr Ser Asn Lys Glu Ile Phe
            20                  25                  30

ctt cgt gag ctc atc agc aat gcc tct gac gct ttg gac aag att cga      144
Leu Arg Glu Leu Ile Ser Asn Ala Ser Asp Ala Leu Asp Lys Ile Arg
        35                  40                  45

ttt gag agt ctt aca gac aag agc aag ctt gat gct cag cca gag ttg      192
Phe Glu Ser Leu Thr Asp Lys Ser Lys Leu Asp Ala Gln Pro Glu Leu
    50                  55                  60

ttc att cac att att cct gac aag acc aac aac aca ttg tct atc att      240
Phe Ile His Ile Ile Pro Asp Lys Thr Asn Asn Thr Leu Ser Ile Ile
65                  70                  75                  80

gac agt ggt att ggc atg act aag gct gat ttg gtg aat aac ctc ggt      288
Asp Ser Gly Ile Gly Met Thr Lys Ala Asp Leu Val Asn Asn Leu Gly
                85                  90                  95

act att gca agg tct ggg acc aag gaa ttc atg gaa gcc ctg gct gct      336
Thr Ile Ala Arg Ser Gly Thr Lys Glu Phe Met Glu Ala Leu Ala Ala
            100                 105                 110

ggt gct gac gtg agc atg att ggg cag ttc ggt gtt ggt ttc tac tcg      384
Gly Ala Asp Val Ser Met Ile Gly Gln Phe Gly Val Gly Phe Tyr Ser
        115                 120                 125

gct tat ctt gta gct gat aag gtt att gtt acc aca aag cac aat gat      432
Ala Tyr Leu Val Ala Asp Lys Val Ile Val Thr Thr Lys His Asn Asp
    130                 135                 140

gat gaa cag tat gtc tgg gag tcc cat gcc ggt ggc tca ttt act gtg      480
Asp Glu Gln Tyr Val Trp Glu Ser His Ala Gly Gly Ser Phe Thr Val
145                 150                 155                 160

aca aga gat aca tct ggt gag aac ctt ggt agg gga aca aag ata act      528
Thr Arg Asp Thr Ser Gly Glu Asn Leu Gly Arg Gly Thr Lys Ile Thr

                165                 170                 175

ttg ttt ctt aag gaa gat caa ctt gag tat ctt gag gaa cgc cgt ctg 576
Leu Phe Leu Lys Glu Asp Gln Leu Glu Tyr Leu Glu Glu Arg Arg Leu
            180                 185                 190

aag gat ttg ata aag aag cat tct gaa ttc att agc tat cca att tct 624
Lys Asp Leu Ile Lys Lys His Ser Glu Phe Ile Ser Tyr Pro Ile Ser
        195                 200                 205

ctc tgg att gag aag act act gaa aag gag att tct gat gat gag gat 672
Leu Trp Ile Glu Lys Thr Thr Glu Lys Glu Ile Ser Asp Asp Glu Asp
    210                 215                 220

gag gaa gag aag aag gat gag gag ggt aaa gtt gaa gat gtg gat gaa 720
Glu Glu Glu Lys Lys Asp Glu Glu Gly Lys Val Glu Asp Val Asp Glu
225                 230                 235                 240

gat aag gag aag gag gag aag aaa aag aag acc att aag gag gta tct 768
Asp Lys Glu Lys Glu Glu Lys Lys Lys Lys Thr Ile Lys Glu Val Ser
                245                 250                 255

cac gag tgg tca ttg gtg aac aag cag aag cct att tgg atg agg aaa 816
His Glu Trp Ser Leu Val Asn Lys Gln Lys Pro Ile Trp Met Arg Lys
            260                 265                 270

cct gaa gag att acg aaa gaa gaa tat gct gct ttc tac aag agt ctt 864
Pro Glu Glu Ile Thr Lys Glu Glu Tyr Ala Ala Phe Tyr Lys Ser Leu
        275                 280                 285

acc aat gat tgg gaa gag cat ttg gct gtt aag cac ttt tct gtt gag 912
Thr Asn Asp Trp Glu Glu His Leu Ala Val Lys His Phe Ser Val Glu
    290                 295                 300

ggt cag ctg gag ttt aag gct gtc ctc ttt atc ccc aag agg gca cct 960
Gly Gln Leu Glu Phe Lys Ala Val Leu Phe Ile Pro Lys Arg Ala Pro
305                 310                 315                 320

ttt gat ctc ttt gac acc agg aag aag cct aac aac atc aaa ctg tat 1008
Phe Asp Leu Phe Asp Thr Arg Lys Lys Pro Asn Asn Ile Lys Leu Tyr
                325                 330                 335

gtc cgc cgt gtc ttc atc atg gac aac tgt gag gag ttg atg cct gaa 1056
Val Arg Arg Val Phe Ile Met Asp Asn Cys Glu Glu Leu Met Pro Glu
            340                 345                 350

tat ctt agc ttt gtt aag ggt att gtg gat tct gag gat ctt cct ctc 1104
Tyr Leu Ser Phe Val Lys Gly Ile Val Asp Ser Glu Asp Leu Pro Leu
        355                 360                 365

aac att tct aga gaa atg ctg cag cag aac aag atc ctg aaa gtc atc 1152
Asn Ile Ser Arg Glu Met Leu Gln Gln Asn Lys Ile Leu Lys Val Ile
    370                 375                 380

agg aag aac ttg gtc aag aag tgc att gag atg ttc ttt gaa att gct 1200
Arg Lys Asn Leu Val Lys Lys Cys Ile Glu Met Phe Phe Glu Ile Ala
385                 390                 395                 400

gaa aac aag gaa gac tat aac aag ttt tat gaa gcc ttc tct aag aac 1248
Glu Asn Lys Glu Asp Tyr Asn Lys Phe Tyr Glu Ala Phe Ser Lys Asn
                405                 410                 415

ctg aaa ctt ggt att cat gag gat tct cag aac aag aca aag cta gct 1296

Leu Lys Leu Gly Ile His Glu Asp Ser Gln Asn Lys Thr Lys Leu Ala
            420                 425                 430

gaa ttg ctc agg tat cac tcc act aag agt ggt gat gag atg acc agc      1344
Glu Leu Leu Arg Tyr His Ser Thr Lys Ser Gly Asp Glu Met Thr Ser
        435                 440                 445

ctc aag gac tat gtt acc agg atg aag gaa gga cag aat gac atc tac      1392
Leu Lys Asp Tyr Val Thr Arg Met Lys Glu Gly Gln Asn Asp Ile Tyr
    450                 455                 460

tac att act ggt gaa agc aag aaa gct gtc gag aat tcc ccc ttc ctt      1440
Tyr Ile Thr Gly Glu Ser Lys Lys Ala Val Glu Asn Ser Pro Phe Leu
465                 470                 475                 480

gaa aag ctt aag aag aag gga tac gag gtt ctc tac atg gtt gat gct      1488
Glu Lys Leu Lys Lys Lys Gly Tyr Glu Val Leu Tyr Met Val Asp Ala
                485                 490                 495

att gat gag tat gct gtt ggc cag ctt aag gaa ttt gag gga aag aag      1536
Ile Asp Glu Tyr Ala Val Gly Gln Leu Lys Glu Phe Glu Gly Lys Lys
            500                 505                 510

ttg gtc tct gct acc aag gaa ggc cta aaa ctt gat gag agc gaa gac      1584
Leu Val Ser Ala Thr Lys Glu Gly Leu Lys Leu Asp Glu Ser Glu Asp
        515                 520                 525

gag aag aaa aag aag gaa gaa cta aag gat aaa ttt gag ggt ctt tgc      1632
Glu Lys Lys Lys Lys Glu Glu Leu Lys Asp Lys Phe Glu Gly Leu Cys
    530                 535                 540

cat gtg atc aag gat gtg ttg ggt gac aag gtg gag aaa gtt gtg gtg      1680
His Val Ile Lys Asp Val Leu Gly Asp Lys Val Glu Lys Val Val Val
545                 550                 555                 560

tct gat cgt gtt gtg gat tct cct tgc tgt ctg gtg aca ggt gaa tat      1728
Ser Asp Arg Val Val Asp Ser Pro Cys Cys Leu Val Thr Gly Glu Tyr
                565                 570                 575

ggg tgg aca gcc aac atg gaa agg att atg aaa gca cag gca ttg agg      1776
Gly Trp Thr Ala Asn Met Glu Arg Ile Met Lys Ala Gln Ala Leu Arg
            580                 585                 590

gac agc agc atg gct gga tac atg tca agc aag aag acg atg gag att      1824
Asp Ser Ser Met Ala Gly Tyr Met Ser Ser Lys Lys Thr Met Glu Ile
        595                 600                 605

aac cct gag aac ccc atc atg gag gag ctg agg aag cga gca gat gct      1872
Asn Pro Glu Asn Pro Ile Met Glu Glu Leu Arg Lys Arg Ala Asp Ala
    610                 615                 620

gat aag aat gac aag tct gtg aag gat ctc gtg ctc ttg ctc ttt gag      1920
Asp Lys Asn Asp Lys Ser Val Lys Asp Leu Val Leu Leu Leu Phe Glu
625                 630                 635                 640

act gct ctt cta act tct ggg ttc agc ctt gat gat ccc aac act ttc      1968
Thr Ala Leu Leu Thr Ser Gly Phe Ser Leu Asp Asp Pro Asn Thr Phe
                645                 650                 655

ggt aac agg att cac agg atg ctg aag ctt gga ctg agc att gat gaa      2016
Gly Asn Arg Ile His Arg Met Leu Lys Leu Gly Leu Ser Ile Asp Glu
            660                 665                 670

gat gct ggt gaa gca gat gct gac atg cct cct ctt gag gac gct gat     2064
Asp Ala Gly Glu Ala Asp Ala Asp Met Pro Pro Leu Glu Asp Ala Asp
        675                 680                 685

gca gat gct gag ggt agc aag atg gaa gaa gtt gat taa                 2103
Ala Asp Ala Glu Gly Ser Lys Met Glu Glu Val Asp
    690                 695                 700

<210>  106
<211>  700
<212>  PRT
<213>  Glycine max

<400>  106

Met Ala Ser Glu Thr Glu Thr Phe Ala Phe Gln Ala Glu Ile Asn Gln
1               5                   10                  15

Leu Leu Ser Leu Ile Ile Asn Thr Phe Tyr Ser Asn Lys Glu Ile Phe
            20                  25                  30

Leu Arg Glu Leu Ile Ser Asn Ala Ser Asp Ala Leu Asp Lys Ile Arg
        35                  40                  45

Phe Glu Ser Leu Thr Asp Lys Ser Lys Leu Asp Ala Gln Pro Glu Leu
    50                  55                  60

Phe Ile His Ile Ile Pro Asp Lys Thr Asn Asn Thr Leu Ser Ile Ile
65                  70                  75                  80

Asp Ser Gly Ile Gly Met Thr Lys Ala Asp Leu Val Asn Asn Leu Gly
                85                  90                  95

Thr Ile Ala Arg Ser Gly Thr Lys Glu Phe Met Glu Ala Leu Ala Ala
            100                 105                 110

Gly Ala Asp Val Ser Met Ile Gly Gln Phe Gly Val Gly Phe Tyr Ser
        115                 120                 125

Ala Tyr Leu Val Ala Asp Lys Val Ile Val Thr Thr Lys His Asn Asp
    130                 135                 140

Asp Glu Gln Tyr Val Trp Glu Ser His Ala Gly Gly Ser Phe Thr Val
145                 150                 155                 160

Thr Arg Asp Thr Ser Gly Glu Asn Leu Gly Arg Gly Thr Lys Ile Thr
                165                 170                 175

Leu Phe Leu Lys Glu Asp Gln Leu Glu Tyr Leu Glu Glu Arg Arg Leu
            180                 185                 190

Lys Asp Leu Ile Lys Lys His Ser Glu Phe Ile Ser Tyr Pro Ile Ser
        195                 200                 205

Leu Trp Ile Glu Lys Thr Thr Glu Lys Glu Ile Ser Asp Asp Glu Asp
    210                 215                 220

Glu Glu Glu Lys Lys Asp Glu Glu Gly Lys Val Glu Asp Val Asp Glu
225                 230                 235                 240

Asp Lys Glu Lys Glu Glu Lys Lys Lys Lys Thr Ile Lys Glu Val Ser
                245                 250                 255

His Glu Trp Ser Leu Val Asn Lys Gln Lys Pro Ile Trp Met Arg Lys
            260                 265                 270

Pro Glu Glu Ile Thr Lys Glu Glu Tyr Ala Ala Phe Tyr Lys Ser Leu
        275                 280                 285

Thr Asn Asp Trp Glu Glu His Leu Ala Val Lys His Phe Ser Val Glu
    290                 295                 300

Gly Gln Leu Glu Phe Lys Ala Val Leu Phe Ile Pro Lys Arg Ala Pro
305                 310                 315                 320

Phe Asp Leu Phe Asp Thr Arg Lys Lys Pro Asn Asn Ile Lys Leu Tyr
                325                 330                 335

Val Arg Arg Val Phe Ile Met Asp Asn Cys Glu Glu Leu Met Pro Glu
            340                 345                 350

Tyr Leu Ser Phe Val Lys Gly Ile Val Asp Ser Glu Asp Leu Pro Leu
        355                 360                 365

Asn Ile Ser Arg Glu Met Leu Gln Gln Asn Lys Ile Leu Lys Val Ile
    370                 375                 380

Arg Lys Asn Leu Val Lys Lys Cys Ile Glu Met Phe Phe Glu Ile Ala
385                 390                 395                 400

Glu Asn Lys Glu Asp Tyr Asn Lys Phe Tyr Glu Ala Phe Ser Lys Asn
                405                 410                 415

Leu Lys Leu Gly Ile His Glu Asp Ser Gln Asn Lys Thr Lys Leu Ala
            420                 425                 430

Glu Leu Leu Arg Tyr His Ser Thr Lys Ser Gly Asp Glu Met Thr Ser
        435                 440                 445

Leu Lys Asp Tyr Val Thr Arg Met Lys Glu Gly Gln Asn Asp Ile Tyr
    450                 455                 460

Tyr Ile Thr Gly Glu Ser Lys Lys Ala Val Glu Asn Ser Pro Phe Leu
465                 470                 475                 480

Glu Lys Leu Lys Lys Lys Gly Tyr Glu Val Leu Tyr Met Val Asp Ala
                485                 490                 495

Ile Asp Glu Tyr Ala Val Gly Gln Leu Lys Glu Phe Glu Gly Lys Lys
            500                 505                 510

Leu Val Ser Ala Thr Lys Glu Gly Leu Lys Leu Asp Glu Ser Glu Asp
        515                 520                 525

Glu Lys Lys Lys Lys Glu Glu Leu Lys Asp Lys Phe Glu Gly Leu Cys
    530                 535                 540

His Val Ile Lys Asp Val Leu Gly Asp Lys Val Glu Lys Val Val Val
545                 550                 555                 560

Ser Asp Arg Val Val Asp Ser Pro Cys Cys Leu Val Thr Gly Glu Tyr
                565                 570                 575

Gly Trp Thr Ala Asn Met Glu Arg Ile Met Lys Ala Gln Ala Leu Arg
            580                 585                 590

Asp Ser Ser Met Ala Gly Tyr Met Ser Ser Lys Lys Thr Met Glu Ile
        595                 600                 605

Asn Pro Glu Asn Pro Ile Met Glu Glu Leu Arg Lys Arg Ala Asp Ala
    610                 615                 620

Asp Lys Asn Asp Lys Ser Val Lys Asp Leu Val Leu Leu Leu Phe Glu
625                 630                 635                 640

Thr Ala Leu Leu Thr Ser Gly Phe Ser Leu Asp Asp Pro Asn Thr Phe
                645                 650                 655

Gly Asn Arg Ile His Arg Met Leu Lys Leu Gly Leu Ser Ile Asp Glu
            660                 665                 670

Asp Ala Gly Glu Ala Asp Ala Asp Met Pro Pro Leu Glu Asp Ala Asp
        675                 680                 685

Ala Asp Ala Glu Gly Ser Lys Met Glu Glu Val Asp

690                 695                 700

<210>  107
<211>  16
<212>  PRT
<213>  Glycine max

<400>  107

Ala Asp Leu Val Asn Asn Leu Gly Thr Ile Ala Arg Ser Gly Thr Lys
1               5                   10                  15

<210>  108
<211>  10
<212>  PRT
<213>  Glycine max

<400>  108

Ala Val Glu Asn Ser Pro Phe Leu Glu Lys
1               5                   10

<210>  109
<211>  13
<212>  PRT
<213>  Glycine max

<400>  109

Glu Asp Gln Leu Glu Tyr Leu Glu Glu Arg Arg Leu Lys
1               5                   10

<210>  110
<211>  8
<212>  PRT
<213>  Glycine max

<400>  110

Glu Glu Tyr Ala Ala Phe Tyr Lys
1               5

<210>  111
<211>  14
<212>  PRT
<213>  Glycine max

<400>  111

Glu Gly Gln Asn Asp Ile Tyr Tyr Ile Thr Gly Glu Ser Lys
1               5                   10

<210>  112
<211>  17
<212>  PRT
<213>  Glycine max

<400>  112

Glu Ile Phe Leu Arg Glu Leu Ile Ser Asn Ala Ser Asp Ala Leu Asp
1               5                   10                  15

Lys

<210>  113
<211>  7
<212>  PRT
<213>  Glycine max

<400>  113

Phe Tyr Glu Ala Phe Ser Lys
1               5

<210>  114
<211>  11
<212>  PRT
<213>  Glycine max

<400>  114

His Phe Ser Val Glu Gly Gln Leu Glu Phe Lys
1               5                   10

<210>  115
<211>  9
<212>  PRT
<213>  Glycine max

<400>  115

Ile Arg Phe Glu Ser Leu Thr Asp Lys
1               5

<210>  116
<211>  11
<212>  PRT
<213>  Glycine max

<400>  116

Leu Ala Glu Leu Leu Arg Tyr His Ser Thr Lys
1               5                   10

<210>  117
<211>  8
<212>  PRT
<213>  Glycine max

<400>  117

Leu Asp Glu Ser Glu Asp Glu Lys
1               5

<210>  118
<211>  10
<212>  PRT
<213>  Glycine max

<400>  118

Leu Gly Ile His Glu Asp Ser Gln Asn Lys
1               5                   10


<210>  119
<211>  11
<212>  PRT
<213>  Glycine max

<400>  119

Arg Ala Pro Phe Asp Leu Phe Asp Thr Arg Lys
1               5                   10


<210>  120
<211>  8
<212>  PRT
<213>  Glycine max

<400>  120

Val Glu Asp Val Asp Glu Asp Lys
1               5


<210>  121
<211>  2109
<212>  DNA
<213>  Glycine max


<220>
<221>  CDS
<222>  (1)..(2109)

<400>  121
atg gcg gag aca gag acg ttc gca ttc caa gca gag atc aac cag ctc        48
Met Ala Glu Thr Glu Thr Phe Ala Phe Gln Ala Glu Ile Asn Gln Leu
1               5                   10                  15

ctc agt ctc atc atc aac acc ttc tac tcc aac aag gag att ttc ctt        96
Leu Ser Leu Ile Ile Asn Thr Phe Tyr Ser Asn Lys Glu Ile Phe Leu
            20                  25                  30

cgt gaa ctc atc agc aac gcc tcc gat gct ttg gac aag att cga ttt       144
Arg Glu Leu Ile Ser Asn Ala Ser Asp Ala Leu Asp Lys Ile Arg Phe
        35                  40                  45

gag agt ttg aca gat aag agc aag ctc gat gct cag cca gag ttg ttc       192
Glu Ser Leu Thr Asp Lys Ser Lys Leu Asp Ala Gln Pro Glu Leu Phe
    50                  55                  60

att cat att att cca gac aag act aac aat aca ctg acc att gtt gac       240

Ile His Ile Ile Pro Asp Lys Thr Asn Asn Thr Leu Thr Ile Val Asp
65                  70                  75                  80

agt ggt att ggc atg acc aaa gct gat ttg gtg aac aac ttg ggt aca      288
Ser Gly Ile Gly Met Thr Lys Ala Asp Leu Val Asn Asn Leu Gly Thr
                85                  90                  95

att gct aga tct ggt acc aag gaa ttc atg gaa gcc ttg gct gct ggt      336
Ile Ala Arg Ser Gly Thr Lys Glu Phe Met Glu Ala Leu Ala Ala Gly
            100                 105                 110

gct gat gtt agt atg att gga cag ttt ggt gtg ggg ttc tac tct gca      384
Ala Asp Val Ser Met Ile Gly Gln Phe Gly Val Gly Phe Tyr Ser Ala
        115                 120                 125

tat ctt gtt gct gag aag gtc att gtt aca acc aaa cac aat gat gat      432
Tyr Leu Val Ala Glu Lys Val Ile Val Thr Thr Lys His Asn Asp Asp
    130                 135                 140

gaa cag tat gtt tgg gag tct caa gct gga ggt tct ttc act gtc acc      480
Glu Gln Tyr Val Trp Glu Ser Gln Ala Gly Gly Ser Phe Thr Val Thr
145                 150                 155                 160

agg gat aac act ggt gag gtt ctt ggc agg ggt act aag atc act ctc      528
Arg Asp Asn Thr Gly Glu Val Leu Gly Arg Gly Thr Lys Ile Thr Leu
                165                 170                 175

tac ctc aag gaa gat cag ctt gag tac ctt gag gag cgt cgc ttg aag      576
Tyr Leu Lys Glu Asp Gln Leu Glu Tyr Leu Glu Glu Arg Arg Leu Lys
            180                 185                 190

gat ctg atc aag aag cat tct gag ttc att agc tac cca att tct ctt      624
Asp Leu Ile Lys Lys His Ser Glu Phe Ile Ser Tyr Pro Ile Ser Leu
        195                 200                 205

tgg att gag aag act act gag aag gag atc tct gac gat gag gat gag      672
Trp Ile Glu Lys Thr Thr Glu Lys Glu Ile Ser Asp Asp Glu Asp Glu
    210                 215                 220

gaa gag aag aag gat gag gag ggt aag gtg gag gat gtt gac gaa gag      720
Glu Glu Lys Lys Asp Glu Glu Gly Lys Val Glu Asp Val Asp Glu Glu
225                 230                 235                 240

aag gag aag gaa gaa aaa aag aaa aag aag atc aag gaa gtg tca cat      768
Lys Glu Lys Glu Glu Lys Lys Lys Lys Lys Ile Lys Glu Val Ser His
                245                 250                 255

gaa tgg tcc ttg gtg aac aag cag aag ccc att tgg atg agg aag cct      816
Glu Trp Ser Leu Val Asn Lys Gln Lys Pro Ile Trp Met Arg Lys Pro
            260                 265                 270

gag gag atc aca aaa gag gag tat tct gct ttc tac aag agt ctt acc      864
Glu Glu Ile Thr Lys Glu Glu Tyr Ser Ala Phe Tyr Lys Ser Leu Thr
        275                 280                 285

aat gac tgg gaa gag cat ttg gct gtc aag cac ttc tct gtt gag ggt      912
Asn Asp Trp Glu Glu His Leu Ala Val Lys His Phe Ser Val Glu Gly
    290                 295                 300

cag ttg gag ttt aag gct ata ctc ttt gtt ccc aag agg gca cct ttt      960
Gln Leu Glu Phe Lys Ala Ile Leu Phe Val Pro Lys Arg Ala Pro Phe
305                 310                 315                 320

gac ctc ttt gac aca agg aag aag cct aac aac att aag ctc tat gtc 1008
Asp Leu Phe Asp Thr Arg Lys Lys Pro Asn Asn Ile Lys Leu Tyr Val
                325                 330                 335

cgc cgt gtc ttt att atg gac aac tgt gag gag ttg att cct gag tat 1056
Arg Arg Val Phe Ile Met Asp Asn Cys Glu Glu Leu Ile Pro Glu Tyr
            340                 345                 350

ttg ggc ttt gtc aag ggt att gtt gat tct gaa gat ctc cca ctc aac 1104
Leu Gly Phe Val Lys Gly Ile Val Asp Ser Glu Asp Leu Pro Leu Asn
        355                 360                 365

att tca aga gaa atg ttg caa cag aac aag atc ttg aag gtc atc cgt 1152
Ile Ser Arg Glu Met Leu Gln Gln Asn Lys Ile Leu Lys Val Ile Arg
    370                 375                 380

aag aac ttg gtc aag aag tgc ctt gag ctc ttc ttt gaa att gca gag 1200
Lys Asn Leu Val Lys Lys Cys Leu Glu Leu Phe Phe Glu Ile Ala Glu
385                 390                 395                 400

aac aag gag gat tac aac aag ttc tat gag gct ttc tct aag aac tta 1248
Asn Lys Glu Asp Tyr Asn Lys Phe Tyr Glu Ala Phe Ser Lys Asn Leu
                405                 410                 415

aag ctt ggt atc cat gag gat tcc cag aac aag gga aag att gct gaa 1296
Lys Leu Gly Ile His Glu Asp Ser Gln Asn Lys Gly Lys Ile Ala Glu
            420                 425                 430

ttg ctc agg tac cac tcc acc aag agt ggt gat gag ctg aca agc ctc 1344
Leu Leu Arg Tyr His Ser Thr Lys Ser Gly Asp Glu Leu Thr Ser Leu
        435                 440                 445

aag gat tat gtg acc agg atg aag gaa ggt cag agt gac atc tac tac 1392
Lys Asp Tyr Val Thr Arg Met Lys Glu Gly Gln Ser Asp Ile Tyr Tyr
    450                 455                 460

atc acc ggt gaa agc aag aag gct gtt gag aac tcc cca ttc ttg gag 1440
Ile Thr Gly Glu Ser Lys Lys Ala Val Glu Asn Ser Pro Phe Leu Glu
465                 470                 475                 480

aag ttg aag aag aaa ggg tat gag gtt ctt ttc atg gtt gat gcc att 1488
Lys Leu Lys Lys Lys Gly Tyr Glu Val Leu Phe Met Val Asp Ala Ile
                485                 490                 495

gat gaa tat gct gtt ggc cag ctc aag gaa ttt gag ggc aag aag ctt 1536
Asp Glu Tyr Ala Val Gly Gln Leu Lys Glu Phe Glu Gly Lys Lys Leu
            500                 505                 510

gtc tct gcc acc aag gag ggt ctc aaa ctt gac gag agt gaa gat gag 1584
Val Ser Ala Thr Lys Glu Gly Leu Lys Leu Asp Glu Ser Glu Asp Glu
        515                 520                 525

aag aag aag caa gag gag ttg aag gag aag ttt gac aac cta tgc aag 1632
Lys Lys Lys Gln Glu Glu Leu Lys Glu Lys Phe Asp Asn Leu Cys Lys
    530                 535                 540

gtg atc aag gat gtt ttg ggt gac aag gtt gag aag gtt gta gtc tct 1680
Val Ile Lys Asp Val Leu Gly Asp Lys Val Glu Lys Val Val Val Ser
545                 550                 555                 560

gac cgt gtt gtt gat tca cca tgc tgt ctg gtg act gga gaa tac ggt 1728
Asp Arg Val Val Asp Ser Pro Cys Cys Leu Val Thr Gly Glu Tyr Gly
                565                 570                 575

tgg act gca aac atg gaa agg atc atg aaa gct caa gcc cta agg gac     1776
Trp Thr Ala Asn Met Glu Arg Ile Met Lys Ala Gln Ala Leu Arg Asp
            580                 585                 590

aac agc atg gca gga tac atg tca agc aag aag aca atg gag atc aac     1824
Asn Ser Met Ala Gly Tyr Met Ser Ser Lys Lys Thr Met Glu Ile Asn
        595                 600                 605

cct gag aac cca atc atg gag gag ctc agg aag cgt gcg gat gct gac     1872
Pro Glu Asn Pro Ile Met Glu Glu Leu Arg Lys Arg Ala Asp Ala Asp
    610                 615                 620

aag aac gac aaa tcc gtg aag gac ctt gtt ctg ttg ctg ttc gag act     1920
Lys Asn Asp Lys Ser Val Lys Asp Leu Val Leu Leu Leu Phe Glu Thr
625                 630                 635                 640

gct ctt ctc act tct ggt ttc agc ctt gac gaa ccc aac act ttt gga     1968
Ala Leu Leu Thr Ser Gly Phe Ser Leu Asp Glu Pro Asn Thr Phe Gly
                645                 650                 655

aac agg atc cac aga atg ttg aag ctt gga ctg agc atc gac gag gat     2016
Asn Arg Ile His Arg Met Leu Lys Leu Gly Leu Ser Ile Asp Glu Asp
            660                 665                 670

gca gct gaa gct gat gct gat gct gat atg ccc cca ctg gag gag gag     2064
Ala Ala Glu Ala Asp Ala Asp Ala Asp Met Pro Pro Leu Glu Glu Glu
        675                 680                 685

gct gaa gct gat gct gag ggc agc aaa atg gag gag gtt gat taa         2109
Ala Glu Ala Asp Ala Glu Gly Ser Lys Met Glu Glu Val Asp
    690                 695                 700


<210>  122
<211>  702
<212>  PRT
<213>  Glycine max

<400>  122

Met Ala Glu Thr Glu Thr Phe Ala Phe Gln Ala Glu Ile Asn Gln Leu
1               5                   10                  15


Leu Ser Leu Ile Ile Asn Thr Phe Tyr Ser Asn Lys Glu Ile Phe Leu
            20                  25                  30


Arg Glu Leu Ile Ser Asn Ala Ser Asp Ala Leu Asp Lys Ile Arg Phe
        35                  40                  45


Glu Ser Leu Thr Asp Lys Ser Lys Leu Asp Ala Gln Pro Glu Leu Phe
    50                  55                  60


Ile His Ile Ile Pro Asp Lys Thr Asn Asn Thr Leu Thr Ile Val Asp
65                  70                  75                  80


Ser Gly Ile Gly Met Thr Lys Ala Asp Leu Val Asn Asn Leu Gly Thr
                85                  90                  95

Ile Ala Arg Ser Gly Thr Lys Glu Phe Met Glu Ala Leu Ala Ala Gly
            100                 105                 110

Ala Asp Val Ser Met Ile Gly Gln Phe Gly Val Gly Phe Tyr Ser Ala
        115                 120                 125

Tyr Leu Val Ala Glu Lys Val Ile Val Thr Thr Lys His Asn Asp Asp
    130                 135                 140

Glu Gln Tyr Val Trp Glu Ser Gln Ala Gly Gly Ser Phe Thr Val Thr
145                 150                 155                 160

Arg Asp Asn Thr Gly Glu Val Leu Gly Arg Gly Thr Lys Ile Thr Leu
                165                 170                 175

Tyr Leu Lys Glu Asp Gln Leu Glu Tyr Leu Glu Glu Arg Arg Leu Lys
            180                 185                 190

Asp Leu Ile Lys Lys His Ser Glu Phe Ile Ser Tyr Pro Ile Ser Leu
        195                 200                 205

Trp Ile Glu Lys Thr Thr Glu Lys Glu Ile Ser Asp Asp Glu Asp Glu
    210                 215                 220

Glu Glu Lys Lys Asp Glu Glu Gly Lys Val Glu Asp Val Asp Glu Glu
225                 230                 235                 240

Lys Glu Lys Glu Glu Lys Lys Lys Lys Lys Ile Lys Glu Val Ser His
                245                 250                 255

Glu Trp Ser Leu Val Asn Lys Gln Lys Pro Ile Trp Met Arg Lys Pro
            260                 265                 270

Glu Glu Ile Thr Lys Glu Glu Tyr Ser Ala Phe Tyr Lys Ser Leu Thr
        275                 280                 285

Asn Asp Trp Glu Glu His Leu Ala Val Lys His Phe Ser Val Glu Gly
    290                 295                 300

Gln Leu Glu Phe Lys Ala Ile Leu Phe Val Pro Lys Arg Ala Pro Phe
305                 310                 315                 320

Asp Leu Phe Asp Thr Arg Lys Lys Pro Asn Asn Ile Lys Leu Tyr Val
                325                 330                 335

Arg Arg Val Phe Ile Met Asp Asn Cys Glu Glu Leu Ile Pro Glu Tyr

            340                 345                 350

Leu Gly Phe Val Lys Gly Ile Val Asp Ser Glu Asp Leu Pro Leu Asn
        355                 360                 365

Ile Ser Arg Glu Met Leu Gln Gln Asn Lys Ile Leu Lys Val Ile Arg
    370                 375                 380

Lys Asn Leu Val Lys Lys Cys Leu Glu Leu Phe Phe Glu Ile Ala Glu
385                 390                 395                 400

Asn Lys Glu Asp Tyr Asn Lys Phe Tyr Glu Ala Phe Ser Lys Asn Leu
                405                 410                 415

Lys Leu Gly Ile His Glu Asp Ser Gln Asn Lys Gly Lys Ile Ala Glu
            420                 425                 430

Leu Leu Arg Tyr His Ser Thr Lys Ser Gly Asp Glu Leu Thr Ser Leu
        435                 440                 445

Lys Asp Tyr Val Thr Arg Met Lys Glu Gly Gln Ser Asp Ile Tyr Tyr
    450                 455                 460

Ile Thr Gly Glu Ser Lys Lys Ala Val Glu Asn Ser Pro Phe Leu Glu
465                 470                 475                 480

Lys Leu Lys Lys Lys Gly Tyr Glu Val Leu Phe Met Val Asp Ala Ile
                485                 490                 495

Asp Glu Tyr Ala Val Gly Gln Leu Lys Glu Phe Glu Gly Lys Lys Leu
            500                 505                 510

Val Ser Ala Thr Lys Glu Gly Leu Lys Leu Asp Glu Ser Glu Asp Glu
        515                 520                 525

Lys Lys Lys Gln Glu Glu Leu Lys Glu Lys Phe Asp Asn Leu Cys Lys
    530                 535                 540

Val Ile Lys Asp Val Leu Gly Asp Lys Val Glu Lys Val Val Val Ser
545                 550                 555                 560

Asp Arg Val Val Asp Ser Pro Cys Cys Leu Val Thr Gly Glu Tyr Gly
                565                 570                 575

Trp Thr Ala Asn Met Glu Arg Ile Met Lys Ala Gln Ala Leu Arg Asp
            580                 585                 590

Asn Ser Met Ala Gly Tyr Met Ser Ser Lys Lys Thr Met Glu Ile Asn
        595                 600                 605

Pro Glu Asn Pro Ile Met Glu Glu Leu Arg Lys Arg Ala Asp Ala Asp
    610                 615                 620

Lys Asn Asp Lys Ser Val Lys Asp Leu Val Leu Leu Leu Phe Glu Thr
625                 630                 635                 640

Ala Leu Leu Thr Ser Gly Phe Ser Leu Asp Glu Pro Asn Thr Phe Gly
                645                 650                 655

Asn Arg Ile His Arg Met Leu Lys Leu Gly Leu Ser Ile Asp Glu Asp
            660                 665                 670

Ala Ala Glu Ala Asp Ala Asp Ala Asp Met Pro Pro Leu Glu Glu Glu
        675                 680                 685

Ala Glu Ala Asp Ala Glu Gly Ser Lys Met Glu Glu Val Asp
    690                 695                 700

<210>  123
<211>  16
<212>  PRT
<213>  Glycine max

<400>  123

Ala Asp Leu Val Asn Asn Leu Gly Thr Ile Ala Arg Ser Gly Thr Lys
1               5                   10                  15

<210>  124
<211>  10
<212>  PRT
<213>  Glycine max

<400>  124

Ala Val Glu Asn Ser Pro Phe Leu Glu Lys
1               5                   10

<210>  125
<211>  13
<212>  PRT
<213>  Glycine max

<400>  125

Glu Asp Gln Leu Glu Tyr Leu Glu Glu Arg Arg Leu Lys
1               5                   10

<210>  126
<211>  8

<212>   PRT
<213>   Glycine max

<400>   126

Glu Glu Tyr Ser Ala Phe Tyr Lys
1               5


<210>   127
<211>   14
<212>   PRT
<213>   Glycine max

<400>   127

Glu Gly Gln Ser Asp Ile Tyr Tyr Ile Thr Gly Glu Ser Lys
1               5                   10


<210>   128
<211>   17
<212>   PRT
<213>   Glycine max

<400>   128

Glu Ile Phe Leu Arg Glu Leu Ile Ser Asn Ala Ser Asp Ala Leu Asp
1               5                   10                  15


Lys


<210>   129
<211>   7
<212>   PRT
<213>   Glycine max

<400>   129

Phe Tyr Glu Ala Phe Ser Lys
1               5


<210>   130
<211>   11
<212>   PRT
<213>   Glycine max

<400>   130

His Phe Ser Val Glu Gly Gln Leu Glu Phe Lys
1               5                   10


<210>   131
<211>   11
<212>   PRT
<213>   Glycine max

<400>   131

Ile Ala Glu Leu Leu Arg Tyr His Ser Thr Lys
1               5                   10

<210>  132
<211>  9
<212>  PRT
<213>  Glycine max

<400>  132

Ile Arg Phe Glu Ser Leu Thr Asp Lys
1               5

<210>  133
<211>  8
<212>  PRT
<213>  Glycine max

<400>  133

Leu Asp Glu Ser Glu Asp Glu Lys
1               5

<210>  134
<211>  10
<212>  PRT
<213>  Glycine max

<400>  134

Leu Gly Ile His Glu Asp Ser Gln Asn Lys
1               5                   10

<210>  135
<211>  11
<212>  PRT
<213>  Glycine max

<400>  135

Arg Ala Pro Phe Asp Leu Phe Asp Thr Arg Lys
1               5                   10

<210>  136
<211>  9
<212>  PRT
<213>  Glycine max

<400>  136

Ser Gly Asp Glu Leu Thr Ser Leu Lys
1               5

<210>  137
<211>  8
<212>  PRT

<213>  Glycine max

<400>  137

Val Glu Asp Val Asp Glu Glu Lys
1               5


<210>  138
<211>  2109
<212>  DNA
<213>  Glycine max


<220>
<221>  CDS
<222>  (1)..(2109)

<400>  138
atg gct tcg gag act gag acg ttt gct ttt cag gcc gag atc aac cag      48
Met Ala Ser Glu Thr Glu Thr Phe Ala Phe Gln Ala Glu Ile Asn Gln
1               5                   10                  15

ctc ctc agt cta atc atc aac acc ttc tac agc aac aag gag atc ttt      96
Leu Leu Ser Leu Ile Ile Asn Thr Phe Tyr Ser Asn Lys Glu Ile Phe
            20                  25                  30

ctt cgt gag ctc atc agc aat gcc tct gat gct ttg gac aag att cga      144
Leu Arg Glu Leu Ile Ser Asn Ala Ser Asp Ala Leu Asp Lys Ile Arg
        35                  40                  45

ttt gag agt ctt aca gac aag agc aag ctt gat gct cag cca gag ttg      192
Phe Glu Ser Leu Thr Asp Lys Ser Lys Leu Asp Ala Gln Pro Glu Leu
    50                  55                  60

ttc att cac att att cct gac aag acc aac aac aca ttg tct atc att      240
Phe Ile His Ile Ile Pro Asp Lys Thr Asn Asn Thr Leu Ser Ile Ile
65                  70                  75                  80

gac agt ggt att ggc atg act aag gcc gat ttg gtg aat aac ctc ggt      288
Asp Ser Gly Ile Gly Met Thr Lys Ala Asp Leu Val Asn Asn Leu Gly
                85                  90                  95

act att gca agg tct ggg acc aag gaa ttc atg gaa gcc ctg gct gct      336
Thr Ile Ala Arg Ser Gly Thr Lys Glu Phe Met Glu Ala Leu Ala Ala
            100                 105                 110

ggt gct gat gtg agc atg att gga cag ttt ggt gtt ggt ttc tac tcg      384
Gly Ala Asp Val Ser Met Ile Gly Gln Phe Gly Val Gly Phe Tyr Ser
        115                 120                 125

gct tat ctt gta gct gac aag gtt att gtt acc aca aag cac aat gat      432
Ala Tyr Leu Val Ala Asp Lys Val Ile Val Thr Thr Lys His Asn Asp
    130                 135                 140

gat gag cag tat gtc tgg gag tcc cat gct ggt ggc tca ttt act gtg      480
Asp Glu Gln Tyr Val Trp Glu Ser His Ala Gly Gly Ser Phe Thr Val
145                 150                 155                 160

aca agg gat aca tct ggt gag aac ctt ggt agg gga aca aag ata act      528
Thr Arg Asp Thr Ser Gly Glu Asn Leu Gly Arg Gly Thr Lys Ile Thr
                165                 170                 175

ttg ttt ctc aag gaa gat caa ctt gag tat ctt gag gaa cgc cgt ctg 576
Leu Phe Leu Lys Glu Asp Gln Leu Glu Tyr Leu Glu Glu Arg Arg Leu
            180                 185                 190

aag gat ttg ata aag aag cat tct gaa ttc att agc tat cca att tct 624
Lys Asp Leu Ile Lys Lys His Ser Glu Phe Ile Ser Tyr Pro Ile Ser
        195                 200                 205

ctt tgg att gag aag acc act gaa aag gag att tct gat gat gag gac 672
Leu Trp Ile Glu Lys Thr Thr Glu Lys Glu Ile Ser Asp Asp Glu Asp
    210                 215                 220

gag gaa gag aag aag gat gag gag ggt aaa gtt gaa gat gtg gat gaa 720
Glu Glu Glu Lys Lys Asp Glu Glu Gly Lys Val Glu Asp Val Asp Glu
225                 230                 235                 240

gat aag gag aag gag gag aag aaa aag aag acc att aag gag gta tct 768
Asp Lys Glu Lys Glu Glu Lys Lys Lys Lys Thr Ile Lys Glu Val Ser
                245                 250                 255

cat gag tgg tca ttg gtg aac aag cag aag cct att tgg atg agg aaa 816
His Glu Trp Ser Leu Val Asn Lys Gln Lys Pro Ile Trp Met Arg Lys
            260                 265                 270

cct gaa gag atc aca aaa gaa gaa tat gct gct ttc tac aag agt ctt 864
Pro Glu Glu Ile Thr Lys Glu Glu Tyr Ala Ala Phe Tyr Lys Ser Leu
        275                 280                 285

acc aat gat tgg gaa gag cat ttg gct gtt aag cac ttt tct gtt gag 912
Thr Asn Asp Trp Glu Glu His Leu Ala Val Lys His Phe Ser Val Glu
    290                 295                 300

ggt cag ctg gag ttt aag gct gtc ctc ttt atc ccc aag agg gca cct 960
Gly Gln Leu Glu Phe Lys Ala Val Leu Phe Ile Pro Lys Arg Ala Pro
305                 310                 315                 320

ttt gat ctt ttt gac act aag aag aag cct aac aac atc aaa ctg tat 1008
Phe Asp Leu Phe Asp Thr Lys Lys Lys Pro Asn Asn Ile Lys Leu Tyr
                325                 330                 335

gtc cgc cgt gtt ttc atc atg gac aac tgt gag gag ttg atg cca gaa 1056
Val Arg Arg Val Phe Ile Met Asp Asn Cys Glu Glu Leu Met Pro Glu
            340                 345                 350

tat ctt agc ttt gtt aag ggt att gtg gat tct gag gat ctt cct ctc 1104
Tyr Leu Ser Phe Val Lys Gly Ile Val Asp Ser Glu Asp Leu Pro Leu
        355                 360                 365

aac att tct aga gaa atg ctg cag cag aac aag atc ctg aaa gtc atc 1152
Asn Ile Ser Arg Glu Met Leu Gln Gln Asn Lys Ile Leu Lys Val Ile
    370                 375                 380

agg aag aac ttg gtc aag aag tgc att gag atg ttc ttt gaa att gct 1200
Arg Lys Asn Leu Val Lys Lys Cys Ile Glu Met Phe Phe Glu Ile Ala
385                 390                 395                 400

gag aac aag gaa gac tat aac aag ttt tat gaa gcc ttc tct aag aat 1248
Glu Asn Lys Glu Asp Tyr Asn Lys Phe Tyr Glu Ala Phe Ser Lys Asn
                405                 410                 415

ctg aaa ctt ggt att cat gag gat tct cag aac aag aca aaa ctt gct 1296
Leu Lys Leu Gly Ile His Glu Asp Ser Gln Asn Lys Thr Lys Leu Ala
            420                 425                 430

gaa ttg ctc agg tat cac tcc acc aag agt ggt gat gag atg acc agc     1344
Glu Leu Leu Arg Tyr His Ser Thr Lys Ser Gly Asp Glu Met Thr Ser
        435                 440                 445

ctc aag gac tat gtt acc agg atg aag gaa ggg cag aat gac atc tac     1392
Leu Lys Asp Tyr Val Thr Arg Met Lys Glu Gly Gln Asn Asp Ile Tyr
    450                 455                 460

tac att act ggt gaa agc aag aaa gct gtt gag aat tcc cct ttc ctt     1440
Tyr Ile Thr Gly Glu Ser Lys Lys Ala Val Glu Asn Ser Pro Phe Leu
465                 470                 475                 480

gaa aag ctt aag aag aag ggg ttc gag gtt ctc tac atg gtt gat gct     1488
Glu Lys Leu Lys Lys Lys Gly Phe Glu Val Leu Tyr Met Val Asp Ala
                485                 490                 495

att gac gag tat gct gtt ggt cag ctt aag gaa ttt gag gga aag aag     1536
Ile Asp Glu Tyr Ala Val Gly Gln Leu Lys Glu Phe Glu Gly Lys Lys
            500                 505                 510

ttg gtc tct gct act aag gaa ggc ctg aaa ctt gac gag agc gaa gac     1584
Leu Val Ser Ala Thr Lys Glu Gly Leu Lys Leu Asp Glu Ser Glu Asp
        515                 520                 525

gaa aag aaa aag aag gaa gaa ttg aag gag aaa ttt gag ggt ctc tgc     1632
Glu Lys Lys Lys Lys Glu Glu Leu Lys Glu Lys Phe Glu Gly Leu Cys
    530                 535                 540

cat gtg atc aag gat gtg ttg ggt gac aag gtg gag aaa gtt gtg gtg     1680
His Val Ile Lys Asp Val Leu Gly Asp Lys Val Glu Lys Val Val Val
545                 550                 555                 560

tct gat cgt gtt gtg gat tct cct tgc tgt ctc gtg act ggt gaa tat     1728
Ser Asp Arg Val Val Asp Ser Pro Cys Cys Leu Val Thr Gly Glu Tyr
                565                 570                 575

ggg tgg aca gcc aac atg gaa agg att atg aaa gca cag gca ttg agg     1776
Gly Trp Thr Ala Asn Met Glu Arg Ile Met Lys Ala Gln Ala Leu Arg
            580                 585                 590

gac agc agc atg gct gga tac atg tcg agc aag aag acc atg gag att     1824
Asp Ser Ser Met Ala Gly Tyr Met Ser Ser Lys Lys Thr Met Glu Ile
        595                 600                 605

aac cct gag aac ccc atc atg gat gaa ctg agg aag cga gct gat gct     1872
Asn Pro Glu Asn Pro Ile Met Asp Glu Leu Arg Lys Arg Ala Asp Ala
    610                 615                 620

gac aag aat gac aaa tct gtg aag gat ctc gtt ctc cta ctc ttt gag     1920
Asp Lys Asn Asp Lys Ser Val Lys Asp Leu Val Leu Leu Leu Phe Glu
625                 630                 635                 640

act gct ctt cta act tct ggg ttc agc ctt gat gat ccc aac act ttc     1968
Thr Ala Leu Leu Thr Ser Gly Phe Ser Leu Asp Asp Pro Asn Thr Phe
                645                 650                 655

ggt aac agg att cac agg atg ctg aag ctt gga ctg agc att gat gaa     2016
Gly Asn Arg Ile His Arg Met Leu Lys Leu Gly Leu Ser Ile Asp Glu
            660                 665                 670

gat gct ggt gaa gca gat gct gac atg cct cct ctc gag gac gcc gat     2064
Asp Ala Gly Glu Ala Asp Ala Asp Met Pro Pro Leu Glu Asp Ala Asp

675                    680                    685

gct gat gcc gat gct gag ggt agc aag atg gaa gaa gtt gat taa          2109
Ala Asp Ala Asp Ala Glu Gly Ser Lys Met Glu Glu Val Asp
    690                 695                 700

<210>  139
<211>  702
<212>  PRT
<213>  Glycine max

<400>  139

Met Ala Ser Glu Thr Glu Thr Phe Ala Phe Gln Ala Glu Ile Asn Gln
1               5                   10                  15

Leu Leu Ser Leu Ile Ile Asn Thr Phe Tyr Ser Asn Lys Glu Ile Phe
            20                  25                  30

Leu Arg Glu Leu Ile Ser Asn Ala Ser Asp Ala Leu Asp Lys Ile Arg
        35                  40                  45

Phe Glu Ser Leu Thr Asp Lys Ser Lys Leu Asp Ala Gln Pro Glu Leu
    50                  55                  60

Phe Ile His Ile Ile Pro Asp Lys Thr Asn Asn Thr Leu Ser Ile Ile
65                  70                  75                  80

Asp Ser Gly Ile Gly Met Thr Lys Ala Asp Leu Val Asn Asn Leu Gly
                85                  90                  95

Thr Ile Ala Arg Ser Gly Thr Lys Glu Phe Met Glu Ala Leu Ala Ala
            100                 105                 110

Gly Ala Asp Val Ser Met Ile Gly Gln Phe Gly Val Gly Phe Tyr Ser
        115                 120                 125

Ala Tyr Leu Val Ala Asp Lys Val Ile Val Thr Thr Lys His Asn Asp
    130                 135                 140

Asp Glu Gln Tyr Val Trp Glu Ser His Ala Gly Gly Ser Phe Thr Val
145                 150                 155                 160

Thr Arg Asp Thr Ser Gly Glu Asn Leu Gly Arg Gly Thr Lys Ile Thr
                165                 170                 175

Leu Phe Leu Lys Glu Asp Gln Leu Glu Tyr Leu Glu Glu Arg Arg Leu
            180                 185                 190

Lys Asp Leu Ile Lys Lys His Ser Glu Phe Ile Ser Tyr Pro Ile Ser

195                 200                 205

Leu Trp Ile Glu Lys Thr Thr Glu Lys Glu Ile Ser Asp Asp Glu Asp
    210                 215                 220

Glu Glu Glu Lys Lys Asp Glu Glu Gly Lys Val Glu Asp Val Asp Glu
225                 230                 235                 240

Asp Lys Glu Lys Glu Glu Lys Lys Lys Lys Thr Ile Lys Glu Val Ser
                245                 250                 255

His Glu Trp Ser Leu Val Asn Lys Gln Lys Pro Ile Trp Met Arg Lys
            260                 265                 270

Pro Glu Glu Ile Thr Lys Glu Glu Tyr Ala Ala Phe Tyr Lys Ser Leu
        275                 280                 285

Thr Asn Asp Trp Glu Glu His Leu Ala Val Lys His Phe Ser Val Glu
    290                 295                 300

Gly Gln Leu Glu Phe Lys Ala Val Leu Phe Ile Pro Lys Arg Ala Pro
305                 310                 315                 320

Phe Asp Leu Phe Asp Thr Lys Lys Lys Pro Asn Asn Ile Lys Leu Tyr
                325                 330                 335

Val Arg Arg Val Phe Ile Met Asp Asn Cys Glu Glu Leu Met Pro Glu
            340                 345                 350

Tyr Leu Ser Phe Val Lys Gly Ile Val Asp Ser Glu Asp Leu Pro Leu
        355                 360                 365

Asn Ile Ser Arg Glu Met Leu Gln Gln Asn Lys Ile Leu Lys Val Ile
    370                 375                 380

Arg Lys Asn Leu Val Lys Lys Cys Ile Glu Met Phe Phe Glu Ile Ala
385                 390                 395                 400

Glu Asn Lys Glu Asp Tyr Asn Lys Phe Tyr Glu Ala Phe Ser Lys Asn
                405                 410                 415

Leu Lys Leu Gly Ile His Glu Asp Ser Gln Asn Lys Thr Lys Leu Ala
            420                 425                 430

Glu Leu Leu Arg Tyr His Ser Thr Lys Ser Gly Asp Glu Met Thr Ser
        435                 440                 445

Leu Lys Asp Tyr Val Thr Arg Met Lys Glu Gly Gln Asn Asp Ile Tyr
    450                 455                 460

Tyr Ile Thr Gly Glu Ser Lys Lys Ala Val Glu Asn Ser Pro Phe Leu
465                 470                 475                 480

Glu Lys Leu Lys Lys Lys Gly Phe Glu Val Leu Tyr Met Val Asp Ala
                485                 490                 495

Ile Asp Glu Tyr Ala Val Gly Gln Leu Lys Glu Phe Glu Gly Lys Lys
            500                 505                 510

Leu Val Ser Ala Thr Lys Glu Gly Leu Lys Leu Asp Glu Ser Glu Asp
        515                 520                 525

Glu Lys Lys Lys Lys Glu Glu Leu Lys Glu Lys Phe Glu Gly Leu Cys
    530                 535                 540

His Val Ile Lys Asp Val Leu Gly Asp Lys Val Glu Lys Val Val Val
545                 550                 555                 560

Ser Asp Arg Val Val Asp Ser Pro Cys Cys Leu Val Thr Gly Glu Tyr
                565                 570                 575

Gly Trp Thr Ala Asn Met Glu Arg Ile Met Lys Ala Gln Ala Leu Arg
            580                 585                 590

Asp Ser Ser Met Ala Gly Tyr Met Ser Ser Lys Lys Thr Met Glu Ile
        595                 600                 605

Asn Pro Glu Asn Pro Ile Met Asp Glu Leu Arg Lys Arg Ala Asp Ala
    610                 615                 620

Asp Lys Asn Asp Lys Ser Val Lys Asp Leu Val Leu Leu Leu Phe Glu
625                 630                 635                 640

Thr Ala Leu Leu Thr Ser Gly Phe Ser Leu Asp Asp Pro Asn Thr Phe
                645                 650                 655

Gly Asn Arg Ile His Arg Met Leu Lys Leu Gly Leu Ser Ile Asp Glu
            660                 665                 670

Asp Ala Gly Glu Ala Asp Ala Asp Met Pro Pro Leu Glu Asp Ala Asp
        675                 680                 685

Ala Asp Ala Asp Ala Glu Gly Ser Lys Met Glu Glu Val Asp
    690                 695                 700

<210>  140
<211>  16
<212>  PRT
<213>  Glycine max

<400>  140

Ala Asp Leu Val Asn Asn Leu Gly Thr Ile Ala Arg Ser Gly Thr Lys
1               5                   10                  15


<210>  141
<211>  10
<212>  PRT
<213>  Glycine max

<400>  141

Ala Val Glu Asn Ser Pro Phe Leu Glu Lys
1               5                   10


<210>  142
<211>  13
<212>  PRT
<213>  Glycine max

<400>  142

Glu Asp Gln Leu Glu Tyr Leu Glu Glu Arg Arg Leu Lys
1               5                   10


<210>  143
<211>  8
<212>  PRT
<213>  Glycine max

<400>  143

Glu Glu Tyr Ala Ala Phe Tyr Lys
1               5


<210>  144
<211>  14
<212>  PRT
<213>  Glycine max

<400>  144

Glu Gly Gln Asn Asp Ile Tyr Tyr Ile Thr Gly Glu Ser Lys
1               5                   10


<210>  145
<211>  17
<212>  PRT
<213>  Glycine max

<400>  145

Glu Ile Phe Leu Arg Glu Leu Ile Ser Asn Ala Ser Asp Ala Leu Asp
1               5                   10                  15

Lys

<210>  146
<211>  7
<212>  PRT
<213>  Glycine max

<400>  146

Phe Tyr Glu Ala Phe Ser Lys
1               5

<210>  147
<211>  11
<212>  PRT
<213>  Glycine max

<400>  147

His Phe Ser Val Glu Gly Gln Leu Glu Phe Lys
1               5                   10

<210>  148
<211>  9
<212>  PRT
<213>  Glycine max

<400>  148

Ile Arg Phe Glu Ser Leu Thr Asp Lys
1               5

<210>  149
<211>  11
<212>  PRT
<213>  Glycine max

<400>  149

Leu Ala Glu Leu Leu Arg Tyr His Ser Thr Lys
1               5                   10

<210>  150
<211>  8
<212>  PRT
<213>  Glycine max

<400>  150

Leu Asp Glu Ser Glu Asp Glu Lys
1               5

<210>  151
<211>  10
<212>  PRT
<213>  Glycine max

<400>  151

Leu Gly Ile His Glu Asp Ser Gln Asn Lys
1               5                   10


<210>  152
<211>  10
<212>  PRT
<213>  Glycine max

<400>  152

Arg Ala Pro Phe Asp Leu Phe Asp Thr Lys
1               5                   10


<210>  153
<211>  8
<212>  PRT
<213>  Glycine max

<400>  153

Val Glu Asp Val Asp Glu Asp Lys
1               5


<210>  154
<211>  2109
<212>  DNA
<213>  Glycine max


<220>
<221>  CDS
<222>  (1)..(2109)

<400>  154
atg gct tcg gag act gag acg ttt gct ttt cag gcc gag atc aac cag       48
Met Ala Ser Glu Thr Glu Thr Phe Ala Phe Gln Ala Glu Ile Asn Gln
1               5                   10                  15

ctc ctc agt cta atc atc aac acc ttc tac agc aac aag gag atc ttt       96
Leu Leu Ser Leu Ile Ile Asn Thr Phe Tyr Ser Asn Lys Glu Ile Phe
            20                  25                  30

ctt cgt gag ctc atc agc aat gcc tct gat gct ttg gac aag att cga       144
Leu Arg Glu Leu Ile Ser Asn Ala Ser Asp Ala Leu Asp Lys Ile Arg
        35                  40                  45

ttt gag agt ctt aca gac aag agc aag ctt gat gct cag cca gag ttg       192
Phe Glu Ser Leu Thr Asp Lys Ser Lys Leu Asp Ala Gln Pro Glu Leu
    50                  55                  60

ttc att cac att att cct gac aag acc aac aac aca ttg tct atc att       240
Phe Ile His Ile Ile Pro Asp Lys Thr Asn Asn Thr Leu Ser Ile Ile
65                  70                  75                  80

gac agt ggt att ggc atg act aag gcc gat ttg gtg aat aac ctc ggt 288
Asp Ser Gly Ile Gly Met Thr Lys Ala Asp Leu Val Asn Asn Leu Gly
                85                  90                  95

act att gca agg tct ggg acc aag gaa ttc atg gaa gcc ctg gct gct 336
Thr Ile Ala Arg Ser Gly Thr Lys Glu Phe Met Glu Ala Leu Ala Ala
            100                 105                 110

ggt gct gat gtg agc atg att gga cag ttt ggt gtt ggt ttc tac tcg 384
Gly Ala Asp Val Ser Met Ile Gly Gln Phe Gly Val Gly Phe Tyr Ser
        115                 120                 125

gct tat ctt gta gct gac aag gtt att gtt acc aca aag cac aat gat 432
Ala Tyr Leu Val Ala Asp Lys Val Ile Val Thr Thr Lys His Asn Asp
    130                 135                 140

gat gag cag tat gtc tgg gag tcc cat gct ggt ggc tca ttt act gtg 480
Asp Glu Gln Tyr Val Trp Glu Ser His Ala Gly Gly Ser Phe Thr Val
145                 150                 155                 160

aca agg gat aca tct ggt gag aac ctt ggt agg gga aca aag ata act 528
Thr Arg Asp Thr Ser Gly Glu Asn Leu Gly Arg Gly Thr Lys Ile Thr
                165                 170                 175

ttg ttt ctc aag gaa gat caa ctt gag tat ctt gag gaa cgc cgt ctg 576
Leu Phe Leu Lys Glu Asp Gln Leu Glu Tyr Leu Glu Glu Arg Arg Leu
            180                 185                 190

aag gat ttg ata aag aag cat tct gaa ttc att agc tat cca att tct 624
Lys Asp Leu Ile Lys Lys His Ser Glu Phe Ile Ser Tyr Pro Ile Ser
        195                 200                 205

ctt tgg att gag aag acc act gaa aag gag att tct gat gat gag gac 672
Leu Trp Ile Glu Lys Thr Thr Glu Lys Glu Ile Ser Asp Asp Glu Asp
    210                 215                 220

gag gaa gag aag aag gat gag gag ggt aaa gtt gaa gat gtg gat gaa 720
Glu Glu Glu Lys Lys Asp Glu Glu Gly Lys Val Glu Asp Val Asp Glu
225                 230                 235                 240

gat aag gag aag gag gag aag aaa aag aag acc att aag gag gta tct 768
Asp Lys Glu Lys Glu Glu Lys Lys Lys Lys Thr Ile Lys Glu Val Ser
                245                 250                 255

cat gag tgg tca ttg gtg aac aag cag aag cct att tgg atg agg aaa 816
His Glu Trp Ser Leu Val Asn Lys Gln Lys Pro Ile Trp Met Arg Lys
            260                 265                 270

cct gaa gag atc aca aaa gaa gaa tat gct gct ttc tac aag agt ctt 864
Pro Glu Glu Ile Thr Lys Glu Glu Tyr Ala Ala Phe Tyr Lys Ser Leu
        275                 280                 285

acc aat gat tgg gaa gag cat ttg gct gtt aag cac ttt tct gtt gag 912
Thr Asn Asp Trp Glu Glu His Leu Ala Val Lys His Phe Ser Val Glu
    290                 295                 300

ggt cag ctg gag ttt aag gct gtc ctc ttt atc ccc aag agg gca cct 960
Gly Gln Leu Glu Phe Lys Ala Val Leu Phe Ile Pro Lys Arg Ala Pro
305                 310                 315                 320

ttt gat ctt ttt gac act aag aag aag cct aac aac atc aaa ctg tat 1008
Phe Asp Leu Phe Asp Thr Lys Lys Lys Pro Asn Asn Ile Lys Leu Tyr

                325                 330                 335

gtc cgc cgt gtt ttc atc atg gac aac tgt gag gag ttg atg cca gaa 1056
Val Arg Arg Val Phe Ile Met Asp Asn Cys Glu Glu Leu Met Pro Glu
            340                 345                 350

tat ctt agc ttt gtt aag ggt att gtg gat tct gag gat ctt cct ctc 1104
Tyr Leu Ser Phe Val Lys Gly Ile Val Asp Ser Glu Asp Leu Pro Leu
        355                 360                 365

aac att tct aga gaa atg ctg cag cag aac aag atc ctg aaa gtc atc 1152
Asn Ile Ser Arg Glu Met Leu Gln Gln Asn Lys Ile Leu Lys Val Ile
    370                 375                 380

agg aag aac ttg gtc aag aag tgc att gag atg ttc ttt gaa att gct 1200
Arg Lys Asn Leu Val Lys Lys Cys Ile Glu Met Phe Phe Glu Ile Ala
385                 390                 395                 400

gag aac aag gaa gac tat aac aag ttt tat gaa gcc ttc tct aag aat 1248
Glu Asn Lys Glu Asp Tyr Asn Lys Phe Tyr Glu Ala Phe Ser Lys Asn
                405                 410                 415

ctg aaa ctt ggt att cat gag gat tct cag aac aag aca aaa ctt gct 1296
Leu Lys Leu Gly Ile His Glu Asp Ser Gln Asn Lys Thr Lys Leu Ala
            420                 425                 430

gaa ttg ctc agg tat cac tcc acc aag agt ggt gat gag atg acc agc 1344
Glu Leu Leu Arg Tyr His Ser Thr Lys Ser Gly Asp Glu Met Thr Ser
        435                 440                 445

ctc aag gac tat gtt acc agg atg aag gaa ggg cag aat gac atc tac 1392
Leu Lys Asp Tyr Val Thr Arg Met Lys Glu Gly Gln Asn Asp Ile Tyr
    450                 455                 460

tac att act ggt gaa agc aag aaa gct gtt gag aat tcc cct ttc ctt 1440
Tyr Ile Thr Gly Glu Ser Lys Lys Ala Val Glu Asn Ser Pro Phe Leu
465                 470                 475                 480

gaa aag ctt aag aag aag ggg ttc gag gtt ctc tac atg gtt gat gct 1488
Glu Lys Leu Lys Lys Lys Gly Phe Glu Val Leu Tyr Met Val Asp Ala
                485                 490                 495

att gac gag tat gct gtt ggt cag ctt aag gaa ttt gag gga aag aag 1536
Ile Asp Glu Tyr Ala Val Gly Gln Leu Lys Glu Phe Glu Gly Lys Lys
            500                 505                 510

ttg gtc tct gct act aag gaa ggc ctg aaa ctt gac gag agc gaa gac 1584
Leu Val Ser Ala Thr Lys Glu Gly Leu Lys Leu Asp Glu Ser Glu Asp
        515                 520                 525

gaa aag aaa aag aag gaa gaa ttg aag gag aaa ttt gag ggt ctc tgc 1632
Glu Lys Lys Lys Lys Glu Glu Leu Lys Glu Lys Phe Glu Gly Leu Cys
    530                 535                 540

cat gtg atc aag gat gtg ttg ggt gac aag gtg gag aaa gtt gtg gtg 1680
His Val Ile Lys Asp Val Leu Gly Asp Lys Val Glu Lys Val Val Val
545                 550                 555                 560

tct gat cgt gtt gtg gat tct cct tgc tgt ctc gtg act ggt gaa tat 1728
Ser Asp Arg Val Val Asp Ser Pro Cys Cys Leu Val Thr Gly Glu Tyr
                565                 570                 575

ggg tgg aca gcc aac atg gaa agg att atg aaa gca cag gca ttg agg 1776

Gly Trp Thr Ala Asn Met Glu Arg Ile Met Lys Ala Gln Ala Leu Arg
            580                 585                 590

gac agc agc atg gct gga tac atg tcg agc aag aag acc atg gag att      1824
Asp Ser Ser Met Ala Gly Tyr Met Ser Ser Lys Lys Thr Met Glu Ile
        595                 600                 605

aac cct gag aac ccc atc atg gat gaa ctg agg aag cga gct gat gct      1872
Asn Pro Glu Asn Pro Ile Met Asp Glu Leu Arg Lys Arg Ala Asp Ala
    610                 615                 620

gac aag aat gac aaa tct gtg aag gat ctc gtt ctc cta ctc ttt gag      1920
Asp Lys Asn Asp Lys Ser Val Lys Asp Leu Val Leu Leu Leu Phe Glu
625                 630                 635                 640

act gct ctt cta act tct ggg ttc agc ctt gat gat ccc aac act ttc      1968
Thr Ala Leu Leu Thr Ser Gly Phe Ser Leu Asp Asp Pro Asn Thr Phe
                645                 650                 655

ggt aac agg att cac agg atg ctg aag ctt gga ctg agc att gat gaa      2016
Gly Asn Arg Ile His Arg Met Leu Lys Leu Gly Leu Ser Ile Asp Glu
            660                 665                 670

gat gct ggt gaa gca gat gct gac atg cct cct ctc gag gac gcc gat      2064
Asp Ala Gly Glu Ala Asp Ala Asp Met Pro Pro Leu Glu Asp Ala Asp
        675                 680                 685

gct gat gcc gat gct gag ggt agc aag atg gaa gaa gtt gat taa          2109
Ala Asp Ala Asp Ala Glu Gly Ser Lys Met Glu Glu Val Asp
    690                 695                 700

<210>  155
<211>  702
<212>  PRT
<213>  Glycine max

<400>  155

Met Ala Ser Glu Thr Glu Thr Phe Ala Phe Gln Ala Glu Ile Asn Gln
1               5                   10                  15

Leu Leu Ser Leu Ile Ile Asn Thr Phe Tyr Ser Asn Lys Glu Ile Phe
            20                  25                  30

Leu Arg Glu Leu Ile Ser Asn Ala Ser Asp Ala Leu Asp Lys Ile Arg
        35                  40                  45

Phe Glu Ser Leu Thr Asp Lys Ser Lys Leu Asp Ala Gln Pro Glu Leu
    50                  55                  60

Phe Ile His Ile Ile Pro Asp Lys Thr Asn Asn Thr Leu Ser Ile Ile
65                  70                  75                  80

Asp Ser Gly Ile Gly Met Thr Lys Ala Asp Leu Val Asn Asn Leu Gly
                85                  90                  95

Thr Ile Ala Arg Ser Gly Thr Lys Glu Phe Met Glu Ala Leu Ala Ala
        100                 105                 110

Gly Ala Asp Val Ser Met Ile Gly Gln Phe Gly Val Gly Phe Tyr Ser
    115                 120                 125

Ala Tyr Leu Val Ala Asp Lys Val Ile Val Thr Thr Lys His Asn Asp
    130                 135                 140

Asp Glu Gln Tyr Val Trp Glu Ser His Ala Gly Gly Ser Phe Thr Val
145                 150                 155                 160

Thr Arg Asp Thr Ser Gly Glu Asn Leu Gly Arg Gly Thr Lys Ile Thr
                165                 170                 175

Leu Phe Leu Lys Glu Asp Gln Leu Glu Tyr Leu Glu Glu Arg Arg Leu
        180                 185                 190

Lys Asp Leu Ile Lys Lys His Ser Glu Phe Ile Ser Tyr Pro Ile Ser
    195                 200                 205

Leu Trp Ile Glu Lys Thr Thr Glu Lys Glu Ile Ser Asp Asp Glu Asp
    210                 215                 220

Glu Glu Glu Lys Lys Asp Glu Glu Gly Lys Val Glu Asp Val Asp Glu
225                 230                 235                 240

Asp Lys Glu Lys Glu Glu Lys Lys Lys Lys Thr Ile Lys Glu Val Ser
                245                 250                 255

His Glu Trp Ser Leu Val Asn Lys Gln Lys Pro Ile Trp Met Arg Lys
        260                 265                 270

Pro Glu Glu Ile Thr Lys Glu Glu Tyr Ala Ala Phe Tyr Lys Ser Leu
    275                 280                 285

Thr Asn Asp Trp Glu Glu His Leu Ala Val Lys His Phe Ser Val Glu
    290                 295                 300

Gly Gln Leu Glu Phe Lys Ala Val Leu Phe Ile Pro Lys Arg Ala Pro
305                 310                 315                 320

Phe Asp Leu Phe Asp Thr Lys Lys Lys Pro Asn Asn Ile Lys Leu Tyr
                325                 330                 335

Val Arg Arg Val Phe Ile Met Asp Asn Cys Glu Glu Leu Met Pro Glu
        340                 345                 350

Tyr Leu Ser Phe Val Lys Gly Ile Val Asp Ser Glu Asp Leu Pro Leu
        355                 360                 365

Asn Ile Ser Arg Glu Met Leu Gln Gln Asn Lys Ile Leu Lys Val Ile
    370                 375                 380

Arg Lys Asn Leu Val Lys Lys Cys Ile Glu Met Phe Phe Glu Ile Ala
385                 390                 395                 400

Glu Asn Lys Glu Asp Tyr Asn Lys Phe Tyr Glu Ala Phe Ser Lys Asn
                405                 410                 415

Leu Lys Leu Gly Ile His Glu Asp Ser Gln Asn Lys Thr Lys Leu Ala
            420                 425                 430

Glu Leu Leu Arg Tyr His Ser Thr Lys Ser Gly Asp Glu Met Thr Ser
        435                 440                 445

Leu Lys Asp Tyr Val Thr Arg Met Lys Glu Gly Gln Asn Asp Ile Tyr
    450                 455                 460

Tyr Ile Thr Gly Glu Ser Lys Lys Ala Val Glu Asn Ser Pro Phe Leu
465                 470                 475                 480

Glu Lys Leu Lys Lys Lys Gly Phe Glu Val Leu Tyr Met Val Asp Ala
                485                 490                 495

Ile Asp Glu Tyr Ala Val Gly Gln Leu Lys Glu Phe Glu Gly Lys Lys
            500                 505                 510

Leu Val Ser Ala Thr Lys Glu Gly Leu Lys Leu Asp Glu Ser Glu Asp
        515                 520                 525

Glu Lys Lys Lys Lys Glu Glu Leu Lys Glu Lys Phe Glu Gly Leu Cys
    530                 535                 540

His Val Ile Lys Asp Val Leu Gly Asp Lys Val Glu Lys Val Val Val
545                 550                 555                 560

Ser Asp Arg Val Val Asp Ser Pro Cys Cys Leu Val Thr Gly Glu Tyr
                565                 570                 575

Gly Trp Thr Ala Asn Met Glu Arg Ile Met Lys Ala Gln Ala Leu Arg
            580                 585                 590

Asp Ser Ser Met Ala Gly Tyr Met Ser Ser Lys Lys Thr Met Glu Ile
        595                 600                 605

Asn Pro Glu Asn Pro Ile Met Asp Glu Leu Arg Lys Arg Ala Asp Ala
    610                 615                 620

Asp Lys Asn Asp Lys Ser Val Lys Asp Leu Val Leu Leu Leu Phe Glu
625                 630                 635                 640

Thr Ala Leu Leu Thr Ser Gly Phe Ser Leu Asp Asp Pro Asn Thr Phe
                645                 650                 655

Gly Asn Arg Ile His Arg Met Leu Lys Leu Gly Leu Ser Ile Asp Glu
            660                 665                 670

Asp Ala Gly Glu Ala Asp Ala Asp Met Pro Pro Leu Glu Asp Ala Asp
        675                 680                 685

Ala Asp Ala Asp Ala Glu Gly Ser Lys Met Glu Glu Val Asp
    690                 695                 700

<210>  156
<211>  12
<212>  PRT
<213>  Glycine max

<400>  156

Ala Gly Gly Ala Leu Ser Ile Ala Phe Val Ser Lys
1               5                   10

<210>  157
<211>  9
<212>  PRT
<213>  Glycine max

<400>  157

Asp Gly Leu Ile Val Ser Ile Gly Lys
1               5

<210>  158
<211>  2946
<212>  DNA
<213>  Glycine max

<220>
<221>  CDS
<222>  (1)..(2946)

<400>  158
atg gct tct tct tcc acg aca atg cgg ttc tcc gcg gcc tcc acc ggc        48
Met Ala Ser Ser Ser Thr Thr Met Arg Phe Ser Ala Ala Ser Thr Gly
1               5                   10                  15

gcc gcg gcg gcg ctt cct cgc cgg agc tcc gtc gcc gga ttc atc ggc     96
Ala Ala Ala Ala Leu Pro Arg Arg Ser Ser Val Ala Gly Phe Ile Gly
            20                  25                  30

gtc gcg gcc aga tcc tcc gcc aaa tcg agg ctc cgc ttc atc gga agg     144
Val Ala Ala Arg Ser Ser Ala Lys Ser Arg Leu Arg Phe Ile Gly Arg
        35                  40                  45

aac gcg aac ttg agc ctc cgg atg agg agg atg agc tct ttc tcc gtc     192
Asn Ala Asn Leu Ser Leu Arg Met Arg Arg Met Ser Ser Phe Ser Val
    50                  55                  60

gtg aag tgc gtc tcc gga agc gag gcc aag gtg cag gat acc gtg gcc     240
Val Lys Cys Val Ser Gly Ser Glu Ala Lys Val Gln Asp Thr Val Ala
65                  70                  75                  80

aaa caa caa gaa gct aca acc tct ctc agc tcc ttc acg cca gat gct     288
Lys Gln Gln Glu Ala Thr Thr Ser Leu Ser Ser Phe Thr Pro Asp Ala
                85                  90                  95

tca tct att gcc tca agt att aaa tac cat gca gag ttc act cca ttg     336
Ser Ser Ile Ala Ser Ser Ile Lys Tyr His Ala Glu Phe Thr Pro Leu
            100                 105                 110

ttt tct cca gag aat ttt gat cta cca cag gct ttt ctt gca act gca     384
Phe Ser Pro Glu Asn Phe Asp Leu Pro Gln Ala Phe Leu Ala Thr Ala
        115                 120                 125

caa agt gtt cga gat tct ctc att ata aac tgg aat gct acc tat gat     432
Gln Ser Val Arg Asp Ser Leu Ile Ile Asn Trp Asn Ala Thr Tyr Asp
    130                 135                 140

tac tat gag aaa ctg aat gtt aag cag gca tat tac ctt tcg atg gaa     480
Tyr Tyr Glu Lys Leu Asn Val Lys Gln Ala Tyr Tyr Leu Ser Met Glu
145                 150                 155                 160

ttt ttg cag ggt aga gca tta tta aat gca ata ggc aat ctg gag cta     528
Phe Leu Gln Gly Arg Ala Leu Leu Asn Ala Ile Gly Asn Leu Glu Leu
                165                 170                 175

act ggt ccc tat gct gag gct ttg agc aag ctc ggg cat aaa tta gaa     576
Thr Gly Pro Tyr Ala Glu Ala Leu Ser Lys Leu Gly His Lys Leu Glu
            180                 185                 190

aat gtg gca tac cag gag cca gat gct gca ctt gga aat ggt ggt ctt     624
Asn Val Ala Tyr Gln Glu Pro Asp Ala Ala Leu Gly Asn Gly Gly Leu
        195                 200                 205

ggg cgt ctt gct tca tgt ttc ttg gac tct ttg gca acc ttg aac tat     672
Gly Arg Leu Ala Ser Cys Phe Leu Asp Ser Leu Ala Thr Leu Asn Tyr
    210                 215                 220

cca gct tgg ggt tat gga ttg aga tat aag tat ggt tta ttt aaa cag     720
Pro Ala Trp Gly Tyr Gly Leu Arg Tyr Lys Tyr Gly Leu Phe Lys Gln
225                 230                 235                 240

cga ata acc aaa gat ggg cag gag gaa gtt gct gaa gat tgg ctt gag     768
Arg Ile Thr Lys Asp Gly Gln Glu Glu Val Ala Glu Asp Trp Leu Glu
                245                 250                 255

atg ggc aat ccc tgg gag att att aga aat gat gtt tca tat cct gta     816
Met Gly Asn Pro Trp Glu Ile Ile Arg Asn Asp Val Ser Tyr Pro Val
            260                 265                 270

aag ttc tat ggc aag gtt gtt tca ggg tca gat ggt aaa aaa cat tgg 864
Lys Phe Tyr Gly Lys Val Val Ser Gly Ser Asp Gly Lys Lys His Trp
        275                 280                 285

att gga gga gaa gat ata aaa gct gtt gca cat gat gtt ccc ata cca 912
Ile Gly Gly Glu Asp Ile Lys Ala Val Ala His Asp Val Pro Ile Pro
    290                 295                 300

gga tat aaa act aag act aca atc aac ttg aga ctt tgg tct aca aaa 960
Gly Tyr Lys Thr Lys Thr Thr Ile Asn Leu Arg Leu Trp Ser Thr Lys
305                 310                 315                 320

gct gca tca gaa gaa ttt gat tta tct gct ttt aat gct gga agg cac 1008
Ala Ala Ser Glu Glu Phe Asp Leu Ser Ala Phe Asn Ala Gly Arg His
                325                 330                 335

act gaa gca tct gag gct cta gca aat gct gaa aag ata tgc tat ata 1056
Thr Glu Ala Ser Glu Ala Leu Ala Asn Ala Glu Lys Ile Cys Tyr Ile
            340                 345                 350

ctc tac cct ggg gat gaa cca ata gag ggc aag atc ctt cgc ctc aag 1104
Leu Tyr Pro Gly Asp Glu Pro Ile Glu Gly Lys Ile Leu Arg Leu Lys
        355                 360                 365

caa caa tat act tta tgt tct gct tct ctt caa gac atc att gct cgt 1152
Gln Gln Tyr Thr Leu Cys Ser Ala Ser Leu Gln Asp Ile Ile Ala Arg
    370                 375                 380

ttt gag aga aga tca gga gca aat gtt aat tgg gaa gag ttt ccc gag 1200
Phe Glu Arg Arg Ser Gly Ala Asn Val Asn Trp Glu Glu Phe Pro Glu
385                 390                 395                 400

aag gtt gca gtg cag atg aat gat act cac cca acg ttg tgc atc cca 1248
Lys Val Ala Val Gln Met Asn Asp Thr His Pro Thr Leu Cys Ile Pro
                405                 410                 415

gag ctc atg aga att tta ata gat gta aag ggc tta aat tgg aag gat 1296
Glu Leu Met Arg Ile Leu Ile Asp Val Lys Gly Leu Asn Trp Lys Asp
            420                 425                 430

gct tgg aat att aca caa agg act gta gca tat aca aac cac act gtt 1344
Ala Trp Asn Ile Thr Gln Arg Thr Val Ala Tyr Thr Asn His Thr Val
        435                 440                 445

ctt cca gag gca tta gag aaa tgg agc ttg gat ctt atg cag aaa ctg 1392
Leu Pro Glu Ala Leu Glu Lys Trp Ser Leu Asp Leu Met Gln Lys Leu
    450                 455                 460

ctt cca cgc cat att gag ata ata gaa atg att gac gag gag ctg gtt 1440
Leu Pro Arg His Ile Glu Ile Ile Glu Met Ile Asp Glu Glu Leu Val
465                 470                 475                 480

cgg acc ata att gca gaa tat ggc aca gaa aat tca gac ttg ctg gag 1488
Arg Thr Ile Ile Ala Glu Tyr Gly Thr Glu Asn Ser Asp Leu Leu Glu
                485                 490                 495

aaa aaa ttg aag gaa atg aga ata cta gaa aat gtc gaa ttg act gca 1536
Lys Lys Leu Lys Glu Met Arg Ile Leu Glu Asn Val Glu Leu Thr Ala
            500                 505                 510

gaa ttt gca gat ata ctg gtt aag tcc aaa gag gcc att gat att cct 1584
Glu Phe Ala Asp Ile Leu Val Lys Ser Lys Glu Ala Ile Asp Ile Pro

515                 520                 525

agt gag gag ctg caa agt tct gaa caa gca gaa gca gaa gac gaa aaa      1632
Ser Glu Glu Leu Gln Ser Ser Glu Gln Ala Glu Ala Glu Asp Glu Lys
    530                 535                 540

gat gat gat gaa gtg gaa gca gtt gct aaa aag aat gga aca gat gaa      1680
Asp Asp Asp Glu Val Glu Ala Val Ala Lys Lys Asn Gly Thr Asp Glu
545                 550                 555                 560

agt tcc att gag gat gag aaa gaa gag ttg cct gag cca gta cca gaa      1728
Ser Ser Ile Glu Asp Glu Lys Glu Glu Leu Pro Glu Pro Val Pro Glu
                565                 570                 575

cca cca aaa ttg gtt cgt atg gca aat ttg tgt gtt gtg ggt ggt cat      1776
Pro Pro Lys Leu Val Arg Met Ala Asn Leu Cys Val Val Gly Gly His
            580                 585                 590

gca gtg aat ggg gtt gca gag ata cat agt gaa att gta aaa gat gat      1824
Ala Val Asn Gly Val Ala Glu Ile His Ser Glu Ile Val Lys Asp Asp
        595                 600                 605

gtt ttc aat gca ttt tat aag ttg tgg ccc gag aag ttc caa aac aaa      1872
Val Phe Asn Ala Phe Tyr Lys Leu Trp Pro Glu Lys Phe Gln Asn Lys
    610                 615                 620

aca aat ggt gtg aca cca agg aga tgg atc agg ttc tgc aat cca gat      1920
Thr Asn Gly Val Thr Pro Arg Arg Trp Ile Arg Phe Cys Asn Pro Asp
625                 630                 635                 640

ctg agt aaa ata ata acc gag tgg ata ggt act gag gat tgg gtc cta      1968
Leu Ser Lys Ile Ile Thr Glu Trp Ile Gly Thr Glu Asp Trp Val Leu
                645                 650                 655

aat act gga aaa ttg gcc gag cta cgg aag ttt gta gac aat gag gat      2016
Asn Thr Gly Lys Leu Ala Glu Leu Arg Lys Phe Val Asp Asn Glu Asp
            660                 665                 670

cta caa gtg cag tgg agg gaa gca aag agg agt aac aaa gtt aaa gtt      2064
Leu Gln Val Gln Trp Arg Glu Ala Lys Arg Ser Asn Lys Val Lys Val
        675                 680                 685

gca gca ttc atc aga gaa aaa aca ggg tat tct gtc agt cct gat gca      2112
Ala Ala Phe Ile Arg Glu Lys Thr Gly Tyr Ser Val Ser Pro Asp Ala
    690                 695                 700

atg ttt gac atc cag gtg aag aga atc cat gaa tat aag cga caa tta      2160
Met Phe Asp Ile Gln Val Lys Arg Ile His Glu Tyr Lys Arg Gln Leu
705                 710                 715                 720

atg aac ata ttt gga att gtt tac cga tac aag aag atg aaa gaa atg      2208
Met Asn Ile Phe Gly Ile Val Tyr Arg Tyr Lys Lys Met Lys Glu Met
                725                 730                 735

agt gcg gca gaa agg gaa gca aat ttt gtt cct aga gtt tgt ata ttt      2256
Ser Ala Ala Glu Arg Glu Ala Asn Phe Val Pro Arg Val Cys Ile Phe
            740                 745                 750

ggg gga aaa gca ttt gct aca tat gtg caa gcc aag aga att gtg aaa      2304
Gly Gly Lys Ala Phe Ala Thr Tyr Val Gln Ala Lys Arg Ile Val Lys
        755                 760                 765

ttt att aca gat gtt ggg gct aca gta aat cat gac cct gaa ata gga      2352

Phe Ile Thr Asp Val Gly Ala Thr Val Asn His Asp Pro Glu Ile Gly
    770                 775                 780

gat cta ctt aag gtt gtt ttt gtc cct gat tat aat gtc agt gtt gcc     2400
Asp Leu Leu Lys Val Val Phe Val Pro Asp Tyr Asn Val Ser Val Ala
785                 790                 795                 800

gag atg ctt att cca gca agt gaa ttg tca caa cat atc agt act gct     2448
Glu Met Leu Ile Pro Ala Ser Glu Leu Ser Gln His Ile Ser Thr Ala
                805                 810                 815

gga atg gag gca agt gga act agc aat atg aaa ttt gca atg aat ggc     2496
Gly Met Glu Ala Ser Gly Thr Ser Asn Met Lys Phe Ala Met Asn Gly
            820                 825                 830

tgc ata ttg att gga act tta gat ggg gcc aat gtt gaa ata agg gaa     2544
Cys Ile Leu Ile Gly Thr Leu Asp Gly Ala Asn Val Glu Ile Arg Glu
        835                 840                 845

gag gtt ggt gca gac aac ttc ttc ctc ttt ggt gcc aag gct cat gaa     2592
Glu Val Gly Ala Asp Asn Phe Phe Leu Phe Gly Ala Lys Ala His Glu
    850                 855                 860

att gct ggg ctg agg aaa gaa aga gca gag gga aag ttt gtg cct gat     2640
Ile Ala Gly Leu Arg Lys Glu Arg Ala Glu Gly Lys Phe Val Pro Asp
865                 870                 875                 880

cca cga ttt gaa gaa gtt aaa gaa ttt gtc aga agt ggc att ttt gga     2688
Pro Arg Phe Glu Glu Val Lys Glu Phe Val Arg Ser Gly Ile Phe Gly
                885                 890                 895

tct tac aac tat gac gaa ttg atg gga tcc tta gaa gga aat gaa ggt     2736
Ser Tyr Asn Tyr Asp Glu Leu Met Gly Ser Leu Glu Gly Asn Glu Gly
            900                 905                 910

ttt ggt cga gca gat tat ttc ctt gta ggg aaa gac ttt ccc agc tac     2784
Phe Gly Arg Ala Asp Tyr Phe Leu Val Gly Lys Asp Phe Pro Ser Tyr
        915                 920                 925

ata gaa tgc caa gag aag gtt gat gaa gca tac cgt gac caa act aaa     2832
Ile Glu Cys Gln Glu Lys Val Asp Glu Ala Tyr Arg Asp Gln Thr Lys
    930                 935                 940

tgg aca aga atg tca ata ttg aac aca gca ggt tca tac aag ttt agc     2880
Trp Thr Arg Met Ser Ile Leu Asn Thr Ala Gly Ser Tyr Lys Phe Ser
945                 950                 955                 960

agt gac cgc acg att cat gaa tat gcc cga gag ata tgg aac att gaa     2928
Ser Asp Arg Thr Ile His Glu Tyr Ala Arg Glu Ile Trp Asn Ile Glu
                965                 970                 975

cca gtc caa ttg cca tag                                             2946
Pro Val Gln Leu Pro
            980


<210>  159
<211>  981
<212>  PRT
<213>  Glycine max

<400>  159

Met Ala Ser Ser Ser Thr Thr Met Arg Phe Ser Ala Ala Ser Thr Gly
1               5                   10                  15

Ala Ala Ala Ala Leu Pro Arg Arg Ser Ser Val Ala Gly Phe Ile Gly
            20                  25                  30

Val Ala Ala Arg Ser Ser Ala Lys Ser Arg Leu Arg Phe Ile Gly Arg
        35                  40                  45

Asn Ala Asn Leu Ser Leu Arg Met Arg Arg Met Ser Ser Phe Ser Val
    50                  55                  60

Val Lys Cys Val Ser Gly Ser Glu Ala Lys Val Gln Asp Thr Val Ala
65                  70                  75                  80

Lys Gln Gln Glu Ala Thr Thr Ser Leu Ser Ser Phe Thr Pro Asp Ala
                85                  90                  95

Ser Ser Ile Ala Ser Ser Ile Lys Tyr His Ala Glu Phe Thr Pro Leu
            100                 105                 110

Phe Ser Pro Glu Asn Phe Asp Leu Pro Gln Ala Phe Leu Ala Thr Ala
        115                 120                 125

Gln Ser Val Arg Asp Ser Leu Ile Ile Asn Trp Asn Ala Thr Tyr Asp
    130                 135                 140

Tyr Tyr Glu Lys Leu Asn Val Lys Gln Ala Tyr Tyr Leu Ser Met Glu
145                 150                 155                 160

Phe Leu Gln Gly Arg Ala Leu Leu Asn Ala Ile Gly Asn Leu Glu Leu
                165                 170                 175

Thr Gly Pro Tyr Ala Glu Ala Leu Ser Lys Leu Gly His Lys Leu Glu
            180                 185                 190

Asn Val Ala Tyr Gln Glu Pro Asp Ala Ala Leu Gly Asn Gly Gly Leu
        195                 200                 205

Gly Arg Leu Ala Ser Cys Phe Leu Asp Ser Leu Ala Thr Leu Asn Tyr
    210                 215                 220

Pro Ala Trp Gly Tyr Gly Leu Arg Tyr Lys Tyr Gly Leu Phe Lys Gln
225                 230                 235                 240

Arg Ile Thr Lys Asp Gly Gln Glu Glu Val Ala Glu Asp Trp Leu Glu
                245                 250                 255

Met Gly Asn Pro Trp Glu Ile Ile Arg Asn Asp Val Ser Tyr Pro Val
            260                 265                 270
Lys Phe Tyr Gly Lys Val Val Ser Gly Ser Asp Gly Lys Lys His Trp
        275                 280                 285
Ile Gly Gly Glu Asp Ile Lys Ala Val Ala His Asp Val Pro Ile Pro
    290                 295                 300
Gly Tyr Lys Thr Lys Thr Thr Ile Asn Leu Arg Leu Trp Ser Thr Lys
305                 310                 315                 320
Ala Ala Ser Glu Glu Phe Asp Leu Ser Ala Phe Asn Ala Gly Arg His
                325                 330                 335
Thr Glu Ala Ser Glu Ala Leu Ala Asn Ala Glu Lys Ile Cys Tyr Ile
            340                 345                 350
Leu Tyr Pro Gly Asp Glu Pro Ile Glu Gly Lys Ile Leu Arg Leu Lys
        355                 360                 365
Gln Gln Tyr Thr Leu Cys Ser Ala Ser Leu Gln Asp Ile Ile Ala Arg
    370                 375                 380
Phe Glu Arg Arg Ser Gly Ala Asn Val Asn Trp Glu Glu Phe Pro Glu
385                 390                 395                 400
Lys Val Ala Val Gln Met Asn Asp Thr His Pro Thr Leu Cys Ile Pro
                405                 410                 415
Glu Leu Met Arg Ile Leu Ile Asp Val Lys Gly Leu Asn Trp Lys Asp
            420                 425                 430
Ala Trp Asn Ile Thr Gln Arg Thr Val Ala Tyr Thr Asn His Thr Val
        435                 440                 445
Leu Pro Glu Ala Leu Glu Lys Trp Ser Leu Asp Leu Met Gln Lys Leu
    450                 455                 460
Leu Pro Arg His Ile Glu Ile Ile Glu Met Ile Asp Glu Glu Leu Val
465                 470                 475                 480
Arg Thr Ile Ile Ala Glu Tyr Gly Thr Glu Asn Ser Asp Leu Leu Glu
                485                 490                 495
Lys Lys Leu Lys Glu Met Arg Ile Leu Glu Asn Val Glu Leu Thr Ala
            500                 505                 510

Glu Phe Ala Asp Ile Leu Val Lys Ser Lys Glu Ala Ile Asp Ile Pro
        515                 520                 525

Ser Glu Glu Leu Gln Ser Ser Glu Gln Ala Glu Ala Glu Asp Glu Lys
    530                 535                 540

Asp Asp Asp Glu Val Glu Ala Val Ala Lys Lys Asn Gly Thr Asp Glu
545                 550                 555                 560

Ser Ser Ile Glu Asp Glu Lys Glu Glu Leu Pro Glu Pro Val Pro Glu
                565                 570                 575

Pro Pro Lys Leu Val Arg Met Ala Asn Leu Cys Val Val Gly Gly His
            580                 585                 590

Ala Val Asn Gly Val Ala Glu Ile His Ser Glu Ile Val Lys Asp Asp
        595                 600                 605

Val Phe Asn Ala Phe Tyr Lys Leu Trp Pro Glu Lys Phe Gln Asn Lys
    610                 615                 620

Thr Asn Gly Val Thr Pro Arg Arg Trp Ile Arg Phe Cys Asn Pro Asp
625                 630                 635                 640

Leu Ser Lys Ile Ile Thr Glu Trp Ile Gly Thr Glu Asp Trp Val Leu
                645                 650                 655

Asn Thr Gly Lys Leu Ala Glu Leu Arg Lys Phe Val Asp Asn Glu Asp
            660                 665                 670

Leu Gln Val Gln Trp Arg Glu Ala Lys Arg Ser Asn Lys Val Lys Val
        675                 680                 685

Ala Ala Phe Ile Arg Glu Lys Thr Gly Tyr Ser Val Ser Pro Asp Ala
    690                 695                 700

Met Phe Asp Ile Gln Val Lys Arg Ile His Glu Tyr Lys Arg Gln Leu
705                 710                 715                 720

Met Asn Ile Phe Gly Ile Val Tyr Arg Tyr Lys Lys Met Lys Glu Met
                725                 730                 735

Ser Ala Ala Glu Arg Glu Ala Asn Phe Val Pro Arg Val Cys Ile Phe
            740                 745                 750

Gly Gly Lys Ala Phe Ala Thr Tyr Val Gln Ala Lys Arg Ile Val Lys

        755                 760                 765

Phe Ile Thr Asp Val Gly Ala Thr Val Asn His Asp Pro Glu Ile Gly
    770                 775                 780

Asp Leu Leu Lys Val Val Phe Val Pro Asp Tyr Asn Val Ser Val Ala
785                 790                 795                 800

Glu Met Leu Ile Pro Ala Ser Glu Leu Ser Gln His Ile Ser Thr Ala
                805                 810                 815

Gly Met Glu Ala Ser Gly Thr Ser Asn Met Lys Phe Ala Met Asn Gly
            820                 825                 830

Cys Ile Leu Ile Gly Thr Leu Asp Gly Ala Asn Val Glu Ile Arg Glu
        835                 840                 845

Glu Val Gly Ala Asp Asn Phe Phe Leu Phe Gly Ala Lys Ala His Glu
    850                 855                 860

Ile Ala Gly Leu Arg Lys Glu Arg Ala Glu Gly Lys Phe Val Pro Asp
865                 870                 875                 880

Pro Arg Phe Glu Glu Val Lys Glu Phe Val Arg Ser Gly Ile Phe Gly
                885                 890                 895

Ser Tyr Asn Tyr Asp Glu Leu Met Gly Ser Leu Glu Gly Asn Glu Gly
            900                 905                 910

Phe Gly Arg Ala Asp Tyr Phe Leu Val Gly Lys Asp Phe Pro Ser Tyr
        915                 920                 925

Ile Glu Cys Gln Glu Lys Val Asp Glu Ala Tyr Arg Asp Gln Thr Lys
    930                 935                 940

Trp Thr Arg Met Ser Ile Leu Asn Thr Ala Gly Ser Tyr Lys Phe Ser
945                 950                 955                 960

Ser Asp Arg Thr Ile His Glu Tyr Ala Arg Glu Ile Trp Asn Ile Glu
                965                 970                 975

Pro Val Gln Leu Pro
            980

<210> 160
<211> 9
<212> PRT
<213> Glycine max

<400>  160

Ala Phe Ala Thr Tyr Val Gln Ala Lys
1               5

<210>  161
<211>  23
<212>  PRT
<213>  Glycine max

<400>  161

Gln Gln Glu Ala Thr Thr Ser Leu Ser Ser Phe Thr Pro Asp Ala Ser
1               5                   10                  15

Ser Ile Ala Ser Ser Ile Lys
            20

<210>  162
<211>  2706
<212>  DNA
<213>  Glycine max

<220>
<221>  CDS
<222>  (1)..(2706)

<400>  162
atg gcg act gag aat cct ttc aat agc att ttg acg acg ctc gaa aag      48
Met Ala Thr Glu Asn Pro Phe Asn Ser Ile Leu Thr Thr Leu Glu Lys
1               5                   10                  15

ccc ggt ggt gct ggc gag ttt gga aag tac ttt agc ttg cct gct ctc      96
Pro Gly Gly Ala Gly Glu Phe Gly Lys Tyr Phe Ser Leu Pro Ala Leu
            20                  25                  30

aac gat cgt aga atc gat aga ctt cct tac tct gtg agg ata ctt ttg      144
Asn Asp Arg Arg Ile Asp Arg Leu Pro Tyr Ser Val Arg Ile Leu Leu
        35                  40                  45

gaa tct gca atc cgg aac tgt gat gag ttt caa gtt aag agt aac gac      192
Glu Ser Ala Ile Arg Asn Cys Asp Glu Phe Gln Val Lys Ser Asn Asp
    50                  55                  60

gtt gaa aaa att att gat tgg gag aat acc tct ccg aag ctg gtg gag      240
Val Glu Lys Ile Ile Asp Trp Glu Asn Thr Ser Pro Lys Leu Val Glu
65                  70                  75                  80

att cca ttc aag cca gct aga gtg ctt ctc cag gat ttt act ggc gtg      288
Ile Pro Phe Lys Pro Ala Arg Val Leu Leu Gln Asp Phe Thr Gly Val
                85                  90                  95

cct gct gtt gtc gat ctt gct tgc atg cga gat gcc atg aac aaa ctt      336
Pro Ala Val Val Asp Leu Ala Cys Met Arg Asp Ala Met Asn Lys Leu
            100                 105                 110

ggt ggt gat tct aat aaa att aat ccc ttg gta cca gtt gat ctt gtc      384
Gly Gly Asp Ser Asn Lys Ile Asn Pro Leu Val Pro Val Asp Leu Val

115                 120                 125

att gat cac tct gtt caa gtt gat gtg gca aga tca gaa aat gct gtc      432
Ile Asp His Ser Val Gln Val Asp Val Ala Arg Ser Glu Asn Ala Val
    130                 135                 140

cag gca aat atg gaa ctt gaa ttc caa aga aac aag gaa aga ttt ggt      480
Gln Ala Asn Met Glu Leu Glu Phe Gln Arg Asn Lys Glu Arg Phe Gly
145                 150                 155                 160

ttc ctc aaa tgg ggt tct aat gca ttc aat aat atg ctt gtc gtt cct      528
Phe Leu Lys Trp Gly Ser Asn Ala Phe Asn Asn Met Leu Val Val Pro
                165                 170                 175

cct gga tca ggg ata gtt cac cag gtt aat tta gaa tac ctt ggt aga      576
Pro Gly Ser Gly Ile Val His Gln Val Asn Leu Glu Tyr Leu Gly Arg
            180                 185                 190

gtt gtg ttc aac aca aat ggt gtg ctt tat cct gac agc gtt gtt gga      624
Val Val Phe Asn Thr Asn Gly Val Leu Tyr Pro Asp Ser Val Val Gly
        195                 200                 205

act gat tca cac aca act atg att gat ggc ctg ggt gtt gct gga tgg      672
Thr Asp Ser His Thr Thr Met Ile Asp Gly Leu Gly Val Ala Gly Trp
    210                 215                 220

gga gtt ggt gga ata gaa gca gaa gct gca atg ctt ggc caa ccc atg      720
Gly Val Gly Gly Ile Glu Ala Glu Ala Ala Met Leu Gly Gln Pro Met
225                 230                 235                 240

agc atg gtc cta ccg ggt gtc gtt ggg ttc aaa tta tta ggc aaa cta      768
Ser Met Val Leu Pro Gly Val Val Gly Phe Lys Leu Leu Gly Lys Leu
                245                 250                 255

cga gat ggt gtc aca gct act gac ttg gtg ttg act gtt aca caa atg      816
Arg Asp Gly Val Thr Ala Thr Asp Leu Val Leu Thr Val Thr Gln Met
            260                 265                 270

cta aga aag cat ggg gtt gtt ggc aag ttt gta gag ttt tat ggg gaa      864
Leu Arg Lys His Gly Val Val Gly Lys Phe Val Glu Phe Tyr Gly Glu
        275                 280                 285

ggc atg agt gaa ctg tct ttg gca gat cgt gcc acc ata gca aac atg      912
Gly Met Ser Glu Leu Ser Leu Ala Asp Arg Ala Thr Ile Ala Asn Met
    290                 295                 300

tct cct gag tat ggt gca acc atg ggc ttc ttt cct gtg gat cat gtc      960
Ser Pro Glu Tyr Gly Ala Thr Met Gly Phe Phe Pro Val Asp His Val
305                 310                 315                 320

act ttg caa tat ttg aga ctg act ggc aga agt gat gag act gtt tct     1008
Thr Leu Gln Tyr Leu Arg Leu Thr Gly Arg Ser Asp Glu Thr Val Ser
                325                 330                 335

atg ata gaa tcg tac tta cga gca aat aag atg ttt gtg gat tac agc     1056
Met Ile Glu Ser Tyr Leu Arg Ala Asn Lys Met Phe Val Asp Tyr Ser
            340                 345                 350

gag ccc caa gtg gag aga gtg tac tca tcg tat ttg gaa ctc aat ctt     1104
Glu Pro Gln Val Glu Arg Val Tyr Ser Ser Tyr Leu Glu Leu Asn Leu
        355                 360                 365

gag gat gta gaa ccc tgt gtc tca ggt cca aaa agg cct cat gac cgt     1152

Glu Asp Val Glu Pro Cys Val Ser Gly Pro Lys Arg Pro His Asp Arg
    370                 375                 380

gtc cct ttg aga gaa atg aag gta gac tgg cat gcc tgc ctc aac aat      1200
Val Pro Leu Arg Glu Met Lys Val Asp Trp His Ala Cys Leu Asn Asn
385                 390                 395                 400

aaa gtt gga ttc aag ggt ttt gct gta cca aaa gaa tct cag aat aag      1248
Lys Val Gly Phe Lys Gly Phe Ala Val Pro Lys Glu Ser Gln Asn Lys
                405                 410                 415

gtt gca gag ttc aca ttc caa ggg aca cca gca cat ctt agg cac ggt      1296
Val Ala Glu Phe Thr Phe Gln Gly Thr Pro Ala His Leu Arg His Gly
            420                 425                 430

gat gtt gtt ata gct gct atc acc agt tgt aca aat act tca aat cct      1344
Asp Val Val Ile Ala Ala Ile Thr Ser Cys Thr Asn Thr Ser Asn Pro
        435                 440                 445

agc gta atg ctt gga gct gca ttg gtt gca aag aaa gca tgt gaa ttg      1392
Ser Val Met Leu Gly Ala Ala Leu Val Ala Lys Lys Ala Cys Glu Leu
    450                 455                 460

gga ttg cag gtg aag cca tgg att aaa aca agc ctt gcc cca ggt tct      1440
Gly Leu Gln Val Lys Pro Trp Ile Lys Thr Ser Leu Ala Pro Gly Ser
465                 470                 475                 480

ggt gtt gtg act aag tat ttg cag agg agt ggc ttg cag aag tat ttg      1488
Gly Val Val Thr Lys Tyr Leu Gln Arg Ser Gly Leu Gln Lys Tyr Leu
                485                 490                 495

aat gag cta ggg ttt aat ata gtt ggg tat gga tgc acc aca tgc att      1536
Asn Glu Leu Gly Phe Asn Ile Val Gly Tyr Gly Cys Thr Thr Cys Ile
            500                 505                 510

gga aat tca ggg gat att aat gaa gct gtt gca tct gca att act gaa      1584
Gly Asn Ser Gly Asp Ile Asn Glu Ala Val Ala Ser Ala Ile Thr Glu
        515                 520                 525

aat gat ata gta gct gca gct gta ctg tct gga aat agg aac ttt gag      1632
Asn Asp Ile Val Ala Ala Ala Val Leu Ser Gly Asn Arg Asn Phe Glu
    530                 535                 540

ggc cga gtt cac cca tta aca aga gct aat tat ctt gct tct cct cct      1680
Gly Arg Val His Pro Leu Thr Arg Ala Asn Tyr Leu Ala Ser Pro Pro
545                 550                 555                 560

ctt gtt gtt gcc tat gcc ctt gct ggc aca gtg gac att gac ttt gac      1728
Leu Val Val Ala Tyr Ala Leu Ala Gly Thr Val Asp Ile Asp Phe Asp
                565                 570                 575

act gaa ccc att ggg ata ggg aag gat ggc aca aag atc ttc ttc agg      1776
Thr Glu Pro Ile Gly Ile Gly Lys Asp Gly Thr Lys Ile Phe Phe Arg
            580                 585                 590

gat att tgg cca tcc agt gaa gaa ata gca aat gtt gta caa tca agt      1824
Asp Ile Trp Pro Ser Ser Glu Glu Ile Ala Asn Val Val Gln Ser Ser
        595                 600                 605

gtg ctg cct gct atg ttt agg gac aca tat aat gcg atc acc caa ggg      1872
Val Leu Pro Ala Met Phe Arg Asp Thr Tyr Asn Ala Ile Thr Gln Gly
    610                 615                 620

aat ccc atg tgg aat aat tta tct gtt cca act ggc act ctt tat gct 1920
Asn Pro Met Trp Asn Asn Leu Ser Val Pro Thr Gly Thr Leu Tyr Ala
625                 630                 635                 640

tgg gac cct aca tca act tat att cat gag cca cct tat ttt aga gat 1968
Trp Asp Pro Thr Ser Thr Tyr Ile His Glu Pro Pro Tyr Phe Arg Asp
                645                 650                 655

atg agc atg tct ccc cca ggc tct cat gga gtg aaa gat gct tac tgt 2016
Met Ser Met Ser Pro Pro Gly Ser His Gly Val Lys Asp Ala Tyr Cys
            660                 665                 670

ttg ctc aac ttt gga gac agt atc aca act gac cac atc tct cca gct 2064
Leu Leu Asn Phe Gly Asp Ser Ile Thr Thr Asp His Ile Ser Pro Ala
        675                 680                 685

ggc agc ata cat aag gac agt cct gca gcc aga tac ctt ata gaa cgt 2112
Gly Ser Ile His Lys Asp Ser Pro Ala Ala Arg Tyr Leu Ile Glu Arg
    690                 695                 700

ggg gtt gat aga cgg gac ttc aac tcc tat gga agt cgc cgt ggt aat 2160
Gly Val Asp Arg Arg Asp Phe Asn Ser Tyr Gly Ser Arg Arg Gly Asn
705                 710                 715                 720

gat gag gtg atg gcc cga gga act ttt gcc aat att cgc att gtc aac 2208
Asp Glu Val Met Ala Arg Gly Thr Phe Ala Asn Ile Arg Ile Val Asn
                725                 730                 735

aaa ttt ttg aat gga gaa gtt gga cct aaa act att cat att cct tct 2256
Lys Phe Leu Asn Gly Glu Val Gly Pro Lys Thr Ile His Ile Pro Ser
            740                 745                 750

ggg gag aag tta tca gtc ttt gat gct gca gag aaa tac aag agt gag 2304
Gly Glu Lys Leu Ser Val Phe Asp Ala Ala Glu Lys Tyr Lys Ser Glu
        755                 760                 765

ggg cat gat atg att atc ttg gct ggt gct gag tat ggg agt gga agc 2352
Gly His Asp Met Ile Ile Leu Ala Gly Ala Glu Tyr Gly Ser Gly Ser
    770                 775                 780

tct cgt gat tgg gct gca aag ggg cca atg cta ctg ggc gtg aaa gct 2400
Ser Arg Asp Trp Ala Ala Lys Gly Pro Met Leu Leu Gly Val Lys Ala
785                 790                 795                 800

gtc ata gca aaa agt ttt gaa agg att cac cga agt aat ttg gtg gga 2448
Val Ile Ala Lys Ser Phe Glu Arg Ile His Arg Ser Asn Leu Val Gly
                805                 810                 815

atg ggt atc att cca ttg tgt ttt aag ccc ggg gat gat gct gat tct 2496
Met Gly Ile Ile Pro Leu Cys Phe Lys Pro Gly Asp Asp Ala Asp Ser
            820                 825                 830

ctt gga tta act ggc cat gaa cgt tac acc att gat cta cca agc aat 2544
Leu Gly Leu Thr Gly His Glu Arg Tyr Thr Ile Asp Leu Pro Ser Asn
        835                 840                 845

gtg aat gaa ata cga ccc ggc caa gat gtc aca gtg gtg aca gat gcc 2592
Val Asn Glu Ile Arg Pro Gly Gln Asp Val Thr Val Val Thr Asp Ala
    850                 855                 860

ggg aaa tca ttt gta tct aca ctg aga ttt gat aca gag gtt gaa cta 2640
Gly Lys Ser Phe Val Ser Thr Leu Arg Phe Asp Thr Glu Val Glu Leu
865                 870                 875                 880

gct tac ttc aac cat gga ggc atc tta caa tat gtc atc aga aac atg     2688
Ala Tyr Phe Asn His Gly Gly Ile Leu Gln Tyr Val Ile Arg Asn Met
                885                 890                 895

gtc aat gct aaa cat tga                                             2706
Val Asn Ala Lys His
            900

<210>  163
<211>  901
<212>  PRT
<213>  Glycine max

<400>  163

Met Ala Thr Glu Asn Pro Phe Asn Ser Ile Leu Thr Thr Leu Glu Lys
1               5                   10                  15

Pro Gly Gly Ala Gly Glu Phe Gly Lys Tyr Phe Ser Leu Pro Ala Leu
            20                  25                  30

Asn Asp Arg Arg Ile Asp Arg Leu Pro Tyr Ser Val Arg Ile Leu Leu
        35                  40                  45

Glu Ser Ala Ile Arg Asn Cys Asp Glu Phe Gln Val Lys Ser Asn Asp
    50                  55                  60

Val Glu Lys Ile Ile Asp Trp Glu Asn Thr Ser Pro Lys Leu Val Glu
65                  70                  75                  80

Ile Pro Phe Lys Pro Ala Arg Val Leu Leu Gln Asp Phe Thr Gly Val
                85                  90                  95

Pro Ala Val Val Asp Leu Ala Cys Met Arg Asp Ala Met Asn Lys Leu
            100                 105                 110

Gly Gly Asp Ser Asn Lys Ile Asn Pro Leu Val Pro Val Asp Leu Val
        115                 120                 125

Ile Asp His Ser Val Gln Val Asp Val Ala Arg Ser Glu Asn Ala Val
    130                 135                 140

Gln Ala Asn Met Glu Leu Glu Phe Gln Arg Asn Lys Glu Arg Phe Gly
145                 150                 155                 160

Phe Leu Lys Trp Gly Ser Asn Ala Phe Asn Asn Met Leu Val Val Pro
                165                 170                 175

Pro Gly Ser Gly Ile Val His Gln Val Asn Leu Glu Tyr Leu Gly Arg
            180                 185                 190

Val Val Phe Asn Thr Asn Gly Val Leu Tyr Pro Asp Ser Val Val Gly
        195                 200                 205

Thr Asp Ser His Thr Thr Met Ile Asp Gly Leu Gly Val Ala Gly Trp
    210                 215                 220

Gly Val Gly Gly Ile Glu Ala Glu Ala Ala Met Leu Gly Gln Pro Met
225                 230                 235                 240

Ser Met Val Leu Pro Gly Val Val Gly Phe Lys Leu Leu Gly Lys Leu
                245                 250                 255

Arg Asp Gly Val Thr Ala Thr Asp Leu Val Leu Thr Val Thr Gln Met
            260                 265                 270

Leu Arg Lys His Gly Val Val Gly Lys Phe Val Glu Phe Tyr Gly Glu
        275                 280                 285

Gly Met Ser Glu Leu Ser Leu Ala Asp Arg Ala Thr Ile Ala Asn Met
    290                 295                 300

Ser Pro Glu Tyr Gly Ala Thr Met Gly Phe Phe Pro Val Asp His Val
305                 310                 315                 320

Thr Leu Gln Tyr Leu Arg Leu Thr Gly Arg Ser Asp Glu Thr Val Ser
                325                 330                 335

Met Ile Glu Ser Tyr Leu Arg Ala Asn Lys Met Phe Val Asp Tyr Ser
            340                 345                 350

Glu Pro Gln Val Glu Arg Val Tyr Ser Ser Tyr Leu Glu Leu Asn Leu
        355                 360                 365

Glu Asp Val Glu Pro Cys Val Ser Gly Pro Lys Arg Pro His Asp Arg
    370                 375                 380

Val Pro Leu Arg Glu Met Lys Val Asp Trp His Ala Cys Leu Asn Asn
385                 390                 395                 400

Lys Val Gly Phe Lys Gly Phe Ala Val Pro Lys Glu Ser Gln Asn Lys
                405                 410                 415

Val Ala Glu Phe Thr Phe Gln Gly Thr Pro Ala His Leu Arg His Gly
            420                 425                 430

Asp Val Val Ile Ala Ala Ile Thr Ser Cys Thr Asn Thr Ser Asn Pro

435                 440                 445

Ser Val Met Leu Gly Ala Ala Leu Val Ala Lys Lys Ala Cys Glu Leu
    450                 455                 460

Gly Leu Gln Val Lys Pro Trp Ile Lys Thr Ser Leu Ala Pro Gly Ser
465                 470                 475                 480

Gly Val Val Thr Lys Tyr Leu Gln Arg Ser Gly Leu Gln Lys Tyr Leu
                485                 490                 495

Asn Glu Leu Gly Phe Asn Ile Val Gly Tyr Gly Cys Thr Thr Cys Ile
            500                 505                 510

Gly Asn Ser Gly Asp Ile Asn Glu Ala Val Ala Ser Ala Ile Thr Glu
        515                 520                 525

Asn Asp Ile Val Ala Ala Ala Val Leu Ser Gly Asn Arg Asn Phe Glu
    530                 535                 540

Gly Arg Val His Pro Leu Thr Arg Ala Asn Tyr Leu Ala Ser Pro Pro
545                 550                 555                 560

Leu Val Val Ala Tyr Ala Leu Ala Gly Thr Val Asp Ile Asp Phe Asp
                565                 570                 575

Thr Glu Pro Ile Gly Ile Gly Lys Asp Gly Thr Lys Ile Phe Phe Arg
            580                 585                 590

Asp Ile Trp Pro Ser Ser Glu Glu Ile Ala Asn Val Val Gln Ser Ser
        595                 600                 605

Val Leu Pro Ala Met Phe Arg Asp Thr Tyr Asn Ala Ile Thr Gln Gly
    610                 615                 620

Asn Pro Met Trp Asn Asn Leu Ser Val Pro Thr Gly Thr Leu Tyr Ala
625                 630                 635                 640

Trp Asp Pro Thr Ser Thr Tyr Ile His Glu Pro Pro Tyr Phe Arg Asp
                645                 650                 655

Met Ser Met Ser Pro Pro Gly Ser His Gly Val Lys Asp Ala Tyr Cys
            660                 665                 670

Leu Leu Asn Phe Gly Asp Ser Ile Thr Thr Asp His Ile Ser Pro Ala
        675                 680                 685

Gly Ser Ile His Lys Asp Ser Pro Ala Ala Arg Tyr Leu Ile Glu Arg
    690                 695                 700

Gly Val Asp Arg Arg Asp Phe Asn Ser Tyr Gly Ser Arg Arg Gly Asn
705                 710                 715                 720

Asp Glu Val Met Ala Arg Gly Thr Phe Ala Asn Ile Arg Ile Val Asn
                725                 730                 735

Lys Phe Leu Asn Gly Glu Val Gly Pro Lys Thr Ile His Ile Pro Ser
            740                 745                 750

Gly Glu Lys Leu Ser Val Phe Asp Ala Ala Glu Lys Tyr Lys Ser Glu
        755                 760                 765

Gly His Asp Met Ile Ile Leu Ala Gly Ala Glu Tyr Gly Ser Gly Ser
    770                 775                 780

Ser Arg Asp Trp Ala Ala Lys Gly Pro Met Leu Leu Gly Val Lys Ala
785                 790                 795                 800

Val Ile Ala Lys Ser Phe Glu Arg Ile His Arg Ser Asn Leu Val Gly
                805                 810                 815

Met Gly Ile Ile Pro Leu Cys Phe Lys Pro Gly Asp Asp Ala Asp Ser
            820                 825                 830

Leu Gly Leu Thr Gly His Glu Arg Tyr Thr Ile Asp Leu Pro Ser Asn
        835                 840                 845

Val Asn Glu Ile Arg Pro Gly Gln Asp Val Thr Val Val Thr Asp Ala
    850                 855                 860

Gly Lys Ser Phe Val Ser Thr Leu Arg Phe Asp Thr Glu Val Glu Leu
865                 870                 875                 880

Ala Tyr Phe Asn His Gly Gly Ile Leu Gln Tyr Val Ile Arg Asn Met
                885                 890                 895

Val Asn Ala Lys His
            900

<210>  164
<211>  9
<212>  PRT
<213>  Glycine max

<400>  164

Phe Leu Asn Gly Glu Val Gly Pro Lys
1               5

<210>  165
<211>  9
<212>  PRT
<213>  Glycine max

<400>  165

Leu Ser Val Phe Asp Ala Ala Glu Lys
1               5

<210>  166
<211>  9
<212>  PRT
<213>  Glycine max

<400>  166

Leu Ser Val Phe Asp Val Ala Glu Lys
1               5

<210>  167
<211>  12
<212>  PRT
<213>  Glycine max

<400>  167

Thr Ser Leu Ala Pro Gly Ser Gly Val Val Thr Lys
1               5                   10

<210>  168
<211>  2706
<212>  DNA
<213>  Glycine max

<220>
<221>  CDS
<222>  (1)..(2706)

<400>  168
atg gcg act gag aac cct ttc aat agc att ttg agg acg ctc gag aag      48
Met Ala Thr Glu Asn Pro Phe Asn Ser Ile Leu Arg Thr Leu Glu Lys
1               5                   10                  15

ccc ggc ggc gct ggc gag ttt gga aag tac ttt agc ttg ccc gct ctc      96
Pro Gly Gly Ala Gly Glu Phe Gly Lys Tyr Phe Ser Leu Pro Ala Leu
            20                  25                  30

aac gat cct aga atc gat aga ctt cct tac tct gtg agg ata ctt ttg      144
Asn Asp Pro Arg Ile Asp Arg Leu Pro Tyr Ser Val Arg Ile Leu Leu
        35                  40                  45

gaa tct gca atc cgt aac tgt gat gag ttt caa gtt aag agt aac gac      192
Glu Ser Ala Ile Arg Asn Cys Asp Glu Phe Gln Val Lys Ser Asn Asp
    50                  55                  60

att gaa aaa att att gat tgg gag aat acc tct ccc aag ctg gtg gag 240
Ile Glu Lys Ile Ile Asp Trp Glu Asn Thr Ser Pro Lys Leu Val Glu
65                  70                  75                  80

att cca ttc aag cca gct aga gtg ctt ctg cag gac ttt act ggg gtg 288
Ile Pro Phe Lys Pro Ala Arg Val Leu Leu Gln Asp Phe Thr Gly Val
                85                  90                  95

cct gct gtg gtt gat ctt gct tgc atg cga gat gca atg aac aaa ctt 336
Pro Ala Val Val Asp Leu Ala Cys Met Arg Asp Ala Met Asn Lys Leu
            100                 105                 110

ggt ggt gat tct aat aaa att aac ccc ttg gta cca gtt gat ctt gtc 384
Gly Gly Asp Ser Asn Lys Ile Asn Pro Leu Val Pro Val Asp Leu Val
        115                 120                 125

att gat cac tct gtt caa gtt gat gtg gca aga tca gaa aat gct gtc 432
Ile Asp His Ser Val Gln Val Asp Val Ala Arg Ser Glu Asn Ala Val
    130                 135                 140

cag gca aat atg gaa ctt gaa ttc caa aga aac aag gaa aga ttt ggt 480
Gln Ala Asn Met Glu Leu Glu Phe Gln Arg Asn Lys Glu Arg Phe Gly
145                 150                 155                 160

ttc ctc aaa tgg ggt tct aat gca ttc aat aat atg ctt gtc gtt cct 528
Phe Leu Lys Trp Gly Ser Asn Ala Phe Asn Asn Met Leu Val Val Pro
                165                 170                 175

cct gga tca ggg ata gtt cat cag gtt aat tta gaa tac ctt ggt aga 576
Pro Gly Ser Gly Ile Val His Gln Val Asn Leu Glu Tyr Leu Gly Arg
            180                 185                 190

gtt gtg ttc aac aca aat ggt gtg ctt tat cca gac agc gtt gtc gga 624
Val Val Phe Asn Thr Asn Gly Val Leu Tyr Pro Asp Ser Val Val Gly
        195                 200                 205

act gat tca cac act act atg ata gat ggc ctg ggt gtt gct gga tgg 672
Thr Asp Ser His Thr Thr Met Ile Asp Gly Leu Gly Val Ala Gly Trp
    210                 215                 220

gga gtt ggt gga ata gaa gca gaa gct gca atg ctt ggc cag ccc atg 720
Gly Val Gly Gly Ile Glu Ala Glu Ala Ala Met Leu Gly Gln Pro Met
225                 230                 235                 240

agc atg gtc cta ccg ggt gtt gtt ggg ttc aaa tta tta ggc aaa cta 768
Ser Met Val Leu Pro Gly Val Val Gly Phe Lys Leu Leu Gly Lys Leu
                245                 250                 255

cga gat ggt gtc aca gct act gac ttg gtg ttg act gtt act caa atg 816
Arg Asp Gly Val Thr Ala Thr Asp Leu Val Leu Thr Val Thr Gln Met
            260                 265                 270

cta aga aag cat ggg gtt gtt ggc aag ttt gta gag ttt tat ggg gaa 864
Leu Arg Lys His Gly Val Val Gly Lys Phe Val Glu Phe Tyr Gly Glu
        275                 280                 285

ggc atg agt gaa ctg tct ttg gca gat cgt gcc act ata gca aac atg 912
Gly Met Ser Glu Leu Ser Leu Ala Asp Arg Ala Thr Ile Ala Asn Met
    290                 295                 300

tct cct gag tat ggt gca acc atg ggc ttc ttt cct gtg gat cat gtc 960
Ser Pro Glu Tyr Gly Ala Thr Met Gly Phe Phe Pro Val Asp His Val

305                 310                 315                 320

act ttg caa tat ttg aga ctg act ggc aga agt gat gag act gtt tct        1008
Thr Leu Gln Tyr Leu Arg Leu Thr Gly Arg Ser Asp Glu Thr Val Ser
                325                 330                 335

atg ata gaa tcc tat tta cga gca aat aag atg ttt gtg gat tat agc        1056
Met Ile Glu Ser Tyr Leu Arg Ala Asn Lys Met Phe Val Asp Tyr Ser
            340                 345                 350

gag ccc caa gtg gag aga gtg tac tca tcc tat ttg gaa ctc aat ctt        1104
Glu Pro Gln Val Glu Arg Val Tyr Ser Ser Tyr Leu Glu Leu Asn Leu
        355                 360                 365

gag gat gta gag ccc tgt gtc tca ggt cca aaa agg cct cac gac cgt        1152
Glu Asp Val Glu Pro Cys Val Ser Gly Pro Lys Arg Pro His Asp Arg
    370                 375                 380

gtc cct ttg aga gaa atg aag gta gac tgg cat gcc tgc ctc aac aat        1200
Val Pro Leu Arg Glu Met Lys Val Asp Trp His Ala Cys Leu Asn Asn
385                 390                 395                 400

aaa gtt gga ttt aag ggt ttt gct gta tca aaa gaa tct cag aat aag        1248
Lys Val Gly Phe Lys Gly Phe Ala Val Ser Lys Glu Ser Gln Asn Lys
                405                 410                 415

gtt gca gag ttc aca ttc caa ggg aca cca gca cat ctt agg cat ggt        1296
Val Ala Glu Phe Thr Phe Gln Gly Thr Pro Ala His Leu Arg His Gly
            420                 425                 430

gat gtt gtt ata gct gct atc acc agt tgt aca aat act tca aat cct        1344
Asp Val Val Ile Ala Ala Ile Thr Ser Cys Thr Asn Thr Ser Asn Pro
        435                 440                 445

agc gta atg ctt gga gct gca ttg gtt gca aag aaa gca tgt gaa ttg        1392
Ser Val Met Leu Gly Ala Ala Leu Val Ala Lys Lys Ala Cys Glu Leu
    450                 455                 460

gga ttg cag gtg aag cca tgg att aaa aca agt ctt gcc cca ggt tct        1440
Gly Leu Gln Val Lys Pro Trp Ile Lys Thr Ser Leu Ala Pro Gly Ser
465                 470                 475                 480

ggt gtt gta act aag tat ttg cag agg agt ggc ttg cag aag tat ttg        1488
Gly Val Val Thr Lys Tyr Leu Gln Arg Ser Gly Leu Gln Lys Tyr Leu
                485                 490                 495

aat gag cta ggg ttt aat ata gtt ggg tat gga tgc acc aca tgc att        1536
Asn Glu Leu Gly Phe Asn Ile Val Gly Tyr Gly Cys Thr Thr Cys Ile
            500                 505                 510

gga aat tca ggg gat att aat gaa gct gtt gca tct gcg att acc gaa        1584
Gly Asn Ser Gly Asp Ile Asn Glu Ala Val Ala Ser Ala Ile Thr Glu
        515                 520                 525

aat gat ata gta gct gca gct gta ttg tct gga aat agg aac ttt gag        1632
Asn Asp Ile Val Ala Ala Ala Val Leu Ser Gly Asn Arg Asn Phe Glu
    530                 535                 540

ggc cga gtt cac cca tta aca aga gct aat tat ctt gct tct cct cct        1680
Gly Arg Val His Pro Leu Thr Arg Ala Asn Tyr Leu Ala Ser Pro Pro
545                 550                 555                 560

ctt gtt gtt gcc tat gcc ctt gct ggc aca gtg gac att gac ttt gac        1728

Leu Val Val Ala Tyr Ala Leu Ala Gly Thr Val Asp Ile Asp Phe Asp
                565                 570                 575

act gaa ccc att ggg ata ggg aag gat gga aca gag atc ttc ttc aag      1776
Thr Glu Pro Ile Gly Ile Gly Lys Asp Gly Thr Glu Ile Phe Phe Lys
            580                 585                 590

gat att tgg cca tcc agt gaa gaa ata gca aat gtt gta caa tca agt      1824
Asp Ile Trp Pro Ser Ser Glu Glu Ile Ala Asn Val Val Gln Ser Ser
        595                 600                 605

gtg ctg cct gat atg ttt agg gac aca tat aat gca atc acc caa ggg      1872
Val Leu Pro Asp Met Phe Arg Asp Thr Tyr Asn Ala Ile Thr Gln Gly
    610                 615                 620

aat ccc atg tgg aat aat tta tct gtt cca acg ggc act ctt tat gct      1920
Asn Pro Met Trp Asn Asn Leu Ser Val Pro Thr Gly Thr Leu Tyr Ala
625                 630                 635                 640

tgg gac cct aca tcg act tat att cat gag cca cct tat ttt aga gat      1968
Trp Asp Pro Thr Ser Thr Tyr Ile His Glu Pro Pro Tyr Phe Arg Asp
                645                 650                 655

atg agc atg tct ccc cca ggc tct cat gga gtg aag gat gct tac tgt      2016
Met Ser Met Ser Pro Pro Gly Ser His Gly Val Lys Asp Ala Tyr Cys
            660                 665                 670

ttg ctc aac ttt gga gac agt atc aca act gac cac atc tct cca gct      2064
Leu Leu Asn Phe Gly Asp Ser Ile Thr Thr Asp His Ile Ser Pro Ala
        675                 680                 685

ggc agc ata cat aag gac agt cct gcg gcc aga tac ctt ata gaa cgt      2112
Gly Ser Ile His Lys Asp Ser Pro Ala Ala Arg Tyr Leu Ile Glu Arg
    690                 695                 700

ggg gtt gat aga cgg gac ttc aac tcc tat gga agt cgc cgt ggt aat      2160
Gly Val Asp Arg Arg Asp Phe Asn Ser Tyr Gly Ser Arg Arg Gly Asn
705                 710                 715                 720

gat gag gtg atg gcc cga gga acg ttt gcc aat att cgc att gtc aac      2208
Asp Glu Val Met Ala Arg Gly Thr Phe Ala Asn Ile Arg Ile Val Asn
                725                 730                 735

aaa ttt ttg aat gga gaa gtt gga cct aaa act att cat att cct tct      2256
Lys Phe Leu Asn Gly Glu Val Gly Pro Lys Thr Ile His Ile Pro Ser
            740                 745                 750

ggg gag aag ttg tca gtc ttt gac gtt gca gag aaa tac aag agt gaa      2304
Gly Glu Lys Leu Ser Val Phe Asp Val Ala Glu Lys Tyr Lys Ser Glu
        755                 760                 765

ggg cat gat atg att atc ttg gct ggt gct gag tat ggg agt gga agc      2352
Gly His Asp Met Ile Ile Leu Ala Gly Ala Glu Tyr Gly Ser Gly Ser
    770                 775                 780

tct cgt gat tgg gct gca aag ggg cca atg cta ctg ggc gtg aaa gct      2400
Ser Arg Asp Trp Ala Ala Lys Gly Pro Met Leu Leu Gly Val Lys Ala
785                 790                 795                 800

gtc ata gca aaa agt ttt gaa agg att cac cga agt aat ttg gtg gga      2448
Val Ile Ala Lys Ser Phe Glu Arg Ile His Arg Ser Asn Leu Val Gly
                805                 810                 815

atg ggt atc att cct ttg tgt ttt aag ccc ggg gag gat gct gat tct      2496
Met Gly Ile Ile Pro Leu Cys Phe Lys Pro Gly Glu Asp Ala Asp Ser
            820                 825                 830

ctt gga tta act ggt caa gaa cgt tac acc att gat cta cca agc aat      2544
Leu Gly Leu Thr Gly Gln Glu Arg Tyr Thr Ile Asp Leu Pro Ser Asn
        835                 840                 845

gtg aat gaa ata cga ccc ggt caa gat gtc aca gtg gtg aca gac acc      2592
Val Asn Glu Ile Arg Pro Gly Gln Asp Val Thr Val Val Thr Asp Thr
    850                 855                 860

ggg aaa tca ttt gta tct aca ttg aga ttt gat act gag gtt gaa cta      2640
Gly Lys Ser Phe Val Ser Thr Leu Arg Phe Asp Thr Glu Val Glu Leu
865                 870                 875                 880

gct tac ttc aac cat gga ggt atc tta caa tat gtc atc aga aac ttg      2688
Ala Tyr Phe Asn His Gly Gly Ile Leu Gln Tyr Val Ile Arg Asn Leu
                885                 890                 895

atc aat gct aaa cat tga                                              2706
Ile Asn Ala Lys His
            900

<210>  169
<211>  901
<212>  PRT
<213>  Glycine max

<400>  169

Met Ala Thr Glu Asn Pro Phe Asn Ser Ile Leu Arg Thr Leu Glu Lys
1               5                   10                  15

Pro Gly Gly Ala Gly Glu Phe Gly Lys Tyr Phe Ser Leu Pro Ala Leu
            20                  25                  30

Asn Asp Pro Arg Ile Asp Arg Leu Pro Tyr Ser Val Arg Ile Leu Leu
        35                  40                  45

Glu Ser Ala Ile Arg Asn Cys Asp Glu Phe Gln Val Lys Ser Asn Asp
    50                  55                  60

Ile Glu Lys Ile Ile Asp Trp Glu Asn Thr Ser Pro Lys Leu Val Glu
65                  70                  75                  80

Ile Pro Phe Lys Pro Ala Arg Val Leu Leu Gln Asp Phe Thr Gly Val
                85                  90                  95

Pro Ala Val Val Asp Leu Ala Cys Met Arg Asp Ala Met Asn Lys Leu
            100                 105                 110

Gly Gly Asp Ser Asn Lys Ile Asn Pro Leu Val Pro Val Asp Leu Val
        115                 120                 125

Ile Asp His Ser Val Gln Val Asp Val Ala Arg Ser Glu Asn Ala Val
    130                 135                 140

Gln Ala Asn Met Glu Leu Glu Phe Gln Arg Asn Lys Glu Arg Phe Gly
145                 150                 155                 160

Phe Leu Lys Trp Gly Ser Asn Ala Phe Asn Asn Met Leu Val Val Pro
                165                 170                 175

Pro Gly Ser Gly Ile Val His Gln Val Asn Leu Glu Tyr Leu Gly Arg
            180                 185                 190

Val Val Phe Asn Thr Asn Gly Val Leu Tyr Pro Asp Ser Val Val Gly
        195                 200                 205

Thr Asp Ser His Thr Thr Met Ile Asp Gly Leu Gly Val Ala Gly Trp
    210                 215                 220

Gly Val Gly Gly Ile Glu Ala Glu Ala Ala Met Leu Gly Gln Pro Met
225                 230                 235                 240

Ser Met Val Leu Pro Gly Val Val Gly Phe Lys Leu Leu Gly Lys Leu
                245                 250                 255

Arg Asp Gly Val Thr Ala Thr Asp Leu Val Leu Thr Val Thr Gln Met
            260                 265                 270

Leu Arg Lys His Gly Val Val Gly Lys Phe Val Glu Phe Tyr Gly Glu
        275                 280                 285

Gly Met Ser Glu Leu Ser Leu Ala Asp Arg Ala Thr Ile Ala Asn Met
    290                 295                 300

Ser Pro Glu Tyr Gly Ala Thr Met Gly Phe Phe Pro Val Asp His Val
305                 310                 315                 320

Thr Leu Gln Tyr Leu Arg Leu Thr Gly Arg Ser Asp Glu Thr Val Ser
                325                 330                 335

Met Ile Glu Ser Tyr Leu Arg Ala Asn Lys Met Phe Val Asp Tyr Ser
            340                 345                 350

Glu Pro Gln Val Glu Arg Val Tyr Ser Ser Tyr Leu Glu Leu Asn Leu
        355                 360                 365

Glu Asp Val Glu Pro Cys Val Ser Gly Pro Lys Arg Pro His Asp Arg
    370                 375                 380

Val Pro Leu Arg Glu Met Lys Val Asp Trp His Ala Cys Leu Asn Asn
385                 390                 395                 400

Lys Val Gly Phe Lys Gly Phe Ala Val Ser Lys Glu Ser Gln Asn Lys
                405                 410                 415

Val Ala Glu Phe Thr Phe Gln Gly Thr Pro Ala His Leu Arg His Gly
            420                 425                 430

Asp Val Val Ile Ala Ala Ile Thr Ser Cys Thr Asn Thr Ser Asn Pro
        435                 440                 445

Ser Val Met Leu Gly Ala Ala Leu Val Ala Lys Lys Ala Cys Glu Leu
    450                 455                 460

Gly Leu Gln Val Lys Pro Trp Ile Lys Thr Ser Leu Ala Pro Gly Ser
465                 470                 475                 480

Gly Val Val Thr Lys Tyr Leu Gln Arg Ser Gly Leu Gln Lys Tyr Leu
                485                 490                 495

Asn Glu Leu Gly Phe Asn Ile Val Gly Tyr Gly Cys Thr Thr Cys Ile
            500                 505                 510

Gly Asn Ser Gly Asp Ile Asn Glu Ala Val Ala Ser Ala Ile Thr Glu
        515                 520                 525

Asn Asp Ile Val Ala Ala Ala Val Leu Ser Gly Asn Arg Asn Phe Glu
    530                 535                 540

Gly Arg Val His Pro Leu Thr Arg Ala Asn Tyr Leu Ala Ser Pro Pro
545                 550                 555                 560

Leu Val Val Ala Tyr Ala Leu Ala Gly Thr Val Asp Ile Asp Phe Asp
                565                 570                 575

Thr Glu Pro Ile Gly Ile Gly Lys Asp Gly Thr Glu Ile Phe Phe Lys
            580                 585                 590

Asp Ile Trp Pro Ser Ser Glu Glu Ile Ala Asn Val Val Gln Ser Ser
        595                 600                 605

Val Leu Pro Asp Met Phe Arg Asp Thr Tyr Asn Ala Ile Thr Gln Gly
    610                 615                 620

Asn Pro Met Trp Asn Asn Leu Ser Val Pro Thr Gly Thr Leu Tyr Ala

625                 630                 635                 640

Trp Asp Pro Thr Ser Thr Tyr Ile His Glu Pro Pro Tyr Phe Arg Asp
                645                 650                 655

Met Ser Met Ser Pro Pro Gly Ser His Gly Val Lys Asp Ala Tyr Cys
            660                 665                 670

Leu Leu Asn Phe Gly Asp Ser Ile Thr Thr Asp His Ile Ser Pro Ala
        675                 680                 685

Gly Ser Ile His Lys Asp Ser Pro Ala Ala Arg Tyr Leu Ile Glu Arg
    690                 695                 700

Gly Val Asp Arg Arg Asp Phe Asn Ser Tyr Gly Ser Arg Arg Gly Asn
705                 710                 715                 720

Asp Glu Val Met Ala Arg Gly Thr Phe Ala Asn Ile Arg Ile Val Asn
                725                 730                 735

Lys Phe Leu Asn Gly Glu Val Gly Pro Lys Thr Ile His Ile Pro Ser
            740                 745                 750

Gly Glu Lys Leu Ser Val Phe Asp Val Ala Glu Lys Tyr Lys Ser Glu
        755                 760                 765

Gly His Asp Met Ile Ile Leu Ala Gly Ala Glu Tyr Gly Ser Gly Ser
    770                 775                 780

Ser Arg Asp Trp Ala Ala Lys Gly Pro Met Leu Leu Gly Val Lys Ala
785                 790                 795                 800

Val Ile Ala Lys Ser Phe Glu Arg Ile His Arg Ser Asn Leu Val Gly
                805                 810                 815

Met Gly Ile Ile Pro Leu Cys Phe Lys Pro Gly Glu Asp Ala Asp Ser
            820                 825                 830

Leu Gly Leu Thr Gly Gln Glu Arg Tyr Thr Ile Asp Leu Pro Ser Asn
        835                 840                 845

Val Asn Glu Ile Arg Pro Gly Gln Asp Val Thr Val Val Thr Asp Thr
    850                 855                 860

Gly Lys Ser Phe Val Ser Thr Leu Arg Phe Asp Thr Glu Val Glu Leu
865                 870                 875                 880

Ala Tyr Phe Asn His Gly Gly Ile Leu Gln Tyr Val Ile Arg Asn Leu
                885                 890                 895

Ile Asn Ala Lys His
            900

<210>  170
<211>  9
<212>  PRT
<213>  Glycine max

<400>  170

Phe Leu Asn Gly Glu Val Gly Pro Lys
1               5

<210>  171
<211>  9
<212>  PRT
<213>  Glycine max

<400>  171

Leu Ser Val Phe Asp Val Ala Glu Lys
1               5

<210>  172
<211>  12
<212>  PRT
<213>  Glycine max

<400>  172

Thr Ser Leu Ala Pro Gly Ser Gly Val Val Thr Lys
1               5                   10

<210>  173
<211>  2292
<212>  DNA
<213>  Glycine max

<220>
<221>  CDS
<222>  (1)..(2292)

<400>  173
atg gca tct cac atc gtt gga tac ccc cgt atg ggt ccc aag aga gag      48
Met Ala Ser His Ile Val Gly Tyr Pro Arg Met Gly Pro Lys Arg Glu
1               5                   10                  15

ctc aag ttc gct ctc gag tct ttc tgg gat ggc aag agc agc gcc gag      96
Leu Lys Phe Ala Leu Glu Ser Phe Trp Asp Gly Lys Ser Ser Ala Glu
            20                  25                  30

gat ttg cag aag gtg tct tct gat ctc agg gca tcc atc tgg aag cag      144
Asp Leu Gln Lys Val Ser Ser Asp Leu Arg Ala Ser Ile Trp Lys Gln
        35                  40                  45

atg gct gat gct ggg atc aag tac atc ccc agc aac act ttc tct cac 192
Met Ala Asp Ala Gly Ile Lys Tyr Ile Pro Ser Asn Thr Phe Ser His
    50                  55                  60

tat gac cag gtt ctc gac gcc acc gcc acc ctc ggt gcc gtt cca cca 240
Tyr Asp Gln Val Leu Asp Ala Thr Ala Thr Leu Gly Ala Val Pro Pro
65                  70                  75                  80

agg tac ggc tgg acc ggc ggc gag att ggg ttt gat acc tac ttc tcc 288
Arg Tyr Gly Trp Thr Gly Gly Glu Ile Gly Phe Asp Thr Tyr Phe Ser
                85                  90                  95

atg gcc aga ggt aat gct acc gtg cca gct atg gag atg acc aag tgg 336
Met Ala Arg Gly Asn Ala Thr Val Pro Ala Met Glu Met Thr Lys Trp
            100                 105                 110

ttc gac acc aac tac cac ttt att gtc cct gaa ttg ggc cct gat gtg 384
Phe Asp Thr Asn Tyr His Phe Ile Val Pro Glu Leu Gly Pro Asp Val
        115                 120                 125

aac ttc acc tat gct tct cac aag gct gtt gat gaa tac aag gag gcc 432
Asn Phe Thr Tyr Ala Ser His Lys Ala Val Asp Glu Tyr Lys Glu Ala
    130                 135                 140

aag gcg ctt gga gtg gat acc gtt ccg gtc ctc gtt ggc cct gtt aca 480
Lys Ala Leu Gly Val Asp Thr Val Pro Val Leu Val Gly Pro Val Thr
145                 150                 155                 160

tac ctg ttg ctc tcc aag cct gcc aag gga gtt gag aaa tcc ttt tct 528
Tyr Leu Leu Leu Ser Lys Pro Ala Lys Gly Val Glu Lys Ser Phe Ser
                165                 170                 175

ctc ctc tct ctc ctt ccc aag gtt ctt gct gtc tac aag gaa gtt att 576
Leu Leu Ser Leu Leu Pro Lys Val Leu Ala Val Tyr Lys Glu Val Ile
            180                 185                 190

gct gac ctt aag gca gct ggt gct tca tgg att cag ttt gat gag cct 624
Ala Asp Leu Lys Ala Ala Gly Ala Ser Trp Ile Gln Phe Asp Glu Pro
        195                 200                 205

acc ctt gtc ttg gac ctt gag tct cac aag ttg caa gca ttc act gac 672
Thr Leu Val Leu Asp Leu Glu Ser His Lys Leu Gln Ala Phe Thr Asp
    210                 215                 220

gca tat gca gaa ctt gcg cct gct ttg tct ggt ttg aat gtt ctt gtt 720
Ala Tyr Ala Glu Leu Ala Pro Ala Leu Ser Gly Leu Asn Val Leu Val
225                 230                 235                 240

gag acc tac ttt gct gac atc cct gct gag gca tac aag acc ctc aca 768
Glu Thr Tyr Phe Ala Asp Ile Pro Ala Glu Ala Tyr Lys Thr Leu Thr
                245                 250                 255

tct ctg aat ggc gtc act gca tat gga ttt gat ttg gtc cgt gga acc 816
Ser Leu Asn Gly Val Thr Ala Tyr Gly Phe Asp Leu Val Arg Gly Thr
            260                 265                 270

aac act ctt gat ttg atc aag ggt gga ttt ccc agc gga aaa tac ctc 864
Asn Thr Leu Asp Leu Ile Lys Gly Gly Phe Pro Ser Gly Lys Tyr Leu
        275                 280                 285

ttt gct gga gtg gtt gat gga agg aac atc tgg gcc aat gac ctt gct 912
Phe Ala Gly Val Val Asp Gly Arg Asn Ile Trp Ala Asn Asp Leu Ala

290                 295                 300

gct tct ctc act acc ttg cag ggt ctt gag ggc att gtg ggc aaa gat      960
Ala Ser Leu Thr Thr Leu Gln Gly Leu Glu Gly Ile Val Gly Lys Asp
305                 310                 315                 320

aag ctt gtt gtg tcc acc tcc tcc tcc ctt ctt cac act gct gtt gat      1008
Lys Leu Val Val Ser Thr Ser Ser Ser Leu Leu His Thr Ala Val Asp
                325                 330                 335

cta gtt aac gag acc aag ttg gat gat gag atc aag tca tgg cta gct      1056
Leu Val Asn Glu Thr Lys Leu Asp Asp Glu Ile Lys Ser Trp Leu Ala
            340                 345                 350

ttt gct gcc caa aaa att gtt gaa gtt aac gca ttg gct aaa gca ttg      1104
Phe Ala Ala Gln Lys Ile Val Glu Val Asn Ala Leu Ala Lys Ala Leu
        355                 360                 365

tct ggc cac aag gat gag gcc ttc ttc tct ggt aat gct gct gct ctg      1152
Ser Gly His Lys Asp Glu Ala Phe Phe Ser Gly Asn Ala Ala Ala Leu
    370                 375                 380

gct tca agg aag tct tct cca aga gtg acc aac gag gct gtt cag aag      1200
Ala Ser Arg Lys Ser Ser Pro Arg Val Thr Asn Glu Ala Val Gln Lys
385                 390                 395                 400

gct gct gct gca ttg aag ggt tca gat cat cgc cgt gca aca aat gtc      1248
Ala Ala Ala Ala Leu Lys Gly Ser Asp His Arg Arg Ala Thr Asn Val
                405                 410                 415

agt gcc aga ctg gat tct caa caa aag aag ctc aac ctt cca atc ctg      1296
Ser Ala Arg Leu Asp Ser Gln Gln Lys Lys Leu Asn Leu Pro Ile Leu
            420                 425                 430

cca acc acc act att gga tcc ttc cct cag act gta gaa ctg agg agg      1344
Pro Thr Thr Thr Ile Gly Ser Phe Pro Gln Thr Val Glu Leu Arg Arg
        435                 440                 445

gta cgc cgt gaa ttc aag gct aac aag atc tcc gag gaa gag tat gta      1392
Val Arg Arg Glu Phe Lys Ala Asn Lys Ile Ser Glu Glu Glu Tyr Val
    450                 455                 460

aag tca att aag gag gaa att cgc aaa gtt gtt gag ctt caa gaa gag      1440
Lys Ser Ile Lys Glu Glu Ile Arg Lys Val Val Glu Leu Gln Glu Glu
465                 470                 475                 480

ctt gat att gat gtt ctt gtt cat gga gaa cca gag aga aat gat atg      1488
Leu Asp Ile Asp Val Leu Val His Gly Glu Pro Glu Arg Asn Asp Met
                485                 490                 495

gtt gag tac ttc ggt gaa caa ttg tca ggc ttt gcc ttc acc gtt aat      1536
Val Glu Tyr Phe Gly Glu Gln Leu Ser Gly Phe Ala Phe Thr Val Asn
            500                 505                 510

ggg tgg gtg caa tcc tat ggt tcc cgt tgc gtg aag cca ccg atc atc      1584
Gly Trp Val Gln Ser Tyr Gly Ser Arg Cys Val Lys Pro Pro Ile Ile
        515                 520                 525

tat ggt gat gtg agc cgc cca aag cca atg acc gtc ttc tgg tca tct      1632
Tyr Gly Asp Val Ser Arg Pro Lys Pro Met Thr Val Phe Trp Ser Ser
    530                 535                 540

ctg gct cag agc ttt acc aag cgc cca atg aag gga atg ctt acc ggt      1680

Leu Ala Gln Ser Phe Thr Lys Arg Pro Met Lys Gly Met Leu Thr Gly
545                 550                 555                 560

cct gtt acc att ctc aac tgg tcc ttt gtt aga aat gac caa cct aga      1728
Pro Val Thr Ile Leu Asn Trp Ser Phe Val Arg Asn Asp Gln Pro Arg
                565                 570                 575

tct gag acc acc tac cag att gct ttg tct atc aag gac gaa gtg gaa      1776
Ser Glu Thr Thr Tyr Gln Ile Ala Leu Ser Ile Lys Asp Glu Val Glu
            580                 585                 590

gac ctt gaa aag gct ggc atc act gtt atc caa att gat gaa gct gct      1824
Asp Leu Glu Lys Ala Gly Ile Thr Val Ile Gln Ile Asp Glu Ala Ala
        595                 600                 605

ttg aga gag ggt ctt cca ctg agg aaa tca gag caa gct cac tac ttg      1872
Leu Arg Glu Gly Leu Pro Leu Arg Lys Ser Glu Gln Ala His Tyr Leu
    610                 615                 620

gac tgg gct gtc cat gcc ttc aga atc acc aat gtt ggt gtc cag gat      1920
Asp Trp Ala Val His Ala Phe Arg Ile Thr Asn Val Gly Val Gln Asp
625                 630                 635                 640

acc acc cag atc cac act cac atg tgc tac tcg aat ttc aac gac atc      1968
Thr Thr Gln Ile His Thr His Met Cys Tyr Ser Asn Phe Asn Asp Ile
                645                 650                 655

atc cac tcc atc atc gac atg gac gcc gat gtt atc acc att gag aac      2016
Ile His Ser Ile Ile Asp Met Asp Ala Asp Val Ile Thr Ile Glu Asn
            660                 665                 670

tct cgc tcc gac gag aag ctt ctg tca gtc ttc cgc gaa ggt gtg aag      2064
Ser Arg Ser Asp Glu Lys Leu Leu Ser Val Phe Arg Glu Gly Val Lys
        675                 680                 685

tat ggt gct gga att ggc cct ggt gtc tat gac atc cac tcc cca aga      2112
Tyr Gly Ala Gly Ile Gly Pro Gly Val Tyr Asp Ile His Ser Pro Arg
    690                 695                 700

ata cca cca act gaa gaa att gct gac aga atc aac aag atg ctg gca      2160
Ile Pro Pro Thr Glu Glu Ile Ala Asp Arg Ile Asn Lys Met Leu Ala
705                 710                 715                 720

gtg ctc gag aag aac atc ttg tgg gtg aac cct gac tgt ggg ctc aag      2208
Val Leu Glu Lys Asn Ile Leu Trp Val Asn Pro Asp Cys Gly Leu Lys
                725                 730                 735

acc cgt aag tac act gag gtg aag cca gcc ctc aca aac atg gtt gcc      2256
Thr Arg Lys Tyr Thr Glu Val Lys Pro Ala Leu Thr Asn Met Val Ala
            740                 745                 750

gca gca aaa ctc atc cgc aac gaa ctt gcc aag tga                      2292
Ala Ala Lys Leu Ile Arg Asn Glu Leu Ala Lys
        755                 760

<210>  174
<211>  763
<212>  PRT
<213>  Glycine max

<400>  174

Met Ala Ser His Ile Val Gly Tyr Pro Arg Met Gly Pro Lys Arg Glu
1               5                   10                  15

Leu Lys Phe Ala Leu Glu Ser Phe Trp Asp Gly Lys Ser Ser Ala Glu
            20                  25                  30

Asp Leu Gln Lys Val Ser Ser Asp Leu Arg Ala Ser Ile Trp Lys Gln
        35                  40                  45

Met Ala Asp Ala Gly Ile Lys Tyr Ile Pro Ser Asn Thr Phe Ser His
    50                  55                  60

Tyr Asp Gln Val Leu Asp Ala Thr Ala Thr Leu Gly Ala Val Pro Pro
65                  70                  75                  80

Arg Tyr Gly Trp Thr Gly Gly Glu Ile Gly Phe Asp Thr Tyr Phe Ser
                85                  90                  95

Met Ala Arg Gly Asn Ala Thr Val Pro Ala Met Glu Met Thr Lys Trp
            100                 105                 110

Phe Asp Thr Asn Tyr His Phe Ile Val Pro Glu Leu Gly Pro Asp Val
        115                 120                 125

Asn Phe Thr Tyr Ala Ser His Lys Ala Val Asp Glu Tyr Lys Glu Ala
    130                 135                 140

Lys Ala Leu Gly Val Asp Thr Val Pro Val Leu Val Gly Pro Val Thr
145                 150                 155                 160

Tyr Leu Leu Leu Ser Lys Pro Ala Lys Gly Val Glu Lys Ser Phe Ser
                165                 170                 175

Leu Leu Ser Leu Leu Pro Lys Val Leu Ala Val Tyr Lys Glu Val Ile
            180                 185                 190

Ala Asp Leu Lys Ala Ala Gly Ala Ser Trp Ile Gln Phe Asp Glu Pro
        195                 200                 205

Thr Leu Val Leu Asp Leu Glu Ser His Lys Leu Gln Ala Phe Thr Asp
    210                 215                 220

Ala Tyr Ala Glu Leu Ala Pro Ala Leu Ser Gly Leu Asn Val Leu Val
225                 230                 235                 240

Glu Thr Tyr Phe Ala Asp Ile Pro Ala Glu Ala Tyr Lys Thr Leu Thr
                245                 250                 255

Ser Leu Asn Gly Val Thr Ala Tyr Gly Phe Asp Leu Val Arg Gly Thr
            260                 265                 270

Asn Thr Leu Asp Leu Ile Lys Gly Gly Phe Pro Ser Gly Lys Tyr Leu
        275                 280                 285

Phe Ala Gly Val Val Asp Gly Arg Asn Ile Trp Ala Asn Asp Leu Ala
    290                 295                 300

Ala Ser Leu Thr Thr Leu Gln Gly Leu Glu Gly Ile Val Gly Lys Asp
305                 310                 315                 320

Lys Leu Val Val Ser Thr Ser Ser Ser Leu Leu His Thr Ala Val Asp
                325                 330                 335

Leu Val Asn Glu Thr Lys Leu Asp Asp Glu Ile Lys Ser Trp Leu Ala
            340                 345                 350

Phe Ala Ala Gln Lys Ile Val Glu Val Asn Ala Leu Ala Lys Ala Leu
        355                 360                 365

Ser Gly His Lys Asp Glu Ala Phe Phe Ser Gly Asn Ala Ala Ala Leu
    370                 375                 380

Ala Ser Arg Lys Ser Ser Pro Arg Val Thr Asn Glu Ala Val Gln Lys
385                 390                 395                 400

Ala Ala Ala Ala Leu Lys Gly Ser Asp His Arg Arg Ala Thr Asn Val
                405                 410                 415

Ser Ala Arg Leu Asp Ser Gln Gln Lys Lys Leu Asn Leu Pro Ile Leu
            420                 425                 430

Pro Thr Thr Thr Ile Gly Ser Phe Pro Gln Thr Val Glu Leu Arg Arg
        435                 440                 445

Val Arg Arg Glu Phe Lys Ala Asn Lys Ile Ser Glu Glu Glu Tyr Val
    450                 455                 460

Lys Ser Ile Lys Glu Glu Ile Arg Lys Val Val Glu Leu Gln Glu Glu
465                 470                 475                 480

Leu Asp Ile Asp Val Leu Val His Gly Glu Pro Glu Arg Asn Asp Met
                485                 490                 495

Val Glu Tyr Phe Gly Glu Gln Leu Ser Gly Phe Ala Phe Thr Val Asn
            500                 505                 510

Gly Trp Val Gln Ser Tyr Gly Ser Arg Cys Val Lys Pro Pro Ile Ile
        515                 520                 525

Tyr Gly Asp Val Ser Arg Pro Lys Pro Met Thr Val Phe Trp Ser Ser
    530                 535                 540

Leu Ala Gln Ser Phe Thr Lys Arg Pro Met Lys Gly Met Leu Thr Gly
545                 550                 555                 560

Pro Val Thr Ile Leu Asn Trp Ser Phe Val Arg Asn Asp Gln Pro Arg
                565                 570                 575

Ser Glu Thr Thr Tyr Gln Ile Ala Leu Ser Ile Lys Asp Glu Val Glu
            580                 585                 590

Asp Leu Glu Lys Ala Gly Ile Thr Val Ile Gln Ile Asp Glu Ala Ala
        595                 600                 605

Leu Arg Glu Gly Leu Pro Leu Arg Lys Ser Glu Gln Ala His Tyr Leu
    610                 615                 620

Asp Trp Ala Val His Ala Phe Arg Ile Thr Asn Val Gly Val Gln Asp
625                 630                 635                 640

Thr Thr Gln Ile His Thr His Met Cys Tyr Ser Asn Phe Asn Asp Ile
                645                 650                 655

Ile His Ser Ile Ile Asp Met Asp Ala Asp Val Ile Thr Ile Glu Asn
            660                 665                 670

Ser Arg Ser Asp Glu Lys Leu Leu Ser Val Phe Arg Glu Gly Val Lys
        675                 680                 685

Tyr Gly Ala Gly Ile Gly Pro Gly Val Tyr Asp Ile His Ser Pro Arg
    690                 695                 700

Ile Pro Pro Thr Glu Glu Ile Ala Asp Arg Ile Asn Lys Met Leu Ala
705                 710                 715                 720

Val Leu Glu Lys Asn Ile Leu Trp Val Asn Pro Asp Cys Gly Leu Lys
                725                 730                 735

Thr Arg Lys Tyr Thr Glu Val Lys Pro Ala Leu Thr Asn Met Val Ala
            740                 745                 750

Ala Ala Lys Leu Ile Arg Asn Glu Leu Ala Lys

755                 760

<210>  175
<211>  21
<212>  PRT
<213>  Glycine max

<400>  175

Ala Gly Ile Thr Val Ile Gln Ile Asp Glu Ala Ala Leu Arg Glu Gly
1               5                   10                  15

Leu Pro Leu Arg Lys
            20

<210>  176
<211>  24
<212>  PRT
<213>  Glycine max

<400>  176

Ala Leu Gly Val Asp Thr Val Pro Val Leu Val Gly Pro Val Thr Tyr
1               5                   10                  15

Leu Leu Leu Ser Lys Pro Ala Lys
            20

<210>  177
<211>  16
<212>  PRT
<213>  Glycine max

<400>  177

Asp Glu Ala Phe Phe Ser Gly Asn Ala Ala Ala Leu Ala Ser Arg Lys
1               5                   10                  15

<210>  178
<211>  8
<212>  PRT
<213>  Glycine max

<400>  178

Asp Glu Val Glu Asp Leu Glu Lys
1               5

<210>  179
<211>  8
<212>  PRT
<213>  Glycine max

<400>  179

Ile Ser Glu Glu Glu Tyr Val Lys

1               5


<210>  180
<211>  9
<212>  PRT
<213>  Glycine max

<400>  180

Ile Val Glu Val Asn Ala Leu Ala Lys
1               5


<210>  181
<211>  8
<212>  PRT
<213>  Glycine max

<400>  181

Leu Ile Arg Asn Glu Leu Ala Lys
1               5


<210>  182
<211>  10
<212>  PRT
<213>  Glycine max

<400>  182

Leu Leu Ser Val Phe Arg Glu Gly Val Lys
1               5                   10


<210>  183
<211>  21
<212>  PRT
<213>  Glycine max

<400>  183

Leu Val Val Ser Thr Ser Ser Ser Leu Leu His Thr Ala Val Asp Leu
1               5                   10                  15


Val Asn Glu Thr Lys
            20


<210>  184
<211>  10
<212>  PRT
<213>  Glycine max

<400>  184

Ser Phe Ser Leu Leu Ser Leu Leu Pro Lys
1               5                   10


<210>  185

<211>  12
<212>  PRT
<213>  Glycine max

<400>  185

Ser Ser Pro Arg Val Thr Asn Glu Ala Val Gln Lys
1               5                   10


<210>  186
<211>  26
<212>  PRT
<213>  Glycine max

<400>  186

Thr Leu Thr Ser Leu Asn Gly Val Thr Ala Tyr Gly Phe Asp Leu Val
1               5                   10                  15


Arg Gly Thr Asn Thr Leu Asp Leu Ile Lys
            20                  25


<210>  187
<211>  29
<212>  PRT
<213>  Glycine max

<400>  187

Tyr Gly Ala Gly Ile Gly Pro Gly Val Tyr Asp Ile His Ser Pro Arg
1               5                   10                  15


Ile Pro Pro Thr Glu Glu Ile Ala Asp Arg Ile Asn Lys
            20                  25


<210>  188
<211>  2196
<212>  DNA
<213>  Glycine max


<220>
<221>  CDS
<222>  (1)..(2196)

<400>  188
atg gct tct tcc act tcc tct atc tct ctc tcc caa gcc ctc ctc cgc      48
Met Ala Ser Ser Thr Ser Ser Ile Ser Leu Ser Gln Ala Leu Leu Arg
1               5                   10                  15

ccc acc acc acc tct ctc ccc tcc tcc tcc tcc tcc ctc cgc ctc acc      96
Pro Thr Thr Thr Ser Leu Pro Ser Ser Ser Ser Ser Leu Arg Leu Thr
            20                  25                  30

ccc act ctc cgc cct acc cca ccc gcc ctc tcc ctc cgc cgc cgc ctc      144
Pro Thr Leu Arg Pro Thr Pro Pro Ala Leu Ser Leu Arg Arg Arg Leu
        35                  40                  45

acc acc tcc atc cgc gcc gcc gcc tcc gtc aaa acc gtc gag aag gca     192
Thr Thr Ser Ile Arg Ala Ala Ala Ser Val Lys Thr Val Glu Lys Ala
    50                  55                  60

acc gcg gac gcc gcg ctg gtc gaa aaa tcc gtc aac acg atc cgc ttc     240
Thr Ala Asp Ala Ala Leu Val Glu Lys Ser Val Asn Thr Ile Arg Phe
65                  70                  75                  80

ctc gcc atc gac gcc gtc gag aag gcg aat tcg ggt cac ccg ggg ctc     288
Leu Ala Ile Asp Ala Val Glu Lys Ala Asn Ser Gly His Pro Gly Leu
                85                  90                  95

ccc atg gga tgc gct ccg atg ggt cac gtg cta tat gat gaa acc atg     336
Pro Met Gly Cys Ala Pro Met Gly His Val Leu Tyr Asp Glu Thr Met
            100                 105                 110

aag tat aac cct aag aac cct ttc tgg ttc aac cgc gat cgc ttc gtt     384
Lys Tyr Asn Pro Lys Asn Pro Phe Trp Phe Asn Arg Asp Arg Phe Val
        115                 120                 125

ctc tcc gct ggg cac ggg tgc atg ctt cag tac gcg ctt ctg cac ctc     432
Leu Ser Ala Gly His Gly Cys Met Leu Gln Tyr Ala Leu Leu His Leu
    130                 135                 140

gct ggg ttc gat agc gtc aag gaa gag gat ttg agg gaa ttt cgc caa     480
Ala Gly Phe Asp Ser Val Lys Glu Glu Asp Leu Arg Glu Phe Arg Gln
145                 150                 155                 160

tgg ggg agc aga act ccg gga cac cct gag aat ttt gag act ccg gga     528
Trp Gly Ser Arg Thr Pro Gly His Pro Glu Asn Phe Glu Thr Pro Gly
                165                 170                 175

att gaa gtt aca aca ggt cct ctt ggt cag ggg att gcc aat gct gtt     576
Ile Glu Val Thr Thr Gly Pro Leu Gly Gln Gly Ile Ala Asn Ala Val
            180                 185                 190

ggt ttg gcc ctt gca gag aag cac ttg gct gca aga tac aac aag cct     624
Gly Leu Ala Leu Ala Glu Lys His Leu Ala Ala Arg Tyr Asn Lys Pro
        195                 200                 205

gac aat gag att gtt gac cat tac acg tat gct att ctg ggt gat ggt     672
Asp Asn Glu Ile Val Asp His Tyr Thr Tyr Ala Ile Leu Gly Asp Gly
    210                 215                 220

tgt caa atg gag gga att gca aat gaa gca tgc tca ctt gct ggc cac     720
Cys Gln Met Glu Gly Ile Ala Asn Glu Ala Cys Ser Leu Ala Gly His
225                 230                 235                 240

tgg gga ctg ggg aag ctg ata gca ttt tat gat gac aat cat att tct     768
Trp Gly Leu Gly Lys Leu Ile Ala Phe Tyr Asp Asp Asn His Ile Ser
                245                 250                 255

att gat ggt aac aca gag att gcg ttc acc gag agt gtt gat agt cgt     816
Ile Asp Gly Asn Thr Glu Ile Ala Phe Thr Glu Ser Val Asp Ser Arg
            260                 265                 270

ttt gag gga ctt ggg tgg cat gtt att tgg gta aag aat gga aat aat     864
Phe Glu Gly Leu Gly Trp His Val Ile Trp Val Lys Asn Gly Asn Asn
        275                 280                 285

ggc tat gat gat att cgt gct gcc att aag gaa gca aaa gct gtc aaa     912
Gly Tyr Asp Asp Ile Arg Ala Ala Ile Lys Glu Ala Lys Ala Val Lys
    290                 295                 300

gac aaa ccc aca tta atc aag gtc aca aca acc att ggt tat ggt tct 960
Asp Lys Pro Thr Leu Ile Lys Val Thr Thr Thr Ile Gly Tyr Gly Ser
305                 310                 315                 320

cct aac aag gct aac tcc tac agt gtg cat gga agt gca ctg ggt gcc 1008
Pro Asn Lys Ala Asn Ser Tyr Ser Val His Gly Ser Ala Leu Gly Ala
                325                 330                 335

aaa gaa gtt gat gcc acc agg cag aac ctt gga tgg tca cat gag cca 1056
Lys Glu Val Asp Ala Thr Arg Gln Asn Leu Gly Trp Ser His Glu Pro
            340                 345                 350

ttc cac gtg cct gag gat gtc aaa aag cat tgg agt cgc cac acc cct 1104
Phe His Val Pro Glu Asp Val Lys Lys His Trp Ser Arg His Thr Pro
        355                 360                 365

gag ggt gct gca ctt gaa gct gag tgg aat gct aag ttt gct gag tat 1152
Glu Gly Ala Ala Leu Glu Ala Glu Trp Asn Ala Lys Phe Ala Glu Tyr
    370                 375                 380

gaa aag aaa tac aag gag gag gct gca gaa ttg aaa tct att atc aat 1200
Glu Lys Lys Tyr Lys Glu Glu Ala Ala Glu Leu Lys Ser Ile Ile Asn
385                 390                 395                 400

ggt gaa ttc cct gct ggt tgg gag aaa gca ctt ccg aca tac act cca 1248
Gly Glu Phe Pro Ala Gly Trp Glu Lys Ala Leu Pro Thr Tyr Thr Pro
                405                 410                 415

gag agc cca gcg gat gcc acc aga aac ctg tct caa aca aac ctt aat 1296
Glu Ser Pro Ala Asp Ala Thr Arg Asn Leu Ser Gln Thr Asn Leu Asn
            420                 425                 430

gcc ctt gca aag gtt ctt ccc ggt ctg ctt ggt ggc agt gca gat ctt 1344
Ala Leu Ala Lys Val Leu Pro Gly Leu Leu Gly Gly Ser Ala Asp Leu
        435                 440                 445

gct tct tcc aac atg acc ttg ctc aaa atg ttc ggg gac ttc caa aag 1392
Ala Ser Ser Asn Met Thr Leu Leu Lys Met Phe Gly Asp Phe Gln Lys
    450                 455                 460

gat act cca gca gag cgt aat gtt aga ttc ggt gtt aga gaa cac gga 1440
Asp Thr Pro Ala Glu Arg Asn Val Arg Phe Gly Val Arg Glu His Gly
465                 470                 475                 480

atg gga gct atc tgc aac ggc att gct ctt cac agc cct gga ctg att 1488
Met Gly Ala Ile Cys Asn Gly Ile Ala Leu His Ser Pro Gly Leu Ile
                485                 490                 495

cca tat tgt gca acc ttc ttt gta ttc act gac tac atg aga ggt gcc 1536
Pro Tyr Cys Ala Thr Phe Phe Val Phe Thr Asp Tyr Met Arg Gly Ala
            500                 505                 510

ata agg ctt tct gcg ctg tct gag gct ggg gtt att tat gtc atg acc 1584
Ile Arg Leu Ser Ala Leu Ser Glu Ala Gly Val Ile Tyr Val Met Thr
        515                 520                 525

cat gat tca ata gga ctt gga gaa gat ggg cca acc cac cag cca att 1632
His Asp Ser Ile Gly Leu Gly Glu Asp Gly Pro Thr His Gln Pro Ile
    530                 535                 540

gag cac cta gca agc ttc cgg gca atg cca aac att ttg atg ctt cgt 1680
Glu His Leu Ala Ser Phe Arg Ala Met Pro Asn Ile Leu Met Leu Arg

545                 550                 555                 560

ccc gcc gac ggt aac gaa aca gcc gga gca tac aaa gtg gcc gtg ctc      1728
Pro Ala Asp Gly Asn Glu Thr Ala Gly Ala Tyr Lys Val Ala Val Leu
                565                 570                 575

aac agg aag aga ccc tcc att ctt gcc cta tcc agg caa aaa ctg ccc      1776
Asn Arg Lys Arg Pro Ser Ile Leu Ala Leu Ser Arg Gln Lys Leu Pro
            580                 585                 590

cag ctt ccc gga act tcc att gaa gga gtt gaa aag ggt ggt tac acc      1824
Gln Leu Pro Gly Thr Ser Ile Glu Gly Val Glu Lys Gly Gly Tyr Thr
        595                 600                 605

att tcg gac aac tcc act ggc aac aag cct gat gtc att ttg atc gga      1872
Ile Ser Asp Asn Ser Thr Gly Asn Lys Pro Asp Val Ile Leu Ile Gly
    610                 615                 620

act ggt tcg gaa ttg gaa atc gct gcc aaa gct gct gat gac cta agg      1920
Thr Gly Ser Glu Leu Glu Ile Ala Ala Lys Ala Ala Asp Asp Leu Arg
625                 630                 635                 640

aag gaa ggg aag gct gtt aga gtt gtt tcc ctt gtt tct tgg gaa ctt      1968
Lys Glu Gly Lys Ala Val Arg Val Val Ser Leu Val Ser Trp Glu Leu
                645                 650                 655

ttt gat gag caa tca gaa gcc tac aag gag agt gtt ttc cct gct gct      2016
Phe Asp Glu Gln Ser Glu Ala Tyr Lys Glu Ser Val Phe Pro Ala Ala
            660                 665                 670

gtt tca gcc aga gtt agc att gag gca gga tca aca ttt ggg tgg gag      2064
Val Ser Ala Arg Val Ser Ile Glu Ala Gly Ser Thr Phe Gly Trp Glu
        675                 680                 685

aaa att gtt gga gca aag gga aaa gca ata ggc att gat cgt ttt gga      2112
Lys Ile Val Gly Ala Lys Gly Lys Ala Ile Gly Ile Asp Arg Phe Gly
    690                 695                 700

gct agt gct cca gct gga aga ata tac aaa gaa ttt ggt atc act aag      2160
Ala Ser Ala Pro Ala Gly Arg Ile Tyr Lys Glu Phe Gly Ile Thr Lys
705                 710                 715                 720

gaa gct gtt gtt gct gca gct aaa gag ctt atc tag                      2196
Glu Ala Val Val Ala Ala Ala Lys Glu Leu Ile
                725                 730


<210>  189
<211>  731
<212>  PRT
<213>  Glycine max

<400>  189

Met Ala Ser Ser Thr Ser Ser Ile Ser Leu Ser Gln Ala Leu Leu Arg
1               5                   10                  15


Pro Thr Thr Thr Ser Leu Pro Ser Ser Ser Ser Leu Arg Leu Thr
            20                  25                  30


Pro Thr Leu Arg Pro Thr Pro Pro Ala Leu Ser Leu Arg Arg Arg Leu

35                  40                  45

Thr Thr Ser Ile Arg Ala Ala Ala Ser Val Lys Thr Val Glu Lys Ala
    50                  55                  60

Thr Ala Asp Ala Ala Leu Val Glu Lys Ser Val Asn Thr Ile Arg Phe
65                  70                  75                  80

Leu Ala Ile Asp Ala Val Glu Lys Ala Asn Ser Gly His Pro Gly Leu
                85                  90                  95

Pro Met Gly Cys Ala Pro Met Gly His Val Leu Tyr Asp Glu Thr Met
            100                 105                 110

Lys Tyr Asn Pro Lys Asn Pro Phe Trp Phe Asn Arg Asp Arg Phe Val
        115                 120                 125

Leu Ser Ala Gly His Gly Cys Met Leu Gln Tyr Ala Leu Leu His Leu
    130                 135                 140

Ala Gly Phe Asp Ser Val Lys Glu Glu Asp Leu Arg Glu Phe Arg Gln
145                 150                 155                 160

Trp Gly Ser Arg Thr Pro Gly His Pro Glu Asn Phe Glu Thr Pro Gly
                165                 170                 175

Ile Glu Val Thr Thr Gly Pro Leu Gly Gln Gly Ile Ala Asn Ala Val
            180                 185                 190

Gly Leu Ala Leu Ala Glu Lys His Leu Ala Ala Arg Tyr Asn Lys Pro
        195                 200                 205

Asp Asn Glu Ile Val Asp His Tyr Thr Tyr Ala Ile Leu Gly Asp Gly
    210                 215                 220

Cys Gln Met Glu Gly Ile Ala Asn Glu Ala Cys Ser Leu Ala Gly His
225                 230                 235                 240

Trp Gly Leu Gly Lys Leu Ile Ala Phe Tyr Asp Asp Asn His Ile Ser
                245                 250                 255

Ile Asp Gly Asn Thr Glu Ile Ala Phe Thr Glu Ser Val Asp Ser Arg
            260                 265                 270

Phe Glu Gly Leu Gly Trp His Val Ile Trp Val Lys Asn Gly Asn Asn
        275                 280                 285

Gly Tyr Asp Asp Ile Arg Ala Ala Ile Lys Glu Ala Lys Ala Val Lys
    290                 295                 300

Asp Lys Pro Thr Leu Ile Lys Val Thr Thr Thr Ile Gly Tyr Gly Ser
305                 310                 315                 320

Pro Asn Lys Ala Asn Ser Tyr Ser Val His Gly Ser Ala Leu Gly Ala
                325                 330                 335

Lys Glu Val Asp Ala Thr Arg Gln Asn Leu Gly Trp Ser His Glu Pro
            340                 345                 350

Phe His Val Pro Glu Asp Val Lys Lys His Trp Ser Arg His Thr Pro
        355                 360                 365

Glu Gly Ala Ala Leu Glu Ala Glu Trp Asn Ala Lys Phe Ala Glu Tyr
    370                 375                 380

Glu Lys Lys Tyr Lys Glu Glu Ala Ala Glu Leu Lys Ser Ile Ile Asn
385                 390                 395                 400

Gly Glu Phe Pro Ala Gly Trp Glu Lys Ala Leu Pro Thr Tyr Thr Pro
                405                 410                 415

Glu Ser Pro Ala Asp Ala Thr Arg Asn Leu Ser Gln Thr Asn Leu Asn
            420                 425                 430

Ala Leu Ala Lys Val Leu Pro Gly Leu Leu Gly Gly Ser Ala Asp Leu
        435                 440                 445

Ala Ser Ser Asn Met Thr Leu Leu Lys Met Phe Gly Asp Phe Gln Lys
    450                 455                 460

Asp Thr Pro Ala Glu Arg Asn Val Arg Phe Gly Val Arg Glu His Gly
465                 470                 475                 480

Met Gly Ala Ile Cys Asn Gly Ile Ala Leu His Ser Pro Gly Leu Ile
                485                 490                 495

Pro Tyr Cys Ala Thr Phe Phe Val Phe Thr Asp Tyr Met Arg Gly Ala
            500                 505                 510

Ile Arg Leu Ser Ala Leu Ser Glu Ala Gly Val Ile Tyr Val Met Thr
        515                 520                 525

His Asp Ser Ile Gly Leu Gly Glu Asp Gly Pro Thr His Gln Pro Ile
    530                 535                 540

Glu His Leu Ala Ser Phe Arg Ala Met Pro Asn Ile Leu Met Leu Arg
545                 550                 555                 560

Pro Ala Asp Gly Asn Glu Thr Ala Gly Ala Tyr Lys Val Ala Val Leu
                565                 570                 575

Asn Arg Lys Arg Pro Ser Ile Leu Ala Leu Ser Arg Gln Lys Leu Pro
            580                 585                 590

Gln Leu Pro Gly Thr Ser Ile Glu Gly Val Glu Lys Gly Gly Tyr Thr
        595                 600                 605

Ile Ser Asp Asn Ser Thr Gly Asn Lys Pro Asp Val Ile Leu Ile Gly
    610                 615                 620

Thr Gly Ser Glu Leu Glu Ile Ala Ala Lys Ala Ala Asp Asp Leu Arg
625                 630                 635                 640

Lys Glu Gly Lys Ala Val Arg Val Val Ser Leu Val Ser Trp Glu Leu
                645                 650                 655

Phe Asp Glu Gln Ser Glu Ala Tyr Lys Glu Ser Val Phe Pro Ala Ala
            660                 665                 670

Val Ser Ala Arg Val Ser Ile Glu Ala Gly Ser Thr Phe Gly Trp Glu
        675                 680                 685

Lys Ile Val Gly Ala Lys Gly Lys Ala Ile Gly Ile Asp Arg Phe Gly
    690                 695                 700

Ala Ser Ala Pro Ala Gly Arg Ile Tyr Lys Glu Phe Gly Ile Thr Lys
705                 710                 715                 720

Glu Ala Val Val Ala Ala Ala Lys Glu Leu Ile
                725                 730

<210>  190
<211>  18
<212>  PRT
<213>  Glycine max

<400>  190

Ala Ile Gly Ile Asp Arg Phe Gly Ala Ser Ala Pro Ala Gly Arg Ile
1               5                   10                  15

Tyr Lys

<210>  191
<211>  27
<212>  PRT
<213>  Glycine max

<400>  191

Ala Leu Pro Thr Tyr Thr Pro Glu Ser Pro Ala Asp Ala Thr Arg Asn
1               5                   10                  15

Leu Ser Gln Thr Asn Leu Asn Ala Leu Ala Lys
            20                  25

<210>  192
<211>  14
<212>  PRT
<213>  Glycine max

<400>  192

Ala Asn Ser Tyr Ser Val His Gly Ser Ala Leu Gly Ala Lys
1               5                   10

<210>  193
<211>  10
<212>  PRT
<213>  Glycine max

<400>  193

Ala Thr Ala Asp Ala Ala Leu Val Glu Lys
1               5                   10

<210>  194
<211>  7
<212>  PRT
<213>  Glycine max

<400>  194

Asp Lys Pro Thr Leu Ile Lys
1               5

<210>  195
<211>  30
<212>  PRT
<213>  Glycine max

<400>  195

Gly Gly Tyr Thr Ile Ser Asp Asn Ser Thr Gly Asn Lys Pro Asp Val
1               5                   10                  15

Ile Leu Ile Gly Thr Gly Ser Glu Leu Glu Ile Ala Ala Lys
            20                  25                  30

<210>  196
<211>  14
<212>  PRT
<213>  Glycine max

<400>  196

Leu Pro Gln Leu Pro Gly Thr Ser Ile Glu Gly Val Glu Lys
1               5                   10

<210>  197
<211>  14
<212>  PRT
<213>  Glycine max

<400>  197

Asn Gly Asn Asn Gly Tyr Asp Asp Ile Arg Ala Ala Ile Lys
1               5                   10

<210>  198
<211>  15
<212>  PRT
<213>  Glycine max

<400>  198

Ser Val Asn Thr Ile Arg Phe Leu Ala Ile Asp Ala Val Glu Lys
1               5                   10                  15

<210>  199
<211>  12
<212>  PRT
<213>  Glycine max

<400>  199

Val Thr Thr Thr Ile Gly Tyr Gly Ser Pro Asn Lys
1               5                   10

<210>  200
<211>  1797
<212>  DNA
<213>  Glycine max

<220>
<221>  CDS
<222>  (1)..(1797)

<400>  200
atg gaa gtt cct tcg gag gta cca tct act gac act ggc acc agc acc        48
Met Glu Val Pro Ser Glu Val Pro Ser Thr Asp Thr Gly Thr Ser Thr
1               5                   10                  15

ggc acc ggc gaa acc atg agc aag aat gcg ttg aag cgg gag ctg aag        96
Gly Thr Gly Glu Thr Met Ser Lys Asn Ala Leu Lys Arg Glu Leu Lys
            20                  25                  30

aac aaa cag aga gaa gaa gaa agg aag cgc aag gat gag gag aag gcc 144
Asn Lys Gln Arg Glu Glu Glu Arg Lys Arg Lys Asp Glu Glu Lys Ala
        35                  40                  45

aaa aag gca gct gaa gca caa cag gca aag gat aac aaa tct gca cca 192
Lys Lys Ala Ala Glu Ala Gln Gln Ala Lys Asp Asn Lys Ser Ala Pro
    50                  55                  60

gct gat gat gag gat atg gat cca act caa tac ctt gag aac agg cta 240
Ala Asp Asp Glu Asp Met Asp Pro Thr Gln Tyr Leu Glu Asn Arg Leu
65                  70                  75                  80

aag tat ctt gca gtt caa aag aca gaa ggg aag aac ccg tat cct cat 288
Lys Tyr Leu Ala Val Gln Lys Thr Glu Gly Lys Asn Pro Tyr Pro His
                85                  90                  95

aaa ttc ttt gtc act atg tct att gat caa tac atc aag gaa tat gga 336
Lys Phe Phe Val Thr Met Ser Ile Asp Gln Tyr Ile Lys Glu Tyr Gly
            100                 105                 110

ggt tta agc aac ggg caa cac att gag gat gtc tct gtg tct ttg gct 384
Gly Leu Ser Asn Gly Gln His Ile Glu Asp Val Ser Val Ser Leu Ala
        115                 120                 125

ggc cgg atc atg cac aaa cgc acc tct ggt tct aag ctt gtc ttt tat 432
Gly Arg Ile Met His Lys Arg Thr Ser Gly Ser Lys Leu Val Phe Tyr
    130                 135                 140

gac ctg cac ggt ggt ggt tgc aag gtc cag gtt atg gct gat gct agt 480
Asp Leu His Gly Gly Gly Cys Lys Val Gln Val Met Ala Asp Ala Ser
145                 150                 155                 160

aaa tca gac ttg gat gag gct gga ttt tcc aaa ttc cat tct aat gtg 528
Lys Ser Asp Leu Asp Glu Ala Gly Phe Ser Lys Phe His Ser Asn Val
                165                 170                 175

aag cgc ggt gac ata gtt ggt gtc act ggg ttc cca ggc aaa agt aag 576
Lys Arg Gly Asp Ile Val Gly Val Thr Gly Phe Pro Gly Lys Ser Lys
            180                 185                 190

aag ggc gaa ctt agt att ttt ccc aag aat ttt gtg tcg ctg tct cat 624
Lys Gly Glu Leu Ser Ile Phe Pro Lys Asn Phe Val Ser Leu Ser His
        195                 200                 205

tgt ttg cac atg atg cca agg caa aag tct gct gct gct gct att gat 672
Cys Leu His Met Met Pro Arg Gln Lys Ser Ala Ala Ala Ala Ile Asp
    210                 215                 220

aat aga aat cca tgg ata ccg gga agt act agg aat cct gaa aca tat 720
Asn Arg Asn Pro Trp Ile Pro Gly Ser Thr Arg Asn Pro Glu Thr Tyr
225                 230                 235                 240

att ttg aag gat cag gaa act aga tat cgg tta cgc cat ttg gat ttg 768
Ile Leu Lys Asp Gln Glu Thr Arg Tyr Arg Leu Arg His Leu Asp Leu
                245                 250                 255

atg ctt aat cca gag gtt cga gac ata ttc aag acc agg tct aaa atc 816
Met Leu Asn Pro Glu Val Arg Asp Ile Phe Lys Thr Arg Ser Lys Ile
            260                 265                 270

att tct tac att agg agg ttc ctt gat gac ctt gat ttc ttg gag gtt 864
Ile Ser Tyr Ile Arg Arg Phe Leu Asp Asp Leu Asp Phe Leu Glu Val

275                 280                 285

gaa aca cct atg atg aat atg att gct ggt gga gct gca gcc cgt cca        912
Glu Thr Pro Met Met Asn Met Ile Ala Gly Gly Ala Ala Ala Arg Pro
    290                 295                 300

ttt gta aca cat cac aat gat ctt aac atg agg tta ttc atg agg att        960
Phe Val Thr His His Asn Asp Leu Asn Met Arg Leu Phe Met Arg Ile
305                 310                 315                 320

gct cca gaa ctg tat ctt aag gag ttg gtt gtt ggt gga ctg gat cgt       1008
Ala Pro Glu Leu Tyr Leu Lys Glu Leu Val Val Gly Gly Leu Asp Arg
                325                 330                 335

gtt tat gaa att ggt aaa caa ttt agg aat gag ggc ata gat ttg act       1056
Val Tyr Glu Ile Gly Lys Gln Phe Arg Asn Glu Gly Ile Asp Leu Thr
            340                 345                 350

cat aat cct gag ttt act acc tgt gag ttc tat atg gct tat aag gac       1104
His Asn Pro Glu Phe Thr Thr Cys Glu Phe Tyr Met Ala Tyr Lys Asp
        355                 360                 365

tac aat gac tta atg gat cta aca gag caa atg ttg agt ggt atg gtt       1152
Tyr Asn Asp Leu Met Asp Leu Thr Glu Gln Met Leu Ser Gly Met Val
    370                 375                 380

aag gag ctt acg aaa ggc agc tat aaa atc aag tat cat gca gat ggg       1200
Lys Glu Leu Thr Lys Gly Ser Tyr Lys Ile Lys Tyr His Ala Asp Gly
385                 390                 395                 400

att gat aag gaa cct att gaa att gac ttt act aca cct ttt aga agg       1248
Ile Asp Lys Glu Pro Ile Glu Ile Asp Phe Thr Thr Pro Phe Arg Arg
                405                 410                 415

att gac atg att gaa gag tta gag aag atg gca ggt cta agt att ccc       1296
Ile Asp Met Ile Glu Glu Leu Glu Lys Met Ala Gly Leu Ser Ile Pro
            420                 425                 430

aaa gac ttg tca agt gag gaa gcc aat caa tat ttg aag gat gca tgc       1344
Lys Asp Leu Ser Ser Glu Glu Ala Asn Gln Tyr Leu Lys Asp Ala Cys
        435                 440                 445

gtg aag tat gag atc aaa tgt cct ccc cct gag aca act gct cgt ttg       1392
Val Lys Tyr Glu Ile Lys Cys Pro Pro Pro Glu Thr Thr Ala Arg Leu
    450                 455                 460

ttg gat aaa ctt gtg ggt cac ttt ttg gaa gaa acg tgt gta aat cct       1440
Leu Asp Lys Leu Val Gly His Phe Leu Glu Glu Thr Cys Val Asn Pro
465                 470                 475                 480

aca ttc atc aaa aac cat cct gag atc atg agt cct cta gca aag tgg       1488
Thr Phe Ile Lys Asn His Pro Glu Ile Met Ser Pro Leu Ala Lys Trp
                485                 490                 495

cac aga tca aaa cca ggc ctg act gaa cgt ttt gaa ttg ttt gtt aat       1536
His Arg Ser Lys Pro Gly Leu Thr Glu Arg Phe Glu Leu Phe Val Asn
            500                 505                 510

aaa cgt gaa ctt tgc aat gca tat act gaa ttg aat gac cct gta gta       1584
Lys Arg Glu Leu Cys Asn Ala Tyr Thr Glu Leu Asn Asp Pro Val Val
        515                 520                 525

caa cga cag aga ttt gct gaa caa ctc aag gat cgg caa tct ggc gat       1632

Gln Arg Gln Arg Phe Ala Glu Gln Leu Lys Asp Arg Gln Ser Gly Asp
    530                 535                 540

gat gaa gca atg gcc tta gat gaa aca ttt tgt acg gct cta gaa tat      1680
Asp Glu Ala Met Ala Leu Asp Glu Thr Phe Cys Thr Ala Leu Glu Tyr
545                 550                 555                 560

ggt tta cca cct act ggt ggt tgg ggt ttg ggc att gat cgg ttg acc      1728
Gly Leu Pro Pro Thr Gly Gly Trp Gly Leu Gly Ile Asp Arg Leu Thr
                565                 570                 575

atg tta ctg act gat tca cag aat att aag gag gtt ctt ctc ttc cct      1776
Met Leu Leu Thr Asp Ser Gln Asn Ile Lys Glu Val Leu Leu Phe Pro
            580                 585                 590

gcc atg aaa cct caa gac tga                                          1797
Ala Met Lys Pro Gln Asp
        595

<210>  201
<211>  598
<212>  PRT
<213>  Glycine max

<400>  201

Met Glu Val Pro Ser Glu Val Pro Ser Thr Asp Thr Gly Thr Ser Thr
1               5                   10                  15

Gly Thr Gly Glu Thr Met Ser Lys Asn Ala Leu Lys Arg Glu Leu Lys
            20                  25                  30

Asn Lys Gln Arg Glu Glu Glu Arg Lys Arg Lys Asp Glu Glu Lys Ala
        35                  40                  45

Lys Lys Ala Ala Glu Ala Gln Gln Ala Lys Asp Asn Lys Ser Ala Pro
    50                  55                  60

Ala Asp Asp Glu Asp Met Asp Pro Thr Gln Tyr Leu Glu Asn Arg Leu
65                  70                  75                  80

Lys Tyr Leu Ala Val Gln Lys Thr Glu Gly Lys Asn Pro Tyr Pro His
                85                  90                  95

Lys Phe Phe Val Thr Met Ser Ile Asp Gln Tyr Ile Lys Glu Tyr Gly
            100                 105                 110

Gly Leu Ser Asn Gly Gln His Ile Glu Asp Val Ser Val Ser Leu Ala
        115                 120                 125

Gly Arg Ile Met His Lys Arg Thr Ser Gly Ser Lys Leu Val Phe Tyr
    130                 135                 140

Asp Leu His Gly Gly Gly Cys Lys Val Gln Val Met Ala Asp Ala Ser
145                 150                 155                 160

Lys Ser Asp Leu Asp Glu Ala Gly Phe Ser Lys Phe His Ser Asn Val
                165                 170                 175

Lys Arg Gly Asp Ile Val Gly Val Thr Gly Phe Pro Gly Lys Ser Lys
            180                 185                 190

Lys Gly Glu Leu Ser Ile Phe Pro Lys Asn Phe Val Ser Leu Ser His
        195                 200                 205

Cys Leu His Met Met Pro Arg Gln Lys Ser Ala Ala Ala Ala Ile Asp
    210                 215                 220

Asn Arg Asn Pro Trp Ile Pro Gly Ser Thr Arg Asn Pro Glu Thr Tyr
225                 230                 235                 240

Ile Leu Lys Asp Gln Glu Thr Arg Tyr Arg Leu Arg His Leu Asp Leu
                245                 250                 255

Met Leu Asn Pro Glu Val Arg Asp Ile Phe Lys Thr Arg Ser Lys Ile
            260                 265                 270

Ile Ser Tyr Ile Arg Arg Phe Leu Asp Asp Leu Asp Phe Leu Glu Val
        275                 280                 285

Glu Thr Pro Met Met Asn Met Ile Ala Gly Gly Ala Ala Ala Arg Pro
    290                 295                 300

Phe Val Thr His His Asn Asp Leu Asn Met Arg Leu Phe Met Arg Ile
305                 310                 315                 320

Ala Pro Glu Leu Tyr Leu Lys Glu Leu Val Val Gly Gly Leu Asp Arg
                325                 330                 335

Val Tyr Glu Ile Gly Lys Gln Phe Arg Asn Glu Gly Ile Asp Leu Thr
            340                 345                 350

His Asn Pro Glu Phe Thr Thr Cys Glu Phe Tyr Met Ala Tyr Lys Asp
        355                 360                 365

Tyr Asn Asp Leu Met Asp Leu Thr Glu Gln Met Leu Ser Gly Met Val
    370                 375                 380

Lys Glu Leu Thr Lys Gly Ser Tyr Lys Ile Lys Tyr His Ala Asp Gly
385                 390                 395                 400

Ile Asp Lys Glu Pro Ile Glu Ile Asp Phe Thr Thr Pro Phe Arg Arg
                405                 410                 415

Ile Asp Met Ile Glu Glu Leu Glu Lys Met Ala Gly Leu Ser Ile Pro
            420                 425                 430

Lys Asp Leu Ser Ser Glu Glu Ala Asn Gln Tyr Leu Lys Asp Ala Cys
        435                 440                 445

Val Lys Tyr Glu Ile Lys Cys Pro Pro Pro Glu Thr Thr Ala Arg Leu
    450                 455                 460

Leu Asp Lys Leu Val Gly His Phe Leu Glu Glu Thr Cys Val Asn Pro
465                 470                 475                 480

Thr Phe Ile Lys Asn His Pro Glu Ile Met Ser Pro Leu Ala Lys Trp
                485                 490                 495

His Arg Ser Lys Pro Gly Leu Thr Glu Arg Phe Glu Leu Phe Val Asn
            500                 505                 510

Lys Arg Glu Leu Cys Asn Ala Tyr Thr Glu Leu Asn Asp Pro Val Val
        515                 520                 525

Gln Arg Gln Arg Phe Ala Glu Gln Leu Lys Asp Arg Gln Ser Gly Asp
    530                 535                 540

Asp Glu Ala Met Ala Leu Asp Glu Thr Phe Cys Thr Ala Leu Glu Tyr
545                 550                 555                 560

Gly Leu Pro Pro Thr Gly Gly Trp Gly Leu Gly Ile Asp Arg Leu Thr
                565                 570                 575

Met Leu Leu Thr Asp Ser Gln Asn Ile Lys Glu Val Leu Leu Phe Pro
            580                 585                 590

Ala Met Lys Pro Gln Asp
        595

<210>  202
<211>  12
<212>  PRT
<213>  Glycine max

<400>  202

Asp Leu Ser Ser Glu Glu Ala Asn Gln Tyr Leu Lys
1               5                   10

<210>  203
<211>  1728
<212>  DNA
<213>  Glycine max

<220>
<221>  CDS
<222>  (1)..(1728)

<400>  203
atg tac cgc ttt gca acc tcc ctc gcc tcc aaa gct aga att gcc agg      48
Met Tyr Arg Phe Ala Thr Ser Leu Ala Ser Lys Ala Arg Ile Ala Arg
1               5                   10                  15

agc agc act caa cag att gga agt agg gtc act tgg aac agg aat tat      96
Ser Ser Thr Gln Gln Ile Gly Ser Arg Val Thr Trp Asn Arg Asn Tyr
            20                  25                  30

gcg gcc aaa gac att aag ttt ggt gtg gag gct cgg gct ctg atg ctt      144
Ala Ala Lys Asp Ile Lys Phe Gly Val Glu Ala Arg Ala Leu Met Leu
        35                  40                  45

aag ggt gtc gaa gag ctc gct gat gcc gtt aaa gta aca atg ggc cct      192
Lys Gly Val Glu Glu Leu Ala Asp Ala Val Lys Val Thr Met Gly Pro
    50                  55                  60

aag ggg cgt aat gtg gtt att gag caa agt ttt gga gca cct aaa gtg      240
Lys Gly Arg Asn Val Val Ile Glu Gln Ser Phe Gly Ala Pro Lys Val
65                  70                  75                  80

acg aaa gat gga gta acc gtt gct aag agc att gaa ttc aag gac aaa      288
Thr Lys Asp Gly Val Thr Val Ala Lys Ser Ile Glu Phe Lys Asp Lys
                85                  90                  95

gtc aag aat atc ggt gcc agt ctt gta aag cag gtt gct aat gct act      336
Val Lys Asn Ile Gly Ala Ser Leu Val Lys Gln Val Ala Asn Ala Thr
            100                 105                 110

aat gac gtg gct ggt gat gga acc act tgt gct aca att ctt act aaa      384
Asn Asp Val Ala Gly Asp Gly Thr Thr Cys Ala Thr Ile Leu Thr Lys
        115                 120                 125

gca ata ttt acg gaa gga tgt aaa tca gtt gca gct gga atg aat gcg      432
Ala Ile Phe Thr Glu Gly Cys Lys Ser Val Ala Ala Gly Met Asn Ala
    130                 135                 140

atg gac ctg aga cga ggt ata aat atg gct gtt gat gct gtg gtg aca      480
Met Asp Leu Arg Arg Gly Ile Asn Met Ala Val Asp Ala Val Val Thr
145                 150                 155                 160

aat ctg aaa agc aga gca cgt atg att agc act tct gaa gaa att gcc      528
Asn Leu Lys Ser Arg Ala Arg Met Ile Ser Thr Ser Glu Glu Ile Ala
                165                 170                 175

cag gtt gga aca ata tct gcc aat ggg gag agg gaa att ggt gag tta      576
Gln Val Gly Thr Ile Ser Ala Asn Gly Glu Arg Glu Ile Gly Glu Leu
            180                 185                 190

att gca aaa gct atg gag aaa gtt ggc aaa gag ggt gtc atc aca atc      624
Ile Ala Lys Ala Met Glu Lys Val Gly Lys Glu Gly Val Ile Thr Ile
        195                 200                 205

tca gat ggc aag aca ttg tac aat gag ttg gaa gtc gtt gaa gga atg 672
Ser Asp Gly Lys Thr Leu Tyr Asn Glu Leu Glu Val Val Glu Gly Met
    210                 215                 220

aag ctc gac agg gga tac att tct cca tat ttc ata aca aac cag aag 720
Lys Leu Asp Arg Gly Tyr Ile Ser Pro Tyr Phe Ile Thr Asn Gln Lys
225                 230                 235                 240

aac cag aaa tgt gaa ctt gag gat cct ctc att ata atc cat gaa aag 768
Asn Gln Lys Cys Glu Leu Glu Asp Pro Leu Ile Ile Ile His Glu Lys
                245                 250                 255

aaa atc tca agc ata aat gct atc gtt aaa gta tta gag tta gct ttg 816
Lys Ile Ser Ser Ile Asn Ala Ile Val Lys Val Leu Glu Leu Ala Leu
            260                 265                 270

aag aga caa aga tct tta ttg att gtc gcc gag gat gtg gaa agt gat 864
Lys Arg Gln Arg Ser Leu Leu Ile Val Ala Glu Asp Val Glu Ser Asp
        275                 280                 285

gcc ctt gca act ctt att cta aat aaa ctt cgt gct gga atc aag gta 912
Ala Leu Ala Thr Leu Ile Leu Asn Lys Leu Arg Ala Gly Ile Lys Val
    290                 295                 300

tgt gcc atc aaa gct cct ggt ttt ggt gaa aac cgt aaa tct ggt ctc 960
Cys Ala Ile Lys Ala Pro Gly Phe Gly Glu Asn Arg Lys Ser Gly Leu
305                 310                 315                 320

cag gat ctt gct gtt ctt aca gga ggt caa ctt atc acc gaa gag ctt 1008
Gln Asp Leu Ala Val Leu Thr Gly Gly Gln Leu Ile Thr Glu Glu Leu
                325                 330                 335

ggt ttg aat ctt gaa aaa gta gac ctg gac tta ttt ggc tct tgc aag 1056
Gly Leu Asn Leu Glu Lys Val Asp Leu Asp Leu Phe Gly Ser Cys Lys
            340                 345                 350

aag ata aca att tct aaa gat gac act gtc atc ctt gat gga gct ggt 1104
Lys Ile Thr Ile Ser Lys Asp Asp Thr Val Ile Leu Asp Gly Ala Gly
        355                 360                 365

gat aag aaa gca att gag gaa aga tgt gag cag att aga tca gca att 1152
Asp Lys Lys Ala Ile Glu Glu Arg Cys Glu Gln Ile Arg Ser Ala Ile
    370                 375                 380

gaa aat agc act tca gat tat gac aag gaa aag tta cag gaa aga tta 1200
Glu Asn Ser Thr Ser Asp Tyr Asp Lys Glu Lys Leu Gln Glu Arg Leu
385                 390                 395                 400

gcc aag ctt tct ggg ggt gtt gca gta ctg aag att gga gga gcc agt 1248
Ala Lys Leu Ser Gly Gly Val Ala Val Leu Lys Ile Gly Gly Ala Ser
                405                 410                 415

gag gct gaa gtt ggt gag aag aag gat aga gtg aca gat gcc tta aat 1296
Glu Ala Glu Val Gly Glu Lys Lys Asp Arg Val Thr Asp Ala Leu Asn
            420                 425                 430

gca acc aag gca gct gta gag gag ggc ata gta cct ggt ggt ggt gtt 1344
Ala Thr Lys Ala Ala Val Glu Glu Gly Ile Val Pro Gly Gly Gly Val
        435                 440                 445

gct ctt ctt tat gca tcg agt gag ttg gat aag ctt caa act gcc aac 1392
Ala Leu Leu Tyr Ala Ser Ser Glu Leu Asp Lys Leu Gln Thr Ala Asn

450                 455                 460

ttt gat caa aag ata ggt gtc cag atc atc caa aat gct ttg aag act        1440
Phe Asp Gln Lys Ile Gly Val Gln Ile Ile Gln Asn Ala Leu Lys Thr
465                 470                 475                 480

cct gtg cac aca att gct tca aat gca gga gtt gag ggt gct gtt gtt        1488
Pro Val His Thr Ile Ala Ser Asn Ala Gly Val Glu Gly Ala Val Val
                485                 490                 495

gtt ggc aaa cta ttg gaa cag aat gat cct gat ctt gga tat gat gca        1536
Val Gly Lys Leu Leu Glu Gln Asn Asp Pro Asp Leu Gly Tyr Asp Ala
            500                 505                 510

gcc aaa ggt gaa tat gtt gat atg gtt aaa act gga att att gat cca        1584
Ala Lys Gly Glu Tyr Val Asp Met Val Lys Thr Gly Ile Ile Asp Pro
        515                 520                 525

ttg aag gtt att agg acc gcc ttg gtt gat gca gcc agt gtg tca tct        1632
Leu Lys Val Ile Arg Thr Ala Leu Val Asp Ala Ala Ser Val Ser Ser
    530                 535                 540

ttg atg aca aca act gaa gct gtt gtg tct gaa ctc ccg aag gac gat        1680
Leu Met Thr Thr Thr Glu Ala Val Val Ser Glu Leu Pro Lys Asp Asp
545                 550                 555                 560

aaa gat gtt cct gcc atg ggt ggt ggc atg ggt ggc atg gat tat taa        1728
Lys Asp Val Pro Ala Met Gly Gly Gly Met Gly Gly Met Asp Tyr
                565                 570                 575


<210>  204
<211>  575
<212>  PRT
<213>  Glycine max

<400>  204

Met Tyr Arg Phe Ala Thr Ser Leu Ala Ser Lys Ala Arg Ile Ala Arg
1               5                   10                  15


Ser Ser Thr Gln Gln Ile Gly Ser Arg Val Thr Trp Asn Arg Asn Tyr
            20                  25                  30


Ala Ala Lys Asp Ile Lys Phe Gly Val Glu Ala Arg Ala Leu Met Leu
        35                  40                  45


Lys Gly Val Glu Glu Leu Ala Asp Ala Val Lys Val Thr Met Gly Pro
    50                  55                  60


Lys Gly Arg Asn Val Val Ile Glu Gln Ser Phe Gly Ala Pro Lys Val
65                  70                  75                  80


Thr Lys Asp Gly Val Thr Val Ala Lys Ser Ile Glu Phe Lys Asp Lys
                85                  90                  95


Val Lys Asn Ile Gly Ala Ser Leu Val Lys Gln Val Ala Asn Ala Thr

100                 105                 110

Asn Asp Val Ala Gly Asp Gly Thr Thr Cys Ala Thr Ile Leu Thr Lys
        115                 120                 125

Ala Ile Phe Thr Glu Gly Cys Lys Ser Val Ala Ala Gly Met Asn Ala
    130                 135                 140

Met Asp Leu Arg Arg Gly Ile Asn Met Ala Val Asp Ala Val Val Thr
145                 150                 155                 160

Asn Leu Lys Ser Arg Ala Arg Met Ile Ser Thr Ser Glu Glu Ile Ala
                165                 170                 175

Gln Val Gly Thr Ile Ser Ala Asn Gly Glu Arg Glu Ile Gly Glu Leu
            180                 185                 190

Ile Ala Lys Ala Met Glu Lys Val Gly Lys Glu Gly Val Ile Thr Ile
        195                 200                 205

Ser Asp Gly Lys Thr Leu Tyr Asn Glu Leu Glu Val Val Glu Gly Met
    210                 215                 220

Lys Leu Asp Arg Gly Tyr Ile Ser Pro Tyr Phe Ile Thr Asn Gln Lys
225                 230                 235                 240

Asn Gln Lys Cys Glu Leu Glu Asp Pro Leu Ile Ile Ile His Glu Lys
                245                 250                 255

Lys Ile Ser Ser Ile Asn Ala Ile Val Lys Val Leu Glu Leu Ala Leu
            260                 265                 270

Lys Arg Gln Arg Ser Leu Leu Ile Val Ala Glu Asp Val Glu Ser Asp
        275                 280                 285

Ala Leu Ala Thr Leu Ile Leu Asn Lys Leu Arg Ala Gly Ile Lys Val
    290                 295                 300

Cys Ala Ile Lys Ala Pro Gly Phe Gly Glu Asn Arg Lys Ser Gly Leu
305                 310                 315                 320

Gln Asp Leu Ala Val Leu Thr Gly Gly Gln Leu Ile Thr Glu Glu Leu
                325                 330                 335

Gly Leu Asn Leu Glu Lys Val Asp Leu Asp Leu Phe Gly Ser Cys Lys
            340                 345                 350

Lys Ile Thr Ile Ser Lys Asp Asp Thr Val Ile Leu Asp Gly Ala Gly
        355                 360                 365

Asp Lys Lys Ala Ile Glu Glu Arg Cys Glu Gln Ile Arg Ser Ala Ile
    370                 375                 380

Glu Asn Ser Thr Ser Asp Tyr Asp Lys Glu Lys Leu Gln Glu Arg Leu
385                 390                 395                 400

Ala Lys Leu Ser Gly Gly Val Ala Val Leu Lys Ile Gly Gly Ala Ser
                405                 410                 415

Glu Ala Glu Val Gly Glu Lys Lys Asp Arg Val Thr Asp Ala Leu Asn
            420                 425                 430

Ala Thr Lys Ala Ala Val Glu Glu Gly Ile Val Pro Gly Gly Gly Val
        435                 440                 445

Ala Leu Leu Tyr Ala Ser Ser Glu Leu Asp Lys Leu Gln Thr Ala Asn
    450                 455                 460

Phe Asp Gln Lys Ile Gly Val Gln Ile Ile Gln Asn Ala Leu Lys Thr
465                 470                 475                 480

Pro Val His Thr Ile Ala Ser Asn Ala Gly Val Glu Gly Ala Val Val
                485                 490                 495

Val Gly Lys Leu Leu Glu Gln Asn Asp Pro Asp Leu Gly Tyr Asp Ala
            500                 505                 510

Ala Lys Gly Glu Tyr Val Asp Met Val Lys Thr Gly Ile Ile Asp Pro
        515                 520                 525

Leu Lys Val Ile Arg Thr Ala Leu Val Asp Ala Ala Ser Val Ser Ser
    530                 535                 540

Leu Met Thr Thr Thr Glu Ala Val Val Ser Glu Leu Pro Lys Asp Asp
545                 550                 555                 560

Lys Asp Val Pro Ala Met Gly Gly Gly Met Gly Gly Met Asp Tyr
                565                 570                 575

<210>  205
<211>  24
<212>  PRT
<213>  Glycine max

<400>  205

Ala Ala Val Glu Glu Gly Ile Val Pro Gly Gly Gly Val Ala Leu Leu
1               5                   10                  15

Tyr Ala Ser Ser Glu Leu Asp Lys
            20

<210>  206
<211>  11
<212>  PRT
<213>  Glycine max

<400>  206

Asp Arg Val Thr Asp Ala Leu Asn Ala Thr Lys
1               5                   10

<210>  207
<211>  10
<212>  PRT
<213>  Glycine max

<400>  207

Glu Gly Val Ile Thr Ile Ser Asp Gly Lys
1               5                   10

<210>  208
<211>  14
<212>  PRT
<213>  Glycine max

<400>  208

Gly Arg Asn Val Val Ile Glu Gln Ser Phe Gly Ala Pro Lys
1               5                   10

<210>  209
<211>  10
<212>  PRT
<213>  Glycine max

<400>  209

Gly Val Glu Glu Leu Ala Asp Ala Val Lys
1               5                   10

<210>  210
<211>  12
<212>  PRT
<213>  Glycine max

<400>  210

Ile Gly Gly Ala Ser Glu Ala Glu Val Gly Glu Lys
1               5                   10

<210>  211
<211>  11
<212>  PRT
<213>  Glycine max

<400>  211

Ile Gly Val Gln Ile Ile Gln Asn Ala Leu Lys
1               5                   10


<210>  212
<211>  9
<212>  PRT
<213>  Glycine max

<400>  212

Ile Ser Ser Ile Asn Ala Ile Val Lys
1               5


<210>  213
<211>  15
<212>  PRT
<213>  Glycine max

<400>  213

Leu Leu Glu Gln Asn Asp Pro Asp Leu Gly Tyr Asp Ala Ala Lys
1               5                   10                  15


<210>  214
<211>  9
<212>  PRT
<213>  Glycine max

<400>  214

Leu Gln Thr Ala Asn Phe Asp Gln Lys
1               5


<210>  215
<211>  9
<212>  PRT
<213>  Glycine max

<400>  215

Leu Ser Gly Gly Val Ala Val Leu Lys
1               5


<210>  216
<211>  8
<212>  PRT
<213>  Glycine max

<400>  216

Asn Ile Gly Ala Ser Leu Val Lys

1               5


<210>  217
<211>  8
<212>  PRT
<213>  Glycine max

<400>  217

Thr Gly Ile Ile Asp Pro Leu Lys
1               5


<210>  218
<211>  20
<212>  PRT
<213>  Glycine max

<400>  218

Thr Pro Val His Thr Ile Ala Ser Asn Ala Gly Val Glu Gly Ala Val
1               5                   10                  15


Val Val Gly Lys
            20


<210>  219
<211>  1725
<212>  DNA
<213>  Glycine max


<220>
<221>  CDS
<222>  (1)..(1725)

<400>  219
atg tat cgc ttt gct tct aac ctc gct tcc aaa gca agg att gct agg      48
Met Tyr Arg Phe Ala Ser Asn Leu Ala Ser Lys Ala Arg Ile Ala Arg
1               5                   10                  15

agt agc agc cac cag att gga agt agg ctg agt tcg agc aga aac tat      96
Ser Ser Ser His Gln Ile Gly Ser Arg Leu Ser Ser Ser Arg Asn Tyr
            20                  25                  30

gcg gct aaa gac att aga ttt ggt gtg gag gct cgg gca ctg atg ctt      144
Ala Ala Lys Asp Ile Arg Phe Gly Val Glu Ala Arg Ala Leu Met Leu
        35                  40                  45

aag ggt gtt gaa gag cta gct gat gct gtc aaa gta acc atg ggt ccc      192
Lys Gly Val Glu Glu Leu Ala Asp Ala Val Lys Val Thr Met Gly Pro
    50                  55                  60

aag ggg cgt aat gtt gtg att gag caa agt ttt ggt gcc cct aaa gta      240
Lys Gly Arg Asn Val Val Ile Glu Gln Ser Phe Gly Ala Pro Lys Val
65                  70                  75                  80

acc aaa gat gga gta act gtt gca aag agc att gaa ttc aag gat aaa      288
Thr Lys Asp Gly Val Thr Val Ala Lys Ser Ile Glu Phe Lys Asp Lys
                85                  90                  95

gtt aag aat gtt ggt gcc agt ctt gta aag cag gtt gca aat gct act 336
Val Lys Asn Val Gly Ala Ser Leu Val Lys Gln Val Ala Asn Ala Thr
            100                 105                 110

aat gat gtg gct ggt gat gga acc acg tgc gct aca gtc ctt aca cga 384
Asn Asp Val Ala Gly Asp Gly Thr Thr Cys Ala Thr Val Leu Thr Arg
        115                 120                 125

gca ata ttt act gaa ggc tgc aaa tca gtt gcg gct gga atg aat gca 432
Ala Ile Phe Thr Glu Gly Cys Lys Ser Val Ala Ala Gly Met Asn Ala
    130                 135                 140

atg gac ctg agg cgt ggt ata agt atg gct gtc gat gct gtg gtg aca 480
Met Asp Leu Arg Arg Gly Ile Ser Met Ala Val Asp Ala Val Val Thr
145                 150                 155                 160

aac ttg aaa agc aga gca agg atg att agc aca tct gaa gaa ata gca 528
Asn Leu Lys Ser Arg Ala Arg Met Ile Ser Thr Ser Glu Glu Ile Ala
                165                 170                 175

cag gta ggg aca ata tct gct aac gga gag aga gaa att ggt gag ttg 576
Gln Val Gly Thr Ile Ser Ala Asn Gly Glu Arg Glu Ile Gly Glu Leu
            180                 185                 190

att gca aaa gct atg gag aaa gtt ggc aaa gag gga gta atc aca att 624
Ile Ala Lys Ala Met Glu Lys Val Gly Lys Glu Gly Val Ile Thr Ile
        195                 200                 205

tca gat ggc aaa acc tta tat aat gag ttg gaa gtt gtt gaa gga atg 672
Ser Asp Gly Lys Thr Leu Tyr Asn Glu Leu Glu Val Val Glu Gly Met
    210                 215                 220

aag ctc gac agg ggc tac ata tct cct tat ttc att aca aat gat aag 720
Lys Leu Asp Arg Gly Tyr Ile Ser Pro Tyr Phe Ile Thr Asn Asp Lys
225                 230                 235                 240

aac cag aaa tgt gaa ctt gaa gat ccc ctc att cta att cat gag aag 768
Asn Gln Lys Cys Glu Leu Glu Asp Pro Leu Ile Leu Ile His Glu Lys
                245                 250                 255

aaa atc tcg agt ata aat gct ata gtt aaa gtc tta gag ttg gct ttg 816
Lys Ile Ser Ser Ile Asn Ala Ile Val Lys Val Leu Glu Leu Ala Leu
            260                 265                 270

aag aga caa aga tct tta ttg att att gcc gag gat gtg gaa agt gat 864
Lys Arg Gln Arg Ser Leu Leu Ile Ile Ala Glu Asp Val Glu Ser Asp
        275                 280                 285

gca ctt gca act ctt att cta aat aag ctt cgg gct gga att aag gta 912
Ala Leu Ala Thr Leu Ile Leu Asn Lys Leu Arg Ala Gly Ile Lys Val
    290                 295                 300

tgt gct att aaa gcc cct ggt ttt ggc gaa aat agg aaa gcc aat ctt 960
Cys Ala Ile Lys Ala Pro Gly Phe Gly Glu Asn Arg Lys Ala Asn Leu
305                 310                 315                 320

caa gat ctt gca gtt ctc aca gga gga gca ctt att act gaa gag ctt 1008
Gln Asp Leu Ala Val Leu Thr Gly Gly Ala Leu Ile Thr Glu Glu Leu
                325                 330                 335

ggc ttg aag ctt gaa aaa gtg gat ttg gat atg ctt ggc acc tgt aaa 1056
Gly Leu Lys Leu Glu Lys Val Asp Leu Asp Met Leu Gly Thr Cys Lys

340                 345                 350

aaa ata aca gtt tct aag gat gac act gtc att ctt gac gga gca ggt      1104
Lys Ile Thr Val Ser Lys Asp Asp Thr Val Ile Leu Asp Gly Ala Gly
        355                 360                 365

gac aag aaa gca ctt gag gaa aga tgc gag cag att agg tct gca att      1152
Asp Lys Lys Ala Leu Glu Glu Arg Cys Glu Gln Ile Arg Ser Ala Ile
    370                 375                 380

gaa aac agc acc tca gat tat gac aag gaa aag tta cag gaa aga tta      1200
Glu Asn Ser Thr Ser Asp Tyr Asp Lys Glu Lys Leu Gln Glu Arg Leu
385                 390                 395                 400

gcc aag ctt tct ggt ggt gtt gcc gtg ctt aag att gga gga gcc agt      1248
Ala Lys Leu Ser Gly Gly Val Ala Val Leu Lys Ile Gly Gly Ala Ser
                405                 410                 415

gaa gct gaa gtt ggt gag aag aag gat aga gtg aca gat gcc tta aat      1296
Glu Ala Glu Val Gly Glu Lys Lys Asp Arg Val Thr Asp Ala Leu Asn
            420                 425                 430

gca act aag gct gct gta gag gaa ggc att gta tca ggt ggt ggt gtt      1344
Ala Thr Lys Ala Ala Val Glu Glu Gly Ile Val Ser Gly Gly Gly Val
        435                 440                 445

gct ctt cta tat gcg tca aaa gag ttg gat aaa ctt caa act gct aac      1392
Ala Leu Leu Tyr Ala Ser Lys Glu Leu Asp Lys Leu Gln Thr Ala Asn
    450                 455                 460

ttt gat cag aag att ggt gtc cag att atc caa aat gct tta aag aca      1440
Phe Asp Gln Lys Ile Gly Val Gln Ile Ile Gln Asn Ala Leu Lys Thr
465                 470                 475                 480

cct gtg ctc aca att gca tca aat gcg ggt gtt gag ggt gca gtt gtt      1488
Pro Val Leu Thr Ile Ala Ser Asn Ala Gly Val Glu Gly Ala Val Val
                485                 490                 495

gtt ggc aaa cta ttg gaa cag gag aat cat gat cta gga tat gat gca      1536
Val Gly Lys Leu Leu Glu Gln Glu Asn His Asp Leu Gly Tyr Asp Ala
            500                 505                 510

gcc aaa ggt gaa tat gtt gat atg gtt aaa gct gga atc ata gac cct      1584
Ala Lys Gly Glu Tyr Val Asp Met Val Lys Ala Gly Ile Ile Asp Pro
        515                 520                 525

ttg aag gtg att agg aca gcc ttg gtt gat gca gcc agt gta tca tct      1632
Leu Lys Val Ile Arg Thr Ala Leu Val Asp Ala Ala Ser Val Ser Ser
    530                 535                 540

ttg atg aca aca act gaa gct gtt gtg tct gaa ctt cca aac gat gat      1680
Leu Met Thr Thr Thr Glu Ala Val Val Ser Glu Leu Pro Asn Asp Asp
545                 550                 555                 560

aaa gat act cca gct atg gct ggt ggc atg ggt ggc atg ggt tat          1725
Lys Asp Thr Pro Ala Met Ala Gly Gly Met Gly Gly Met Gly Tyr
                565                 570                 575

<210>  220
<211>  575
<212>  PRT
<213>  Glycine max

<400>  220

Met Tyr Arg Phe Ala Ser Asn Leu Ala Ser Lys Ala Arg Ile Ala Arg
1               5                   10                  15

Ser Ser Ser His Gln Ile Gly Ser Arg Leu Ser Ser Ser Arg Asn Tyr
            20                  25                  30

Ala Ala Lys Asp Ile Arg Phe Gly Val Glu Ala Arg Ala Leu Met Leu
        35                  40                  45

Lys Gly Val Glu Glu Leu Ala Asp Ala Val Lys Val Thr Met Gly Pro
    50                  55                  60

Lys Gly Arg Asn Val Val Ile Glu Gln Ser Phe Gly Ala Pro Lys Val
65                  70                  75                  80

Thr Lys Asp Gly Val Thr Val Ala Lys Ser Ile Glu Phe Lys Asp Lys
                85                  90                  95

Val Lys Asn Val Gly Ala Ser Leu Val Lys Gln Val Ala Asn Ala Thr
            100                 105                 110

Asn Asp Val Ala Gly Asp Gly Thr Thr Cys Ala Thr Val Leu Thr Arg
        115                 120                 125

Ala Ile Phe Thr Glu Gly Cys Lys Ser Val Ala Ala Gly Met Asn Ala
    130                 135                 140

Met Asp Leu Arg Arg Gly Ile Ser Met Ala Val Asp Ala Val Val Thr
145                 150                 155                 160

Asn Leu Lys Ser Arg Ala Arg Met Ile Ser Thr Ser Glu Glu Ile Ala
                165                 170                 175

Gln Val Gly Thr Ile Ser Ala Asn Gly Glu Arg Glu Ile Gly Glu Leu
            180                 185                 190

Ile Ala Lys Ala Met Glu Lys Val Gly Lys Glu Gly Val Ile Thr Ile
        195                 200                 205

Ser Asp Gly Lys Thr Leu Tyr Asn Glu Leu Glu Val Val Glu Gly Met
    210                 215                 220

Lys Leu Asp Arg Gly Tyr Ile Ser Pro Tyr Phe Ile Thr Asn Asp Lys
225                 230                 235                 240

Asn Gln Lys Cys Glu Leu Glu Asp Pro Leu Ile Leu Ile His Glu Lys
                245                 250                 255

Lys Ile Ser Ser Ile Asn Ala Ile Val Lys Val Leu Glu Leu Ala Leu
            260                 265                 270

Lys Arg Gln Arg Ser Leu Leu Ile Ile Ala Glu Asp Val Glu Ser Asp
        275                 280                 285

Ala Leu Ala Thr Leu Ile Leu Asn Lys Leu Arg Ala Gly Ile Lys Val
    290                 295                 300

Cys Ala Ile Lys Ala Pro Gly Phe Gly Glu Asn Arg Lys Ala Asn Leu
305                 310                 315                 320

Gln Asp Leu Ala Val Leu Thr Gly Gly Ala Leu Ile Thr Glu Glu Leu
                325                 330                 335

Gly Leu Lys Leu Glu Lys Val Asp Leu Asp Met Leu Gly Thr Cys Lys
            340                 345                 350

Lys Ile Thr Val Ser Lys Asp Asp Thr Val Ile Leu Asp Gly Ala Gly
        355                 360                 365

Asp Lys Lys Ala Leu Glu Glu Arg Cys Glu Gln Ile Arg Ser Ala Ile
    370                 375                 380

Glu Asn Ser Thr Ser Asp Tyr Asp Lys Glu Lys Leu Gln Glu Arg Leu
385                 390                 395                 400

Ala Lys Leu Ser Gly Gly Val Ala Val Leu Lys Ile Gly Gly Ala Ser
                405                 410                 415

Glu Ala Glu Val Gly Glu Lys Lys Asp Arg Val Thr Asp Ala Leu Asn
            420                 425                 430

Ala Thr Lys Ala Ala Val Glu Glu Gly Ile Val Ser Gly Gly Gly Val
        435                 440                 445

Ala Leu Leu Tyr Ala Ser Lys Glu Leu Asp Lys Leu Gln Thr Ala Asn
    450                 455                 460

Phe Asp Gln Lys Ile Gly Val Gln Ile Ile Gln Asn Ala Leu Lys Thr
465                 470                 475                 480

Pro Val Leu Thr Ile Ala Ser Asn Ala Gly Val Glu Gly Ala Val Val
                485                 490                 495

Val Gly Lys Leu Leu Glu Gln Glu Asn His Asp Leu Gly Tyr Asp Ala
            500                 505                 510

Ala Lys Gly Glu Tyr Val Asp Met Val Lys Ala Gly Ile Ile Asp Pro
        515                 520                 525

Leu Lys Val Ile Arg Thr Ala Leu Val Asp Ala Ala Ser Val Ser Ser
    530                 535                 540

Leu Met Thr Thr Thr Glu Ala Val Val Ser Glu Leu Pro Asn Asp Asp
545                 550                 555                 560

Lys Asp Thr Pro Ala Met Ala Gly Gly Met Gly Gly Met Gly Tyr
                565                 570                 575

<210>  221
<211>  8
<212>  PRT
<213>  Glycine max

<400>  221

Ala Gly Ile Ile Asp Pro Leu Lys
1               5

<210>  222
<211>  11
<212>  PRT
<213>  Glycine max

<400>  222

Asp Arg Val Thr Asp Ala Leu Asn Ala Thr Lys
1               5                   10

<210>  223
<211>  10
<212>  PRT
<213>  Glycine max

<400>  223

Glu Gly Val Ile Thr Ile Ser Asp Gly Lys
1               5                   10

<210>  224
<211>  14
<212>  PRT
<213>  Glycine max

<400>  224

Gly Arg Asn Val Val Ile Glu Gln Ser Phe Gly Ala Pro Lys
1               5                   10

<210>  225
<211>  10
<212>  PRT
<213>  Glycine max

<400>  225

Gly Val Glu Glu Leu Ala Asp Ala Val Lys
1               5                   10


<210>  226
<211>  12
<212>  PRT
<213>  Glycine max

<400>  226

Ile Gly Gly Ala Ser Glu Ala Glu Val Gly Glu Lys
1               5                   10


<210>  227
<211>  11
<212>  PRT
<213>  Glycine max

<400>  227

Ile Gly Val Gln Ile Ile Gln Asn Ala Leu Lys
1               5                   10


<210>  228
<211>  9
<212>  PRT
<213>  Glycine max

<400>  228

Ile Ser Ser Ile Asn Ala Ile Val Lys
1               5


<210>  229
<211>  15
<212>  PRT
<213>  Glycine max

<400>  229

Leu Leu Glu Gln Glu Asn His Asp Leu Gly Tyr Asp Ala Ala Lys
1               5                   10                  15


<210>  230
<211>  9
<212>  PRT
<213>  Glycine max

<400>  230

Leu Gln Thr Ala Asn Phe Asp Gln Lys
1               5

<210>  231
<211>  9
<212>  PRT
<213>  Glycine max

<400>  231

Leu Ser Gly Gly Val Ala Val Leu Lys
1               5

<210>  232
<211>  1869
<212>  DNA
<213>  Glycine max

<220>
<221>  CDS
<222>  (1)..(1869)

<400>  232
atg gct caa gtt ctt gag aac ccc acc att gac gcg gcg gct tcc ggc          48
Met Ala Gln Val Leu Glu Asn Pro Thr Ile Asp Ala Ala Ala Ser Gly
1               5                   10                  15

gcg aac ccc tcg ctg acg acc atc tcg ttg tac gtc ggg gat ctt cac          96
Ala Asn Pro Ser Leu Thr Thr Ile Ser Leu Tyr Val Gly Asp Leu His
            20                  25                  30

cac gac gtc aac gat ccg cag ctg tat gat ctc ttc aac cag gtt gcc         144
His Asp Val Asn Asp Pro Gln Leu Tyr Asp Leu Phe Asn Gln Val Ala
        35                  40                  45

caa gtc gtt tcc gtt cga att tgc agg gac gtg gcc acc caa caa tcc         192
Gln Val Val Ser Val Arg Ile Cys Arg Asp Val Ala Thr Gln Gln Ser
    50                  55                  60

ctt ggt tac ggc tac gtc aat ttc agc aac gct cac gat gcc gca aaa         240
Leu Gly Tyr Gly Tyr Val Asn Phe Ser Asn Ala His Asp Ala Ala Lys
65                  70                  75                  80

gca att gat gtg ctg aat ttc act cca ctg aac ggc aaa atc att agg         288
Ala Ile Asp Val Leu Asn Phe Thr Pro Leu Asn Gly Lys Ile Ile Arg
                85                  90                  95

ata atg tac tct att cgg gac cct agt gct cgg aag agt ggt gca gct         336
Ile Met Tyr Ser Ile Arg Asp Pro Ser Ala Arg Lys Ser Gly Ala Ala
            100                 105                 110

aat gtt ttc ata aag aat ttg gac aag gcg att gat cac aag gct ttg         384
Asn Val Phe Ile Lys Asn Leu Asp Lys Ala Ile Asp His Lys Ala Leu
        115                 120                 125

tac gat aca ttt tcg gct ttt ggg aat att cta tct tgc aag gtt gcg         432
Tyr Asp Thr Phe Ser Ala Phe Gly Asn Ile Leu Ser Cys Lys Val Ala
    130                 135                 140

aca gat gct tct ggc cag tct aaa ggc cat ggt ttt gtg cag ttt gag 480
Thr Asp Ala Ser Gly Gln Ser Lys Gly His Gly Phe Val Gln Phe Glu
145                 150                 155                 160

agt gaa gaa tct gca caa aat gca att gac aag tta aac ggc atg ctg 528
Ser Glu Glu Ser Ala Gln Asn Ala Ile Asp Lys Leu Asn Gly Met Leu
                165                 170                 175

atc aat gat aag cag gtc ttt gtg ggt cct ttc ctg cgg aag cag gat 576
Ile Asn Asp Lys Gln Val Phe Val Gly Pro Phe Leu Arg Lys Gln Asp
            180                 185                 190

aga gag agt gct ctc agt ggc aca aag ttt aat aac gtt ttc gtg aaa 624
Arg Glu Ser Ala Leu Ser Gly Thr Lys Phe Asn Asn Val Phe Val Lys
        195                 200                 205

aac ctg ttg gat tca atg act gag gct gac ttg gag aga att ttt gga 672
Asn Leu Leu Asp Ser Met Thr Glu Ala Asp Leu Glu Arg Ile Phe Gly
    210                 215                 220

gaa tat gga gct att act agc gct gta gtg atg aga gat gta gat ggt 720
Glu Tyr Gly Ala Ile Thr Ser Ala Val Val Met Arg Asp Val Asp Gly
225                 230                 235                 240

aaa tca aaa ggt ttt ggt ttt gtc aat ttt gca aat gta gac gat gct 768
Lys Ser Lys Gly Phe Gly Phe Val Asn Phe Ala Asn Val Asp Asp Ala
                245                 250                 255

gct aaa gct gtg gag gct ctt aat gga aag aat ttt gat ggt aag gag 816
Ala Lys Ala Val Glu Ala Leu Asn Gly Lys Asn Phe Asp Gly Lys Glu
            260                 265                 270

tgg tat gtt gga aaa gcc cag aaa aag tct gaa agg gag ctt gaa ctg 864
Trp Tyr Val Gly Lys Ala Gln Lys Lys Ser Glu Arg Glu Leu Glu Leu
        275                 280                 285

aaa ggg caa cat gaa cag att aca aag gag act gtt gat aaa tat cat 912
Lys Gly Gln His Glu Gln Ile Thr Lys Glu Thr Val Asp Lys Tyr His
    290                 295                 300

ggt aca aac ttg tat atc aag aac ttg gat gat agt gtt ggt gat gaa 960
Gly Thr Asn Leu Tyr Ile Lys Asn Leu Asp Asp Ser Val Gly Asp Glu
305                 310                 315                 320

gaa ctc atg gaa ctg ttc tct gag ttt ggt acg ata acc tca tgc aag 1008
Glu Leu Met Glu Leu Phe Ser Glu Phe Gly Thr Ile Thr Ser Cys Lys
                325                 330                 335

gtt atg cga gat cca aat gga att agt aga gga tct gga ttt gtt tca 1056
Val Met Arg Asp Pro Asn Gly Ile Ser Arg Gly Ser Gly Phe Val Ser
            340                 345                 350

ttt tca att gct gag gga gca act cgg gct ctt ggt gag atg aat ggt 1104
Phe Ser Ile Ala Glu Gly Ala Thr Arg Ala Leu Gly Glu Met Asn Gly
        355                 360                 365

aaa atg gtt gct ggc aaa cct ctc tat gtt gcc ctt gca cag aga aaa 1152
Lys Met Val Ala Gly Lys Pro Leu Tyr Val Ala Leu Ala Gln Arg Lys
    370                 375                 380

gaa gat aga aga gca agg tta cag gca caa ttt tca caa tcg agg cct 1200
Glu Asp Arg Arg Ala Arg Leu Gln Ala Gln Phe Ser Gln Ser Arg Pro
385                 390                 395                 400

gct gca ata acc cct aat gtt tca cct cga atg cct cta tac cct cta 1248
Ala Ala Ile Thr Pro Asn Val Ser Pro Arg Met Pro Leu Tyr Pro Leu
                405                 410                 415

ggg gct cct gcg ata gga caa caa ttt ttg tat ggg caa gca gct cca 1296
Gly Ala Pro Ala Ile Gly Gln Gln Phe Leu Tyr Gly Gln Ala Ala Pro
            420                 425                 430

gcc acg ata cct caa cat ttt gtt cct gga atg agg cca ggt ggt gct 1344
Ala Thr Ile Pro Gln His Phe Val Pro Gly Met Arg Pro Gly Gly Ala
        435                 440                 445

cca aac ttc tat gtt cca ctg gtt cag cag ggt caa cag gga cag cgc 1392
Pro Asn Phe Tyr Val Pro Leu Val Gln Gln Gly Gln Gln Gly Gln Arg
    450                 455                 460

cca gga gga cac cga gga tca ggt cct atg caa cag ctc cag cag cca 1440
Pro Gly Gly His Arg Gly Ser Gly Pro Met Gln Gln Leu Gln Gln Pro
465                 470                 475                 480

ttg cca ttg atg cac caa cag atg ctt cca agg gga cat gtc tat cga 1488
Leu Pro Leu Met His Gln Gln Met Leu Pro Arg Gly His Val Tyr Arg
                485                 490                 495

tat cct tct ggc tcc aat atg caa aat att caa ctg gca gga gta cct 1536
Tyr Pro Ser Gly Ser Asn Met Gln Asn Ile Gln Leu Ala Gly Val Pro
            500                 505                 510

gga gga atg gtc act tat ggc att ggt caa caa atg ccc acg cag gcc 1584
Gly Gly Met Val Thr Tyr Gly Ile Gly Gln Gln Met Pro Thr Gln Ala
        515                 520                 525

ctg gcc tcg gcc ctt gcc aat gct act cct gaa cag cag agg act atg 1632
Leu Ala Ser Ala Leu Ala Asn Ala Thr Pro Glu Gln Gln Arg Thr Met
    530                 535                 540

ctt ggt gaa gct tta tac ccg tta gta gat aag ctg gag cat gaa gca 1680
Leu Gly Glu Ala Leu Tyr Pro Leu Val Asp Lys Leu Glu His Glu Ala
545                 550                 555                 560

gca gca aag gtt act ggt atg ctc tta gag atg gat cag cct gag gta 1728
Ala Ala Lys Val Thr Gly Met Leu Leu Glu Met Asp Gln Pro Glu Val
                565                 570                 575

tta cat ctg att gag tca ccg gat gct ctc aag gct aaa gtt gtt gag 1776
Leu His Leu Ile Glu Ser Pro Asp Ala Leu Lys Ala Lys Val Val Glu
            580                 585                 590

gcc atg gat gtg ttg aag aaa gtt aca cag cag caa tca aac agc cca 1824
Ala Met Asp Val Leu Lys Lys Val Thr Gln Gln Gln Ser Asn Ser Pro
        595                 600                 605

gcc gat caa ctg gct tca ctc tct ctg aat gat aat gtc gag tct 1869
Ala Asp Gln Leu Ala Ser Leu Ser Leu Asn Asp Asn Val Glu Ser
    610                 615                 620

<210> 233
<211> 623
<212> PRT
<213> Glycine max

<400> 233

Met Ala Gln Val Leu Glu Asn Pro Thr Ile Asp Ala Ala Ala Ser Gly
1               5                   10                  15

Ala Asn Pro Ser Leu Thr Thr Ile Ser Leu Tyr Val Gly Asp Leu His
            20                  25                  30

His Asp Val Asn Asp Pro Gln Leu Tyr Asp Leu Phe Asn Gln Val Ala
        35                  40                  45

Gln Val Val Ser Val Arg Ile Cys Arg Asp Val Ala Thr Gln Gln Ser
    50                  55                  60

Leu Gly Tyr Gly Tyr Val Asn Phe Ser Asn Ala His Asp Ala Ala Lys
65                  70                  75                  80

Ala Ile Asp Val Leu Asn Phe Thr Pro Leu Asn Gly Lys Ile Ile Arg
                85                  90                  95

Ile Met Tyr Ser Ile Arg Asp Pro Ser Ala Arg Lys Ser Gly Ala Ala
            100                 105                 110

Asn Val Phe Ile Lys Asn Leu Asp Lys Ala Ile Asp His Lys Ala Leu
        115                 120                 125

Tyr Asp Thr Phe Ser Ala Phe Gly Asn Ile Leu Ser Cys Lys Val Ala
    130                 135                 140

Thr Asp Ala Ser Gly Gln Ser Lys Gly His Gly Phe Val Gln Phe Glu
145                 150                 155                 160

Ser Glu Glu Ser Ala Gln Asn Ala Ile Asp Lys Leu Asn Gly Met Leu
                165                 170                 175

Ile Asn Asp Lys Gln Val Phe Val Gly Pro Phe Leu Arg Lys Gln Asp
            180                 185                 190

Arg Glu Ser Ala Leu Ser Gly Thr Lys Phe Asn Asn Val Phe Val Lys
        195                 200                 205

Asn Leu Leu Asp Ser Met Thr Glu Ala Asp Leu Glu Arg Ile Phe Gly
    210                 215                 220

Glu Tyr Gly Ala Ile Thr Ser Ala Val Val Met Arg Asp Val Asp Gly
225                 230                 235                 240

Lys Ser Lys Gly Phe Gly Phe Val Asn Phe Ala Asn Val Asp Asp Ala

245                 250                 255

Ala Lys Ala Val Glu Ala Leu Asn Gly Lys Asn Phe Asp Gly Lys Glu
            260                 265                 270

Trp Tyr Val Gly Lys Ala Gln Lys Lys Ser Glu Arg Glu Leu Glu Leu
        275                 280                 285

Lys Gly Gln His Glu Gln Ile Thr Lys Glu Thr Val Asp Lys Tyr His
    290                 295                 300

Gly Thr Asn Leu Tyr Ile Lys Asn Leu Asp Asp Ser Val Gly Asp Glu
305                 310                 315                 320

Glu Leu Met Glu Leu Phe Ser Glu Phe Gly Thr Ile Thr Ser Cys Lys
                325                 330                 335

Val Met Arg Asp Pro Asn Gly Ile Ser Arg Gly Ser Gly Phe Val Ser
            340                 345                 350

Phe Ser Ile Ala Glu Gly Ala Thr Arg Ala Leu Gly Glu Met Asn Gly
        355                 360                 365

Lys Met Val Ala Gly Lys Pro Leu Tyr Val Ala Leu Ala Gln Arg Lys
    370                 375                 380

Glu Asp Arg Arg Ala Arg Leu Gln Ala Gln Phe Ser Gln Ser Arg Pro
385                 390                 395                 400

Ala Ala Ile Thr Pro Asn Val Ser Pro Arg Met Pro Leu Tyr Pro Leu
                405                 410                 415

Gly Ala Pro Ala Ile Gly Gln Gln Phe Leu Tyr Gly Gln Ala Ala Pro
            420                 425                 430

Ala Thr Ile Pro Gln His Phe Val Pro Gly Met Arg Pro Gly Gly Ala
        435                 440                 445

Pro Asn Phe Tyr Val Pro Leu Val Gln Gln Gly Gln Gln Gly Gln Arg
    450                 455                 460

Pro Gly Gly His Arg Gly Ser Gly Pro Met Gln Gln Leu Gln Gln Pro
465                 470                 475                 480

Leu Pro Leu Met His Gln Gln Met Leu Pro Arg Gly His Val Tyr Arg
                485                 490                 495

Tyr Pro Ser Gly Ser Asn Met Gln Asn Ile Gln Leu Ala Gly Val Pro
            500                 505                 510

Gly Gly Met Val Thr Tyr Gly Ile Gly Gln Gln Met Pro Thr Gln Ala
        515                 520                 525

Leu Ala Ser Ala Leu Ala Asn Ala Thr Pro Glu Gln Gln Arg Thr Met
    530                 535                 540

Leu Gly Glu Ala Leu Tyr Pro Leu Val Asp Lys Leu Glu His Glu Ala
545                 550                 555                 560

Ala Ala Lys Val Thr Gly Met Leu Leu Glu Met Asp Gln Pro Glu Val
                565                 570                 575

Leu His Leu Ile Glu Ser Pro Asp Ala Leu Lys Ala Lys Val Val Glu
            580                 585                 590

Ala Met Asp Val Leu Lys Lys Val Thr Gln Gln Gln Ser Asn Ser Pro
        595                 600                 605

Ala Asp Gln Leu Ala Ser Leu Ser Leu Asn Asp Asn Val Glu Ser
    610                 615                 620

<210>  234
<211>  13
<212>  PRT
<213>  Glycine max

<400>  234

Ala Ile Asp Val Leu Asn Phe Thr Pro Leu Asn Gly Lys
1               5                   10

<210>  235
<211>  7
<212>  PRT
<213>  Glycine max

<400>  235

Phe Asn Asn Val Phe Val Lys
1               5

<210>  236
<211>  15
<212>  PRT
<213>  Glycine max

<400>  236

Gly Phe Gly Phe Val Asn Phe Ala Asn Val Asp Asp Ala Ala Lys
1               5                   10                  15

<210>  237
<211>  9
<212>  PRT
<213>  Glycine max

<400>  237

Ser Gly Ala Ala Asn Val Phe Ile Lys
1               5

<210>  238
<211>  1851
<212>  DNA
<213>  Glycine max

<220>
<221>  CDS
<222>  (1)..(1851)

<400>  238
atg gat tct gat tat ggt att ccc agg gaa ctc tca gat ctt caa aag      48
Met Asp Ser Asp Tyr Gly Ile Pro Arg Glu Leu Ser Asp Leu Gln Lys
1               5                   10                  15

att cgg tct ttg tac cag cca gag ctc cct cct tgt ctc cag gga acc      96
Ile Arg Ser Leu Tyr Gln Pro Glu Leu Pro Pro Cys Leu Gln Gly Thr
            20                  25                  30

act gtg agg gtt gaa ttt ggt gac gca acc acc act gct gac ccc act      144
Thr Val Arg Val Glu Phe Gly Asp Ala Thr Thr Thr Ala Asp Pro Thr
        35                  40                  45

gat gca ctc acc gtc tgc agg gct ttt cct aac act tat gga cac cct      192
Asp Ala Leu Thr Val Cys Arg Ala Phe Pro Asn Thr Tyr Gly His Pro
    50                  55                  60

ttg gct cac ttt ctc agg gcc act gcc aaa gtc cct gat gct cag atc      240
Leu Ala His Phe Leu Arg Ala Thr Ala Lys Val Pro Asp Ala Gln Ile
65                  70                  75                  80

ata act gag ttc cct cct att agg gtg ggg att gtc ttt tgt ggg aga      288
Ile Thr Glu Phe Pro Pro Ile Arg Val Gly Ile Val Phe Cys Gly Arg
                85                  90                  95

caa tct cct gga ggg cat aat gtc att tgg ggt atc cac aat gct ctc      336
Gln Ser Pro Gly Gly His Asn Val Ile Trp Gly Ile His Asn Ala Leu
            100                 105                 110

aaa att cac aat ccc aac agt gtt ttg ctt gga ttt ctt ggt ggt tca      384
Lys Ile His Asn Pro Asn Ser Val Leu Leu Gly Phe Leu Gly Gly Ser
        115                 120                 125

gaa ggt cta ttt gcc cag aaa act ctg gag ata act gac gat att ctt      432
Glu Gly Leu Phe Ala Gln Lys Thr Leu Glu Ile Thr Asp Asp Ile Leu
    130                 135                 140

tcg acg tac aaa aat caa ggt ggt tat gat ttg ctt ggg cgg acg aag      480
Ser Thr Tyr Lys Asn Gln Gly Gly Tyr Asp Leu Leu Gly Arg Thr Lys
145                 150                 155                 160

gat caa att agg acc aca gaa caa gtt aat gct gca ctt gct gca tgc 528
Asp Gln Ile Arg Thr Thr Glu Gln Val Asn Ala Ala Leu Ala Ala Cys
                165                 170                 175

aac aat ttg aaa ctt gat ggc ctt gtc ata atc gga ggg gtg aca tca 576
Asn Asn Leu Lys Leu Asp Gly Leu Val Ile Ile Gly Gly Val Thr Ser
            180                 185                 190

aac aca gat gct gca cag ctt gct gaa aca ttt gtt gta gca aaa tgc 624
Asn Thr Asp Ala Ala Gln Leu Ala Glu Thr Phe Val Val Ala Lys Cys
        195                 200                 205

ccc aca aag gtg gtt ggt gtt cct gtc act ctg aat gga gat ctt aaa 672
Pro Thr Lys Val Val Gly Val Pro Val Thr Leu Asn Gly Asp Leu Lys
    210                 215                 220

aac cag ttt gta gaa act aat gtt ggt ttt gat aca att tgt aag gtt 720
Asn Gln Phe Val Glu Thr Asn Val Gly Phe Asp Thr Ile Cys Lys Val
225                 230                 235                 240

aat tct cag ctc atc agc aat gtc tgc act gat gct ctc tct gca gag 768
Asn Ser Gln Leu Ile Ser Asn Val Cys Thr Asp Ala Leu Ser Ala Glu
                245                 250                 255

aag tat tat tat ttc atc cgt ctt atg gga cgc aag gca tca cac gtt 816
Lys Tyr Tyr Tyr Phe Ile Arg Leu Met Gly Arg Lys Ala Ser His Val
            260                 265                 270

gct ttg gaa tgc acc ctc cag tcc cat cca aat atg gta att ctt ggt 864
Ala Leu Glu Cys Thr Leu Gln Ser His Pro Asn Met Val Ile Leu Gly
        275                 280                 285

gag gag gtt gct gca tca aag ctt acc ctt tct gaa att gca aaa cag 912
Glu Glu Val Ala Ala Ser Lys Leu Thr Leu Ser Glu Ile Ala Lys Gln
    290                 295                 300

att act gat gca att cag gct aga gca gag caa gac aaa tac cat gga 960
Ile Thr Asp Ala Ile Gln Ala Arg Ala Glu Gln Asp Lys Tyr His Gly
305                 310                 315                 320

gtc att ctc ctt cca gaa ggg ctg ata gag agt att cct gaa gtg tat 1008
Val Ile Leu Leu Pro Glu Gly Leu Ile Glu Ser Ile Pro Glu Val Tyr
                325                 330                 335

gcc ctc ttg aag gaa att cat ggt tta ctc aga caa ggt gtt gaa gtt 1056
Ala Leu Leu Lys Glu Ile His Gly Leu Leu Arg Gln Gly Val Glu Val
            340                 345                 350

gac aag ata tct tct cag cta aca cca tgg gca tct gct tta ttt gaa 1104
Asp Lys Ile Ser Ser Gln Leu Thr Pro Trp Ala Ser Ala Leu Phe Glu
        355                 360                 365

ttt ctg cca ccc ttt att agg aga cag ttg ctc ctt tac cct gaa tcc 1152
Phe Leu Pro Pro Phe Ile Arg Arg Gln Leu Leu Leu Tyr Pro Glu Ser
    370                 375                 380

gac gac tca gca cag tta tca cag att gaa act gag aaa tta cta gca 1200
Asp Asp Ser Ala Gln Leu Ser Gln Ile Glu Thr Glu Lys Leu Leu Ala
385                 390                 395                 400

tat ctt gtg gaa gtc gag att aac aag cgt ctg aaa gaa ggc aca tac 1248
Tyr Leu Val Glu Val Glu Ile Asn Lys Arg Leu Lys Glu Gly Thr Tyr

405                 410                 415

aag ggg aag aaa ttc aat gcc att tgc cac ttt ttt ggt tat caa gct      1296
Lys Gly Lys Lys Phe Asn Ala Ile Cys His Phe Phe Gly Tyr Gln Ala
            420                 425                 430

cgt gga tct ctg cca tca aaa ttt gac tgt gat tac gca tat gtc ctt      1344
Arg Gly Ser Leu Pro Ser Lys Phe Asp Cys Asp Tyr Ala Tyr Val Leu
        435                 440                 445

ggg cac atc tgc tac cat ata ctg gcc gct ggt cta aat gga tac atg      1392
Gly His Ile Cys Tyr His Ile Leu Ala Ala Gly Leu Asn Gly Tyr Met
    450                 455                 460

gca act tta act aac ctg aag aat cca gta aac aag tgg aaa tgt ggg      1440
Ala Thr Leu Thr Asn Leu Lys Asn Pro Val Asn Lys Trp Lys Cys Gly
465                 470                 475                 480

gct gct cca att gca tct atg atg act gta aag cgc tgg tct cca aat      1488
Ala Ala Pro Ile Ala Ser Met Met Thr Val Lys Arg Trp Ser Pro Asn
                485                 490                 495

cca gga gct aca tca att gga aaa cct gct att cat cca gct act gtg      1536
Pro Gly Ala Thr Ser Ile Gly Lys Pro Ala Ile His Pro Ala Thr Val
            500                 505                 510

gac ttg aga ggc aaa gca tat gag ctg ttg aga caa aag gca caa agc      1584
Asp Leu Arg Gly Lys Ala Tyr Glu Leu Leu Arg Gln Lys Ala Gln Ser
        515                 520                 525

ttt ctt atg gat gac atc tac aga aat cca ggt cca ctt cag ttt gat      1632
Phe Leu Met Asp Asp Ile Tyr Arg Asn Pro Gly Pro Leu Gln Phe Asp
    530                 535                 540

ggt ccc ggc gca gat gcc aag gtt ata act ctt tct gtt gaa gac cag      1680
Gly Pro Gly Ala Asp Ala Lys Val Ile Thr Leu Ser Val Glu Asp Gln
545                 550                 555                 560

gac tac atg ggc cgc att aag aaa ctt cag gag tac ctt gaa cag gtt      1728
Asp Tyr Met Gly Arg Ile Lys Lys Leu Gln Glu Tyr Leu Glu Gln Val
                565                 570                 575

cga acc att gtc aag cca gga tgc cct cag gag gtt ttg aaa gct gct      1776
Arg Thr Ile Val Lys Pro Gly Cys Pro Gln Glu Val Leu Lys Ala Ala
            580                 585                 590

cta agc gtc atg gcg tcg gtt acc gaa gtt cta gct gca atg tca aca      1824
Leu Ser Val Met Ala Ser Val Thr Glu Val Leu Ala Ala Met Ser Thr
        595                 600                 605

tct tat acc aac acc ctg gca tcc ctg                                  1851
Ser Tyr Thr Asn Thr Leu Ala Ser Leu
    610                 615


<210>  239
<211>  617
<212>  PRT
<213>  Glycine max

<400>  239

Met Asp Ser Asp Tyr Gly Ile Pro Arg Glu Leu Ser Asp Leu Gln Lys

1               5                   10                  15

Ile Arg Ser Leu Tyr Gln Pro Glu Leu Pro Pro Cys Leu Gln Gly Thr
            20                  25                  30

Thr Val Arg Val Glu Phe Gly Asp Ala Thr Thr Thr Ala Asp Pro Thr
        35                  40                  45

Asp Ala Leu Thr Val Cys Arg Ala Phe Pro Asn Thr Tyr Gly His Pro
    50                  55                  60

Leu Ala His Phe Leu Arg Ala Thr Ala Lys Val Pro Asp Ala Gln Ile
65                  70                  75                  80

Ile Thr Glu Phe Pro Pro Ile Arg Val Gly Ile Val Phe Cys Gly Arg
                85                  90                  95

Gln Ser Pro Gly Gly His Asn Val Ile Trp Gly Ile His Asn Ala Leu
            100                 105                 110

Lys Ile His Asn Pro Asn Ser Val Leu Leu Gly Phe Leu Gly Gly Ser
        115                 120                 125

Glu Gly Leu Phe Ala Gln Lys Thr Leu Glu Ile Thr Asp Asp Ile Leu
    130                 135                 140

Ser Thr Tyr Lys Asn Gln Gly Gly Tyr Asp Leu Leu Gly Arg Thr Lys
145                 150                 155                 160

Asp Gln Ile Arg Thr Thr Glu Gln Val Asn Ala Ala Leu Ala Ala Cys
                165                 170                 175

Asn Asn Leu Lys Leu Asp Gly Leu Val Ile Ile Gly Gly Val Thr Ser
            180                 185                 190

Asn Thr Asp Ala Ala Gln Leu Ala Glu Thr Phe Val Val Ala Lys Cys
        195                 200                 205

Pro Thr Lys Val Val Gly Val Pro Val Thr Leu Asn Gly Asp Leu Lys
    210                 215                 220

Asn Gln Phe Val Glu Thr Asn Val Gly Phe Asp Thr Ile Cys Lys Val
225                 230                 235                 240

Asn Ser Gln Leu Ile Ser Asn Val Cys Thr Asp Ala Leu Ser Ala Glu
                245                 250                 255

Lys Tyr Tyr Tyr Phe Ile Arg Leu Met Gly Arg Lys Ala Ser His Val
            260                 265                 270

Ala Leu Glu Cys Thr Leu Gln Ser His Pro Asn Met Val Ile Leu Gly
        275                 280                 285

Glu Glu Val Ala Ala Ser Lys Leu Thr Leu Ser Glu Ile Ala Lys Gln
    290                 295                 300

Ile Thr Asp Ala Ile Gln Ala Arg Ala Glu Gln Asp Lys Tyr His Gly
305                 310                 315                 320

Val Ile Leu Leu Pro Glu Gly Leu Ile Glu Ser Ile Pro Glu Val Tyr
                325                 330                 335

Ala Leu Leu Lys Glu Ile His Gly Leu Leu Arg Gln Gly Val Glu Val
            340                 345                 350

Asp Lys Ile Ser Ser Gln Leu Thr Pro Trp Ala Ser Ala Leu Phe Glu
        355                 360                 365

Phe Leu Pro Pro Phe Ile Arg Arg Gln Leu Leu Leu Tyr Pro Glu Ser
    370                 375                 380

Asp Asp Ser Ala Gln Leu Ser Gln Ile Glu Thr Glu Lys Leu Leu Ala
385                 390                 395                 400

Tyr Leu Val Glu Val Glu Ile Asn Lys Arg Leu Lys Glu Gly Thr Tyr
                405                 410                 415

Lys Gly Lys Lys Phe Asn Ala Ile Cys His Phe Phe Gly Tyr Gln Ala
            420                 425                 430

Arg Gly Ser Leu Pro Ser Lys Phe Asp Cys Asp Tyr Ala Tyr Val Leu
        435                 440                 445

Gly His Ile Cys Tyr His Ile Leu Ala Ala Gly Leu Asn Gly Tyr Met
    450                 455                 460

Ala Thr Leu Thr Asn Leu Lys Asn Pro Val Asn Lys Trp Lys Cys Gly
465                 470                 475                 480

Ala Ala Pro Ile Ala Ser Met Met Thr Val Lys Arg Trp Ser Pro Asn
                485                 490                 495

Pro Gly Ala Thr Ser Ile Gly Lys Pro Ala Ile His Pro Ala Thr Val
            500                 505                 510

Asp Leu Arg Gly Lys Ala Tyr Glu Leu Leu Arg Gln Lys Ala Gln Ser
        515                 520                 525

Phe Leu Met Asp Asp Ile Tyr Arg Asn Pro Gly Pro Leu Gln Phe Asp
    530                 535                 540

Gly Pro Gly Ala Asp Ala Lys Val Ile Thr Leu Ser Val Glu Asp Gln
545                 550                 555                 560

Asp Tyr Met Gly Arg Ile Lys Lys Leu Gln Glu Tyr Leu Glu Gln Val
                565                 570                 575

Arg Thr Ile Val Lys Pro Gly Cys Pro Gln Glu Val Leu Lys Ala Ala
            580                 585                 590

Leu Ser Val Met Ala Ser Val Thr Glu Val Leu Ala Ala Met Ser Thr
        595                 600                 605

Ser Tyr Thr Asn Thr Leu Ala Ser Leu
    610                 615

<210>  240
<211>  13
<212>  PRT
<213>  Glycine max

<400>  240

Val Val Gly Val Pro Val Thr Leu Asn Gly Asp Leu Lys
1               5                   10

<210>  241
<211>  1575
<212>  DNA
<213>  Glycine max

<220>
<221>  CDS
<222>  (1)..(1575)

<400>  241
atg ccc aaa ttt ttt cat agc att ttc aga gga aca atg gcg ggt agg      48
Met Pro Lys Phe Phe His Ser Ile Phe Arg Gly Thr Met Ala Gly Arg
1               5                   10                  15

gtt tcg acg tgc ttc ttc ttc gtg ttt gct ctt tct ctc ctt ctt cct      96
Val Ser Thr Cys Phe Phe Phe Val Phe Ala Leu Ser Leu Leu Leu Pro
            20                  25                  30

ttt cag atc tcc gct gag gaa tca tcg gag aaa gag ttc gta ctc aca      144
Phe Gln Ile Ser Ala Glu Glu Ser Ser Glu Lys Glu Phe Val Leu Thr
        35                  40                  45

ttg gat cac tct aac ttt cac gac act gtc agc aag cac gat ttc atc 192
Leu Asp His Ser Asn Phe His Asp Thr Val Ser Lys His Asp Phe Ile
    50                  55                  60

gtc gtc gag ttc tac gct cca tgg tgt ggc cac tgt aag aag ctt gct 240
Val Val Glu Phe Tyr Ala Pro Trp Cys Gly His Cys Lys Lys Leu Ala
65                  70                  75                  80

ccc gag tat gag aaa gct gct tct atc ttg agt agt cat gat cct ccg 288
Pro Glu Tyr Glu Lys Ala Ala Ser Ile Leu Ser Ser His Asp Pro Pro
                85                  90                  95

gtt gtt ttg gct aaa att gat gcc aat gag gag aag aac aaa gac ctt 336
Val Val Leu Ala Lys Ile Asp Ala Asn Glu Glu Lys Asn Lys Asp Leu
            100                 105                 110

gca tct caa tat gat gtt agg gga tac cca aca att aag att ctg aga 384
Ala Ser Gln Tyr Asp Val Arg Gly Tyr Pro Thr Ile Lys Ile Leu Arg
        115                 120                 125

aat ggg gga aag aat gtt cag gaa tac aaa ggt ccc cgt gaa gca gat 432
Asn Gly Gly Lys Asn Val Gln Glu Tyr Lys Gly Pro Arg Glu Ala Asp
    130                 135                 140

ggc atc gtt gac tat ttg aag aaa caa agt ggt cct gct tca act gaa 480
Gly Ile Val Asp Tyr Leu Lys Lys Gln Ser Gly Pro Ala Ser Thr Glu
145                 150                 155                 160

att aaa tct gct gat gaa gct act gct ttc att ggt gaa aat aaa gtt 528
Ile Lys Ser Ala Asp Glu Ala Thr Ala Phe Ile Gly Glu Asn Lys Val
                165                 170                 175

gct att gtt gga gtt ttc ccg aaa ttt tct gga gag gag ttt gac aac 576
Ala Ile Val Gly Val Phe Pro Lys Phe Ser Gly Glu Glu Phe Asp Asn
            180                 185                 190

ttt tct gcc tta gca gag aag ttg cgt tct gac tat gac ttc ggt cac 624
Phe Ser Ala Leu Ala Glu Lys Leu Arg Ser Asp Tyr Asp Phe Gly His
        195                 200                 205

act ctg aat gcc aaa cac ctt cca aga gga gag tca tca gtt tct ggt 672
Thr Leu Asn Ala Lys His Leu Pro Arg Gly Glu Ser Ser Val Ser Gly
    210                 215                 220

ccc gta gtt agg ttg ttc aag cca ttc gat gaa ctt ttt gtt gac ttc 720
Pro Val Val Arg Leu Phe Lys Pro Phe Asp Glu Leu Phe Val Asp Phe
225                 230                 235                 240

cag gat ttc aat gtg gaa gct cta gag aaa ttt gtt gag gaa tct agt 768
Gln Asp Phe Asn Val Glu Ala Leu Glu Lys Phe Val Glu Glu Ser Ser
                245                 250                 255

acc cct gtt gtt act gtc ttt aac aat gat ccg agc aat cac cct ttc 816
Thr Pro Val Val Thr Val Phe Asn Asn Asp Pro Ser Asn His Pro Phe
            260                 265                 270

gtt gcc aaa ttc ttc aac agt ccc aat gca aag gca atg ttg ttc atc 864
Val Ala Lys Phe Phe Asn Ser Pro Asn Ala Lys Ala Met Leu Phe Ile
        275                 280                 285

aac ttc act gcc gaa ggt gct gaa tct ttc aaa tca aaa tac cgt gaa 912
Asn Phe Thr Ala Glu Gly Ala Glu Ser Phe Lys Ser Lys Tyr Arg Glu
    290                 295                 300

gct gct gag caa cat aaa caa cag ggt gta agc ttt ttg gtg gga gat 960
Ala Ala Glu Gln His Lys Gln Gln Gly Val Ser Phe Leu Val Gly Asp
305                 310                 315                 320

gtt gag tct agt caa ggt gct ttc cag tat ttt gga ctg aaa gaa gaa 1008
Val Glu Ser Ser Gln Gly Ala Phe Gln Tyr Phe Gly Leu Lys Glu Glu
                325                 330                 335

caa gta cct cta att atc att caa cac aat gac ggg aaa aag ttt ttt 1056
Gln Val Pro Leu Ile Ile Ile Gln His Asn Asp Gly Lys Lys Phe Phe
            340                 345                 350

aaa ccc aat ttg gaa gct gat cac att cca act tgg ttg aag gcg tac 1104
Lys Pro Asn Leu Glu Ala Asp His Ile Pro Thr Trp Leu Lys Ala Tyr
        355                 360                 365

aag gat ggt aat gtt gca cca ttt gtg aag tct gaa ccc att cct gaa 1152
Lys Asp Gly Asn Val Ala Pro Phe Val Lys Ser Glu Pro Ile Pro Glu
    370                 375                 380

gct aac gat gaa cct gtt aaa gtg gta gtt ggg aac agt ctt gag gac 1200
Ala Asn Asp Glu Pro Val Lys Val Val Val Gly Asn Ser Leu Glu Asp
385                 390                 395                 400

att gtt ttc aaa tct ggg aag aat gtt ctg ctg gag ttt tat gct ccc 1248
Ile Val Phe Lys Ser Gly Lys Asn Val Leu Leu Glu Phe Tyr Ala Pro
                405                 410                 415

tgg tgt ggt cat tgc aaa cag ttg gct cca ata ttg gac gaa gtt gct 1296
Trp Cys Gly His Cys Lys Gln Leu Ala Pro Ile Leu Asp Glu Val Ala
            420                 425                 430

atc tca tat caa agt gat gct gat gtt gtt att gca aaa ctg gat gca 1344
Ile Ser Tyr Gln Ser Asp Ala Asp Val Val Ile Ala Lys Leu Asp Ala
        435                 440                 445

act gcc aac gat atc cca agt gaa acc ttt gat gtc caa ggt tat cca 1392
Thr Ala Asn Asp Ile Pro Ser Glu Thr Phe Asp Val Gln Gly Tyr Pro
    450                 455                 460

acc gtg tac ttc agg tca gca agt gga aag tta tca caa tac gaa ggg 1440
Thr Val Tyr Phe Arg Ser Ala Ser Gly Lys Leu Ser Gln Tyr Glu Gly
465                 470                 475                 480

ggt agg acc aag gaa gac atc ata gaa ttc atc gaa aag aac cgg gac 1488
Gly Arg Thr Lys Glu Asp Ile Ile Glu Phe Ile Glu Lys Asn Arg Asp
                485                 490                 495

aaa cct gct cag caa gaa caa gga caa gat aaa cct gct cag caa gaa 1536
Lys Pro Ala Gln Gln Glu Gln Gly Gln Asp Lys Pro Ala Gln Gln Glu
            500                 505                 510

caa gga caa gat gag caa gaa aaa gga aaa gat gag ctt 1575
Gln Gly Gln Asp Glu Gln Glu Lys Gly Lys Asp Glu Leu
        515                 520                 525

<210> 242
<211> 525
<212> PRT
<213> Glycine max

<400>  242

Met Pro Lys Phe Phe His Ser Ile Phe Arg Gly Thr Met Ala Gly Arg
1               5                   10                  15

Val Ser Thr Cys Phe Phe Phe Val Phe Ala Leu Ser Leu Leu Leu Pro
            20                  25                  30

Phe Gln Ile Ser Ala Glu Glu Ser Ser Glu Lys Glu Phe Val Leu Thr
        35                  40                  45

Leu Asp His Ser Asn Phe His Asp Thr Val Ser Lys His Asp Phe Ile
    50                  55                  60

Val Val Glu Phe Tyr Ala Pro Trp Cys Gly His Cys Lys Lys Leu Ala
65                  70                  75                  80

Pro Glu Tyr Glu Lys Ala Ala Ser Ile Leu Ser Ser His Asp Pro Pro
                85                  90                  95

Val Val Leu Ala Lys Ile Asp Ala Asn Glu Glu Lys Asn Lys Asp Leu
            100                 105                 110

Ala Ser Gln Tyr Asp Val Arg Gly Tyr Pro Thr Ile Lys Ile Leu Arg
        115                 120                 125

Asn Gly Gly Lys Asn Val Gln Glu Tyr Lys Gly Pro Arg Glu Ala Asp
    130                 135                 140

Gly Ile Val Asp Tyr Leu Lys Lys Gln Ser Gly Pro Ala Ser Thr Glu
145                 150                 155                 160

Ile Lys Ser Ala Asp Glu Ala Thr Ala Phe Ile Gly Glu Asn Lys Val
                165                 170                 175

Ala Ile Val Gly Val Phe Pro Lys Phe Ser Gly Glu Glu Phe Asp Asn
            180                 185                 190

Phe Ser Ala Leu Ala Glu Lys Leu Arg Ser Asp Tyr Asp Phe Gly His
        195                 200                 205

Thr Leu Asn Ala Lys His Leu Pro Arg Gly Glu Ser Ser Val Ser Gly
    210                 215                 220

Pro Val Val Arg Leu Phe Lys Pro Phe Asp Glu Leu Phe Val Asp Phe
225                 230                 235                 240

Gln Asp Phe Asn Val Glu Ala Leu Glu Lys Phe Val Glu Glu Ser Ser

                245                 250                 255

Thr Pro Val Val Thr Val Phe Asn Asn Asp Pro Ser Asn His Pro Phe
            260                 265                 270

Val Ala Lys Phe Phe Asn Ser Pro Asn Ala Lys Ala Met Leu Phe Ile
        275                 280                 285

Asn Phe Thr Ala Glu Gly Ala Glu Ser Phe Lys Ser Lys Tyr Arg Glu
    290                 295                 300

Ala Ala Glu Gln His Lys Gln Gln Gly Val Ser Phe Leu Val Gly Asp
305                 310                 315                 320

Val Glu Ser Ser Gln Gly Ala Phe Gln Tyr Phe Gly Leu Lys Glu Glu
                325                 330                 335

Gln Val Pro Leu Ile Ile Ile Gln His Asn Asp Gly Lys Lys Phe Phe
            340                 345                 350

Lys Pro Asn Leu Glu Ala Asp His Ile Pro Thr Trp Leu Lys Ala Tyr
        355                 360                 365

Lys Asp Gly Asn Val Ala Pro Phe Val Lys Ser Glu Pro Ile Pro Glu
    370                 375                 380

Ala Asn Asp Glu Pro Val Lys Val Val Val Gly Asn Ser Leu Glu Asp
385                 390                 395                 400

Ile Val Phe Lys Ser Gly Lys Asn Val Leu Leu Glu Phe Tyr Ala Pro
                405                 410                 415

Trp Cys Gly His Cys Lys Gln Leu Ala Pro Ile Leu Asp Glu Val Ala
            420                 425                 430

Ile Ser Tyr Gln Ser Asp Ala Asp Val Val Ile Ala Lys Leu Asp Ala
        435                 440                 445

Thr Ala Asn Asp Ile Pro Ser Glu Thr Phe Asp Val Gln Gly Tyr Pro
    450                 455                 460

Thr Val Tyr Phe Arg Ser Ala Ser Gly Lys Leu Ser Gln Tyr Glu Gly
465                 470                 475                 480

Gly Arg Thr Lys Glu Asp Ile Ile Glu Phe Ile Glu Lys Asn Arg Asp
                485                 490                 495

Lys Pro Ala Gln Gln Glu Gln Gly Gln Asp Lys Pro Ala Gln Gln Glu
            500                 505                 510

Gln Gly Gln Asp Glu Gln Glu Lys Gly Lys Asp Glu Leu
        515                 520                 525

<210>  243
<211>  16
<212>  PRT
<213>  Glycine max

<400>  243

Ala Ala Ser Ile Leu Ser Ser His Asp Pro Pro Val Val Leu Ala Lys
1               5                   10                  15

<210>  244
<211>  9
<212>  PRT
<213>  Glycine max

<400>  244

Asp Gly Asn Val Ala Pro Phe Val Lys
1               5

<210>  245
<211>  15
<212>  PRT
<213>  Glycine max

<400>  245

Asp Leu Ala Ser Gln Tyr Asp Val Arg Gly Tyr Pro Thr Ile Lys
1               5                   10                  15

<210>  246
<211>  9
<212>  PRT
<213>  Glycine max

<400>  246

Glu Asp Ile Ile Glu Phe Ile Glu Lys
1               5

<210>  247
<211>  15
<212>  PRT
<213>  Glycine max

<400>  247

Glu Glu Gln Val Pro Leu Ile Ile Ile Gln His Asn Asp Gly Lys
1               5                   10                  15

<210>  248
<211>  17
<212>  PRT
<213>  Glycine max

<400>  248

Glu Phe Val Leu Thr Leu Asp His Ser Asn Phe His Asp Thr Val Ser
1               5                   10                  15

Lys

<210>  249
<211>  16
<212>  PRT
<213>  Glycine max

<400>  249

Phe Phe Lys Pro Asn Leu Glu Ala Asp His Ile Pro Thr Trp Leu Lys
1               5                   10                  15

<210>  250
<211>  8
<212>  PRT
<213>  Glycine max

<400>  250

Phe Phe Asn Ser Pro Asn Ala Lys
1               5

<210>  251
<211>  15
<212>  PRT
<213>  Glycine max

<400>  251

Phe Ser Gly Glu Glu Phe Asp Asn Phe Ser Ala Leu Ala Glu Lys
1               5                   10                  15

<210>  252
<211>  25
<212>  PRT
<213>  Glycine max

<400>  252

Phe Val Glu Glu Ser Ser Thr Pro Val Val Thr Val Phe Asn Asn Asp
1               5                   10                  15

Pro Ser Asn His Pro Phe Val Ala Lys
            20                  25

<210>  253
<211>  1509
<212>  DNA
<213>  Glycine max

<220>
<221>  CDS
<222>  (1)..(1509)

<400>  253
atg gcg ggt acg gtt tcg act tgt ttc ttc ttc gtg ttt ctt ctt tct      48
Met Ala Gly Thr Val Ser Thr Cys Phe Phe Phe Val Phe Leu Leu Ser
1               5                   10                  15

ctc ctt ctt cct ttt cag atc tcc gct gag gaa tcc tcg gag aag gag      96
Leu Leu Leu Pro Phe Gln Ile Ser Ala Glu Glu Ser Ser Glu Lys Glu
            20                  25                  30

ttc gta ctc aca ttg gat cac tct aac ttt cac gac act gtc agc aag      144
Phe Val Leu Thr Leu Asp His Ser Asn Phe His Asp Thr Val Ser Lys
        35                  40                  45

cac gat ttc atc gtc gtc gag ttc tac gcc cca tgg tgt ggt cac tgt      192
His Asp Phe Ile Val Val Glu Phe Tyr Ala Pro Trp Cys Gly His Cys
    50                  55                  60

aag aag ctt gct ccg gag tat gag aaa gct gct tct atc ttg agt agt      240
Lys Lys Leu Ala Pro Glu Tyr Glu Lys Ala Ala Ser Ile Leu Ser Ser
65                  70                  75                  80

cat gat cct cct att gtt ttg gct aaa gta gat gcc aat gag gag aag      288
His Asp Pro Pro Ile Val Leu Ala Lys Val Asp Ala Asn Glu Glu Lys
                85                  90                  95

aac aaa gac ctt gca tca caa tat gat gtt aag gga ttc cca aca att      336
Asn Lys Asp Leu Ala Ser Gln Tyr Asp Val Lys Gly Phe Pro Thr Ile
            100                 105                 110

aat att ctg aga aat ggg gga aag aat gtt cag gaa tac aaa ggt ccc      384
Asn Ile Leu Arg Asn Gly Gly Lys Asn Val Gln Glu Tyr Lys Gly Pro
        115                 120                 125

cgt gaa gca gat ggc atc gtt gac tat ttg aag aaa caa agt ggt cct      432
Arg Glu Ala Asp Gly Ile Val Asp Tyr Leu Lys Lys Gln Ser Gly Pro
    130                 135                 140

gct tca act gaa att aaa tct gct gat gaa gct act gct ttt att ggt      480
Ala Ser Thr Glu Ile Lys Ser Ala Asp Glu Ala Thr Ala Phe Ile Gly
145                 150                 155                 160

gaa aat aaa gtt gct att gtt gga gtt ttc ccg aaa ttt tct gga gag      528
Glu Asn Lys Val Ala Ile Val Gly Val Phe Pro Lys Phe Ser Gly Glu
                165                 170                 175

gag ttt gac aac ttt tct gcc tta gca gag aag ttg cgt tct gat tat      576
Glu Phe Asp Asn Phe Ser Ala Leu Ala Glu Lys Leu Arg Ser Asp Tyr
            180                 185                 190

gac ttt ggt cac act ctg aat gcc aaa ctc ctt cca aga gga gag tca      624
Asp Phe Gly His Thr Leu Asn Ala Lys Leu Leu Pro Arg Gly Glu Ser
        195                 200                 205

tca gtt tct ggg ccc gta gtt agg ctg ttc aag cca ttc gat gaa ctt 672
Ser Val Ser Gly Pro Val Val Arg Leu Phe Lys Pro Phe Asp Glu Leu
    210                 215                 220

ttt gtt gac ttc cag gat ttc aat gtg gaa gct cta gag aag ttt gtt 720
Phe Val Asp Phe Gln Asp Phe Asn Val Glu Ala Leu Glu Lys Phe Val
225                 230                 235                 240

gag gaa tct agt acc cct gtt gtt act gtc ttt aac aat gaa ccg agc 768
Glu Glu Ser Ser Thr Pro Val Val Thr Val Phe Asn Asn Glu Pro Ser
                245                 250                 255

aat cac cct ttc gtt gtc aaa ttc ttc aac agt ccc aac gca aag gca 816
Asn His Pro Phe Val Val Lys Phe Phe Asn Ser Pro Asn Ala Lys Ala
            260                 265                 270

atg ttg ttc atc aac ttc act gcc gaa ggt gct gaa gct atc aaa tca 864
Met Leu Phe Ile Asn Phe Thr Ala Glu Gly Ala Glu Ala Ile Lys Ser
        275                 280                 285

aaa tac cgt gaa gct gct gag caa tat aaa caa cag ggt gtc agc ttt 912
Lys Tyr Arg Glu Ala Ala Glu Gln Tyr Lys Gln Gln Gly Val Ser Phe
    290                 295                 300

ttg gtg gga gat gtt gag tct agt caa ggt gct ttc cag tat ttt gga 960
Leu Val Gly Asp Val Glu Ser Ser Gln Gly Ala Phe Gln Tyr Phe Gly
305                 310                 315                 320

ctg aaa gaa gag caa gta cct cta att atc att caa cat aat gat ggg 1008
Leu Lys Glu Glu Gln Val Pro Leu Ile Ile Ile Gln His Asn Asp Gly
                325                 330                 335

aaa aag ttt ttt aaa ccc aat ttg gaa gct gat cac att cca acc tgg 1056
Lys Lys Phe Phe Lys Pro Asn Leu Glu Ala Asp His Ile Pro Thr Trp
            340                 345                 350

ttg aag gca tac aag gat ggt cat gtt gca cca ttt gtt aag tct gaa 1104
Leu Lys Ala Tyr Lys Asp Gly His Val Ala Pro Phe Val Lys Ser Glu
        355                 360                 365

ccc att ccc gag act aac gat gaa cct gtt aaa gtg gta gtt ggg gcc 1152
Pro Ile Pro Glu Thr Asn Asp Glu Pro Val Lys Val Val Val Gly Ala
    370                 375                 380

agt ctt gag gac att gtt ttc aaa tct ggg aag aat gtt ctg ctg gag 1200
Ser Leu Glu Asp Ile Val Phe Lys Ser Gly Lys Asn Val Leu Leu Glu
385                 390                 395                 400

ttt tat gct ccc tgg tgt ggt cat tgc aaa cag ttg gct cca ata ttg 1248
Phe Tyr Ala Pro Trp Cys Gly His Cys Lys Gln Leu Ala Pro Ile Leu
                405                 410                 415

gat gaa gtt gct atc tca tat caa aat gaa gct gat gtt gtt att gca 1296
Asp Glu Val Ala Ile Ser Tyr Gln Asn Glu Ala Asp Val Val Ile Ala
            420                 425                 430

aaa ctg gat gca act gcc aac gat atc cca agt gaa acc ttt gat gtc 1344
Lys Leu Asp Ala Thr Ala Asn Asp Ile Pro Ser Glu Thr Phe Asp Val
        435                 440                 445

caa ggt tat cct acc gtg tac ttc agg tca gca agt gga aag tta tca 1392
Gln Gly Tyr Pro Thr Val Tyr Phe Arg Ser Ala Ser Gly Lys Leu Ser
    450                 455                 460

caa tac gac ggc ggt agg acc aag gaa gac atc ata gaa ttc atc gaa 1440
Gln Tyr Asp Gly Gly Arg Thr Lys Glu Asp Ile Ile Glu Phe Ile Glu
465                 470                 475                 480

aag aac cgg gat aaa cct gct cag caa gaa caa gga aaa gat gag caa 1488
Lys Asn Arg Asp Lys Pro Ala Gln Gln Glu Gln Gly Lys Asp Glu Gln
                485                 490                 495

gaa caa gga aaa gat gag ctt 1509
Glu Gln Gly Lys Asp Glu Leu
            500

<210> 254
<211> 503
<212> PRT
<213> Glycine max

<400> 254

Met Ala Gly Thr Val Ser Thr Cys Phe Phe Phe Val Phe Leu Leu Ser
1               5                   10                  15

Leu Leu Leu Pro Phe Gln Ile Ser Ala Glu Glu Ser Ser Glu Lys Glu
            20                  25                  30

Phe Val Leu Thr Leu Asp His Ser Asn Phe His Asp Thr Val Ser Lys
        35                  40                  45

His Asp Phe Ile Val Val Glu Phe Tyr Ala Pro Trp Cys Gly His Cys
    50                  55                  60

Lys Lys Leu Ala Pro Glu Tyr Glu Lys Ala Ala Ser Ile Leu Ser Ser
65                  70                  75                  80

His Asp Pro Pro Ile Val Leu Ala Lys Val Asp Ala Asn Glu Glu Lys
                85                  90                  95

Asn Lys Asp Leu Ala Ser Gln Tyr Asp Val Lys Gly Phe Pro Thr Ile
            100                 105                 110

Asn Ile Leu Arg Asn Gly Gly Lys Asn Val Gln Glu Tyr Lys Gly Pro
        115                 120                 125

Arg Glu Ala Asp Gly Ile Val Asp Tyr Leu Lys Lys Gln Ser Gly Pro
    130                 135                 140

Ala Ser Thr Glu Ile Lys Ser Ala Asp Glu Ala Thr Ala Phe Ile Gly
145                 150                 155                 160

Glu Asn Lys Val Ala Ile Val Gly Val Phe Pro Lys Phe Ser Gly Glu
                165                 170                 175

Glu Phe Asp Asn Phe Ser Ala Leu Ala Glu Lys Leu Arg Ser Asp Tyr
            180                 185                 190

Asp Phe Gly His Thr Leu Asn Ala Lys Leu Leu Pro Arg Gly Glu Ser
        195                 200                 205

Ser Val Ser Gly Pro Val Val Arg Leu Phe Lys Pro Phe Asp Glu Leu
    210                 215                 220

Phe Val Asp Phe Gln Asp Phe Asn Val Glu Ala Leu Glu Lys Phe Val
225                 230                 235                 240

Glu Glu Ser Ser Thr Pro Val Val Thr Val Phe Asn Asn Glu Pro Ser
                245                 250                 255

Asn His Pro Phe Val Val Lys Phe Phe Asn Ser Pro Asn Ala Lys Ala
            260                 265                 270

Met Leu Phe Ile Asn Phe Thr Ala Glu Gly Ala Glu Ala Ile Lys Ser
        275                 280                 285

Lys Tyr Arg Glu Ala Ala Glu Gln Tyr Lys Gln Gln Gly Val Ser Phe
    290                 295                 300

Leu Val Gly Asp Val Glu Ser Ser Gln Gly Ala Phe Gln Tyr Phe Gly
305                 310                 315                 320

Leu Lys Glu Glu Gln Val Pro Leu Ile Ile Ile Gln His Asn Asp Gly
                325                 330                 335

Lys Lys Phe Phe Lys Pro Asn Leu Glu Ala Asp His Ile Pro Thr Trp
            340                 345                 350

Leu Lys Ala Tyr Lys Asp Gly His Val Ala Pro Phe Val Lys Ser Glu
        355                 360                 365

Pro Ile Pro Glu Thr Asn Asp Glu Pro Val Lys Val Val Val Gly Ala
    370                 375                 380

Ser Leu Glu Asp Ile Val Phe Lys Ser Gly Lys Asn Val Leu Leu Glu
385                 390                 395                 400

Phe Tyr Ala Pro Trp Cys Gly His Cys Lys Gln Leu Ala Pro Ile Leu
                405                 410                 415

Asp Glu Val Ala Ile Ser Tyr Gln Asn Glu Ala Asp Val Val Ile Ala

420                         425                         430

Lys Leu Asp Ala Thr Ala Asn Asp Ile Pro Ser Glu Thr Phe Asp Val
        435                         440                         445

Gln Gly Tyr Pro Thr Val Tyr Phe Arg Ser Ala Ser Gly Lys Leu Ser
    450                         455                         460

Gln Tyr Asp Gly Gly Arg Thr Lys Glu Asp Ile Ile Glu Phe Ile Glu
465                         470                         475                         480

Lys Asn Arg Asp Lys Pro Ala Gln Gln Glu Gln Gly Lys Asp Glu Gln
                        485                         490                         495

Glu Gln Gly Lys Asp Glu Leu
            500

<210>  255
<211>  16
<212>  PRT
<213>  Glycine max

<400>  255

Ala Ala Ser Ile Leu Ser Ser His Asp Pro Pro Ile Val Leu Ala Lys
1               5                   10                  15

<210>  256
<211>  9
<212>  PRT
<213>  Glycine max

<400>  256

Asp Gly His Val Ala Pro Phe Val Lys
1               5

<210>  257
<211>  9
<212>  PRT
<213>  Glycine max

<400>  257

Asp Leu Ala Ser Gln Tyr Asp Val Lys
1               5

<210>  258
<211>  9
<212>  PRT
<213>  Glycine max

<400>  258

Glu Asp Ile Ile Glu Phe Ile Glu Lys
1               5

<210>  259
<211>  15
<212>  PRT
<213>  Glycine max

<400>  259

Glu Glu Gln Val Pro Leu Ile Ile Ile Gln His Asn Asp Gly Lys
1               5                   10                  15

<210>  260
<211>  17
<212>  PRT
<213>  Glycine max

<400>  260

Glu Phe Val Leu Thr Leu Asp His Ser Asn Phe His Asp Thr Val Ser
1               5                   10                  15

Lys

<210>  261
<211>  16
<212>  PRT
<213>  Glycine max

<400>  261

Phe Phe Lys Pro Asn Leu Glu Ala Asp His Ile Pro Thr Trp Leu Lys
1               5                   10                  15

<210>  262
<211>  8
<212>  PRT
<213>  Glycine max

<400>  262

Phe Phe Asn Ser Pro Asn Ala Lys
1               5

<210>  263
<211>  15
<212>  PRT
<213>  Glycine max

<400>  263

Phe Ser Gly Glu Glu Phe Asp Asn Phe Ser Ala Leu Ala Glu Lys
1               5                   10                  15

<210>  264
<211>  25
<212>  PRT
<213>  Glycine max

<400>  264

Phe Val Glu Glu Ser Ser Thr Pro Val Val Thr Val Phe Asn Asn Glu
1               5                   10                  15

Pro Ser Asn His Pro Phe Val Val Lys
            20                  25

<210>  265
<211>  13
<212>  PRT
<213>  Glycine max

<400>  265

Gly Phe Pro Thr Ile Asn Ile Leu Arg Asn Gly Gly Lys
1               5                   10

<210>  266
<211>  13
<212>  PRT
<213>  Glycine max

<400>  266

Gly Pro Arg Glu Ala Asp Gly Ile Val Asp Tyr Leu Lys
1               5                   10

<210>  267
<211>  7
<212>  PRT
<213>  Glycine max

<400>  267

Leu Ala Pro Glu Tyr Glu Lys
1               5

<210>  268
<211>  29
<212>  PRT
<213>  Glycine max

<400>  268

Leu Asp Ala Thr Ala Asn Asp Ile Pro Ser Glu Thr Phe Asp Val Gln
1               5                   10                  15

Gly Tyr Pro Thr Val Tyr Phe Arg Ser Ala Ser Gly Lys
            20                  25

<210>  269
<211>  14
<212>  PRT
<213>  Glycine max

<400>  269

Leu Arg Ser Asp Tyr Asp Phe Gly His Thr Leu Asn Ala Lys
1               5                   10


<210>  270
<211>  10
<212>  PRT
<213>  Glycine max

<400>  270

Leu Ser Gln Tyr Asp Gly Gly Arg Thr Lys
1               5                   10


<210>  271
<211>  23
<212>  PRT
<213>  Glycine max

<400>  271

Gln Leu Ala Pro Ile Leu Asp Glu Val Ala Ile Ser Tyr Gln Asn Glu
1               5                   10                  15


Ala Asp Val Val Ile Ala Lys
            20


<210>  272
<211>  24
<212>  PRT
<213>  Glycine max

<400>  272

Gln Gln Gly Val Ser Phe Leu Val Gly Asp Val Glu Ser Ser Gln Gly
1               5                   10                  15


Ala Phe Gln Tyr Phe Gly Leu Lys
            20


<210>  273
<211>  10
<212>  PRT
<213>  Glycine max

<400>  273

Gln Ser Gly Pro Ala Ser Thr Glu Ile Lys
1               5                   10

<210>  274
<211>  13
<212>  PRT
<213>  Glycine max

<400>  274

Ser Ala Asp Glu Ala Thr Ala Phe Ile Gly Glu Asn Lys
1               5                   10


<210>  275
<211>  13
<212>  PRT
<213>  Glycine max

<400>  275

Ser Glu Pro Ile Pro Glu Thr Asn Asp Glu Pro Val Lys
1               5                   10


<210>  276
<211>  9
<212>  PRT
<213>  Glycine max

<400>  276

Val Ala Ile Val Gly Val Phe Pro Lys
1               5


<210>  277
<211>  13
<212>  PRT
<213>  Glycine max

<400>  277

Val Val Val Gly Ala Ser Leu Glu Asp Ile Val Phe Lys
1               5                   10


<210>  278
<211>  9
<212>  PRT
<213>  Glycine max

<400>  278

Tyr Arg Glu Ala Ala Glu Gln Tyr Lys
1               5


<210>  279
<211>  1467
<212>  DNA
<213>  Glycine max


<220>
<221>  CDS

<222>  (1)..(1467)

<400>  279
atg ggt gtg gct caa aac att ccc gat gcg gag gag gga acc ttg gag      48
Met Gly Val Ala Gln Asn Ile Pro Asp Ala Glu Glu Gly Thr Leu Glu
1               5                   10                  15

att gga atg gaa tac aga act gtt tct gga gtt gct ggg cca ttg gtc      96
Ile Gly Met Glu Tyr Arg Thr Val Ser Gly Val Ala Gly Pro Leu Val
            20                  25                  30

att ctt gat aaa gtt aag gga ccc aag ttt caa gag att gtt aac att      144
Ile Leu Asp Lys Val Lys Gly Pro Lys Phe Gln Glu Ile Val Asn Ile
        35                  40                  45

cgc tta gga gat ggt act act cgg cgt gga caa gtg cta gaa gtt gat      192
Arg Leu Gly Asp Gly Thr Thr Arg Arg Gly Gln Val Leu Glu Val Asp
    50                  55                  60

ggt gaa aag gct gtt gtt cag gtc ttt gag ggt aca tct ggg att gac      240
Gly Glu Lys Ala Val Val Gln Val Phe Glu Gly Thr Ser Gly Ile Asp
65                  70                  75                  80

aat aaa ttt aca act gtg caa ttt act gga gag gtg tta aaa act cca      288
Asn Lys Phe Thr Thr Val Gln Phe Thr Gly Glu Val Leu Lys Thr Pro
                85                  90                  95

gta tca ttg gac atg ctt ggc cgc ata ttc aat ggt tct gga aaa cca      336
Val Ser Leu Asp Met Leu Gly Arg Ile Phe Asn Gly Ser Gly Lys Pro
            100                 105                 110

att gat aat ggt cca ccc att tta cct gag gct tac ttg gac ata tca      384
Ile Asp Asn Gly Pro Pro Ile Leu Pro Glu Ala Tyr Leu Asp Ile Ser
        115                 120                 125

gga agt tcc atc aac cct agt gag aga acc tat cca gag gag atg ata      432
Gly Ser Ser Ile Asn Pro Ser Glu Arg Thr Tyr Pro Glu Glu Met Ile
    130                 135                 140

cag aca gga ata tct acc att gat gtc atg aat tct att gct aga ggt      480
Gln Thr Gly Ile Ser Thr Ile Asp Val Met Asn Ser Ile Ala Arg Gly
145                 150                 155                 160

caa aag atc cct cta ttc tca gct gct ggt ctc ccc cat aat gaa att      528
Gln Lys Ile Pro Leu Phe Ser Ala Ala Gly Leu Pro His Asn Glu Ile
                165                 170                 175

gct gca cag ata tgc cgt cag gct ggc tta gtc aag cga cta gaa aag      576
Ala Ala Gln Ile Cys Arg Gln Ala Gly Leu Val Lys Arg Leu Glu Lys
            180                 185                 190

tct gat aat ctt ctc gag ggt gga gga gaa gag gac aat ttt gcc att      624
Ser Asp Asn Leu Leu Glu Gly Gly Gly Glu Glu Asp Asn Phe Ala Ile
        195                 200                 205

gtg ttt gca gct atg gga gtt aac atg gag act gca cag ttt ttc aaa      672
Val Phe Ala Ala Met Gly Val Asn Met Glu Thr Ala Gln Phe Phe Lys
    210                 215                 220

cgt gac ttt gag gaa aat ggc tcg atg gag aga gtg acc ctt ttc ctt      720
Arg Asp Phe Glu Glu Asn Gly Ser Met Glu Arg Val Thr Leu Phe Leu
225                 230                 235                 240

aat ctg gca aat gat cct acg att gag cgt atc atc act cct cgt att 768
Asn Leu Ala Asn Asp Pro Thr Ile Glu Arg Ile Ile Thr Pro Arg Ile
                245                 250                 255

gct ctt act act gct gaa tat ttg gca tat gaa tgt ggc aag cat gtt 816
Ala Leu Thr Thr Ala Glu Tyr Leu Ala Tyr Glu Cys Gly Lys His Val
            260                 265                 270

ctt gtg ata cta act gat atg agt tct tat gct gat gct ctt cgt gag 864
Leu Val Ile Leu Thr Asp Met Ser Ser Tyr Ala Asp Ala Leu Arg Glu
        275                 280                 285

gta tct gct gcc cga gaa gaa gta cct ggt aga cgt ggg tat ccc ggc 912
Val Ser Ala Ala Arg Glu Glu Val Pro Gly Arg Arg Gly Tyr Pro Gly
    290                 295                 300

tac atg tat aca gat ctt gca aca ata tac gaa cgt gct gga aga att 960
Tyr Met Tyr Thr Asp Leu Ala Thr Ile Tyr Glu Arg Ala Gly Arg Ile
305                 310                 315                 320

gag ggg cga aaa ggc tct att aca caa att cca att cta acc atg cct 1008
Glu Gly Arg Lys Gly Ser Ile Thr Gln Ile Pro Ile Leu Thr Met Pro
                325                 330                 335

aat gat gat att acg cat cca act cct gat ctt aca gga tat att act 1056
Asn Asp Asp Ile Thr His Pro Thr Pro Asp Leu Thr Gly Tyr Ile Thr
            340                 345                 350

gag gga cag ata tat atc gac aga cag ttg tat aac agg cag ata tat 1104
Glu Gly Gln Ile Tyr Ile Asp Arg Gln Leu Tyr Asn Arg Gln Ile Tyr
        355                 360                 365

cct cca atc aat gtc ctt cca tca ttg tct cgt ttg atg aag agt gcc 1152
Pro Pro Ile Asn Val Leu Pro Ser Leu Ser Arg Leu Met Lys Ser Ala
    370                 375                 380

att ggt gag gga atg aca cga aag gat cat tct gat gtg tcc aac cag 1200
Ile Gly Glu Gly Met Thr Arg Lys Asp His Ser Asp Val Ser Asn Gln
385                 390                 395                 400

ctc tat gca aat tat gcc att gga aag gat gtc cag gca atg aaa gcc 1248
Leu Tyr Ala Asn Tyr Ala Ile Gly Lys Asp Val Gln Ala Met Lys Ala
                405                 410                 415

gtg gtc gga gaa gaa gca ctt tca tct gag gac ctg ctg tat ttg gaa 1296
Val Val Gly Glu Glu Ala Leu Ser Ser Glu Asp Leu Leu Tyr Leu Glu
            420                 425                 430

ttt ttg gag aag ttt gag agg aag ttt gtt gcc caa gga gca tac gat 1344
Phe Leu Glu Lys Phe Glu Arg Lys Phe Val Ala Gln Gly Ala Tyr Asp
        435                 440                 445

aca cgc aat atc ttc cag tca cta gat ctt gca tgg act ttg ctt cgg 1392
Thr Arg Asn Ile Phe Gln Ser Leu Asp Leu Ala Trp Thr Leu Leu Arg
    450                 455                 460

att ttc cct cgc gag ctt ctc cac cgt att cct gca aag act ctt gat 1440
Ile Phe Pro Arg Glu Leu Leu His Arg Ile Pro Ala Lys Thr Leu Asp
465                 470                 475                 480

cag ttc tac agc cga gat gcc agt aac 1467
Gln Phe Tyr Ser Arg Asp Ala Ser Asn
                485

<210>  280
<211>  489
<212>  PRT
<213>  Glycine max

<400>  280

Met Gly Val Ala Gln Asn Ile Pro Asp Ala Glu Glu Gly Thr Leu Glu
1               5                   10                  15

Ile Gly Met Glu Tyr Arg Thr Val Ser Gly Val Ala Gly Pro Leu Val
            20                  25                  30

Ile Leu Asp Lys Val Lys Gly Pro Lys Phe Gln Glu Ile Val Asn Ile
        35                  40                  45

Arg Leu Gly Asp Gly Thr Thr Arg Arg Gly Gln Val Leu Glu Val Asp
    50                  55                  60

Gly Glu Lys Ala Val Val Gln Val Phe Glu Gly Thr Ser Gly Ile Asp
65                  70                  75                  80

Asn Lys Phe Thr Thr Val Gln Phe Thr Gly Glu Val Leu Lys Thr Pro
                85                  90                  95

Val Ser Leu Asp Met Leu Gly Arg Ile Phe Asn Gly Ser Gly Lys Pro
            100                 105                 110

Ile Asp Asn Gly Pro Pro Ile Leu Pro Glu Ala Tyr Leu Asp Ile Ser
        115                 120                 125

Gly Ser Ser Ile Asn Pro Ser Glu Arg Thr Tyr Pro Glu Glu Met Ile
    130                 135                 140

Gln Thr Gly Ile Ser Thr Ile Asp Val Met Asn Ser Ile Ala Arg Gly
145                 150                 155                 160

Gln Lys Ile Pro Leu Phe Ser Ala Ala Gly Leu Pro His Asn Glu Ile
                165                 170                 175

Ala Ala Gln Ile Cys Arg Gln Ala Gly Leu Val Lys Arg Leu Glu Lys
            180                 185                 190

Ser Asp Asn Leu Leu Glu Gly Gly Gly Glu Glu Asp Asn Phe Ala Ile
        195                 200                 205

Val Phe Ala Ala Met Gly Val Asn Met Glu Thr Ala Gln Phe Phe Lys
    210                 215                 220

Arg Asp Phe Glu Glu Asn Gly Ser Met Glu Arg Val Thr Leu Phe Leu
225                 230                 235                 240

Asn Leu Ala Asn Asp Pro Thr Ile Glu Arg Ile Ile Thr Pro Arg Ile
                245                 250                 255

Ala Leu Thr Thr Ala Glu Tyr Leu Ala Tyr Glu Cys Gly Lys His Val
            260                 265                 270

Leu Val Ile Leu Thr Asp Met Ser Ser Tyr Ala Asp Ala Leu Arg Glu
        275                 280                 285

Val Ser Ala Ala Arg Glu Glu Val Pro Gly Arg Arg Gly Tyr Pro Gly
    290                 295                 300

Tyr Met Tyr Thr Asp Leu Ala Thr Ile Tyr Glu Arg Ala Gly Arg Ile
305                 310                 315                 320

Glu Gly Arg Lys Gly Ser Ile Thr Gln Ile Pro Ile Leu Thr Met Pro
                325                 330                 335

Asn Asp Asp Ile Thr His Pro Thr Pro Asp Leu Thr Gly Tyr Ile Thr
            340                 345                 350

Glu Gly Gln Ile Tyr Ile Asp Arg Gln Leu Tyr Asn Arg Gln Ile Tyr
        355                 360                 365

Pro Pro Ile Asn Val Leu Pro Ser Leu Ser Arg Leu Met Lys Ser Ala
    370                 375                 380

Ile Gly Glu Gly Met Thr Arg Lys Asp His Ser Asp Val Ser Asn Gln
385                 390                 395                 400

Leu Tyr Ala Asn Tyr Ala Ile Gly Lys Asp Val Gln Ala Met Lys Ala
                405                 410                 415

Val Val Gly Glu Glu Ala Leu Ser Ser Glu Asp Leu Leu Tyr Leu Glu
            420                 425                 430

Phe Leu Glu Lys Phe Glu Arg Lys Phe Val Ala Gln Gly Ala Tyr Asp
        435                 440                 445

Thr Arg Asn Ile Phe Gln Ser Leu Asp Leu Ala Trp Thr Leu Leu Arg
    450                 455                 460

Ile Phe Pro Arg Glu Leu Leu His Arg Ile Pro Ala Lys Thr Leu Asp

465                 470                 475                 480

Gln Phe Tyr Ser Arg Asp Ala Ser Asn
                485

<210>  281
<211>  17
<212>  PRT
<213>  Glycine max

<400>  281

Asp His Ser Asp Val Ser Asn Gln Leu Tyr Ala Asn Tyr Ala Ile Gly
1               5                   10                  15

Lys

<210>  282
<211>  26
<212>  PRT
<213>  Glycine max

<400>  282

Phe Gln Glu Ile Val Asn Ile Arg Leu Gly Asp Gly Thr Thr Arg Arg
1               5                   10                  15

Gly Gln Val Leu Glu Val Asp Gly Glu Lys
            20                  25

<210>  283
<211>  12
<212>  PRT
<213>  Glycine max

<400>  283

Phe Thr Thr Val Gln Phe Thr Gly Glu Val Leu Lys
1               5                   10

<210>  284
<211>  12
<212>  PRT
<213>  Glycine max

<400>  284

Thr Leu Asp Gln Phe Tyr Ser Arg Asp Ala Ser Asn
1               5                   10

<210>  285
<211>  1464
<212>  DNA
<213>  Glycine max

<220>
<221>  CDS
<222>  (1)..(1464)

<400>  285
atg ggt gtg gct gag aac att ccc gat atg gag gag ggt acc ttg gag      48
Met Gly Val Ala Glu Asn Ile Pro Asp Met Glu Glu Gly Thr Leu Glu
1               5                   10                  15

att gga atg gaa tac aga act gta tct gga gtt gct gga cca ttg gtc      96
Ile Gly Met Glu Tyr Arg Thr Val Ser Gly Val Ala Gly Pro Leu Val
            20                  25                  30

att ctt gac aaa gtt aag gga ccc aaa ttt caa gag att gtt aat att      144
Ile Leu Asp Lys Val Lys Gly Pro Lys Phe Gln Glu Ile Val Asn Ile
        35                  40                  45

cgt tta gga gat gga act act cgg cgt gga caa gtt cta gag gtt gat      192
Arg Leu Gly Asp Gly Thr Thr Arg Arg Gly Gln Val Leu Glu Val Asp
    50                  55                  60

ggc gaa aag gct gtt gtt cag gta ttt gaa ggc aca tct ggg atc gac      240
Gly Glu Lys Ala Val Val Gln Val Phe Glu Gly Thr Ser Gly Ile Asp
65                  70                  75                  80

aat aag ttt aca act gtg caa ttt act gga gag gtg tta aaa act cct      288
Asn Lys Phe Thr Thr Val Gln Phe Thr Gly Glu Val Leu Lys Thr Pro
                85                  90                  95

gtc tca ctg gac atg ctt gga cgc ata ttt aat ggt tct gga aaa cca      336
Val Ser Leu Asp Met Leu Gly Arg Ile Phe Asn Gly Ser Gly Lys Pro
            100                 105                 110

att gat aat ggt cca cca att tta cct gag gct tac ctg gat att tct      384
Ile Asp Asn Gly Pro Pro Ile Leu Pro Glu Ala Tyr Leu Asp Ile Ser
        115                 120                 125

gga agt tcc atc aat cct agt gag aga acc tat cct gag gag atg ata      432
Gly Ser Ser Ile Asn Pro Ser Glu Arg Thr Tyr Pro Glu Glu Met Ile
    130                 135                 140

cag aca gga ata tct aca att gat gtc atg aat tct att gct aga ggt      480
Gln Thr Gly Ile Ser Thr Ile Asp Val Met Asn Ser Ile Ala Arg Gly
145                 150                 155                 160

caa aag atc cct cta ttc tca gca gct ggt ctc ccc cat aat gaa att      528
Gln Lys Ile Pro Leu Phe Ser Ala Ala Gly Leu Pro His Asn Glu Ile
                165                 170                 175

gct gca cag atc tgt cgt cag gct ggc tta gtc aag cga cta gag aaa      576
Ala Ala Gln Ile Cys Arg Gln Ala Gly Leu Val Lys Arg Leu Glu Lys
            180                 185                 190

tct gat aat ctt ctt gag ggt gga gaa gag gac aat ttt gct att gtg      624
Ser Asp Asn Leu Leu Glu Gly Gly Glu Glu Asp Asn Phe Ala Ile Val
        195                 200                 205

ttt gca gct atg gga gtt aac atg gag act gca cag ttt ttc aag cgt      672
Phe Ala Ala Met Gly Val Asn Met Glu Thr Ala Gln Phe Phe Lys Arg
    210                 215                 220

gac ttt gag gag aat gga tca atg gag aga gtg act ctt ttc ctt aat 720
Asp Phe Glu Glu Asn Gly Ser Met Glu Arg Val Thr Leu Phe Leu Asn
225                 230                 235                 240

ttg gca aat gat cct aca att gag cgt att atc act cct cgt att gct 768
Leu Ala Asn Asp Pro Thr Ile Glu Arg Ile Ile Thr Pro Arg Ile Ala
                245                 250                 255

ctt act act gct gaa tat ttg gct tat gaa tgt ggc aag cac gtt ctt 816
Leu Thr Thr Ala Glu Tyr Leu Ala Tyr Glu Cys Gly Lys His Val Leu
            260                 265                 270

gtg ata ctc aca gac atg agt tct tat gct gat gct ctt cgt gag gta 864
Val Ile Leu Thr Asp Met Ser Ser Tyr Ala Asp Ala Leu Arg Glu Val
        275                 280                 285

tct gct gcc cga gaa gaa gtg ccg ggt aga cgt ggt tat cct gga tat 912
Ser Ala Ala Arg Glu Glu Val Pro Gly Arg Arg Gly Tyr Pro Gly Tyr
    290                 295                 300

atg tat aca gat ctt gca aca att tat gaa cga gct ggg aga att gag 960
Met Tyr Thr Asp Leu Ala Thr Ile Tyr Glu Arg Ala Gly Arg Ile Glu
305                 310                 315                 320

ggg cgt aaa ggc tct att aca caa att cca att cta acc atg ccc aat 1008
Gly Arg Lys Gly Ser Ile Thr Gln Ile Pro Ile Leu Thr Met Pro Asn
                325                 330                 335

gat gat att acg cac cca act cct gat cta aca gga tat atc act gag 1056
Asp Asp Ile Thr His Pro Thr Pro Asp Leu Thr Gly Tyr Ile Thr Glu
            340                 345                 350

ggg cag ata tac atc gac agg cag ctg tat aat aga cag ata tac cca 1104
Gly Gln Ile Tyr Ile Asp Arg Gln Leu Tyr Asn Arg Gln Ile Tyr Pro
        355                 360                 365

cca atc aac gtc ctc cca tca cta tct cgg ttg atg aag agt gct att 1152
Pro Ile Asn Val Leu Pro Ser Leu Ser Arg Leu Met Lys Ser Ala Ile
    370                 375                 380

ggt gag gga atg act aga agg gat cat tct gat gtg tcc aac cag ctc 1200
Gly Glu Gly Met Thr Arg Arg Asp His Ser Asp Val Ser Asn Gln Leu
385                 390                 395                 400

tat gca aat tat gct att gga aag gat gtc cag gca atg aaa gca gtg 1248
Tyr Ala Asn Tyr Ala Ile Gly Lys Asp Val Gln Ala Met Lys Ala Val
                405                 410                 415

gtt gga gaa gaa gca ctt tca tct gag gac ctg ctg tat ctg gaa ttt 1296
Val Gly Glu Glu Ala Leu Ser Ser Glu Asp Leu Leu Tyr Leu Glu Phe
            420                 425                 430

ttg gag aag ttt gag agg aag ttt gtt gcc cag gga gcc tat gac acc 1344
Leu Glu Lys Phe Glu Arg Lys Phe Val Ala Gln Gly Ala Tyr Asp Thr
        435                 440                 445

cgc aat ata ttc cag tca ctt gat ctt gca tgg act tta ctc cgg att 1392
Arg Asn Ile Phe Gln Ser Leu Asp Leu Ala Trp Thr Leu Leu Arg Ile
    450                 455                 460

ttc cct cgt gaa ctt ctt cat cgt ata cca gcc aag act ctt gac cag 1440
Phe Pro Arg Glu Leu Leu His Arg Ile Pro Ala Lys Thr Leu Asp Gln
465                 470                 475                 480

ttt tac agc aga gat gct ggt aat                                    1464
Phe Tyr Ser Arg Asp Ala Gly Asn
                485

<210>  286
<211>  488
<212>  PRT
<213>  Glycine max

<400>  286

Met Gly Val Ala Glu Asn Ile Pro Asp Met Glu Glu Gly Thr Leu Glu
1               5                   10                  15

Ile Gly Met Glu Tyr Arg Thr Val Ser Gly Val Ala Gly Pro Leu Val
            20                  25                  30

Ile Leu Asp Lys Val Lys Gly Pro Lys Phe Gln Glu Ile Val Asn Ile
        35                  40                  45

Arg Leu Gly Asp Gly Thr Thr Arg Arg Gly Gln Val Leu Glu Val Asp
    50                  55                  60

Gly Glu Lys Ala Val Val Gln Val Phe Glu Gly Thr Ser Gly Ile Asp
65                  70                  75                  80

Asn Lys Phe Thr Thr Val Gln Phe Thr Gly Glu Val Leu Lys Thr Pro
                85                  90                  95

Val Ser Leu Asp Met Leu Gly Arg Ile Phe Asn Gly Ser Gly Lys Pro
            100                 105                 110

Ile Asp Asn Gly Pro Pro Ile Leu Pro Glu Ala Tyr Leu Asp Ile Ser
        115                 120                 125

Gly Ser Ser Ile Asn Pro Ser Glu Arg Thr Tyr Pro Glu Glu Met Ile
    130                 135                 140

Gln Thr Gly Ile Ser Thr Ile Asp Val Met Asn Ser Ile Ala Arg Gly
145                 150                 155                 160

Gln Lys Ile Pro Leu Phe Ser Ala Ala Gly Leu Pro His Asn Glu Ile
                165                 170                 175

Ala Ala Gln Ile Cys Arg Gln Ala Gly Leu Val Lys Arg Leu Glu Lys
            180                 185                 190

Ser Asp Asn Leu Leu Glu Gly Gly Glu Glu Asp Asn Phe Ala Ile Val
        195                 200                 205

Phe Ala Ala Met Gly Val Asn Met Glu Thr Ala Gln Phe Phe Lys Arg
    210                 215                 220

Asp Phe Glu Glu Asn Gly Ser Met Glu Arg Val Thr Leu Phe Leu Asn
225                 230                 235                 240

Leu Ala Asn Asp Pro Thr Ile Glu Arg Ile Ile Thr Pro Arg Ile Ala
                245                 250                 255

Leu Thr Thr Ala Glu Tyr Leu Ala Tyr Glu Cys Gly Lys His Val Leu
            260                 265                 270

Val Ile Leu Thr Asp Met Ser Ser Tyr Ala Asp Ala Leu Arg Glu Val
        275                 280                 285

Ser Ala Ala Arg Glu Glu Val Pro Gly Arg Arg Gly Tyr Pro Gly Tyr
    290                 295                 300

Met Tyr Thr Asp Leu Ala Thr Ile Tyr Glu Arg Ala Gly Arg Ile Glu
305                 310                 315                 320

Gly Arg Lys Gly Ser Ile Thr Gln Ile Pro Ile Leu Thr Met Pro Asn
                325                 330                 335

Asp Asp Ile Thr His Pro Thr Pro Asp Leu Thr Gly Tyr Ile Thr Glu
            340                 345                 350

Gly Gln Ile Tyr Ile Asp Arg Gln Leu Tyr Asn Arg Gln Ile Tyr Pro
        355                 360                 365

Pro Ile Asn Val Leu Pro Ser Leu Ser Arg Leu Met Lys Ser Ala Ile
    370                 375                 380

Gly Glu Gly Met Thr Arg Arg Asp His Ser Asp Val Ser Asn Gln Leu
385                 390                 395                 400

Tyr Ala Asn Tyr Ala Ile Gly Lys Asp Val Gln Ala Met Lys Ala Val
                405                 410                 415

Val Gly Glu Glu Ala Leu Ser Ser Glu Asp Leu Leu Tyr Leu Glu Phe
            420                 425                 430

Leu Glu Lys Phe Glu Arg Lys Phe Val Ala Gln Gly Ala Tyr Asp Thr
        435                 440                 445

Arg Asn Ile Phe Gln Ser Leu Asp Leu Ala Trp Thr Leu Leu Arg Ile

450                 455                 460

Phe Pro Arg Glu Leu Leu His Arg Ile Pro Ala Lys Thr Leu Asp Gln
465                 470                 475                 480

Phe Tyr Ser Arg Asp Ala Gly Asn
                485

<210>  287
<211>  26
<212>  PRT
<213>  Glycine max

<400>  287

Phe Gln Glu Ile Val Asn Ile Arg Leu Gly Asp Gly Thr Thr Arg Arg
1               5                   10                  15

Gly Gln Val Leu Glu Val Asp Gly Glu Lys
            20                  25

<210>  288
<211>  12
<212>  PRT
<213>  Glycine max

<400>  288

Phe Thr Thr Val Gln Phe Thr Gly Glu Val Leu Lys
1               5                   10

<210>  289
<211>  12
<212>  PRT
<213>  Glycine max

<400>  289

Thr Leu Asp Gln Phe Tyr Ser Arg Asp Ala Gly Asn
1               5                   10

<210>  290
<211>  1407
<212>  DNA
<213>  Glycine max

<220>
<221>  CDS
<222>  (1)..(1407)

<400>  290
atg gcc acc gct gcc gag aaa ctc tcc gct ctc aaa tcc gcc gtc gcc       48
Met Ala Thr Ala Ala Glu Lys Leu Ser Ala Leu Lys Ser Ala Val Ala
1               5                   10                  15

gga ttg aac gaa atc agt gag aat gag aag aac gga ttc atc agc ctc          96
Gly Leu Asn Glu Ile Ser Glu Asn Glu Lys Asn Gly Phe Ile Ser Leu
            20                  25                  30

gtc ggc cgc tat ctc agt ggc gaa gcg cag cat gtg gaa tgg agc aag         144
Val Gly Arg Tyr Leu Ser Gly Glu Ala Gln His Val Glu Trp Ser Lys
        35                  40                  45

atc cag acg cct acg gac gaa gtg gtt gtg cct tac gac act ttg gcg         192
Ile Gln Thr Pro Thr Asp Glu Val Val Val Pro Tyr Asp Thr Leu Ala
    50                  55                  60

cca act cct gaa ggt tct tcg gag gtg aag aat cta ttg gac aag ctt         240
Pro Thr Pro Glu Gly Ser Ser Glu Val Lys Asn Leu Leu Asp Lys Leu
65                  70                  75                  80

gtg gtg ttg aag cta aat gga ggc ttg gga aca act atg ggt tgc act         288
Val Val Leu Lys Leu Asn Gly Gly Leu Gly Thr Thr Met Gly Cys Thr
                85                  90                  95

ggt cct aaa tct gta att gaa gtt cgt gat ggg ttg aca ttt cta gat         336
Gly Pro Lys Ser Val Ile Glu Val Arg Asp Gly Leu Thr Phe Leu Asp
            100                 105                 110

tta att gtc atc caa att gag aat ctc aat tcc aaa tat gga agc aat         384
Leu Ile Val Ile Gln Ile Glu Asn Leu Asn Ser Lys Tyr Gly Ser Asn
        115                 120                 125

gtt cct ttg ctt ttg atg aat tca ttc aac act cat gat gac act caa         432
Val Pro Leu Leu Leu Met Asn Ser Phe Asn Thr His Asp Asp Thr Gln
    130                 135                 140

aag att gtt gaa aaa tac caa aac tca aat att gag att cat act ttt         480
Lys Ile Val Glu Lys Tyr Gln Asn Ser Asn Ile Glu Ile His Thr Phe
145                 150                 155                 160

aac cag agc cag tat cct cga ttg gtt gtt gag gac ttt ttg cca ttg         528
Asn Gln Ser Gln Tyr Pro Arg Leu Val Val Glu Asp Phe Leu Pro Leu
                165                 170                 175

cct tcc aaa ggg cat act gac aag gat gga tgg tac cct cct ggc cat         576
Pro Ser Lys Gly His Thr Asp Lys Asp Gly Trp Tyr Pro Pro Gly His
            180                 185                 190

ggt gat gtc ttc cca tca tta ttg aac agt ggc aaa ctt gat gca cta         624
Gly Asp Val Phe Pro Ser Leu Leu Asn Ser Gly Lys Leu Asp Ala Leu
        195                 200                 205

ttg tca cag ggt aaa gag tat gtg ttt gtt gcc aat tcg gat aac ttg         672
Leu Ser Gln Gly Lys Glu Tyr Val Phe Val Ala Asn Ser Asp Asn Leu
    210                 215                 220

gga gct ata gtt gac ttg aaa atc ttg aat cat ttg atc cag aac aag         720
Gly Ala Ile Val Asp Leu Lys Ile Leu Asn His Leu Ile Gln Asn Lys
225                 230                 235                 240

aat gaa tac tgt atg gag gtg act ccc aaa aca ttg gct gat gta aag         768
Asn Glu Tyr Cys Met Glu Val Thr Pro Lys Thr Leu Ala Asp Val Lys
                245                 250                 255

ggt ggc act ttg att tct tac gaa gga agg gtt cag ctc ctg gaa att         816
Gly Gly Thr Leu Ile Ser Tyr Glu Gly Arg Val Gln Leu Leu Glu Ile
            260                 265                 270

gca caa gtc ccc gat gaa cat gtc aat gag ttc aag tca ata gag aag 864
Ala Gln Val Pro Asp Glu His Val Asn Glu Phe Lys Ser Ile Glu Lys
        275                 280                 285

ttc aaa att ttc aac aca aat aat ttg tgg gtg aac tta aac gca gtt 912
Phe Lys Ile Phe Asn Thr Asn Asn Leu Trp Val Asn Leu Asn Ala Val
    290                 295                 300

aaa agg ctt gtt gaa gct gat gct ctt aag atg gaa att att ccc aat 960
Lys Arg Leu Val Glu Ala Asp Ala Leu Lys Met Glu Ile Ile Pro Asn
305                 310                 315                 320

cca aag gaa gtt gac gga ata aaa gtt ctt cag ctg gaa act gca gct 1008
Pro Lys Glu Val Asp Gly Ile Lys Val Leu Gln Leu Glu Thr Ala Ala
                325                 330                 335

ggt gct gca ata agg ttc ttt gac aag gct att ggg att aat gtt cct 1056
Gly Ala Ala Ile Arg Phe Phe Asp Lys Ala Ile Gly Ile Asn Val Pro
            340                 345                 350

cga tca cga ttc ctt cct gtg aag gca act tca gat ttg ctt ctt gtc 1104
Arg Ser Arg Phe Leu Pro Val Lys Ala Thr Ser Asp Leu Leu Leu Val
        355                 360                 365

cag tct gac ctc tac act ttg gaa gac gga ttt gtc att cgg aac aaa 1152
Gln Ser Asp Leu Tyr Thr Leu Glu Asp Gly Phe Val Ile Arg Asn Lys
    370                 375                 380

gct agg gaa aat cct gaa aac cct tct att gaa ctg gga cca gaa ttt 1200
Ala Arg Glu Asn Pro Glu Asn Pro Ser Ile Glu Leu Gly Pro Glu Phe
385                 390                 395                 400

aag aag gtt agc aac ttc ttg ggc cgc ttc aag tca att cct agt atc 1248
Lys Lys Val Ser Asn Phe Leu Gly Arg Phe Lys Ser Ile Pro Ser Ile
                405                 410                 415

gtt gag ctt gac agt cta aaa gtg gct ggt gat gta tgg ttt gga gct 1296
Val Glu Leu Asp Ser Leu Lys Val Ala Gly Asp Val Trp Phe Gly Ala
            420                 425                 430

ggt gtt atc ctc aag ggg aaa gtc agc att gta tca aag ccc ggt gta 1344
Gly Val Ile Leu Lys Gly Lys Val Ser Ile Val Ser Lys Pro Gly Val
        435                 440                 445

aag ctg gaa gtt ccc gac ggt gtt gcc att gta gac aag gaa att aat 1392
Lys Leu Glu Val Pro Asp Gly Val Ala Ile Val Asp Lys Glu Ile Asn
    450                 455                 460

ggc cca gag gac ctg 1407
Gly Pro Glu Asp Leu
465

<210> 291
<211> 469
<212> PRT
<213> Glycine max

<400> 291

Met Ala Thr Ala Ala Glu Lys Leu Ser Ala Leu Lys Ser Ala Val Ala
1               5                   10                  15

Gly Leu Asn Glu Ile Ser Glu Asn Glu Lys Asn Gly Phe Ile Ser Leu
            20                  25                  30

Val Gly Arg Tyr Leu Ser Gly Glu Ala Gln His Val Glu Trp Ser Lys
        35                  40                  45

Ile Gln Thr Pro Thr Asp Glu Val Val Val Pro Tyr Asp Thr Leu Ala
    50                  55                  60

Pro Thr Pro Glu Gly Ser Ser Glu Val Lys Asn Leu Leu Asp Lys Leu
65                  70                  75                  80

Val Val Leu Lys Leu Asn Gly Gly Leu Gly Thr Thr Met Gly Cys Thr
                85                  90                  95

Gly Pro Lys Ser Val Ile Glu Val Arg Asp Gly Leu Thr Phe Leu Asp
            100                 105                 110

Leu Ile Val Ile Gln Ile Glu Asn Leu Asn Ser Lys Tyr Gly Ser Asn
        115                 120                 125

Val Pro Leu Leu Leu Met Asn Ser Phe Asn Thr His Asp Asp Thr Gln
    130                 135                 140

Lys Ile Val Glu Lys Tyr Gln Asn Ser Asn Ile Glu Ile His Thr Phe
145                 150                 155                 160

Asn Gln Ser Gln Tyr Pro Arg Leu Val Val Glu Asp Phe Leu Pro Leu
                165                 170                 175

Pro Ser Lys Gly His Thr Asp Lys Asp Gly Trp Tyr Pro Pro Gly His
            180                 185                 190

Gly Asp Val Phe Pro Ser Leu Leu Asn Ser Gly Lys Leu Asp Ala Leu
        195                 200                 205

Leu Ser Gln Gly Lys Glu Tyr Val Phe Val Ala Asn Ser Asp Asn Leu
    210                 215                 220

Gly Ala Ile Val Asp Leu Lys Ile Leu Asn His Leu Ile Gln Asn Lys
225                 230                 235                 240

Asn Glu Tyr Cys Met Glu Val Thr Pro Lys Thr Leu Ala Asp Val Lys
                245                 250                 255

Gly Gly Thr Leu Ile Ser Tyr Glu Gly Arg Val Gln Leu Leu Glu Ile

260                 265                 270

Ala Gln Val Pro Asp Glu His Val Asn Glu Phe Lys Ser Ile Glu Lys
        275                 280                 285

Phe Lys Ile Phe Asn Thr Asn Asn Leu Trp Val Asn Leu Asn Ala Val
    290                 295                 300

Lys Arg Leu Val Glu Ala Asp Ala Leu Lys Met Glu Ile Ile Pro Asn
305                 310                 315                 320

Pro Lys Glu Val Asp Gly Ile Lys Val Leu Gln Leu Glu Thr Ala Ala
                325                 330                 335

Gly Ala Ala Ile Arg Phe Phe Asp Lys Ala Ile Gly Ile Asn Val Pro
            340                 345                 350

Arg Ser Arg Phe Leu Pro Val Lys Ala Thr Ser Asp Leu Leu Leu Val
        355                 360                 365

Gln Ser Asp Leu Tyr Thr Leu Glu Asp Gly Phe Val Ile Arg Asn Lys
    370                 375                 380

Ala Arg Glu Asn Pro Glu Asn Pro Ser Ile Glu Leu Gly Pro Glu Phe
385                 390                 395                 400

Lys Lys Val Ser Asn Phe Leu Gly Arg Phe Lys Ser Ile Pro Ser Ile
                405                 410                 415

Val Glu Leu Asp Ser Leu Lys Val Ala Gly Asp Val Trp Phe Gly Ala
            420                 425                 430

Gly Val Ile Leu Lys Gly Lys Val Ser Ile Val Ser Lys Pro Gly Val
        435                 440                 445

Lys Leu Glu Val Pro Asp Gly Val Ala Ile Val Asp Lys Glu Ile Asn
    450                 455                 460

Gly Pro Glu Asp Leu
465

<210>  292
<211>  15
<212>  PRT
<213>  Glycine max

<400>  292

Ala Ile Gly Ile Asn Val Pro Arg Ser Arg Phe Leu Pro Val Lys

1               5                   10                  15


<210>  293
<211>  17
<212>  PRT
<213>  Glycine max

<400>  293

Ala Arg Glu Asn Pro Glu Asn Pro Ser Ile Glu Leu Gly Pro Glu Phe
1               5                   10                  15


Lys




<210>  294
<211>  24
<212>  PRT
<213>  Glycine max

<400>  294

Ala Thr Ser Asp Leu Leu Leu Val Gln Ser Asp Leu Tyr Thr Leu Glu
1               5                   10                  15


Asp Gly Phe Val Ile Arg Asn Lys
            20


<210>  295
<211>  8
<212>  PRT
<213>  Glycine max

<400>  295

Glu Ile Asn Gly Pro Glu Asp Leu
1               5


<210>  296
<211>  18
<212>  PRT
<213>  Glycine max

<400>  296

Glu Tyr Val Phe Val Ala Asn Ser Asp Asn Leu Gly Ala Ile Val Asp
1               5                   10                  15


Leu Lys




<210>  297
<211>  9
<212>  PRT
<213>  Glycine max

<400>  297

Ile Leu Asn His Leu Ile Gln Asn Lys
1               5

<210>  298
<211>  26
<212>  PRT
<213>  Glycine max

<400>  298

Ile Gln Thr Pro Thr Asp Glu Val Val Val Pro Tyr Asp Thr Leu Ala
1               5                   10                  15

Pro Thr Pro Glu Gly Ser Ser Glu Val Lys
            20                  25

<210>  299
<211>  9
<212>  PRT
<213>  Glycine max

<400>  299

Leu Asp Ala Leu Leu Ser Gln Gly Lys
1               5

<210>  300
<211>  9
<212>  PRT
<213>  Glycine max

<400>  300

Arg Leu Val Glu Ala Asp Ala Leu Lys
1               5

<210>  301
<211>  14
<212>  PRT
<213>  Glycine max

<400>  301

Ser Ala Val Ala Gly Leu Asn Glu Ile Ser Glu Asn Glu Lys
1               5                   10

<210>  302
<211>  12
<212>  PRT
<213>  Glycine max

<400>  302

Ser Ile Pro Ser Ile Val Glu Leu Asp Ser Leu Lys

1               5                   10


<210>  303
<211>  17
<212>  PRT
<213>  Glycine max

<400>  303

Val Leu Gln Leu Glu Thr Ala Ala Gly Ala Ala Ile Arg Phe Phe Asp
1               5                   10                  15


Lys




<210>  304
<211>  9
<212>  PRT
<213>  Glycine max

<400>  304

Val Ser Asn Phe Leu Gly Arg Phe Lys
1               5


<210>  305
<211>  1353
<212>  DNA
<213>  Glycine max


<220>
<221>  CDS
<222>  (1)..(1353)

<400>  305
atg acc acc gcc acc gag aag ctc tcc gct ctc aaa tcc gcc gtc gcc      48
Met Thr Thr Ala Thr Glu Lys Leu Ser Ala Leu Lys Ser Ala Val Ala
1               5                   10                  15

gga ttg aac gaa atc agt gag agt gag aag aac gga ttc atc agc ctc      96
Gly Leu Asn Glu Ile Ser Glu Ser Glu Lys Asn Gly Phe Ile Ser Leu
            20                  25                  30

gtc agc cgc tat ctc agt ggc gaa gcg cag cat gtg gaa tgg agc aag      144
Val Ser Arg Tyr Leu Ser Gly Glu Ala Gln His Val Glu Trp Ser Lys
        35                  40                  45

atc cag acg cct acg gac gaa gtg gtt gtg cct tac gac act ttg gcg      192
Ile Gln Thr Pro Thr Asp Glu Val Val Val Pro Tyr Asp Thr Leu Ala
    50                  55                  60

cca act cct gat ggt tct tcg gac gtg aag aat cta ttg gac aag ctt      240
Pro Thr Pro Asp Gly Ser Ser Asp Val Lys Asn Leu Leu Asp Lys Leu
65                  70                  75                  80

gtg gtg ttg aag cta aat gga ggc ttg ggc aca act atg ggt tgc act      288
Val Val Leu Lys Leu Asn Gly Gly Leu Gly Thr Thr Met Gly Cys Thr
                85                  90                  95

ggt cct aaa tct gta att gaa gtt cgt gat ggg ttg aca ttt cta gat     336
Gly Pro Lys Ser Val Ile Glu Val Arg Asp Gly Leu Thr Phe Leu Asp
            100                 105                 110

tta att gtg atc cag att gag aat ctc aat tcc aaa tat gga agc aat     384
Leu Ile Val Ile Gln Ile Glu Asn Leu Asn Ser Lys Tyr Gly Ser Asn
        115                 120                 125

gtt cct ttg ctt ttg atg aat tca ttc aac act cat gat gac act caa     432
Val Pro Leu Leu Leu Met Asn Ser Phe Asn Thr His Asp Asp Thr Gln
    130                 135                 140

aag att gtt gaa aaa tac caa aac tcc aat att gag att cat act ttt     480
Lys Ile Val Glu Lys Tyr Gln Asn Ser Asn Ile Glu Ile His Thr Phe
145                 150                 155                 160

aac cag ggc ata ctg aca agg atg gat ggt acc ctc ctg gcc cat gga     528
Asn Gln Gly Ile Leu Thr Arg Met Asp Gly Thr Leu Leu Ala His Gly
                165                 170                 175

gat gtc ttt cca tca tta ttg aac agt ggc aaa ctt gat gca cta ttg     576
Asp Val Phe Pro Ser Leu Leu Asn Ser Gly Lys Leu Asp Ala Leu Leu
            180                 185                 190

tca cag ggt aaa gag tat gta ttt gtt gcc aat tca gat aac ttg gga     624
Ser Gln Gly Lys Glu Tyr Val Phe Val Ala Asn Ser Asp Asn Leu Gly
        195                 200                 205

gct ata gtt gac ttg aaa atc tta aat cat ttg atc cag aac aag aat     672
Ala Ile Val Asp Leu Lys Ile Leu Asn His Leu Ile Gln Asn Lys Asn
    210                 215                 220

gaa tac tgt atg gag gtg act ccc aaa aca ttg gct gat gta aag ggt     720
Glu Tyr Cys Met Glu Val Thr Pro Lys Thr Leu Ala Asp Val Lys Gly
225                 230                 235                 240

ggc act ttg att tct tac gaa gga agg ctt ttg gaa att gca caa gtc     768
Gly Thr Leu Ile Ser Tyr Glu Gly Arg Leu Leu Glu Ile Ala Gln Val
                245                 250                 255

cca gat gaa cat gtc aat gag ttc aag tca ata gag aag ttc aaa att     816
Pro Asp Glu His Val Asn Glu Phe Lys Ser Ile Glu Lys Phe Lys Ile
            260                 265                 270

ttc aac aca aat aat ttg tgg gtg aac tta aat gca gtt aaa agg ctt     864
Phe Asn Thr Asn Asn Leu Trp Val Asn Leu Asn Ala Val Lys Arg Leu
        275                 280                 285

gtt gaa gct gat gct ctt aag atg gaa att att ccc aat cca aag gaa     912
Val Glu Ala Asp Ala Leu Lys Met Glu Ile Ile Pro Asn Pro Lys Glu
    290                 295                 300

gtt gat gga ata aaa gtt ctt cag ctg gaa act gca gct ggt gct gca     960
Val Asp Gly Ile Lys Val Leu Gln Leu Glu Thr Ala Ala Gly Ala Ala
305                 310                 315                 320

ata agg ttc ttt gac aag gct att ggg att aat gtt cct cga tca cga     1008
Ile Arg Phe Phe Asp Lys Ala Ile Gly Ile Asn Val Pro Arg Ser Arg
                325                 330                 335

ttc ctt cct gtg aag gca act tca gat ttg ctt ctt gtc cag tct gac     1056
Phe Leu Pro Val Lys Ala Thr Ser Asp Leu Leu Leu Val Gln Ser Asp

340                 345                 350

ctc tac act ttg gaa gat gga ttt gtc att cgg aac aaa gct agg gca      1104
Leu Tyr Thr Leu Glu Asp Gly Phe Val Ile Arg Asn Lys Ala Arg Ala
        355                 360                 365

aat cct gaa aac cct tct att gaa ctt ggg cca gaa ttt aag aag gtt      1152
Asn Pro Glu Asn Pro Ser Ile Glu Leu Gly Pro Glu Phe Lys Lys Val
    370                 375                 380

agc aac ttc ttg ggc cgc ttc aag tca att ccc agt att gtt gag ctt      1200
Ser Asn Phe Leu Gly Arg Phe Lys Ser Ile Pro Ser Ile Val Glu Leu
385                 390                 395                 400

gac agt cta aaa gtg gct ggc aat gta tgg ttt gga gct ggt gtt atc      1248
Asp Ser Leu Lys Val Ala Gly Asn Val Trp Phe Gly Ala Gly Val Ile
                405                 410                 415

ctc aag gga aaa atc agt atc gtg gcc aat cct ggt gtt aag ctg gaa      1296
Leu Lys Gly Lys Ile Ser Ile Val Ala Asn Pro Gly Val Lys Leu Glu
            420                 425                 430

gtt ccc gat ggt gct gtc att tcg gat aag gaa att aat ggc cca gag      1344
Val Pro Asp Gly Ala Val Ile Ser Asp Lys Glu Ile Asn Gly Pro Glu
        435                 440                 445

gac ctc ctg                                                          1353
Asp Leu Leu
    450


<210>  306
<211>  451
<212>  PRT
<213>  Glycine max

<400>  306

Met Thr Thr Ala Thr Glu Lys Leu Ser Ala Leu Lys Ser Ala Val Ala
1               5                   10                  15


Gly Leu Asn Glu Ile Ser Glu Ser Glu Lys Asn Gly Phe Ile Ser Leu
            20                  25                  30


Val Ser Arg Tyr Leu Ser Gly Glu Ala Gln His Val Glu Trp Ser Lys
        35                  40                  45


Ile Gln Thr Pro Thr Asp Glu Val Val Val Pro Tyr Asp Thr Leu Ala
    50                  55                  60


Pro Thr Pro Asp Gly Ser Ser Asp Val Lys Asn Leu Leu Asp Lys Leu
65                  70                  75                  80


Val Val Leu Lys Leu Asn Gly Gly Leu Gly Thr Thr Met Gly Cys Thr
                85                  90                  95


Gly Pro Lys Ser Val Ile Glu Val Arg Asp Gly Leu Thr Phe Leu Asp

            100                 105                 110

Leu Ile Val Ile Gln Ile Glu Asn Leu Asn Ser Lys Tyr Gly Ser Asn
        115                 120                 125

Val Pro Leu Leu Leu Met Asn Ser Phe Asn Thr His Asp Asp Thr Gln
    130                 135                 140

Lys Ile Val Glu Lys Tyr Gln Asn Ser Asn Ile Glu Ile His Thr Phe
145                 150                 155                 160

Asn Gln Gly Ile Leu Thr Arg Met Asp Gly Thr Leu Leu Ala His Gly
                165                 170                 175

Asp Val Phe Pro Ser Leu Leu Asn Ser Gly Lys Leu Asp Ala Leu Leu
            180                 185                 190

Ser Gln Gly Lys Glu Tyr Val Phe Val Ala Asn Ser Asp Asn Leu Gly
        195                 200                 205

Ala Ile Val Asp Leu Lys Ile Leu Asn His Leu Ile Gln Asn Lys Asn
    210                 215                 220

Glu Tyr Cys Met Glu Val Thr Pro Lys Thr Leu Ala Asp Val Lys Gly
225                 230                 235                 240

Gly Thr Leu Ile Ser Tyr Glu Gly Arg Leu Leu Glu Ile Ala Gln Val
                245                 250                 255

Pro Asp Glu His Val Asn Glu Phe Lys Ser Ile Glu Lys Phe Lys Ile
            260                 265                 270

Phe Asn Thr Asn Asn Leu Trp Val Asn Leu Asn Ala Val Lys Arg Leu
        275                 280                 285

Val Glu Ala Asp Ala Leu Lys Met Glu Ile Ile Pro Asn Pro Lys Glu
    290                 295                 300

Val Asp Gly Ile Lys Val Leu Gln Leu Glu Thr Ala Ala Gly Ala Ala
305                 310                 315                 320

Ile Arg Phe Phe Asp Lys Ala Ile Gly Ile Asn Val Pro Arg Ser Arg
                325                 330                 335

Phe Leu Pro Val Lys Ala Thr Ser Asp Leu Leu Leu Val Gln Ser Asp
            340                 345                 350

Leu Tyr Thr Leu Glu Asp Gly Phe Val Ile Arg Asn Lys Ala Arg Ala
        355                 360                 365

Asn Pro Glu Asn Pro Ser Ile Glu Leu Gly Pro Glu Phe Lys Lys Val
    370                 375                 380

Ser Asn Phe Leu Gly Arg Phe Lys Ser Ile Pro Ser Ile Val Glu Leu
385                 390                 395                 400

Asp Ser Leu Lys Val Ala Gly Asn Val Trp Phe Gly Ala Gly Val Ile
                405                 410                 415

Leu Lys Gly Lys Ile Ser Ile Val Ala Asn Pro Gly Val Lys Leu Glu
            420                 425                 430

Val Pro Asp Gly Ala Val Ile Ser Asp Lys Glu Ile Asn Gly Pro Glu
        435                 440                 445

Asp Leu Leu
    450

<210>  307
<211>  15
<212>  PRT
<213>  Glycine max

<400>  307

Ala Ile Gly Ile Asn Val Pro Arg Ser Arg Phe Leu Pro Val Lys
1               5                   10                  15

<210>  308
<211>  24
<212>  PRT
<213>  Glycine max

<400>  308

Ala Thr Ser Asp Leu Leu Leu Val Gln Ser Asp Leu Tyr Thr Leu Glu
1               5                   10                  15

Asp Gly Phe Val Ile Arg Asn Lys
            20

<210>  309
<211>  18
<212>  PRT
<213>  Glycine max

<400>  309

Glu Tyr Val Phe Val Ala Asn Ser Asp Asn Leu Gly Ala Ile Val Asp
1               5                   10                  15

Leu Lys

<210> 310
<211> 9
<212> PRT
<213> Glycine max

<400> 310

Ile Leu Asn His Leu Ile Gln Asn Lys
1               5

<210> 311
<211> 26
<212> PRT
<213> Glycine max

<400> 311

Ile Gln Thr Pro Thr Asp Glu Val Val Val Pro Tyr Asp Thr Leu Ala
1               5                   10                  15

Pro Thr Pro Asp Gly Ser Ser Asp Val Lys
            20                  25

<210> 312
<211> 9
<212> PRT
<213> Glycine max

<400> 312

Leu Asp Ala Leu Leu Ser Gln Gly Lys
1               5

<210> 313
<211> 12
<212> PRT
<213> Glycine max

<400> 313

Leu Glu Val Pro Asp Gly Ala Val Ile Ser Asp Lys
1               5                   10

<210> 314
<211> 9
<212> PRT
<213> Glycine max

<400> 314

Arg Leu Val Glu Ala Asp Ala Leu Lys
1               5

<210>  315
<211>  14
<212>  PRT
<213>  Glycine max

<400>  315

Ser Ala Val Ala Gly Leu Asn Glu Ile Ser Glu Ser Glu Lys
1               5                   10


<210>  316
<211>  12
<212>  PRT
<213>  Glycine max

<400>  316

Ser Ile Pro Ser Ile Val Glu Leu Asp Ser Leu Lys
1               5                   10


<210>  317
<211>  17
<212>  PRT
<213>  Glycine max

<400>  317

Val Leu Gln Leu Glu Thr Ala Ala Gly Ala Ala Ile Arg Phe Phe Asp
1               5                   10                  15


Lys



<210>  318
<211>  9
<212>  PRT
<213>  Glycine max

<400>  318

Val Ser Asn Phe Leu Gly Arg Phe Lys
1               5


<210>  319
<211>  1332
<212>  DNA
<213>  Glycine max


<220>
<221>  CDS
<222>  (1)..(1332)

<400>  319
atg gat gag gag tac gat gtg atc gtg ttg ggc acg ggt ctc aag gag          48
Met Asp Glu Glu Tyr Asp Val Ile Val Leu Gly Thr Gly Leu Lys Glu

1               5                   10                  15

tgc att ctc agc ggt ctt ctc tcc gtc gat ggc ctc aag gtt ctg cac          96
Cys Ile Leu Ser Gly Leu Leu Ser Val Asp Gly Leu Lys Val Leu His
            20                  25                  30

atg gac agg aat gac tat tac gga gga gaa tcc act tcg ctc aat ctt          144
Met Asp Arg Asn Asp Tyr Tyr Gly Gly Glu Ser Thr Ser Leu Asn Leu
        35                  40                  45

att cag ctt tgg aag aga ttc agg gga gat gac aag cct cct cca cat          192
Ile Gln Leu Trp Lys Arg Phe Arg Gly Asp Asp Lys Pro Pro Pro His
    50                  55                  60

ttg gga gct agc agg gac tat aac atc gat atg aac cct aag ttt att          240
Leu Gly Ala Ser Arg Asp Tyr Asn Ile Asp Met Asn Pro Lys Phe Ile
65                  70                  75                  80

atg gct aat ggc aat ttg gtg cgg gtt ctc ata cat act gat gtt act          288
Met Ala Asn Gly Asn Leu Val Arg Val Leu Ile His Thr Asp Val Thr
                85                  90                  95

aag tat ctc tct ttc aaa gct gtg gat ggg agc ttt gtg ttt aat aaa          336
Lys Tyr Leu Ser Phe Lys Ala Val Asp Gly Ser Phe Val Phe Asn Lys
            100                 105                 110

gga aag gtt cat aag gtg ccc gca aat gac atg gag gcc ttg aag tct          384
Gly Lys Val His Lys Val Pro Ala Asn Asp Met Glu Ala Leu Lys Ser
        115                 120                 125

cca ctt atg ggg cta ttt gaa aaa cgc cga gca cgc aag ttt ttc att          432
Pro Leu Met Gly Leu Phe Glu Lys Arg Arg Ala Arg Lys Phe Phe Ile
    130                 135                 140

tat gtg caa aat tac aat gag agt gat ccc aag acc cat gaa ggg atg          480
Tyr Val Gln Asn Tyr Asn Glu Ser Asp Pro Lys Thr His Glu Gly Met
145                 150                 155                 160

gac ttg aca aga gtg act act aaa gag ttg ata gca aaa tac ggt ctt          528
Asp Leu Thr Arg Val Thr Thr Lys Glu Leu Ile Ala Lys Tyr Gly Leu
                165                 170                 175

gat gat aac act ata gac ttc att ggt cat gct ttg gcg ctt cat agt          576
Asp Asp Asn Thr Ile Asp Phe Ile Gly His Ala Leu Ala Leu His Ser
            180                 185                 190

gat gat cgc tac ttg gct gag cca gca ctg gac act gtg aag aga atg          624
Asp Asp Arg Tyr Leu Ala Glu Pro Ala Leu Asp Thr Val Lys Arg Met
        195                 200                 205

aag tta tat gca gag tct ctt gca cgc ttt caa gga ggg tca cct tac          672
Lys Leu Tyr Ala Glu Ser Leu Ala Arg Phe Gln Gly Gly Ser Pro Tyr
    210                 215                 220

ata tat cct ttg tat ggt tta gga gag ctt ccc cag gca ttt gca cgg          720
Ile Tyr Pro Leu Tyr Gly Leu Gly Glu Leu Pro Gln Ala Phe Ala Arg
225                 230                 235                 240

ctt agt gct gtt tat ggt ggg aca tat atg ttg aat aag cct gag tgc          768
Leu Ser Ala Val Tyr Gly Gly Thr Tyr Met Leu Asn Lys Pro Glu Cys
                245                 250                 255

aag gta gaa ttt gat gag gaa gga aag gtt gtt ggt gtc aca tct gaa          816

Lys Val Glu Phe Asp Glu Glu Gly Lys Val Val Gly Val Thr Ser Glu
            260                 265                 270

ggg gaa act gca aag tgc aaa aaa gtt gtt tgt gat cca tca tat ttg      864
Gly Glu Thr Ala Lys Cys Lys Lys Val Val Cys Asp Pro Ser Tyr Leu
        275                 280                 285

ccc ggc aag gtg agg aag gtt ggt agg gtt gca aga gca ata gcc ata      912
Pro Gly Lys Val Arg Lys Val Gly Arg Val Ala Arg Ala Ile Ala Ile
    290                 295                 300

atg agt cac cca atc ccc agc act gat gat tcc cat tcg gtg caa att      960
Met Ser His Pro Ile Pro Ser Thr Asp Asp Ser His Ser Val Gln Ile
305                 310                 315                 320

att tta cca cag aaa cag ttg ggt cgg aag tca gac atg tac cta ttt      1008
Ile Leu Pro Gln Lys Gln Leu Gly Arg Lys Ser Asp Met Tyr Leu Phe
                325                 330                 335

tgt tgt tca tat act cac aat gtt gct cca aag gga aaa ttt att gca      1056
Cys Cys Ser Tyr Thr His Asn Val Ala Pro Lys Gly Lys Phe Ile Ala
            340                 345                 350

ttt gta tca acc gag gca gag aca gat cag cca gca att gaa cta aaa      1104
Phe Val Ser Thr Glu Ala Glu Thr Asp Gln Pro Ala Ile Glu Leu Lys
        355                 360                 365

cct gga att gac ctt cta gga cct gtt gat gag ata ttt ttt gat atc      1152
Pro Gly Ile Asp Leu Leu Gly Pro Val Asp Glu Ile Phe Phe Asp Ile
    370                 375                 380

tat gac aga tat gaa ccg gtc aat gaa ccc act ttg gac aac tgc ttt      1200
Tyr Asp Arg Tyr Glu Pro Val Asn Glu Pro Thr Leu Asp Asn Cys Phe
385                 390                 395                 400

gta tca aca agt tat gat gct act act cac ttt gag tcg acc gtg gat      1248
Val Ser Thr Ser Tyr Asp Ala Thr Thr His Phe Glu Ser Thr Val Asp
                405                 410                 415

gat gtt ctc aac atg tat aca ttg ata act gga aag gtt att gac ctg      1296
Asp Val Leu Asn Met Tyr Thr Leu Ile Thr Gly Lys Val Ile Asp Leu
            420                 425                 430

agt gtg gat ctt agt gct gct agt gct gca gaa gag                      1332
Ser Val Asp Leu Ser Ala Ala Ser Ala Ala Glu Glu
        435                 440

<210>  320
<211>  444
<212>  PRT
<213>  Glycine max

<400>  320

Met Asp Glu Glu Tyr Asp Val Ile Val Leu Gly Thr Gly Leu Lys Glu
1               5                   10                  15

Cys Ile Leu Ser Gly Leu Leu Ser Val Asp Gly Leu Lys Val Leu His
            20                  25                  30

Met Asp Arg Asn Asp Tyr Tyr Gly Gly Glu Ser Thr Ser Leu Asn Leu
        35                  40                  45

Ile Gln Leu Trp Lys Arg Phe Arg Gly Asp Asp Lys Pro Pro Pro His
    50                  55                  60

Leu Gly Ala Ser Arg Asp Tyr Asn Ile Asp Met Asn Pro Lys Phe Ile
65                  70                  75                  80

Met Ala Asn Gly Asn Leu Val Arg Val Leu Ile His Thr Asp Val Thr
                85                  90                  95

Lys Tyr Leu Ser Phe Lys Ala Val Asp Gly Ser Phe Val Phe Asn Lys
            100                 105                 110

Gly Lys Val His Lys Val Pro Ala Asn Asp Met Glu Ala Leu Lys Ser
        115                 120                 125

Pro Leu Met Gly Leu Phe Glu Lys Arg Arg Ala Arg Lys Phe Phe Ile
    130                 135                 140

Tyr Val Gln Asn Tyr Asn Glu Ser Asp Pro Lys Thr His Glu Gly Met
145                 150                 155                 160

Asp Leu Thr Arg Val Thr Thr Lys Glu Leu Ile Ala Lys Tyr Gly Leu
                165                 170                 175

Asp Asp Asn Thr Ile Asp Phe Ile Gly His Ala Leu Ala Leu His Ser
            180                 185                 190

Asp Asp Arg Tyr Leu Ala Glu Pro Ala Leu Asp Thr Val Lys Arg Met
        195                 200                 205

Lys Leu Tyr Ala Glu Ser Leu Ala Arg Phe Gln Gly Gly Ser Pro Tyr
    210                 215                 220

Ile Tyr Pro Leu Tyr Gly Leu Gly Glu Leu Pro Gln Ala Phe Ala Arg
225                 230                 235                 240

Leu Ser Ala Val Tyr Gly Gly Thr Tyr Met Leu Asn Lys Pro Glu Cys
                245                 250                 255

Lys Val Glu Phe Asp Glu Glu Gly Lys Val Val Gly Val Thr Ser Glu
            260                 265                 270

Gly Glu Thr Ala Lys Cys Lys Lys Val Val Cys Asp Pro Ser Tyr Leu
        275                 280                 285

Pro Gly Lys Val Arg Lys Val Gly Arg Val Ala Arg Ala Ile Ala Ile
    290                 295                 300

Met Ser His Pro Ile Pro Ser Thr Asp Asp Ser His Ser Val Gln Ile
305                 310                 315                 320

Ile Leu Pro Gln Lys Gln Leu Gly Arg Lys Ser Asp Met Tyr Leu Phe
                325                 330                 335

Cys Cys Ser Tyr Thr His Asn Val Ala Pro Lys Gly Lys Phe Ile Ala
            340                 345                 350

Phe Val Ser Thr Glu Ala Glu Thr Asp Gln Pro Ala Ile Glu Leu Lys
        355                 360                 365

Pro Gly Ile Asp Leu Leu Gly Pro Val Asp Glu Ile Phe Phe Asp Ile
    370                 375                 380

Tyr Asp Arg Tyr Glu Pro Val Asn Glu Pro Thr Leu Asp Asn Cys Phe
385                 390                 395                 400

Val Ser Thr Ser Tyr Asp Ala Thr Thr His Phe Glu Ser Thr Val Asp
                405                 410                 415

Asp Val Leu Asn Met Tyr Thr Leu Ile Thr Gly Lys Val Ile Asp Leu
            420                 425                 430

Ser Val Asp Leu Ser Ala Ala Ser Ala Ala Glu Glu
        435                 440

<210>  321
<211>  10
<212>  PRT
<213>  Glycine max

<400>  321

Ala Val Asp Gly Ser Phe Val Phe Asn Lys
1               5                   10

<210>  322
<211>  14
<212>  PRT
<213>  Glycine max

<400>  322

Phe Phe Ile Tyr Val Gln Asn Tyr Asn Glu Ser Asp Pro Lys
1               5                   10

<210> 323
<211> 8
<212> PRT
<213> Glycine max

<400> 323

Val Glu Phe Asp Glu Glu Gly Lys
1               5

<210> 324
<211> 16
<212> PRT
<213> Glycine max

<400> 324

Val Ile Asp Leu Ser Val Asp Leu Ser Ala Ala Ser Ala Ala Glu Glu
1               5                   10                  15

<210> 325
<211> 12
<212> PRT
<213> Glycine max

<400> 325

Val Val Gly Val Thr Ser Glu Gly Glu Thr Ala Lys
1               5                   10

<210> 326
<211> 1332
<212> DNA
<213> Glycine max

<220>
<221> CDS
<222> (1)..(1332)

<400> 326
atg gat gag gag tac gat gtg atc gtg ttg ggc acg ggt ctc aag gag 48
Met Asp Glu Glu Tyr Asp Val Ile Val Leu Gly Thr Gly Leu Lys Glu
1               5                   10                  15

tgc att ctc agc ggt ctt ctc tcc gtc gat ggc ctc aag gtt ctg cat 96
Cys Ile Leu Ser Gly Leu Leu Ser Val Asp Gly Leu Lys Val Leu His
            20                  25                  30

atg gat agg aac gac tat tac gga gga gaa tcc act tcg ctc aat ctt 144
Met Asp Arg Asn Asp Tyr Tyr Gly Gly Glu Ser Thr Ser Leu Asn Leu
        35                  40                  45

att cag ctt tgg aag aga ttc agg gga gat gac aag cct cct cca cat 192
Ile Gln Leu Trp Lys Arg Phe Arg Gly Asp Asp Lys Pro Pro Pro His
    50                  55                  60

ttg ggc gct agc agg gac tac aac atc gat atg aac ccc aag ttt att 240
Leu Gly Ala Ser Arg Asp Tyr Asn Ile Asp Met Asn Pro Lys Phe Ile
65                  70                  75                  80

atg gct aat ggc aat ttg gtg cgg gtt ctc ata cat acc gat gtt aca 288
Met Ala Asn Gly Asn Leu Val Arg Val Leu Ile His Thr Asp Val Thr
                85                  90                  95

aag tat ctc tat ttc aaa gct gtg gat ggg agc tat gtg ttt aat aaa 336
Lys Tyr Leu Tyr Phe Lys Ala Val Asp Gly Ser Tyr Val Phe Asn Lys
            100                 105                 110

gga aag gtt cat aag gtg cct gca aac gac atg gag gcc ttg aag tcc 384
Gly Lys Val His Lys Val Pro Ala Asn Asp Met Glu Ala Leu Lys Ser
        115                 120                 125

cca ctt atg ggg cta ttt gaa aaa cgc cgg gca cgc aag ttt ttc att 432
Pro Leu Met Gly Leu Phe Glu Lys Arg Arg Ala Arg Lys Phe Phe Ile
    130                 135                 140

tat gtg caa aac tac aac gag agc gat ccc aag acc cac gag ggg atg 480
Tyr Val Gln Asn Tyr Asn Glu Ser Asp Pro Lys Thr His Glu Gly Met
145                 150                 155                 160

gac ttg aca aga gtg act act aaa gag ttg ata gca aaa tac ggc ctt 528
Asp Leu Thr Arg Val Thr Thr Lys Glu Leu Ile Ala Lys Tyr Gly Leu
                165                 170                 175

gat gac aac act ata gac ttc att ggt cat gct ttg gcg ctt cat agc 576
Asp Asp Asn Thr Ile Asp Phe Ile Gly His Ala Leu Ala Leu His Ser
            180                 185                 190

gat gat cgc tac ttg gct gag cca gca ctg gac acc gtg aag aga atg 624
Asp Asp Arg Tyr Leu Ala Glu Pro Ala Leu Asp Thr Val Lys Arg Met
        195                 200                 205

aag tta tat gca gag tct ctt gca cgc ttt caa gga ggg tca cct tac 672
Lys Leu Tyr Ala Glu Ser Leu Ala Arg Phe Gln Gly Gly Ser Pro Tyr
    210                 215                 220

ata tat cct ttg tat ggt tta gga gag ctt ccc cag gca ttt gca cgg 720
Ile Tyr Pro Leu Tyr Gly Leu Gly Glu Leu Pro Gln Ala Phe Ala Arg
225                 230                 235                 240

ctt agt gct gtt tat ggt gga aca tat atg ttg aat aag cct gag tgc 768
Leu Ser Ala Val Tyr Gly Gly Thr Tyr Met Leu Asn Lys Pro Glu Cys
                245                 250                 255

aag gta gaa ttt gat gag gaa gga aag gtt gtt ggt gtt aca tct gaa 816
Lys Val Glu Phe Asp Glu Glu Gly Lys Val Val Gly Val Thr Ser Glu
            260                 265                 270

ggg gaa act gca aag tgt aaa aaa gtt gtt tgt gat cca tca tat ttg 864
Gly Glu Thr Ala Lys Cys Lys Lys Val Val Cys Asp Pro Ser Tyr Leu
        275                 280                 285

cct ggc aag gtg agg aag gtt ggt agg gtt gca agg gcg ata gcc atc 912
Pro Gly Lys Val Arg Lys Val Gly Arg Val Ala Arg Ala Ile Ala Ile
    290                 295                 300

atg agc cac cca atc ccc agc act gat gat tcc cat tca gtg caa att 960
Met Ser His Pro Ile Pro Ser Thr Asp Asp Ser His Ser Val Gln Ile
305                 310                 315                 320

att tta cca cag aaa cag ttg ggt cgg aag tca gac atg tac cta ttt 1008
Ile Leu Pro Gln Lys Gln Leu Gly Arg Lys Ser Asp Met Tyr Leu Phe

325                 330                 335

tgt tgt tcg tat act cac aat gtt gct cca aag gga aaa ttt att gca       1056
Cys Cys Ser Tyr Thr His Asn Val Ala Pro Lys Gly Lys Phe Ile Ala
            340                 345                 350

ttt gta tca act gag gca gag aca gat cag ccg gca att gga ctg aaa       1104
Phe Val Ser Thr Glu Ala Glu Thr Asp Gln Pro Ala Ile Gly Leu Lys
        355                 360                 365

cct gga att gac ctt cta gga cct gtt gat gag ata ttt ttt gat atc       1152
Pro Gly Ile Asp Leu Leu Gly Pro Val Asp Glu Ile Phe Phe Asp Ile
    370                 375                 380

tat gac aga tat gaa cca gtc aac gaa ccc act ttg gac aac tgc ttt       1200
Tyr Asp Arg Tyr Glu Pro Val Asn Glu Pro Thr Leu Asp Asn Cys Phe
385                 390                 395                 400

ata tca aca agt tat gat gct act act cac ttt gag tcg act gtg gat       1248
Ile Ser Thr Ser Tyr Asp Ala Thr Thr His Phe Glu Ser Thr Val Asp
                405                 410                 415

gac gtt ctc aac atg tat aca ttg ata act gga aag gtt att gac ctc       1296
Asp Val Leu Asn Met Tyr Thr Leu Ile Thr Gly Lys Val Ile Asp Leu
            420                 425                 430

agt gtg gat ctt agt gct gct agt gct gca gaa gag                       1332
Ser Val Asp Leu Ser Ala Ala Ser Ala Ala Glu Glu
        435                 440

<210>  327
<211>  444
<212>  PRT
<213>  Glycine max

<400>  327

Met Asp Glu Glu Tyr Asp Val Ile Val Leu Gly Thr Gly Leu Lys Glu
1               5                   10                  15

Cys Ile Leu Ser Gly Leu Leu Ser Val Asp Gly Leu Lys Val Leu His
            20                  25                  30

Met Asp Arg Asn Asp Tyr Tyr Gly Gly Glu Ser Thr Ser Leu Asn Leu
        35                  40                  45

Ile Gln Leu Trp Lys Arg Phe Arg Gly Asp Asp Lys Pro Pro Pro His
    50                  55                  60

Leu Gly Ala Ser Arg Asp Tyr Asn Ile Asp Met Asn Pro Lys Phe Ile
65                  70                  75                  80

Met Ala Asn Gly Asn Leu Val Arg Val Leu Ile His Thr Asp Val Thr
                85                  90                  95

Lys Tyr Leu Tyr Phe Lys Ala Val Asp Gly Ser Tyr Val Phe Asn Lys

100                 105                 110

Gly Lys Val His Lys Val Pro Ala Asn Asp Met Glu Ala Leu Lys Ser
        115                 120                 125

Pro Leu Met Gly Leu Phe Glu Lys Arg Arg Ala Arg Lys Phe Phe Ile
    130                 135                 140

Tyr Val Gln Asn Tyr Asn Glu Ser Asp Pro Lys Thr His Glu Gly Met
145                 150                 155                 160

Asp Leu Thr Arg Val Thr Thr Lys Glu Leu Ile Ala Lys Tyr Gly Leu
                165                 170                 175

Asp Asp Asn Thr Ile Asp Phe Ile Gly His Ala Leu Ala Leu His Ser
            180                 185                 190

Asp Asp Arg Tyr Leu Ala Glu Pro Ala Leu Asp Thr Val Lys Arg Met
        195                 200                 205

Lys Leu Tyr Ala Glu Ser Leu Ala Arg Phe Gln Gly Gly Ser Pro Tyr
    210                 215                 220

Ile Tyr Pro Leu Tyr Gly Leu Gly Glu Leu Pro Gln Ala Phe Ala Arg
225                 230                 235                 240

Leu Ser Ala Val Tyr Gly Gly Thr Tyr Met Leu Asn Lys Pro Glu Cys
                245                 250                 255

Lys Val Glu Phe Asp Glu Glu Gly Lys Val Val Gly Val Thr Ser Glu
            260                 265                 270

Gly Glu Thr Ala Lys Cys Lys Lys Val Val Cys Asp Pro Ser Tyr Leu
        275                 280                 285

Pro Gly Lys Val Arg Lys Val Gly Arg Val Ala Arg Ala Ile Ala Ile
    290                 295                 300

Met Ser His Pro Ile Pro Ser Thr Asp Asp Ser His Ser Val Gln Ile
305                 310                 315                 320

Ile Leu Pro Gln Lys Gln Leu Gly Arg Lys Ser Asp Met Tyr Leu Phe
                325                 330                 335

Cys Cys Ser Tyr Thr His Asn Val Ala Pro Lys Gly Lys Phe Ile Ala
            340                 345                 350

Phe Val Ser Thr Glu Ala Glu Thr Asp Gln Pro Ala Ile Gly Leu Lys
        355                 360                 365

Pro Gly Ile Asp Leu Leu Gly Pro Val Asp Glu Ile Phe Phe Asp Ile
    370                 375                 380

Tyr Asp Arg Tyr Glu Pro Val Asn Glu Pro Thr Leu Asp Asn Cys Phe
385                 390                 395                 400

Ile Ser Thr Ser Tyr Asp Ala Thr Thr His Phe Glu Ser Thr Val Asp
                405                 410                 415

Asp Val Leu Asn Met Tyr Thr Leu Ile Thr Gly Lys Val Ile Asp Leu
            420                 425                 430

Ser Val Asp Leu Ser Ala Ala Ser Ala Ala Glu Glu
        435                 440

<210>  328
<211>  10
<212>  PRT
<213>  Glycine max

<400>  328

Ala Val Asp Gly Ser Tyr Val Phe Asn Lys
1               5                   10

<210>  329
<211>  14
<212>  PRT
<213>  Glycine max

<400>  329

Phe Phe Ile Tyr Val Gln Asn Tyr Asn Glu Ser Asp Pro Lys
1               5                   10

<210>  330
<211>  8
<212>  PRT
<213>  Glycine max

<400>  330

Val Glu Phe Asp Glu Glu Gly Lys
1               5

<210>  331
<211>  16
<212>  PRT
<213>  Glycine max

<400>  331

Val Ile Asp Leu Ser Val Asp Leu Ser Ala Ala Ser Ala Ala Glu Glu
1               5                   10                  15

<210>  332
<211>  12
<212>  PRT
<213>  Glycine max

<400>  332

Val Val Gly Val Thr Ser Glu Gly Glu Thr Ala Lys
1               5                   10

<210>  333
<211>  1512
<212>  DNA
<213>  Glycine max

<220>
<221>  CDS
<222>  (1)..(1512)

<400>  333
atg gcg atg aga acc aaa ctt tct tta gct atc ttt ttc ttt ttt ctt      48
Met Ala Met Arg Thr Lys Leu Ser Leu Ala Ile Phe Phe Phe Phe Leu
1               5                   10                  15

tta gcc tta ttt tca aac cta gcc ttt ggc aaa tgt aaa gaa acc gag      96
Leu Ala Leu Phe Ser Asn Leu Ala Phe Gly Lys Cys Lys Glu Thr Glu
            20                  25                  30

gta gaa gaa gaa gac cct gag ctc gta aca tgc aaa cac caa tgc caa      144
Val Glu Glu Glu Asp Pro Glu Leu Val Thr Cys Lys His Gln Cys Gln
        35                  40                  45

cag cca cag caa tac act gag ggt gac aag cgg gta tgc ttg caa aga      192
Gln Pro Gln Gln Tyr Thr Glu Gly Asp Lys Arg Val Cys Leu Gln Arg
    50                  55                  60

tgt gac agg tat cat cgt atg aag caa gag cga gag aaa caa atc caa      240
Cys Asp Arg Tyr His Arg Met Lys Gln Glu Arg Glu Lys Gln Ile Gln
65                  70                  75                  80

gag gaa act cgt gag aag aaa gag gag gaa agc cgt gaa agg gaa gaa      288
Glu Glu Thr Arg Glu Lys Lys Glu Glu Glu Ser Arg Glu Arg Glu Glu
                85                  90                  95

gaa caa caa gag cag cat gag gaa caa gat gaa aat ccc tac att ttt      336
Glu Gln Gln Glu Gln His Glu Glu Gln Asp Glu Asn Pro Tyr Ile Phe
            100                 105                 110

gag gaa gat aag gat ttt gag acc aga gtc gaa act gaa ggt ggc aga      384
Glu Glu Asp Lys Asp Phe Glu Thr Arg Val Glu Thr Glu Gly Gly Arg
        115                 120                 125

att cgg gtt ctc aag aag ttc act gaa aaa tcc aag ctt ctt cag ggc      432
Ile Arg Val Leu Lys Lys Phe Thr Glu Lys Ser Lys Leu Leu Gln Gly
    130                 135                 140

att gag aac ttc cgt ttg gcc att ttg gaa gct aga gca cac acg ttc 480
Ile Glu Asn Phe Arg Leu Ala Ile Leu Glu Ala Arg Ala His Thr Phe
145                 150                 155                 160

gtg tct cct cgc cac ttt gat tcc gag gtt gtt ttt ttc aac atc aag 528
Val Ser Pro Arg His Phe Asp Ser Glu Val Val Phe Phe Asn Ile Lys
                165                 170                 175

ggg cga gcc gta ctt ggg ttg gtg agt gaa agt gaa aca gaa aaa atc 576
Gly Arg Ala Val Leu Gly Leu Val Ser Glu Ser Glu Thr Glu Lys Ile
            180                 185                 190

acc ctt gaa cct ggg gac atg ata cac ata cca gct ggc acc cca ctg 624
Thr Leu Glu Pro Gly Asp Met Ile His Ile Pro Ala Gly Thr Pro Leu
        195                 200                 205

tac atc gtt aac aga gat gag aat gat aag ctc ttc ctt gcc atg ctc 672
Tyr Ile Val Asn Arg Asp Glu Asn Asp Lys Leu Phe Leu Ala Met Leu
    210                 215                 220

cat ata cct gtc tct gtc tct act cct gga aaa ttt gag gaa ttt ttc 720
His Ile Pro Val Ser Val Ser Thr Pro Gly Lys Phe Glu Glu Phe Phe
225                 230                 235                 240

ggg cct gga gga cga gac cca gaa tcg gtc ctc tca gca ttc agc tgg 768
Gly Pro Gly Gly Arg Asp Pro Glu Ser Val Leu Ser Ala Phe Ser Trp
                245                 250                 255

aat gtg ctg caa gct gcg ctc caa acc cca aaa gga aag tta gaa aag 816
Asn Val Leu Gln Ala Ala Leu Gln Thr Pro Lys Gly Lys Leu Glu Lys
            260                 265                 270

ctt ttt gat caa cag aac gag gga agt att ttc gca ata agc aga gaa 864
Leu Phe Asp Gln Gln Asn Glu Gly Ser Ile Phe Ala Ile Ser Arg Glu
        275                 280                 285

cag gtg cgt gcg ttg gcc cct acc aag aaa agc tct tgg tgg cca ttc 912
Gln Val Arg Ala Leu Ala Pro Thr Lys Lys Ser Ser Trp Trp Pro Phe
    290                 295                 300

ggt ggc gaa tcc aag cct caa ttc aat att ttc agc aag cgt ccc act 960
Gly Gly Glu Ser Lys Pro Gln Phe Asn Ile Phe Ser Lys Arg Pro Thr
305                 310                 315                 320

atc tcc aac gga tat ggt cgt tta act gaa gtt ggt cct gac gat gat 1008
Ile Ser Asn Gly Tyr Gly Arg Leu Thr Glu Val Gly Pro Asp Asp Asp
                325                 330                 335

gaa aag agt tgg ctt caa aga ctc aat ctc atg ctt acc ttt acc aac 1056
Glu Lys Ser Trp Leu Gln Arg Leu Asn Leu Met Leu Thr Phe Thr Asn
            340                 345                 350

atc acc cag aga tct atg agt act att cac tac aac tca cat gca acg 1104
Ile Thr Gln Arg Ser Met Ser Thr Ile His Tyr Asn Ser His Ala Thr
        355                 360                 365

aag ata gca ctg gtg atc gat ggc aga ggg cat ctt caa ata tca tgt 1152
Lys Ile Ala Leu Val Ile Asp Gly Arg Gly His Leu Gln Ile Ser Cys
    370                 375                 380

cca cac atg tca tca agg tcg tca cac tcg aag cat gat aag agt agc 1200
Pro His Met Ser Ser Arg Ser Ser His Ser Lys His Asp Lys Ser Ser
385                 390                 395                 400

cct tca tac cat aga atc agt tcc gat ttg aag cct gga atg gtg ttt 1248
Pro Ser Tyr His Arg Ile Ser Ser Asp Leu Lys Pro Gly Met Val Phe
                405                 410                 415

gtt gtc cct ccc ggt cat ccc ttc gtc acc ata gct tcc aat aaa gag 1296
Val Val Pro Pro Gly His Pro Phe Val Thr Ile Ala Ser Asn Lys Glu
            420                 425                 430

aat ctc ctc atg att tgc ttc gag gtt aac gct cga gac aac aag aag 1344
Asn Leu Leu Met Ile Cys Phe Glu Val Asn Ala Arg Asp Asn Lys Lys
        435                 440                 445

ttt aca ttt gca ggg aag gac aac att gtg agt tct ctg gac aac gtt 1392
Phe Thr Phe Ala Gly Lys Asp Asn Ile Val Ser Ser Leu Asp Asn Val
    450                 455                 460

gct aag gag ctt gcc ttt aac tat cct tct gag atg gtg aac gga gta 1440
Ala Lys Glu Leu Ala Phe Asn Tyr Pro Ser Glu Met Val Asn Gly Val
465                 470                 475                 480

ttc gac cga aag gag agt ttc ttt ttc cct ttt gag ttg ccg aga gag 1488
Phe Asp Arg Lys Glu Ser Phe Phe Phe Pro Phe Glu Leu Pro Arg Glu
                485                 490                 495

gag cgt ggt cgt cgc gct gat gcg 1512
Glu Arg Gly Arg Arg Ala Asp Ala
            500

<210> 334
<211> 504
<212> PRT
<213> Glycine max

<400> 334

Met Ala Met Arg Thr Lys Leu Ser Leu Ala Ile Phe Phe Phe Phe Leu
1               5                   10                  15

Leu Ala Leu Phe Ser Asn Leu Ala Phe Gly Lys Cys Lys Glu Thr Glu
            20                  25                  30

Val Glu Glu Glu Asp Pro Glu Leu Val Thr Cys Lys His Gln Cys Gln
        35                  40                  45

Gln Pro Gln Gln Tyr Thr Glu Gly Asp Lys Arg Val Cys Leu Gln Arg
    50                  55                  60

Cys Asp Arg Tyr His Arg Met Lys Gln Glu Arg Glu Lys Gln Ile Gln
65                  70                  75                  80

Glu Glu Thr Arg Glu Lys Lys Glu Glu Glu Ser Arg Glu Arg Glu Glu
                85                  90                  95

Glu Gln Gln Glu Gln His Glu Glu Gln Asp Glu Asn Pro Tyr Ile Phe
            100                 105                 110

Glu Glu Asp Lys Asp Phe Glu Thr Arg Val Glu Thr Glu Gly Gly Arg
        115                 120                 125

Ile Arg Val Leu Lys Lys Phe Thr Glu Lys Ser Lys Leu Leu Gln Gly
    130                 135                 140

Ile Glu Asn Phe Arg Leu Ala Ile Leu Glu Ala Arg Ala His Thr Phe
145                 150                 155                 160

Val Ser Pro Arg His Phe Asp Ser Glu Val Val Phe Phe Asn Ile Lys
                165                 170                 175

Gly Arg Ala Val Leu Gly Leu Val Ser Glu Ser Glu Thr Glu Lys Ile
            180                 185                 190

Thr Leu Glu Pro Gly Asp Met Ile His Ile Pro Ala Gly Thr Pro Leu
        195                 200                 205

Tyr Ile Val Asn Arg Asp Glu Asn Asp Lys Leu Phe Leu Ala Met Leu
    210                 215                 220

His Ile Pro Val Ser Val Ser Thr Pro Gly Lys Phe Glu Glu Phe Phe
225                 230                 235                 240

Gly Pro Gly Gly Arg Asp Pro Glu Ser Val Leu Ser Ala Phe Ser Trp
                245                 250                 255

Asn Val Leu Gln Ala Ala Leu Gln Thr Pro Lys Gly Lys Leu Glu Lys
            260                 265                 270

Leu Phe Asp Gln Gln Asn Glu Gly Ser Ile Phe Ala Ile Ser Arg Glu
        275                 280                 285

Gln Val Arg Ala Leu Ala Pro Thr Lys Lys Ser Ser Trp Trp Pro Phe
    290                 295                 300

Gly Gly Glu Ser Lys Pro Gln Phe Asn Ile Phe Ser Lys Arg Pro Thr
305                 310                 315                 320

Ile Ser Asn Gly Tyr Gly Arg Leu Thr Glu Val Gly Pro Asp Asp Asp
                325                 330                 335

Glu Lys Ser Trp Leu Gln Arg Leu Asn Leu Met Leu Thr Phe Thr Asn
            340                 345                 350

Ile Thr Gln Arg Ser Met Ser Thr Ile His Tyr Asn Ser His Ala Thr

355                 360                 365

Lys Ile Ala Leu Val Ile Asp Gly Arg Gly His Leu Gln Ile Ser Cys
    370                 375                 380

Pro His Met Ser Ser Arg Ser Ser His Ser Lys His Asp Lys Ser Ser
385                 390                 395                 400

Pro Ser Tyr His Arg Ile Ser Ser Asp Leu Lys Pro Gly Met Val Phe
                405                 410                 415

Val Val Pro Pro Gly His Pro Phe Val Thr Ile Ala Ser Asn Lys Glu
            420                 425                 430

Asn Leu Leu Met Ile Cys Phe Glu Val Asn Ala Arg Asp Asn Lys Lys
        435                 440                 445

Phe Thr Phe Ala Gly Lys Asp Asn Ile Val Ser Ser Leu Asp Asn Val
    450                 455                 460

Ala Lys Glu Leu Ala Phe Asn Tyr Pro Ser Glu Met Val Asn Gly Val
465                 470                 475                 480

Phe Asp Arg Lys Glu Ser Phe Phe Phe Pro Phe Glu Leu Pro Arg Glu
                485                 490                 495

Glu Arg Gly Arg Arg Ala Asp Ala
            500

<210>  335
<211>  17
<212>  PRT
<213>  Glycine max

<400>  335

Asp Phe Glu Thr Arg Val Glu Thr Glu Gly Gly Arg Ile Arg Val Leu
1               5                   10                  15

Lys

<210>  336
<211>  12
<212>  PRT
<213>  Glycine max

<400>  336

Asp Asn Ile Val Ser Ser Leu Asp Asn Val Ala Lys
1               5                   10

<210>  337
<211>  15
<212>  PRT
<213>  Glycine max

<400>  337

Gly Arg Ala Val Leu Gly Leu Val Ser Glu Ser Glu Thr Glu Lys
1               5                   10                  15


<210>  338
<211>  25
<212>  PRT
<213>  Glycine max

<400>  338

Leu Phe Asp Gln Gln Asn Glu Gly Ser Ile Phe Ala Ile Ser Arg Glu
1               5                   10                  15


Gln Val Arg Ala Leu Ala Pro Thr Lys
            20                  25


<210>  339
<211>  21
<212>  PRT
<213>  Glycine max

<400>  339

Arg Pro Thr Ile Ser Asn Gly Tyr Gly Arg Leu Thr Glu Val Gly Pro
1               5                   10                  15


Asp Asp Asp Glu Lys
            20


<210>  340
<211>  1467
<212>  DNA
<213>  Glycine max


<220>
<221>  CDS
<222>  (1)..(1467)

<400>  340
atg gcg acc aga gcc aag ctt tct tta gct atc ttc ctt ttc ttt ctt      48
Met Ala Thr Arg Ala Lys Leu Ser Leu Ala Ile Phe Leu Phe Phe Leu
1               5                   10                  15

tta gcc ttg att tca aac cta gcc ttg ggc aaa ctt aaa gaa acc gag      96
Leu Ala Leu Ile Ser Asn Leu Ala Leu Gly Lys Leu Lys Glu Thr Glu
            20                  25                  30

gtc gaa gaa gat ccc gag ctc gta aca tgc aaa cac cag tgc caa cag      144

Val Glu Glu Asp Pro Glu Leu Val Thr Cys Lys His Gln Cys Gln Gln
        35                  40                  45

caa cgg caa tac act gag agt gac aag cga aca tgc ttg caa caa tgt      192
Gln Arg Gln Tyr Thr Glu Ser Asp Lys Arg Thr Cys Leu Gln Gln Cys
    50                  55                  60

gac agt atg aag caa gag cga gag aaa caa gtc gaa gag gaa act cgc      240
Asp Ser Met Lys Gln Glu Arg Glu Lys Gln Val Glu Glu Glu Thr Arg
65                  70                  75                  80

gag aag gaa gaa gaa cat caa gag cag cat gag gag gag caa gac caa      288
Glu Lys Glu Glu Glu His Gln Glu Gln His Glu Glu Glu Gln Asp Gln
                85                  90                  95

aat ccc tac gtt ttt gaa gaa gat aag gat ttt tcg acc aga gtc gaa      336
Asn Pro Tyr Val Phe Glu Glu Asp Lys Asp Phe Ser Thr Arg Val Glu
            100                 105                 110

aca gaa ggt ggc agc att cgg gtt ctc aag aag ttc act gag aaa tcc      384
Thr Glu Gly Gly Ser Ile Arg Val Leu Lys Lys Phe Thr Glu Lys Ser
        115                 120                 125

aag ctt ctt caa ggc aat gag aat ttc cgt ttg gcc atc tta gaa gct      432
Lys Leu Leu Gln Gly Asn Glu Asn Phe Arg Leu Ala Ile Leu Glu Ala
    130                 135                 140

aga gca cac acg ttc gtg tcc cca cgc cac ttt gat tcc gag gtt gtc      480
Arg Ala His Thr Phe Val Ser Pro Arg His Phe Asp Ser Glu Val Val
145                 150                 155                 160

ttg ttc aac att aag ggg aga gcc gta ctt ggg ttg gtg agg gaa agt      528
Leu Phe Asn Ile Lys Gly Arg Ala Val Leu Gly Leu Val Arg Glu Ser
                165                 170                 175

gaa aca gaa aaa atc acc cta gaa cct gga gac atg ata cac ata cca      576
Glu Thr Glu Lys Ile Thr Leu Glu Pro Gly Asp Met Ile His Ile Pro
            180                 185                 190

gca ggc aca cca ctg tac atc gtt aac aga gat gag aat gag aag ctc      624
Ala Gly Thr Pro Leu Tyr Ile Val Asn Arg Asp Glu Asn Glu Lys Leu
        195                 200                 205

ctc ctt gcc atg ctc cat ata cct gtc tct act cct gga aaa ttt gag      672
Leu Leu Ala Met Leu His Ile Pro Val Ser Thr Pro Gly Lys Phe Glu
    210                 215                 220

gaa ttt ttc ggg cct gga gga cga gac cca gaa tcg gtc ctc tca gca      720
Glu Phe Phe Gly Pro Gly Gly Arg Asp Pro Glu Ser Val Leu Ser Ala
225                 230                 235                 240

ttc agc tgg aat gtg ctg caa gct gcg ctc caa acc cca aaa gga aag      768
Phe Ser Trp Asn Val Leu Gln Ala Ala Leu Gln Thr Pro Lys Gly Lys
                245                 250                 255

tta gaa agg ctt ttt aat caa cag aac gag gga agt att ttc aaa ata      816
Leu Glu Arg Leu Phe Asn Gln Gln Asn Glu Gly Ser Ile Phe Lys Ile
            260                 265                 270

agc aga gaa cgg gtg cgt gcg ttg gcc ccc acc aag aaa agc tct tgg      864
Ser Arg Glu Arg Val Arg Ala Leu Ala Pro Thr Lys Lys Ser Ser Trp
        275                 280                 285

tgg cca ttc ggc ggc gaa tcc aag gct caa ttc aat att ttc agc aag     912
Trp Pro Phe Gly Gly Glu Ser Lys Ala Gln Phe Asn Ile Phe Ser Lys
    290                 295                 300

cgt ccc act ttc tcc aac gga tat ggc cgt tta act gaa gtt ggt cct     960
Arg Pro Thr Phe Ser Asn Gly Tyr Gly Arg Leu Thr Glu Val Gly Pro
305                 310                 315                 320

gat gat gaa aag agt tgg ctt caa aga ctc aac ctc atg ctt acc ttt     1008
Asp Asp Glu Lys Ser Trp Leu Gln Arg Leu Asn Leu Met Leu Thr Phe
                325                 330                 335

acc aac atc acc cag aga tct atg agt act att cac tac aac tca cat     1056
Thr Asn Ile Thr Gln Arg Ser Met Ser Thr Ile His Tyr Asn Ser His
            340                 345                 350

gca acg aag ata gca ctg gtg atg gat ggt aga ggg cat ctt caa ata     1104
Ala Thr Lys Ile Ala Leu Val Met Asp Gly Arg Gly His Leu Gln Ile
        355                 360                 365

tca tgt cca cac atg tca tca agg tca gac tca aag cat gat aag agt     1152
Ser Cys Pro His Met Ser Ser Arg Ser Asp Ser Lys His Asp Lys Ser
    370                 375                 380

agc ccc tca tac cat aga atc agt gcg gac ttg aag cct gga atg gtg     1200
Ser Pro Ser Tyr His Arg Ile Ser Ala Asp Leu Lys Pro Gly Met Val
385                 390                 395                 400

ttt gtt gtc cct cct ggt cat ccc ttc gtc act ata gct tcc aat aaa     1248
Phe Val Val Pro Pro Gly His Pro Phe Val Thr Ile Ala Ser Asn Lys
                405                 410                 415

gag aat ctc ctc ata att tgc ttc gag gtt aac gtt cga gac aac aag     1296
Glu Asn Leu Leu Ile Ile Cys Phe Glu Val Asn Val Arg Asp Asn Lys
            420                 425                 430

aag ttt acg ttt gca ggg aag gac aac att gtg agc tct ctg gac aac     1344
Lys Phe Thr Phe Ala Gly Lys Asp Asn Ile Val Ser Ser Leu Asp Asn
        435                 440                 445

gta gct aag gag ctg gcc ttt aac tat cct tct gag atg gtg aac gga     1392
Val Ala Lys Glu Leu Ala Phe Asn Tyr Pro Ser Glu Met Val Asn Gly
    450                 455                 460

gtc ttc gaa aga aag gag agt ctc ttt ttc ccc ttc gag ttg ccg agc     1440
Val Phe Glu Arg Lys Glu Ser Leu Phe Phe Pro Phe Glu Leu Pro Ser
465                 470                 475                 480

gag gag cgt ggt cgt cgc gct gtt gcg                                 1467
Glu Glu Arg Gly Arg Arg Ala Val Ala
                485

<210>  341
<211>  489
<212>  PRT
<213>  Glycine max

<400>  341

Met Ala Thr Arg Ala Lys Leu Ser Leu Ala Ile Phe Leu Phe Phe Leu
1               5                   10                  15

Leu Ala Leu Ile Ser Asn Leu Ala Leu Gly Lys Leu Lys Glu Thr Glu
            20                  25                  30

Val Glu Glu Asp Pro Glu Leu Val Thr Cys Lys His Gln Cys Gln Gln
        35                  40                  45

Gln Arg Gln Tyr Thr Glu Ser Asp Lys Arg Thr Cys Leu Gln Gln Cys
    50                  55                  60

Asp Ser Met Lys Gln Glu Arg Glu Lys Gln Val Glu Glu Glu Thr Arg
65                  70                  75                  80

Glu Lys Glu Glu Glu His Gln Glu Gln His Glu Glu Glu Gln Asp Gln
                85                  90                  95

Asn Pro Tyr Val Phe Glu Glu Asp Lys Asp Phe Ser Thr Arg Val Glu
            100                 105                 110

Thr Glu Gly Gly Ser Ile Arg Val Leu Lys Lys Phe Thr Glu Lys Ser
        115                 120                 125

Lys Leu Leu Gln Gly Asn Glu Asn Phe Arg Leu Ala Ile Leu Glu Ala
    130                 135                 140

Arg Ala His Thr Phe Val Ser Pro Arg His Phe Asp Ser Glu Val Val
145                 150                 155                 160

Leu Phe Asn Ile Lys Gly Arg Ala Val Leu Gly Leu Val Arg Glu Ser
                165                 170                 175

Glu Thr Glu Lys Ile Thr Leu Glu Pro Gly Asp Met Ile His Ile Pro
            180                 185                 190

Ala Gly Thr Pro Leu Tyr Ile Val Asn Arg Asp Glu Asn Glu Lys Leu
        195                 200                 205

Leu Leu Ala Met Leu His Ile Pro Val Ser Thr Pro Gly Lys Phe Glu
    210                 215                 220

Glu Phe Phe Gly Pro Gly Gly Arg Asp Pro Glu Ser Val Leu Ser Ala
225                 230                 235                 240

Phe Ser Trp Asn Val Leu Gln Ala Ala Leu Gln Thr Pro Lys Gly Lys
                245                 250                 255

Leu Glu Arg Leu Phe Asn Gln Gln Asn Glu Gly Ser Ile Phe Lys Ile
            260                 265                 270

Ser Arg Glu Arg Val Arg Ala Leu Ala Pro Thr Lys Lys Ser Ser Trp
        275                 280                 285

Trp Pro Phe Gly Gly Glu Ser Lys Ala Gln Phe Asn Ile Phe Ser Lys
    290                 295                 300

Arg Pro Thr Phe Ser Asn Gly Tyr Gly Arg Leu Thr Glu Val Gly Pro
305                 310                 315                 320

Asp Asp Glu Lys Ser Trp Leu Gln Arg Leu Asn Leu Met Leu Thr Phe
                325                 330                 335

Thr Asn Ile Thr Gln Arg Ser Met Ser Thr Ile His Tyr Asn Ser His
            340                 345                 350

Ala Thr Lys Ile Ala Leu Val Met Asp Gly Arg Gly His Leu Gln Ile
        355                 360                 365

Ser Cys Pro His Met Ser Ser Arg Ser Asp Ser Lys His Asp Lys Ser
    370                 375                 380

Ser Pro Ser Tyr His Arg Ile Ser Ala Asp Leu Lys Pro Gly Met Val
385                 390                 395                 400

Phe Val Val Pro Pro Gly His Pro Phe Val Thr Ile Ala Ser Asn Lys
                405                 410                 415

Glu Asn Leu Leu Ile Ile Cys Phe Glu Val Asn Val Arg Asp Asn Lys
            420                 425                 430

Lys Phe Thr Phe Ala Gly Lys Asp Asn Ile Val Ser Ser Leu Asp Asn
        435                 440                 445

Val Ala Lys Glu Leu Ala Phe Asn Tyr Pro Ser Glu Met Val Asn Gly
    450                 455                 460

Val Phe Glu Arg Lys Glu Ser Leu Phe Phe Pro Phe Glu Leu Pro Ser
465                 470                 475                 480

Glu Glu Arg Gly Arg Arg Ala Val Ala
                485

<210>  342
<211>  8
<212>  PRT
<213>  Glycine max

<400>  342

Ala Gln Phe Asn Ile Phe Ser Lys
1               5

<210>  343
<211>  17
<212>  PRT
<213>  Glycine max

<400>  343

Asp Phe Ser Thr Arg Val Glu Thr Glu Gly Gly Ser Ile Arg Val Leu
1               5                   10                  15

Lys

<210>  344
<211>  12
<212>  PRT
<213>  Glycine max

<400>  344

Asp Asn Ile Val Ser Ser Leu Asp Asn Val Ala Lys
1               5                   10

<210>  345
<211>  15
<212>  PRT
<213>  Glycine max

<400>  345

Gly Arg Ala Val Leu Gly Leu Val Arg Glu Ser Glu Thr Glu Lys
1               5                   10                  15

<210>  346
<211>  20
<212>  PRT
<213>  Glycine max

<400>  346

Arg Pro Thr Phe Ser Asn Gly Tyr Gly Arg Leu Thr Glu Val Gly Pro
1               5                   10                  15

Asp Asp Glu Lys
            20

<210>  347
<211>  1266
<212>  DNA
<213>  Glycine max

<220>
<221> CDS
<222> (1)..(1266)

<400> 347
atg gtg aga gga ttg ctc aac aag ctc gtc tct cgc tcc ctc tcg gtc 48
Met Val Arg Gly Leu Leu Asn Lys Leu Val Ser Arg Ser Leu Ser Val
1               5                   10                  15

gcc gga aaa tgg cag cac aat caa ctc cgc cgc ctc aac atc cac gag 96
Ala Gly Lys Trp Gln His Asn Gln Leu Arg Arg Leu Asn Ile His Glu
            20                  25                  30

tac cag ggt gct gaa ttg atg agc aaa cac gga gtc aac gtt ccg aga 144
Tyr Gln Gly Ala Glu Leu Met Ser Lys His Gly Val Asn Val Pro Arg
        35                  40                  45

ggc gtg gct gtt tcc tct gtt gaa gag gcc aga aag gtc atc aag gac 192
Gly Val Ala Val Ser Ser Val Glu Glu Ala Arg Lys Val Ile Lys Asp
    50                  55                  60

ctt ttc cct aat gaa aat gag ttg gtg gtt aag agt caa att tta gcc 240
Leu Phe Pro Asn Glu Asn Glu Leu Val Val Lys Ser Gln Ile Leu Ala
65                  70                  75                  80

ggc gga cga ggt ttg gga act ttc aaa agc ggc ctt aag gga gga gtg 288
Gly Gly Arg Gly Leu Gly Thr Phe Lys Ser Gly Leu Lys Gly Gly Val
                85                  90                  95

cac att gtt aag act gac cag gtt gaa gac ata gct ggg aag atg ctt 336
His Ile Val Lys Thr Asp Gln Val Glu Asp Ile Ala Gly Lys Met Leu
            100                 105                 110

gga cag ata cta gtt acc aaa cag act ggt cct cag gga aaa ata gtt 384
Gly Gln Ile Leu Val Thr Lys Gln Thr Gly Pro Gln Gly Lys Ile Val
        115                 120                 125

agc aag gtt tac ttg tgt gaa aag ctg tca ctt gta aat gag atg tac 432
Ser Lys Val Tyr Leu Cys Glu Lys Leu Ser Leu Val Asn Glu Met Tyr
    130                 135                 140

ttt gct ata aca ctg gat cgt acg agt gct ggt cca att ata att gct 480
Phe Ala Ile Thr Leu Asp Arg Thr Ser Ala Gly Pro Ile Ile Ile Ala
145                 150                 155                 160

tgt agt aag gga gga acc agc att gaa gac ctt gca gag aaa ttt ccg 528
Cys Ser Lys Gly Gly Thr Ser Ile Glu Asp Leu Ala Glu Lys Phe Pro
                165                 170                 175

gac atg att ata aag gta cct gtt gat gtt ttt gaa ggt att act gat 576
Asp Met Ile Ile Lys Val Pro Val Asp Val Phe Glu Gly Ile Thr Asp
            180                 185                 190

gaa ggt gct gca aag gtt gtt gat ggt ttg gct ctc aaa gtg gct gat 624
Glu Gly Ala Ala Lys Val Val Asp Gly Leu Ala Leu Lys Val Ala Asp
        195                 200                 205

aga aat aag tcg att gaa caa gtg aag aat ttg tat aaa ctt ttt gtt 672
Arg Asn Lys Ser Ile Glu Gln Val Lys Asn Leu Tyr Lys Leu Phe Val
    210                 215                 220

gat tgt gac tgc act ctt ttg gaa atc aat ccc ata gcg gag aca gct 720

Asp Cys Asp Cys Thr Leu Leu Glu Ile Asn Pro Ile Ala Glu Thr Ala
225                 230                 235                 240

gat aac caa ttg gtg gct gct gat gct aag ttg aat ttt gat gat aat      768
Asp Asn Gln Leu Val Ala Ala Asp Ala Lys Leu Asn Phe Asp Asp Asn
                245                 250                 255

gct gca tat cgc cag aaa gag ata ttt tct ctc cgt gat aca aca caa      816
Ala Ala Tyr Arg Gln Lys Glu Ile Phe Ser Leu Arg Asp Thr Thr Gln
            260                 265                 270

gag gac cct cga gag gtg act gct gca aag gca gat tta aat tat att      864
Glu Asp Pro Arg Glu Val Thr Ala Ala Lys Ala Asp Leu Asn Tyr Ile
        275                 280                 285

ggg tta gat gga gaa att ggc tgc atg gtg aat gga gca ggg tta gca      912
Gly Leu Asp Gly Glu Ile Gly Cys Met Val Asn Gly Ala Gly Leu Ala
    290                 295                 300

atg gcc aca atg gat ata att aaa ttg cat ggg ggt act cct gcc aat      960
Met Ala Thr Met Asp Ile Ile Lys Leu His Gly Gly Thr Pro Ala Asn
305                 310                 315                 320

ttt ctg gat gta ggt ggc aat gcc tct gaa aac cag gtt gtt gag gca     1008
Phe Leu Asp Val Gly Gly Asn Ala Ser Glu Asn Gln Val Val Glu Ala
                325                 330                 335

ttt aaa ata ttg act gct gat gac aaa gtg aag gca att ttg gtt aac     1056
Phe Lys Ile Leu Thr Ala Asp Asp Lys Val Lys Ala Ile Leu Val Asn
            340                 345                 350

att ttt ggt ggt ata atg aag tgt gat gtt ata gca agt gga ata gtg     1104
Ile Phe Gly Gly Ile Met Lys Cys Asp Val Ile Ala Ser Gly Ile Val
        355                 360                 365

aat gct gca aaa gag gtt gca ctg aaa gta cca gtt gta gtt cgt ctg     1152
Asn Ala Ala Lys Glu Val Ala Leu Lys Val Pro Val Val Val Arg Leu
    370                 375                 380

gaa ggc acc aat gtt gat caa gga aaa aga ata ttg aag gaa agt ggt     1200
Glu Gly Thr Asn Val Asp Gln Gly Lys Arg Ile Leu Lys Glu Ser Gly
385                 390                 395                 400

atg gca tta ata acg gct gaa gat tta gat gat gca gct cag aaa gca     1248
Met Ala Leu Ile Thr Ala Glu Asp Leu Asp Asp Ala Ala Gln Lys Ala
                405                 410                 415

gtg aaa gct gct tac aaa                                             1266
Val Lys Ala Ala Tyr Lys
            420


<210>  348
<211>  422
<212>  PRT
<213>  Glycine max

<400>  348

Met Val Arg Gly Leu Leu Asn Lys Leu Val Ser Arg Ser Leu Ser Val
1               5                   10                  15

Ala Gly Lys Trp Gln His Asn Gln Leu Arg Arg Leu Asn Ile His Glu
            20                  25                  30
Tyr Gln Gly Ala Glu Leu Met Ser Lys His Gly Val Asn Val Pro Arg
        35                  40                  45
Gly Val Ala Val Ser Ser Val Glu Glu Ala Arg Lys Val Ile Lys Asp
    50                  55                  60
Leu Phe Pro Asn Glu Asn Glu Leu Val Val Lys Ser Gln Ile Leu Ala
65                  70                  75                  80
Gly Gly Arg Gly Leu Gly Thr Phe Lys Ser Gly Leu Lys Gly Gly Val
                85                  90                  95
His Ile Val Lys Thr Asp Gln Val Glu Asp Ile Ala Gly Lys Met Leu
            100                 105                 110
Gly Gln Ile Leu Val Thr Lys Gln Thr Gly Pro Gln Gly Lys Ile Val
        115                 120                 125
Ser Lys Val Tyr Leu Cys Glu Lys Leu Ser Leu Val Asn Glu Met Tyr
    130                 135                 140
Phe Ala Ile Thr Leu Asp Arg Thr Ser Ala Gly Pro Ile Ile Ile Ala
145                 150                 155                 160
Cys Ser Lys Gly Gly Thr Ser Ile Glu Asp Leu Ala Glu Lys Phe Pro
                165                 170                 175
Asp Met Ile Ile Lys Val Pro Val Asp Val Phe Glu Gly Ile Thr Asp
            180                 185                 190
Glu Gly Ala Ala Lys Val Val Asp Gly Leu Ala Leu Lys Val Ala Asp
        195                 200                 205
Arg Asn Lys Ser Ile Glu Gln Val Lys Asn Leu Tyr Lys Leu Phe Val
    210                 215                 220
Asp Cys Asp Cys Thr Leu Leu Glu Ile Asn Pro Ile Ala Glu Thr Ala
225                 230                 235                 240
Asp Asn Gln Leu Val Ala Ala Asp Ala Lys Leu Asn Phe Asp Asp Asn
                245                 250                 255
Ala Ala Tyr Arg Gln Lys Glu Ile Phe Ser Leu Arg Asp Thr Thr Gln
            260                 265                 270

Glu Asp Pro Arg Glu Val Thr Ala Ala Lys Ala Asp Leu Asn Tyr Ile
        275                 280                 285

Gly Leu Asp Gly Glu Ile Gly Cys Met Val Asn Gly Ala Gly Leu Ala
    290                 295                 300

Met Ala Thr Met Asp Ile Ile Lys Leu His Gly Gly Thr Pro Ala Asn
305                 310                 315                 320

Phe Leu Asp Val Gly Gly Asn Ala Ser Glu Asn Gln Val Val Glu Ala
                325                 330                 335

Phe Lys Ile Leu Thr Ala Asp Asp Lys Val Lys Ala Ile Leu Val Asn
            340                 345                 350

Ile Phe Gly Gly Ile Met Lys Cys Asp Val Ile Ala Ser Gly Ile Val
        355                 360                 365

Asn Ala Ala Lys Glu Val Ala Leu Lys Val Pro Val Val Val Arg Leu
    370                 375                 380

Glu Gly Thr Asn Val Asp Gln Gly Lys Arg Ile Leu Lys Glu Ser Gly
385                 390                 395                 400

Met Ala Leu Ile Thr Ala Glu Asp Leu Asp Asp Ala Ala Gln Lys Ala
                405                 410                 415

Val Lys Ala Ala Tyr Lys
            420

<210>  349
<211>  12
<212>  PRT
<213>  Glycine max

<400>  349

Asp Leu Phe Pro Asn Glu Asn Glu Leu Val Val Lys
1               5                   10

<210>  350
<211>  20
<212>  PRT
<213>  Glycine max

<400>  350

Glu Ile Phe Ser Leu Arg Asp Thr Thr Gln Glu Asp Pro Arg Glu Val
1               5                   10                  15

Thr Ala Ala Lys
            20

<210>  351
<211>  26
<212>  PRT
<213>  Glycine max

<400>  351

Leu His Gly Gly Thr Pro Ala Asn Phe Leu Asp Val Gly Gly Asn Ala
1               5                   10                  15

Ser Glu Asn Gln Val Val Glu Ala Phe Lys
            20                  25

<210>  352
<211>  12
<212>  PRT
<213>  Glycine max

<400>  352

Leu Asn Phe Asp Asp Asn Ala Ala Tyr Arg Gln Lys
1               5                   10

<210>  353
<211>  14
<212>  PRT
<213>  Glycine max

<400>  353

Ser Gln Ile Leu Ala Gly Gly Arg Gly Leu Gly Thr Phe Lys
1               5                   10

<210>  354
<211>  10
<212>  PRT
<213>  Glycine max

<400>  354

Thr Asp Gln Val Glu Asp Ile Ala Gly Lys
1               5                   10

<210>  355
<211>  16
<212>  PRT
<213>  Glycine max

<400>  355

Val Pro Val Val Val Arg Leu Glu Gly Thr Asn Val Asp Gln Gly Lys
1               5                   10                  15

<210>  356
<211>  8
<212>  PRT
<213>  Glycine max

<400>  356

Val Val Asp Gly Leu Ala Leu Lys
1               5


<210>  357
<211>  1266
<212>  DNA
<213>  Glycine max


<220>
<221>  CDS
<222>  (1)..(1266)

<400>  357
atg gtg agg gga ttg ctc aac aag ctc gtc tct cgc tcc ctc tcc gtc      48
Met Val Arg Gly Leu Leu Asn Lys Leu Val Ser Arg Ser Leu Ser Val
1               5                   10                  15

gcc gga aaa tgg cag cac aac caa ctc cgc cgc ctc aac atc cac gag      96
Ala Gly Lys Trp Gln His Asn Gln Leu Arg Arg Leu Asn Ile His Glu
            20                  25                  30

tac cag ggc gct gaa ttg atg agc aaa cac gga gtt aat gtt ccg aga      144
Tyr Gln Gly Ala Glu Leu Met Ser Lys His Gly Val Asn Val Pro Arg
        35                  40                  45

ggc gtg gct gtt tcc tct gtt gaa gag gtc aga aag gcc atc aag gac      192
Gly Val Ala Val Ser Ser Val Glu Glu Val Arg Lys Ala Ile Lys Asp
    50                  55                  60

ctt ttc cct aat gaa aat gag ttg gtg gtt aag agt caa att tta gcc      240
Leu Phe Pro Asn Glu Asn Glu Leu Val Val Lys Ser Gln Ile Leu Ala
65                  70                  75                  80

ggt gga aga ggt ttg gga act ttc aaa agc ggc ctt aag gga gga gtg      288
Gly Gly Arg Gly Leu Gly Thr Phe Lys Ser Gly Leu Lys Gly Gly Val
                85                  90                  95

cac att gtt aag act gac cag gtt gaa gac ata gct ggg aag atg ctt      336
His Ile Val Lys Thr Asp Gln Val Glu Asp Ile Ala Gly Lys Met Leu
            100                 105                 110

ggg cag ata cta gtt acc aaa cag act ggt cct cag gga aaa ata gtt      384
Gly Gln Ile Leu Val Thr Lys Gln Thr Gly Pro Gln Gly Lys Ile Val
        115                 120                 125

agc aag gtt tac ttg tgt gaa aag ctg tca cta gta aat gag atg tac      432
Ser Lys Val Tyr Leu Cys Glu Lys Leu Ser Leu Val Asn Glu Met Tyr
    130                 135                 140

ttt gct ata aca ctg gat cgt acg agt gct ggt cca att ata att gct      480
Phe Ala Ile Thr Leu Asp Arg Thr Ser Ala Gly Pro Ile Ile Ile Ala
145                 150                 155                 160

tgt agt aag gga gga acc agc att gaa gac cta gca gag aaa ttt ccg      528

Cys Ser Lys Gly Gly Thr Ser Ile Glu Asp Leu Ala Glu Lys Phe Pro
                165                 170                 175

gac atg att ata aag gta cct gtt gat gtt ttt gaa gga att act gat      576
Asp Met Ile Ile Lys Val Pro Val Asp Val Phe Glu Gly Ile Thr Asp
            180                 185                 190

gaa gat gct gca aag gtt gtt gat ggt ttg gct ctc aaa gtg gct gat      624
Glu Asp Ala Ala Lys Val Val Asp Gly Leu Ala Leu Lys Val Ala Asp
        195                 200                 205

aga aat aag tcg att gaa caa gtg aag aat ttg tat aaa ctt ttt gtt      672
Arg Asn Lys Ser Ile Glu Gln Val Lys Asn Leu Tyr Lys Leu Phe Val
    210                 215                 220

gat tgt gac tgc acc ctt ttg gaa atc aat ccc att gcg gag aca gct      720
Asp Cys Asp Cys Thr Leu Leu Glu Ile Asn Pro Ile Ala Glu Thr Ala
225                 230                 235                 240

gat aac caa ttg gtg gct gct gat gct aag ttg aat ttt gac gat aat      768
Asp Asn Gln Leu Val Ala Ala Asp Ala Lys Leu Asn Phe Asp Asp Asn
                245                 250                 255

gct gca tat cgc cag aaa gag ata ttt gct ctc cgt gat aca aca caa      816
Ala Ala Tyr Arg Gln Lys Glu Ile Phe Ala Leu Arg Asp Thr Thr Gln
            260                 265                 270

gag gac cct cga gag gtg act gct gca aag gca gac tta aat tat att      864
Glu Asp Pro Arg Glu Val Thr Ala Ala Lys Ala Asp Leu Asn Tyr Ile
        275                 280                 285

ggg tta gat gga gaa ata ggc tgc atg gtg aat gga gca ggg tta gca      912
Gly Leu Asp Gly Glu Ile Gly Cys Met Val Asn Gly Ala Gly Leu Ala
    290                 295                 300

atg gcc aca atg gat ata att aaa ttg cat ggg ggt act cct gcc aat      960
Met Ala Thr Met Asp Ile Ile Lys Leu His Gly Gly Thr Pro Ala Asn
305                 310                 315                 320

ttt ttg gat gta ggt ggc aat gcc tct gaa ggc cag gtg gtt gag gca     1008
Phe Leu Asp Val Gly Gly Asn Ala Ser Glu Gly Gln Val Val Glu Ala
                325                 330                 335

ttt aaa ata ttg act gct gat gac aaa gtg aag gca att ttg gtt aac     1056
Phe Lys Ile Leu Thr Ala Asp Asp Lys Val Lys Ala Ile Leu Val Asn
            340                 345                 350

att ttt ggg ggt ata atg aag tgt gat gtt ata gca agt gga ata gtg     1104
Ile Phe Gly Gly Ile Met Lys Cys Asp Val Ile Ala Ser Gly Ile Val
        355                 360                 365

aat gct gca aaa gag gtt gca ctg aaa gta ccc gta gta gtt cgt ctg     1152
Asn Ala Ala Lys Glu Val Ala Leu Lys Val Pro Val Val Val Arg Leu
    370                 375                 380

gaa ggc acc aat gtt gat cat gga aaa aga att ttg aag gaa agt ggt     1200
Glu Gly Thr Asn Val Asp His Gly Lys Arg Ile Leu Lys Glu Ser Gly
385                 390                 395                 400

atg gcg ttg ata acg gct gaa gat tta gat gat gct gct cag aaa gca     1248
Met Ala Leu Ile Thr Ala Glu Asp Leu Asp Asp Ala Ala Gln Lys Ala
                405                 410                 415

gtg aaa gct gct tac aaa                                                    1266
Val Lys Ala Ala Tyr Lys
                420

<210>  358
<211>  422
<212>  PRT
<213>  Glycine max

<400>  358

Met Val Arg Gly Leu Leu Asn Lys Leu Val Ser Arg Ser Leu Ser Val
1               5                   10                  15

Ala Gly Lys Trp Gln His Asn Gln Leu Arg Arg Leu Asn Ile His Glu
            20                  25                  30

Tyr Gln Gly Ala Glu Leu Met Ser Lys His Gly Val Asn Val Pro Arg
        35                  40                  45

Gly Val Ala Val Ser Ser Val Glu Glu Val Arg Lys Ala Ile Lys Asp
    50                  55                  60

Leu Phe Pro Asn Glu Asn Glu Leu Val Val Lys Ser Gln Ile Leu Ala
65                  70                  75                  80

Gly Gly Arg Gly Leu Gly Thr Phe Lys Ser Gly Leu Lys Gly Gly Val
                85                  90                  95

His Ile Val Lys Thr Asp Gln Val Glu Asp Ile Ala Gly Lys Met Leu
            100                 105                 110

Gly Gln Ile Leu Val Thr Lys Gln Thr Gly Pro Gln Gly Lys Ile Val
        115                 120                 125

Ser Lys Val Tyr Leu Cys Glu Lys Leu Ser Leu Val Asn Glu Met Tyr
    130                 135                 140

Phe Ala Ile Thr Leu Asp Arg Thr Ser Ala Gly Pro Ile Ile Ile Ala
145                 150                 155                 160

Cys Ser Lys Gly Gly Thr Ser Ile Glu Asp Leu Ala Glu Lys Phe Pro
                165                 170                 175

Asp Met Ile Ile Lys Val Pro Val Asp Val Phe Glu Gly Ile Thr Asp
            180                 185                 190

Glu Asp Ala Ala Lys Val Val Asp Gly Leu Ala Leu Lys Val Ala Asp
        195                 200                 205

Arg Asn Lys Ser Ile Glu Gln Val Lys Asn Leu Tyr Lys Leu Phe Val
    210                 215                 220

Asp Cys Asp Cys Thr Leu Leu Glu Ile Asn Pro Ile Ala Glu Thr Ala
225                 230                 235                 240

Asp Asn Gln Leu Val Ala Ala Asp Ala Lys Leu Asn Phe Asp Asp Asn
                245                 250                 255

Ala Ala Tyr Arg Gln Lys Glu Ile Phe Ala Leu Arg Asp Thr Thr Gln
            260                 265                 270

Glu Asp Pro Arg Glu Val Thr Ala Ala Lys Ala Asp Leu Asn Tyr Ile
        275                 280                 285

Gly Leu Asp Gly Glu Ile Gly Cys Met Val Asn Gly Ala Gly Leu Ala
    290                 295                 300

Met Ala Thr Met Asp Ile Ile Lys Leu His Gly Gly Thr Pro Ala Asn
305                 310                 315                 320

Phe Leu Asp Val Gly Gly Asn Ala Ser Glu Gly Gln Val Val Glu Ala
                325                 330                 335

Phe Lys Ile Leu Thr Ala Asp Asp Lys Val Lys Ala Ile Leu Val Asn
            340                 345                 350

Ile Phe Gly Gly Ile Met Lys Cys Asp Val Ile Ala Ser Gly Ile Val
        355                 360                 365

Asn Ala Ala Lys Glu Val Ala Leu Lys Val Pro Val Val Val Arg Leu
    370                 375                 380

Glu Gly Thr Asn Val Asp His Gly Lys Arg Ile Leu Lys Glu Ser Gly
385                 390                 395                 400

Met Ala Leu Ile Thr Ala Glu Asp Leu Asp Asp Ala Ala Gln Lys Ala
                405                 410                 415

Val Lys Ala Ala Tyr Lys
            420

<210>  359
<211>  12
<212>  PRT
<213>  Glycine max

<400>  359

Asp Leu Phe Pro Asn Glu Asn Glu Leu Val Val Lys
1               5                   10

<210>  360
<211>  20
<212>  PRT
<213>  Glycine max

<400>  360

Glu Ile Phe Ala Leu Arg Asp Thr Thr Gln Glu Asp Pro Arg Glu Val
1               5                   10                  15

Thr Ala Ala Lys
            20

<210>  361
<211>  26
<212>  PRT
<213>  Glycine max

<400>  361

Leu His Gly Gly Thr Pro Ala Asn Phe Leu Asp Val Gly Gly Asn Ala
1               5                   10                  15

Ser Glu Gly Gln Val Val Glu Ala Phe Lys
            20                  25

<210>  362
<211>  12
<212>  PRT
<213>  Glycine max

<400>  362

Leu Asn Phe Asp Asp Asn Ala Ala Tyr Arg Gln Lys
1               5                   10

<210>  363
<211>  14
<212>  PRT
<213>  Glycine max

<400>  363

Ser Gln Ile Leu Ala Gly Gly Arg Gly Leu Gly Thr Phe Lys
1               5                   10

<210>  364
<211>  10
<212>  PRT
<213>  Glycine max

<400>  364

Thr Asp Gln Val Glu Asp Ile Ala Gly Lys
1               5                   10

<210>  365
<211>  8
<212>  PRT
<213>  Glycine max

<400>  365

Val Val Asp Gly Leu Ala Leu Lys
1               5

<210>  366
<211>  1203
<212>  DNA
<213>  Glycine max

<220>
<221>  CDS
<222>  (1)..(1203)

<400>  366
atg gcg act aag agg agc gtg gga acg ttg aag gag ggt gat ttg aag      48
Met Ala Thr Lys Arg Ser Val Gly Thr Leu Lys Glu Gly Asp Leu Lys
1               5                   10                  15

ggg aag agg gta ttc gtg agg gtc gat ctg aac gtg cct ttg gat gac      96
Gly Lys Arg Val Phe Val Arg Val Asp Leu Asn Val Pro Leu Asp Asp
            20                  25                  30

aac ctt aac atc acc gat gac act aga gtc cgt gct gct gtt ccc acc      144
Asn Leu Asn Ile Thr Asp Asp Thr Arg Val Arg Ala Ala Val Pro Thr
        35                  40                  45

atc aag tac ttg act ggt tat ggt gct aaa gtg atc ctt tct agc cat      192
Ile Lys Tyr Leu Thr Gly Tyr Gly Ala Lys Val Ile Leu Ser Ser His
    50                  55                  60

ttg gga cgt cca aaa ggt gta aca cct aaa tac agt ttg aag cct ctt      240
Leu Gly Arg Pro Lys Gly Val Thr Pro Lys Tyr Ser Leu Lys Pro Leu
65                  70                  75                  80

gtg cca agg ctg tct caa ctt ctt gga att gag gtt acg atg gcc aat      288
Val Pro Arg Leu Ser Gln Leu Leu Gly Ile Glu Val Thr Met Ala Asn
                85                  90                  95

gac tct att ggg gag gaa gtt gaa aag ttg gtt aca caa ctt cca gaa      336
Asp Ser Ile Gly Glu Glu Val Glu Lys Leu Val Thr Gln Leu Pro Glu
            100                 105                 110

ggt ggt gtt ttg ctt cta gag aat gtg agg ttc tac aag gag gaa gag      384
Gly Gly Val Leu Leu Leu Glu Asn Val Arg Phe Tyr Lys Glu Glu Glu
        115                 120                 125

aag aat gat cct gaa ttt gca aag aag ttg gct tct cta gct gat ctc      432
Lys Asn Asp Pro Glu Phe Ala Lys Lys Leu Ala Ser Leu Ala Asp Leu
    130                 135                 140

tat gtg aat gat gca ttt ggc act gcc cac aga gct cat gct tct aca 480
Tyr Val Asn Asp Ala Phe Gly Thr Ala His Arg Ala His Ala Ser Thr
145                 150                 155                 160

gaa gga gtt gcc aaa tac ttg aag ccc tct gtt gca gga ttc cta atg 528
Glu Gly Val Ala Lys Tyr Leu Lys Pro Ser Val Ala Gly Phe Leu Met
                165                 170                 175

cag aag gag ctt gat tat cta gtt ggg gct gtg tca aac ccc aag aga 576
Gln Lys Glu Leu Asp Tyr Leu Val Gly Ala Val Ser Asn Pro Lys Arg
            180                 185                 190

cca ttt gct gct att gtc ggt gga tcg aag gtg tct tcc aag att gga 624
Pro Phe Ala Ala Ile Val Gly Gly Ser Lys Val Ser Ser Lys Ile Gly
        195                 200                 205

gtt att gaa tcc ttg ttg gag aaa gtt aat gtt ctc ttg ctt ggt gga 672
Val Ile Glu Ser Leu Leu Glu Lys Val Asn Val Leu Leu Leu Gly Gly
    210                 215                 220

gga atg atc ttt acc ttt tac aag gct cag ggt tat tca gtt ggg tca 720
Gly Met Ile Phe Thr Phe Tyr Lys Ala Gln Gly Tyr Ser Val Gly Ser
225                 230                 235                 240

tct ctt gtg gag gaa gac aag ctt agc ctt gca act aca ctt ctt gaa 768
Ser Leu Val Glu Glu Asp Lys Leu Ser Leu Ala Thr Thr Leu Leu Glu
                245                 250                 255

aag gcc aag gct aaa ggg gtt tct ttg ttg ctt cca act gat gtt gtc 816
Lys Ala Lys Ala Lys Gly Val Ser Leu Leu Leu Pro Thr Asp Val Val
            260                 265                 270

ata gca gac aag ttt gct gct gat gct aac agc aag act gtg ccg gca 864
Ile Ala Asp Lys Phe Ala Ala Asp Ala Asn Ser Lys Thr Val Pro Ala
        275                 280                 285

tca agc atc cca gat ggg tgg atg ggg ttg gat att ggt cct gat tcc 912
Ser Ser Ile Pro Asp Gly Trp Met Gly Leu Asp Ile Gly Pro Asp Ser
    290                 295                 300

atc aag aca ttt ggt gaa gca ttg gat acg act cag acc att att tgg 960
Ile Lys Thr Phe Gly Glu Ala Leu Asp Thr Thr Gln Thr Ile Ile Trp
305                 310                 315                 320

aat gga cca atg ggt gtt ttt gag ttt gat aag ttt gcc aca gga aca 1008
Asn Gly Pro Met Gly Val Phe Glu Phe Asp Lys Phe Ala Thr Gly Thr
                325                 330                 335

gag gct ata gcc aag aaa ctg gca gag ctg agt ggg aag gga gtg aca 1056
Glu Ala Ile Ala Lys Lys Leu Ala Glu Leu Ser Gly Lys Gly Val Thr
            340                 345                 350

acc atc att gga gga ggt gac tcc gtt gcc gct gtg gag aag gtt gga 1104
Thr Ile Ile Gly Gly Gly Asp Ser Val Ala Ala Val Glu Lys Val Gly
        355                 360                 365

ctt gca gac aag atg agc cac atc tcc act ggt ggt ggt gcc agc tta 1152
Leu Ala Asp Lys Met Ser His Ile Ser Thr Gly Gly Gly Ala Ser Leu
    370                 375                 380

gag ctt ctt gag gga aag caa ctc cct ggt gtc ctt gct ctt gat gat 1200
Glu Leu Leu Glu Gly Lys Gln Leu Pro Gly Val Leu Ala Leu Asp Asp
385                 390                 395                 400

gct                                                                 1203
Ala

<210>  367
<211>  401
<212>  PRT
<213>  Glycine max

<400>  367

Met Ala Thr Lys Arg Ser Val Gly Thr Leu Lys Glu Gly Asp Leu Lys
1               5                   10                  15

Gly Lys Arg Val Phe Val Arg Val Asp Leu Asn Val Pro Leu Asp Asp
            20                  25                  30

Asn Leu Asn Ile Thr Asp Asp Thr Arg Val Arg Ala Ala Val Pro Thr
        35                  40                  45

Ile Lys Tyr Leu Thr Gly Tyr Gly Ala Lys Val Ile Leu Ser Ser His
    50                  55                  60

Leu Gly Arg Pro Lys Gly Val Thr Pro Lys Tyr Ser Leu Lys Pro Leu
65                  70                  75                  80

Val Pro Arg Leu Ser Gln Leu Leu Gly Ile Glu Val Thr Met Ala Asn
                85                  90                  95

Asp Ser Ile Gly Glu Glu Val Glu Lys Leu Val Thr Gln Leu Pro Glu
            100                 105                 110

Gly Gly Val Leu Leu Leu Glu Asn Val Arg Phe Tyr Lys Glu Glu Glu
        115                 120                 125

Lys Asn Asp Pro Glu Phe Ala Lys Lys Leu Ala Ser Leu Ala Asp Leu
    130                 135                 140

Tyr Val Asn Asp Ala Phe Gly Thr Ala His Arg Ala His Ala Ser Thr
145                 150                 155                 160

Glu Gly Val Ala Lys Tyr Leu Lys Pro Ser Val Ala Gly Phe Leu Met
                165                 170                 175

Gln Lys Glu Leu Asp Tyr Leu Val Gly Ala Val Ser Asn Pro Lys Arg
            180                 185                 190

Pro Phe Ala Ala Ile Val Gly Gly Ser Lys Val Ser Ser Lys Ile Gly
        195                 200                 205

Val Ile Glu Ser Leu Leu Glu Lys Val Asn Val Leu Leu Leu Gly Gly
    210                 215                 220

Gly Met Ile Phe Thr Phe Tyr Lys Ala Gln Gly Tyr Ser Val Gly Ser
225                 230                 235                 240

Ser Leu Val Glu Glu Asp Lys Leu Ser Leu Ala Thr Thr Leu Leu Glu
                245                 250                 255

Lys Ala Lys Ala Lys Gly Val Ser Leu Leu Leu Pro Thr Asp Val Val
            260                 265                 270

Ile Ala Asp Lys Phe Ala Ala Asp Ala Asn Ser Lys Thr Val Pro Ala
        275                 280                 285

Ser Ser Ile Pro Asp Gly Trp Met Gly Leu Asp Ile Gly Pro Asp Ser
    290                 295                 300

Ile Lys Thr Phe Gly Glu Ala Leu Asp Thr Thr Gln Thr Ile Ile Trp
305                 310                 315                 320

Asn Gly Pro Met Gly Val Phe Glu Phe Asp Lys Phe Ala Thr Gly Thr
                325                 330                 335

Glu Ala Ile Ala Lys Lys Leu Ala Glu Leu Ser Gly Lys Gly Val Thr
            340                 345                 350

Thr Ile Ile Gly Gly Gly Asp Ser Val Ala Ala Val Glu Lys Val Gly
        355                 360                 365

Leu Ala Asp Lys Met Ser His Ile Ser Thr Gly Gly Gly Ala Ser Leu
    370                 375                 380

Glu Leu Leu Glu Gly Lys Gln Leu Pro Gly Val Leu Ala Leu Asp Asp
385                 390                 395                 400

Ala

<210>  368
<211>  15
<212>  PRT
<213>  Glycine max

<400>  368

Ala Gln Gly Tyr Ser Val Gly Ser Ser Leu Val Glu Glu Asp Lys
1               5                   10                  15

<210>  369
<211>  13
<212>  PRT
<213>  Glycine max

<400>  369

Glu Leu Asp Tyr Leu Val Gly Ala Val Ser Asn Pro Lys
1               5                   10


<210>  370
<211>  8
<212>  PRT
<213>  Glycine max

<400>  370

Phe Ala Ala Asp Ala Asn Ser Lys
1               5


<210>  371
<211>  10
<212>  PRT
<213>  Glycine max

<400>  371

Phe Ala Thr Gly Thr Glu Ala Ile Ala Lys
1               5                   10


<210>  372
<211>  15
<212>  PRT
<213>  Glycine max

<400>  372

Gly Val Ser Leu Leu Leu Pro Thr Asp Val Val Ile Ala Asp Lys
1               5                   10                  15


<210>  373
<211>  17
<212>  PRT
<213>  Glycine max

<400>  373

Gly Val Thr Thr Ile Ile Gly Gly Gly Asp Ser Val Ala Ala Val Glu
1               5                   10                  15


Lys


<210>  374
<211>  10

<212>  PRT
<213>  Glycine max

<400>  374

Ile Gly Val Ile Glu Ser Leu Leu Glu Lys
1               5                   10


<210>  375
<211>  28
<212>  PRT
<213>  Glycine max

<400>  375

Leu Ala Ser Leu Ala Asp Leu Tyr Val Asn Asp Ala Phe Gly Thr Ala
1               5                   10                  15


His Arg Ala His Ala Ser Thr Glu Gly Val Ala Lys
            20                  25


<210>  376
<211>  10
<212>  PRT
<213>  Glycine max

<400>  376

Leu Ser Leu Ala Thr Thr Leu Leu Glu Lys
1               5                   10


<210>  377
<211>  20
<212>  PRT
<213>  Glycine max

<400>  377

Leu Val Thr Gln Leu Pro Glu Gly Gly Val Leu Leu Leu Glu Asn Val
1               5                   10                  15


Arg Phe Tyr Lys
            20


<210>  378
<211>  7
<212>  PRT
<213>  Glycine max

<400>  378

Asn Asp Pro Glu Phe Ala Lys
1               5


<210>  379
<211>  11

<212>  PRT
<213>  Glycine max

<400>  379

Gln Leu Pro Gly Val Leu Ala Leu Asp Asp Ala
1               5                   10


<210>  380
<211>  11
<212>  PRT
<213>  Glycine max

<400>  380

Arg Pro Phe Ala Ala Ile Val Gly Gly Ser Lys
1               5                   10


<210>  381
<211>  11
<212>  PRT
<213>  Glycine max

<400>  381

Val Ile Leu Ser Ser His Leu Gly Arg Pro Lys
1               5                   10


<210>  382
<211>  8
<212>  PRT
<213>  Glycine max

<400>  382

Tyr Leu Thr Gly Tyr Gly Ala Lys
1               5


<210>  383
<211>  1455
<212>  DNA
<213>  Glycine max


<220>
<221>  CDS
<222>  (1)..(1455)

<400>  383
atg gcc tca gcc gca tcc ccc acc act ttc tct ctc ctc cac tcc gcc          48
Met Ala Ser Ala Ala Ser Pro Thr Thr Phe Ser Leu Leu His Ser Ala
1               5                   10                  15

acc cct tcc tcc tcc tcc acc gcc acc tcc gcc tcc ccc tcc ccc tcg          96
Thr Pro Ser Ser Ser Ser Thr Ala Thr Ser Ala Ser Pro Ser Pro Ser
            20                  25                  30

cgc gtg cag ctc acc tcc tcc ctc cgc gcc cgc ccg ctg cgc cgc ctg          144
Arg Val Gln Leu Thr Ser Ser Leu Arg Ala Arg Pro Leu Arg Arg Leu

35                  40                  45

ggc ttc gcc gcg gcg gac ccg ctg ctc gcg gcg agt gta gcg gcg cgc      192
Gly Phe Ala Ala Ala Asp Pro Leu Leu Ala Ala Ser Val Ala Ala Arg
    50                  55                  60

gtg gcg gcg gtg aga gga gga gga aag ggc gcg agg ggg gtg gtg tcg      240
Val Ala Ala Val Arg Gly Gly Gly Lys Gly Ala Arg Gly Val Val Ser
65                  70                  75                  80

atg gcg aag aag agc gtg ggg gac ctg agc gcg gcg gac ctg aag ggg      288
Met Ala Lys Lys Ser Val Gly Asp Leu Ser Ala Ala Asp Leu Lys Gly
                85                  90                  95

aag aag gtc ttc gtc agg gcc gac ctc aac gtc ccg ctc gac gac aac      336
Lys Lys Val Phe Val Arg Ala Asp Leu Asn Val Pro Leu Asp Asp Asn
            100                 105                 110

cag aac atc acc gac gac acc aga atc cgc gcc gcc att ccc acc atc      384
Gln Asn Ile Thr Asp Asp Thr Arg Ile Arg Ala Ala Ile Pro Thr Ile
        115                 120                 125

aaa cat ctc atc caa aac ggc gcc aaa gtc att ctc tcc agc cac ttg      432
Lys His Leu Ile Gln Asn Gly Ala Lys Val Ile Leu Ser Ser His Leu
    130                 135                 140

ggg cgg ccg aag ggt gtc act ccc aaa tac agc ttg gcg cct ctt gta      480
Gly Arg Pro Lys Gly Val Thr Pro Lys Tyr Ser Leu Ala Pro Leu Val
145                 150                 155                 160

ccc cgg ctt tct gag ctc att ggt atc cag att gtc aag gcc gag gac      528
Pro Arg Leu Ser Glu Leu Ile Gly Ile Gln Ile Val Lys Ala Glu Asp
                165                 170                 175

agt att ggt cca gaa gta gaa aag ttg gtg gct tct ctt cca gat gga      576
Ser Ile Gly Pro Glu Val Glu Lys Leu Val Ala Ser Leu Pro Asp Gly
            180                 185                 190

ggt gtt ctc ctt cta gaa aat gtg agg ttt tac aag gag gaa gaa aag      624
Gly Val Leu Leu Leu Glu Asn Val Arg Phe Tyr Lys Glu Glu Glu Lys
        195                 200                 205

aat gac ccc gag cat gca aaa aag ctt gcc tct ctg gca gat ctg tat      672
Asn Asp Pro Glu His Ala Lys Lys Leu Ala Ser Leu Ala Asp Leu Tyr
    210                 215                 220

gtg aat gat gca ttt ggt act gct cac agg gca cat gca tca aca gag      720
Val Asn Asp Ala Phe Gly Thr Ala His Arg Ala His Ala Ser Thr Glu
225                 230                 235                 240

ggt gtt act aaa tac ctg aag cca tct gtt gct ggt ttt ctt ttg caa      768
Gly Val Thr Lys Tyr Leu Lys Pro Ser Val Ala Gly Phe Leu Leu Gln
                245                 250                 255

aag gaa ctt gat tac ctt gtt ggg gca gta tca aac ccc aaa agg cca      816
Lys Glu Leu Asp Tyr Leu Val Gly Ala Val Ser Asn Pro Lys Arg Pro
            260                 265                 270

ttt gct gcc att gtt ggt ggt tcc aaa gtc tca tct aaa att gga gtg      864
Phe Ala Ala Ile Val Gly Gly Ser Lys Val Ser Ser Lys Ile Gly Val
        275                 280                 285

att gag tca ctt tta gaa aaa gtt gac atc ctt ctt ctt ggt gga gga      912

Ile Glu Ser Leu Leu Glu Lys Val Asp Ile Leu Leu Leu Gly Gly Gly
    290                 295                 300

atg atc ttc aca ttt tac aag gca caa ggt ctt tca gtg ggt tca tcc         960
Met Ile Phe Thr Phe Tyr Lys Ala Gln Gly Leu Ser Val Gly Ser Ser
305                 310                 315                 320

ctt gta gaa gaa gat aag ttg gat ctt gct aca tca cta ctt gca aaa         1008
Leu Val Glu Glu Asp Lys Leu Asp Leu Ala Thr Ser Leu Leu Ala Lys
                325                 330                 335

gcc aag gaa aag ggg gtg tct ctc ttg tta cca agt gat gtg gtg att         1056
Ala Lys Glu Lys Gly Val Ser Leu Leu Leu Pro Ser Asp Val Val Ile
            340                 345                 350

gca gac aaa ttt gct cct gat gct aat agc aag gtc gtg cca gca tct         1104
Ala Asp Lys Phe Ala Pro Asp Ala Asn Ser Lys Val Val Pro Ala Ser
        355                 360                 365

gcc atc cct gat ggc tgg atg gga ttg gat att ggt cca gat tct gtt         1152
Ala Ile Pro Asp Gly Trp Met Gly Leu Asp Ile Gly Pro Asp Ser Val
    370                 375                 380

aaa tca ttc agt gaa gca ttg gat act acc caa acc atc ata tgg aat         1200
Lys Ser Phe Ser Glu Ala Leu Asp Thr Thr Gln Thr Ile Ile Trp Asn
385                 390                 395                 400

gga cca atg gga gtg ttt gag ttt gac aag ttt gct gtt ggt aca gag         1248
Gly Pro Met Gly Val Phe Glu Phe Asp Lys Phe Ala Val Gly Thr Glu
                405                 410                 415

gct att gca aag aag ctg gct gac ctc agt gga aag ggg gtg acc aca         1296
Ala Ile Ala Lys Lys Leu Ala Asp Leu Ser Gly Lys Gly Val Thr Thr
            420                 425                 430

att att gga gga gga gat tct gtt gca gct gtg gag aaa gta gga gtt         1344
Ile Ile Gly Gly Gly Asp Ser Val Ala Ala Val Glu Lys Val Gly Val
        435                 440                 445

gct agt gtc atg agc cac ata tct act ggt ggt ggt gcc agt ttg gag         1392
Ala Ser Val Met Ser His Ile Ser Thr Gly Gly Gly Ala Ser Leu Glu
    450                 455                 460

tta ttg gaa ggc aaa gag ctt cca gga gtc ctt gct ctt gat gaa gcc         1440
Leu Leu Glu Gly Lys Glu Leu Pro Gly Val Leu Ala Leu Asp Glu Ala
465                 470                 475                 480

gtc cca gtc gct gtg                                                     1455
Val Pro Val Ala Val
                485


<210>  384
<211>  485
<212>  PRT
<213>  Glycine max

<400>  384

Met Ala Ser Ala Ala Ser Pro Thr Thr Phe Ser Leu Leu His Ser Ala
1               5                   10                  15

Thr Pro Ser Ser Ser Ser Thr Ala Thr Ser Ala Ser Pro Ser Pro Ser
            20                  25                  30

Arg Val Gln Leu Thr Ser Ser Leu Arg Ala Arg Pro Leu Arg Arg Leu
        35                  40                  45

Gly Phe Ala Ala Ala Asp Pro Leu Leu Ala Ala Ser Val Ala Ala Arg
    50                  55                  60

Val Ala Ala Val Arg Gly Gly Gly Lys Gly Ala Arg Gly Val Val Ser
65                  70                  75                  80

Met Ala Lys Lys Ser Val Gly Asp Leu Ser Ala Ala Asp Leu Lys Gly
                85                  90                  95

Lys Lys Val Phe Val Arg Ala Asp Leu Asn Val Pro Leu Asp Asp Asn
            100                 105                 110

Gln Asn Ile Thr Asp Asp Thr Arg Ile Arg Ala Ala Ile Pro Thr Ile
        115                 120                 125

Lys His Leu Ile Gln Asn Gly Ala Lys Val Ile Leu Ser Ser His Leu
    130                 135                 140

Gly Arg Pro Lys Gly Val Thr Pro Lys Tyr Ser Leu Ala Pro Leu Val
145                 150                 155                 160

Pro Arg Leu Ser Glu Leu Ile Gly Ile Gln Ile Val Lys Ala Glu Asp
                165                 170                 175

Ser Ile Gly Pro Glu Val Glu Lys Leu Val Ala Ser Leu Pro Asp Gly
            180                 185                 190

Gly Val Leu Leu Leu Glu Asn Val Arg Phe Tyr Lys Glu Glu Glu Lys
        195                 200                 205

Asn Asp Pro Glu His Ala Lys Lys Leu Ala Ser Leu Ala Asp Leu Tyr
    210                 215                 220

Val Asn Asp Ala Phe Gly Thr Ala His Arg Ala His Ala Ser Thr Glu
225                 230                 235                 240

Gly Val Thr Lys Tyr Leu Lys Pro Ser Val Ala Gly Phe Leu Leu Gln
                245                 250                 255

Lys Glu Leu Asp Tyr Leu Val Gly Ala Val Ser Asn Pro Lys Arg Pro
            260                 265                 270

Phe Ala Ala Ile Val Gly Gly Ser Lys Val Ser Ser Lys Ile Gly Val
        275                 280                 285

Ile Glu Ser Leu Leu Glu Lys Val Asp Ile Leu Leu Leu Gly Gly Gly
    290                 295                 300

Met Ile Phe Thr Phe Tyr Lys Ala Gln Gly Leu Ser Val Gly Ser Ser
305                 310                 315                 320

Leu Val Glu Glu Asp Lys Leu Asp Leu Ala Thr Ser Leu Leu Ala Lys
                325                 330                 335

Ala Lys Glu Lys Gly Val Ser Leu Leu Leu Pro Ser Asp Val Val Ile
            340                 345                 350

Ala Asp Lys Phe Ala Pro Asp Ala Asn Ser Lys Val Val Pro Ala Ser
        355                 360                 365

Ala Ile Pro Asp Gly Trp Met Gly Leu Asp Ile Gly Pro Asp Ser Val
    370                 375                 380

Lys Ser Phe Ser Glu Ala Leu Asp Thr Thr Gln Thr Ile Ile Trp Asn
385                 390                 395                 400

Gly Pro Met Gly Val Phe Glu Phe Asp Lys Phe Ala Val Gly Thr Glu
                405                 410                 415

Ala Ile Ala Lys Lys Leu Ala Asp Leu Ser Gly Lys Gly Val Thr Thr
            420                 425                 430

Ile Ile Gly Gly Gly Asp Ser Val Ala Ala Val Glu Lys Val Gly Val
        435                 440                 445

Ala Ser Val Met Ser His Ile Ser Thr Gly Gly Gly Ala Ser Leu Glu
    450                 455                 460

Leu Leu Glu Gly Lys Glu Leu Pro Gly Val Leu Ala Leu Asp Glu Ala
465                 470                 475                 480

Val Pro Val Ala Val
                485

<210>  385
<211>  13
<212>  PRT
<213>  Glycine max

<400>  385

Glu Leu Asp Tyr Leu Val Gly Ala Val Ser Asn Pro Lys
1               5                   10

<210>  386
<211>  16
<212>  PRT
<213>  Glycine max

<400>  386

Glu Leu Pro Gly Val Leu Ala Leu Asp Glu Ala Val Pro Val Ala Val
1               5                   10                  15

<210>  387
<211>  10
<212>  PRT
<213>  Glycine max

<400>  387

Phe Ala Val Gly Thr Glu Ala Ile Ala Lys
1               5                   10

<210>  388
<211>  15
<212>  PRT
<213>  Glycine max

<400>  388

Gly Val Ser Leu Leu Leu Pro Ser Asp Val Val Ile Ala Asp Lys
1               5                   10                  15

<210>  389
<211>  17
<212>  PRT
<213>  Glycine max

<400>  389

Gly Val Thr Thr Ile Ile Gly Gly Gly Asp Ser Val Ala Ala Val Glu
1               5                   10                  15

Lys

<210>  390
<211>  10
<212>  PRT
<213>  Glycine max

<400>  390

Ile Gly Val Ile Glu Ser Leu Leu Glu Lys
1               5                   10

<210>  391
<211>  11
<212>  PRT
<213>  Glycine max

<400>  391

Arg Pro Phe Ala Ala Ile Val Gly Gly Ser Lys
1               5                   10


<210>  392
<211>  11
<212>  PRT
<213>  Glycine max

<400>  392

Val Ile Leu Ser Ser His Leu Gly Arg Pro Lys
1               5                   10


<210>  393
<211>  1203
<212>  DNA
<213>  Glycine max


<220>
<221>  CDS
<222>  (1)..(1203)

<400>  393
atg gcg act aag agg agc gtg gga acg ttg aag gag ggt gat ttg aaa      48
Met Ala Thr Lys Arg Ser Val Gly Thr Leu Lys Glu Gly Asp Leu Lys
1               5                   10                  15

ggg aag agg gtg ttc gtg agg gtc gat ctg aac gtg cct ttg gat gac      96
Gly Lys Arg Val Phe Val Arg Val Asp Leu Asn Val Pro Leu Asp Asp
            20                  25                  30

aac ctt aac atc acc gat gac act aga gtc cgt gct gct gtt ccc acc      144
Asn Leu Asn Ile Thr Asp Asp Thr Arg Val Arg Ala Ala Val Pro Thr
        35                  40                  45

atc aag tac tta act ggt cat gga gcc aaa gtg atc ctt tct agc cac      192
Ile Lys Tyr Leu Thr Gly His Gly Ala Lys Val Ile Leu Ser Ser His
    50                  55                  60

ttg gga cgt cct aaa ggt gta aca cct aaa tac agt ttg aag cct ctt      240
Leu Gly Arg Pro Lys Gly Val Thr Pro Lys Tyr Ser Leu Lys Pro Leu
65                  70                  75                  80

gtg cca agg ctg tct caa ctt ctc gga att gag gtt acg atg gca aat      288
Val Pro Arg Leu Ser Gln Leu Leu Gly Ile Glu Val Thr Met Ala Asn
                85                  90                  95

gac tct att ggg gag gaa gtt gag aag ttg gtt gca caa ctt cca gaa      336
Asp Ser Ile Gly Glu Glu Val Glu Lys Leu Val Ala Gln Leu Pro Glu
            100                 105                 110

ggt ggt gtt ttg ctt cta gag aat gtg agg ttc tat aag gag gaa gag      384

Gly Gly Val Leu Leu Leu Glu Asn Val Arg Phe Tyr Lys Glu Glu Glu
        115                 120                 125

aag aat gac cct gag ttt gca aag aag ttg gct tct ctt gct gat ctc        432
Lys Asn Asp Pro Glu Phe Ala Lys Lys Leu Ala Ser Leu Ala Asp Leu
    130                 135                 140

tac gtg aat gat gct ttc ggc act gcc cac aga gct cat gct tct aca        480
Tyr Val Asn Asp Ala Phe Gly Thr Ala His Arg Ala His Ala Ser Thr
145                 150                 155                 160

gaa gga gtg gcc aaa tac ttg aag ccc tct gtt gca gga ttc ctt atg        528
Glu Gly Val Ala Lys Tyr Leu Lys Pro Ser Val Ala Gly Phe Leu Met
                165                 170                 175

cag aag gaa ctt gac tat cta gtt gga gct gtg tca aac ccc aag aga        576
Gln Lys Glu Leu Asp Tyr Leu Val Gly Ala Val Ser Asn Pro Lys Arg
            180                 185                 190

cca ttt gct gct atc gtt ggt ggg tca aag gtg tct tcc aag att gga        624
Pro Phe Ala Ala Ile Val Gly Gly Ser Lys Val Ser Ser Lys Ile Gly
        195                 200                 205

gtt att gag tcc ttg ttg gag aaa gtt aac gtt ctc ttg ctt ggt gga        672
Val Ile Glu Ser Leu Leu Glu Lys Val Asn Val Leu Leu Leu Gly Gly
    210                 215                 220

gga atg atc ttt acc ttt tac aag gct cag ggt tat tca gtt ggt tca        720
Gly Met Ile Phe Thr Phe Tyr Lys Ala Gln Gly Tyr Ser Val Gly Ser
225                 230                 235                 240

tct ctt gtg gag gaa gac aag ctt agc ctt gca act aca ctt ctc gaa        768
Ser Leu Val Glu Glu Asp Lys Leu Ser Leu Ala Thr Thr Leu Leu Glu
                245                 250                 255

aag gcc aag gct aaa ggg gtt tct ttg ttg ctt cca act gat gtg gtc        816
Lys Ala Lys Ala Lys Gly Val Ser Leu Leu Leu Pro Thr Asp Val Val
            260                 265                 270

ata gca gac aag ttt gct gct gat gct aac agc aag act gtg cca gca        864
Ile Ala Asp Lys Phe Ala Ala Asp Ala Asn Ser Lys Thr Val Pro Ala
        275                 280                 285

tca agc atc cca gac ggg tgg atg gga tta gat att ggt ccc gat tcc        912
Ser Ser Ile Pro Asp Gly Trp Met Gly Leu Asp Ile Gly Pro Asp Ser
    290                 295                 300

atc aag aca ttt ggt gaa gca ttg gat aag acc cag acc gtc att tgg        960
Ile Lys Thr Phe Gly Glu Ala Leu Asp Lys Thr Gln Thr Val Ile Trp
305                 310                 315                 320

aat gga cca atg ggt gtt ttt gag ttt gat aag ttt gcc aca gga aca       1008
Asn Gly Pro Met Gly Val Phe Glu Phe Asp Lys Phe Ala Thr Gly Thr
                325                 330                 335

gag gcc ata gcc aag aaa ctg gca gag ctg agt ggg aag gga gta acg       1056
Glu Ala Ile Ala Lys Lys Leu Ala Glu Leu Ser Gly Lys Gly Val Thr
            340                 345                 350

acc atc att gga gga ggt gac tct gtt gcc gct gtg gag aag gtt ggt       1104
Thr Ile Ile Gly Gly Gly Asp Ser Val Ala Ala Val Glu Lys Val Gly
        355                 360                 365

ctt gca gac aag atg agc cac atc tcc act ggt ggt ggt gcc agc tta 1152
Leu Ala Asp Lys Met Ser His Ile Ser Thr Gly Gly Gly Ala Ser Leu
    370                 375                 380

gag ctt ctt gag ggg aag caa ctc cct ggt gtc ctt gcc ctt gat gat 1200
Glu Leu Leu Glu Gly Lys Gln Leu Pro Gly Val Leu Ala Leu Asp Asp
385                 390                 395                 400

gct 1203
Ala

<210> 394
<211> 401
<212> PRT
<213> Glycine max

<400> 394

Met Ala Thr Lys Arg Ser Val Gly Thr Leu Lys Glu Gly Asp Leu Lys
1               5                   10                  15

Gly Lys Arg Val Phe Val Arg Val Asp Leu Asn Val Pro Leu Asp Asp
            20                  25                  30

Asn Leu Asn Ile Thr Asp Asp Thr Arg Val Arg Ala Ala Val Pro Thr
        35                  40                  45

Ile Lys Tyr Leu Thr Gly His Gly Ala Lys Val Ile Leu Ser Ser His
    50                  55                  60

Leu Gly Arg Pro Lys Gly Val Thr Pro Lys Tyr Ser Leu Lys Pro Leu
65                  70                  75                  80

Val Pro Arg Leu Ser Gln Leu Leu Gly Ile Glu Val Thr Met Ala Asn
                85                  90                  95

Asp Ser Ile Gly Glu Glu Val Glu Lys Leu Val Ala Gln Leu Pro Glu
            100                 105                 110

Gly Gly Val Leu Leu Leu Glu Asn Val Arg Phe Tyr Lys Glu Glu Glu
        115                 120                 125

Lys Asn Asp Pro Glu Phe Ala Lys Lys Leu Ala Ser Leu Ala Asp Leu
    130                 135                 140

Tyr Val Asn Asp Ala Phe Gly Thr Ala His Arg Ala His Ala Ser Thr
145                 150                 155                 160

Glu Gly Val Ala Lys Tyr Leu Lys Pro Ser Val Ala Gly Phe Leu Met
                165                 170                 175

Gln Lys Glu Leu Asp Tyr Leu Val Gly Ala Val Ser Asn Pro Lys Arg
            180                 185                 190

Pro Phe Ala Ala Ile Val Gly Gly Ser Lys Val Ser Ser Lys Ile Gly
        195                 200                 205

Val Ile Glu Ser Leu Leu Glu Lys Val Asn Val Leu Leu Leu Gly Gly
    210                 215                 220

Gly Met Ile Phe Thr Phe Tyr Lys Ala Gln Gly Tyr Ser Val Gly Ser
225                 230                 235                 240

Ser Leu Val Glu Glu Asp Lys Leu Ser Leu Ala Thr Thr Leu Leu Glu
                245                 250                 255

Lys Ala Lys Ala Lys Gly Val Ser Leu Leu Leu Pro Thr Asp Val Val
            260                 265                 270

Ile Ala Asp Lys Phe Ala Ala Asp Ala Asn Ser Lys Thr Val Pro Ala
        275                 280                 285

Ser Ser Ile Pro Asp Gly Trp Met Gly Leu Asp Ile Gly Pro Asp Ser
    290                 295                 300

Ile Lys Thr Phe Gly Glu Ala Leu Asp Lys Thr Gln Thr Val Ile Trp
305                 310                 315                 320

Asn Gly Pro Met Gly Val Phe Glu Phe Asp Lys Phe Ala Thr Gly Thr
                325                 330                 335

Glu Ala Ile Ala Lys Lys Leu Ala Glu Leu Ser Gly Lys Gly Val Thr
            340                 345                 350

Thr Ile Ile Gly Gly Gly Asp Ser Val Ala Ala Val Glu Lys Val Gly
        355                 360                 365

Leu Ala Asp Lys Met Ser His Ile Ser Thr Gly Gly Gly Ala Ser Leu
    370                 375                 380

Glu Leu Leu Glu Gly Lys Gln Leu Pro Gly Val Leu Ala Leu Asp Asp
385                 390                 395                 400

Ala

<210> 395
<211> 15

<212>  PRT
<213>  Glycine max

<400>  395

Ala Gln Gly Tyr Ser Val Gly Ser Ser Leu Val Glu Glu Asp Lys
1               5                   10                  15


<210>  396
<211>  13
<212>  PRT
<213>  Glycine max

<400>  396

Glu Leu Asp Tyr Leu Val Gly Ala Val Ser Asn Pro Lys
1               5                   10


<210>  397
<211>  8
<212>  PRT
<213>  Glycine max

<400>  397

Phe Ala Ala Asp Ala Asn Ser Lys
1               5


<210>  398
<211>  10
<212>  PRT
<213>  Glycine max

<400>  398

Phe Ala Thr Gly Thr Glu Ala Ile Ala Lys
1               5                   10


<210>  399
<211>  15
<212>  PRT
<213>  Glycine max

<400>  399

Gly Val Ser Leu Leu Leu Pro Thr Asp Val Val Ile Ala Asp Lys
1               5                   10                  15


<210>  400
<211>  17
<212>  PRT
<213>  Glycine max

<400>  400

Gly Val Thr Thr Ile Ile Gly Gly Gly Asp Ser Val Ala Ala Val Glu
1               5                   10                  15

Lys

<210> 401
<211> 10
<212> PRT
<213> Glycine max

<400> 401

Ile Gly Val Ile Glu Ser Leu Leu Glu Lys
1               5                   10

<210> 402
<211> 28
<212> PRT
<213> Glycine max

<400> 402

Leu Ala Ser Leu Ala Asp Leu Tyr Val Asn Asp Ala Phe Gly Thr Ala
1               5                   10                  15

His Arg Ala His Ala Ser Thr Glu Gly Val Ala Lys
            20                  25

<210> 403
<211> 10
<212> PRT
<213> Glycine max

<400> 403

Leu Ser Leu Ala Thr Thr Leu Leu Glu Lys
1               5                   10

<210> 404
<211> 20
<212> PRT
<213> Glycine max

<400> 404

Leu Val Ala Gln Leu Pro Glu Gly Gly Val Leu Leu Leu Glu Asn Val
1               5                   10                  15

Arg Phe Tyr Lys
            20

<210> 405
<211> 7
<212> PRT
<213> Glycine max

<400> 405

Asn Asp Pro Glu Phe Ala Lys
1               5

<210>  406
<211>  11
<212>  PRT
<213>  Glycine max

<400>  406

Gln Leu Pro Gly Val Leu Ala Leu Asp Asp Ala
1               5                   10

<210>  407
<211>  11
<212>  PRT
<213>  Glycine max

<400>  407

Arg Pro Phe Ala Ala Ile Val Gly Gly Ser Lys
1               5                   10

<210>  408
<211>  8
<212>  PRT
<213>  Glycine max

<400>  408

Thr Phe Gly Glu Ala Leu Asp Lys
1               5

<210>  409
<211>  11
<212>  PRT
<213>  Glycine max

<400>  409

Val Ile Leu Ser Ser His Leu Gly Arg Pro Lys
1               5                   10

<210>  410
<211>  1143
<212>  DNA
<213>  Glycine max

<220>
<221>  CDS
<222>  (1)..(1143)

<400>  410
atg tcg tcg agc aca gtt ggt caa gtc atc aaa tgc aaa gct gcg gtt          48
Met Ser Ser Ser Thr Val Gly Gln Val Ile Lys Cys Lys Ala Ala Val
1               5                   10                  15

tca tgg gag gca ggg aag cca ctt gtg att gaa gaa gta gag gtg gca     96
Ser Trp Glu Ala Gly Lys Pro Leu Val Ile Glu Glu Val Glu Val Ala
        20                  25                  30

cca cca caa gct ggt gaa gtg cgt ttg aag atc ctc tac acc tct ctt     144
Pro Pro Gln Ala Gly Glu Val Arg Leu Lys Ile Leu Tyr Thr Ser Leu
    35                  40                  45

tgc cac act gat gtt tac ttc tgg gaa gcc aag ggc caa act cca ttg     192
Cys His Thr Asp Val Tyr Phe Trp Glu Ala Lys Gly Gln Thr Pro Leu
    50                  55                  60

ttt cct cgt ata ttt ggt cat gaa gct gga ggg att gtg gag agc gta     240
Phe Pro Arg Ile Phe Gly His Glu Ala Gly Gly Ile Val Glu Ser Val
65                  70                  75                  80

ggt gag ggt gtg act cat ctc aaa cca ggg gac cat gcc ctc cca gtg     288
Gly Glu Gly Val Thr His Leu Lys Pro Gly Asp His Ala Leu Pro Val
                85                  90                  95

ttt acc ggg gag tgt gga gaa tgc cct cat tgc aag tca gag gag agc     336
Phe Thr Gly Glu Cys Gly Glu Cys Pro His Cys Lys Ser Glu Glu Ser
            100                 105                 110

aac atg tgt gat ctt ctc agg atc aac act gat agg ggt gtc atg atc     384
Asn Met Cys Asp Leu Leu Arg Ile Asn Thr Asp Arg Gly Val Met Ile
        115                 120                 125

cat gac agc caa aca aga ttt tct att aag gga caa cct att tac cat     432
His Asp Ser Gln Thr Arg Phe Ser Ile Lys Gly Gln Pro Ile Tyr His
    130                 135                 140

ttt gtt ggt acc tcc aca ttc agt gaa tac act gtt gtt cat gcc gga     480
Phe Val Gly Thr Ser Thr Phe Ser Glu Tyr Thr Val Val His Ala Gly
145                 150                 155                 160

tgt gtt gcc aaa gtc aac cct gct gcc cca ctt gac aaa att tgt gtt     528
Cys Val Ala Lys Val Asn Pro Ala Ala Pro Leu Asp Lys Ile Cys Val
                165                 170                 175

ctc agt tgt gga att tgc aca ggt ctt ggg gct act atc aat gtt gca     576
Leu Ser Cys Gly Ile Cys Thr Gly Leu Gly Ala Thr Ile Asn Val Ala
            180                 185                 190

aaa cca aaa cct ggt tct tct gtt gcc att ttt gga ctt gga gct gtt     624
Lys Pro Lys Pro Gly Ser Ser Val Ala Ile Phe Gly Leu Gly Ala Val
        195                 200                 205

ggc ctt gct gct gct gaa ggg gca agg att tct ggt gca tca aga att     672
Gly Leu Ala Ala Ala Glu Gly Ala Arg Ile Ser Gly Ala Ser Arg Ile
    210                 215                 220

att ggg gtt gat tta gtt tct agc aga ttt gaa gaa gct aag aag ttt     720
Ile Gly Val Asp Leu Val Ser Ser Arg Phe Glu Glu Ala Lys Lys Phe
225                 230                 235                 240

ggg gtc aat gaa ttt gtg aac cca aaa gac cat gac aag cct gta caa     768
Gly Val Asn Glu Phe Val Asn Pro Lys Asp His Asp Lys Pro Val Gln
                245                 250                 255

gag gtg att gct gca atg acg aat ggg ggt gtg gat cgt gct gtt gaa     816
Glu Val Ile Ala Ala Met Thr Asn Gly Gly Val Asp Arg Ala Val Glu

260                 265                 270

tgt act ggc agc atc caa gcc atg atc tcg gca ttc gaa tgc gtc cac     864
Cys Thr Gly Ser Ile Gln Ala Met Ile Ser Ala Phe Glu Cys Val His
        275                 280                 285

gat ggt tgg ggt gtt gct gta ctt gtt gga gtg cca aac aaa gat gat     912
Asp Gly Trp Gly Val Ala Val Leu Val Gly Val Pro Asn Lys Asp Asp
    290                 295                 300

gca ttc aaa act cat cca gtg aat ttc ttg aac gag aga act cta aag     960
Ala Phe Lys Thr His Pro Val Asn Phe Leu Asn Glu Arg Thr Leu Lys
305                 310                 315                 320

ggt acc ttc tac gga aac tac aaa ccc cgc act gat ctt cct tct gtt     1008
Gly Thr Phe Tyr Gly Asn Tyr Lys Pro Arg Thr Asp Leu Pro Ser Val
                325                 330                 335

gtg gag aag tac atg aat ggg gaa ttg gaa ctg gag aaa ttc atc act     1056
Val Glu Lys Tyr Met Asn Gly Glu Leu Glu Leu Glu Lys Phe Ile Thr
            340                 345                 350

cac aca gtt cca ttc tca gag att aac aag gct ttt gat tac atg ctg     1104
His Thr Val Pro Phe Ser Glu Ile Asn Lys Ala Phe Asp Tyr Met Leu
        355                 360                 365

aaa ggg gag tcc atc aga tgc atc att cga atg ggg gag                 1143
Lys Gly Glu Ser Ile Arg Cys Ile Ile Arg Met Gly Glu
    370                 375                 380


<210>  411
<211>  381
<212>  PRT
<213>  Glycine max

<400>  411

Met Ser Ser Ser Thr Val Gly Gln Val Ile Lys Cys Lys Ala Ala Val
1               5                   10                  15


Ser Trp Glu Ala Gly Lys Pro Leu Val Ile Glu Glu Val Glu Val Ala
            20                  25                  30


Pro Pro Gln Ala Gly Glu Val Arg Leu Lys Ile Leu Tyr Thr Ser Leu
        35                  40                  45


Cys His Thr Asp Val Tyr Phe Trp Glu Ala Lys Gly Gln Thr Pro Leu
    50                  55                  60


Phe Pro Arg Ile Phe Gly His Glu Ala Gly Gly Ile Val Glu Ser Val
65                  70                  75                  80


Gly Glu Gly Val Thr His Leu Lys Pro Gly Asp His Ala Leu Pro Val
                85                  90                  95


Phe Thr Gly Glu Cys Gly Glu Cys Pro His Cys Lys Ser Glu Glu Ser

            100                 105                 110

Asn Met Cys Asp Leu Leu Arg Ile Asn Thr Asp Arg Gly Val Met Ile
        115                 120                 125

His Asp Ser Gln Thr Arg Phe Ser Ile Lys Gly Gln Pro Ile Tyr His
    130                 135                 140

Phe Val Gly Thr Ser Thr Phe Ser Glu Tyr Thr Val Val His Ala Gly
145                 150                 155                 160

Cys Val Ala Lys Val Asn Pro Ala Ala Pro Leu Asp Lys Ile Cys Val
                165                 170                 175

Leu Ser Cys Gly Ile Cys Thr Gly Leu Gly Ala Thr Ile Asn Val Ala
            180                 185                 190

Lys Pro Lys Pro Gly Ser Ser Val Ala Ile Phe Gly Leu Gly Ala Val
        195                 200                 205

Gly Leu Ala Ala Ala Glu Gly Ala Arg Ile Ser Gly Ala Ser Arg Ile
    210                 215                 220

Ile Gly Val Asp Leu Val Ser Ser Arg Phe Glu Glu Ala Lys Lys Phe
225                 230                 235                 240

Gly Val Asn Glu Phe Val Asn Pro Lys Asp His Asp Lys Pro Val Gln
                245                 250                 255

Glu Val Ile Ala Ala Met Thr Asn Gly Gly Val Asp Arg Ala Val Glu
            260                 265                 270

Cys Thr Gly Ser Ile Gln Ala Met Ile Ser Ala Phe Glu Cys Val His
        275                 280                 285

Asp Gly Trp Gly Val Ala Val Leu Val Gly Val Pro Asn Lys Asp Asp
    290                 295                 300

Ala Phe Lys Thr His Pro Val Asn Phe Leu Asn Glu Arg Thr Leu Lys
305                 310                 315                 320

Gly Thr Phe Tyr Gly Asn Tyr Lys Pro Arg Thr Asp Leu Pro Ser Val
                325                 330                 335

Val Glu Lys Tyr Met Asn Gly Glu Leu Glu Leu Glu Lys Phe Ile Thr
            340                 345                 350

His Thr Val Pro Phe Ser Glu Ile Asn Lys Ala Phe Asp Tyr Met Leu
        355                 360                 365

Lys Gly Glu Ser Ile Arg Cys Ile Ile Arg Met Gly Glu
    370                 375                 380

<210>  412
<211>  10
<212>  PRT
<213>  Glycine max

<400>  412

Phe Gly Val Asn Glu Phe Val Asn Pro Lys
1               5                   10

<210>  413
<211>  13
<212>  PRT
<213>  Glycine max

<400>  413

Phe Ile Thr His Thr Val Pro Phe Ser Glu Ile Asn Lys
1               5                   10

<210>  414
<211>  19
<212>  PRT
<213>  Glycine max

<400>  414

Gly Thr Phe Tyr Gly Asn Tyr Lys Pro Arg Thr Asp Leu Pro Ser Val
1               5                   10                  15

Val Glu Lys

<210>  415
<211>  13
<212>  PRT
<213>  Glycine max

<400>  415

Thr His Pro Val Asn Phe Leu Asn Glu Arg Thr Leu Lys
1               5                   10

<210>  416
<211>  9
<212>  PRT
<213>  Glycine max

<400>  416

Val Asn Pro Ala Ala Pro Leu Asp Lys
1               5

<210>  417
<211>  1113
<212>  DNA
<213>  Glycine max

<220>
<221>  CDS
<222>  (1)..(1113)

<400>  417
atg cgt gca gct gcg gtg gca tgg gaa gca ggg aag cca ctg tca ata      48
Met Arg Ala Ala Ala Val Ala Trp Glu Ala Gly Lys Pro Leu Ser Ile
1               5                   10                  15

gaa acc ata gaa gtg gca cca ccc cag aaa ggt gaa gtg cgt ttg agg      96
Glu Thr Ile Glu Val Ala Pro Pro Gln Lys Gly Glu Val Arg Leu Arg
            20                  25                  30

atc ctc ttc aac tcc ctt tgc cgt agt gat gtt tat tgg tgg gat gct      144
Ile Leu Phe Asn Ser Leu Cys Arg Ser Asp Val Tyr Trp Trp Asp Ala
        35                  40                  45

aag gac cag act cct ctg ttt cct cgt atc tta ggc cac gaa gct tca      192
Lys Asp Gln Thr Pro Leu Phe Pro Arg Ile Leu Gly His Glu Ala Ser
    50                  55                  60

ggg att gtg gag agc gtg ggc gag ggt gtg aca cat ctg aaa cca ggt      240
Gly Ile Val Glu Ser Val Gly Glu Gly Val Thr His Leu Lys Pro Gly
65                  70                  75                  80

gac cat gca ctc cca ata ttc act ggg gag tgt ggt gaa tgc act tat      288
Asp His Ala Leu Pro Ile Phe Thr Gly Glu Cys Gly Glu Cys Thr Tyr
                85                  90                  95

tgt aag tct gaa gag agc aac ctg tgt gag cta ctc agg atc aac act      336
Cys Lys Ser Glu Glu Ser Asn Leu Cys Glu Leu Leu Arg Ile Asn Thr
            100                 105                 110

gac agg ggt gtt atg ctg agt gat gga aaa aca agg ttc tcc aaa aat      384
Asp Arg Gly Val Met Leu Ser Asp Gly Lys Thr Arg Phe Ser Lys Asn
        115                 120                 125

ggc caa ccc att tac cac ttc gtt gga acc tcc act ttc agt gaa tac      432
Gly Gln Pro Ile Tyr His Phe Val Gly Thr Ser Thr Phe Ser Glu Tyr
    130                 135                 140

act gtt ctc cat gaa gga tgt gta gcc aag att aac cct aat gcc cca      480
Thr Val Leu His Glu Gly Cys Val Ala Lys Ile Asn Pro Asn Ala Pro
145                 150                 155                 160

ctt gac aaa gtt gct att gtc agc tgt gga ttc tgc aca ggt ttt ggg      528
Leu Asp Lys Val Ala Ile Val Ser Cys Gly Phe Cys Thr Gly Phe Gly
                165                 170                 175

gct act gtg aat gtt gca aaa cca aag cct aat aat act gtt gct gtc      576
Ala Thr Val Asn Val Ala Lys Pro Lys Pro Asn Asn Thr Val Ala Val
            180                 185                 190

ttt ggc tta gga gct gtt ggt ctt gct gcc tgt gaa ggg gca agg gtt 624
Phe Gly Leu Gly Ala Val Gly Leu Ala Ala Cys Glu Gly Ala Arg Val
        195                 200                 205

tct ggt gca tct aga atc atc ggg gtt gat tta ctt ccc aat cga ttt 672
Ser Gly Ala Ser Arg Ile Ile Gly Val Asp Leu Leu Pro Asn Arg Phe
    210                 215                 220

gaa caa gcc aaa aaa ttt ggg gtc act gat ttt gta aac ccg aaa gat 720
Glu Gln Ala Lys Lys Phe Gly Val Thr Asp Phe Val Asn Pro Lys Asp
225                 230                 235                 240

cac aac aaa cca gtc caa gag gtc att gct gaa atg act aat gga gga 768
His Asn Lys Pro Val Gln Glu Val Ile Ala Glu Met Thr Asn Gly Gly
                245                 250                 255

gta gat cgt gct att gaa tgc act gga agc atc caa gct tca atc tca 816
Val Asp Arg Ala Ile Glu Cys Thr Gly Ser Ile Gln Ala Ser Ile Ser
            260                 265                 270

gca ttt gaa tgc act cat gat ggt tgg ggt act gct gtc ctt gtt ggt 864
Ala Phe Glu Cys Thr His Asp Gly Trp Gly Thr Ala Val Leu Val Gly
        275                 280                 285

gtg cca aag aag gat gtt gaa ttc aaa act aat cct atg aag ttc atg 912
Val Pro Lys Lys Asp Val Glu Phe Lys Thr Asn Pro Met Lys Phe Met
    290                 295                 300

gaa ggg aga act ctt aag ggt acc ttc tat ggt cac tac aga ccc cgc 960
Glu Gly Arg Thr Leu Lys Gly Thr Phe Tyr Gly His Tyr Arg Pro Arg
305                 310                 315                 320

act gac att cct ggt gtt gtg gag aag tac ctc aac aag gag ttg gaa 1008
Thr Asp Ile Pro Gly Val Val Glu Lys Tyr Leu Asn Lys Glu Leu Glu
                325                 330                 335

cta gac aaa ttt atc act cac tct gtg cca ttc tca aag atc aac act 1056
Leu Asp Lys Phe Ile Thr His Ser Val Pro Phe Ser Lys Ile Asn Thr
            340                 345                 350

gca ttt gat ctt atg ctg aaa ggg gag ggc ata agg tgc ctc atc tgc 1104
Ala Phe Asp Leu Met Leu Lys Gly Glu Gly Ile Arg Cys Leu Ile Cys
        355                 360                 365

atg gaa gag 1113
Met Glu Glu
    370

<210> 418
<211> 371
<212> PRT
<213> Glycine max

<400> 418

Met Arg Ala Ala Ala Val Ala Trp Glu Ala Gly Lys Pro Leu Ser Ile
1               5                   10                  15

Glu Thr Ile Glu Val Ala Pro Pro Gln Lys Gly Glu Val Arg Leu Arg
            20                  25                  30

Ile Leu Phe Asn Ser Leu Cys Arg Ser Asp Val Tyr Trp Trp Asp Ala
        35                  40                  45

Lys Asp Gln Thr Pro Leu Phe Pro Arg Ile Leu Gly His Glu Ala Ser
    50                  55                  60

Gly Ile Val Glu Ser Val Gly Glu Gly Val Thr His Leu Lys Pro Gly
65                  70                  75                  80

Asp His Ala Leu Pro Ile Phe Thr Gly Glu Cys Gly Glu Cys Thr Tyr
                85                  90                  95

Cys Lys Ser Glu Glu Ser Asn Leu Cys Glu Leu Leu Arg Ile Asn Thr
            100                 105                 110

Asp Arg Gly Val Met Leu Ser Asp Gly Lys Thr Arg Phe Ser Lys Asn
        115                 120                 125

Gly Gln Pro Ile Tyr His Phe Val Gly Thr Ser Thr Phe Ser Glu Tyr
    130                 135                 140

Thr Val Leu His Glu Gly Cys Val Ala Lys Ile Asn Pro Asn Ala Pro
145                 150                 155                 160

Leu Asp Lys Val Ala Ile Val Ser Cys Gly Phe Cys Thr Gly Phe Gly
                165                 170                 175

Ala Thr Val Asn Val Ala Lys Pro Lys Pro Asn Asn Thr Val Ala Val
            180                 185                 190

Phe Gly Leu Gly Ala Val Gly Leu Ala Ala Cys Glu Gly Ala Arg Val
        195                 200                 205

Ser Gly Ala Ser Arg Ile Ile Gly Val Asp Leu Leu Pro Asn Arg Phe
    210                 215                 220

Glu Gln Ala Lys Lys Phe Gly Val Thr Asp Phe Val Asn Pro Lys Asp
225                 230                 235                 240

His Asn Lys Pro Val Gln Glu Val Ile Ala Glu Met Thr Asn Gly Gly
                245                 250                 255

Val Asp Arg Ala Ile Glu Cys Thr Gly Ser Ile Gln Ala Ser Ile Ser
            260                 265                 270

Ala Phe Glu Cys Thr His Asp Gly Trp Gly Thr Ala Val Leu Val Gly
        275                 280                 285

Val Pro Lys Lys Asp Val Glu Phe Lys Thr Asn Pro Met Lys Phe Met
    290                 295                 300

Glu Gly Arg Thr Leu Lys Gly Thr Phe Tyr Gly His Tyr Arg Pro Arg
305                 310                 315                 320

Thr Asp Ile Pro Gly Val Val Glu Lys Tyr Leu Asn Lys Glu Leu Glu
                325                 330                 335

Leu Asp Lys Phe Ile Thr His Ser Val Pro Phe Ser Lys Ile Asn Thr
            340                 345                 350

Ala Phe Asp Leu Met Leu Lys Gly Glu Gly Ile Arg Cys Leu Ile Cys
        355                 360                 365

Met Glu Glu
    370

<210>  419
<211>  19
<212>  PRT
<213>  Glycine max

<400>  419

Gly Thr Phe Tyr Gly His Tyr Arg Pro Arg Thr Asp Ile Pro Gly Val
1               5                   10                  15

Val Glu Lys

<210>  420
<211>  9
<212>  PRT
<213>  Glycine max

<400>  420

Ile Asn Pro Asn Ala Pro Leu Asp Lys
1               5

<210>  421
<211>  1140
<212>  DNA
<213>  Glycine max

<220>
<221>  CDS
<222>  (1)..(1140)

<400>  421

atg tct agc acc gtt ggc cag acc atc aag tgc aaa gct gcg att gca     48
Met Ser Ser Thr Val Gly Gln Thr Ile Lys Cys Lys Ala Ala Ile Ala
1               5                   10                  15

tgg gag gct ggg aag ccg ctg gtg att gaa gaa gtg gag gtt gcg cca     96
Trp Glu Ala Gly Lys Pro Leu Val Ile Glu Glu Val Glu Val Ala Pro
            20                  25                  30

ccg caa gcc ggt gaa gtc cgt ttg aag atc ctc tac acc tct ctt tgc     144
Pro Gln Ala Gly Glu Val Arg Leu Lys Ile Leu Tyr Thr Ser Leu Cys
        35                  40                  45

cac act gat gtt tac ttc tgg gat gcg aag ggc cag act cca ttg ttt     192
His Thr Asp Val Tyr Phe Trp Asp Ala Lys Gly Gln Thr Pro Leu Phe
    50                  55                  60

cct cgc att ttt ggc cat gaa gct tcg ggg att gtg gag agc gta ggc     240
Pro Arg Ile Phe Gly His Glu Ala Ser Gly Ile Val Glu Ser Val Gly
65                  70                  75                  80

gag ggt gtg act cat ctg aaa cca ggt gac cat gcc ctc cct gtg ttc     288
Glu Gly Val Thr His Leu Lys Pro Gly Asp His Ala Leu Pro Val Phe
                85                  90                  95

aca gga gag tgt gga gat tgc gcc cat tgc aag tca gag gag agc aac     336
Thr Gly Glu Cys Gly Asp Cys Ala His Cys Lys Ser Glu Glu Ser Asn
            100                 105                 110

atg tgt gag cta ctc agg atc aac acc gat agg ggt gtc atg atc cat     384
Met Cys Glu Leu Leu Arg Ile Asn Thr Asp Arg Gly Val Met Ile His
        115                 120                 125

gat ggc caa tca agg ttc tct aag aat gga caa ccc ata cac cat ttc     432
Asp Gly Gln Ser Arg Phe Ser Lys Asn Gly Gln Pro Ile His His Phe
    130                 135                 140

ttg gga acc tct aca ttc agt gaa tac act gtt gtc cat gct gga tgt     480
Leu Gly Thr Ser Thr Phe Ser Glu Tyr Thr Val Val His Ala Gly Cys
145                 150                 155                 160

gtt gca aag atc aac cct gct gct cca ctt gac aaa gtt tgt gtt ctc     528
Val Ala Lys Ile Asn Pro Ala Ala Pro Leu Asp Lys Val Cys Val Leu
                165                 170                 175

agt tgt gga att tgc aca ggt ttt ggt gct act gta aat gta gca aaa     576
Ser Cys Gly Ile Cys Thr Gly Phe Gly Ala Thr Val Asn Val Ala Lys
            180                 185                 190

cca aaa cct ggt tcc tct gtt gcc ata ttt gga ctt gga gct gtt ggc     624
Pro Lys Pro Gly Ser Ser Val Ala Ile Phe Gly Leu Gly Ala Val Gly
        195                 200                 205

ctt gcg gct gct gaa ggt gca aga gtt tca ggt gct tca aga atc att     672
Leu Ala Ala Ala Glu Gly Ala Arg Val Ser Gly Ala Ser Arg Ile Ile
    210                 215                 220

gga gtt gat tta gtt tct gcc cga ttt gaa gaa gct aag aag ttt ggg     720
Gly Val Asp Leu Val Ser Ala Arg Phe Glu Glu Ala Lys Lys Phe Gly
225                 230                 235                 240

gtt aat gag ttt gtg aac cca aag gat cat gat aaa cct gtg caa cag     768
Val Asn Glu Phe Val Asn Pro Lys Asp His Asp Lys Pro Val Gln Gln
                245                 250                 255

gta att gct gaa atg acc aat gga ggt gtg gat cgg gct gtt gaa tgt     816
Val Ile Ala Glu Met Thr Asn Gly Gly Val Asp Arg Ala Val Glu Cys
            260                 265                 270

act ggc agc atc caa gcc atg gtc tca gca ttc gaa tgc gtc cac gat     864
Thr Gly Ser Ile Gln Ala Met Val Ser Ala Phe Glu Cys Val His Asp
        275                 280                 285

ggt tgg ggt ctt gct gta ctt gtt ggt gtg cct agt aaa gat gat gca     912
Gly Trp Gly Leu Ala Val Leu Val Gly Val Pro Ser Lys Asp Asp Ala
    290                 295                 300

ttc aaa act gct cct att aat ttc ctg aac gag agg act ctt aag ggc     960
Phe Lys Thr Ala Pro Ile Asn Phe Leu Asn Glu Arg Thr Leu Lys Gly
305                 310                 315                 320

acc ttt tat ggc aac tac aaa cca cgc acc gat ctt cca tct gtt gtc     1008
Thr Phe Tyr Gly Asn Tyr Lys Pro Arg Thr Asp Leu Pro Ser Val Val
                325                 330                 335

gag aag tac atg agt ggg gag cta gaa gtg gac aaa ttc atc act cac     1056
Glu Lys Tyr Met Ser Gly Glu Leu Glu Val Asp Lys Phe Ile Thr His
            340                 345                 350

aca gtt cca ttc tca gag atc aac aaa gct ttt gat tta atg ctg aag     1104
Thr Val Pro Phe Ser Glu Ile Asn Lys Ala Phe Asp Leu Met Leu Lys
        355                 360                 365

gga cag tcc att agg tgt atc atc cgc atg caa gag                     1140
Gly Gln Ser Ile Arg Cys Ile Ile Arg Met Gln Glu
    370                 375                 380


<210>  422
<211>  380
<212>  PRT
<213>  Glycine max

<400>  422

Met Ser Ser Thr Val Gly Gln Thr Ile Lys Cys Lys Ala Ala Ile Ala
1               5                   10                  15


Trp Glu Ala Gly Lys Pro Leu Val Ile Glu Glu Val Glu Val Ala Pro
            20                  25                  30


Pro Gln Ala Gly Glu Val Arg Leu Lys Ile Leu Tyr Thr Ser Leu Cys
        35                  40                  45


His Thr Asp Val Tyr Phe Trp Asp Ala Lys Gly Gln Thr Pro Leu Phe
    50                  55                  60


Pro Arg Ile Phe Gly His Glu Ala Ser Gly Ile Val Glu Ser Val Gly
65                  70                  75                  80


Glu Gly Val Thr His Leu Lys Pro Gly Asp His Ala Leu Pro Val Phe
                85                  90                  95

Thr Gly Glu Cys Gly Asp Cys Ala His Cys Lys Ser Glu Glu Ser Asn
        100                 105                 110

Met Cys Glu Leu Leu Arg Ile Asn Thr Asp Arg Gly Val Met Ile His
    115                 120                 125

Asp Gly Gln Ser Arg Phe Ser Lys Asn Gly Gln Pro Ile His His Phe
    130                 135                 140

Leu Gly Thr Ser Thr Phe Ser Glu Tyr Thr Val Val His Ala Gly Cys
145                 150                 155                 160

Val Ala Lys Ile Asn Pro Ala Ala Pro Leu Asp Lys Val Cys Val Leu
                165                 170                 175

Ser Cys Gly Ile Cys Thr Gly Phe Gly Ala Thr Val Asn Val Ala Lys
        180                 185                 190

Pro Lys Pro Gly Ser Ser Val Ala Ile Phe Gly Leu Gly Ala Val Gly
    195                 200                 205

Leu Ala Ala Ala Glu Gly Ala Arg Val Ser Gly Ala Ser Arg Ile Ile
    210                 215                 220

Gly Val Asp Leu Val Ser Ala Arg Phe Glu Glu Ala Lys Lys Phe Gly
225                 230                 235                 240

Val Asn Glu Phe Val Asn Pro Lys Asp His Asp Lys Pro Val Gln Gln
                245                 250                 255

Val Ile Ala Glu Met Thr Asn Gly Gly Val Asp Arg Ala Val Glu Cys
        260                 265                 270

Thr Gly Ser Ile Gln Ala Met Val Ser Ala Phe Glu Cys Val His Asp
    275                 280                 285

Gly Trp Gly Leu Ala Val Leu Val Gly Val Pro Ser Lys Asp Asp Ala
    290                 295                 300

Phe Lys Thr Ala Pro Ile Asn Phe Leu Asn Glu Arg Thr Leu Lys Gly
305                 310                 315                 320

Thr Phe Tyr Gly Asn Tyr Lys Pro Arg Thr Asp Leu Pro Ser Val Val
                325                 330                 335

Glu Lys Tyr Met Ser Gly Glu Leu Glu Val Asp Lys Phe Ile Thr His

340                 345                 350

Thr Val Pro Phe Ser Glu Ile Asn Lys Ala Phe Asp Leu Met Leu Lys
        355                 360                 365

Gly Gln Ser Ile Arg Cys Ile Ile Arg Met Gln Glu
    370                 375                 380

<210>  423
<211>  10
<212>  PRT
<213>  Glycine max

<400>  423

Phe Gly Val Asn Glu Phe Val Asn Pro Lys
1               5                   10

<210>  424
<211>  13
<212>  PRT
<213>  Glycine max

<400>  424

Phe Ile Thr His Thr Val Pro Phe Ser Glu Ile Asn Lys
1               5                   10

<210>  425
<211>  19
<212>  PRT
<213>  Glycine max

<400>  425

Gly Thr Phe Tyr Gly Asn Tyr Lys Pro Arg Thr Asp Leu Pro Ser Val
1               5                   10                  15

Val Glu Lys

<210>  426
<211>  9
<212>  PRT
<213>  Glycine max

<400>  426

Ile Asn Pro Ala Ala Pro Leu Asp Lys
1               5

<210>  427
<211>  13
<212>  PRT
<213>  Glycine max

<400>  427

Thr Ala Pro Ile Asn Phe Leu Asn Glu Arg Thr Leu Lys
1               5                   10


<210>  428
<211>  927
<212>  DNA
<213>  Glycine max


<220>
<221>  CDS
<222>  (1)..(927)

<400>  428
atg gcg tca aga cag caa ttc aag gaa gac aga gcc gag gca gct gca      48
Met Ala Ser Arg Gln Gln Phe Lys Glu Asp Arg Ala Glu Ala Ala Ala
1               5                   10                  15

aaa ctc gca gcg aaa gac atc ggc gac atc aac aga aca aac gag cgc      96
Lys Leu Ala Ala Lys Asp Ile Gly Asp Ile Asn Arg Thr Asn Glu Arg
            20                  25                  30

gac gag ggg tac gac ttg cac gac gaa gcc aac ttc aag caa gca cga      144
Asp Glu Gly Tyr Asp Leu His Asp Glu Ala Asn Phe Lys Gln Ala Arg
        35                  40                  45

gct gaa gca gct gca aag ctg gcg gcc aag gat ctg gag gac gct aac      192
Ala Glu Ala Ala Ala Lys Leu Ala Ala Lys Asp Leu Glu Asp Ala Asn
    50                  55                  60

aga tta aga gac aac act ttc aac aca tgg aaa gag caa cat cag cat      240
Arg Leu Arg Asp Asn Thr Phe Asn Thr Trp Lys Glu Gln His Gln His
65                  70                  75                  80

gat gag aac aaa ccc ggt gtg ata ggg tcc atg ttt aga gcc gcc aag      288
Asp Glu Asn Lys Pro Gly Val Ile Gly Ser Met Phe Arg Ala Ala Lys
                85                  90                  95

gaa gcc gtg gtt ggg aag cct cac cac gag gaa gtt tat gca aag gaa      336
Glu Ala Val Val Gly Lys Pro His His Glu Glu Val Tyr Ala Lys Glu
            100                 105                 110

aca aag atg ggt gaa tat tca gat tat gcg act cag aag gcg agg gaa      384
Thr Lys Met Gly Glu Tyr Ser Asp Tyr Ala Thr Gln Lys Ala Arg Glu
        115                 120                 125

acg aag cag aag gct ggg gag tat aca gac tat gcg gct cag aaa gca      432
Thr Lys Gln Lys Ala Gly Glu Tyr Thr Asp Tyr Ala Ala Gln Lys Ala
    130                 135                 140

aaa gaa acc aaa gac tat gct gct gag aag gct aag aat gct aaa gat      480
Lys Glu Thr Lys Asp Tyr Ala Ala Glu Lys Ala Lys Asn Ala Lys Asp
145                 150                 155                 160

acc act gtg cag aaa gct gga gag tac aag gat tat acc gct gag aag      528
Thr Thr Val Gln Lys Ala Gly Glu Tyr Lys Asp Tyr Thr Ala Glu Lys
                165                 170                 175

gct aag aat gct aaa gat acc act gtg cag aaa gct gga gaa tac aag      576

Ala Lys Asn Ala Lys Asp Thr Thr Val Gln Lys Ala Gly Glu Tyr Lys
            180                 185                 190

gat tat acc gct gag aag gca att gaa ggg aag gac agt gca gtg ggg      624
Asp Tyr Thr Ala Glu Lys Ala Ile Glu Gly Lys Asp Ser Ala Val Gly
        195                 200                 205

aag ctt ggg gag cta aag gaa tct gct gct gat gct gct aaa agg gct      672
Lys Leu Gly Glu Leu Lys Glu Ser Ala Ala Asp Ala Ala Lys Arg Ala
    210                 215                 220

atg ggt ttc ttc acc ggt ggc aac aaa gac caa act aga gag gag gag      720
Met Gly Phe Phe Thr Gly Gly Asn Lys Asp Gln Thr Arg Glu Glu Glu
225                 230                 235                 240

gag gaa acg agg cgg agt atg cag gag gtg agg gtg caa gat aag gag      768
Glu Glu Thr Arg Arg Ser Met Gln Glu Val Arg Val Gln Asp Lys Glu
                245                 250                 255

tat gga acg gga cgt ggt act gga gag aag ttg gtg ata aaa atg gaa      816
Tyr Gly Thr Gly Arg Gly Thr Gly Glu Lys Leu Val Ile Lys Met Glu
            260                 265                 270

gag tcg cgg cca gga gcg gtg gcg gat gcg ctc aaa gca gcg aat agg      864
Glu Ser Arg Pro Gly Ala Val Ala Asp Ala Leu Lys Ala Ala Asn Arg
        275                 280                 285

gcc atg gac ggc gat atg gag gag gaa ggc gtg ctt cat gtg gag cgt      912
Ala Met Asp Gly Asp Met Glu Glu Glu Gly Val Leu His Val Glu Arg
    290                 295                 300

cgt agg gag aaa atg                                                  927
Arg Arg Glu Lys Met
305


<210>  429
<211>  309
<212>  PRT
<213>  Glycine max

<400>  429

Met Ala Ser Arg Gln Gln Phe Lys Glu Asp Arg Ala Glu Ala Ala Ala
1               5                   10                  15


Lys Leu Ala Ala Lys Asp Ile Gly Asp Ile Asn Arg Thr Asn Glu Arg
            20                  25                  30


Asp Glu Gly Tyr Asp Leu His Asp Glu Ala Asn Phe Lys Gln Ala Arg
        35                  40                  45


Ala Glu Ala Ala Ala Lys Leu Ala Ala Lys Asp Leu Glu Asp Ala Asn
    50                  55                  60


Arg Leu Arg Asp Asn Thr Phe Asn Thr Trp Lys Glu Gln His Gln His
65                  70                  75                  80

Asp Glu Asn Lys Pro Gly Val Ile Gly Ser Met Phe Arg Ala Ala Lys
                85                  90                  95

Glu Ala Val Val Gly Lys Pro His His Glu Glu Val Tyr Ala Lys Glu
            100                 105                 110

Thr Lys Met Gly Glu Tyr Ser Asp Tyr Ala Thr Gln Lys Ala Arg Glu
        115                 120                 125

Thr Lys Gln Lys Ala Gly Glu Tyr Thr Asp Tyr Ala Ala Gln Lys Ala
    130                 135                 140

Lys Glu Thr Lys Asp Tyr Ala Ala Glu Lys Ala Lys Asn Ala Lys Asp
145                 150                 155                 160

Thr Thr Val Gln Lys Ala Gly Glu Tyr Lys Asp Tyr Thr Ala Glu Lys
                165                 170                 175

Ala Lys Asn Ala Lys Asp Thr Thr Val Gln Lys Ala Gly Glu Tyr Lys
            180                 185                 190

Asp Tyr Thr Ala Glu Lys Ala Ile Glu Gly Lys Asp Ser Ala Val Gly
        195                 200                 205

Lys Leu Gly Glu Leu Lys Glu Ser Ala Ala Asp Ala Ala Lys Arg Ala
    210                 215                 220

Met Gly Phe Phe Thr Gly Gly Asn Lys Asp Gln Thr Arg Glu Glu Glu
225                 230                 235                 240

Glu Glu Thr Arg Arg Ser Met Gln Glu Val Arg Val Gln Asp Lys Glu
                245                 250                 255

Tyr Gly Thr Gly Arg Gly Thr Gly Glu Lys Leu Val Ile Lys Met Glu
            260                 265                 270

Glu Ser Arg Pro Gly Ala Val Ala Asp Ala Leu Lys Ala Ala Asn Arg
        275                 280                 285

Ala Met Asp Gly Asp Met Glu Glu Glu Gly Val Leu His Val Glu Arg
    290                 295                 300

Arg Arg Glu Lys Met
305

<210>  430
<211>  11
<212>  PRT

<213>  Glycine max

<400>  430

Ala Gly Glu Tyr Thr Asp Tyr Ala Ala Gln Lys
1               5                   10

<210>  431
<211>  24
<212>  PRT
<213>  Glycine max

<400>  431

Asp Ile Gly Asp Ile Asn Arg Thr Asn Glu Arg Asp Glu Gly Tyr Asp
1               5                   10                  15

Leu His Asp Glu Ala Asn Phe Lys
            20

<210>  432
<211>  17
<212>  PRT
<213>  Glycine max

<400>  432

Asp Leu Glu Asp Ala Asn Arg Leu Arg Asp Asn Thr Phe Asn Thr Trp
1               5                   10                  15

Lys

<210>  433
<211>  15
<212>  PRT
<213>  Glycine max

<400>  433

Glu Ala Val Val Gly Lys Pro His His Glu Glu Val Tyr Ala Lys
1               5                   10                  15

<210>  434
<211>  1104
<212>  DNA
<213>  Glycine max

<220>
<221>  CDS
<222>  (1)..(1104)

<400>  434
atg ggc aag gtc aag atc gga atc aac gga ttt gga aga att ggc cgt          48
Met Gly Lys Val Lys Ile Gly Ile Asn Gly Phe Gly Arg Ile Gly Arg
1               5                   10                  15

ttg gta gcc aga gtg gct ctg cag aga gac gat gtt gaa ctc gtt gcc 96
Leu Val Ala Arg Val Ala Leu Gln Arg Asp Asp Val Glu Leu Val Ala
            20                  25                  30

gtt aac gac cct ttc atc acc acc gat tac atg aca tac atg ttt aaa 144
Val Asn Asp Pro Phe Ile Thr Thr Asp Tyr Met Thr Tyr Met Phe Lys
        35                  40                  45

tac gac agt gtt cat gga cac tgg aag cat cac gat gtc acc gtt aag 192
Tyr Asp Ser Val His Gly His Trp Lys His His Asp Val Thr Val Lys
    50                  55                  60

gac gag aag acc ctt ctc ttc ggt gac aag cca gtc act att ttt gga 240
Asp Glu Lys Thr Leu Leu Phe Gly Asp Lys Pro Val Thr Ile Phe Gly
65                  70                  75                  80

cac agg ttt tta ttt ttc act tta att ttt ctt act gtt tat gat tgt 288
His Arg Phe Leu Phe Phe Thr Leu Ile Phe Leu Thr Val Tyr Asp Cys
                85                  90                  95

tgt gtc ctg cat gta aac gtt gag tgt tgt ttt ctc tgt gaa ctc aga 336
Cys Val Leu His Val Asn Val Glu Cys Cys Phe Leu Cys Glu Leu Arg
            100                 105                 110

aac cct gaa gag atc cca tgg ggg tca act gga gct gac atc att gtt 384
Asn Pro Glu Glu Ile Pro Trp Gly Ser Thr Gly Ala Asp Ile Ile Val
        115                 120                 125

gag tcc acc gga gtt ttc acc gat aag gac aag gcc gcc gca cat ttg 432
Glu Ser Thr Gly Val Phe Thr Asp Lys Asp Lys Ala Ala Ala His Leu
    130                 135                 140

aag ggt ggt gca aag aag gtt att att tct gcc ccc agt aag gat gcc 480
Lys Gly Gly Ala Lys Lys Val Ile Ile Ser Ala Pro Ser Lys Asp Ala
145                 150                 155                 160

ccc atg ttt gtt gtt ggt gtc aac gag cac gag tac aag cca gag ctt 528
Pro Met Phe Val Val Gly Val Asn Glu His Glu Tyr Lys Pro Glu Leu
                165                 170                 175

gat att att tcc aat gct agc tgc aca acc aac tgc ctt gcc cca ctt 576
Asp Ile Ile Ser Asn Ala Ser Cys Thr Thr Asn Cys Leu Ala Pro Leu
            180                 185                 190

gcc aag gtt atc aat gac agg ttt ggc att gtt gag ggt ttg atg acc 624
Ala Lys Val Ile Asn Asp Arg Phe Gly Ile Val Glu Gly Leu Met Thr
        195                 200                 205

act gtt cat tcc atc acc gct acc cag aag act gtt gat gga cca tca 672
Thr Val His Ser Ile Thr Ala Thr Gln Lys Thr Val Asp Gly Pro Ser
    210                 215                 220

gcc aag gac tgg aga ggt gga aga gct gct tca ttt aac atc att cct 720
Ala Lys Asp Trp Arg Gly Gly Arg Ala Ala Ser Phe Asn Ile Ile Pro
225                 230                 235                 240

agc agc act gga gct gcc aag gct gtt ggg aaa gtc ctc cct gct ttg 768
Ser Ser Thr Gly Ala Ala Lys Ala Val Gly Lys Val Leu Pro Ala Leu
                245                 250                 255

aat gga aaa ttg act ggt atg gca ttc cgt gtt ccc acc gtg gat gtc 816
Asn Gly Lys Leu Thr Gly Met Ala Phe Arg Val Pro Thr Val Asp Val

260                 265                 270

tct gtt gtt gac ctc aca gtg agg ctg gag aaa gaa gct tcc tac gat        864
Ser Val Val Asp Leu Thr Val Arg Leu Glu Lys Glu Ala Ser Tyr Asp
        275                 280                 285

gaa att aaa aat gct atc aag gag gaa tca gag ggc aag ttg aag gga        912
Glu Ile Lys Asn Ala Ile Lys Glu Glu Ser Glu Gly Lys Leu Lys Gly
    290                 295                 300

att ctt ggt tac act gaa gat gat gtg gtc tcc act gac ttt atc ggc        960
Ile Leu Gly Tyr Thr Glu Asp Asp Val Val Ser Thr Asp Phe Ile Gly
305                 310                 315                 320

gat agc aga tca agt att ttt gat gca aag gct gga att gca ttg aat       1008
Asp Ser Arg Ser Ser Ile Phe Asp Ala Lys Ala Gly Ile Ala Leu Asn
                325                 330                 335

aag aac ttt gtg aag ctt gtt tct tgg tac gac aac gag tgg gga tac       1056
Lys Asn Phe Val Lys Leu Val Ser Trp Tyr Asp Asn Glu Trp Gly Tyr
            340                 345                 350

agc tca cgt gtc att gat ctt ctt gta ttc gtt gcc aag aag tct ctt       1104
Ser Ser Arg Val Ile Asp Leu Leu Val Phe Val Ala Lys Lys Ser Leu
        355                 360                 365

<210>  435
<211>  368
<212>  PRT
<213>  Glycine max

<400>  435

Met Gly Lys Val Lys Ile Gly Ile Asn Gly Phe Gly Arg Ile Gly Arg
1               5                   10                  15

Leu Val Ala Arg Val Ala Leu Gln Arg Asp Asp Val Glu Leu Val Ala
            20                  25                  30

Val Asn Asp Pro Phe Ile Thr Thr Asp Tyr Met Thr Tyr Met Phe Lys
        35                  40                  45

Tyr Asp Ser Val His Gly His Trp Lys His His Asp Val Thr Val Lys
    50                  55                  60

Asp Glu Lys Thr Leu Leu Phe Gly Asp Lys Pro Val Thr Ile Phe Gly
65                  70                  75                  80

His Arg Phe Leu Phe Phe Thr Leu Ile Phe Leu Thr Val Tyr Asp Cys
                85                  90                  95

Cys Val Leu His Val Asn Val Glu Cys Cys Phe Leu Cys Glu Leu Arg
            100                 105                 110

Asn Pro Glu Glu Ile Pro Trp Gly Ser Thr Gly Ala Asp Ile Ile Val

115                 120                 125

Glu Ser Thr Gly Val Phe Thr Asp Lys Asp Lys Ala Ala Ala His Leu
    130                 135                 140

Lys Gly Gly Ala Lys Lys Val Ile Ile Ser Ala Pro Ser Lys Asp Ala
145                 150                 155                 160

Pro Met Phe Val Val Gly Val Asn Glu His Glu Tyr Lys Pro Glu Leu
                165                 170                 175

Asp Ile Ile Ser Asn Ala Ser Cys Thr Thr Asn Cys Leu Ala Pro Leu
            180                 185                 190

Ala Lys Val Ile Asn Asp Arg Phe Gly Ile Val Glu Gly Leu Met Thr
        195                 200                 205

Thr Val His Ser Ile Thr Ala Thr Gln Lys Thr Val Asp Gly Pro Ser
    210                 215                 220

Ala Lys Asp Trp Arg Gly Gly Arg Ala Ala Ser Phe Asn Ile Ile Pro
225                 230                 235                 240

Ser Ser Thr Gly Ala Ala Lys Ala Val Gly Lys Val Leu Pro Ala Leu
                245                 250                 255

Asn Gly Lys Leu Thr Gly Met Ala Phe Arg Val Pro Thr Val Asp Val
            260                 265                 270

Ser Val Val Asp Leu Thr Val Arg Leu Glu Lys Glu Ala Ser Tyr Asp
        275                 280                 285

Glu Ile Lys Asn Ala Ile Lys Glu Glu Ser Glu Gly Lys Leu Lys Gly
    290                 295                 300

Ile Leu Gly Tyr Thr Glu Asp Asp Val Val Ser Thr Asp Phe Ile Gly
305                 310                 315                 320

Asp Ser Arg Ser Ser Ile Phe Asp Ala Lys Ala Gly Ile Ala Leu Asn
                325                 330                 335

Lys Asn Phe Val Lys Leu Val Ser Trp Tyr Asp Asn Glu Trp Gly Tyr
            340                 345                 350

Ser Ser Arg Val Ile Asp Leu Leu Val Phe Val Ala Lys Lys Ser Leu
        355                 360                 365

<210>  436
<211>  8
<212>  PRT
<213>  Glycine max

<400>  436

Glu Ala Ser Tyr Asp Glu Ile Lys
1               5


<210>  437
<211>  27
<212>  PRT
<213>  Glycine max

<400>  437

Gly Ile Leu Gly Tyr Thr Glu Asp Asp Val Val Ser Thr Asp Phe Ile
1               5                   10                  15


Gly Asp Ser Arg Ser Ser Ile Phe Asp Ala Lys
            20                  25


<210>  438
<211>  8
<212>  PRT
<213>  Glycine max

<400>  438

Val Ile Ile Ser Ala Pro Ser Lys
1               5


<210>  439
<211>  8
<212>  PRT
<213>  Glycine max

<400>  439

Val Leu Pro Ala Leu Asn Gly Lys
1               5


<210>  440
<211>  1014
<212>  DNA
<213>  Glycine max


<220>
<221>  CDS
<222>  (1)..(1014)

<400>  440
atg ggc aag gtc aag atc gga atc aac gga ttt gga aga att ggc cgt          48
Met Gly Lys Val Lys Ile Gly Ile Asn Gly Phe Gly Arg Ile Gly Arg
1               5                   10                  15

ttg gtt gcc aga gtc gct ctg caa aga gac gat gtg gag ctc gtt gcc 96
Leu Val Ala Arg Val Ala Leu Gln Arg Asp Asp Val Glu Leu Val Ala
            20                  25                  30

gtt aac gac cct ttc att acc acc gat tac atg aca tac atg ttt aaa 144
Val Asn Asp Pro Phe Ile Thr Thr Asp Tyr Met Thr Tyr Met Phe Lys
        35                  40                  45

tac gac agt gtt cac gga cac tgg aag cat cac gat gtc acc gtt aag 192
Tyr Asp Ser Val His Gly His Trp Lys His His Asp Val Thr Val Lys
    50                  55                  60

gac gag aag acc ctt ctc ttc ggt gac aag cca gtc act gtt ttt gga 240
Asp Glu Lys Thr Leu Leu Phe Gly Asp Lys Pro Val Thr Val Phe Gly
65                  70                  75                  80

cac agg aac cct gaa gag atc cca tgg ggg tca act gga gct gat atc 288
His Arg Asn Pro Glu Glu Ile Pro Trp Gly Ser Thr Gly Ala Asp Ile
                85                  90                  95

att gtt gag tcc acc gga gtt ttc acc gat aag gac aag gcc gcc gca 336
Ile Val Glu Ser Thr Gly Val Phe Thr Asp Lys Asp Lys Ala Ala Ala
            100                 105                 110

cat ttg aag ggt ggt gcg aag aag gtt att att tct gcc ccc agt aag 384
His Leu Lys Gly Gly Ala Lys Lys Val Ile Ile Ser Ala Pro Ser Lys
        115                 120                 125

gat gcc ccc atg ttt gtt gtt ggt gtt aac gag cac gag tac aag cca 432
Asp Ala Pro Met Phe Val Val Gly Val Asn Glu His Glu Tyr Lys Pro
    130                 135                 140

gag ctt gat att att tcc aat gct agc tgc aca acc aac tgc ctt gct 480
Glu Leu Asp Ile Ile Ser Asn Ala Ser Cys Thr Thr Asn Cys Leu Ala
145                 150                 155                 160

cca ctt gct aag gtt att aat gac agg ttt ggc att gtt gag ggt ttg 528
Pro Leu Ala Lys Val Ile Asn Asp Arg Phe Gly Ile Val Glu Gly Leu
                165                 170                 175

atg acc act gtt cat tcc atc act gcc acc cag aag act gtt gat ggg 576
Met Thr Thr Val His Ser Ile Thr Ala Thr Gln Lys Thr Val Asp Gly
            180                 185                 190

cca tca gcc aaa gac tgg aga ggt gga aga gct gct tca ttt aac atc 624
Pro Ser Ala Lys Asp Trp Arg Gly Gly Arg Ala Ala Ser Phe Asn Ile
        195                 200                 205

att cct agc agc act gga gct gcc aag gct gtt gga aaa gtc ctt cct 672
Ile Pro Ser Ser Thr Gly Ala Ala Lys Ala Val Gly Lys Val Leu Pro
    210                 215                 220

gct ttg aac gga aaa ttg act ggt atg gca ttc cgt gtt ccc acc gtg 720
Ala Leu Asn Gly Lys Leu Thr Gly Met Ala Phe Arg Val Pro Thr Val
225                 230                 235                 240

gat gtc tct gtt gtt gac ctc aca gtg agg ctg gag aag gaa gcc tcc 768
Asp Val Ser Val Val Asp Leu Thr Val Arg Leu Glu Lys Glu Ala Ser
                245                 250                 255

tac gat gaa att aaa aat gct atc aag gag gaa tca gag ggc aag ttg 816
Tyr Asp Glu Ile Lys Asn Ala Ile Lys Glu Glu Ser Glu Gly Lys Leu
            260                 265                 270

aag gga att ctt ggt tac act gaa gat gat gtg gtc tcc acc gac ttt 864
Lys Gly Ile Leu Gly Tyr Thr Glu Asp Asp Val Val Ser Thr Asp Phe
        275                 280                 285

gtg ggc gat agc aga tca agt att ttt gat gca aag gct gga att gca 912
Val Gly Asp Ser Arg Ser Ser Ile Phe Asp Ala Lys Ala Gly Ile Ala
    290                 295                 300

ttg aat aag aac ttt gtg aag ctt gtc tct tgg tat gac aac gag tgg 960
Leu Asn Lys Asn Phe Val Lys Leu Val Ser Trp Tyr Asp Asn Glu Trp
305                 310                 315                 320

gga tac agc tca cgt gtc att gac ctg ctt gta ttc gtt gcc aag aag 1008
Gly Tyr Ser Ser Arg Val Ile Asp Leu Leu Val Phe Val Ala Lys Lys
                325                 330                 335

tct ttt 1014
Ser Phe

<210> 441
<211> 338
<212> PRT
<213> Glycine max

<400> 441

Met Gly Lys Val Lys Ile Gly Ile Asn Gly Phe Gly Arg Ile Gly Arg
1               5                   10                  15

Leu Val Ala Arg Val Ala Leu Gln Arg Asp Asp Val Glu Leu Val Ala
            20                  25                  30

Val Asn Asp Pro Phe Ile Thr Thr Asp Tyr Met Thr Tyr Met Phe Lys
        35                  40                  45

Tyr Asp Ser Val His Gly His Trp Lys His His Asp Val Thr Val Lys
    50                  55                  60

Asp Glu Lys Thr Leu Leu Phe Gly Asp Lys Pro Val Thr Val Phe Gly
65                  70                  75                  80

His Arg Asn Pro Glu Glu Ile Pro Trp Gly Ser Thr Gly Ala Asp Ile
                85                  90                  95

Ile Val Glu Ser Thr Gly Val Phe Thr Asp Lys Asp Lys Ala Ala Ala
            100                 105                 110

His Leu Lys Gly Gly Ala Lys Lys Val Ile Ile Ser Ala Pro Ser Lys
        115                 120                 125

Asp Ala Pro Met Phe Val Val Gly Val Asn Glu His Glu Tyr Lys Pro
    130                 135                 140

Glu Leu Asp Ile Ile Ser Asn Ala Ser Cys Thr Thr Asn Cys Leu Ala
145                 150                 155                 160

Pro Leu Ala Lys Val Ile Asn Asp Arg Phe Gly Ile Val Glu Gly Leu
                165                 170                 175

Met Thr Thr Val His Ser Ile Thr Ala Thr Gln Lys Thr Val Asp Gly
            180                 185                 190

Pro Ser Ala Lys Asp Trp Arg Gly Gly Arg Ala Ala Ser Phe Asn Ile
        195                 200                 205

Ile Pro Ser Ser Thr Gly Ala Ala Lys Ala Val Gly Lys Val Leu Pro
    210                 215                 220

Ala Leu Asn Gly Lys Leu Thr Gly Met Ala Phe Arg Val Pro Thr Val
225                 230                 235                 240

Asp Val Ser Val Val Asp Leu Thr Val Arg Leu Glu Lys Glu Ala Ser
                245                 250                 255

Tyr Asp Glu Ile Lys Asn Ala Ile Lys Glu Glu Ser Glu Gly Lys Leu
            260                 265                 270

Lys Gly Ile Leu Gly Tyr Thr Glu Asp Asp Val Val Ser Thr Asp Phe
        275                 280                 285

Val Gly Asp Ser Arg Ser Ser Ile Phe Asp Ala Lys Ala Gly Ile Ala
    290                 295                 300

Leu Asn Lys Asn Phe Val Lys Leu Val Ser Trp Tyr Asp Asn Glu Trp
305                 310                 315                 320

Gly Tyr Ser Ser Arg Val Ile Asp Leu Leu Val Phe Val Ala Lys Lys
                325                 330                 335

Ser Phe

<210>  442
<211>  8
<212>  PRT
<213>  Glycine max

<400>  442

Glu Ala Ser Tyr Asp Glu Ile Lys
1               5

<210>  443
<211>  27
<212>  PRT
<213>  Glycine max

<400>  443

Gly Ile Leu Gly Tyr Thr Glu Asp Asp Val Val Ser Thr Asp Phe Val
1               5                   10                  15

Gly Asp Ser Arg Ser Ser Ile Phe Asp Ala Lys
            20                  25

<210>  444
<211>  8
<212>  PRT
<213>  Glycine max

<400>  444

Val Ile Ile Ser Ala Pro Ser Lys
1               5

<210>  445
<211>  8
<212>  PRT
<213>  Glycine max

<400>  445

Val Leu Pro Ala Leu Asn Gly Lys
1               5

<210>  446
<211>  1020
<212>  DNA
<213>  Glycine max

<220>
<221>  CDS
<222>  (1)..(1020)

<400>  446
atg gca tca gat aag aag att agg atc ggc atc aac gga ttt gga agg        48
Met Ala Ser Asp Lys Lys Ile Arg Ile Gly Ile Asn Gly Phe Gly Arg
1               5                   10                  15

att ggc cgt ttg gtg gcc agg gtt gcc ctt caa agg aat gat gtt gaa        96
Ile Gly Arg Leu Val Ala Arg Val Ala Leu Gln Arg Asn Asp Val Glu
            20                  25                  30

ctt gtt gct gtc aac gat cct ttc atc acc act gat tac atg aca tac        144
Leu Val Ala Val Asn Asp Pro Phe Ile Thr Thr Asp Tyr Met Thr Tyr
        35                  40                  45

atg ttc aag tac gac acc gtt cat ggc cag tgg aag cat ttt gat gtg        192

Met Phe Lys Tyr Asp Thr Val His Gly Gln Trp Lys His Phe Asp Val
    50                  55                  60

aag gtg aag gac tct aag acc ctt ctc ttt ggt gag aag ccc gtc acc      240
Lys Val Lys Asp Ser Lys Thr Leu Leu Phe Gly Glu Lys Pro Val Thr
65                  70                  75                  80

gtt ttt gga atc agg aac cct gag gaa att cca tgg ggt gag gtt ggt      288
Val Phe Gly Ile Arg Asn Pro Glu Glu Ile Pro Trp Gly Glu Val Gly
                85                  90                  95

gct gat tat gtt gtt gag tca act ggt gta ttc act gac aag gac aag      336
Ala Asp Tyr Val Val Glu Ser Thr Gly Val Phe Thr Asp Lys Asp Lys
            100                 105                 110

gct gct gct cac ttg aag ggt ggt gcc aag aag gtt gtc atc tct gcc      384
Ala Ala Ala His Leu Lys Gly Gly Ala Lys Lys Val Val Ile Ser Ala
        115                 120                 125

cca agc aaa gat gca ccc atg ttt gtt gtt ggt gtt aac gag aag gag      432
Pro Ser Lys Asp Ala Pro Met Phe Val Val Gly Val Asn Glu Lys Glu
    130                 135                 140

tac aag cca gag ctt gac att gtt tcc aat gct agc tgc act acc aat      480
Tyr Lys Pro Glu Leu Asp Ile Val Ser Asn Ala Ser Cys Thr Thr Asn
145                 150                 155                 160

tgt ctt gct ccc ctc gcc aag gtt atc aac gac cga ttt gga att gtg      528
Cys Leu Ala Pro Leu Ala Lys Val Ile Asn Asp Arg Phe Gly Ile Val
                165                 170                 175

gag ggt ctt atg acc act gtt cat gcc atc aca gct act cag aag act      576
Glu Gly Leu Met Thr Thr Val His Ala Ile Thr Ala Thr Gln Lys Thr
            180                 185                 190

gtt gat ggt cca tca aac aag gac tgg agg ggt gga aga gct gct tcc      624
Val Asp Gly Pro Ser Asn Lys Asp Trp Arg Gly Gly Arg Ala Ala Ser
        195                 200                 205

ttc aat ata att ccc agc agc act gga gct gct aag gct gta gga aaa      672
Phe Asn Ile Ile Pro Ser Ser Thr Gly Ala Ala Lys Ala Val Gly Lys
    210                 215                 220

gtt ctt cca gca ctg aat ggt aaa ttg act ggc atg tcc ttc cga gtc      720
Val Leu Pro Ala Leu Asn Gly Lys Leu Thr Gly Met Ser Phe Arg Val
225                 230                 235                 240

ccc act gta gat gtt tca gtt gtt gac ctc act gtc agg cta gag aag      768
Pro Thr Val Asp Val Ser Val Val Asp Leu Thr Val Arg Leu Glu Lys
                245                 250                 255

cca gca acc tat gag caa atc aaa tct gct atc aag gag gaa tca gag      816
Pro Ala Thr Tyr Glu Gln Ile Lys Ser Ala Ile Lys Glu Glu Ser Glu
            260                 265                 270

ggc aaa ttg aag ggt att ttg ggt tac acc gaa gat gat gtt gtg tct      864
Gly Lys Leu Lys Gly Ile Leu Gly Tyr Thr Glu Asp Asp Val Val Ser
        275                 280                 285

act gat ttt gtt ggt gat aac agg tct agc ata ttt gac gcc aag gcc      912
Thr Asp Phe Val Gly Asp Asn Arg Ser Ser Ile Phe Asp Ala Lys Ala
    290                 295                 300

gga att tct ttg aac gac aac ttt gta aaa ctt gtt tcc tgg tat gac 960
Gly Ile Ser Leu Asn Asp Asn Phe Val Lys Leu Val Ser Trp Tyr Asp
305                 310                 315                 320

aac gaa tgg ggt tac agt tcc cgt gtg att gat ttg att gtc cac att 1008
Asn Glu Trp Gly Tyr Ser Ser Arg Val Ile Asp Leu Ile Val His Ile
                325                 330                 335

gca tcc gtg gct 1020
Ala Ser Val Ala
            340

<210> 447
<211> 340
<212> PRT
<213> Glycine max

<400> 447

Met Ala Ser Asp Lys Lys Ile Arg Ile Gly Ile Asn Gly Phe Gly Arg
1               5                   10                  15

Ile Gly Arg Leu Val Ala Arg Val Ala Leu Gln Arg Asn Asp Val Glu
            20                  25                  30

Leu Val Ala Val Asn Asp Pro Phe Ile Thr Thr Asp Tyr Met Thr Tyr
        35                  40                  45

Met Phe Lys Tyr Asp Thr Val His Gly Gln Trp Lys His Phe Asp Val
    50                  55                  60

Lys Val Lys Asp Ser Lys Thr Leu Leu Phe Gly Glu Lys Pro Val Thr
65                  70                  75                  80

Val Phe Gly Ile Arg Asn Pro Glu Glu Ile Pro Trp Gly Glu Val Gly
                85                  90                  95

Ala Asp Tyr Val Val Glu Ser Thr Gly Val Phe Thr Asp Lys Asp Lys
            100                 105                 110

Ala Ala Ala His Leu Lys Gly Gly Ala Lys Lys Val Val Ile Ser Ala
        115                 120                 125

Pro Ser Lys Asp Ala Pro Met Phe Val Val Gly Val Asn Glu Lys Glu
    130                 135                 140

Tyr Lys Pro Glu Leu Asp Ile Val Ser Asn Ala Ser Cys Thr Thr Asn
145                 150                 155                 160

Cys Leu Ala Pro Leu Ala Lys Val Ile Asn Asp Arg Phe Gly Ile Val
                165                 170                 175

Glu Gly Leu Met Thr Thr Val His Ala Ile Thr Ala Thr Gln Lys Thr
            180                 185                 190

Val Asp Gly Pro Ser Asn Lys Asp Trp Arg Gly Gly Arg Ala Ala Ser
        195                 200                 205

Phe Asn Ile Ile Pro Ser Ser Thr Gly Ala Ala Lys Ala Val Gly Lys
    210                 215                 220

Val Leu Pro Ala Leu Asn Gly Lys Leu Thr Gly Met Ser Phe Arg Val
225                 230                 235                 240

Pro Thr Val Asp Val Ser Val Val Asp Leu Thr Val Arg Leu Glu Lys
                245                 250                 255

Pro Ala Thr Tyr Glu Gln Ile Lys Ser Ala Ile Lys Glu Glu Ser Glu
            260                 265                 270

Gly Lys Leu Lys Gly Ile Leu Gly Tyr Thr Glu Asp Asp Val Val Ser
        275                 280                 285

Thr Asp Phe Val Gly Asp Asn Arg Ser Ser Ile Phe Asp Ala Lys Ala
    290                 295                 300

Gly Ile Ser Leu Asn Asp Asn Phe Val Lys Leu Val Ser Trp Tyr Asp
305                 310                 315                 320

Asn Glu Trp Gly Tyr Ser Ser Arg Val Ile Asp Leu Ile Val His Ile
                325                 330                 335

Ala Ser Val Ala
            340

<210>  448
<211>  11
<212>  PRT
<213>  Glycine max

<400>  448

Ala Gly Ile Ser Leu Asn Asp Asn Phe Val Lys
1               5                   10

<210>  449
<211>  27
<212>  PRT
<213>  Glycine max

<400>  449

Gly Ile Leu Gly Tyr Thr Glu Asp Asp Val Val Ser Thr Asp Phe Val
1               5                   10                  15

Gly Asp Asn Arg Ser Ser Ile Phe Asp Ala Lys
            20                  25

<210>  450
<211>  8
<212>  PRT
<213>  Glycine max

<400>  450

Val Leu Pro Ala Leu Asn Gly Lys
1               5

<210>  451
<211>  8
<212>  PRT
<213>  Glycine max

<400>  451

Val Val Ile Ser Ala Pro Ser Lys
1               5

<210>  452
<211>  1050
<212>  DNA
<213>  Glycine max

<220>
<221>  CDS
<222>  (1)..(1050)

<400>  452
atg gtg acc tcg tcg caa aag att ctg gtt acc ggc ggt gcc ggt ttc      48
Met Val Thr Ser Ser Gln Lys Ile Leu Val Thr Gly Gly Ala Gly Phe
1               5                   10                  15

ata ggc acc cac acc gtg gtc cag ctt ctc aaa gga ggc ttc agt gtt      96
Ile Gly Thr His Thr Val Val Gln Leu Leu Lys Gly Gly Phe Ser Val
            20                  25                  30

tca ata atc gac aat ttc gat aac tcc gtc gtg gaa gca gtg gac cgc      144
Ser Ile Ile Asp Asn Phe Asp Asn Ser Val Val Glu Ala Val Asp Arg
        35                  40                  45

gtc cgc caa gtg gtg ggc cct cag ctt tct cag aac ctg gaa ttc acc      192
Val Arg Gln Val Val Gly Pro Gln Leu Ser Gln Asn Leu Glu Phe Thr
    50                  55                  60

cag ggc gat ctc agg aat agg gat gac ttg gag aaa ctc ttc tcc aga      240
Gln Gly Asp Leu Arg Asn Arg Asp Asp Leu Glu Lys Leu Phe Ser Arg
65                  70                  75                  80

aca act ttt gat gcc gtg atc cac ttt gct ggc ctg aaa gcg gtt gct      288
Thr Thr Phe Asp Ala Val Ile His Phe Ala Gly Leu Lys Ala Val Ala

85                  90                  95

gaa agc gtt tcc aag ccc cgt cgc tat ttt gat ttt aat ttg gtc ggc      336
Glu Ser Val Ser Lys Pro Arg Arg Tyr Phe Asp Phe Asn Leu Val Gly
            100                 105                 110

acc atc aac ctc tac caa gtt atg gca aag tat aat tgc aga aag atg      384
Thr Ile Asn Leu Tyr Gln Val Met Ala Lys Tyr Asn Cys Arg Lys Met
        115                 120                 125

gtt ttc tca tca tct gca acc gtt tat ggc caa cct gaa aag ata ccg      432
Val Phe Ser Ser Ser Ala Thr Val Tyr Gly Gln Pro Glu Lys Ile Pro
    130                 135                 140

tgt gag gag gat ttc agg tta caa gct atg aat ccc tat gga cgg acc      480
Cys Glu Glu Asp Phe Arg Leu Gln Ala Met Asn Pro Tyr Gly Arg Thr
145                 150                 155                 160

aag ctt ttc ctg gaa gaa att gcc cga gat att cag aaa gct gaa cca      528
Lys Leu Phe Leu Glu Glu Ile Ala Arg Asp Ile Gln Lys Ala Glu Pro
                165                 170                 175

gaa tgg aag atc ata tta ctg agg tac ttc aat ccg gtt ggg gct cat      576
Glu Trp Lys Ile Ile Leu Leu Arg Tyr Phe Asn Pro Val Gly Ala His
            180                 185                 190

gaa agt ggt aaa ctc ggt gaa gat ccc aag ggc atc cca aat aac ctc      624
Glu Ser Gly Lys Leu Gly Glu Asp Pro Lys Gly Ile Pro Asn Asn Leu
        195                 200                 205

atg cct tac att cag caa gta gcc gtt gga aga ttg ccg gaa ctc aat      672
Met Pro Tyr Ile Gln Gln Val Ala Val Gly Arg Leu Pro Glu Leu Asn
    210                 215                 220

gta tat ggg cat gat tat ccc aca agg gat ggc tct gcg atc cgg gac      720
Val Tyr Gly His Asp Tyr Pro Thr Arg Asp Gly Ser Ala Ile Arg Asp
225                 230                 235                 240

tat atc cat gtg atg gac tta gca gat ggt cat att gct gcc ttg cga      768
Tyr Ile His Val Met Asp Leu Ala Asp Gly His Ile Ala Ala Leu Arg
                245                 250                 255

aag ctc ttc aca aca gag aac ata ggt tgt act gct tac aac ctg gga      816
Lys Leu Phe Thr Thr Glu Asn Ile Gly Cys Thr Ala Tyr Asn Leu Gly
            260                 265                 270

act ggt cgt gga aca tcc gtg ctt gaa atg gtt aca gca ttt gaa aag      864
Thr Gly Arg Gly Thr Ser Val Leu Glu Met Val Thr Ala Phe Glu Lys
        275                 280                 285

gcg tct ggc aag aaa att cca gta aaa tta tgt cca aga aga ccg gga      912
Ala Ser Gly Lys Lys Ile Pro Val Lys Leu Cys Pro Arg Arg Pro Gly
    290                 295                 300

gat gct act gag gtt tat gca tct aca gag aga gct gag aaa gaa ctt      960
Asp Ala Thr Glu Val Tyr Ala Ser Thr Glu Arg Ala Glu Lys Glu Leu
305                 310                 315                 320

gga tgg aaa gca aaa tat ggt gtg gat gag atg tgc agg gac caa tgg     1008
Gly Trp Lys Ala Lys Tyr Gly Val Asp Glu Met Cys Arg Asp Gln Trp
                325                 330                 335

aat tgg gca aag aac aat ccc cag ggt tac acg ggg aag cct             1050

Asn Trp Ala Lys Asn Asn Pro Gln Gly Tyr Thr Gly Lys Pro
            340                 345                 350

<210> 453
<211> 350
<212> PRT
<213> Glycine max

<400> 453

Met Val Thr Ser Ser Gln Lys Ile Leu Val Thr Gly Gly Ala Gly Phe
1               5                   10                  15

Ile Gly Thr His Thr Val Val Gln Leu Leu Lys Gly Gly Phe Ser Val
            20                  25                  30

Ser Ile Ile Asp Asn Phe Asp Asn Ser Val Val Glu Ala Val Asp Arg
        35                  40                  45

Val Arg Gln Val Val Gly Pro Gln Leu Ser Gln Asn Leu Glu Phe Thr
    50                  55                  60

Gln Gly Asp Leu Arg Asn Arg Asp Asp Leu Glu Lys Leu Phe Ser Arg
65                  70                  75                  80

Thr Thr Phe Asp Ala Val Ile His Phe Ala Gly Leu Lys Ala Val Ala
                85                  90                  95

Glu Ser Val Ser Lys Pro Arg Arg Tyr Phe Asp Phe Asn Leu Val Gly
            100                 105                 110

Thr Ile Asn Leu Tyr Gln Val Met Ala Lys Tyr Asn Cys Arg Lys Met
        115                 120                 125

Val Phe Ser Ser Ser Ala Thr Val Tyr Gly Gln Pro Glu Lys Ile Pro
    130                 135                 140

Cys Glu Glu Asp Phe Arg Leu Gln Ala Met Asn Pro Tyr Gly Arg Thr
145                 150                 155                 160

Lys Leu Phe Leu Glu Glu Ile Ala Arg Asp Ile Gln Lys Ala Glu Pro
                165                 170                 175

Glu Trp Lys Ile Ile Leu Leu Arg Tyr Phe Asn Pro Val Gly Ala His
            180                 185                 190

Glu Ser Gly Lys Leu Gly Glu Asp Pro Lys Gly Ile Pro Asn Asn Leu
        195                 200                 205

Met Pro Tyr Ile Gln Gln Val Ala Val Gly Arg Leu Pro Glu Leu Asn
    210                 215                 220

Val Tyr Gly His Asp Tyr Pro Thr Arg Asp Gly Ser Ala Ile Arg Asp
225                 230                 235                 240

Tyr Ile His Val Met Asp Leu Ala Asp Gly His Ile Ala Ala Leu Arg
                245                 250                 255

Lys Leu Phe Thr Thr Glu Asn Ile Gly Cys Thr Ala Tyr Asn Leu Gly
            260                 265                 270

Thr Gly Arg Gly Thr Ser Val Leu Glu Met Val Thr Ala Phe Glu Lys
        275                 280                 285

Ala Ser Gly Lys Lys Ile Pro Val Lys Leu Cys Pro Arg Arg Pro Gly
    290                 295                 300

Asp Ala Thr Glu Val Tyr Ala Ser Thr Glu Arg Ala Glu Lys Glu Leu
305                 310                 315                 320

Gly Trp Lys Ala Lys Tyr Gly Val Asp Glu Met Cys Arg Asp Gln Trp
                325                 330                 335

Asn Trp Ala Lys Asn Asn Pro Gln Gly Tyr Thr Gly Lys Pro
            340                 345                 350

<210>  454
<211>  12
<212>  PRT
<213>  Glycine max

<400>  454

Leu Phe Leu Glu Glu Ile Ala Arg Asp Ile Gln Lys
1               5                   10

<210>  455
<211>  1050
<212>  DNA
<213>  Glycine max

<220>
<221>  CDS
<222>  (1)..(1050)

<400>  455
atg gtg tct tcc tcc caa cac att ctg gtc acc ggt ggt gcc ggt ttc      48
Met Val Ser Ser Ser Gln His Ile Leu Val Thr Gly Gly Ala Gly Phe
1               5                   10                  15

att ggc acc cac acc gtc gtt cag ctt ctc aaa gct ggc ttc agc gtt      96

Ile Gly Thr His Thr Val Val Gln Leu Leu Lys Ala Gly Phe Ser Val
            20                  25                  30

tca ata atc gac aat ttc gat aac tcc gtc atg gaa gca gtg gac cgc      144
Ser Ile Ile Asp Asn Phe Asp Asn Ser Val Met Glu Ala Val Asp Arg
        35                  40                  45

gtc cgc caa gtg gtt ggc cct ctg ctt tct cag aac ctc caa ttc acc      192
Val Arg Gln Val Val Gly Pro Leu Leu Ser Gln Asn Leu Gln Phe Thr
    50                  55                  60

cag ggc gat ctc cgg aat agg gat gac ttg gag aaa ctc ttc tcc aaa      240
Gln Gly Asp Leu Arg Asn Arg Asp Asp Leu Glu Lys Leu Phe Ser Lys
65                  70                  75                  80

aca aca ttt gat gcc gtg atc cac ttt gct ggc ttg aaa gcg gtt gct      288
Thr Thr Phe Asp Ala Val Ile His Phe Ala Gly Leu Lys Ala Val Ala
                85                  90                  95

gaa agc gtt gcg aag ccc cgt cgc tat ttt gat ttt aat ttg gtt ggc      336
Glu Ser Val Ala Lys Pro Arg Arg Tyr Phe Asp Phe Asn Leu Val Gly
            100                 105                 110

acc atc aac ctc tac gaa ttt atg gca aag tat aat tgc aaa aag atg      384
Thr Ile Asn Leu Tyr Glu Phe Met Ala Lys Tyr Asn Cys Lys Lys Met
        115                 120                 125

gtt ttc tca tca tct gca acc gtt tat ggc caa cct gaa aag ata ccg      432
Val Phe Ser Ser Ser Ala Thr Val Tyr Gly Gln Pro Glu Lys Ile Pro
    130                 135                 140

tgt gag gag gat ttc aag tta caa gct atg aat ccc tat gga cgg acc      480
Cys Glu Glu Asp Phe Lys Leu Gln Ala Met Asn Pro Tyr Gly Arg Thr
145                 150                 155                 160

aag ctt ttc ctg gaa gaa att gcc cga gat att cag aaa gct gaa cca      528
Lys Leu Phe Leu Glu Glu Ile Ala Arg Asp Ile Gln Lys Ala Glu Pro
                165                 170                 175

gaa tgg aag atc ata tta ctg aga tac ttc aat cca gtt ggg gct cat      576
Glu Trp Lys Ile Ile Leu Leu Arg Tyr Phe Asn Pro Val Gly Ala His
            180                 185                 190

gaa agt ggc aaa ctc ggt gaa gat ccc aag ggc atc cca aat aac ctc      624
Glu Ser Gly Lys Leu Gly Glu Asp Pro Lys Gly Ile Pro Asn Asn Leu
        195                 200                 205

atg cct tac att cag caa gta gct gtt gga aga ttg act gaa ctc aat      672
Met Pro Tyr Ile Gln Gln Val Ala Val Gly Arg Leu Thr Glu Leu Asn
    210                 215                 220

gta tac ggt cat gat tat cca acg agg gat ggc tct gcg atc cgg gac      720
Val Tyr Gly His Asp Tyr Pro Thr Arg Asp Gly Ser Ala Ile Arg Asp
225                 230                 235                 240

tat atc cat gtg atg gac ttg gca gat ggc cat att gct gcc ctg cga      768
Tyr Ile His Val Met Asp Leu Ala Asp Gly His Ile Ala Ala Leu Arg
                245                 250                 255

aag ctc ttc aca acg gag aac ata ggt tgt act gct tac aac ctg gga      816
Lys Leu Phe Thr Thr Glu Asn Ile Gly Cys Thr Ala Tyr Asn Leu Gly
            260                 265                 270

act ggt cgt gga aca tct gtg ctt gaa atg gtt gca gca ttt gaa aag 864
Thr Gly Arg Gly Thr Ser Val Leu Glu Met Val Ala Ala Phe Glu Lys
        275                 280                 285

gct tct ggc aag aaa att cca gta aaa tta tgt cca aga aga ccg gga 912
Ala Ser Gly Lys Lys Ile Pro Val Lys Leu Cys Pro Arg Arg Pro Gly
    290                 295                 300

gat gcg act gag gtt tat gca tct aca gag aga gct gag aaa gaa ctt 960
Asp Ala Thr Glu Val Tyr Ala Ser Thr Glu Arg Ala Glu Lys Glu Leu
305                 310                 315                 320

ggt tgg aag gca aac tat ggt gtg gag gag atg tgc agg gac caa tgg 1008
Gly Trp Lys Ala Asn Tyr Gly Val Glu Glu Met Cys Arg Asp Gln Trp
                325                 330                 335

aat tgg gca aag aac aat ccc tgg ggt tac gcg ggg aag cct 1050
Asn Trp Ala Lys Asn Asn Pro Trp Gly Tyr Ala Gly Lys Pro
            340                 345                 350

<210> 456
<211> 350
<212> PRT
<213> Glycine max

<400> 456

Met Val Ser Ser Ser Gln His Ile Leu Val Thr Gly Gly Ala Gly Phe
1               5                   10                  15

Ile Gly Thr His Thr Val Val Gln Leu Leu Lys Ala Gly Phe Ser Val
            20                  25                  30

Ser Ile Ile Asp Asn Phe Asp Asn Ser Val Met Glu Ala Val Asp Arg
        35                  40                  45

Val Arg Gln Val Val Gly Pro Leu Leu Ser Gln Asn Leu Gln Phe Thr
    50                  55                  60

Gln Gly Asp Leu Arg Asn Arg Asp Asp Leu Glu Lys Leu Phe Ser Lys
65                  70                  75                  80

Thr Thr Phe Asp Ala Val Ile His Phe Ala Gly Leu Lys Ala Val Ala
                85                  90                  95

Glu Ser Val Ala Lys Pro Arg Arg Tyr Phe Asp Phe Asn Leu Val Gly
            100                 105                 110

Thr Ile Asn Leu Tyr Glu Phe Met Ala Lys Tyr Asn Cys Lys Lys Met
        115                 120                 125

Val Phe Ser Ser Ser Ala Thr Val Tyr Gly Gln Pro Glu Lys Ile Pro
    130                 135                 140

Cys Glu Glu Asp Phe Lys Leu Gln Ala Met Asn Pro Tyr Gly Arg Thr
145                 150                 155                 160

Lys Leu Phe Leu Glu Glu Ile Ala Arg Asp Ile Gln Lys Ala Glu Pro
                165                 170                 175

Glu Trp Lys Ile Ile Leu Leu Arg Tyr Phe Asn Pro Val Gly Ala His
            180                 185                 190

Glu Ser Gly Lys Leu Gly Glu Asp Pro Lys Gly Ile Pro Asn Asn Leu
        195                 200                 205

Met Pro Tyr Ile Gln Gln Val Ala Val Gly Arg Leu Thr Glu Leu Asn
    210                 215                 220

Val Tyr Gly His Asp Tyr Pro Thr Arg Asp Gly Ser Ala Ile Arg Asp
225                 230                 235                 240

Tyr Ile His Val Met Asp Leu Ala Asp Gly His Ile Ala Ala Leu Arg
                245                 250                 255

Lys Leu Phe Thr Thr Glu Asn Ile Gly Cys Thr Ala Tyr Asn Leu Gly
            260                 265                 270

Thr Gly Arg Gly Thr Ser Val Leu Glu Met Val Ala Ala Phe Glu Lys
        275                 280                 285

Ala Ser Gly Lys Lys Ile Pro Val Lys Leu Cys Pro Arg Arg Pro Gly
    290                 295                 300

Asp Ala Thr Glu Val Tyr Ala Ser Thr Glu Arg Ala Glu Lys Glu Leu
305                 310                 315                 320

Gly Trp Lys Ala Asn Tyr Gly Val Glu Glu Met Cys Arg Asp Gln Trp
                325                 330                 335

Asn Trp Ala Lys Asn Asn Pro Trp Gly Tyr Ala Gly Lys Pro
            340                 345                 350

<210>  457
<211>  12
<212>  PRT
<213>  Glycine max

<400>  457

Leu Phe Leu Glu Glu Ile Ala Arg Asp Ile Gln Lys
1               5                   10

<210>  458
<211>  13
<212>  PRT
<213>  Glycine max

<400>  458

Thr Thr Phe Asp Ala Val Ile His Phe Ala Gly Leu Lys
1               5                   10


<210>  459
<211>  1341
<212>  DNA
<213>  Glycine max


<220>
<221>  CDS
<222>  (1)..(1341)

<400>  459
atg ggt aag gaa aag act cac atc aac att gtc gtc att gga cat gtc      48
Met Gly Lys Glu Lys Thr His Ile Asn Ile Val Val Ile Gly His Val
1               5                   10                  15

gac tct ggg aag tca act acc act ggt cat ttg atc tac aag ctt gga      96
Asp Ser Gly Lys Ser Thr Thr Thr Gly His Leu Ile Tyr Lys Leu Gly
            20                  25                  30

ggt att gac aag cgt gtg att gag agg ttc gag aag gag gct gct gag      144
Gly Ile Asp Lys Arg Val Ile Glu Arg Phe Glu Lys Glu Ala Ala Glu
        35                  40                  45

atg aac aag agg tca ttc aag tat gcc tgg gtg ctt gac aag ctc aag      192
Met Asn Lys Arg Ser Phe Lys Tyr Ala Trp Val Leu Asp Lys Leu Lys
    50                  55                  60

gct gag cgc gaa aga gga att acc att gat att gct ttg tgg aag ttt      240
Ala Glu Arg Glu Arg Gly Ile Thr Ile Asp Ile Ala Leu Trp Lys Phe
65                  70                  75                  80

gaa acc acc aag tac tac tgc aca gtc atc gat gcc ccc gga cat cgt      288
Glu Thr Thr Lys Tyr Tyr Cys Thr Val Ile Asp Ala Pro Gly His Arg
                85                  90                  95

gac ttt atc aag aac atg att act ggt acc tcc cag gct gat tgt gct      336
Asp Phe Ile Lys Asn Met Ile Thr Gly Thr Ser Gln Ala Asp Cys Ala
            100                 105                 110

gtc ctt atc att gac tcc acc act ggt ggt ttt gaa gct ggt att tct      384
Val Leu Ile Ile Asp Ser Thr Thr Gly Gly Phe Glu Ala Gly Ile Ser
        115                 120                 125

aag gat gga cag act cgt gag cat gct ctt ctt gct ttc acc ctt ggt      432
Lys Asp Gly Gln Thr Arg Glu His Ala Leu Leu Ala Phe Thr Leu Gly
    130                 135                 140

gtg aag cag atg atc tgc tgc tgt aac aag atg gat gcc act acc ccg      480
Val Lys Gln Met Ile Cys Cys Cys Asn Lys Met Asp Ala Thr Thr Pro
145                 150                 155                 160

aag tac tct aag gct agg tat gat gaa att gtg aag gaa gtc tct tcc 528
Lys Tyr Ser Lys Ala Arg Tyr Asp Glu Ile Val Lys Glu Val Ser Ser
                165                 170                 175

tac ttg aag aag gtt ggt tac aac cca gac aag att cca ttt gtg ccc 576
Tyr Leu Lys Lys Val Gly Tyr Asn Pro Asp Lys Ile Pro Phe Val Pro
            180                 185                 190

atc tct ggt ttc gag ggt gac aac atg att gag agg tcc acc aac ctt 624
Ile Ser Gly Phe Glu Gly Asp Asn Met Ile Glu Arg Ser Thr Asn Leu
        195                 200                 205

gac tgg tac aag gga cca act ctc ctt gag gct ctt gac caa atc aat 672
Asp Trp Tyr Lys Gly Pro Thr Leu Leu Glu Ala Leu Asp Gln Ile Asn
    210                 215                 220

gag ccc aag agg ccc tca gac aag cca ctc agg ctt cca ttg cag gat 720
Glu Pro Lys Arg Pro Ser Asp Lys Pro Leu Arg Leu Pro Leu Gln Asp
225                 230                 235                 240

gtc tac aaa att ggt ggt att ggt acc gtg cca gtg gga cgt gtt gag 768
Val Tyr Lys Ile Gly Gly Ile Gly Thr Val Pro Val Gly Arg Val Glu
                245                 250                 255

act ggt gtc gtg aag ccc ggt atg gtg gtg act ttt ggt ccc act ggg 816
Thr Gly Val Val Lys Pro Gly Met Val Val Thr Phe Gly Pro Thr Gly
            260                 265                 270

ctg aca act gag gtt aag tct gtt gag atg cac cat gag gct ctc aca 864
Leu Thr Thr Glu Val Lys Ser Val Glu Met His His Glu Ala Leu Thr
        275                 280                 285

gag gct ctt cca ggt gac aat gtt ggc ttt aat gtg aag aat gtt gca 912
Glu Ala Leu Pro Gly Asp Asn Val Gly Phe Asn Val Lys Asn Val Ala
    290                 295                 300

gtc aag gat ctc aag cgt ggt ttt gtt gca tcc aac tcc aag gat gac 960
Val Lys Asp Leu Lys Arg Gly Phe Val Ala Ser Asn Ser Lys Asp Asp
305                 310                 315                 320

cct gcc aag gaa gct gcc aac ttc aca tct caa gtc att atc atg aac 1008
Pro Ala Lys Glu Ala Ala Asn Phe Thr Ser Gln Val Ile Ile Met Asn
                325                 330                 335

cac cct ggc cag att gga aat gga tac gca ccg gtg ctt gat tgc cac 1056
His Pro Gly Gln Ile Gly Asn Gly Tyr Ala Pro Val Leu Asp Cys His
            340                 345                 350

act tct cac att gct gtc aag ttt tct gaa atc ttg acc aag att gac 1104
Thr Ser His Ile Ala Val Lys Phe Ser Glu Ile Leu Thr Lys Ile Asp
        355                 360                 365

agg cga tct ggt aag gag ctt gag aag gag ccc aaa ttt ttg aag aat 1152
Arg Arg Ser Gly Lys Glu Leu Glu Lys Glu Pro Lys Phe Leu Lys Asn
    370                 375                 380

ggt gat gca ggt atg gtt aag atg att cca acc aag ccc atg gtg gtt 1200
Gly Asp Ala Gly Met Val Lys Met Ile Pro Thr Lys Pro Met Val Val
385                 390                 395                 400

gaa act ttc tct gag tat cct ccc ctt ggt cgt ttt gct gtg agg gac 1248
Glu Thr Phe Ser Glu Tyr Pro Pro Leu Gly Arg Phe Ala Val Arg Asp
                405                 410                 415

atg cgt caa act gtg gct gtc gga gtc atc aag agt gtt gag aag aaa          1296
Met Arg Gln Thr Val Ala Val Gly Val Ile Lys Ser Val Glu Lys Lys
            420                 425                 430

gac ccc acc gga gcc aag gtc acc aag gcc gct gcc aag aag aag             1341
Asp Pro Thr Gly Ala Lys Val Thr Lys Ala Ala Ala Lys Lys Lys
        435                 440                 445

<210>  460
<211>  447
<212>  PRT
<213>  Glycine max

<400>  460

Met Gly Lys Glu Lys Thr His Ile Asn Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ala Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Asp Glu Ile Val Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Glu Ala Leu Asp Gln Ile Asn
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Val Lys Pro Gly Met Val Val Thr Phe Gly Pro Thr Gly
            260                 265                 270

Leu Thr Thr Glu Val Lys Ser Val Glu Met His His Glu Ala Leu Thr
        275                 280                 285

Glu Ala Leu Pro Gly Asp Asn Val Gly Phe Asn Val Lys Asn Val Ala
    290                 295                 300

Val Lys Asp Leu Lys Arg Gly Phe Val Ala Ser Asn Ser Lys Asp Asp
305                 310                 315                 320

Pro Ala Lys Glu Ala Ala Asn Phe Thr Ser Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ser Glu Ile Leu Thr Lys Ile Asp
        355                 360                 365

Arg Arg Ser Gly Lys Glu Leu Glu Lys Glu Pro Lys Phe Leu Lys Asn
    370                 375                 380

Gly Asp Ala Gly Met Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Ser Val Glu Lys Lys
            420                 425                 430

Asp Pro Thr Gly Ala Lys Val Thr Lys Ala Ala Ala Lys Lys Lys

435                 440                 445

<210>  461
<211>  8
<212>  PRT
<213>  Glycine max

<400>  461

Ala Arg Tyr Asp Glu Ile Val Lys
1               5

<210>  462
<211>  17
<212>  PRT
<213>  Glycine max

<400>  462

Asp Gly Gln Thr Arg Glu His Ala Leu Leu Ala Phe Thr Leu Gly Val
1               5                   10                  15

Lys

<210>  463
<211>  7
<212>  PRT
<213>  Glycine max

<400>  463

Glu Val Ser Ser Tyr Leu Lys
1               5

<210>  464
<211>  7
<212>  PRT
<213>  Glycine max

<400>  464

Phe Ser Glu Ile Leu Thr Lys
1               5

<210>  465
<211>  10
<212>  PRT
<213>  Glycine max

<400>  465

Ser Thr Thr Thr Gly His Leu Ile Tyr Lys
1               5                   10

<210>  466

<211>  15
<212>  PRT
<213>  Glycine max

<400>  466

Thr His Ile Asn Ile Val Val Ile Gly His Val Asp Ser Gly Lys
1               5                   10                  15


<210>  467
<211>  1341
<212>  DNA
<213>  Glycine max


<220>
<221>  CDS
<222>  (1)..(1341)

<400>  467
atg ggt aaa gag aag gtt cac att aac att gtg gtc att ggc cat gtt      48
Met Gly Lys Glu Lys Val His Ile Asn Ile Val Val Ile Gly His Val
1               5                   10                  15

gac tct ggg aag tca acc acc act ggc cac ttg att tac aag ctt gga      96
Asp Ser Gly Lys Ser Thr Thr Thr Gly His Leu Ile Tyr Lys Leu Gly
            20                  25                  30

ggt att gac aag cgt gtt att gag agg ttt gag aaa gaa gct gct gag      144
Gly Ile Asp Lys Arg Val Ile Glu Arg Phe Glu Lys Glu Ala Ala Glu
        35                  40                  45

atg aac aag agg tca ttc aaa tac gcc tgg gtg ctt gac aag ctt aag      192
Met Asn Lys Arg Ser Phe Lys Tyr Ala Trp Val Leu Asp Lys Leu Lys
    50                  55                  60

gct gag cgt gag aga gga att acc att gat att gca tta tgg aag ttt      240
Ala Glu Arg Glu Arg Gly Ile Thr Ile Asp Ile Ala Leu Trp Lys Phe
65                  70                  75                  80

gag acc acc aaa tac tac tgc act gtt att gat gct cca gga cat cgt      288
Glu Thr Thr Lys Tyr Tyr Cys Thr Val Ile Asp Ala Pro Gly His Arg
                85                  90                  95

gat ttc atc aag aac atg att act gga act tcc cag gct gat tgt gct      336
Asp Phe Ile Lys Asn Met Ile Thr Gly Thr Ser Gln Ala Asp Cys Ala
            100                 105                 110

gtc ctt atc att gat tcc aca act ggt ggt ttt gaa gct ggt att tcc      384
Val Leu Ile Ile Asp Ser Thr Thr Gly Gly Phe Glu Ala Gly Ile Ser
        115                 120                 125

aaa gat gga cag act cgc gag cat gct cta ctt gcc ttc acc ctt ggt      432
Lys Asp Gly Gln Thr Arg Glu His Ala Leu Leu Ala Phe Thr Leu Gly
    130                 135                 140

gtc aag caa atg atc tgt tgc tgt aac aag atg gat gcc act acc cca      480
Val Lys Gln Met Ile Cys Cys Cys Asn Lys Met Asp Ala Thr Thr Pro
145                 150                 155                 160

aag tac tct aag gcc agg tat gaa gaa att gtg aag gaa gtt tct tcc      528
Lys Tyr Ser Lys Ala Arg Tyr Glu Glu Ile Val Lys Glu Val Ser Ser

165                 170                 175

tac ttg aag aag gta ggt tac aac cca gac aag att cca ttt gtt ccc      576
Tyr Leu Lys Lys Val Gly Tyr Asn Pro Asp Lys Ile Pro Phe Val Pro
            180                 185                 190

atc tca ggt ttt gag ggt gac aac atg att gag agg tcc acc aat ctt      624
Ile Ser Gly Phe Glu Gly Asp Asn Met Ile Glu Arg Ser Thr Asn Leu
        195                 200                 205

gat tgg tac aag gga cca acc ctt ctc gag gct ctt gac cag atc aat      672
Asp Trp Tyr Lys Gly Pro Thr Leu Leu Glu Ala Leu Asp Gln Ile Asn
    210                 215                 220

gag ccc aag aga ccc tca gac aag cct ttg agg ctt ccc ttg cag gat      720
Glu Pro Lys Arg Pro Ser Asp Lys Pro Leu Arg Leu Pro Leu Gln Asp
225                 230                 235                 240

gtc tac aag att ggt ggt att gga aca gtg cca gtg gga cga gtt gaa      768
Val Tyr Lys Ile Gly Gly Ile Gly Thr Val Pro Val Gly Arg Val Glu
                245                 250                 255

act ggt gtc ttg aag ccc ggt atg gtg gtg act ttt gct ccc act ggc      816
Thr Gly Val Leu Lys Pro Gly Met Val Val Thr Phe Ala Pro Thr Gly
            260                 265                 270

ctg aca act gaa gtt aag tct gtg gag atg cac cat gaa gct ctc cag      864
Leu Thr Thr Glu Val Lys Ser Val Glu Met His His Glu Ala Leu Gln
        275                 280                 285

gag gcc ctt cca ggt gac aac gta gga ttt aat gtg aag aat gtt gca      912
Glu Ala Leu Pro Gly Asp Asn Val Gly Phe Asn Val Lys Asn Val Ala
    290                 295                 300

gtc aag gat ctc aag cgt ggt ttt gtt gcc tca aac tct aag gat gat      960
Val Lys Asp Leu Lys Arg Gly Phe Val Ala Ser Asn Ser Lys Asp Asp
305                 310                 315                 320

cct gcc aag gag gct gcc aac ttc aca tcc caa gtc att ata atg aac     1008
Pro Ala Lys Glu Ala Ala Asn Phe Thr Ser Gln Val Ile Ile Met Asn
                325                 330                 335

cat cct ggc caa att gga aat ggt tat gct cct gtg ctt gac tgc cac     1056
His Pro Gly Gln Ile Gly Asn Gly Tyr Ala Pro Val Leu Asp Cys His
            340                 345                 350

act tct cac att gcc gtc aag ttt gcc gaa ctt gtg acc aag att gac     1104
Thr Ser His Ile Ala Val Lys Phe Ala Glu Leu Val Thr Lys Ile Asp
        355                 360                 365

agg cga tct ggt aag gag att gag aag gag ccc aaa ttc ttg aaa aat     1152
Arg Arg Ser Gly Lys Glu Ile Glu Lys Glu Pro Lys Phe Leu Lys Asn
    370                 375                 380

gga gat gca ggt tat gtt aag atg att ccc acc aag cct atg gtg gtc     1200
Gly Asp Ala Gly Tyr Val Lys Met Ile Pro Thr Lys Pro Met Val Val
385                 390                 395                 400

gaa act ttc tcc gag tat cct cct ctt ggt cgt ttt gcc gtg agg gac     1248
Glu Thr Phe Ser Glu Tyr Pro Pro Leu Gly Arg Phe Ala Val Arg Asp
                405                 410                 415

atg cga caa act gtg gct gtt gga gtc atc aag agt gtg gag aag aag     1296

Met Arg Gln Thr Val Ala Val Gly Val Ile Lys Ser Val Glu Lys Lys
            420                 425                 430

gac cca act gga gcc aag gtc acc aag gct gca cag aag aag aag         1341
Asp Pro Thr Gly Ala Lys Val Thr Lys Ala Ala Gln Lys Lys Lys
        435                 440                 445

<210>  468
<211>  447
<212>  PRT
<213>  Glycine max

<400>  468

Met Gly Lys Glu Lys Val His Ile Asn Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ala Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Glu Glu Ile Val Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Glu Ala Leu Asp Gln Ile Asn
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Leu Lys Pro Gly Met Val Val Thr Phe Ala Pro Thr Gly
            260                 265                 270

Leu Thr Thr Glu Val Lys Ser Val Glu Met His His Glu Ala Leu Gln
        275                 280                 285

Glu Ala Leu Pro Gly Asp Asn Val Gly Phe Asn Val Lys Asn Val Ala
    290                 295                 300

Val Lys Asp Leu Lys Arg Gly Phe Val Ala Ser Asn Ser Lys Asp Asp
305                 310                 315                 320

Pro Ala Lys Glu Ala Ala Asn Phe Thr Ser Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ala Glu Leu Val Thr Lys Ile Asp
        355                 360                 365

Arg Arg Ser Gly Lys Glu Ile Glu Lys Glu Pro Lys Phe Leu Lys Asn
    370                 375                 380

Gly Asp Ala Gly Tyr Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Ser Val Glu Lys Lys
            420                 425                 430

Asp Pro Thr Gly Ala Lys Val Thr Lys Ala Ala Gln Lys Lys Lys
        435                 440                 445

<210>  469
<211>  17
<212>  PRT
<213>  Glycine max

<400>  469

Asp Gly Gln Thr Arg Glu His Ala Leu Leu Ala Phe Thr Leu Gly Val
1               5                   10                  15

Lys

<210>  470
<211>  7
<212>  PRT
<213>  Glycine max

<400>  470

Glu Val Ser Ser Tyr Leu Lys
1               5

<210>  471
<211>  10
<212>  PRT
<213>  Glycine max

<400>  471

Ser Thr Thr Thr Gly His Leu Ile Tyr Lys
1               5                   10

<210>  472
<211>  1344
<212>  DNA
<213>  Glycine max

<220>
<221>  CDS
<222>  (1)..(1344)

<400>  472
atg ggt aag gaa aag gtt cac atc agt att gtg gtc att ggc cat gtc       48
Met Gly Lys Glu Lys Val His Ile Ser Ile Val Val Ile Gly His Val
1               5                   10                  15

gac tct ggg aaa tcc act acc act ggt cac ctg att tac aag ctt gga       96
Asp Ser Gly Lys Ser Thr Thr Thr Gly His Leu Ile Tyr Lys Leu Gly
            20                  25                  30

ggc att gac aag cgt gtt att gag agg ttt gag aag gaa gct gct gag      144
Gly Ile Asp Lys Arg Val Ile Glu Arg Phe Glu Lys Glu Ala Ala Glu
        35                  40                  45

atg aac aag agg tct ttc aag tat gcc tgg gtg ctg gac aaa ctt aag      192
Met Asn Lys Arg Ser Phe Lys Tyr Ala Trp Val Leu Asp Lys Leu Lys

50                  55                  60

gct gag cgt gaa aga gga atc acc att gat att gct ttg tgg aag ttt          240
Ala Glu Arg Glu Arg Gly Ile Thr Ile Asp Ile Ala Leu Trp Lys Phe
65                  70                  75                  80

gaa aca aca aag tat tat tgc aca gtt att gat gcg cct gga cat agg          288
Glu Thr Thr Lys Tyr Tyr Cys Thr Val Ile Asp Ala Pro Gly His Arg
                85                  90                  95

gat ttc att aag aat atg att act ggg aca tcc caa gct gac tgt gct          336
Asp Phe Ile Lys Asn Met Ile Thr Gly Thr Ser Gln Ala Asp Cys Ala
            100                 105                 110

gtt ctt atc att gat tcg acc act ggt ggt ttt gaa gct ggt att tcc          384
Val Leu Ile Ile Asp Ser Thr Thr Gly Gly Phe Glu Ala Gly Ile Ser
        115                 120                 125

aag gat gga cag act cgt gaa cat gct ctg ctt tca ttc acc ctt ggt          432
Lys Asp Gly Gln Thr Arg Glu His Ala Leu Leu Ser Phe Thr Leu Gly
    130                 135                 140

gtg aaa cag atg att tgc tgc tgt aac aaa atg gat gct act aca ccc          480
Val Lys Gln Met Ile Cys Cys Cys Asn Lys Met Asp Ala Thr Thr Pro
145                 150                 155                 160

aag tat tcc aag gcc agg tat gat gaa att gtg aag gaa gtc tct tcc          528
Lys Tyr Ser Lys Ala Arg Tyr Asp Glu Ile Val Lys Glu Val Ser Ser
                165                 170                 175

tac ttg aag aaa gta gga tac aac cct gac aag att cct ttt gtt cct          576
Tyr Leu Lys Lys Val Gly Tyr Asn Pro Asp Lys Ile Pro Phe Val Pro
            180                 185                 190

atc tct ggt ttt gag gga gac aac atg att gag agg tcc aca aac ctt          624
Ile Ser Gly Phe Glu Gly Asp Asn Met Ile Glu Arg Ser Thr Asn Leu
        195                 200                 205

gac tgg tac aag ggt cca act ctg ctt gat gca ctt gac cag att agt          672
Asp Trp Tyr Lys Gly Pro Thr Leu Leu Asp Ala Leu Asp Gln Ile Ser
    210                 215                 220

gag ccc aag agg ccc tct gac aag ccc ctc agg ctt ccc ctt cag gat          720
Glu Pro Lys Arg Pro Ser Asp Lys Pro Leu Arg Leu Pro Leu Gln Asp
225                 230                 235                 240

gtg tac aag att gga ggt att gga act gtg cca gtg gga cgt gtt gag          768
Val Tyr Lys Ile Gly Gly Ile Gly Thr Val Pro Val Gly Arg Val Glu
                245                 250                 255

acc ggt gtc ttg aag cct gga atg gtg gtg act ttt gca cca act gga          816
Thr Gly Val Leu Lys Pro Gly Met Val Val Thr Phe Ala Pro Thr Gly
            260                 265                 270

ctg aca act gaa gtc aag tct gtg gag atg cac cat gaa tct ctt aca          864
Leu Thr Thr Glu Val Lys Ser Val Glu Met His His Glu Ser Leu Thr
        275                 280                 285

gag gca cat cct ggt gac aat gtg gga ttc aat gtt aag aat gtt gct          912
Glu Ala His Pro Gly Asp Asn Val Gly Phe Asn Val Lys Asn Val Ala
    290                 295                 300

gtt aag gat ttg aag cgt ggt tat gtt gcc tca aac tca aag gat gac          960

Val Lys Asp Leu Lys Arg Gly Tyr Val Ala Ser Asn Ser Lys Asp Asp
305                 310                 315                 320

cct gca aag gag gct gct aac ttc aca gcc caa gtc atc atc atg aac      1008
Pro Ala Lys Glu Ala Ala Asn Phe Thr Ala Gln Val Ile Ile Met Asn
                325                 330                 335

cac cct ggt cag att gga aat ggc tat gcc cct gtc ctc gac tgc cac      1056
His Pro Gly Gln Ile Gly Asn Gly Tyr Ala Pro Val Leu Asp Cys His
            340                 345                 350

act tct cac att gct gtc aag ttt gct gaa ctc atg acc aag att gac      1104
Thr Ser His Ile Ala Val Lys Phe Ala Glu Leu Met Thr Lys Ile Asp
        355                 360                 365

agg cga tcc ggc aaa gag ctt gag aag gag ccc aag ttt ttg aag aac      1152
Arg Arg Ser Gly Lys Glu Leu Glu Lys Glu Pro Lys Phe Leu Lys Asn
    370                 375                 380

ggt gat gct ggt ttt gtt aag atg att cca acc aaa ccc atg gtt gtt      1200
Gly Asp Ala Gly Phe Val Lys Met Ile Pro Thr Lys Pro Met Val Val
385                 390                 395                 400

gaa act ttc tcc gag tat cct cca ctt ggt agg ttt gct gtt agg gac      1248
Glu Thr Phe Ser Glu Tyr Pro Pro Leu Gly Arg Phe Ala Val Arg Asp
                405                 410                 415

atg cgt caa act gtt gct gtg gga gtc atc aag aac gtt gag aag aag      1296
Met Arg Gln Thr Val Ala Val Gly Val Ile Lys Asn Val Glu Lys Lys
            420                 425                 430

gat cct acc gga gcc aag gtc acc aag gct gcc cag aag aag aag tga      1344
Asp Pro Thr Gly Ala Lys Val Thr Lys Ala Ala Gln Lys Lys Lys
        435                 440                 445

<210>  473
<211>  447
<212>  PRT
<213>  Glycine max

<400>  473

Met Gly Lys Glu Lys Val His Ile Ser Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ser Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Asp Glu Ile Val Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Asp Ala Leu Asp Gln Ile Ser
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Leu Lys Pro Gly Met Val Val Thr Phe Ala Pro Thr Gly
            260                 265                 270

Leu Thr Thr Glu Val Lys Ser Val Glu Met His His Glu Ser Leu Thr
        275                 280                 285

Glu Ala His Pro Gly Asp Asn Val Gly Phe Asn Val Lys Asn Val Ala
    290                 295                 300

Val Lys Asp Leu Lys Arg Gly Tyr Val Ala Ser Asn Ser Lys Asp Asp
305                 310                 315                 320

Pro Ala Lys Glu Ala Ala Asn Phe Thr Ala Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ala Glu Leu Met Thr Lys Ile Asp
        355                 360                 365

Arg Arg Ser Gly Lys Glu Leu Glu Lys Glu Pro Lys Phe Leu Lys Asn
    370                 375                 380

Gly Asp Ala Gly Phe Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Asn Val Glu Lys Lys
            420                 425                 430

Asp Pro Thr Gly Ala Lys Val Thr Lys Ala Ala Gln Lys Lys Lys
        435                 440                 445

<210>  474
<211>  8
<212>  PRT
<213>  Glycine max

<400>  474

Ala Arg Tyr Asp Glu Ile Val Lys
1               5

<210>  475
<211>  17
<212>  PRT
<213>  Glycine max

<400>  475

Asp Gly Gln Thr Arg Glu His Ala Leu Leu Ser Phe Thr Leu Gly Val
1               5                   10                  15

Lys

<210>  476
<211>  7
<212>  PRT
<213>  Glycine max

<400>  476

Glu Val Ser Ser Tyr Leu Lys

1               5


<210>  477
<211>  10
<212>  PRT
<213>  Glycine max

<400>  477

Ser Thr Thr Thr Gly His Leu Ile Tyr Lys
1               5                   10


<210>  478
<211>  882
<212>  DNA
<213>  Glycine max


<220>
<221>  CDS
<222>  (1)..(882)

<400>  478
atg gct tcc ggt gaa cag aaa ttc cct cct caa caa caa caa aca cag      48
Met Ala Ser Gly Glu Gln Lys Phe Pro Pro Gln Gln Gln Gln Thr Gln
1               5                   10                  15

cct ggg aag gag cat gct atg act cca gta ccc caa ttc act agc cct      96
Pro Gly Lys Glu His Ala Met Thr Pro Val Pro Gln Phe Thr Ser Pro
            20                  25                  30

gac tac aag cct tca aat aaa ctt caa ggg aag att gca tta gtc act      144
Asp Tyr Lys Pro Ser Asn Lys Leu Gln Gly Lys Ile Ala Leu Val Thr
        35                  40                  45

ggg ggt gat tct ggg att gga cga gcg gtg tgt aac ttg ttt gcc tta      192
Gly Gly Asp Ser Gly Ile Gly Arg Ala Val Cys Asn Leu Phe Ala Leu
    50                  55                  60

gaa ggt gct acc gtg gcc ttc acg tat gtg aag ggg cat gag gac aag      240
Glu Gly Ala Thr Val Ala Phe Thr Tyr Val Lys Gly His Glu Asp Lys
65                  70                  75                  80

gac gcg agg gac aca ttg gaa atg atc aag aga gca aag act tcg gat      288
Asp Ala Arg Asp Thr Leu Glu Met Ile Lys Arg Ala Lys Thr Ser Asp
                85                  90                  95

gcc aag gat cca atg gca ata gca tct gat ttg ggt tac gat gag aac      336
Ala Lys Asp Pro Met Ala Ile Ala Ser Asp Leu Gly Tyr Asp Glu Asn
            100                 105                 110

tgc aag agg gtg gtt gat gag gtc gtg agt gct tat ggt tgt att gac      384
Cys Lys Arg Val Val Asp Glu Val Val Ser Ala Tyr Gly Cys Ile Asp
        115                 120                 125

att ctg gtc aac aat gca gct gag cag tac gag tgt gga acc gtg gag      432
Ile Leu Val Asn Asn Ala Ala Glu Gln Tyr Glu Cys Gly Thr Val Glu
    130                 135                 140

gac ata gac gag cct agg ctt gag agg gtc ttt cgt aca aat atc ttc      480
Asp Ile Asp Glu Pro Arg Leu Glu Arg Val Phe Arg Thr Asn Ile Phe

145                 150                 155                 160

tcc tat ttc ttc atg gcg agg cat gcc ttg aag cac atg aag gaa gga         528
Ser Tyr Phe Phe Met Ala Arg His Ala Leu Lys His Met Lys Glu Gly
                165                 170                 175

agc agc att atc aac acg aca tca gtg aat gca tac aag gga cat gcg         576
Ser Ser Ile Ile Asn Thr Thr Ser Val Asn Ala Tyr Lys Gly His Ala
            180                 185                 190

aaa cta ttg gac tac acg tcc acc aag ggg gca att gtg gcc tat aca         624
Lys Leu Leu Asp Tyr Thr Ser Thr Lys Gly Ala Ile Val Ala Tyr Thr
        195                 200                 205

agg ggt ctt gcc ctt cag ctg gtg agt aag gga att cgg gtt aat ggg         672
Arg Gly Leu Ala Leu Gln Leu Val Ser Lys Gly Ile Arg Val Asn Gly
    210                 215                 220

gtg gct cca ggg ccc att tgg acc cct ttg ata cca gcc tct ttc aag         720
Val Ala Pro Gly Pro Ile Trp Thr Pro Leu Ile Pro Ala Ser Phe Lys
225                 230                 235                 240

gag gaa gaa acg gcc caa ttt gga gcg cag gtc cca atg aag aga gct         768
Glu Glu Glu Thr Ala Gln Phe Gly Ala Gln Val Pro Met Lys Arg Ala
                245                 250                 255

ggt caa cct att gag gtt gct cct tcc tat gtt ttt ctt gct tcc aac         816
Gly Gln Pro Ile Glu Val Ala Pro Ser Tyr Val Phe Leu Ala Ser Asn
            260                 265                 270

caa tgc tcc tct tac ata act gga caa gtc ctt cac ccc aat ggt gga         864
Gln Cys Ser Ser Tyr Ile Thr Gly Gln Val Leu His Pro Asn Gly Gly
        275                 280                 285

acc gtt gtg aat ggt taa                                                 882
Thr Val Val Asn Gly
    290

<210>  479
<211>  293
<212>  PRT
<213>  Glycine max

<400>  479

Met Ala Ser Gly Glu Gln Lys Phe Pro Pro Gln Gln Gln Gln Thr Gln
1               5                   10                  15

Pro Gly Lys Glu His Ala Met Thr Pro Val Pro Gln Phe Thr Ser Pro
            20                  25                  30

Asp Tyr Lys Pro Ser Asn Lys Leu Gln Gly Lys Ile Ala Leu Val Thr
        35                  40                  45

Gly Gly Asp Ser Gly Ile Gly Arg Ala Val Cys Asn Leu Phe Ala Leu
    50                  55                  60

Glu Gly Ala Thr Val Ala Phe Thr Tyr Val Lys Gly His Glu Asp Lys

65                  70                  75                  80

Asp Ala Arg Asp Thr Leu Glu Met Ile Lys Arg Ala Lys Thr Ser Asp
                85                  90                  95

Ala Lys Asp Pro Met Ala Ile Ala Ser Asp Leu Gly Tyr Asp Glu Asn
            100                 105                 110

Cys Lys Arg Val Val Asp Glu Val Val Ser Ala Tyr Gly Cys Ile Asp
        115                 120                 125

Ile Leu Val Asn Asn Ala Ala Glu Gln Tyr Glu Cys Gly Thr Val Glu
    130                 135                 140

Asp Ile Asp Glu Pro Arg Leu Glu Arg Val Phe Arg Thr Asn Ile Phe
145                 150                 155                 160

Ser Tyr Phe Phe Met Ala Arg His Ala Leu Lys His Met Lys Glu Gly
                165                 170                 175

Ser Ser Ile Ile Asn Thr Thr Ser Val Asn Ala Tyr Lys Gly His Ala
            180                 185                 190

Lys Leu Leu Asp Tyr Thr Ser Thr Lys Gly Ala Ile Val Ala Tyr Thr
        195                 200                 205

Arg Gly Leu Ala Leu Gln Leu Val Ser Lys Gly Ile Arg Val Asn Gly
    210                 215                 220

Val Ala Pro Gly Pro Ile Trp Thr Pro Leu Ile Pro Ala Ser Phe Lys
225                 230                 235                 240

Glu Glu Glu Thr Ala Gln Phe Gly Ala Gln Val Pro Met Lys Arg Ala
                245                 250                 255

Gly Gln Pro Ile Glu Val Ala Pro Ser Tyr Val Phe Leu Ala Ser Asn
            260                 265                 270

Gln Cys Ser Ser Tyr Ile Thr Gly Gln Val Leu His Pro Asn Gly Gly
        275                 280                 285

Thr Val Val Asn Gly
    290

<210>  480
<211>  15
<212>  PRT
<213>  Glycine max

<400>  480

Glu Gly Ser Ser Ile Ile Asn Thr Thr Ser Val Asn Ala Tyr Lys
1               5                   10                  15


<210>  481
<211>  12
<212>  PRT
<213>  Glycine max

<400>  481

Phe Pro Pro Gln Gln Gln Gln Thr Gln Pro Gly Lys
1               5                   10


<210>  482
<211>  17
<212>  PRT
<213>  Glycine max

<400>  482

Gly Ala Ile Val Ala Tyr Thr Arg Gly Leu Ala Leu Gln Leu Val Ser
1               5                   10                  15


Lys



<210>  483
<211>  8
<212>  PRT
<213>  Glycine max

<400>  483

Leu Leu Asp Tyr Thr Ser Thr Lys
1               5


<210>  484
<211>  882
<212>  DNA
<213>  Glycine max


<220>
<221>  CDS
<222>  (1)..(882)

<400>  484
atg gca agc aat aag gag tct aag ttc cca gca cag agc cag aaa act          48
Met Ala Ser Asn Lys Glu Ser Lys Phe Pro Ala Gln Ser Gln Lys Thr
1               5                   10                  15

cag cca gga aaa gaa cat gta atg aat cca ctc cca caa gcc aca aat          96
Gln Pro Gly Lys Glu His Val Met Asn Pro Leu Pro Gln Ala Thr Asn
            20                  25                  30

cct gat cac aag gcc gcc aat aaa ctc cag gga aag gtg gcg ttg gtg     144
Pro Asp His Lys Ala Ala Asn Lys Leu Gln Gly Lys Val Ala Leu Val
        35                  40                  45

aca gga ggt gac tca gga att ggc aga gcg gtt tgc ctg tgt ttc gca     192
Thr Gly Gly Asp Ser Gly Ile Gly Arg Ala Val Cys Leu Cys Phe Ala
    50                  55                  60

aaa gag ggt gca acc gtg gcc ttt aca tac gta aag ggc cat gag gac     240
Lys Glu Gly Ala Thr Val Ala Phe Thr Tyr Val Lys Gly His Glu Asp
65                  70                  75                  80

agg gat aaa gat gat act ctg aag atg ctg ctt gaa gct aag aca agt     288
Arg Asp Lys Asp Asp Thr Leu Lys Met Leu Leu Glu Ala Lys Thr Ser
                85                  90                  95

ggt gca gac aat cca ttg gca ata gca gcg gat att ggc ttt gat gag     336
Gly Ala Asp Asn Pro Leu Ala Ile Ala Ala Asp Ile Gly Phe Asp Glu
            100                 105                 110

aac tgc aaa cag gtc att gac ctt gtt gtc aaa gaa tat ggc cgc ctt     384
Asn Cys Lys Gln Val Ile Asp Leu Val Val Lys Glu Tyr Gly Arg Leu
        115                 120                 125

gat gtt ctg gtc aac aat gca gct gag cag cat ttg aca aac tct gtt     432
Asp Val Leu Val Asn Asn Ala Ala Glu Gln His Leu Thr Asn Ser Val
    130                 135                 140

gag gaa atc aca caa cag cag ctt gag aga gtc ttc gga acc aac atc     480
Glu Glu Ile Thr Gln Gln Gln Leu Glu Arg Val Phe Gly Thr Asn Ile
145                 150                 155                 160

ttt tct cag ttc ttt ttg gtc aag cat gct ctg aag cac atg aaa gaa     528
Phe Ser Gln Phe Phe Leu Val Lys His Ala Leu Lys His Met Lys Glu
                165                 170                 175

ggg agc tgc atc ata aac tct act tca gtt aat gca tac aat ggg aat     576
Gly Ser Cys Ile Ile Asn Ser Thr Ser Val Asn Ala Tyr Asn Gly Asn
            180                 185                 190

cca gaa gcg ttg gac tac act gct acc aag gga gca att gtg gcc ttc     624
Pro Glu Ala Leu Asp Tyr Thr Ala Thr Lys Gly Ala Ile Val Ala Phe
        195                 200                 205

acc aga ggt ctt tct cag cag cta gcg agt agg gga att agg gtg aat     672
Thr Arg Gly Leu Ser Gln Gln Leu Ala Ser Arg Gly Ile Arg Val Asn
    210                 215                 220

ggt gtg gca cct ggc cca gtt tgg acg cca ata caa cca gct tca aag     720
Gly Val Ala Pro Gly Pro Val Trp Thr Pro Ile Gln Pro Ala Ser Lys
225                 230                 235                 240

cct gct gag atg att cag aac ttg ggg tgt gag gtg cca atg aac cgc     768
Pro Ala Glu Met Ile Gln Asn Leu Gly Cys Glu Val Pro Met Asn Arg
                245                 250                 255

gtg gct cag cct tgt gag att gca cca tgt tat ttg ttc ttg gca act     816
Val Ala Gln Pro Cys Glu Ile Ala Pro Cys Tyr Leu Phe Leu Ala Thr
            260                 265                 270

tgt cag gac tct tcc tac ttt act ggc caa gtc ctc cat cca aat ggt     864
Cys Gln Asp Ser Ser Tyr Phe Thr Gly Gln Val Leu His Pro Asn Gly
        275                 280                 285

ggg atg gtc gtc aac gct                                                    882
Gly Met Val Val Asn Ala
    290

<210>  485
<211>  294
<212>  PRT
<213>  Glycine max

<400>  485

Met Ala Ser Asn Lys Glu Ser Lys Phe Pro Ala Gln Ser Gln Lys Thr
1               5                   10                  15

Gln Pro Gly Lys Glu His Val Met Asn Pro Leu Pro Gln Ala Thr Asn
            20                  25                  30

Pro Asp His Lys Ala Ala Asn Lys Leu Gln Gly Lys Val Ala Leu Val
        35                  40                  45

Thr Gly Gly Asp Ser Gly Ile Gly Arg Ala Val Cys Leu Cys Phe Ala
    50                  55                  60

Lys Glu Gly Ala Thr Val Ala Phe Thr Tyr Val Lys Gly His Glu Asp
65                  70                  75                  80

Arg Asp Lys Asp Asp Thr Leu Lys Met Leu Leu Glu Ala Lys Thr Ser
                85                  90                  95

Gly Ala Asp Asn Pro Leu Ala Ile Ala Ala Asp Ile Gly Phe Asp Glu
            100                 105                 110

Asn Cys Lys Gln Val Ile Asp Leu Val Val Lys Glu Tyr Gly Arg Leu
        115                 120                 125

Asp Val Leu Val Asn Asn Ala Ala Glu Gln His Leu Thr Asn Ser Val
    130                 135                 140

Glu Glu Ile Thr Gln Gln Gln Leu Glu Arg Val Phe Gly Thr Asn Ile
145                 150                 155                 160

Phe Ser Gln Phe Phe Leu Val Lys His Ala Leu Lys His Met Lys Glu
                165                 170                 175

Gly Ser Cys Ile Ile Asn Ser Thr Ser Val Asn Ala Tyr Asn Gly Asn
            180                 185                 190

Pro Glu Ala Leu Asp Tyr Thr Ala Thr Lys Gly Ala Ile Val Ala Phe
        195                 200                 205

Thr Arg Gly Leu Ser Gln Gln Leu Ala Ser Arg Gly Ile Arg Val Asn
    210                 215                 220

Gly Val Ala Pro Gly Pro Val Trp Thr Pro Ile Gln Pro Ala Ser Lys
225                 230                 235                 240

Pro Ala Glu Met Ile Gln Asn Leu Gly Cys Glu Val Pro Met Asn Arg
                245                 250                 255

Val Ala Gln Pro Cys Glu Ile Ala Pro Cys Tyr Leu Phe Leu Ala Thr
            260                 265                 270

Cys Gln Asp Ser Ser Tyr Phe Thr Gly Gln Val Leu His Pro Asn Gly
        275                 280                 285

Gly Met Val Val Asn Ala
    290

<210>  486
<211>  11
<212>  PRT
<213>  Glycine max

<400>  486

Glu Gly Ala Thr Val Ala Phe Thr Tyr Val Lys
1               5                   10

<210>  487
<211>  8
<212>  PRT
<213>  Glycine max

<400>  487

Gln Val Ile Asp Leu Val Val Lys
1               5

<210>  488
<211>  879
<212>  DNA
<213>  Glycine max

<220>
<221>  CDS
<222>  (1)..(879)

<400>  488
atg gct tcg ggt gaa aag aaa ttc cca cct caa caa caa caa aca cag      48
Met Ala Ser Gly Glu Lys Lys Phe Pro Pro Gln Gln Gln Gln Thr Gln
1               5                   10                  15

cct ggg aag gag cat gct atg aat cca gta ccc caa ttc act agc cct     96
Pro Gly Lys Glu His Ala Met Asn Pro Val Pro Gln Phe Thr Ser Pro
        20                  25                  30

gac tac aag cct tca aat aaa ctt caa gga aag ata gca tta gtg act     144
Asp Tyr Lys Pro Ser Asn Lys Leu Gln Gly Lys Ile Ala Leu Val Thr
    35                  40                  45

ggg ggt gac tct ggg att gga cga gcg gtg tgt aac ttg ttt gcc tta     192
Gly Gly Asp Ser Gly Ile Gly Arg Ala Val Cys Asn Leu Phe Ala Leu
    50                  55                  60

gaa ggt gct acc gtg ggc ttc aca tat gtg aag ggg cat gag gac aag     240
Glu Gly Ala Thr Val Gly Phe Thr Tyr Val Lys Gly His Glu Asp Lys
65                  70                  75                  80

gac gcg agg gac acg ttg gaa atg atc aag agg gca aag act tcg gat     288
Asp Ala Arg Asp Thr Leu Glu Met Ile Lys Arg Ala Lys Thr Ser Asp
                85                  90                  95

gct aaa gat cca atg gcg gta cca gct gat ttg ggt tac gac gag aat     336
Ala Lys Asp Pro Met Ala Val Pro Ala Asp Leu Gly Tyr Asp Glu Asn
            100                 105                 110

tgc aag aga gtg gtt gat gag gtc gtg aat gct tat ggt tgt att gac     384
Cys Lys Arg Val Val Asp Glu Val Val Asn Ala Tyr Gly Cys Ile Asp
        115                 120                 125

att ctg gtc aac aat gca gct gag caa tac gag tgt gga aca gtg gag     432
Ile Leu Val Asn Asn Ala Ala Glu Gln Tyr Glu Cys Gly Thr Val Glu
    130                 135                 140

gac att gat gag cct agg ctt gag agg gtc ttt cgt aca aat atc ttc     480
Asp Ile Asp Glu Pro Arg Leu Glu Arg Val Phe Arg Thr Asn Ile Phe
145                 150                 155                 160

tcc tat ttc ttc atg acc agg cat gcc ttg aag cac atg aag gaa gga     528
Ser Tyr Phe Phe Met Thr Arg His Ala Leu Lys His Met Lys Glu Gly
                165                 170                 175

agc agc att atc aac acg aca tcg gtg aat gca tac aag gga aat gcg     576
Ser Ser Ile Ile Asn Thr Thr Ser Val Asn Ala Tyr Lys Gly Asn Ala
            180                 185                 190

aaa cta ttg gac tac acg tcc acg aag gga gca att gtg gcc tat aca     624
Lys Leu Leu Asp Tyr Thr Ser Thr Lys Gly Ala Ile Val Ala Tyr Thr
        195                 200                 205

agg gga ctt gct ctt cag ttg gtg agt aag gga att cgg gtt aat ggg     672
Arg Gly Leu Ala Leu Gln Leu Val Ser Lys Gly Ile Arg Val Asn Gly
    210                 215                 220

gtg gct cct ggg cct att tgg acc cca ttg ata ccc tcc tct ttc aag     720
Val Ala Pro Gly Pro Ile Trp Thr Pro Leu Ile Pro Ser Ser Phe Lys
225                 230                 235                 240

gag gaa gaa acg gct caa ttt ggt gcc cag gtg cca atg aag aga gct     768
Glu Glu Glu Thr Ala Gln Phe Gly Ala Gln Val Pro Met Lys Arg Ala
                245                 250                 255

ggc cag cct att gag gtt gct ccg tct tat gtt ttt ctt gct tgc aac     816
Gly Gln Pro Ile Glu Val Ala Pro Ser Tyr Val Phe Leu Ala Cys Asn
            260                 265                 270

caa tgc tcc tct tac ata act gga caa gtc ctt cac ccc aat ggt gga     864
Gln Cys Ser Ser Tyr Ile Thr Gly Gln Val Leu His Pro Asn Gly Gly
        275                 280                 285

acc gtt gtc aat ggt                                                 879
Thr Val Val Asn Gly
    290

<210>  489
<211>  293
<212>  PRT
<213>  Glycine max

<400>  489

Met Ala Ser Gly Glu Lys Lys Phe Pro Pro Gln Gln Gln Gln Thr Gln
1               5                   10                  15

Pro Gly Lys Glu His Ala Met Asn Pro Val Pro Gln Phe Thr Ser Pro
            20                  25                  30

Asp Tyr Lys Pro Ser Asn Lys Leu Gln Gly Lys Ile Ala Leu Val Thr
        35                  40                  45

Gly Gly Asp Ser Gly Ile Gly Arg Ala Val Cys Asn Leu Phe Ala Leu
    50                  55                  60

Glu Gly Ala Thr Val Gly Phe Thr Tyr Val Lys Gly His Glu Asp Lys
65                  70                  75                  80

Asp Ala Arg Asp Thr Leu Glu Met Ile Lys Arg Ala Lys Thr Ser Asp
                85                  90                  95

Ala Lys Asp Pro Met Ala Val Pro Ala Asp Leu Gly Tyr Asp Glu Asn
            100                 105                 110

Cys Lys Arg Val Val Asp Glu Val Val Asn Ala Tyr Gly Cys Ile Asp
        115                 120                 125

Ile Leu Val Asn Asn Ala Ala Glu Gln Tyr Glu Cys Gly Thr Val Glu
    130                 135                 140

Asp Ile Asp Glu Pro Arg Leu Glu Arg Val Phe Arg Thr Asn Ile Phe
145                 150                 155                 160

Ser Tyr Phe Phe Met Thr Arg His Ala Leu Lys His Met Lys Glu Gly
                165                 170                 175

Ser Ser Ile Ile Asn Thr Thr Ser Val Asn Ala Tyr Lys Gly Asn Ala
            180                 185                 190

Lys Leu Leu Asp Tyr Thr Ser Thr Lys Gly Ala Ile Val Ala Tyr Thr
        195                 200                 205

Arg Gly Leu Ala Leu Gln Leu Val Ser Lys Gly Ile Arg Val Asn Gly
    210                 215                 220

Val Ala Pro Gly Pro Ile Trp Thr Pro Leu Ile Pro Ser Ser Phe Lys
225                 230                 235                 240

Glu Glu Glu Thr Ala Gln Phe Gly Ala Gln Val Pro Met Lys Arg Ala
                245                 250                 255

Gly Gln Pro Ile Glu Val Ala Pro Ser Tyr Val Phe Leu Ala Cys Asn
            260                 265                 270

Gln Cys Ser Ser Tyr Ile Thr Gly Gln Val Leu His Pro Asn Gly Gly
        275                 280                 285

Thr Val Val Asn Gly
    290

<210>  490
<211>  15
<212>  PRT
<213>  Glycine max

<400>  490

Glu Gly Ser Ser Ile Ile Asn Thr Thr Ser Val Asn Ala Tyr Lys
1               5                   10                  15

<210>  491
<211>  12
<212>  PRT
<213>  Glycine max

<400>  491

Phe Pro Pro Gln Gln Gln Gln Thr Gln Pro Gly Lys
1               5                   10

<210>  492
<211>  17
<212>  PRT
<213>  Glycine max

<400>  492

Gly Ala Ile Val Ala Tyr Thr Arg Gly Leu Ala Leu Gln Leu Val Ser
1               5                   10                  15

Lys

<210> 493
<211> 8
<212> PRT
<213> Glycine max

<400> 493

Leu Leu Asp Tyr Thr Ser Thr Lys
1               5

<210> 494
<211> 3207
<212> DNA
<213> Glycine max

<220>
<221> CDS
<222> (1)..(3207)

<400> 494
atg gct tct gag caa atg cct cgc aga gag aac aac agc aat agg gtg 48
Met Ala Ser Glu Gln Met Pro Arg Arg Glu Asn Asn Ser Asn Arg Val
1               5                   10                  15

ata gag aga gag gtt cac gtt gag aca cac aga aaa gag tcc ctc cct 96
Ile Glu Arg Glu Val His Val Glu Thr His Arg Lys Glu Ser Leu Pro
            20                  25                  30

gat aag gga agg gtt gtg ata gag agt gag aag aag gag aaa gag aga 144
Asp Lys Gly Arg Val Val Ile Glu Ser Glu Lys Lys Glu Lys Glu Arg
        35                  40                  45

gag ttg gct tct gat aga gcc agg tct gga aat ata gtt aag gag gac 192
Glu Leu Ala Ser Asp Arg Ala Arg Ser Gly Asn Ile Val Lys Glu Asp
    50                  55                  60

aaa gag gtt gaa gaa gga aga aga gga aaa gaa agt ggt ggt ggt ggt 240
Lys Glu Val Glu Glu Gly Arg Arg Gly Lys Glu Ser Gly Gly Gly Gly
65                  70                  75                  80

ggt gtt cag cat gag agg aag ttt gaa acg aaa gtt gaa gag aag aag 288
Gly Val Gln His Glu Arg Lys Phe Glu Thr Lys Val Glu Glu Lys Lys
                85                  90                  95

ggt ttt ggg gaa aag gct gaa ctc gag gga ata aca aga gtg gtg gtg 336
Gly Phe Gly Glu Lys Ala Glu Leu Glu Gly Ile Thr Arg Val Val Val
            100                 105                 110

gga aga gag tgc agc aaa gag gag aaa caa gga aga aca aga gag aaa 384
Gly Arg Glu Cys Ser Lys Glu Glu Lys Gln Gly Arg Thr Arg Glu Lys
        115                 120                 125

gta gag gca gag aaa gca aga tca atg gag aaa act aga gat gaa gct 432
Val Glu Ala Glu Lys Ala Arg Ser Met Glu Lys Thr Arg Asp Glu Ala
    130                 135                 140

aaa gga aga gaa caa gct gaa acc aaa ggt caa acc aag aag ggt ggt 480

Lys Gly Arg Glu Gln Ala Glu Thr Lys Gly Gln Thr Lys Lys Gly Gly
145                 150                 155                 160

ggt gag gaa cag gga aga aga aga gaa gaa ata gga gaa gaa aat aaa      528
Gly Glu Glu Gln Gly Arg Arg Arg Glu Glu Ile Gly Glu Glu Asn Lys
            165                 170                 175

caa tcc agc ctc gaa gag att tct aaa tgc aga gct gaa gct gaa gag      576
Gln Ser Ser Leu Glu Glu Ile Ser Lys Cys Arg Ala Glu Ala Glu Glu
        180                 185                 190

aaa gtt aag gag aga aag gag aga tca aaa caa gca gca agt aat gaa      624
Lys Val Lys Glu Arg Lys Glu Arg Ser Lys Gln Ala Ala Ser Asn Glu
        195                 200                 205

gct gcc aaa gag aga aac aag cag gca aat aag gag gca aca aag gca      672
Ala Ala Lys Glu Arg Asn Lys Gln Ala Asn Lys Glu Ala Thr Lys Ala
    210                 215                 220

aaa gat gtc aca aga gag aag ggt caa caa ggc aat ctt aaa aca gga      720
Lys Asp Val Thr Arg Glu Lys Gly Gln Gln Gly Asn Leu Lys Thr Gly
225                 230                 235                 240

aat gag aca caa gac att ggc caa aag ggt tat ggt gga gca aag gat      768
Asn Glu Thr Gln Asp Ile Gly Gln Lys Gly Tyr Gly Gly Ala Lys Asp
            245                 250                 255

acc att ggt aga gtg gac aac att act aca cca ctt gct aaa aag gct      816
Thr Ile Gly Arg Val Asp Asn Ile Thr Thr Pro Leu Ala Lys Lys Ala
        260                 265                 270

caa gtt gct ggt agc acc act gtt cag tat gct gga gag aaa tca gca      864
Gln Val Ala Gly Ser Thr Thr Val Gln Tyr Ala Gly Glu Lys Ser Ala
        275                 280                 285

caa gcc aaa gat gct acg gta gga tca atg gag aaa act aca aag gat      912
Gln Ala Lys Asp Ala Thr Val Gly Ser Met Glu Lys Thr Thr Lys Asp
    290                 295                 300

gct aaa gga aga gaa aaa gca gga acc aaa ggt caa aca aag aag ggt      960
Ala Lys Gly Arg Glu Lys Ala Gly Thr Lys Gly Gln Thr Lys Lys Gly
305                 310                 315                 320

ggt ggt gag gaa caa gga aaa aga aga gaa gaa aca gga gaa gaa aaa      1008
Gly Gly Glu Glu Gln Gly Lys Arg Arg Glu Glu Thr Gly Glu Glu Lys
            325                 330                 335

caa tcc agc ctg gag gag att tct aag cac aga gct gaa gct caa caa      1056
Gln Ser Ser Leu Glu Glu Ile Ser Lys His Arg Ala Glu Ala Gln Gln
        340                 345                 350

aaa gca atg agt tca att gaa ggt gct aag gag aga tac gag aga gca      1104
Lys Ala Met Ser Ser Ile Glu Gly Ala Lys Glu Arg Tyr Glu Arg Ala
        355                 360                 365

aaa caa gaa gca agt aaa gct tcc aaa gag aga aac att caa gca aat      1152
Lys Gln Glu Ala Ser Lys Ala Ser Lys Glu Arg Asn Ile Gln Ala Asn
    370                 375                 380

aag agg gct gaa gaa gaa aga aga aaa gca gaa agt ggt agg ggt gtt      1200
Lys Arg Ala Glu Glu Glu Arg Arg Lys Ala Glu Ser Gly Arg Gly Val
385                 390                 395                 400

cat gtg gat aag ttt gaa gag aag aag gat tca gtg ctg aaa gca gaa     1248
His Val Asp Lys Phe Glu Glu Lys Lys Asp Ser Val Leu Lys Ala Glu
                405                 410                 415

ctc gag aga aga aca aga gag gta gag gga aga gag tgg aac aaa gag     1296
Leu Glu Arg Arg Thr Arg Glu Val Glu Gly Arg Glu Trp Asn Lys Glu
            420                 425                 430

gag aaa caa aga agt tgt cca aga ggg aaa gta gag gca gag aaa gca     1344
Glu Lys Gln Arg Ser Cys Pro Arg Gly Lys Val Glu Ala Glu Lys Ala
        435                 440                 445

aga cca atg gaa gaa aca aaa ctg gaa gct aaa gga aga gaa aaa gct     1392
Arg Pro Met Glu Glu Thr Lys Leu Glu Ala Lys Gly Arg Glu Lys Ala
    450                 455                 460

gaa acc aaa gag ggt ggt ggt gag gaa aaa gga atg aga aga gaa gaa     1440
Glu Thr Lys Glu Gly Gly Gly Glu Glu Lys Gly Met Arg Arg Glu Glu
465                 470                 475                 480

ata gga gaa gaa aat aaa caa tcc agc ctg gaa gag att ttt aag tac     1488
Ile Gly Glu Glu Asn Lys Gln Ser Ser Leu Glu Glu Ile Phe Lys Tyr
                485                 490                 495

aga gct gaa gct caa cag aaa gtt aag gag aga aac gag aga gca aaa     1536
Arg Ala Glu Ala Gln Gln Lys Val Lys Glu Arg Asn Glu Arg Ala Lys
            500                 505                 510

caa gca gca gaa gct gcc aaa gag aga aac aag cag gca aat aaa ggg     1584
Gln Ala Ala Glu Ala Ala Lys Glu Arg Asn Lys Gln Ala Asn Lys Gly
        515                 520                 525

aat gaa gaa ggc aga aga gaa aga gaa agt ggt ggg ggt gtt cat cat     1632
Asn Glu Glu Gly Arg Arg Glu Arg Glu Ser Gly Gly Gly Val His His
    530                 535                 540

gag gct aag ttt gaa act aaa ggt gaa aaa aag aag gat tta gtg gga     1680
Glu Ala Lys Phe Glu Thr Lys Gly Glu Lys Lys Lys Asp Leu Val Gly
545                 550                 555                 560

aga gag cgg aac aaa gag gag aaa caa aga agc agt cca aga gga aaa     1728
Arg Glu Arg Asn Lys Glu Glu Lys Gln Arg Ser Ser Pro Arg Gly Lys
                565                 570                 575

gta gag gca gag aaa gca aga cca atg gaa gaa aca aag gtg gaa gct     1776
Val Glu Ala Glu Lys Ala Arg Pro Met Glu Glu Thr Lys Val Glu Ala
            580                 585                 590

aaa gga aga gaa aaa gat gta acc aaa gag ggt ggt gtt gag gaa caa     1824
Lys Gly Arg Glu Lys Asp Val Thr Lys Glu Gly Gly Val Glu Glu Gln
        595                 600                 605

gga aga gga aga aaa gaa aat gaa caa tct agc ttg aaa gac att tcc     1872
Gly Arg Gly Arg Lys Glu Asn Glu Gln Ser Ser Leu Lys Asp Ile Ser
    610                 615                 620

aag tac aga gct gaa gct caa caa aat tca ttc aat gct act gaa gct     1920
Lys Tyr Arg Ala Glu Ala Gln Gln Asn Ser Phe Asn Ala Thr Glu Ala
625                 630                 635                 640

gct aag gaa aga tac gag agg gca aac caa gcg aca agt gaa gct gcc     1968
Ala Lys Glu Arg Tyr Glu Arg Ala Asn Gln Ala Thr Ser Glu Ala Ala
                645                 650                 655

aaa gag aga aac aag cag gca aat aag gag gca aca aag gca aaa gat 2016
Lys Glu Arg Asn Lys Gln Ala Asn Lys Glu Ala Thr Lys Ala Lys Asp
            660                 665                 670

gtc aca aga gaa atg gat caa caa ggc aat ctt aaa aca gaa aat gag 2064
Val Thr Arg Glu Met Asp Gln Gln Gly Asn Leu Lys Thr Glu Asn Glu
        675                 680                 685

aca cag gac aat gac cag aag ggt tat ggt gga gca aag tac acc att 2112
Thr Gln Asp Asn Asp Gln Lys Gly Tyr Gly Gly Ala Lys Tyr Thr Ile
    690                 695                 700

ggt agt gtg gac cac att act aca cca ttt att gta gag gcc aat ctt 2160
Gly Ser Val Asp His Ile Thr Thr Pro Phe Ile Val Glu Ala Asn Leu
705                 710                 715                 720

aaa tca gaa aat cag ata caa gac aat ggc caa aag ggt tat ggt gga 2208
Lys Ser Glu Asn Gln Ile Gln Asp Asn Gly Gln Lys Gly Tyr Gly Gly
                725                 730                 735

gca aag gac aca att ggt agt gta gac cac att act aca cca ctt gct 2256
Ala Lys Asp Thr Ile Gly Ser Val Asp His Ile Thr Thr Pro Leu Ala
            740                 745                 750

gaa aag gcc aaa gct gct ggt agc acc act gct cag tat gtt ggg gag 2304
Glu Lys Ala Lys Ala Ala Gly Ser Thr Thr Ala Gln Tyr Val Gly Glu
        755                 760                 765

aaa gca gca caa gcc aaa gat gct act gtg caa act ggc aag agt gct 2352
Lys Ala Ala Gln Ala Lys Asp Ala Thr Val Gln Thr Gly Lys Ser Ala
    770                 775                 780

gca aaa gtt gcc tca gat ttg agg gac aag gct ttg gcc aca ggg tgg 2400
Ala Lys Val Ala Ser Asp Leu Arg Asp Lys Ala Leu Ala Thr Gly Trp
785                 790                 795                 800

agt gct gca cat tat tct act gag ata act gtg gag gga acc aag gct 2448
Ser Ala Ala His Tyr Ser Thr Glu Ile Thr Val Glu Gly Thr Lys Ala
                805                 810                 815

gca aca aat gtt gtt aag gga gtt gct gag tat gca gga cag aag gct 2496
Ala Thr Asn Val Val Lys Gly Val Ala Glu Tyr Ala Gly Gln Lys Ala
            820                 825                 830

tct gag ctt gca gct atg tca gtg gat act gcg aaa ggt tta gct gct 2544
Ser Glu Leu Ala Ala Met Ser Val Asp Thr Ala Lys Gly Leu Ala Ala
        835                 840                 845

gct gca gct ggg gag aat gcc aag gag tac act gca agg aag aag gtg 2592
Ala Ala Ala Gly Glu Asn Ala Lys Glu Tyr Thr Ala Arg Lys Lys Val
    850                 855                 860

gaa gct gag atg gat ttg gag gcc aaa agg gaa gct caa aat cag gaa 2640
Glu Ala Glu Met Asp Leu Glu Ala Lys Arg Glu Ala Gln Asn Gln Glu
865                 870                 875                 880

tcg aag gag tgg cca tca gca aaa tcc acc act gag agc att caa aga 2688
Ser Lys Glu Trp Pro Ser Ala Lys Ser Thr Thr Glu Ser Ile Gln Arg
                885                 890                 895

gga caa gga caa caa gaa cag cat gag caa gga gaa aaa tgg gaa cat 2736
Gly Gln Gly Gln Gln Glu Gln His Glu Gln Gly Glu Lys Trp Glu His

900                 905                 910

ggt gca caa agg act aat aag cca cat gaa agc aac att gtg gaa acc       2784
Gly Ala Gln Arg Thr Asn Lys Pro His Glu Ser Asn Ile Val Glu Thr
        915                 920                 925

ttt gag gaa aac cta ggt ggg att ggg gaa aaa agg gaa caa aaa cca       2832
Phe Glu Glu Asn Leu Gly Gly Ile Gly Glu Lys Arg Glu Gln Lys Pro
    930                 935                 940

ctt gga agc atc att gga gag agc ttt caa gga gtt caa gaa aaa ggg       2880
Leu Gly Ser Ile Ile Gly Glu Ser Phe Gln Gly Val Gln Glu Lys Gly
945                 950                 955                 960

agt gaa gtg atg gca agc att ggt gaa aaa ctg gga agt gct gca cat       2928
Ser Glu Val Met Ala Ser Ile Gly Glu Lys Leu Gly Ser Ala Ala His
                965                 970                 975

aca tta gtg aag aag cca ttg gat aag gcc act gag gga ggg aga gag       2976
Thr Leu Val Lys Lys Pro Leu Asp Lys Ala Thr Glu Gly Gly Arg Glu
            980                 985                 990

gtg ttg gga gct gta ggt gaa act  gtg gtt gag att ggt  gaa aac atg     3024
Val Leu Gly Ala Val Gly Glu Thr  Val Val Glu Ile Gly  Glu Asn Met
        995                 1000                1005

gtg aaa  aaa cca gca cag aaa  gtg cag cag cat caa  ggt gaa ggt       3069
Val Lys  Lys Pro Ala Gln Lys  Val Gln Gln His Gln  Gly Glu Gly
    1010                1015                1020

gga gga  ggg gtg ctg aat gcc  att ggt gaa acc ata  gca gag ata       3114
Gly Gly  Gly Val Leu Asn Ala  Ile Gly Glu Thr Ile  Ala Glu Ile
    1025                1030                1035

gca gag  aca act aag gtg att  gtt gct gga gag gga  gaa aag gag       3159
Ala Glu  Thr Thr Lys Val Ile  Val Ala Gly Glu Gly  Glu Lys Glu
    1040                1045                1050

ata ata  gta aaa aca cat gaa  ttt gaa tat gga gac  ttg aaa ctt       3204
Ile Ile  Val Lys Thr His Glu  Phe Glu Tyr Gly Asp  Leu Lys Leu
    1055                1060                1065

gat                                                                   3207
Asp

<210>  495
<211>  1069
<212>  PRT
<213>  Glycine max

<400>  495

Met Ala Ser Glu Gln Met Pro Arg Arg Glu Asn Asn Ser Asn Arg Val
1               5                   10                  15

Ile Glu Arg Glu Val His Val Glu Thr His Arg Lys Glu Ser Leu Pro
            20                  25                  30

Asp Lys Gly Arg Val Val Ile Glu Ser Glu Lys Lys Glu Lys Glu Arg

35                  40                  45

Glu Leu Ala Ser Asp Arg Ala Arg Ser Gly Asn Ile Val Lys Glu Asp
    50                  55                  60

Lys Glu Val Glu Glu Gly Arg Arg Gly Lys Glu Ser Gly Gly Gly Gly
65                  70                  75                  80

Gly Val Gln His Glu Arg Lys Phe Glu Thr Lys Val Glu Glu Lys Lys
                85                  90                  95

Gly Phe Gly Glu Lys Ala Glu Leu Glu Gly Ile Thr Arg Val Val Val
            100                 105                 110

Gly Arg Glu Cys Ser Lys Glu Glu Lys Gln Gly Arg Thr Arg Glu Lys
        115                 120                 125

Val Glu Ala Glu Lys Ala Arg Ser Met Glu Lys Thr Arg Asp Glu Ala
    130                 135                 140

Lys Gly Arg Glu Gln Ala Glu Thr Lys Gly Gln Thr Lys Lys Gly Gly
145                 150                 155                 160

Gly Glu Glu Gln Gly Arg Arg Arg Glu Glu Ile Gly Glu Glu Asn Lys
                165                 170                 175

Gln Ser Ser Leu Glu Glu Ile Ser Lys Cys Arg Ala Glu Ala Glu Glu
            180                 185                 190

Lys Val Lys Glu Arg Lys Glu Arg Ser Lys Gln Ala Ala Ser Asn Glu
        195                 200                 205

Ala Ala Lys Glu Arg Asn Lys Gln Ala Asn Lys Glu Ala Thr Lys Ala
    210                 215                 220

Lys Asp Val Thr Arg Glu Lys Gly Gln Gln Gly Asn Leu Lys Thr Gly
225                 230                 235                 240

Asn Glu Thr Gln Asp Ile Gly Gln Lys Gly Tyr Gly Gly Ala Lys Asp
                245                 250                 255

Thr Ile Gly Arg Val Asp Asn Ile Thr Thr Pro Leu Ala Lys Lys Ala
            260                 265                 270

Gln Val Ala Gly Ser Thr Thr Val Gln Tyr Ala Gly Glu Lys Ser Ala
        275                 280                 285

Gln Ala Lys Asp Ala Thr Val Gly Ser Met Glu Lys Thr Thr Lys Asp
    290                 295                 300

Ala Lys Gly Arg Glu Lys Ala Gly Thr Lys Gly Gln Thr Lys Lys Gly
305                 310                 315                 320

Gly Gly Glu Glu Gln Gly Lys Arg Arg Glu Glu Thr Gly Glu Glu Lys
                325                 330                 335

Gln Ser Ser Leu Glu Glu Ile Ser Lys His Arg Ala Glu Ala Gln Gln
            340                 345                 350

Lys Ala Met Ser Ser Ile Glu Gly Ala Lys Glu Arg Tyr Glu Arg Ala
        355                 360                 365

Lys Gln Glu Ala Ser Lys Ala Ser Lys Glu Arg Asn Ile Gln Ala Asn
    370                 375                 380

Lys Arg Ala Glu Glu Glu Arg Arg Lys Ala Glu Ser Gly Arg Gly Val
385                 390                 395                 400

His Val Asp Lys Phe Glu Glu Lys Lys Asp Ser Val Leu Lys Ala Glu
                405                 410                 415

Leu Glu Arg Arg Thr Arg Glu Val Glu Gly Arg Glu Trp Asn Lys Glu
            420                 425                 430

Glu Lys Gln Arg Ser Cys Pro Arg Gly Lys Val Glu Ala Glu Lys Ala
        435                 440                 445

Arg Pro Met Glu Glu Thr Lys Leu Glu Ala Lys Gly Arg Glu Lys Ala
    450                 455                 460

Glu Thr Lys Glu Gly Gly Gly Glu Glu Lys Gly Met Arg Arg Glu Glu
465                 470                 475                 480

Ile Gly Glu Glu Asn Lys Gln Ser Ser Leu Glu Glu Ile Phe Lys Tyr
                485                 490                 495

Arg Ala Glu Ala Gln Gln Lys Val Lys Glu Arg Asn Glu Arg Ala Lys
            500                 505                 510

Gln Ala Ala Glu Ala Ala Lys Glu Arg Asn Lys Gln Ala Asn Lys Gly
        515                 520                 525

Asn Glu Glu Gly Arg Arg Glu Arg Glu Ser Gly Gly Gly Val His His
    530                 535                 540

Glu Ala Lys Phe Glu Thr Lys Gly Glu Lys Lys Lys Asp Leu Val Gly
545                 550                 555                 560

Arg Glu Arg Asn Lys Glu Glu Lys Gln Arg Ser Ser Pro Arg Gly Lys
                565                 570                 575

Val Glu Ala Glu Lys Ala Arg Pro Met Glu Glu Thr Lys Val Glu Ala
            580                 585                 590

Lys Gly Arg Glu Lys Asp Val Thr Lys Glu Gly Gly Val Glu Glu Gln
        595                 600                 605

Gly Arg Gly Arg Lys Glu Asn Glu Gln Ser Ser Leu Lys Asp Ile Ser
    610                 615                 620

Lys Tyr Arg Ala Glu Ala Gln Gln Asn Ser Phe Asn Ala Thr Glu Ala
625                 630                 635                 640

Ala Lys Glu Arg Tyr Glu Arg Ala Asn Gln Ala Thr Ser Glu Ala Ala
                645                 650                 655

Lys Glu Arg Asn Lys Gln Ala Asn Lys Glu Ala Thr Lys Ala Lys Asp
            660                 665                 670

Val Thr Arg Glu Met Asp Gln Gln Gly Asn Leu Lys Thr Glu Asn Glu
        675                 680                 685

Thr Gln Asp Asn Asp Gln Lys Gly Tyr Gly Gly Ala Lys Tyr Thr Ile
    690                 695                 700

Gly Ser Val Asp His Ile Thr Thr Pro Phe Ile Val Glu Ala Asn Leu
705                 710                 715                 720

Lys Ser Glu Asn Gln Ile Gln Asp Asn Gly Gln Lys Gly Tyr Gly Gly
                725                 730                 735

Ala Lys Asp Thr Ile Gly Ser Val Asp His Ile Thr Thr Pro Leu Ala
            740                 745                 750

Glu Lys Ala Lys Ala Ala Gly Ser Thr Thr Ala Gln Tyr Val Gly Glu
        755                 760                 765

Lys Ala Ala Gln Ala Lys Asp Ala Thr Val Gln Thr Gly Lys Ser Ala
    770                 775                 780

Ala Lys Val Ala Ser Asp Leu Arg Asp Lys Ala Leu Ala Thr Gly Trp
785                 790                 795                 800

Ser Ala Ala His Tyr Ser Thr Glu Ile Thr Val Glu Gly Thr Lys Ala
                805                 810                 815

Ala Thr Asn Val Val Lys Gly Val Ala Glu Tyr Ala Gly Gln Lys Ala
            820                 825                 830

Ser Glu Leu Ala Ala Met Ser Val Asp Thr Ala Lys Gly Leu Ala Ala
        835                 840                 845

Ala Ala Ala Gly Glu Asn Ala Lys Glu Tyr Thr Ala Arg Lys Lys Val
    850                 855                 860

Glu Ala Glu Met Asp Leu Glu Ala Lys Arg Glu Ala Gln Asn Gln Glu
865                 870                 875                 880

Ser Lys Glu Trp Pro Ser Ala Lys Ser Thr Thr Glu Ser Ile Gln Arg
                885                 890                 895

Gly Gln Gly Gln Gln Glu Gln His Glu Gln Gly Glu Lys Trp Glu His
            900                 905                 910

Gly Ala Gln Arg Thr Asn Lys Pro His Glu Ser Asn Ile Val Glu Thr
        915                 920                 925

Phe Glu Glu Asn Leu Gly Gly Ile Gly Glu Lys Arg Glu Gln Lys Pro
    930                 935                 940

Leu Gly Ser Ile Ile Gly Glu Ser Phe Gln Gly Val Gln Glu Lys Gly
945                 950                 955                 960

Ser Glu Val Met Ala Ser Ile Gly Glu Lys Leu Gly Ser Ala Ala His
                965                 970                 975

Thr Leu Val Lys Lys Pro Leu Asp Lys Ala Thr Glu Gly Gly Arg Glu
            980                 985                 990

Val Leu Gly Ala Val Gly Glu Thr  Val Val Glu Ile Gly  Glu Asn Met
        995                 1000                 1005

Val Lys  Lys Pro Ala Gln Lys  Val Gln Gln His Gln  Gly Glu Gly
    1010                 1015                 1020

Gly Gly  Gly Val Leu Asn Ala  Ile Gly Glu Thr Ile  Ala Glu Ile
    1025                 1030                 1035

Ala Glu  Thr Thr Lys Val Ile  Val Ala Gly Glu Gly  Glu Lys Glu

1040                    1045                    1050

Ile Ile  Val Lys Thr His Glu  Phe Glu Tyr Gly Asp  Leu Lys Leu
    1055                    1060                    1065

Asp

<210>  496
<211>  13
<212>  PRT
<213>  Glycine max

<400>  496

Ala Ala Gly Ser Thr Thr Ala Gln Tyr Val Gly Glu Lys
1               5                   10

<210>  497
<211>  21
<212>  PRT
<213>  Glycine max

<400>  497

Ala Leu Ala Thr Gly Trp Ser Ala Ala His Tyr Ser Thr Glu Ile Thr
1               5                   10                  15

Val Glu Gly Thr Lys
            20

<210>  498
<211>  15
<212>  PRT
<213>  Glycine max

<400>  498

Ala Gln Val Ala Gly Ser Thr Thr Val Gln Tyr Ala Gly Glu Lys
1               5                   10                  15

<210>  499
<211>  15
<212>  PRT
<213>  Glycine max

<400>  499

Asp Thr Ile Gly Arg Val Asp Asn Ile Thr Thr Pro Leu Ala Lys
1               5                   10                  15

<210>  500
<211>  16
<212>  PRT
<213>  Glycine max

<400>  500

Asp Thr Ile Gly Ser Val Asp His Ile Thr Thr Pro Leu Ala Glu Lys
1               5                   10                  15


<210>  501
<211>  16
<212>  PRT
<213>  Glycine max

<400>  501

Glu Arg Glu Leu Ala Ser Asp Arg Ala Arg Ser Gly Asn Ile Val Lys
1               5                   10                  15


<210>  502
<211>  15
<212>  PRT
<213>  Glycine max

<400>  502

Glu Arg Tyr Glu Arg Ala Asn Gln Ala Thr Ser Glu Ala Ala Lys
1               5                   10                  15


<210>  503
<211>  18
<212>  PRT
<213>  Glycine max

<400>  503

Gly Gly Gly Glu Glu Gln Gly Arg Arg Arg Glu Glu Ile Gly Glu Glu
1               5                   10                  15


Asn Lys




<210>  504
<211>  12
<212>  PRT
<213>  Glycine max

<400>  504

Gly Leu Ala Ala Ala Ala Ala Gly Glu Asn Ala Lys
1               5                   10


<210>  505
<211>  9
<212>  PRT
<213>  Glycine max

<400>  505

Gly Arg Val Val Ile Glu Ser Glu Lys

1               5


<210>  506
<211>  9
<212>  PRT
<213>  Glycine max

<400>  506

Gly Val Ala Glu Tyr Ala Gly Gln Lys
1               5


<210>  507
<211>  10
<212>  PRT
<213>  Glycine max

<400>  507

Leu Gly Ser Ala Ala His Thr Leu Val Lys
1               5                   10


<210>  508
<211>  9
<212>  PRT
<213>  Glycine max

<400>  508

Gln Ser Ser Leu Glu Glu Ile Phe Lys
1               5


<210>  509
<211>  9
<212>  PRT
<213>  Glycine max

<400>  509

Gln Ser Ser Leu Glu Glu Ile Ser Lys
1               5


<210>  510
<211>  20
<212>  PRT
<213>  Glycine max

<400>  510

Arg Glu Gln Lys Pro Leu Gly Ser Ile Ile Gly Glu Ser Phe Gln Gly
1               5                   10                  15


Val Gln Glu Lys
            20


<210>  511

<211>  11
<212>  PRT
<213>  Glycine max

<400>  511

Thr Gly Asn Glu Thr Gln Asp Ile Gly Gln Lys
1               5                   10


<210>  512
<211>  9
<212>  PRT
<213>  Glycine max

<400>  512

Val Ile Val Ala Gly Glu Gly Glu Lys
1               5


<210>  513
<211>  28
<212>  PRT
<213>  Glycine max

<400>  513

Val Gln Gln His Gln Gly Glu Gly Gly Gly Gly Val Leu Asn Ala Ile
1               5                   10                  15


Gly Glu Thr Ile Ala Glu Ile Ala Glu Thr Thr Lys
            20                  25


<210>  514
<211>  735
<212>  DNA
<213>  Glycine max


<220>
<221>  CDS
<222>  (1)..(735)

<400>  514
atg gca ctg tgc aac cgc gtc aga tcg gga atc tct ctc ttc acc aaa       48
Met Ala Leu Cys Asn Arg Val Arg Ser Gly Ile Ser Leu Phe Thr Lys
1               5                   10                  15

tcc acg ttc ctc act tcc cag cga tcc act ctc caa cga tcc ctc gtt       96
Ser Thr Phe Leu Thr Ser Gln Arg Ser Thr Leu Gln Arg Ser Leu Val
            20                  25                  30

gcc cct ttc att tcc ctc gcc tcc aga aat tac gcc aac gtg ccg ggg      144
Ala Pro Phe Ile Ser Leu Ala Ser Arg Asn Tyr Ala Asn Val Pro Gly
        35                  40                  45

caa aag gaa acc aaa gtt aag gtc cct ctg gct ttg ttt gga ggt tca      192
Gln Lys Glu Thr Lys Val Lys Val Pro Leu Ala Leu Phe Gly Gly Ser
    50                  55                  60

gga aac tat gcc tct gct ttg tat att gca tct gtg aaa gcc aat gca          240
Gly Asn Tyr Ala Ser Ala Leu Tyr Ile Ala Ser Val Lys Ala Asn Ala
65                  70                  75                  80

gtg gaa aag gtt gag act gag ctt ctg caa ttt gtt gag gca gta aag          288
Val Glu Lys Val Glu Thr Glu Leu Leu Gln Phe Val Glu Ala Val Lys
                85                  90                  95

aat agt ccc ata ttt tca cag ttt ata agg gac ttg tct gtg gct aag          336
Asn Ser Pro Ile Phe Ser Gln Phe Ile Arg Asp Leu Ser Val Ala Lys
            100                 105                 110

gat gtt aga gtt aaa gtt atc cag gat att tgt ggg gaa gcc aag ttt          384
Asp Val Arg Val Lys Val Ile Gln Asp Ile Cys Gly Glu Ala Lys Phe
        115                 120                 125

tcg gat gtt aca aag aac ttc ctt gtt att gtt gct gag aat gga agg          432
Ser Asp Val Thr Lys Asn Phe Leu Val Ile Val Ala Glu Asn Gly Arg
    130                 135                 140

ctt aaa aac ata gac acc att gca aag agg ttt gca cag ttg tca atg          480
Leu Lys Asn Ile Asp Thr Ile Ala Lys Arg Phe Ala Gln Leu Ser Met
145                 150                 155                 160

gca tat aag gga gaa gtg caa gcg act gtg acc act gtt gtt cct ctt          528
Ala Tyr Lys Gly Glu Val Gln Ala Thr Val Thr Thr Val Val Pro Leu
                165                 170                 175

ccc cct gaa gag gaa aag gca ttg aag gag act ctt cag gaa ata ata          576
Pro Pro Glu Glu Glu Lys Ala Leu Lys Glu Thr Leu Gln Glu Ile Ile
            180                 185                 190

ggt tct gga gcg aag gtt cat ctt gaa cag aag att gat cca agc ata          624
Gly Ser Gly Ala Lys Val His Leu Glu Gln Lys Ile Asp Pro Ser Ile
        195                 200                 205

ctt ggt ggg ctt gtg ctt gaa ttt agc cag aag gtc ttt gac atg tca          672
Leu Gly Gly Leu Val Leu Glu Phe Ser Gln Lys Val Phe Asp Met Ser
    210                 215                 220

att aag acc agg gca caa cag atg gag agg atc ctc cga gag ccc gtg          720
Ile Lys Thr Arg Ala Gln Gln Met Glu Arg Ile Leu Arg Glu Pro Val
225                 230                 235                 240

tcc att gcc aac atc                                                      735
Ser Ile Ala Asn Ile
                245

<210>  515
<211>  245
<212>  PRT
<213>  Glycine max

<400>  515

Met Ala Leu Cys Asn Arg Val Arg Ser Gly Ile Ser Leu Phe Thr Lys
1               5                   10                  15

Ser Thr Phe Leu Thr Ser Gln Arg Ser Thr Leu Gln Arg Ser Leu Val
            20                  25                  30

Ala Pro Phe Ile Ser Leu Ala Ser Arg Asn Tyr Ala Asn Val Pro Gly
        35                  40                  45

Gln Lys Glu Thr Lys Val Lys Val Pro Leu Ala Leu Phe Gly Gly Ser
    50                  55                  60

Gly Asn Tyr Ala Ser Ala Leu Tyr Ile Ala Ser Val Lys Ala Asn Ala
65                  70                  75                  80

Val Glu Lys Val Glu Thr Glu Leu Leu Gln Phe Val Glu Ala Val Lys
                85                  90                  95

Asn Ser Pro Ile Phe Ser Gln Phe Ile Arg Asp Leu Ser Val Ala Lys
            100                 105                 110

Asp Val Arg Val Lys Val Ile Gln Asp Ile Cys Gly Glu Ala Lys Phe
        115                 120                 125

Ser Asp Val Thr Lys Asn Phe Leu Val Ile Val Ala Glu Asn Gly Arg
    130                 135                 140

Leu Lys Asn Ile Asp Thr Ile Ala Lys Arg Phe Ala Gln Leu Ser Met
145                 150                 155                 160

Ala Tyr Lys Gly Glu Val Gln Ala Thr Val Thr Thr Val Val Pro Leu
                165                 170                 175

Pro Pro Glu Glu Glu Lys Ala Leu Lys Glu Thr Leu Gln Glu Ile Ile
            180                 185                 190

Gly Ser Gly Ala Lys Val His Leu Glu Gln Lys Ile Asp Pro Ser Ile
        195                 200                 205

Leu Gly Gly Leu Val Leu Glu Phe Ser Gln Lys Val Phe Asp Met Ser
    210                 215                 220

Ile Lys Thr Arg Ala Gln Gln Met Glu Arg Ile Leu Arg Glu Pro Val
225                 230                 235                 240

Ser Ile Ala Asn Ile
                245

<210>  516
<211>  12
<212>  PRT
<213>  Glycine max

<400>  516

Glu Thr Leu Gln Glu Ile Ile Gly Ser Gly Ala Lys
1               5                   10

<210>  517
<211>  678
<212>  DNA
<213>  Glycine max

<220>
<221>  CDS
<222>  (1)..(678)

<400>  517
atg acc aca caa gta cca cca cac agt gtc caa gtc cac aca aca aca      48
Met Thr Thr Gln Val Pro Pro His Ser Val Gln Val His Thr Thr Thr
1               5                   10                  15

cac cgc tac gaa gct ggc gtc gtc ccc ccc gga gcc cgt ttc gaa ccg      96
His Arg Tyr Glu Ala Gly Val Val Pro Pro Gly Ala Arg Phe Glu Pro
            20                  25                  30

ccg cgt tat gaa gcc ggc gtc aaa gcg ccc tcc att tac cat tcc gag      144
Pro Arg Tyr Glu Ala Gly Val Lys Ala Pro Ser Ile Tyr His Ser Glu
        35                  40                  45

aga ggt cca acg act tcc cag gtc ctc gcc gtc ctc gcc ggc ctc ccg      192
Arg Gly Pro Thr Thr Ser Gln Val Leu Ala Val Leu Ala Gly Leu Pro
    50                  55                  60

gtc ggt ggc atc ctg ctc ctc ctg gcg gga ttg acc ctg gca gga acc      240
Val Gly Gly Ile Leu Leu Leu Leu Ala Gly Leu Thr Leu Ala Gly Thr
65                  70                  75                  80

ctc acc gga ctg gca gtt gcc acg ccg ctc ttc gtc ctc ttc agc ccg      288
Leu Thr Gly Leu Ala Val Ala Thr Pro Leu Phe Val Leu Phe Ser Pro
                85                  90                  95

gtg ctg gtt cca gcc acg gtc gcc atc ggc ctg gcc gtg gcc ggg ttc      336
Val Leu Val Pro Ala Thr Val Ala Ile Gly Leu Ala Val Ala Gly Phe
            100                 105                 110

ctg acg tcg ggg gcc ttc ggg ctc acg gcg ctg tct tcc ttc tcc tgg      384
Leu Thr Ser Gly Ala Phe Gly Leu Thr Ala Leu Ser Ser Phe Ser Trp
        115                 120                 125

atc ctg aac tac atc cgg gag acc cag cca gcc tcg gag aac ctc gcc      432
Ile Leu Asn Tyr Ile Arg Glu Thr Gln Pro Ala Ser Glu Asn Leu Ala
    130                 135                 140

gcc gcc gcg aag cat cac ctc gcc gag gcg gcg gag tac gtg ggg cag      480
Ala Ala Ala Lys His His Leu Ala Glu Ala Ala Glu Tyr Val Gly Gln
145                 150                 155                 160

aag acg aag gaa gtg ggg cag aag acc aag gag gtt ggg cag gat att      528
Lys Thr Lys Glu Val Gly Gln Lys Thr Lys Glu Val Gly Gln Asp Ile
                165                 170                 175

cag agc aag gca cag gat aca agg gag gca gct gca agg gat gca agg      576
Gln Ser Lys Ala Gln Asp Thr Arg Glu Ala Ala Ala Arg Asp Ala Arg
            180                 185                 190

gag gca gct gca agg gat gca agg gaa gct gct gca agg gat gca aag     624
Glu Ala Ala Ala Arg Asp Ala Arg Glu Ala Ala Ala Arg Asp Ala Lys
        195                 200                 205

gtg gag gca agg gat gta aag aga aca aca gtg aca gca aca acc gca     672
Val Glu Ala Arg Asp Val Lys Arg Thr Thr Val Thr Ala Thr Thr Ala
    210                 215                 220

acc gca                                                             678
Thr Ala
225

<210>  518
<211>  226
<212>  PRT
<213>  Glycine max

<400>  518

Met Thr Thr Gln Val Pro Pro His Ser Val Gln Val His Thr Thr Thr
1               5                   10                  15

His Arg Tyr Glu Ala Gly Val Val Pro Pro Gly Ala Arg Phe Glu Pro
            20                  25                  30

Pro Arg Tyr Glu Ala Gly Val Lys Ala Pro Ser Ile Tyr His Ser Glu
        35                  40                  45

Arg Gly Pro Thr Thr Ser Gln Val Leu Ala Val Leu Ala Gly Leu Pro
    50                  55                  60

Val Gly Gly Ile Leu Leu Leu Leu Ala Gly Leu Thr Leu Ala Gly Thr
65                  70                  75                  80

Leu Thr Gly Leu Ala Val Ala Thr Pro Leu Phe Val Leu Phe Ser Pro
                85                  90                  95

Val Leu Val Pro Ala Thr Val Ala Ile Gly Leu Ala Val Ala Gly Phe
            100                 105                 110

Leu Thr Ser Gly Ala Phe Gly Leu Thr Ala Leu Ser Ser Phe Ser Trp
        115                 120                 125

Ile Leu Asn Tyr Ile Arg Glu Thr Gln Pro Ala Ser Glu Asn Leu Ala
    130                 135                 140

Ala Ala Ala Lys His His Leu Ala Glu Ala Ala Glu Tyr Val Gly Gln
145                 150                 155                 160

Lys Thr Lys Glu Val Gly Gln Lys Thr Lys Glu Val Gly Gln Asp Ile
                165                 170                 175

Gln Ser Lys Ala Gln Asp Thr Arg Glu Ala Ala Ala Arg Asp Ala Arg
            180                 185                 190

Glu Ala Ala Ala Arg Asp Ala Arg Glu Ala Ala Ala Arg Asp Ala Lys
        195                 200                 205

Val Glu Ala Arg Asp Val Lys Arg Thr Thr Val Thr Ala Thr Thr Ala
    210                 215                 220

Thr Ala
225

<210>  519
<211>  9
<212>  PRT
<213>  Glycine max

<400>  519

Glu Val Gly Gln Asp Ile Gln Ser Lys
1               5

<210>  520
<211>  11
<212>  PRT
<213>  Glycine max

<400>  520

Arg Thr Thr Val Thr Ala Thr Thr Ala Thr Ala
1               5                   10

<210>  521
<211>  627
<212>  DNA
<213>  Glycine max

<220>
<221>  CDS
<222>  (1)..(627)

<400>  521
atg aca agg aaa caa gca agt tcc acg aga cgt ggc ggt ctt tca gga      48
Met Thr Arg Lys Gln Ala Ser Ser Thr Arg Arg Gly Gly Leu Ser Gly
1               5                   10                  15

gtc ttg cat tcc aaa tcc aaa tct tct cct cta ctc tcc ata tcc ctt      96
Val Leu His Ser Lys Ser Lys Ser Ser Pro Leu Leu Ser Ile Ser Leu
            20                  25                  30

gtc cta ttt gta gcc atc ctt ctc atc ctt tat gct tgt atc ggg tca      144
Val Leu Phe Val Ala Ile Leu Leu Ile Leu Tyr Ala Cys Ile Gly Ser
        35                  40                  45

ggt ata ttg gga gga aga aag gat gtg att agc atg gct gaa gat gat 192
Gly Ile Leu Gly Gly Arg Lys Asp Val Ile Ser Met Ala Glu Asp Asp
    50                  55                  60

ttt tca tgc act ttt gaa gtt cca agt gca att cct gtt ttg aag aat 240
Phe Ser Cys Thr Phe Glu Val Pro Ser Ala Ile Pro Val Leu Lys Asn
65                  70                  75                  80

gca tat ggt ggc agc atg aaa aat gtt ttg cac gta ggc cct gag agt 288
Ala Tyr Gly Gly Ser Met Lys Asn Val Leu His Val Gly Pro Glu Ser
                85                  90                  95

tgt tcg gtg gtc tcc aaa ttt tta aga gaa ggg gaa act gaa gca tgg 336
Cys Ser Val Val Ser Lys Phe Leu Arg Glu Gly Glu Thr Glu Ala Trp
            100                 105                 110

ggt gtg gag cca tat gac tta gac gat gct gat agg aac tgc aag gct 384
Gly Val Glu Pro Tyr Asp Leu Asp Asp Ala Asp Arg Asn Cys Lys Ala
        115                 120                 125

ctt gtg cag aaa ggc att ata cgt gtt gca gat ata aaa ttc cct tta 432
Leu Val Gln Lys Gly Ile Ile Arg Val Ala Asp Ile Lys Phe Pro Leu
    130                 135                 140

cca tac agg gtg aag tct ttt tct cat gtt ata gtg tca gat gct ttg 480
Pro Tyr Arg Val Lys Ser Phe Ser His Val Ile Val Ser Asp Ala Leu
145                 150                 155                 160

gat tac ctg tct cca aaa tat ata aac aaa act ctt cct gag cta gca 528
Asp Tyr Leu Ser Pro Lys Tyr Ile Asn Lys Thr Leu Pro Glu Leu Ala
                165                 170                 175

agg gta tct tct gat ggt att att att ttc aca ggt tac cca ggt caa 576
Arg Val Ser Ser Asp Gly Ile Ile Ile Phe Thr Gly Tyr Pro Gly Gln
            180                 185                 190

ccg aga gct aaa ata gca cca tta tcc aaa ttt ggc cgt cca cat agg 624
Pro Arg Ala Lys Ile Ala Pro Leu Ser Lys Phe Gly Arg Pro His Arg
        195                 200                 205

tgc 627
Cys

<210> 522
<211> 209
<212> PRT
<213> Glycine max

<400> 522

Met Thr Arg Lys Gln Ala Ser Ser Thr Arg Arg Gly Gly Leu Ser Gly
1               5                   10                  15

Val Leu His Ser Lys Ser Lys Ser Ser Pro Leu Leu Ser Ile Ser Leu
            20                  25                  30

Val Leu Phe Val Ala Ile Leu Leu Ile Leu Tyr Ala Cys Ile Gly Ser
        35                  40                  45

Gly Ile Leu Gly Gly Arg Lys Asp Val Ile Ser Met Ala Glu Asp Asp
    50                  55                  60

Phe Ser Cys Thr Phe Glu Val Pro Ser Ala Ile Pro Val Leu Lys Asn
65                  70                  75                  80

Ala Tyr Gly Gly Ser Met Lys Asn Val Leu His Val Gly Pro Glu Ser
                85                  90                  95

Cys Ser Val Val Ser Lys Phe Leu Arg Glu Gly Glu Thr Glu Ala Trp
            100                 105                 110

Gly Val Glu Pro Tyr Asp Leu Asp Asp Ala Asp Arg Asn Cys Lys Ala
        115                 120                 125

Leu Val Gln Lys Gly Ile Ile Arg Val Ala Asp Ile Lys Phe Pro Leu
    130                 135                 140

Pro Tyr Arg Val Lys Ser Phe Ser His Val Ile Val Ser Asp Ala Leu
145                 150                 155                 160

Asp Tyr Leu Ser Pro Lys Tyr Ile Asn Lys Thr Leu Pro Glu Leu Ala
                165                 170                 175

Arg Val Ser Ser Asp Gly Ile Ile Ile Phe Thr Gly Tyr Pro Gly Gln
            180                 185                 190

Pro Arg Ala Lys Ile Ala Pro Leu Ser Lys Phe Gly Arg Pro His Arg
        195                 200                 205

Cys

<210>  523
<211>  8
<212>  PRT
<213>  Glycine max

<400>  523

Asn Ala Tyr Gly Gly Ser Met Lys
1               5

<210>  524
<211>  723
<212>  DNA
<213>  Glycine max

<220>

<221>  CDS
<222>  (1)..(723)

<400>  524
atg gcc gcg cga gct ctg ttg acc aga aaa acc cta gcc acc gtg ctc      48
Met Ala Ala Arg Ala Leu Leu Thr Arg Lys Thr Leu Ala Thr Val Leu
1               5                   10                  15

cgc aac gac gcg aag ccc ata atc gga gtt ggc ata aca gca gcg gct      96
Arg Asn Asp Ala Lys Pro Ile Ile Gly Val Gly Ile Thr Ala Ala Ala
            20                  25                  30

act cat tca cgc ggg ttg cac gtg tac acg cta ccc gat ctg gat tac      144
Thr His Ser Arg Gly Leu His Val Tyr Thr Leu Pro Asp Leu Asp Tyr
        35                  40                  45

gac tat ggc gca ctg gag cca gcc atc agc ggc gac atc atg cag ctg      192
Asp Tyr Gly Ala Leu Glu Pro Ala Ile Ser Gly Asp Ile Met Gln Leu
    50                  55                  60

cac cac cag aag cac cac cag act tac atc acc aac tac aac aag gcc      240
His His Gln Lys His His Gln Thr Tyr Ile Thr Asn Tyr Asn Lys Ala
65                  70                  75                  80

ctc gag cag ctc caa gac gcc atc gcc aag aaa gat tcc tcc gcc gtc      288
Leu Glu Gln Leu Gln Asp Ala Ile Ala Lys Lys Asp Ser Ser Ala Val
                85                  90                  95

gtt aag ctc cag ggc gcc atc aag ttc aac ggc gga ggt cat gtc aac      336
Val Lys Leu Gln Gly Ala Ile Lys Phe Asn Gly Gly Gly His Val Asn
            100                 105                 110

cat tct att ttc tgg aaa aat cta gct cct gtt cgt gaa gga ggt ggt      384
His Ser Ile Phe Trp Lys Asn Leu Ala Pro Val Arg Glu Gly Gly Gly
        115                 120                 125

gaa cca ccc aag ggt tca ctg gga tgg gct att gac aca cat ttt ggt      432
Glu Pro Pro Lys Gly Ser Leu Gly Trp Ala Ile Asp Thr His Phe Gly
    130                 135                 140

tct ttt gaa gca tta ata caa aaa gtt aac gca gaa ggt gct gca cta      480
Ser Phe Glu Ala Leu Ile Gln Lys Val Asn Ala Glu Gly Ala Ala Leu
145                 150                 155                 160

cag ggg tct gga tgg gtg tgg ctt ggt ctg gac aaa gag ttg aag agg      528
Gln Gly Ser Gly Trp Val Trp Leu Gly Leu Asp Lys Glu Leu Lys Arg
                165                 170                 175

ctt gta gtt gaa acc act gcc aac cag gac cca ctg gtt act aag gga      576
Leu Val Val Glu Thr Thr Ala Asn Gln Asp Pro Leu Val Thr Lys Gly
            180                 185                 190

cca aat ttg gtt cca ttg att ggt att gat gtt tgg gag cat gcg tac      624
Pro Asn Leu Val Pro Leu Ile Gly Ile Asp Val Trp Glu His Ala Tyr
        195                 200                 205

tac tta cag tac aag aat gtt aga cca gac tat ctg aag aac att tgg      672
Tyr Leu Gln Tyr Lys Asn Val Arg Pro Asp Tyr Leu Lys Asn Ile Trp
    210                 215                 220

aaa gtt att aat tgg aaa tat gcc agt gaa gtg tat gag aaa gag agc      720
Lys Val Ile Asn Trp Lys Tyr Ala Ser Glu Val Tyr Glu Lys Glu Ser
225                 230                 235                 240

tct                                                                    723
Ser

<210>  525
<211>  241
<212>  PRT
<213>  Glycine max

<400>  525

Met Ala Ala Arg Ala Leu Leu Thr Arg Lys Thr Leu Ala Thr Val Leu
1               5                   10                  15

Arg Asn Asp Ala Lys Pro Ile Ile Gly Val Gly Ile Thr Ala Ala Ala
            20                  25                  30

Thr His Ser Arg Gly Leu His Val Tyr Thr Leu Pro Asp Leu Asp Tyr
        35                  40                  45

Asp Tyr Gly Ala Leu Glu Pro Ala Ile Ser Gly Asp Ile Met Gln Leu
    50                  55                  60

His His Gln Lys His His Gln Thr Tyr Ile Thr Asn Tyr Asn Lys Ala
65                  70                  75                  80

Leu Glu Gln Leu Gln Asp Ala Ile Ala Lys Lys Asp Ser Ser Ala Val
                85                  90                  95

Val Lys Leu Gln Gly Ala Ile Lys Phe Asn Gly Gly Gly His Val Asn
            100                 105                 110

His Ser Ile Phe Trp Lys Asn Leu Ala Pro Val Arg Glu Gly Gly Gly
        115                 120                 125

Glu Pro Pro Lys Gly Ser Leu Gly Trp Ala Ile Asp Thr His Phe Gly
    130                 135                 140

Ser Phe Glu Ala Leu Ile Gln Lys Val Asn Ala Glu Gly Ala Ala Leu
145                 150                 155                 160

Gln Gly Ser Gly Trp Val Trp Leu Gly Leu Asp Lys Glu Leu Lys Arg
                165                 170                 175

Leu Val Val Glu Thr Thr Ala Asn Gln Asp Pro Leu Val Thr Lys Gly
            180                 185                 190

Pro Asn Leu Val Pro Leu Ile Gly Ile Asp Val Trp Glu His Ala Tyr
        195                 200                 205

Tyr Leu Gln Tyr Lys Asn Val Arg Pro Asp Tyr Leu Lys Asn Ile Trp
    210                 215                 220

Lys Val Ile Asn Trp Lys Tyr Ala Ser Glu Val Tyr Glu Lys Glu Ser
225                 230                 235                 240

Ser

<210>  526
<211>  11
<212>  PRT
<213>  Glycine max

<400>  526

Ala Leu Glu Gln Leu Gln Asp Ala Ile Ala Lys
1               5                   10

<210>  527
<211>  11
<212>  PRT
<213>  Glycine max

<400>  527

His His Gln Thr Tyr Ile Thr Asn Tyr Asn Lys
1               5                   10

<210>  528
<211>  14
<212>  PRT
<213>  Glycine max

<400>  528

Asn Leu Ala Pro Val Arg Glu Gly Gly Gly Glu Pro Pro Lys
1               5                   10

<210>  529
<211>  8
<212>  PRT
<213>  Glycine max

<400>  529

Asn Val Arg Pro Asp Tyr Leu Lys
1               5

<210>  530
<211>  16
<212>  PRT
<213>  Glycine max

<400>  530

Arg Leu Val Val Glu Thr Thr Ala Asn Gln Asp Pro Leu Val Thr Lys
1               5                   10                  15


<210>  531
<211>  8
<212>  PRT
<213>  Glycine max

<400>  531

Tyr Ala Ser Glu Val Tyr Glu Lys
1               5


<210>  532
<211>  720
<212>  DNA
<213>  Glycine max


<220>
<221>  CDS
<222>  (1)..(720)

<400>  532
atg gcc gcg cga gcc ctg ttg acc aga aaa acc cta gcc acc gtt ctc      48
Met Ala Ala Arg Ala Leu Leu Thr Arg Lys Thr Leu Ala Thr Val Leu
1               5                   10                  15

cgc aac gac gcg aag ccc ata atc gga gct ggc ata gct gca gct act      96
Arg Asn Asp Ala Lys Pro Ile Ile Gly Ala Gly Ile Ala Ala Ala Thr
            20                  25                  30

cat tct cgt ggg ttg cac gtg tac acg ctc ccc gat ctg gat tac gac      144
His Ser Arg Gly Leu His Val Tyr Thr Leu Pro Asp Leu Asp Tyr Asp
        35                  40                  45

tat ggc gct ctg gag cca gcc atc agc ggc gaa atc atg cag ctg cac      192
Tyr Gly Ala Leu Glu Pro Ala Ile Ser Gly Glu Ile Met Gln Leu His
    50                  55                  60

cac cag aag cac cac cag act tac atc acc aac ttc aac aag gcc ctc      240
His Gln Lys His His Gln Thr Tyr Ile Thr Asn Phe Asn Lys Ala Leu
65                  70                  75                  80

gag cag ctc caa gac gcc gtc gcc aag aaa gat tcc tcc gcc gtc gtt      288
Glu Gln Leu Gln Asp Ala Val Ala Lys Lys Asp Ser Ser Ala Val Val
                85                  90                  95

aag ctc cag ggc gcc atc aag ttc aac ggc gga ggt cac gtc aac cat      336
Lys Leu Gln Gly Ala Ile Lys Phe Asn Gly Gly Gly His Val Asn His
            100                 105                 110

tct att ttc tgg aaa aat cta gct cct gtt cgt gaa gga ggt ggt gaa      384
Ser Ile Phe Trp Lys Asn Leu Ala Pro Val Arg Glu Gly Gly Gly Glu
        115                 120                 125

cca ccc aag ggt tca ctg gga tgg gcc att gac aca cat ttt ggc tct      432
Pro Pro Lys Gly Ser Leu Gly Trp Ala Ile Asp Thr His Phe Gly Ser
    130                 135                 140

ttt gaa gca tta gta caa aaa gtt aat gca gaa ggt gct gca cta cag 480
Phe Glu Ala Leu Val Gln Lys Val Asn Ala Glu Gly Ala Ala Leu Gln
145                 150                 155                 160

ggg tct gga tgg gtg tgg ctt ggt ctg gac aaa gag ttg aag agg ctt 528
Gly Ser Gly Trp Val Trp Leu Gly Leu Asp Lys Glu Leu Lys Arg Leu
                165                 170                 175

gta gtt gaa acc act gcc aac cag gac cca ctg gtt act aag gga cca 576
Val Val Glu Thr Thr Ala Asn Gln Asp Pro Leu Val Thr Lys Gly Pro
            180                 185                 190

aat ttg gtt cca ttg ctt ggt att gat gtt tgg gag cat gcg tac tac 624
Asn Leu Val Pro Leu Leu Gly Ile Asp Val Trp Glu His Ala Tyr Tyr
        195                 200                 205

tta cag tac aag aat gtt aga cca gac tat ctg aag aac att tgg aaa 672
Leu Gln Tyr Lys Asn Val Arg Pro Asp Tyr Leu Lys Asn Ile Trp Lys
    210                 215                 220

gtt att aat tgg aaa tat gcc agt gaa gtg tat gag aaa gag agc tct 720
Val Ile Asn Trp Lys Tyr Ala Ser Glu Val Tyr Glu Lys Glu Ser Ser
225                 230                 235                 240

<210> 533
<211> 240
<212> PRT
<213> Glycine max

<400> 533

Met Ala Ala Arg Ala Leu Leu Thr Arg Lys Thr Leu Ala Thr Val Leu
1               5                   10                  15

Arg Asn Asp Ala Lys Pro Ile Ile Gly Ala Gly Ile Ala Ala Ala Thr
            20                  25                  30

His Ser Arg Gly Leu His Val Tyr Thr Leu Pro Asp Leu Asp Tyr Asp
        35                  40                  45

Tyr Gly Ala Leu Glu Pro Ala Ile Ser Gly Glu Ile Met Gln Leu His
    50                  55                  60

His Gln Lys His His Gln Thr Tyr Ile Thr Asn Phe Asn Lys Ala Leu
65                  70                  75                  80

Glu Gln Leu Gln Asp Ala Val Ala Lys Lys Asp Ser Ser Ala Val Val
                85                  90                  95

Lys Leu Gln Gly Ala Ile Lys Phe Asn Gly Gly Gly His Val Asn His
            100                 105                 110

Ser Ile Phe Trp Lys Asn Leu Ala Pro Val Arg Glu Gly Gly Gly Glu
        115                 120                 125

Pro Pro Lys Gly Ser Leu Gly Trp Ala Ile Asp Thr His Phe Gly Ser
    130                 135                 140

Phe Glu Ala Leu Val Gln Lys Val Asn Ala Glu Gly Ala Ala Leu Gln
145                 150                 155                 160

Gly Ser Gly Trp Val Trp Leu Gly Leu Asp Lys Glu Leu Lys Arg Leu
                165                 170                 175

Val Val Glu Thr Thr Ala Asn Gln Asp Pro Leu Val Thr Lys Gly Pro
            180                 185                 190

Asn Leu Val Pro Leu Leu Gly Ile Asp Val Trp Glu His Ala Tyr Tyr
        195                 200                 205

Leu Gln Tyr Lys Asn Val Arg Pro Asp Tyr Leu Lys Asn Ile Trp Lys
    210                 215                 220

Val Ile Asn Trp Lys Tyr Ala Ser Glu Val Tyr Glu Lys Glu Ser Ser
225                 230                 235                 240

<210>  534
<211>  11
<212>  PRT
<213>  Glycine max

<400>  534

Ala Leu Glu Gln Leu Gln Asp Ala Val Ala Lys
1               5                   10

<210>  535
<211>  11
<212>  PRT
<213>  Glycine max

<400>  535

His His Gln Thr Tyr Ile Thr Asn Phe Asn Lys
1               5                   10

<210>  536
<211>  14
<212>  PRT
<213>  Glycine max

<400>  536

Asn Leu Ala Pro Val Arg Glu Gly Gly Gly Glu Pro Pro Lys
1               5                   10

<210>  537
<211>  8
<212>  PRT
<213>  Glycine max

<400>  537

Asn Val Arg Pro Asp Tyr Leu Lys
1               5


<210>  538
<211>  16
<212>  PRT
<213>  Glycine max

<400>  538

Arg Leu Val Val Glu Thr Thr Ala Asn Gln Asp Pro Leu Val Thr Lys
1               5                   10                  15


<210>  539
<211>  8
<212>  PRT
<213>  Glycine max

<400>  539

Tyr Ala Ser Glu Val Tyr Glu Lys
1               5


<210>  540
<211>  486
<212>  DNA
<213>  Glycine max


<220>
<221>  CDS
<222>  (1)..(486)

<400>  540
atg gct cca att gca gtg gga gat gtc att cca gac ggc att ttg gct      48
Met Ala Pro Ile Ala Val Gly Asp Val Ile Pro Asp Gly Ile Leu Ala
1               5                   10                  15

tac ttg gat gaa gaa aac aaa ccc caa act gtc tct att cac tct ctc      96
Tyr Leu Asp Glu Glu Asn Lys Pro Gln Thr Val Ser Ile His Ser Leu
            20                  25                  30

gcc gcc ggc aag aaa gtc atc atc ttt ggg gtt ccc ggt gcc ttc act      144
Ala Ala Gly Lys Lys Val Ile Ile Phe Gly Val Pro Gly Ala Phe Thr
        35                  40                  45

cct aca tgc agc ttg aag cac gtg cct ggc ttc att gag aga gca gaa      192
Pro Thr Cys Ser Leu Lys His Val Pro Gly Phe Ile Glu Arg Ala Glu
    50                  55                  60

gag ctg aaa gga aag ggt gtg gac gaa ata atc tgt atc agc gtg aat      240
Glu Leu Lys Gly Lys Gly Val Asp Glu Ile Ile Cys Ile Ser Val Asn
65                  70                  75                  80

gat ccc ttt gtg atg aac tca tgg gcc aaa acg ttc cca gag aac aag     288
Asp Pro Phe Val Met Asn Ser Trp Ala Lys Thr Phe Pro Glu Asn Lys
                85                  90                  95

cat gtt aag ttc ctt gct gat ggt gca gcc aaa tac acc aat gcc ctc     336
His Val Lys Phe Leu Ala Asp Gly Ala Ala Lys Tyr Thr Asn Ala Leu
            100                 105                 110

ggt ctt gag ctt gac ctc acc gac aag ggt ctc ggc gtc cgc tca aag     384
Gly Leu Glu Leu Asp Leu Thr Asp Lys Gly Leu Gly Val Arg Ser Lys
        115                 120                 125

agg ttt gct ttg ctg gtg gaa gac ctc aag gtg aag gtt gca aat gtt     432
Arg Phe Ala Leu Leu Val Glu Asp Leu Lys Val Lys Val Ala Asn Val
    130                 135                 140

gaa agt gga gga gag ttc acc atc tcc agt gct gaa gag atc atc aag     480
Glu Ser Gly Gly Glu Phe Thr Ile Ser Ser Ala Glu Glu Ile Ile Lys
145                 150                 155                 160

gcc ctc                                                             486
Ala Leu

<210>  541
<211>  162
<212>  PRT
<213>  Glycine max

<400>  541

Met Ala Pro Ile Ala Val Gly Asp Val Ile Pro Asp Gly Ile Leu Ala
1               5                   10                  15

Tyr Leu Asp Glu Glu Asn Lys Pro Gln Thr Val Ser Ile His Ser Leu
            20                  25                  30

Ala Ala Gly Lys Lys Val Ile Ile Phe Gly Val Pro Gly Ala Phe Thr
        35                  40                  45

Pro Thr Cys Ser Leu Lys His Val Pro Gly Phe Ile Glu Arg Ala Glu
    50                  55                  60

Glu Leu Lys Gly Lys Gly Val Asp Glu Ile Ile Cys Ile Ser Val Asn
65                  70                  75                  80

Asp Pro Phe Val Met Asn Ser Trp Ala Lys Thr Phe Pro Glu Asn Lys
                85                  90                  95

His Val Lys Phe Leu Ala Asp Gly Ala Ala Lys Tyr Thr Asn Ala Leu
            100                 105                 110

Gly Leu Glu Leu Asp Leu Thr Asp Lys Gly Leu Gly Val Arg Ser Lys
        115                 120                 125

Arg Phe Ala Leu Leu Val Glu Asp Leu Lys Val Lys Val Ala Asn Val
    130                 135                 140

Glu Ser Gly Gly Glu Phe Thr Ile Ser Ser Ala Glu Glu Ile Ile Lys
145                 150                 155                 160

Ala Leu

<210>  542
<211>  13
<212>  PRT
<213>  Glycine max

<400>  542

His Val Pro Gly Phe Ile Glu Arg Ala Glu Glu Leu Lys
1               5                   10

<210>  543
<211>  10
<212>  PRT
<213>  Glycine max

<400>  543

Arg Phe Ala Leu Leu Val Glu Asp Leu Lys
1               5                   10

<210>  544
<211>  20
<212>  PRT
<213>  Glycine max

<400>  544

Val Ala Asn Val Glu Ser Gly Gly Glu Phe Thr Ile Ser Ser Ala Glu
1               5                   10                  15

Glu Ile Ile Lys
            20

<210>  545
<211>  14
<212>  PRT
<213>  Glycine max

<400>  545

Tyr Thr Asn Ala Leu Gly Leu Glu Leu Asp Leu Thr Asp Lys
1               5                   10

<210>  546

<211>  486
<212>  DNA
<213>  Glycine max

<220>
<221>  CDS
<222>  (1)..(486)

<400>  546
atg gct cca att gca gtg gga aac gtg att cca gac ggc att ttg gct      48
Met Ala Pro Ile Ala Val Gly Asn Val Ile Pro Asp Gly Ile Leu Ala
1               5                   10                  15

tac ttg gat gaa gaa aac aaa ccc caa act gtc tcc att cac tct ctc      96
Tyr Leu Asp Glu Glu Asn Lys Pro Gln Thr Val Ser Ile His Ser Leu
            20                  25                  30

gcc acc ggc aag aaa gtc atc atc ttt ggg gtt ccc ggt gcc ttc act      144
Ala Thr Gly Lys Lys Val Ile Ile Phe Gly Val Pro Gly Ala Phe Thr
        35                  40                  45

cct act tgc agc ttg aag cac gtt cct ggc ttc att gag aga gca gaa      192
Pro Thr Cys Ser Leu Lys His Val Pro Gly Phe Ile Glu Arg Ala Glu
    50                  55                  60

gag ctg aaa ggg aag ggt gtg gac gaa ata atc tgt atc agt gtg aat      240
Glu Leu Lys Gly Lys Gly Val Asp Glu Ile Ile Cys Ile Ser Val Asn
65                  70                  75                  80

gat cca ttt gtg atg aac tca tgg gcc aaa aca ttc cca gag aac aag      288
Asp Pro Phe Val Met Asn Ser Trp Ala Lys Thr Phe Pro Glu Asn Lys
                85                  90                  95

cat gtt aag ttc ctt gct gat ggt gca gcc aaa tac acc aat gcc ctt      336
His Val Lys Phe Leu Ala Asp Gly Ala Ala Lys Tyr Thr Asn Ala Leu
            100                 105                 110

ggt ctt gag ctt gac ctc act gac aag ggt ctc ggc gtc cgc tca aag      384
Gly Leu Glu Leu Asp Leu Thr Asp Lys Gly Leu Gly Val Arg Ser Lys
        115                 120                 125

agg ttt gct ctg ctg gtg gaa gac ctc aag gtg aag gtt gcg aat gtt      432
Arg Phe Ala Leu Leu Val Glu Asp Leu Lys Val Lys Val Ala Asn Val
    130                 135                 140

gaa agt gga gga gag ttc acc atc tcc agt gct gaa gag atc atc aag      480
Glu Ser Gly Gly Glu Phe Thr Ile Ser Ser Ala Glu Glu Ile Ile Lys
145                 150                 155                 160

gcc ctt                                                              486
Ala Leu

<210>  547
<211>  162
<212>  PRT
<213>  Glycine max

<400>  547

Met Ala Pro Ile Ala Val Gly Asn Val Ile Pro Asp Gly Ile Leu Ala

1               5                   10                  15

Tyr Leu Asp Glu Glu Asn Lys Pro Gln Thr Val Ser Ile His Ser Leu
            20                  25                  30

Ala Thr Gly Lys Lys Val Ile Ile Phe Gly Val Pro Gly Ala Phe Thr
        35                  40                  45

Pro Thr Cys Ser Leu Lys His Val Pro Gly Phe Ile Glu Arg Ala Glu
    50                  55                  60

Glu Leu Lys Gly Lys Gly Val Asp Glu Ile Ile Cys Ile Ser Val Asn
65                  70                  75                  80

Asp Pro Phe Val Met Asn Ser Trp Ala Lys Thr Phe Pro Glu Asn Lys
                85                  90                  95

His Val Lys Phe Leu Ala Asp Gly Ala Ala Lys Tyr Thr Asn Ala Leu
            100                 105                 110

Gly Leu Glu Leu Asp Leu Thr Asp Lys Gly Leu Gly Val Arg Ser Lys
        115                 120                 125

Arg Phe Ala Leu Leu Val Glu Asp Leu Lys Val Lys Val Ala Asn Val
    130                 135                 140

Glu Ser Gly Gly Glu Phe Thr Ile Ser Ser Ala Glu Glu Ile Ile Lys
145                 150                 155                 160

Ala Leu

<210>  548
<211>  13
<212>  PRT
<213>  Glycine max

<400>  548

His Val Pro Gly Phe Ile Glu Arg Ala Glu Glu Leu Lys
1               5                   10

<210>  549
<211>  10
<212>  PRT
<213>  Glycine max

<400>  549

Arg Phe Ala Leu Leu Val Glu Asp Leu Lys
1               5                   10

<210>  550
<211>  20
<212>  PRT
<213>  Glycine max

<400>  550

Val Ala Asn Val Glu Ser Gly Gly Glu Phe Thr Ile Ser Ser Ala Glu
1               5                   10                  15

Glu Ile Ile Lys
            20

<210>  551
<211>  14
<212>  PRT
<213>  Glycine max

<400>  551

Tyr Thr Asn Ala Leu Gly Leu Glu Leu Asp Leu Thr Asp Lys
1               5                   10

<210>  552
<211>  456
<212>  DNA
<213>  Glycine max

<220>
<221>  CDS
<222>  (1)..(456)

<400>  552
atg tcg cag ttg ctg gat aaa gcc aag aac ttc gtg tcg gag aag gta      48
Met Ser Gln Leu Leu Asp Lys Ala Lys Asn Phe Val Ser Glu Lys Val
1               5                   10                  15

aat gat atg gcc aag ccg gag gcg agt gtc act gac gtg gac ttc aag      96
Asn Asp Met Ala Lys Pro Glu Ala Ser Val Thr Asp Val Asp Phe Lys
            20                  25                  30

cgc gtg agc aaa gat aat gtc gag tac ttg gcc aag gtc tct gtt cgt      144
Arg Val Ser Lys Asp Asn Val Glu Tyr Leu Ala Lys Val Ser Val Arg
        35                  40                  45

aac cct tat tca act tcc ata ccc att tgt gag atc aac tac tct ttc      192
Asn Pro Tyr Ser Thr Ser Ile Pro Ile Cys Glu Ile Asn Tyr Ser Phe
    50                  55                  60

aaa agc gca tcc agg gag ata gca tca ggg aaa att cca gac cca gga      240
Lys Ser Ala Ser Arg Glu Ile Ala Ser Gly Lys Ile Pro Asp Pro Gly
65                  70                  75                  80

tcg ttg aag gca aag gac aca aca atg gtg gat gtg cca gtt aag gtt      288
Ser Leu Lys Ala Lys Asp Thr Thr Met Val Asp Val Pro Val Lys Val
                85                  90                  95

cca tac agc ata ttg atg agc ttg gca aag gac att ggt gct gac tgg     336
Pro Tyr Ser Ile Leu Met Ser Leu Ala Lys Asp Ile Gly Ala Asp Trp
            100                 105                 110

gac ata gac tat caa ttg gat ctt ggt ctg gtt att gat gtt cct gtc     384
Asp Ile Asp Tyr Gln Leu Asp Leu Gly Leu Val Ile Asp Val Pro Val
        115                 120                 125

att ggc atc ttc acc att cct ctc tct cag aaa gga gag atc aag cta     432
Ile Gly Ile Phe Thr Ile Pro Leu Ser Gln Lys Gly Glu Ile Lys Leu
    130                 135                 140

cca acc ctc tct acc atg ttc gcc                                     456
Pro Thr Leu Ser Thr Met Phe Ala
145                 150

<210>  553
<211>  152
<212>  PRT
<213>  Glycine max

<400>  553

Met Ser Gln Leu Leu Asp Lys Ala Lys Asn Phe Val Ser Glu Lys Val
1               5                   10                  15

Asn Asp Met Ala Lys Pro Glu Ala Ser Val Thr Asp Val Asp Phe Lys
            20                  25                  30

Arg Val Ser Lys Asp Asn Val Glu Tyr Leu Ala Lys Val Ser Val Arg
        35                  40                  45

Asn Pro Tyr Ser Thr Ser Ile Pro Ile Cys Glu Ile Asn Tyr Ser Phe
    50                  55                  60

Lys Ser Ala Ser Arg Glu Ile Ala Ser Gly Lys Ile Pro Asp Pro Gly
65                  70                  75                  80

Ser Leu Lys Ala Lys Asp Thr Thr Met Val Asp Val Pro Val Lys Val
                85                  90                  95

Pro Tyr Ser Ile Leu Met Ser Leu Ala Lys Asp Ile Gly Ala Asp Trp
            100                 105                 110

Asp Ile Asp Tyr Gln Leu Asp Leu Gly Leu Val Ile Asp Val Pro Val
        115                 120                 125

Ile Gly Ile Phe Thr Ile Pro Leu Ser Gln Lys Gly Glu Ile Lys Leu
    130                 135                 140

Pro Thr Leu Ser Thr Met Phe Ala
145                 150

<210>  554
<211>  8
<212>  PRT
<213>  Glycine max

<400>  554

Asp Asn Val Glu Tyr Leu Ala Lys
1               5


<210>  555
<211>  480
<212>  DNA
<213>  Glycine max


<220>
<221>  CDS
<222>  (1)..(480)

<400>  555
atg tcg gac gaa gag cac cat ttc gag tcc aag gca gac gcc ggt gcc      48
Met Ser Asp Glu Glu His His Phe Glu Ser Lys Ala Asp Ala Gly Ala
1               5                   10                  15

tcc aaa acc tac cct cag caa gcc ggt acc att cgc aag aac ggc tac      96
Ser Lys Thr Tyr Pro Gln Gln Ala Gly Thr Ile Arg Lys Asn Gly Tyr
            20                  25                  30

atc gtc atc aaa gga cgc ccc tgc aag gtt gtg gaa gtt tcg act tcc      144
Ile Val Ile Lys Gly Arg Pro Cys Lys Val Val Glu Val Ser Thr Ser
        35                  40                  45

aaa act gga aag cac ggt cac gcc aag tgc cac ttt gtt gga att gat      192
Lys Thr Gly Lys His Gly His Ala Lys Cys His Phe Val Gly Ile Asp
    50                  55                  60

att ttc act gcc aag aaa ctt gag gat att gtg ccc tct tct cac aac      240
Ile Phe Thr Ala Lys Lys Leu Glu Asp Ile Val Pro Ser Ser His Asn
65                  70                  75                  80

tgt gat gtt cct cat gtg aat cgt act gat tac cag ctc att gat att      288
Cys Asp Val Pro His Val Asn Arg Thr Asp Tyr Gln Leu Ile Asp Ile
                85                  90                  95

gct gag gat gga ttt ttg agt ctg ctt act gaa aat ggg aac acc aag      336
Ala Glu Asp Gly Phe Leu Ser Leu Leu Thr Glu Asn Gly Asn Thr Lys
            100                 105                 110

gat gac ctc aag ctt ccc act gat gag agt ctg ctc act cag ata aag      384
Asp Asp Leu Lys Leu Pro Thr Asp Glu Ser Leu Leu Thr Gln Ile Lys
        115                 120                 125

gat gga ttt gct gag ggc aag gat ctt gtt gtg tct gtc atg tct gct      432
Asp Gly Phe Ala Glu Gly Lys Asp Leu Val Val Ser Val Met Ser Ala
    130                 135                 140

atg ggt gag gaa cag att tgc gcc ctg aag gat att ggg cca aag aac      480
Met Gly Glu Glu Gln Ile Cys Ala Leu Lys Asp Ile Gly Pro Lys Asn
145                 150                 155                 160

<210>  556
<211>  160
<212>  PRT
<213>  Glycine max

<400>  556

Met Ser Asp Glu Glu His His Phe Glu Ser Lys Ala Asp Ala Gly Ala
1               5                   10                  15

Ser Lys Thr Tyr Pro Gln Gln Ala Gly Thr Ile Arg Lys Asn Gly Tyr
            20                  25                  30

Ile Val Ile Lys Gly Arg Pro Cys Lys Val Val Glu Val Ser Thr Ser
        35                  40                  45

Lys Thr Gly Lys His Gly His Ala Lys Cys His Phe Val Gly Ile Asp
    50                  55                  60

Ile Phe Thr Ala Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ala Glu Asp Gly Phe Leu Ser Leu Leu Thr Glu Asn Gly Asn Thr Lys
            100                 105                 110

Asp Asp Leu Lys Leu Pro Thr Asp Glu Ser Leu Leu Thr Gln Ile Lys
        115                 120                 125

Asp Gly Phe Ala Glu Gly Lys Asp Leu Val Val Ser Val Met Ser Ala
    130                 135                 140

Met Gly Glu Glu Gln Ile Cys Ala Leu Lys Asp Ile Gly Pro Lys Asn
145                 150                 155                 160

<210>  557
<211>  12
<212>  PRT
<213>  Glycine max

<400>  557

Leu Pro Thr Asp Glu Ser Leu Leu Thr Gln Ile Lys
1               5                   10

<210>  558
<211>  7
<212>  PRT
<213>  Glycine max

<400>  558

Asn Gly Tyr Ile Val Ile Lys
1               5

<210>  559
<211>  480
<212>  DNA
<213>  Glycine max

<220>
<221>  CDS
<222>  (1)..(480)

<400>  559
atg tcg gac gaa gag cat cac ttc gat tct cag gcg gat gcg gga gcg      48
Met Ser Asp Glu Glu His His Phe Asp Ser Gln Ala Asp Ala Gly Ala
1               5                   10                  15

tcc aag acg tat cct cag cag gct ggt acc att cgc aag aat ggc tac      96
Ser Lys Thr Tyr Pro Gln Gln Ala Gly Thr Ile Arg Lys Asn Gly Tyr
            20                  25                  30

atc gtc atc aaa tcc agg cct tgc aag gtt gta gag gtc tct acg tca      144
Ile Val Ile Lys Ser Arg Pro Cys Lys Val Val Glu Val Ser Thr Ser
        35                  40                  45

aag aca ggc aaa cat gga cat gca aag tgt cac ttt gct gca att gat      192
Lys Thr Gly Lys His Gly His Ala Lys Cys His Phe Ala Ala Ile Asp
    50                  55                  60

att ttt aat ggg aaa aag ctt gaa gat att gtt cca tca tcc cac aac      240
Ile Phe Asn Gly Lys Lys Leu Glu Asp Ile Val Pro Ser Ser His Asn
65                  70                  75                  80

tgt gat gtt cca cat gtg aat cgc act gac tat cag ctt att gat atc      288
Cys Asp Val Pro His Val Asn Arg Thr Asp Tyr Gln Leu Ile Asp Ile
                85                  90                  95

tct gaa gac ggc ttt gtg agt ctg ttg act gat aat gga aat acc aag      336
Ser Glu Asp Gly Phe Val Ser Leu Leu Thr Asp Asn Gly Asn Thr Lys
            100                 105                 110

gat gat ctg aag ctt cct act gat gag gca ttg ctt gct cag att aaa      384
Asp Asp Leu Lys Leu Pro Thr Asp Glu Ala Leu Leu Ala Gln Ile Lys
        115                 120                 125

gat gga ttt tct gaa gga aaa gac ctt gtg gtt tct gtt atg tct gca      432
Asp Gly Phe Ser Glu Gly Lys Asp Leu Val Val Ser Val Met Ser Ala
    130                 135                 140

atg gga gaa gaa cag atc tgt tca ctg aag gac atc ggt ccc aaa aat      480
Met Gly Glu Glu Gln Ile Cys Ser Leu Lys Asp Ile Gly Pro Lys Asn
145                 150                 155                 160

<210>  560
<211>  160
<212>  PRT
<213>  Glycine max

<400>  560

Met Ser Asp Glu Glu His His Phe Asp Ser Gln Ala Asp Ala Gly Ala
1               5                   10                  15

Ser Lys Thr Tyr Pro Gln Gln Ala Gly Thr Ile Arg Lys Asn Gly Tyr
            20                  25                  30

Ile Val Ile Lys Ser Arg Pro Cys Lys Val Val Glu Val Ser Thr Ser
        35                  40                  45

Lys Thr Gly Lys His Gly His Ala Lys Cys His Phe Ala Ala Ile Asp
    50                  55                  60

Ile Phe Asn Gly Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Asp Asn Gly Asn Thr Lys
            100                 105                 110

Asp Asp Leu Lys Leu Pro Thr Asp Glu Ala Leu Leu Ala Gln Ile Lys
        115                 120                 125

Asp Gly Phe Ser Glu Gly Lys Asp Leu Val Val Ser Val Met Ser Ala
    130                 135                 140

Met Gly Glu Glu Gln Ile Cys Ser Leu Lys Asp Ile Gly Pro Lys Asn
145                 150                 155                 160


<210>  561
<211>  7
<212>  PRT
<213>  Glycine max

<400>  561

Asn Gly Tyr Ile Val Ile Lys
1               5


<210>  562
<211>  11
<212>  PRT
<213>  Glycine max

<400>  562

Thr Tyr Pro Gln Gln Ala Gly Thr Ile Arg Lys
1               5                   10

<210>  563
<211>  8
<212>  PRT
<213>  Glycine max

<400>  563

Val Val Glu Val Ser Thr Ser Lys
1               5


<210>  564
<211>  501
<212>  DNA
<213>  Glycine max


<220>
<221>  CDS
<222>  (1)..(501)

<400>  564
atg gct agc caa tca aac act aaa tca gtt cac gat ttc aca gtt aaa      48
Met Ala Ser Gln Ser Asn Thr Lys Ser Val His Asp Phe Thr Val Lys
1               5                   10                  15

gat gcc agg gga aat aat gtt aat ctc gcc gat tac aaa gga aag gtc      96
Asp Ala Arg Gly Asn Asn Val Asn Leu Ala Asp Tyr Lys Gly Lys Val
            20                  25                  30

ctt ctc att gtc aat gtt gcc tca caa tgc ggc ttg act aat tca aac      144
Leu Leu Ile Val Asn Val Ala Ser Gln Cys Gly Leu Thr Asn Ser Asn
        35                  40                  45

tac aca gag ctt aac caa ttg tat gag aaa tac aaa ggg aaa gga ctt      192
Tyr Thr Glu Leu Asn Gln Leu Tyr Glu Lys Tyr Lys Gly Lys Gly Leu
    50                  55                  60

gaa att ctg gca ttc cca tgc aat cag ttt ggg gct cag gag cca gga      240
Glu Ile Leu Ala Phe Pro Cys Asn Gln Phe Gly Ala Gln Glu Pro Gly
65                  70                  75                  80

act aat gaa gag att gtg gag ttt gct tgt act cgc ttc aaa gct gag      288
Thr Asn Glu Glu Ile Val Glu Phe Ala Cys Thr Arg Phe Lys Ala Glu
                85                  90                  95

ttt ccc att ttc gac aag gtg gat gtg aat ggt gac aat gct gct cca      336
Phe Pro Ile Phe Asp Lys Val Asp Val Asn Gly Asp Asn Ala Ala Pro
            100                 105                 110

ctg tac aag ttt ctg aaa tca agc aaa ggt gga ctc ttt ggg gat agt      384
Leu Tyr Lys Phe Leu Lys Ser Ser Lys Gly Gly Leu Phe Gly Asp Ser
        115                 120                 125

atc aaa tgg aac ttc tcc aaa ttc ctt gtt gat aag gat ggg aat gtc      432
Ile Lys Trp Asn Phe Ser Lys Phe Leu Val Asp Lys Asp Gly Asn Val
    130                 135                 140

gtt gac cgt tat gca ccc aca act tct cct ctc agc att gag aaa gac      480
Val Asp Arg Tyr Ala Pro Thr Thr Ser Pro Leu Ser Ile Glu Lys Asp
145                 150                 155                 160

ata aag aag ctg cta gat gca                                                501
Ile Lys Lys Leu Leu Asp Ala
                165

<210>  565
<211>  167
<212>  PRT
<213>  Glycine max

<400>  565

Met Ala Ser Gln Ser Asn Thr Lys Ser Val His Asp Phe Thr Val Lys
1               5                   10                  15

Asp Ala Arg Gly Asn Asn Val Asn Leu Ala Asp Tyr Lys Gly Lys Val
            20                  25                  30

Leu Leu Ile Val Asn Val Ala Ser Gln Cys Gly Leu Thr Asn Ser Asn
        35                  40                  45

Tyr Thr Glu Leu Asn Gln Leu Tyr Glu Lys Tyr Lys Gly Lys Gly Leu
    50                  55                  60

Glu Ile Leu Ala Phe Pro Cys Asn Gln Phe Gly Ala Gln Glu Pro Gly
65                  70                  75                  80

Thr Asn Glu Glu Ile Val Glu Phe Ala Cys Thr Arg Phe Lys Ala Glu
                85                  90                  95

Phe Pro Ile Phe Asp Lys Val Asp Val Asn Gly Asp Asn Ala Ala Pro
            100                 105                 110

Leu Tyr Lys Phe Leu Lys Ser Ser Lys Gly Gly Leu Phe Gly Asp Ser
        115                 120                 125

Ile Lys Trp Asn Phe Ser Lys Phe Leu Val Asp Lys Asp Gly Asn Val
    130                 135                 140

Val Asp Arg Tyr Ala Pro Thr Thr Ser Pro Leu Ser Ile Glu Lys Asp
145                 150                 155                 160

Ile Lys Lys Leu Leu Asp Ala
                165

<210>  566
<211>  8
<212>  PRT
<213>  Glycine max

<400>  566

Ala Glu Phe Pro Ile Phe Asp Lys
1               5

<210>  567
<211>  13
<212>  PRT
<213>  Glycine max

<400>  567

Asp Ala Arg Gly Asn Asn Val Asn Leu Ala Asp Tyr Lys
1               5                   10

<210>  568
<211>  19
<212>  PRT
<213>  Glycine max

<400>  568

Asp Gly Asn Val Val Asp Arg Tyr Ala Pro Thr Thr Ser Pro Leu Ser
1               5                   10                  15

Ile Glu Lys

<210>  569
<211>  9
<212>  PRT
<213>  Glycine max

<400>  569

Gly Gly Leu Phe Gly Asp Ser Ile Lys
1               5

<210>  570
<211>  13
<212>  PRT
<213>  Glycine max

<400>  570

Val Asp Val Asn Gly Asp Asn Ala Ala Pro Leu Tyr Lys
1               5                   10

<210>  571
<211>  357
<212>  DNA
<213>  Glycine max

<220>
<221>  CDS
<222>  (1)..(357)

<400>  571
atg gct tcc gaa gaa tcc cag aac aag ccc ccc agc gag cag tcc act      48
Met Ala Ser Glu Glu Ser Gln Asn Lys Pro Pro Ser Glu Gln Ser Thr
1               5                   10                  15

acc gaa ctc atc acg agc gcg aag ctg gtg gcg gag gcg gcg cag tcg      96
Thr Glu Leu Ile Thr Ser Ala Lys Leu Val Ala Glu Ala Ala Gln Ser
            20                  25                  30

gct ctg aag agc gac agc gac aaa gtg gac aag gcc aag gtg gct gac      144
Ala Leu Lys Ser Asp Ser Asp Lys Val Asp Lys Ala Lys Val Ala Asp
        35                  40                  45

gct gcc ggc gat ctt ctc gac gcg gcg ggg aag tat gga aaa ctt gat      192
Ala Ala Gly Asp Leu Leu Asp Ala Ala Gly Lys Tyr Gly Lys Leu Asp
    50                  55                  60

gac aaa caa ggc ata ggg caa tat gtt gac aag gct gct gat tat ctg      240
Asp Lys Gln Gly Ile Gly Gln Tyr Val Asp Lys Ala Ala Asp Tyr Leu
65                  70                  75                  80

cat aat tac cag ggt gat aat acc act gct cct tca cag cct gca gag      288
His Asn Tyr Gln Gly Asp Asn Thr Thr Ala Pro Ser Gln Pro Ala Glu
                85                  90                  95

tct aaa ggt gaa gaa agt ggt ggt ggg tta ggt ggt ctt gca aac ttg      336
Ser Lys Gly Glu Glu Ser Gly Gly Gly Leu Gly Gly Leu Ala Asn Leu
            100                 105                 110

gct gga ggc ttc ttc aaa aag                                          357
Ala Gly Gly Phe Phe Lys Lys
        115


<210>  572
<211>  119
<212>  PRT
<213>  Glycine max

<400>  572

Met Ala Ser Glu Glu Ser Gln Asn Lys Pro Pro Ser Glu Gln Ser Thr
1               5                   10                  15


Thr Glu Leu Ile Thr Ser Ala Lys Leu Val Ala Glu Ala Ala Gln Ser
            20                  25                  30


Ala Leu Lys Ser Asp Ser Asp Lys Val Asp Lys Ala Lys Val Ala Asp
        35                  40                  45


Ala Ala Gly Asp Leu Leu Asp Ala Ala Gly Lys Tyr Gly Lys Leu Asp
    50                  55                  60


Asp Lys Gln Gly Ile Gly Gln Tyr Val Asp Lys Ala Ala Asp Tyr Leu
65                  70                  75                  80


His Asn Tyr Gln Gly Asp Asn Thr Thr Ala Pro Ser Gln Pro Ala Glu
                85                  90                  95

Ser Lys Gly Glu Glu Ser Gly Gly Gly Leu Gly Gly Leu Ala Asn Leu
            100                 105                 110

Ala Gly Gly Phe Phe Lys Lys
        115

<210>  573
<211>  23
<212>  PRT
<213>  Glycine max

<400>  573

Ala Ala Asp Tyr Leu His Asn Tyr Gln Gly Asp Asn Thr Thr Ala Pro
1               5                   10                  15

Ser Gln Pro Ala Glu Ser Lys
            20

<210>  574
<211>  11
<212>  PRT
<213>  Glycine max

<400>  574

Leu Val Ala Glu Ala Ala Gln Ser Ala Leu Lys
1               5                   10

<210>  575
<211>  9
<212>  PRT
<213>  Glycine max

<400>  575

Gln Gly Ile Gly Gln Tyr Val Asp Lys
1               5

<210>  576
<211>  14
<212>  PRT
<213>  Glycine max

<400>  576

Val Ala Asp Ala Ala Gly Asp Leu Leu Asp Ala Ala Gly Lys
1               5                   10

<210>  577
<211>  777
<212>  DNA
<213>  Glycine max

<220>
<221>  CDS
<222>  (1)..(777)

<400>  577
atg gcg gcc gct ccc tcg cct cgc gaa gag aac gtc tac atg gcg aag        48
Met Ala Ala Ala Pro Ser Pro Arg Glu Glu Asn Val Tyr Met Ala Lys
1               5                   10                  15

ctc gcc gag cag gcc gag cgc tac gaa gag atg gtg gag ttc atg gag        96
Leu Ala Glu Gln Ala Glu Arg Tyr Glu Glu Met Val Glu Phe Met Glu
            20                  25                  30

aag gtc tcc gcc gcc gcc gac aac gag gaa ctt acc gtg gag gag agg       144
Lys Val Ser Ala Ala Ala Asp Asn Glu Glu Leu Thr Val Glu Glu Arg
        35                  40                  45

aac ctc ctc tcc gtc gcc tac aag aac gtg atc gga gcg cgc cgc gcc       192
Asn Leu Leu Ser Val Ala Tyr Lys Asn Val Ile Gly Ala Arg Arg Ala
    50                  55                  60

tcg tgg cgc atc atc tcc tcg atc gag cag aag gag gag agc cgc ggc       240
Ser Trp Arg Ile Ile Ser Ser Ile Glu Gln Lys Glu Glu Ser Arg Gly
65                  70                  75                  80

aac gag gat cac gtc tcc gtc atc cgc gac tac cga tcc aag atc gag       288
Asn Glu Asp His Val Ser Val Ile Arg Asp Tyr Arg Ser Lys Ile Glu
                85                  90                  95

tcc gag ctc tcc aac atc tgc gac ggt atc ctc aag ctc ctc gac tct       336
Ser Glu Leu Ser Asn Ile Cys Asp Gly Ile Leu Lys Leu Leu Asp Ser
            100                 105                 110

cgc ctc att cca tcc gct tcc tcc ggc gat tcc aag gtc ttc tac ctc       384
Arg Leu Ile Pro Ser Ala Ser Ser Gly Asp Ser Lys Val Phe Tyr Leu
        115                 120                 125

aag atg aag gga gat tac cac aga tac ctt gcc gag ttc aag acc ggc       432
Lys Met Lys Gly Asp Tyr His Arg Tyr Leu Ala Glu Phe Lys Thr Gly
    130                 135                 140

gcc gag cgt aag gag gct gcc gag agc act ctc tct gca tac aaa gcc       480
Ala Glu Arg Lys Glu Ala Ala Glu Ser Thr Leu Ser Ala Tyr Lys Ala
145                 150                 155                 160

gca cag gac att gcg aat gcc gaa ctg cct cct act cat cca att agg       528
Ala Gln Asp Ile Ala Asn Ala Glu Leu Pro Pro Thr His Pro Ile Arg
                165                 170                 175

ctt ggc ctt gct ctt aac ttt tct gta ttt tac tac gaa atc ctt aat       576
Leu Gly Leu Ala Leu Asn Phe Ser Val Phe Tyr Tyr Glu Ile Leu Asn
            180                 185                 190

tct ccg gat cgt gcc tgc aac ctt gca aaa cag gct ttt gat gaa gct       624
Ser Pro Asp Arg Ala Cys Asn Leu Ala Lys Gln Ala Phe Asp Glu Ala
        195                 200                 205

att gct gaa ttg gat aca ctg gga gag gag tca tac aag gat agc act       672
Ile Ala Glu Leu Asp Thr Leu Gly Glu Glu Ser Tyr Lys Asp Ser Thr
    210                 215                 220

ttg atc atg caa ctc ctt cgt gat aac ctc aca ctt tgg acc tct gac       720

Leu Ile Met Gln Leu Leu Arg Asp Asn Leu Thr Leu Trp Thr Ser Asp
225                 230                 235                 240

atg cag gac gat gga gca gat gaa att aaa gaa gca gca ccg aaa ccg       768
Met Gln Asp Asp Gly Ala Asp Glu Ile Lys Glu Ala Ala Pro Lys Pro
                245                 250                 255

gat gac cag                                                           777
Asp Asp Gln

<210>  578
<211>  259
<212>  PRT
<213>  Glycine max

<400>  578

Met Ala Ala Ala Pro Ser Pro Arg Glu Glu Asn Val Tyr Met Ala Lys
1               5                   10                  15

Leu Ala Glu Gln Ala Glu Arg Tyr Glu Glu Met Val Glu Phe Met Glu
            20                  25                  30

Lys Val Ser Ala Ala Ala Asp Asn Glu Glu Leu Thr Val Glu Glu Arg
        35                  40                  45

Asn Leu Leu Ser Val Ala Tyr Lys Asn Val Ile Gly Ala Arg Arg Ala
    50                  55                  60

Ser Trp Arg Ile Ile Ser Ser Ile Glu Gln Lys Glu Glu Ser Arg Gly
65                  70                  75                  80

Asn Glu Asp His Val Ser Val Ile Arg Asp Tyr Arg Ser Lys Ile Glu
                85                  90                  95

Ser Glu Leu Ser Asn Ile Cys Asp Gly Ile Leu Lys Leu Leu Asp Ser
            100                 105                 110

Arg Leu Ile Pro Ser Ala Ser Ser Gly Asp Ser Lys Val Phe Tyr Leu
        115                 120                 125

Lys Met Lys Gly Asp Tyr His Arg Tyr Leu Ala Glu Phe Lys Thr Gly
    130                 135                 140

Ala Glu Arg Lys Glu Ala Ala Glu Ser Thr Leu Ser Ala Tyr Lys Ala
145                 150                 155                 160

Ala Gln Asp Ile Ala Asn Ala Glu Leu Pro Pro Thr His Pro Ile Arg
                165                 170                 175

Leu Gly Leu Ala Leu Asn Phe Ser Val Phe Tyr Tyr Glu Ile Leu Asn
            180                 185                 190

Ser Pro Asp Arg Ala Cys Asn Leu Ala Lys Gln Ala Phe Asp Glu Ala
        195                 200                 205

Ile Ala Glu Leu Asp Thr Leu Gly Glu Glu Ser Tyr Lys Asp Ser Thr
    210                 215                 220

Leu Ile Met Gln Leu Leu Arg Asp Asn Leu Thr Leu Trp Thr Ser Asp
225                 230                 235                 240

Met Gln Asp Asp Gly Ala Asp Glu Ile Lys Glu Ala Ala Pro Lys Pro
                245                 250                 255

Asp Asp Gln

<210>  579
<211>  11
<212>  PRT
<213>  Glycine max

<400>  579

Glu Ala Ala Glu Ser Thr Leu Ser Ala Tyr Lys
1               5                   10

<210>  580
<211>  16
<212>  PRT
<213>  Glycine max

<400>  580

Leu Leu Asp Ser Arg Leu Ile Pro Ser Ala Ser Ser Gly Asp Ser Lys
1               5                   10                  15

<210>  581
<211>  19
<212>  PRT
<213>  Glycine max

<400>  581

Gln Ala Phe Asp Glu Ala Ile Ala Glu Leu Asp Thr Leu Gly Glu Glu
1               5                   10                  15

Ser Tyr Lys

<210>  582
<211>  23

<212>  PRT
<213>  Glycine max

<400>  582

Val Ser Ala Ala Ala Asp Asn Glu Glu Leu Thr Val Glu Glu Arg Asn
1               5                   10                  15

Leu Leu Ser Val Ala Tyr Lys
            20

<210>  583
<211>  993
<212>  DNA
<213>  Glycine max

<220>
<221>  CDS
<222>  (1)..(993)

<400>  583
atg gcg ttg aac aat ggc gtc ccc gcc acc ggc acc ggc ctc atc gtc      48
Met Ala Leu Asn Asn Gly Val Pro Ala Thr Gly Thr Gly Leu Ile Val
1               5                   10                  15

agc ttc ggt gag atg ctc atc gac ttc gtc ccc acc gtc tct ggc gtg      96
Ser Phe Gly Glu Met Leu Ile Asp Phe Val Pro Thr Val Ser Gly Val
            20                  25                  30

tcc ctg gcc gag gcc cct ggc ttc ctc aag gcc ccc ggc ggc gcc ccc      144
Ser Leu Ala Glu Ala Pro Gly Phe Leu Lys Ala Pro Gly Gly Ala Pro
        35                  40                  45

gct aac gtc gcc atc gcc gtg tcg cgc ctc ggc ggc aaa gcc gcc ttc      192
Ala Asn Val Ala Ile Ala Val Ser Arg Leu Gly Gly Lys Ala Ala Phe
    50                  55                  60

gtc ggc aag ctc ggc gac gac gag ttc ggc cac atg ctc gcc gga atc      240
Val Gly Lys Leu Gly Asp Asp Glu Phe Gly His Met Leu Ala Gly Ile
65                  70                  75                  80

ctc aag gaa aac ggc gtt cgc gcc gac ggc atc aac ttt gac cag ggc      288
Leu Lys Glu Asn Gly Val Arg Ala Asp Gly Ile Asn Phe Asp Gln Gly
                85                  90                  95

gca cgc acc gcc ctg gcc ttc gtg acc cta cgc gcc gac ggg gag cgt      336
Ala Arg Thr Ala Leu Ala Phe Val Thr Leu Arg Ala Asp Gly Glu Arg
            100                 105                 110

gag ttc atg ttc tac aga aac ccc agc gcc gac atg ctc ctc aag ccc      384
Glu Phe Met Phe Tyr Arg Asn Pro Ser Ala Asp Met Leu Leu Lys Pro
        115                 120                 125

gaa gaa ctc aat ctc gaa ctc atc aga tct gca aaa gtt ttc cat tac      432
Glu Glu Leu Asn Leu Glu Leu Ile Arg Ser Ala Lys Val Phe His Tyr
    130                 135                 140

gga tca atc agt ttg atc gtg gag cca tgc aga tca gca cac ttg aag      480
Gly Ser Ile Ser Leu Ile Val Glu Pro Cys Arg Ser Ala His Leu Lys
145                 150                 155                 160

gca atg gaa gta gcc aag gaa tct ggg tgc ttg ctc tcc tat gac ccc 528
Ala Met Glu Val Ala Lys Glu Ser Gly Cys Leu Leu Ser Tyr Asp Pro
                165                 170                 175

aac ctt cgt cta cct ttg tgg cct tcg gct gag gaa gct cgt aag caa 576
Asn Leu Arg Leu Pro Leu Trp Pro Ser Ala Glu Glu Ala Arg Lys Gln
            180                 185                 190

ata ctg agc att tgg gag aag gct gat ttg atc aag gtc agt gat gcg 624
Ile Leu Ser Ile Trp Glu Lys Ala Asp Leu Ile Lys Val Ser Asp Ala
        195                 200                 205

gag ctt gag ttc ctc aca gga agt gac aag att gat gat gaa tct gct 672
Glu Leu Glu Phe Leu Thr Gly Ser Asp Lys Ile Asp Asp Glu Ser Ala
    210                 215                 220

ttg tca ttg tgg cac ccc aat ttg aag ttg ctc ctt gtc act ctt ggg 720
Leu Ser Leu Trp His Pro Asn Leu Lys Leu Leu Leu Val Thr Leu Gly
225                 230                 235                 240

gaa cat ggt tcc aga tac tac acc aag agt ttc aaa gga tcg gta gat 768
Glu His Gly Ser Arg Tyr Tyr Thr Lys Ser Phe Lys Gly Ser Val Asp
                245                 250                 255

gct ttc cat gtc aat aca gtt gat aca act ggt gcc ggt gat tcc ttt 816
Ala Phe His Val Asn Thr Val Asp Thr Thr Gly Ala Gly Asp Ser Phe
            260                 265                 270

gtt ggt gct tta ttg gcc aag att gtc gat gat cag tcc ata ctt gaa 864
Val Gly Ala Leu Leu Ala Lys Ile Val Asp Asp Gln Ser Ile Leu Glu
        275                 280                 285

gat gaa cca agg tta aga gaa gta cta aag ttt gca aat gca tgt gga 912
Asp Glu Pro Arg Leu Arg Glu Val Leu Lys Phe Ala Asn Ala Cys Gly
    290                 295                 300

gct att aca act acc caa aag gga gca att ccg gcc ctt ccc aaa gag 960
Ala Ile Thr Thr Thr Gln Lys Gly Ala Ile Pro Ala Leu Pro Lys Glu
305                 310                 315                 320

gag gct gca ctg aaa ctg atc aaa ggg ggg tca 993
Glu Ala Ala Leu Lys Leu Ile Lys Gly Gly Ser
                325                 330

<210> 584
<211> 331
<212> PRT
<213> Glycine max

<400> 584

Met Ala Leu Asn Asn Gly Val Pro Ala Thr Gly Thr Gly Leu Ile Val
1               5                   10                  15

Ser Phe Gly Glu Met Leu Ile Asp Phe Val Pro Thr Val Ser Gly Val
            20                  25                  30

Ser Leu Ala Glu Ala Pro Gly Phe Leu Lys Ala Pro Gly Gly Ala Pro
        35                  40                  45

Ala Asn Val Ala Ile Ala Val Ser Arg Leu Gly Gly Lys Ala Ala Phe
    50                  55                  60

Val Gly Lys Leu Gly Asp Asp Glu Phe Gly His Met Leu Ala Gly Ile
65                  70                  75                  80

Leu Lys Glu Asn Gly Val Arg Ala Asp Gly Ile Asn Phe Asp Gln Gly
                85                  90                  95

Ala Arg Thr Ala Leu Ala Phe Val Thr Leu Arg Ala Asp Gly Glu Arg
            100                 105                 110

Glu Phe Met Phe Tyr Arg Asn Pro Ser Ala Asp Met Leu Leu Lys Pro
        115                 120                 125

Glu Glu Leu Asn Leu Glu Leu Ile Arg Ser Ala Lys Val Phe His Tyr
    130                 135                 140

Gly Ser Ile Ser Leu Ile Val Glu Pro Cys Arg Ser Ala His Leu Lys
145                 150                 155                 160

Ala Met Glu Val Ala Lys Glu Ser Gly Cys Leu Leu Ser Tyr Asp Pro
                165                 170                 175

Asn Leu Arg Leu Pro Leu Trp Pro Ser Ala Glu Glu Ala Arg Lys Gln
            180                 185                 190

Ile Leu Ser Ile Trp Glu Lys Ala Asp Leu Ile Lys Val Ser Asp Ala
        195                 200                 205

Glu Leu Glu Phe Leu Thr Gly Ser Asp Lys Ile Asp Asp Glu Ser Ala
    210                 215                 220

Leu Ser Leu Trp His Pro Asn Leu Lys Leu Leu Leu Val Thr Leu Gly
225                 230                 235                 240

Glu His Gly Ser Arg Tyr Tyr Thr Lys Ser Phe Lys Gly Ser Val Asp
                245                 250                 255

Ala Phe His Val Asn Thr Val Asp Thr Thr Gly Ala Gly Asp Ser Phe
            260                 265                 270

Val Gly Ala Leu Leu Ala Lys Ile Val Asp Asp Gln Ser Ile Leu Glu
        275                 280                 285

Asp Glu Pro Arg Leu Arg Glu Val Leu Lys Phe Ala Asn Ala Cys Gly

290                 295                 300

Ala Ile Thr Thr Thr Gln Lys Gly Ala Ile Pro Ala Leu Pro Lys Glu
305                 310                 315                 320

Glu Ala Ala Leu Lys Leu Ile Lys Gly Gly Ser
                325                 330

<210>  585
<211>  19
<212>  PRT
<213>  Glycine max

<400>  585

Ala Pro Gly Gly Ala Pro Ala Asn Val Ala Ile Ala Val Ser Arg Leu
1               5                   10                  15

Gly Gly Lys

<210>  586
<211>  27
<212>  PRT
<213>  Glycine max

<400>  586

Gly Ser Val Asp Ala Phe His Val Asn Thr Val Asp Thr Thr Gly Ala
1               5                   10                  15

Gly Asp Ser Phe Val Gly Ala Leu Leu Ala Lys
            20                  25

<210>  587
<211>  19
<212>  PRT
<213>  Glycine max

<400>  587

Ile Val Asp Asp Gln Ser Ile Leu Glu Asp Glu Pro Arg Leu Arg Glu
1               5                   10                  15

Val Leu Lys

<210>  588
<211>  14
<212>  PRT
<213>  Glycine max

<400>  588

Val Ser Asp Ala Glu Leu Glu Phe Leu Thr Gly Ser Asp Lys
1               5                   10

<210>  589
<211>  759
<212>  DNA
<213>  Glycine max

<220>
<221>  CDS
<222>  (1)..(759)

<400>  589
atg ggc aga aag ttc ttc gtt ggt ggc aac tgg aaa tgc aat ggg acc      48
Met Gly Arg Lys Phe Phe Val Gly Gly Asn Trp Lys Cys Asn Gly Thr
1               5                   10                  15

act gag gag gtg aag aag att gtt act act tta aat gaa gct aaa gtc      96
Thr Glu Glu Val Lys Lys Ile Val Thr Thr Leu Asn Glu Ala Lys Val
            20                  25                  30

cct gga gaa gat gtt gta gaa gtt gtt gtg agc cct cct ttt gtg ttc      144
Pro Gly Glu Asp Val Val Glu Val Val Val Ser Pro Pro Phe Val Phe
        35                  40                  45

ctt cct ttt gta aaa agt ttg ctg cgc cct gat ttc cat gtc tcg gcc      192
Leu Pro Phe Val Lys Ser Leu Leu Arg Pro Asp Phe His Val Ser Ala
    50                  55                  60

caa aat tgt tgg gtt cgc aaa ggt ggt gct tat act gga gag gtt agt      240
Gln Asn Cys Trp Val Arg Lys Gly Gly Ala Tyr Thr Gly Glu Val Ser
65                  70                  75                  80

gct gaa atg ctt gtt aat ttg gga att cct tgg gtt att att ggt cac      288
Ala Glu Met Leu Val Asn Leu Gly Ile Pro Trp Val Ile Ile Gly His
                85                  90                  95

tct gaa cgg agg cag ctt ttg aat gaa tca aat gag ttt gtg gga gat      336
Ser Glu Arg Arg Gln Leu Leu Asn Glu Ser Asn Glu Phe Val Gly Asp
            100                 105                 110

aaa gtt gcc tat gca ctt caa caa ggt ctg aaa gtt ata gca tgc att      384
Lys Val Ala Tyr Ala Leu Gln Gln Gly Leu Lys Val Ile Ala Cys Ile
        115                 120                 125

ggg gaa act ctt gaa cag cgt gaa gct ggt aca aca acg gct gtt gtt      432
Gly Glu Thr Leu Glu Gln Arg Glu Ala Gly Thr Thr Thr Ala Val Val
    130                 135                 140

gct gag caa aca aaa gca att gca gct aaa ata tca aat tgg gac aat      480
Ala Glu Gln Thr Lys Ala Ile Ala Ala Lys Ile Ser Asn Trp Asp Asn
145                 150                 155                 160

gtc gtt ttg gcc tat gag cca gtt tgg gcc att gga aca gga aag gtt      528
Val Val Leu Ala Tyr Glu Pro Val Trp Ala Ile Gly Thr Gly Lys Val
                165                 170                 175

gca act cct gct cag gct caa gag gtt cat gct gat tta agg aaa tgg      576
Ala Thr Pro Ala Gln Ala Gln Glu Val His Ala Asp Leu Arg Lys Trp
            180                 185                 190

gtt cat gac aat gtg agt gct gaa gtt gct gca tct gta aga att atc     624
Val His Asp Asn Val Ser Ala Glu Val Ala Ala Ser Val Arg Ile Ile
        195                 200                 205

tat gga ggc tct gta aat gga gga aac tgc aaa gaa ttg gca gca cag     672
Tyr Gly Gly Ser Val Asn Gly Gly Asn Cys Lys Glu Leu Ala Ala Gln
    210                 215                 220

ccc gat gtt gat gga ttt ttg gtt ggt ggt gca tcc ctc aag gcg gaa     720
Pro Asp Val Asp Gly Phe Leu Val Gly Gly Ala Ser Leu Lys Ala Glu
225                 230                 235                 240

ttt gtg gac atc ata aac gct gcc act gtg aag aag aat                 759
Phe Val Asp Ile Ile Asn Ala Ala Thr Val Lys Lys Asn
                245                 250

<210>  590
<211>  253
<212>  PRT
<213>  Glycine max

<400>  590

Met Gly Arg Lys Phe Phe Val Gly Gly Asn Trp Lys Cys Asn Gly Thr
1               5                   10                  15

Thr Glu Glu Val Lys Lys Ile Val Thr Thr Leu Asn Glu Ala Lys Val
            20                  25                  30

Pro Gly Glu Asp Val Val Glu Val Val Val Ser Pro Pro Phe Val Phe
        35                  40                  45

Leu Pro Phe Val Lys Ser Leu Leu Arg Pro Asp Phe His Val Ser Ala
    50                  55                  60

Gln Asn Cys Trp Val Arg Lys Gly Gly Ala Tyr Thr Gly Glu Val Ser
65                  70                  75                  80

Ala Glu Met Leu Val Asn Leu Gly Ile Pro Trp Val Ile Ile Gly His
                85                  90                  95

Ser Glu Arg Arg Gln Leu Leu Asn Glu Ser Asn Glu Phe Val Gly Asp
            100                 105                 110

Lys Val Ala Tyr Ala Leu Gln Gln Gly Leu Lys Val Ile Ala Cys Ile
        115                 120                 125

Gly Glu Thr Leu Glu Gln Arg Glu Ala Gly Thr Thr Thr Ala Val Val
    130                 135                 140

Ala Glu Gln Thr Lys Ala Ile Ala Ala Lys Ile Ser Asn Trp Asp Asn
145                 150                 155                 160

Val Val Leu Ala Tyr Glu Pro Val Trp Ala Ile Gly Thr Gly Lys Val
                165                 170                 175

Ala Thr Pro Ala Gln Ala Gln Glu Val His Ala Asp Leu Arg Lys Trp
            180                 185                 190

Val His Asp Asn Val Ser Ala Glu Val Ala Ala Ser Val Arg Ile Ile
        195                 200                 205

Tyr Gly Gly Ser Val Asn Gly Gly Asn Cys Lys Glu Leu Ala Ala Gln
    210                 215                 220

Pro Asp Val Asp Gly Phe Leu Val Gly Gly Ala Ser Leu Lys Ala Glu
225                 230                 235                 240

Phe Val Asp Ile Ile Asn Ala Ala Thr Val Lys Lys Asn
                245                 250

<210>  591
<211>  13
<212>  PRT
<213>  Glycine max

<400>  591

Ala Glu Phe Val Asp Ile Ile Asn Ala Ala Thr Val Lys
1               5                   10

<210>  592
<211>  19
<212>  PRT
<213>  Glycine max

<400>  592

Glu Leu Ala Ala Gln Pro Asp Val Asp Gly Phe Leu Val Gly Gly Ala
1               5                   10                  15

Ser Leu Lys

<210>  593
<211>  8
<212>  PRT
<213>  Glycine max

<400>  593

Phe Phe Val Gly Gly Asn Trp Lys
1               5

<210>  594

<211>  9
<212>  PRT
<213>  Glycine max

<400>  594

Ile Val Thr Thr Leu Asn Glu Ala Lys
1               5


<210>  595
<211>  16
<212>  PRT
<213>  Glycine max

<400>  595

Val Ala Thr Pro Ala Gln Ala Gln Glu Val His Ala Asp Leu Arg Lys
1               5                   10                  15


<210>  596
<211>  10
<212>  PRT
<213>  Glycine max

<400>  596

Val Ala Tyr Ala Leu Gln Gln Gly Leu Lys
1               5                   10


<210>  597
<211>  22
<212>  PRT
<213>  Glycine max

<400>  597

Val Pro Gly Glu Asp Val Val Glu Val Val Val Ser Pro Pro Phe Val
1               5                   10                  15


Phe Leu Pro Phe Val Lys
            20


<210>  598
<211>  759
<212>  DNA
<213>  Glycine max


<220>
<221>  CDS
<222>  (1)..(759)

<400>  598
atg ggc aga aaa ttc ttc gtt ggt ggc aac tgg aaa tgc aat ggg acc          48
Met Gly Arg Lys Phe Phe Val Gly Gly Asn Trp Lys Cys Asn Gly Thr
1               5                   10                  15

act gag gag gta aag aag att gtt act act ttg aat gag gct aaa gtc          96

Thr Glu Glu Val Lys Lys Ile Val Thr Thr Leu Asn Glu Ala Lys Val
            20                  25                  30

cct gga gaa gat gtc gta gaa gtt gtt gtg agc cct cct ttt gtg ttc 144
Pro Gly Glu Asp Val Val Glu Val Val Val Ser Pro Pro Phe Val Phe
        35                  40                  45

ctt cct gtt gta aaa agt ttg ctg cgc cct gat ttc cat gtt tcg gca 192
Leu Pro Val Val Lys Ser Leu Leu Arg Pro Asp Phe His Val Ser Ala
    50                  55                  60

caa aac tgt tgg gtt cgc aaa ggt ggt gct tat acc ggt gag gtt agt 240
Gln Asn Cys Trp Val Arg Lys Gly Gly Ala Tyr Thr Gly Glu Val Ser
65                  70                  75                  80

gct gaa atg ctt gtt aat ttg gga att cct tgg gtt att att ggt cac 288
Ala Glu Met Leu Val Asn Leu Gly Ile Pro Trp Val Ile Ile Gly His
                85                  90                  95

tct gaa cgg agg cag ctt tta aat gaa tca aat gag ttt gtg gga gat 336
Ser Glu Arg Arg Gln Leu Leu Asn Glu Ser Asn Glu Phe Val Gly Asp
            100                 105                 110

aaa gtt gcc tat gca ctt caa caa ggt cta aaa gtt att gca tgc att 384
Lys Val Ala Tyr Ala Leu Gln Gln Gly Leu Lys Val Ile Ala Cys Ile
        115                 120                 125

ggg gag act ctc gaa cag cgt gaa gct ggt aca aca acg gct gtt gtt 432
Gly Glu Thr Leu Glu Gln Arg Glu Ala Gly Thr Thr Thr Ala Val Val
    130                 135                 140

tct gag caa aca aaa gca att gca gct aaa ata tca aat tgg gac aat 480
Ser Glu Gln Thr Lys Ala Ile Ala Ala Lys Ile Ser Asn Trp Asp Asn
145                 150                 155                 160

gtc gtt ttg gcc tac gag cca gtt tgg gcc att gga aca gga aag gtt 528
Val Val Leu Ala Tyr Glu Pro Val Trp Ala Ile Gly Thr Gly Lys Val
                165                 170                 175

gct act cct gct cag gct caa gag gtc cat gct gat ttg agg aaa tgg 576
Ala Thr Pro Ala Gln Ala Gln Glu Val His Ala Asp Leu Arg Lys Trp
            180                 185                 190

gtt cat gac aat gtg agt gct gaa gtt gct gca tct gta aga att atc 624
Val His Asp Asn Val Ser Ala Glu Val Ala Ala Ser Val Arg Ile Ile
        195                 200                 205

tat gga ggt tct gta aat gga gga aac tgc aaa gaa ttg gcc gca cag 672
Tyr Gly Gly Ser Val Asn Gly Gly Asn Cys Lys Glu Leu Ala Ala Gln
    210                 215                 220

ccc gat gtt gat gga ttt ttg gtt ggt ggt gcc tcc ctg aag ccg gag 720
Pro Asp Val Asp Gly Phe Leu Val Gly Gly Ala Ser Leu Lys Pro Glu
225                 230                 235                 240

ttc gtg gac atc ata aat gct gcc act gtg aag aag aat 759
Phe Val Asp Ile Ile Asn Ala Ala Thr Val Lys Lys Asn
                245                 250

<210> 599
<211> 253
<212> PRT

<213>  Glycine max

<400>  599

Met Gly Arg Lys Phe Phe Val Gly Gly Asn Trp Lys Cys Asn Gly Thr
1               5                   10                  15

Thr Glu Glu Val Lys Lys Ile Val Thr Thr Leu Asn Glu Ala Lys Val
            20                  25                  30

Pro Gly Glu Asp Val Val Glu Val Val Val Ser Pro Pro Phe Val Phe
        35                  40                  45

Leu Pro Val Val Lys Ser Leu Leu Arg Pro Asp Phe His Val Ser Ala
    50                  55                  60

Gln Asn Cys Trp Val Arg Lys Gly Gly Ala Tyr Thr Gly Glu Val Ser
65                  70                  75                  80

Ala Glu Met Leu Val Asn Leu Gly Ile Pro Trp Val Ile Ile Gly His
                85                  90                  95

Ser Glu Arg Arg Gln Leu Leu Asn Glu Ser Asn Glu Phe Val Gly Asp
            100                 105                 110

Lys Val Ala Tyr Ala Leu Gln Gln Gly Leu Lys Val Ile Ala Cys Ile
        115                 120                 125

Gly Glu Thr Leu Glu Gln Arg Glu Ala Gly Thr Thr Thr Ala Val Val
    130                 135                 140

Ser Glu Gln Thr Lys Ala Ile Ala Ala Lys Ile Ser Asn Trp Asp Asn
145                 150                 155                 160

Val Val Leu Ala Tyr Glu Pro Val Trp Ala Ile Gly Thr Gly Lys Val
                165                 170                 175

Ala Thr Pro Ala Gln Ala Gln Glu Val His Ala Asp Leu Arg Lys Trp
            180                 185                 190

Val His Asp Asn Val Ser Ala Glu Val Ala Ala Ser Val Arg Ile Ile
        195                 200                 205

Tyr Gly Gly Ser Val Asn Gly Gly Asn Cys Lys Glu Leu Ala Ala Gln
    210                 215                 220

Pro Asp Val Asp Gly Phe Leu Val Gly Gly Ala Ser Leu Lys Pro Glu
225                 230                 235                 240

Phe Val Asp Ile Ile Asn Ala Ala Thr Val Lys Lys Asn
                245                 250

<210>  600
<211>  32
<212>  PRT
<213>  Glycine max

<400>  600

Glu Leu Ala Ala Gln Pro Asp Val Asp Gly Phe Leu Val Gly Gly Ala
1               5                   10                  15

Ser Leu Lys Pro Glu Phe Val Asp Ile Ile Asn Ala Ala Thr Val Lys
            20                  25                  30

<210>  601
<211>  8
<212>  PRT
<213>  Glycine max

<400>  601

Phe Phe Val Gly Gly Asn Trp Lys
1               5

<210>  602
<211>  9
<212>  PRT
<213>  Glycine max

<400>  602

Ile Val Thr Thr Leu Asn Glu Ala Lys
1               5

<210>  603
<211>  16
<212>  PRT
<213>  Glycine max

<400>  603

Val Ala Thr Pro Ala Gln Ala Gln Glu Val His Ala Asp Leu Arg Lys
1               5                   10                  15

<210>  604
<211>  10
<212>  PRT
<213>  Glycine max

<400>  604

Val Ala Tyr Ala Leu Gln Gln Gly Leu Lys
1               5                   10

<210>  605
<211>  765
<212>  DNA
<213>  Glycine max

<220>
<221>  CDS
<222>  (1)..(765)

<400>  605
atg gcc tca ttg ggt ggg tta caa aat gtg agc ggc atc aat ttt ctt      48
Met Ala Ser Leu Gly Gly Leu Gln Asn Val Ser Gly Ile Asn Phe Leu
1               5                   10                  15

atc aaa gag aaa gga tgt tgt atg ctg cag ggt cca aaa gtc aat gca      96
Ile Lys Glu Lys Gly Cys Cys Met Leu Gln Gly Pro Lys Val Asn Ala
            20                  25                  30

aag ttc gag ctg aag ccg cca cca tat cca ctg aat ggt ttg gag ccg      144
Lys Phe Glu Leu Lys Pro Pro Pro Tyr Pro Leu Asn Gly Leu Glu Pro
        35                  40                  45

gtg atg agc cag cag aca ctt gag ttt cac tgg ggg aag cac cac aag      192
Val Met Ser Gln Gln Thr Leu Glu Phe His Trp Gly Lys His His Lys
    50                  55                  60

act tat gtg gaa aat ctg aaa aaa caa gtt gtt ggg aca gag ctt gat      240
Thr Tyr Val Glu Asn Leu Lys Lys Gln Val Val Gly Thr Glu Leu Asp
65                  70                  75                  80

ggg aag tca cta gaa gag att att gtc aca tca tac aat aag ggt gac      288
Gly Lys Ser Leu Glu Glu Ile Ile Val Thr Ser Tyr Asn Lys Gly Asp
                85                  90                  95

att ctt cca gct ttc aac aat gca gca cag gta tgg aac cat gac ttc      336
Ile Leu Pro Ala Phe Asn Asn Ala Ala Gln Val Trp Asn His Asp Phe
            100                 105                 110

ttc tgg gag tgc atg aaa cca ggt gga ggt gga aag cca tcc ggg gag      384
Phe Trp Glu Cys Met Lys Pro Gly Gly Gly Gly Lys Pro Ser Gly Glu
        115                 120                 125

ctt cta gaa ctg att gaa aga gac ttt ggt tca ttt gta aaa ttc ctt      432
Leu Leu Glu Leu Ile Glu Arg Asp Phe Gly Ser Phe Val Lys Phe Leu
    130                 135                 140

gat gag ttc aag gct gct gct gca aca caa ttt ggt tca ggg tgg gct      480
Asp Glu Phe Lys Ala Ala Ala Ala Thr Gln Phe Gly Ser Gly Trp Ala
145                 150                 155                 160

tgg cta gca tat aga gca aga aaa ttt gat ggg gaa aat gta gca aat      528
Trp Leu Ala Tyr Arg Ala Arg Lys Phe Asp Gly Glu Asn Val Ala Asn
                165                 170                 175

cct cct tca ccc gat gag gac aac aag cta gtg gtg ctc aag agt ccc      576
Pro Pro Ser Pro Asp Glu Asp Asn Lys Leu Val Val Leu Lys Ser Pro
            180                 185                 190

aat gct gtg aac ccc ctt gtt tgg gga ggt tac tac cca ctt ctt acc      624
Asn Ala Val Asn Pro Leu Val Trp Gly Gly Tyr Tyr Pro Leu Leu Thr
        195                 200                 205

att gat gtt tgg gag cat gct tac tac ctt gat ttt cag aac cgg cgt 672
Ile Asp Val Trp Glu His Ala Tyr Tyr Leu Asp Phe Gln Asn Arg Arg
    210                 215                 220

cct gat tat ata tca gtg ttc atg gat aag ctt gtt tcc tgg gat gca 720
Pro Asp Tyr Ile Ser Val Phe Met Asp Lys Leu Val Ser Trp Asp Ala
225                 230                 235                 240

gtg agc tct aga ctt gaa caa gct aag gct tta att acc agt gca 765
Val Ser Ser Arg Leu Glu Gln Ala Lys Ala Leu Ile Thr Ser Ala
                245                 250                 255

<210> 606
<211> 255
<212> PRT
<213> Glycine max

<400> 606

Met Ala Ser Leu Gly Gly Leu Gln Asn Val Ser Gly Ile Asn Phe Leu
1               5                   10                  15

Ile Lys Glu Lys Gly Cys Cys Met Leu Gln Gly Pro Lys Val Asn Ala
            20                  25                  30

Lys Phe Glu Leu Lys Pro Pro Pro Tyr Pro Leu Asn Gly Leu Glu Pro
        35                  40                  45

Val Met Ser Gln Gln Thr Leu Glu Phe His Trp Gly Lys His His Lys
    50                  55                  60

Thr Tyr Val Glu Asn Leu Lys Lys Gln Val Val Gly Thr Glu Leu Asp
65                  70                  75                  80

Gly Lys Ser Leu Glu Glu Ile Ile Val Thr Ser Tyr Asn Lys Gly Asp
                85                  90                  95

Ile Leu Pro Ala Phe Asn Asn Ala Ala Gln Val Trp Asn His Asp Phe
            100                 105                 110

Phe Trp Glu Cys Met Lys Pro Gly Gly Gly Gly Lys Pro Ser Gly Glu
        115                 120                 125

Leu Leu Glu Leu Ile Glu Arg Asp Phe Gly Ser Phe Val Lys Phe Leu
    130                 135                 140

Asp Glu Phe Lys Ala Ala Ala Ala Thr Gln Phe Gly Ser Gly Trp Ala
145                 150                 155                 160

Trp Leu Ala Tyr Arg Ala Arg Lys Phe Asp Gly Glu Asn Val Ala Asn
                165                 170                 175

Pro Pro Ser Pro Asp Glu Asp Asn Lys Leu Val Val Leu Lys Ser Pro
            180                 185                 190

Asn Ala Val Asn Pro Leu Val Trp Gly Gly Tyr Tyr Pro Leu Leu Thr
        195                 200                 205

Ile Asp Val Trp Glu His Ala Tyr Tyr Leu Asp Phe Gln Asn Arg Arg
    210                 215                 220

Pro Asp Tyr Ile Ser Val Phe Met Asp Lys Leu Val Ser Trp Asp Ala
225                 230                 235                 240

Val Ser Ser Arg Leu Glu Gln Ala Lys Ala Leu Ile Thr Ser Ala
                245                 250                 255

<210>  607
<211>  17
<212>  PRT
<213>  Glycine max

<400>  607

Phe Asp Gly Glu Asn Val Ala Asn Pro Pro Ser Pro Asp Glu Asp Asn
1               5                   10                  15

Lys

<210>  608
<211>  15
<212>  PRT
<213>  Glycine max

<400>  608

Leu Val Ser Trp Asp Ala Val Ser Ser Arg Leu Glu Gln Ala Lys
1               5                   10                  15

<210>  609
<211>  10
<212>  PRT
<213>  Glycine max

<400>  609

Gln Val Val Gly Thr Glu Leu Asp Gly Lys
1               5                   10

<210>  610
<211>  12
<212>  PRT
<213>  Glycine max

<400>  610

Ser Leu Glu Glu Ile Ile Val Thr Ser Tyr Asn Lys
1               5                   10


<210>  611
<211>  7
<212>  PRT
<213>  Glycine max

<400>  611

Thr Tyr Val Glu Asn Leu Lys
1               5


<210>  612
<211>  732
<212>  DNA
<213>  Glycine max


<220>
<221>  CDS
<222>  (1)..(732)

<400>  612
atg gcc tca ttg ggt ggg tta caa aat cta agc ggc atc aat ttt ctt          48
Met Ala Ser Leu Gly Gly Leu Gln Asn Leu Ser Gly Ile Asn Phe Leu
1               5                   10                  15

atc aaa aaa gag ggt cca aaa gtc aat gcg aag ttc gag ctg aag ccg          96
Ile Lys Lys Glu Gly Pro Lys Val Asn Ala Lys Phe Glu Leu Lys Pro
            20                  25                  30

cca cca tat cca ctg aat ggt ttg gag ccg gtg atg agc cag cag aca         144
Pro Pro Tyr Pro Leu Asn Gly Leu Glu Pro Val Met Ser Gln Gln Thr
        35                  40                  45

ctt gag ttt cat tgg ggg aaa cac cac aag aca tat gtg gaa aat ctg         192
Leu Glu Phe His Trp Gly Lys His His Lys Thr Tyr Val Glu Asn Leu
    50                  55                  60

aaa aaa caa att gtt ggg aca gag ctt gat ggg aaa tca cta gaa gag         240
Lys Lys Gln Ile Val Gly Thr Glu Leu Asp Gly Lys Ser Leu Glu Glu
65                  70                  75                  80

att ata gtg aca tca tat aat aag ggt gac att ctt cca gct ttc aac         288
Ile Ile Val Thr Ser Tyr Asn Lys Gly Asp Ile Leu Pro Ala Phe Asn
                85                  90                  95

aat gca gca cag gta tgg aac cat gac ttc ttc tgg gag tgc atg aaa         336
Asn Ala Ala Gln Val Trp Asn His Asp Phe Phe Trp Glu Cys Met Lys
            100                 105                 110

cca ggt gga ggt gga aag cca tcc ggg gag ctt cta gaa ctg att gaa         384
Pro Gly Gly Gly Gly Lys Pro Ser Gly Glu Leu Leu Glu Leu Ile Glu
        115                 120                 125

aga gac ttt ggc tca ttt gaa aaa ttc ctt gat gag ttt aag gct gct         432
Arg Asp Phe Gly Ser Phe Glu Lys Phe Leu Asp Glu Phe Lys Ala Ala

130                 135                 140

gct gca aca caa ttt ggt tca gga tgg gct tgg ctt gca tat aga gca        480
Ala Ala Thr Gln Phe Gly Ser Gly Trp Ala Trp Leu Ala Tyr Arg Ala
145                 150                 155                 160

agc aaa ttt gat ggg gaa aat gca gca aat cct cct tca cca gat gag        528
Ser Lys Phe Asp Gly Glu Asn Ala Ala Asn Pro Pro Ser Pro Asp Glu
                165                 170                 175

gac aac aag cta gtg gtg ctc aag agt ccc aat gct gtg aac ccc ctt        576
Asp Asn Lys Leu Val Val Leu Lys Ser Pro Asn Ala Val Asn Pro Leu
            180                 185                 190

gtt tgg gga ggc tat tat cca ctt ctt acc att gat gtt tgg gag cat        624
Val Trp Gly Gly Tyr Tyr Pro Leu Leu Thr Ile Asp Val Trp Glu His
        195                 200                 205

gct tac aac ctt gat ttt cag aac cgg cgc cct gat tat ata tca gtg        672
Ala Tyr Asn Leu Asp Phe Gln Asn Arg Arg Pro Asp Tyr Ile Ser Val
    210                 215                 220

ttc atg gat aag ctt gtt tcc tgg gat gca gtg agt tct aga ctt gaa        720
Phe Met Asp Lys Leu Val Ser Trp Asp Ala Val Ser Ser Arg Leu Glu
225                 230                 235                 240

caa gca agg ctt                                                        732
Gln Ala Arg Leu

<210>  613
<211>  244
<212>  PRT
<213>  Glycine max

<400>  613

Met Ala Ser Leu Gly Gly Leu Gln Asn Leu Ser Gly Ile Asn Phe Leu
1               5                   10                  15

Ile Lys Lys Glu Gly Pro Lys Val Asn Ala Lys Phe Glu Leu Lys Pro
            20                  25                  30

Pro Pro Tyr Pro Leu Asn Gly Leu Glu Pro Val Met Ser Gln Gln Thr
        35                  40                  45

Leu Glu Phe His Trp Gly Lys His His Lys Thr Tyr Val Glu Asn Leu
    50                  55                  60

Lys Lys Gln Ile Val Gly Thr Glu Leu Asp Gly Lys Ser Leu Glu Glu
65                  70                  75                  80

Ile Ile Val Thr Ser Tyr Asn Lys Gly Asp Ile Leu Pro Ala Phe Asn
                85                  90                  95

Asn Ala Ala Gln Val Trp Asn His Asp Phe Phe Trp Glu Cys Met Lys

            100                 105                 110

Pro Gly Gly Gly Gly Lys Pro Ser Gly Glu Leu Leu Glu Leu Ile Glu
        115                 120                 125

Arg Asp Phe Gly Ser Phe Glu Lys Phe Leu Asp Glu Phe Lys Ala Ala
    130                 135                 140

Ala Ala Thr Gln Phe Gly Ser Gly Trp Ala Trp Leu Ala Tyr Arg Ala
145                 150                 155                 160

Ser Lys Phe Asp Gly Glu Asn Ala Ala Asn Pro Pro Ser Pro Asp Glu
                165                 170                 175

Asp Asn Lys Leu Val Val Leu Lys Ser Pro Asn Ala Val Asn Pro Leu
            180                 185                 190

Val Trp Gly Gly Tyr Tyr Pro Leu Leu Thr Ile Asp Val Trp Glu His
        195                 200                 205

Ala Tyr Asn Leu Asp Phe Gln Asn Arg Arg Pro Asp Tyr Ile Ser Val
    210                 215                 220

Phe Met Asp Lys Leu Val Ser Trp Asp Ala Val Ser Ser Arg Leu Glu
225                 230                 235                 240

Gln Ala Arg Leu

<210> 614
<211> 10
<212> PRT
<213> Glycine max

<400> 614

Gln Ile Val Gly Thr Glu Leu Asp Gly Lys
1               5                   10

<210> 615
<211> 12
<212> PRT
<213> Glycine max

<400> 615

Ser Leu Glu Glu Ile Ile Val Thr Ser Tyr Asn Lys
1               5                   10

<210> 616
<211> 7

<212>  PRT
<213>  Glycine max

<400>  616

Thr Tyr Val Glu Asn Leu Lys
1               5


<210>  617
<211>  1419
<212>  DNA
<213>  Glycine max


<220>
<221>  CDS
<222>  (1)..(1419)

<400>  617
atg gct tct aag gtg gtt tcg gtt ttg gtt ata gcg atg atg ttg ttt      48
Met Ala Ser Lys Val Val Ser Val Leu Val Ile Ala Met Met Leu Phe
1               5                   10                  15

gcc atg aac tgt aac tgc act agt gtt ggc cac atg cct tca acc aaa      96
Ala Met Asn Cys Asn Cys Thr Ser Val Gly His Met Pro Ser Thr Lys
            20                  25                  30

gag gaa ggt cac gat ttt cag gag tct aaa gcc aag acc aca caa act      144
Glu Glu Gly His Asp Phe Gln Glu Ser Lys Ala Lys Thr Thr Gln Thr
        35                  40                  45

gcg aac aag gcc atg gaa act ggt aaa gag gga caa gaa gcc gca gag      192
Ala Asn Lys Ala Met Glu Thr Gly Lys Glu Gly Gln Glu Ala Ala Glu
    50                  55                  60

tca tgg aca gaa tgg gct aaa gag aaa ctc agt gaa ggt ctt ggc ttc      240
Ser Trp Thr Glu Trp Ala Lys Glu Lys Leu Ser Glu Gly Leu Gly Phe
65                  70                  75                  80

aaa cat gat cag gaa tcc aaa gaa tcc acc act aac aaa gtc tct gat      288
Lys His Asp Gln Glu Ser Lys Glu Ser Thr Thr Asn Lys Val Ser Asp
                85                  90                  95

tat gcc act gac act gca cag aaa tcc aag gat tat gca act gac act      336
Tyr Ala Thr Asp Thr Ala Gln Lys Ser Lys Asp Tyr Ala Thr Asp Thr
            100                 105                 110

gct cag aaa tca aag gac tat gca ggt gat gct gct cag aaa tca aag      384
Ala Gln Lys Ser Lys Asp Tyr Ala Gly Asp Ala Ala Gln Lys Ser Lys
        115                 120                 125

gac tac gca ggt gat gct gct cag aaa acc aaa gac tat gcc agt gac      432
Asp Tyr Ala Gly Asp Ala Ala Gln Lys Thr Lys Asp Tyr Ala Ser Asp
    130                 135                 140

act gca cag aca tca aag gac tat gct ggt gat gct gct cag aaa tca      480
Thr Ala Gln Thr Ser Lys Asp Tyr Ala Gly Asp Ala Ala Gln Lys Ser
145                 150                 155                 160

aag ggc tat gtt ggt gat gct gca caa aag acc aaa gaa tat gtt ggt      528
Lys Gly Tyr Val Gly Asp Ala Ala Gln Lys Thr Lys Glu Tyr Val Gly
                165                 170                 175

gat gca gca cag aag aca aag gat tat gcc act gac gca gca cag aag 576
Asp Ala Ala Gln Lys Thr Lys Asp Tyr Ala Thr Asp Ala Ala Gln Lys
            180                 185                 190

acc aaa gat tat gct act cag aag acc aag gac tat gca agt gat gca 624
Thr Lys Asp Tyr Ala Thr Gln Lys Thr Lys Asp Tyr Ala Ser Asp Ala
        195                 200                 205

act gat gct gca aag aag act aaa gat tat gct gct cag aag act aag 672
Thr Asp Ala Ala Lys Lys Thr Lys Asp Tyr Ala Ala Gln Lys Thr Lys
    210                 215                 220

gac tat gca agt gaa gct agt gat gtt gca cag aac aca aaa gat tat 720
Asp Tyr Ala Ser Glu Ala Ser Asp Val Ala Gln Asn Thr Lys Asp Tyr
225                 230                 235                 240

gct gct cag aag acc aag gac tat gcc agc ggt ggt gcg cag aag acc 768
Ala Ala Gln Lys Thr Lys Asp Tyr Ala Ser Gly Gly Ala Gln Lys Thr
                245                 250                 255

aag gat tat gcc agc ggt ggt gcg cag aag acc aag gac tat gca agt 816
Lys Asp Tyr Ala Ser Gly Gly Ala Gln Lys Thr Lys Asp Tyr Ala Ser
            260                 265                 270

gat gct gcg cag aag acc aag gac tat gca agt gat ggt gct caa aag 864
Asp Ala Ala Gln Lys Thr Lys Asp Tyr Ala Ser Asp Gly Ala Gln Lys
        275                 280                 285

agc aaa gaa tat gcg ggt gat gta gca ctg aat gcc aaa gat tat gct 912
Ser Lys Glu Tyr Ala Gly Asp Val Ala Leu Asn Ala Lys Asp Tyr Ala
    290                 295                 300

caa aaa agc aaa gat tac gca ggt gat gct gca cag aat gtc aaa gat 960
Gln Lys Ser Lys Asp Tyr Ala Gly Asp Ala Ala Gln Asn Val Lys Asp
305                 310                 315                 320

tat gca agt gat gca gtg cag aag aga aaa gag tat tct ggt gat gct 1008
Tyr Ala Ser Asp Ala Val Gln Lys Arg Lys Glu Tyr Ser Gly Asp Ala
                325                 330                 335

tct cac aag agc aaa gag gct tct gat tat gcc agt gag acg gct aag 1056
Ser His Lys Ser Lys Glu Ala Ser Asp Tyr Ala Ser Glu Thr Ala Lys
            340                 345                 350

aag acc aaa gat tat gtc ggt gat gct gct cag aga agc aaa ggg gct 1104
Lys Thr Lys Asp Tyr Val Gly Asp Ala Ala Gln Arg Ser Lys Gly Ala
        355                 360                 365

gct gaa tat gct agt gat gcg gct cag agg acc aaa gag tat gct ggt 1152
Ala Glu Tyr Ala Ser Asp Ala Ala Gln Arg Thr Lys Glu Tyr Ala Gly
    370                 375                 380

gat gct acc aag aga agc aaa gag gct tct aat gat cat gcc aat gac 1200
Asp Ala Thr Lys Arg Ser Lys Glu Ala Ser Asn Asp His Ala Asn Asp
385                 390                 395                 400

atg gct cag aag acc aaa gac tac gcc agt gac act gct cag aga acc 1248
Met Ala Gln Lys Thr Lys Asp Tyr Ala Ser Asp Thr Ala Gln Arg Thr
                405                 410                 415

aag gaa aaa ctc caa gac att gca tct gaa gct ggt caa tac agt gca 1296
Lys Glu Lys Leu Gln Asp Ile Ala Ser Glu Ala Gly Gln Tyr Ser Ala

420                 425                 430

gag aaa gct aga gaa atg aag gat gca gca gca gag aag gca agt gac        1344
Glu Lys Ala Arg Glu Met Lys Asp Ala Ala Ala Glu Lys Ala Ser Asp
        435                 440                 445

ata gca aag gct gca aag caa aaa tca caa gag gtg aaa gag aaa tta        1392
Ile Ala Lys Ala Ala Lys Gln Lys Ser Gln Glu Val Lys Glu Lys Leu
    450                 455                 460

ggt gga caa cac cgg gat gcc gag ctt                                    1419
Gly Gly Gln His Arg Asp Ala Glu Leu
465                 470

<210>  618
<211>  473
<212>  PRT
<213>  Glycine max

<400>  618

Met Ala Ser Lys Val Val Ser Val Leu Val Ile Ala Met Met Leu Phe
1               5                   10                  15

Ala Met Asn Cys Asn Cys Thr Ser Val Gly His Met Pro Ser Thr Lys
            20                  25                  30

Glu Glu Gly His Asp Phe Gln Glu Ser Lys Ala Lys Thr Thr Gln Thr
        35                  40                  45

Ala Asn Lys Ala Met Glu Thr Gly Lys Glu Gly Gln Glu Ala Ala Glu
    50                  55                  60

Ser Trp Thr Glu Trp Ala Lys Glu Lys Leu Ser Glu Gly Leu Gly Phe
65                  70                  75                  80

Lys His Asp Gln Glu Ser Lys Glu Ser Thr Thr Asn Lys Val Ser Asp
                85                  90                  95

Tyr Ala Thr Asp Thr Ala Gln Lys Ser Lys Asp Tyr Ala Thr Asp Thr
            100                 105                 110

Ala Gln Lys Ser Lys Asp Tyr Ala Gly Asp Ala Ala Gln Lys Ser Lys
        115                 120                 125

Asp Tyr Ala Gly Asp Ala Ala Gln Lys Thr Lys Asp Tyr Ala Ser Asp
    130                 135                 140

Thr Ala Gln Thr Ser Lys Asp Tyr Ala Gly Asp Ala Ala Gln Lys Ser
145                 150                 155                 160

Lys Gly Tyr Val Gly Asp Ala Ala Gln Lys Thr Lys Glu Tyr Val Gly

                165                 170                 175

Asp Ala Ala Gln Lys Thr Lys Asp Tyr Ala Thr Asp Ala Ala Gln Lys
            180                 185                 190

Thr Lys Asp Tyr Ala Thr Gln Lys Thr Lys Asp Tyr Ala Ser Asp Ala
        195                 200                 205

Thr Asp Ala Ala Lys Lys Thr Lys Asp Tyr Ala Ala Gln Lys Thr Lys
    210                 215                 220

Asp Tyr Ala Ser Glu Ala Ser Asp Val Ala Gln Asn Thr Lys Asp Tyr
225                 230                 235                 240

Ala Ala Gln Lys Thr Lys Asp Tyr Ala Ser Gly Gly Ala Gln Lys Thr
                245                 250                 255

Lys Asp Tyr Ala Ser Gly Gly Ala Gln Lys Thr Lys Asp Tyr Ala Ser
            260                 265                 270

Asp Ala Ala Gln Lys Thr Lys Asp Tyr Ala Ser Asp Gly Ala Gln Lys
        275                 280                 285

Ser Lys Glu Tyr Ala Gly Asp Val Ala Leu Asn Ala Lys Asp Tyr Ala
    290                 295                 300

Gln Lys Ser Lys Asp Tyr Ala Gly Asp Ala Ala Gln Asn Val Lys Asp
305                 310                 315                 320

Tyr Ala Ser Asp Ala Val Gln Lys Arg Lys Glu Tyr Ser Gly Asp Ala
                325                 330                 335

Ser His Lys Ser Lys Glu Ala Ser Asp Tyr Ala Ser Glu Thr Ala Lys
            340                 345                 350

Lys Thr Lys Asp Tyr Val Gly Asp Ala Ala Gln Arg Ser Lys Gly Ala
        355                 360                 365

Ala Glu Tyr Ala Ser Asp Ala Ala Gln Arg Thr Lys Glu Tyr Ala Gly
    370                 375                 380

Asp Ala Thr Lys Arg Ser Lys Glu Ala Ser Asn Asp His Ala Asn Asp
385                 390                 395                 400

Met Ala Gln Lys Thr Lys Asp Tyr Ala Ser Asp Thr Ala Gln Arg Thr
                405                 410                 415

Lys Glu Lys Leu Gln Asp Ile Ala Ser Glu Ala Gly Gln Tyr Ser Ala
            420                 425                 430

Glu Lys Ala Arg Glu Met Lys Asp Ala Ala Ala Glu Lys Ala Ser Asp
        435                 440                 445

Ile Ala Lys Ala Ala Lys Gln Lys Ser Gln Glu Val Lys Glu Lys Leu
    450                 455                 460

Gly Gly Gln His Arg Asp Ala Glu Leu
465                 470

<210>  619
<211>  11
<212>  PRT
<213>  Glycine max

<400>  619

Asp Tyr Ala Gly Asp Ala Ala Gln Asn Val Lys
1               5                   10

<210>  620
<211>  9
<212>  PRT
<213>  Glycine max

<400>  620

Asp Tyr Ala Ser Asp Ala Ala Gln Lys
1               5

<210>  621
<211>  11
<212>  PRT
<213>  Glycine max

<400>  621

Asp Tyr Ala Ser Asp Ala Thr Asp Ala Ala Lys
1               5                   10

<210>  622
<211>  9
<212>  PRT
<213>  Glycine max

<400>  622

Asp Tyr Ala Ser Asp Ala Val Gln Lys
1               5

<210>  623
<211>  11
<212>  PRT

<213>  Glycine max

<400>  623

Asp Tyr Ala Ser Asp Thr Ala Gln Arg Thr Lys
1               5                   10


<210>  624
<211>  11
<212>  PRT
<213>  Glycine max

<400>  624

Asp Tyr Ala Ser Asp Thr Ala Gln Thr Ser Lys
1               5                   10


<210>  625
<211>  14
<212>  PRT
<213>  Glycine max

<400>  625

Asp Tyr Ala Ser Glu Ala Ser Asp Val Ala Gln Asn Thr Lys
1               5                   10


<210>  626
<211>  9
<212>  PRT
<213>  Glycine max

<400>  626

Asp Tyr Ala Thr Asp Ala Ala Gln Lys
1               5


<210>  627
<211>  11
<212>  PRT
<213>  Glycine max

<400>  627

Asp Tyr Val Gly Asp Ala Ala Gln Arg Ser Lys
1               5                   10


<210>  628
<211>  11
<212>  PRT
<213>  Glycine max

<400>  628

Glu Ala Ser Asp Tyr Ala Ser Glu Thr Ala Lys
1               5                   10

<210>  629
<211>  11
<212>  PRT
<213>  Glycine max

<400>  629

Glu Tyr Ala Gly Asp Val Ala Leu Asn Ala Lys
1               5                   10


<210>  630
<211>  9
<212>  PRT
<213>  Glycine max

<400>  630

Glu Tyr Val Gly Asp Ala Ala Gln Lys
1               5


<210>  631
<211>  14
<212>  PRT
<213>  Glycine max

<400>  631

Gly Ala Ala Glu Tyr Ala Ser Asp Ala Ala Gln Arg Thr Lys
1               5                   10


<210>  632
<211>  9
<212>  PRT
<213>  Glycine max

<400>  632

Gly Tyr Val Gly Asp Ala Ala Gln Lys
1               5


<210>  633
<211>  15
<212>  PRT
<213>  Glycine max

<400>  633

Leu Gln Asp Ile Ala Ser Glu Ala Gly Gln Tyr Ser Ala Glu Lys
1               5                   10                  15


<210>  634
<211>  8
<212>  PRT
<213>  Glycine max

<400>  634

Leu Ser Glu Gly Leu Gly Phe Lys

1               5


<210>  635
<211>  11
<212>  PRT
<213>  Glycine max

<400>  635

Val Ser Asp Tyr Ala Thr Asp Thr Ala Gln Lys
1               5                   10


<210>  636
<211>  1374
<212>  DNA
<213>  Glycine max


<220>
<221>  CDS
<222>  (1)..(1374)

<400>  636
atg gct tcg agg gtg gtt tcg gtt ttg gtg ata gcg atg atg ttg ttt      48
Met Ala Ser Arg Val Val Ser Val Leu Val Ile Ala Met Met Leu Phe
1               5                   10                  15

gcc atg aac tgt aac tgc act agt gtt ggc cac atg cct tca acc aaa      96
Ala Met Asn Cys Asn Cys Thr Ser Val Gly His Met Pro Ser Thr Lys
            20                  25                  30

gag gaa ggt cac gat ttt cag gag gct aaa gcc aag aca aca caa act      144
Glu Glu Gly His Asp Phe Gln Glu Ala Lys Ala Lys Thr Thr Gln Thr
        35                  40                  45

gcg aac aag gcc atg gaa act ggt aaa gat ggg aaa gaa gcc gca gag      192
Ala Asn Lys Ala Met Glu Thr Gly Lys Asp Gly Lys Glu Ala Ala Glu
    50                  55                  60

tca tgg aca gaa tgg gct aaa gag aaa ctc act gaa ggt ctt ggc ttc      240
Ser Trp Thr Glu Trp Ala Lys Glu Lys Leu Thr Glu Gly Leu Gly Phe
65                  70                  75                  80

aaa cat gat caa gaa tcc acc act aag aaa gtc tct gac tat gcc ggt      288
Lys His Asp Gln Glu Ser Thr Thr Lys Lys Val Ser Asp Tyr Ala Gly
                85                  90                  95

gat gct gct cag aaa tca aag gac tat gcc ggt gac act gct cag aaa      336
Asp Ala Ala Gln Lys Ser Lys Asp Tyr Ala Gly Asp Thr Ala Gln Lys
            100                 105                 110

acc aag gac tat gcc ggt gac act gct cag aaa acc aag gac tat gcc      384
Thr Lys Asp Tyr Ala Gly Asp Thr Ala Gln Lys Thr Lys Asp Tyr Ala
        115                 120                 125

ggt gac act gca cag aca tca aag gac tat gcc ggt gat gct gct cag      432
Gly Asp Thr Ala Gln Thr Ser Lys Asp Tyr Ala Gly Asp Ala Ala Gln
    130                 135                 140

aaa tca aag ggc tat gtc ggt gat gct gca caa aag acc aaa gaa tat      480
Lys Ser Lys Gly Tyr Val Gly Asp Ala Ala Gln Lys Thr Lys Glu Tyr

145                 150                 155                 160

gtt ggt gat gct gct cag aag acc atg gat tat gcc act gat gca gca      528
Val Gly Asp Ala Ala Gln Lys Thr Met Asp Tyr Ala Thr Asp Ala Ala
                165                 170                 175

cag aag acc aaa gat tat gct act cag aag act aag gac tat gca agt      576
Gln Lys Thr Lys Asp Tyr Ala Thr Gln Lys Thr Lys Asp Tyr Ala Ser
            180                 185                 190

gat gca act gat gct gca aaa aag act aaa gat tat gct gct cag aag      624
Asp Ala Thr Asp Ala Ala Lys Lys Thr Lys Asp Tyr Ala Ala Gln Lys
        195                 200                 205

acc aag gat tat gca agt gat gca act gat gct gca aaa aag act aag      672
Thr Lys Asp Tyr Ala Ser Asp Ala Thr Asp Ala Ala Lys Lys Thr Lys
    210                 215                 220

gat tat gct gcg cag aag acc aag gac tat gcc agt ggt ggt gcg cag      720
Asp Tyr Ala Ala Gln Lys Thr Lys Asp Tyr Ala Ser Gly Gly Ala Gln
225                 230                 235                 240

aag acc aag gac tat gcc agt ggt gct gcg cag aag acc aag gac tat      768
Lys Thr Lys Asp Tyr Ala Ser Gly Ala Ala Gln Lys Thr Lys Asp Tyr
                245                 250                 255

gca agt gat gct gcg cag agg acc aag gac cat gca agt gat ggt gct      816
Ala Ser Asp Ala Ala Gln Arg Thr Lys Asp His Ala Ser Asp Gly Ala
            260                 265                 270

caa aag agc aaa gaa tat gcg ggt gat gta gca cag aat gcc aaa gat      864
Gln Lys Ser Lys Glu Tyr Ala Gly Asp Val Ala Gln Asn Ala Lys Asp
        275                 280                 285

tat gct caa aag agc aaa gat tat gcg ggt gat gct gta cag aat atc      912
Tyr Ala Gln Lys Ser Lys Asp Tyr Ala Gly Asp Ala Val Gln Asn Ile
    290                 295                 300

aaa gat tat gca aat gat gca gcg cac aag agc aaa gat tat gct ggt      960
Lys Asp Tyr Ala Asn Asp Ala Ala His Lys Ser Lys Asp Tyr Ala Gly
305                 310                 315                 320

gat gct tct cac aag agc aaa gaa gct tct gat tat gcc agt gag acg      1008
Asp Ala Ser His Lys Ser Lys Glu Ala Ser Asp Tyr Ala Ser Glu Thr
                325                 330                 335

gct cag aag acc aaa gac tat gtt ggt gat gct gct cag aaa agc aaa      1056
Ala Gln Lys Thr Lys Asp Tyr Val Gly Asp Ala Ala Gln Lys Ser Lys
            340                 345                 350

gag gct tct gaa tat gct agt gat gcg gct cag agg acc aaa gag tat      1104
Glu Ala Ser Glu Tyr Ala Ser Asp Ala Ala Gln Arg Thr Lys Glu Tyr
        355                 360                 365

gct ggt gat gcc acc aag aga agc aaa gag gct tct gat cat gcc aat      1152
Ala Gly Asp Ala Thr Lys Arg Ser Lys Glu Ala Ser Asp His Ala Asn
    370                 375                 380

gac atg gct cgg aag acc aaa gac tac gcc agt gac act gct cag aga      1200
Asp Met Ala Arg Lys Thr Lys Asp Tyr Ala Ser Asp Thr Ala Gln Arg
385                 390                 395                 400

acc aag gaa aaa ctc caa gac att gca tct gag gct ggt caa tac agt      1248

Thr Lys Glu Lys Leu Gln Asp Ile Ala Ser Glu Ala Gly Gln Tyr Ser
                405                 410                 415

aca gag aag gcc aga gaa atg aag gat gca gca gca gag aag gct agt      1296
Thr Glu Lys Ala Arg Glu Met Lys Asp Ala Ala Ala Glu Lys Ala Ser
            420                 425                 430

gac att gca aag gct gca aag caa aaa tca caa gag ctg aaa gac aaa      1344
Asp Ile Ala Lys Ala Ala Lys Gln Lys Ser Gln Glu Leu Lys Asp Lys
        435                 440                 445

tta ggt gga caa cac cgg gat gct gag ctt                              1374
Leu Gly Gly Gln His Arg Asp Ala Glu Leu
    450                 455

<210>  637
<211>  458
<212>  PRT
<213>  Glycine max

<400>  637

Met Ala Ser Arg Val Val Ser Val Leu Val Ile Ala Met Met Leu Phe
1               5                   10                  15

Ala Met Asn Cys Asn Cys Thr Ser Val Gly His Met Pro Ser Thr Lys
            20                  25                  30

Glu Glu Gly His Asp Phe Gln Glu Ala Lys Ala Lys Thr Thr Gln Thr
        35                  40                  45

Ala Asn Lys Ala Met Glu Thr Gly Lys Asp Gly Lys Glu Ala Ala Glu
    50                  55                  60

Ser Trp Thr Glu Trp Ala Lys Glu Lys Leu Thr Glu Gly Leu Gly Phe
65                  70                  75                  80

Lys His Asp Gln Glu Ser Thr Thr Lys Lys Val Ser Asp Tyr Ala Gly
                85                  90                  95

Asp Ala Ala Gln Lys Ser Lys Asp Tyr Ala Gly Asp Thr Ala Gln Lys
            100                 105                 110

Thr Lys Asp Tyr Ala Gly Asp Thr Ala Gln Lys Thr Lys Asp Tyr Ala
        115                 120                 125

Gly Asp Thr Ala Gln Thr Ser Lys Asp Tyr Ala Gly Asp Ala Ala Gln
    130                 135                 140

Lys Ser Lys Gly Tyr Val Gly Asp Ala Ala Gln Lys Thr Lys Glu Tyr
145                 150                 155                 160

Val Gly Asp Ala Ala Gln Lys Thr Met Asp Tyr Ala Thr Asp Ala Ala
                165                 170                 175

Gln Lys Thr Lys Asp Tyr Ala Thr Gln Lys Thr Lys Asp Tyr Ala Ser
            180                 185                 190

Asp Ala Thr Asp Ala Ala Lys Lys Thr Lys Asp Tyr Ala Ala Gln Lys
        195                 200                 205

Thr Lys Asp Tyr Ala Ser Asp Ala Thr Asp Ala Ala Lys Lys Thr Lys
    210                 215                 220

Asp Tyr Ala Ala Gln Lys Thr Lys Asp Tyr Ala Ser Gly Gly Ala Gln
225                 230                 235                 240

Lys Thr Lys Asp Tyr Ala Ser Gly Ala Ala Gln Lys Thr Lys Asp Tyr
                245                 250                 255

Ala Ser Asp Ala Ala Gln Arg Thr Lys Asp His Ala Ser Asp Gly Ala
            260                 265                 270

Gln Lys Ser Lys Glu Tyr Ala Gly Asp Val Ala Gln Asn Ala Lys Asp
        275                 280                 285

Tyr Ala Gln Lys Ser Lys Asp Tyr Ala Gly Asp Ala Val Gln Asn Ile
    290                 295                 300

Lys Asp Tyr Ala Asn Asp Ala Ala His Lys Ser Lys Asp Tyr Ala Gly
305                 310                 315                 320

Asp Ala Ser His Lys Ser Lys Glu Ala Ser Asp Tyr Ala Ser Glu Thr
                325                 330                 335

Ala Gln Lys Thr Lys Asp Tyr Val Gly Asp Ala Ala Gln Lys Ser Lys
            340                 345                 350

Glu Ala Ser Glu Tyr Ala Ser Asp Ala Ala Gln Arg Thr Lys Glu Tyr
        355                 360                 365

Ala Gly Asp Ala Thr Lys Arg Ser Lys Glu Ala Ser Asp His Ala Asn
    370                 375                 380

Asp Met Ala Arg Lys Thr Lys Asp Tyr Ala Ser Asp Thr Ala Gln Arg
385                 390                 395                 400

Thr Lys Glu Lys Leu Gln Asp Ile Ala Ser Glu Ala Gly Gln Tyr Ser
                405                 410                 415

Thr Glu Lys Ala Arg Glu Met Lys Asp Ala Ala Ala Glu Lys Ala Ser
            420                 425                 430

Asp Ile Ala Lys Ala Ala Lys Gln Lys Ser Gln Glu Leu Lys Asp Lys
        435                 440                 445

Leu Gly Gly Gln His Arg Asp Ala Glu Leu
    450                 455

<210>  638
<211>  11
<212>  PRT
<213>  Glycine max

<400>  638

Asp Tyr Ala Gly Asp Ala Val Gln Asn Ile Lys
1               5                   10

<210>  639
<211>  11
<212>  PRT
<213>  Glycine max

<400>  639

Asp Tyr Ala Ser Asp Ala Ala Gln Arg Thr Lys
1               5                   10

<210>  640
<211>  11
<212>  PRT
<213>  Glycine max

<400>  640

Asp Tyr Ala Ser Asp Ala Thr Asp Ala Ala Lys
1               5                   10

<210>  641
<211>  11
<212>  PRT
<213>  Glycine max

<400>  641

Asp Tyr Ala Ser Asp Thr Ala Gln Arg Thr Lys
1               5                   10

<210>  642
<211>  9
<212>  PRT
<213>  Glycine max

<400>  642

Asp Tyr Val Gly Asp Ala Ala Gln Lys
1               5

<210>  643
<211>  12
<212>  PRT
<213>  Glycine max

<400>  643

Glu Ala Ser Asp Tyr Ala Ser Glu Thr Ala Gln Lys
1               5                   10

<210>  644
<211>  14
<212>  PRT
<213>  Glycine max

<400>  644

Glu Ala Ser Glu Tyr Ala Ser Asp Ala Ala Gln Arg Thr Lys
1               5                   10

<210>  645
<211>  11
<212>  PRT
<213>  Glycine max

<400>  645

Glu Tyr Ala Gly Asp Val Ala Gln Asn Ala Lys
1               5                   10

<210>  646
<211>  9
<212>  PRT
<213>  Glycine max

<400>  646

Gly Tyr Val Gly Asp Ala Ala Gln Lys
1               5

<210>  647
<211>  15
<212>  PRT
<213>  Glycine max

<400>  647

Leu Gln Asp Ile Ala Ser Glu Ala Gly Gln Tyr Ser Thr Glu Lys
1               5                   10                  15

<210>  648
<211>  8
<212>  PRT

<213>  Glycine max

<400>  648

Leu Thr Glu Gly Leu Gly Phe Lys
1               5


<210>  649
<211>  11
<212>  PRT
<213>  Glycine max

<400>  649

Val Ser Asp Tyr Ala Gly Asp Ala Ala Gln Lys
1               5                   10